(19) **Europäisches Patentamt / European Patent Office / Office européen des brevets**

(11) **EP 2 791 683 B1**

(12) **EUROPEAN PATENT SPECIFICATION**

(45) Date of publication and mention of the grant of the patent:
**15.11.2017 Bulletin 2017/46**

(21) Application number: **12812560.6**

(22) Date of filing: **14.12.2012**

(51) Int Cl.:
**G01N 33/68** (2006.01)

(86) International application number:
**PCT/EP2012/075643**

(87) International publication number:
**WO 2013/087887 (20.06.2013 Gazette 2013/25)**

(54) **BIOMARKERS AND PARAMETERS FOR PREECLAMPSIA**

BIOMARKER UND PARAMETER FÜR PRÄEKLAMPSIE

BIOMARQUEURS ET PARAMÈTRES POUR PRÉ-ÉCLAMPSIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2011 EP 11193847**
**15.12.2011 US 201161576273 P**
**22.05.2012 EP 12168955**
**22.05.2012 US 201261650145 P**

(43) Date of publication of application:
**22.10.2014 Bulletin 2014/43**

(73) Proprietor: **Mycartis N.V.**
**9052 Gent (BE)**

(72) Inventors:
• **THOMAS, Gregoire**
**B-9160 Lokeren (BE)**
• **TUYTTEN, Robin**
**Glanmire Village,**
**Glanmire,**
**Co. Cork (IE)**
• **MOERMAN, Piet**
**B-9831 Deurle (BE)**

(74) Representative: **Denjean, Eric**
**Cabinet Laurent et Charras**
**Le Contemporain**
**50, chemin de la Bruyère**
**69574 Dardilly (FR)**

(56) References cited:
**WO-A1-2009/094665    WO-A1-2010/059952**
**WO-A2-2004/008946    WO-A2-2011/128357**
**KR-A- 20100 088 217**

• JISOOK PARK ET AL: "Discovery of the serum biomarker proteins in severe preeclampsia by proteomic analysis", EXPERIMENTAL AND MOLECULAR MEDICINE, vol. 43, no. 7, 1 January 2011 (2011-01-01), page 427, XP055019907, ISSN: 1226-3613, DOI: 10.3858/emm.2011.43.7.047
• ROHRA: "Biochemical screening for the prediction of preeclampsia", JOURNAL OF THE PAKISTAN MEDICAL ASSOCIATION, vol. 55, no. 2, 1 January 2005 (2005-01-01), page 79, XP055045761, ISSN: 0030-9982
• KAAJA R: "Predictors and risk factors of pre-eclampsia", MINERVA GINECOLOGICA, TORINO, IT, vol. 60, no. 5, 1 October 2008 (2008-10-01), pages 421-429, XP009156780, ISSN: 0026-4784
• LEWITT ET AL: "Regulation of insulin-like growth factor-binding protein-3 ternary complex formation in pregnancy", JOURNAL OF ENDOCRINOLOGY, vol. 159, no. 2, 1 November 1998 (1998-11-01), pages 265-274, XP055001958, ISSN: 0022-0795, DOI: 10.1677/joe.0.1590265

Remarks:
The file contains technical information submitted after the application was filed and not included in this specification

SEQUENCE LISTING

<110> PRONOTA N.V.

<120> BIOMARKERS AND PARAMETERS FOR HYPERTENSIVE DISORDERS OF PREGNANCY

<130> PRNO-045-PCT

<150> 11193847.8
<151> 2011-12-15

<150> 61/576,273
<151> 2011-12-15

<150> 12168955.8
<151> 2012-05-22

<150> 61/650,145
<151> 2012-05-22

<160> 38

<170> PatentIn version 3.3

<210> 1
<211> 9
<212> PRT
<213> Homo sapiens

<400> 1

```
Glu Ala Glu Pro Leu Val Asp Ile Arg
1               5
```

<210> 2
<211> 8
<212> PRT
<213> Homo sapiens

<400> 2

```
Glu Leu Ile Ile Asn Leu Glu Arg
1               5
```

<210> 3
<211> 10
<212> PRT
<213> Homo sapiens

<400> 3

```
Ala Asp Glu Val Val Ser Ala Ser Val Arg
1               5                   10
```

<210> 4
<211> 13

<212> PRT
<213> Homo sapiens

<400> 4

Asn Val Ala Leu Val Ser Gly Asp Thr Glu Asn Ala Lys
1               5               10

<210> 5
<211> 15
<212> PRT
<213> Homo sapiens

<400> 5

Glu Val Gly Pro Pro Leu Pro Gln Glu Ala Val Pro Leu Gln Lys
1               5               10                  15

<210> 6
<211> 13
<212> PRT
<213> Homo sapiens

<400> 6

Leu Pro Asp Thr Pro Gln Gly Leu Leu Gly Glu Ala Arg
1               5               10

<210> 7
<211> 11
<212> PRT
<213> Homo sapiens

<400> 7

Thr Tyr Ser Val Glu Tyr Leu Asp Ser Ser Lys
1               5               10

<210> 8
<211> 11
<212> PRT
<213> Homo sapiens

<400> 8

Leu Glu Pro Gly Gln Gln Glu Glu Tyr Tyr Arg
1               5               10

<210> 9
<211> 10
<212> PRT
<213> Homo sapiens

<400> 9

Tyr Ile Ser Ile Gly Ser Glu Ala Glu Lys
1               5               10

<210> 10
<211> 17
<212> PRT
<213> Homo sapiens

<400> 10

```
Glu Gly Gly Leu Gly Pro Leu Asn Ile Pro Leu Leu Ala Asp Val Thr
1               5               10                  15

Arg
```

<210> 11
<211> 10
<212> PRT
<213> Homo sapiens

<400> 11

```
Glu Ser Asp Thr Ser Tyr Val Ser Leu Lys
1               5               10
```

<210> 12
<211> 9
<212> PRT
<213> Homo sapiens

<400> 12

```
Phe Phe Asp Ala Asn Tyr Asp Gly Lys
1               5
```

<210> 13
<211> 14
<212> PRT
<213> Homo sapiens

<400> 13

```
Tyr Tyr Gly Glu Ser Leu Pro Phe Gly Asp Asn Ser Phe Lys
1               5               10
```

<210> 14
<211> 13
<212> PRT
<213> Homo sapiens

<400> 14

```
Ser Leu Asp Glu Ile Ser Gln Pro Ala Gln Glu Leu Lys
1               5               10
```

<210> 15
<211> 10
<212> PRT
<213> Homo sapiens

<400> 15

```
Tyr Thr Ser Gly Phe Asp Glu Leu Gln Arg
1               5                   10
```

<210> 16
<211> 10
<212> PRT
<213> Homo sapiens

<400> 16

```
Leu Pro Thr Asp Ser Glu Leu Ala Pro Arg
1               5                   10
```

<210> 17
<211> 13
<212> PRT
<213> Homo sapiens

<400> 17

```
Leu Ala Gly Ala Pro Ser Glu Asp Pro Gln Phe Pro Lys
1               5                   10
```

<210> 18
<211> 14
<212> PRT
<213> Homo sapiens

<400> 18

```
Leu Ala Glu Leu Pro Ala Asp Ala Leu Gly Pro Leu Gln Arg
1               5                   10
```

<210> 19
<211> 14
<212> PRT
<213> Homo sapiens

<400> 19

```
Gly Ile Leu Ala Ala Asp Glu Ser Thr Gly Ser Ile Ala Lys
1               5                   10
```

<210> 20
<211> 15
<212> PRT
<213> Homo sapiens

<400> 20

```
Gly Ala Thr Leu Ala Leu Thr Gln Val Thr Pro Gln Asp Glu Arg
1               5                   10                  15
```

<210> 21
<211> 8
<212> PRT

<213> Homo sapiens

<400> 21

```
                          Glu Asp Phe Gln Ile Gln Pro Arg
                          1               5
```

<210> 22
<211> 12
<212> PRT
<213> Homo sapiens

<400> 22

```
                      Asp Ile Glu His Leu Thr Ser Leu Asp Phe Phe Arg
                      1               5                   10
```

<210> 23
<211> 11
<212> PRT
<213> Homo sapiens

<400> 23

```
                      Ala Leu Asp Phe Ala Val Gly Glu Tyr Asn Lys
                      1               5                   10
```

<210> 24
<211> 14
<212> PRT
<213> Homo sapiens

<400> 24

```
                  Gly Thr Val Asp Glu Phe Ser Gly Ala Glu Ile Val Asp Lys
                  1               5                   10
```

<210> 25
<211> 14
<212> PRT
<213> Homo sapiens

<400> 25

```
                  Gly Ser Ile Gln Val Asp Gly Glu Glu Leu Val Ser Gly Arg
                  1               5                   10
```

<210> 26
<211> 11
<212> PRT
<213> Homo sapiens

<400> 26

```
                      Thr Val Thr Val Glu Asp Leu Glu Pro Gly Lys
                      1               5                   10
```

<210> 27

<211> 15
<212> PRT
<213> Homo sapiens

<400> 27

```
Ser Asp Phe Tyr Asp Ile Val Leu Val Ala Thr Pro Leu Asn Arg
1               5                   10                  15
```

<210> 28
<211> 14
<212> PRT
<213> Homo sapiens

<400> 28

```
Gly Pro Ile Leu Glu Ala Thr Ala Gly Asp Glu Leu Val Lys
1               5                   10
```

<210> 29
<211> 10
<212> PRT
<213> Homo sapiens

<400> 29

```
Ala Thr Ala Val Val Asp Gly Ala Phe Lys
1               5                   10
```

<210> 30
<211> 149
<212> PRT
<213> Homo sapiens

<400> 30

```
Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
                20                  25                  30

Asn Gly Ser Ser Glu Val Glu Val Val Pro Phe Gln Glu Val Trp Gly
                35                  40                  45

Arg Ser Tyr Cys Arg Ala Leu Glu Arg Leu Val Asp Val Val Ser Glu
                50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95
```

```
Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg Pro Leu Arg Glu Lys Met Lys Pro Glu Arg Cys Gly Asp
        130                 135                 140

Ala Val Pro Arg Arg
145
```

<210> 31
<211> 221
<212> PRT
<213> Homo sapiens

<400> 31

```
Met Pro Val Met Arg Leu Phe Pro Cys Phe Leu Gln Leu Leu Ala Gly
1               5               10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
            35                  40                  45

Arg Ser Tyr Cys Arg Ala Leu Glu Arg Leu Val Asp Val Val Ser Glu
        50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg His Ser Pro Gly Arg Gln Ser Pro Asp Met Pro Gly Asp
        130                 135                 140

Phe Arg Ala Asp Ala Pro Ser Phe Leu Pro Pro Arg Arg Ser Leu Pro
145                 150                 155                 160
```

```
Met Leu Phe Arg Met Glu Trp Gly Cys Ala Leu Thr Gly Ser Gln Ser
                165             170             175

Ala Val Trp Pro Ser Ser Pro Val Pro Glu Glu Ile Pro Arg Met His
            180             185             190

Pro Gly Arg Asn Gly Lys Lys Gln Gln Arg Lys Pro Leu Arg Glu Lys
            195             200             205

Met Lys Pro Glu Arg Cys Gly Asp Ala Val Pro Arg Arg
    210             215             220
```

<210> 32
<211> 170
<212> PRT
<213> Homo sapiens

<400> 32

```
Met Pro Val Met Arg Leu Phe Pro Cys Phe Leu Gln Leu Leu Ala Gly
1               5               10              15

Leu Ala Leu Pro Ala Val Pro Pro Gln Gln Trp Ala Leu Ser Ala Gly
            20              25              30

Asn Gly Ser Ser Glu Val Glu Val Val Pro Phe Gln Glu Val Trp Gly
            35              40              45

Arg Ser Tyr Cys Arg Ala Leu Glu Arg Leu Val Asp Val Val Ser Glu
    50              55              60

Tyr Pro Ser Glu Val Glu His Met Phe Ser Pro Ser Cys Val Ser Leu
65              70              75              80

Leu Arg Cys Thr Gly Cys Cys Gly Asp Glu Asn Leu His Cys Val Pro
            85              90              95

Val Glu Thr Ala Asn Val Thr Met Gln Leu Leu Lys Ile Arg Ser Gly
            100             105             110

Asp Arg Pro Ser Tyr Val Glu Leu Thr Phe Ser Gln His Val Arg Cys
            115             120             125

Glu Cys Arg Pro Leu Arg Glu Lys Met Lys Pro Glu Arg Arg Arg Pro
            130             135             140

Lys Gly Arg Gly Lys Arg Arg Arg Glu Lys Gln Arg Pro Thr Asp Cys
145             150             155             160
```

```
                    His Leu Cys Gly Asp Ala Val Pro Arg Arg
                                        165                 170
```

<210> 33
<211> 10
<212> PRT
<213> Homo sapiens


<400> 33

```
                    Ile Leu Asp Tyr Glu Val Thr Leu Thr Arg
                    1               5                   10
```

<210> 34
<211> 13
<212> PRT
<213> Homo sapiens


<400> 34

```
              Gly Asp Ser Pro Trp Gln Val Val Leu Leu Asp Ser Lys
              1               5                   10
```

<210> 35
<211> 11
<212> PRT
<213> Homo sapiens


<400> 35

```
                 Asn Pro Ala Tyr Glu Val Asp Val Gln Ala Arg
                 1               5                   10
```

<210> 36
<211> 16
<212> PRT
<213> Homo sapiens


<400> 36

```
        Val Ala Gly Leu Leu Glu Asp Thr Phe Pro Gly Leu Leu Gly Leu Arg
        1               5                   10                  15
```

<210> 37
<211> 8
<212> PRT
<213> Homo sapiens


<400> 37

```
                       Ala Asp Glu Gly Ile Ser Phe Arg
                       1               5
```

<210> 38
<211> 8
<212> PRT
<213> Homo sapiens

<400> 38

```
Asp Leu Glu Thr Ser Leu Glu Lys
1               5
```

**Description**

**FIELD OF THE INVENTION**

The invention relates to biomarkers and parameters useful for the prediction of preeclampsia in a subject.

**BACKGROUND OF THE INVENTION**

In many diseases and conditions, a favourable outcome of prophylactic and/or therapeutic treatments is strongly correlated with early and/or accurate prediction, diagnosis, prognosis and/or monitoring of a disease or condition. Therefore, there exists a continuous need for additional and preferably improved manners for early and/or accurate prediction, diagnosis, prognosis and/or monitoring of diseases and conditions to guide the treatment choices.

Hypertensive disorders occurring during pregnancy represent a major cause of maternal morbidity and mortality worldwide, and are also associated with increased perinatal mortality.

A prominent place among hypertensive disorders of pregnancy belongs to preeclampsia (PE), which develops in about 5% to 10% of pregnant females (Solomon & Seely 2006, Endocrinol Metab Clin North Am 35(1): 157-71, vii).

PE may be described as new onset hypertension and proteinuria past 20 weeks gestation in a previously normotensive pregnant female, which may be mild or severe. Patients with mild disease display blood pressures > 140/90 and proteinuria with >300mg protein noted on a 24 hour urine sample after 20 weeks gestation, and usually deliver near term without significant co-morbidities. However, about 25% of PE tends to be severe, involving symptoms and signs of central nervous system dysfunction, hepatocellular injury, reduced urine output and markedly elevated blood pressure (systolic >160 mmHg or diastolic >1 10 mmHg). Severe PE typically occurs in late 2nd and early 3rd trimester and is associated with increased maternal and perinatal morbidity and mortality.

Severe complications of PE include 1) HELLP syndrome characterised by haemolysis, elevated liver enzymes and low platelets, and 2) eclampsia characterised by the development of seizures. Whereas both these conditions are rare, they are associated with poor prognosis (Solomon & Seely 2006, supra).

Preeclampsia is also associated with foetal complications such as intrauterine growth retardation (IUGR) and small for gestational age (SGA).

The only cure for PE is delivery of the baby and placenta. Beyond 37 weeks of gestation, delivery is warranted. At gestational ages of less than 34 weeks, treatment of hypertension and close foetal surveillance may prevent cerebral vascular accidents and prolong the pregnancy, without curing the underlying disease process. Delivery is also warranted for development of severe PE or eclampsia (Sibai & Barton 2007, Am J Obstet Gynecol 196(6):514.e1 -9).

The aetiology and pathophysiology of PE remains largely unresolved and its diagnosis is currently based entirely on clinical criteria once the disease unfolds (Roberts et al. 2003, Hypertension 41 (3): 37-45). However, recent data suggests that events leading to PE may begin and progress insidiously as early as 1 st trimester.

Dependable and early prediction and/or diagnosis is therefore crucial for successful treatment interventions in hypertensive disorders of pregnancy including inter alia PE. Consequently, provision of further, alternative and preferably improved methods and means for diagnosis, prediction, prognosis and/or monitoring of hypertensive disorders of pregnancy continues to be of prime importance.

However, clinically useful screening tests to predict the development of PE are sparse (Conde-Agudelo et al. 2004, Obstet Gynecol 104: 1367-91). Reliance on risk factors is also substandard, since (although several risk factors for PE have been identified) over 50% of cases occur among otherwise young, low risk, nulliparous females. Hence, hypertensive disorders of pregnancy and particularly PE remain largely unpredictable in their onset and disease progression.

Lewitt et al. 1998 (Journal of Endocrinology 159: 265) mentioned that the insulin-like growth factor (IGF) system is believed to be important in pregnancy and implicated in the pathophysiology of pre-eclampsia.

Mistry et al. 2008 (Hypertension 52: 881) reported reduced selenium concentrations and glutathione peroxidase activity in preeclamptic pregnancies.

Rayman et al. 2003 (Am J Obstet Gynecol 189: 1343) observed that median toenail selenium concentrations in preeclamptic subjects were significantly lower than in their matched controls.

WO 2009/094665 to PerkinElmer Health Sciences Inc. concerns methods for determining the risk of preeclampsia in a pregnant individual using a test panel comprising the level of placental growth factor (PIGF) and the level of pregnancy-associated plasma protein A (PAPP-A) in a blood sample from a subject and the measurement of blood pressure in the subject.

WO 2011/128357 to Pronota NV describes several new biomarkers for hypertensive disorders of pregnancy, more in particular preeclampsia.

Park et al (Experimental and Molecular Medicine (2011) 43(7): 427-435) identifies a2-HS-glycoprotein (AHSG), retinol binding protein 4 (RBP4), a -1-micro-globulin/bikunin (AMBP) and acid labile subunit (IGFBP-ALS) as having statistically significant increased expression levels in serum samples taken from pregnant women with significant preeclampsia compared to controls.

WO 2004/008946 discloses methods for diagnosing pre-eclampsia and eclampsia using vascular endothelial growth factor (VEGF), placental growth factor (PIGF) and the soluble form of the fms-like tyrosine kinase receptor (sFlt-1).

WO 2010/059952 discloses a method for determining the risk of pre-eclampsia in a pregnant woman using PIGF-2 and PIGF-3 markers.

Rohra and Qidwai (J. Pakistan Medical Association (2005) 55(2): 79-82) provides a preview of biochemical screening for the prediction of pre-eclampsia, in which the roles of inter alla sFlt-1, PIGF and VEGF polypeptides are discussed.

## SUMMARY OF THE INVENTION

Having conducted extensive experiments and tests, the inventors identified panels comprising biomarker(s) and/or clinical parameter(s), said panels being closely predictive and/or indicative of hypertensive disorders of pregnancy (henceforth "HDP"), more specifically preeclampsia (henceforth "PE").

The invention thus pertains to an *in vitro* method for the prediction of pre-eclampsia (PE) in a subject comprising testing or evaluating a sample obtained from the subject, the method comprising:

- measurement of the level of placental growth factor (PIGF) in the subject; and

- measurement of the level of insulin-like growth factor-binding protein complex acid labile subunit (IGFALS) in the subject.

Preferably, the in vitro method according to the invention further comprises:

- i) measurement of the level of multimerin-2 (MMRN2) in the subject.

Preferably, the in vitro method of the invention further comprises two or more measurements selected from the group consisting of: measurement of the level of cell surface glycoprotein (CD146, MUC18, MCAM) in the subject, measurement of the level of endoglin (soluble endoglin, s-ENG or ENG) in the subject, measurement of the level of disintegrin and metalloproteinase domain-containing protein 12 (ADAM12) in the subject, measurement of the level of multimerin-2 (MMRN2) in the subject, measurement of the level of Kunitz-type protease inhibitor 1 (SPINT1) in the subject, measurement of the level of sulfhydryl oxidase 1 (QSOX1) in the subject, measurement of the level of selenoprotein P (SEPP1) in the subject, measurement of the level of extracellular matrix protein 1 (ECM1) in the subject, measurement of the level of roundabout homolog 4 (ROBO4) in the subject, measurement of the level of leucyl-cystinyl aminopeptidase (LNPEP, OTASE) in the subject, measurement of the level of fructose-bisphosphate aldolase A (ALDOA) in the subject, measurement of the level of microtubule-associated protein RP (MAPRE1 and/or 3) in the subject, measurement of the level of ectonucleotide pyrophosphatase/phosphodiesterase family member 2 (ENPP2) in the subject, measurement of the level of phosphatidylcholine-sterol acyltransferase (LCAT) in the subject, measurement of the level of peroxiredoxin-2 (PRDX2) in the subject, measurement of the level of lysosomal Pro-X carboxypeptidase (PRCP) in the subject, measurement of the level of trefoil factor 3 (TFF3) in the subject, measurement of the level of cystatin-C (CST3) in the subject, measurement of the level of C-reactive protein (CRP) in the subject, measurement of the level of collagen alpha-3(VI) chain (COL6A3) in the subject, measurement of the level of Interleukin-6 receptor subunit beta (IL6ST) in the subject, measurement of the level of Vitamin K-dependent protein C (PROC) in the subject, measurement of the level of Protocadherin-12 (PCDH12) in the subject, measurement of blood pressure of the subject (BP), a score for the parameter 'alcohol consumed in the 1 st trimester' (yes/no) (esp. 1 st trimester) (*"alcoh"*), measurement of body mass index of the subject (bmi), a score for the maternal history parameter 'father of subject has/had ischemic heart disease' in the subject (*"father any ihd"* or *"fihd"*), a score for the maternal history parameter 'mother or sister of subject has/had preeclampsia' in the subject (*"ft_pet"* or *"fhpet'*), a score for the parameter 'occurrence of vaginal bleeding (esp. for (more than) 5 days before 15 weeks visit)' (yes/no) (*"vagbl"*), a value for the parameter 'birth weight of the subject' (*"pbwgr"*), a value for the parameter 'the gestational age at blood sampling calculated from the date of the last menstrual period and/or from an ultrasound dating scan' ("g*est"*), a value for the parameter 'age of the subject' ("*age"*), a score for the maternal history parameter 'mother of subject has/had preeclampsia' (*"mothpet"*), a score for the maternal history parameter 'sister of subject has/had preeclampsia' (*"sispet"*), and measurement of the waist circumference in the subject ("*waist"*).

Preferably, in the method of the invention, the sample is whole blood, plasma, serum, or a cell pellet.

As herein disclosed, additional and markedly improved methods and means for diagnosis, prediction, prognosis and/or monitoring of HDP or particularly PE in a subject, more preferably for prediction of HDP or particularly PE in the subject, are realised through provision of a test panel comprising or consisting of two or more constituents selected from the group consisting of: measurement of the level of insulin-like growth factor-binding protein complex acid labile subunit (IGFALS) in the subject, measurement of the level of cell surface glycoprotein (CD146, MUC18, MCAM) in the subject, measurement of the level of endoglin (soluble endoglin, s-ENG or ENG) in the subject, measurement of the level of disintegrin and metalloproteinase domain-containing protein 12 (ADAM12) in the subject, measurement of the level of placental growth factor (PIGF) in the subject, measurement of the level of multimerin-2 (MMRN2) in the subject, measurement of the level of Kunitz-type protease inhibitor 1 (SPINT1) in the subject, measurement of the level of sulfhydryl oxidase 1 (QSOX1) in the subject, measurement of the level of selenoprotein P (SEPP1) in the subject, measurement of the level of extracellular matrix protein 1 (ECM1) in the subject, measurement of the level of roundabout homolog 4 (ROB04) in the subject, measurement of the level of leucyl-cystinyl aminopeptidase (LNPEP, OTASE) in the subject, measurement of the level of fructose-bisphosphate aldolase A (ALDOA) in the subject, measurement of the level of microtubule-associated protein RP (in particular, measurement of MAPRE1 and/or MAPRE3) in the subject, measurement of the level of ectonucleotide pyrophosphatase/phosphodiesterase family member 2 (ENPP2) in the subject, measurement of the level of phosphatidylcholine-sterol acyltransferase (LCAT) in the subject, measurement of the level of peroxiredoxin-2 (PRDX2) in the subject, measurement of the level of lysosomal Pro-X carboxypeptidase (PRCP) in the subject, measurement of the level of trefoil factor 3 (TFF3) in the subject, measurement of the level of cystatin-C (CST3) in the subject, measurement of the level of C-reactive protein (CRP) in the subject, measurement of the level of collagen alpha-3(VI) chain (COL6A3) in the subject, measurement of the level of Interleukin-6 receptor subunit beta (IL6ST) in the subject, measurement of the level of Vitamin K-dependent protein C (PROC) in the subject, measurement of the level of Protocadherin-12 (PCDH12) in the subject, measurement of blood pressure of the subject (BP), a score for the parameter 'alcohol consumed in the 1st trimester' (yes/no) (esp. 1st trimester) (*"alcoh"*), measurement of body mass index of the subject (bmi), a score for the maternal history parameter 'father of subject has/had ischemic heart disease' in the subject (*"father_any_ihd"* or *"fihd'*, a score for the maternal history parameter 'mother or sister of subject has/had preeclampsia' in the subject (*"fh_pet"* or *"fhpet"*, a score for the parameter 'occurrence of vaginal bleeding (esp. for (more than) 5 days before 15 weeks visit)' (yes/no) (*"vagbf"*, a value for the parameter 'birth weight of the subject' (*"pbwgt"*, a value for the parameter 'the gestational age at blood sampling calculated from the date of the last menstrual period and/or from an ultrasound dating scan' (*"gent"*, a value for the parameter 'age of the subject' ("*age*"), a score for the maternal history parameter 'mother of subject has/had preeclampsia' (*"mothpet"*, a score for the maternal history parameter 'sister of subject has/had preeclampsia' ("*sispet"*, and measurement of the waist circumference in the subject ("*waist"*).

As herein disclosed, additional and markedly improved methods and means for diagnosis, prediction, prognosis and/or monitoring of HDP or particularly PE in a subject, more preferably for prediction of HDP or particularly PE in the subject, are realised through provision, , of a test panel comprising or consisting of two or more constituents selected from the group consisting of: measurement of the level of IGFALS in the subject, measurement of the level of MCAM in the subject, measurement of the level of s-ENG in the subject, measurement of the level of ADAM 12 in the subject, measurement of the level of PIGF in the subject, measurement of the level of MMRN2 in the subject, measurement of the level of SPINT1 in the subject, measurement of the level of QSOX1 in the subject, measurement of the level of SEPP1 in the subject, measurement of the level of ECM1 in the subject, measurement of the level of ROB04 in the subject, measurement of the level of LNPEP in the subject, measurement of the level of ALDOA in the subject, measurement of the level of MAPRE1 and/or MAPRE3 in the subject, measurement of the level of ENPP2 in the subject, measurement of the level of LCAT in the subject, measurement of the level of PRDX2 in the subject, measurement of the level of PRCP in the subject, measurement of the level of TFF3 in the subject, measurement of the level of CST3 in the subject, measurement of the level of CRP in the subject, measurement of the level of COL6A3 in the subject, measurement of BP of the subject (BP), a score for *alcoh,* measurement of bmi of the subject, a score for *father_any_ihd,* a score for *fh_pet,* a score for *vagbl,* a value for *pbwgt,* a value for gest, a value for *age,* a score for *mothpet,* a score for *sispet,* and measurement of *waist.*

For the sake of conciseness, IGFALS, MCAM, ENG, ADAM 12, PIGF, MMRN2, SPINT1, QSOX1, SEPP1 , ECM1 , ROB04, LNPEP, ALDOA, MAP RE 1/3, ENPP2, LCAT, PRDX2, PRCP, TFF3, CST3, CRP, COL6A3, IL6ST, PROC, and PCDH 12 may also be denoted or referred to throughout the present specification as "markers" or "biomarkers". BP, *alcoh,* bmi, *fihd, fhpet, vagbl, pbwgt, gest, age, mothpet, sispet* and waist may also be denoted or referred to throughout the present specification as *"clinical parameters"* or *"clinicals".* Further, the phrase "measurement of the level of [a biomarker]" may be used herein synonymously with phrases such as "measurement of [a biomarker]", "[a biomarker] level" or even simply "[a biomarker]" provided the context does not dictate otherwise. Hence, by means of example, "a test panel comprising IGFALS" denotes a test panel comprising the measurement of the level of IGFALS in a subject.

Further, the peptide FFDANYDGK (SEQ ID NO: 12) used in the examples section (see Table 3) to measure the level of microtubule-associated protein RP is present both in MAPRE1 and in MAPRE3. Accordingly, the present specification may suitably refer to measurement of the level of MAPRE1 and/or MAPRE3, which denotes measurement of the level of any one or both of MAPRE1 and MAPRE3, i.e., measurement of the level of MAPRE1 or measurement of the level of MAPRE3 or measurement of the level of MAPRE1 and MAPRE3 (separately or collectively). For reasons of conciseness, phrases "measurement of the level of MAPRE1/3", "measurement of MAPRE1/3", "MAPRE1/3 level" or simply "MAPRE1/3" are intended to denote the measurement of the level of MAPRE1 and/or MAPRE3, such as, the measurement of the level of MAPRE1 , or, preferably, the measurement of the level of MAPRE1 and MAPRE3. Furthermore, in any one test panel disclosed throughout this specification, the measurement of the level of selenoprotein P (SEPP) may be supplemented or substituted by the measurement of the level of selenium. Hence, for any one panel specified herein as comprising the measurement of the level of selenoprotein P (SEPP1), the present specification also discloses an otherwise identical panel comprising the measurement of the level of selenium instead of the measurement of the level of SEPP1, as well as another otherwise identical panel comprising the measurement of the level of selenium in addition to the measurement of the level of SEPP1. The measurement of the level of at least or only SEPP1 may, however, be preferred.

In particularly preferred embodiments, the biomarkers used in the present test panels may be protein-, polypeptide- or peptide-based biomarkers. Particularly preferred, such protein-, polypeptide- or peptide-based biomarkers can be detected in blood, plasma or serum samples.

Any test panel disclosed in the present specification may comprise or consist of three or more of the aforementioned constituents, such as preferably between 3 and 6 constituents, for example 3, 4, 5 or 6 constituents.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of three or more constituents, such as preferably between 3 and 6 constituents, for example 3, 4, 5 or 6 constituents, said constituents selected from the group consisting of: IGFALS, MCAM, ENG, ADAM 12, PIGF, MMRN2, SPINT1 , QSOX1, SEPP1, ECM1 , ROB04, LNPEP, ALDOA, MAP RE 1/3, ENPP2, LCAT, PRDX2, PRCP, TFF3, CST3, CRP, COL6A3, IL6ST, PROC, and PCDH12, BP, *alcoh,* bmi, *fihd, fhpet, vagbl, pbwgt, gest, age, mothpet, sispet* and *waist;* or selected from the group consisting of:IGFALS, MCAM, ENG, ADAM12, PIGF, MMRN2, SPINT1, QSOX1, SEPP1, ECM1 , ROB04, LNPEP, ALDOA, MAPRE1/3, ENPP2, LCAT, PRDX2, PRCP, TFF3, CST3, CRP, COL6A3,BP, *alcoh,* bmi, *fihd, fhpet, vagbl, pbwgt, gest, age, mothpet, sispet* and *waist;* or selected from the group consisting of: IGFALS, MCAM, ENG, ADAM12, PIGF, MMRN2, SPINT1, QSOX1, SEPP1, ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age,* and *mothpet.*

Preferably, clinical parameters comprised in any test panel disclosed in the present specification may be clinical parameters that can be objectively measured in the subject, such as by routine analytical methods. Such clinical parameters tend to be comparatively more reliable. Examples of such clinical parameters include blood pressure (BP), body mass index (bmi) and waist circumference.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of: IGFALS, MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, and PCDH12, BP, bmi, and *waist;* or selected from the group consisting of: IGFALS, MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, PRCP, TFF3, CST3, CRP, COL6A3, BP, bmi, and *waist;* or selected from the group consisting of: IGFALS, MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, PRCP, BP, bmi, and *waist;* or selected from the group consisting of: IGFALS, MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, PRCP, BP, and bmi.

Hence, preferably, any test panel disclosed in the present specification may exclude (i.e., not comprise) anamnesis-based clinical parameters,which tend to be less reliable, such as in particular the clinical parameters *alcoh, fihd, fhpet, vagbl, pbwgt, gest, mothpet,* and *sispet.* Some clinical parameters, such as age and pbwgt, may be reliable in populations where reports such as birth and medical reports are well kept, but comparatively less reliable in societies where such report keeping is substandard.

Preferably, any test panel disclosed in the present specification may exclude all clinical parameters (i.e., biomarker-only panel), or may exclude the clinical parameters BP, *alcoh,* BP, *fihd, fhpet, vagbl, pbwgt, gest, age* and *mothpet,* while including one or more other clinical parameters.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of: IGFALS, MCAM, ENG, ADAM 12, PIGF, MMRN2, SPINT1 , QSOX1, SEPP1, ECM1, ROB04, LNPEP, ALDOA, MAPRE1/3, ENPP2, LCAT, PRDX2, PRCP, TFF3, CST3, CRP, COL6A3, IL6ST, PROC, and PCDH12; or selected from the group consisting of: IGFALS, MCAM, ENG, ADAM 12, PIGF, MMRN2, SPINT1, QSOX1 , SEPP1 ,

ECM1 , ROB04, LNPEP, ALDOA, MAPRE1/3, ENPP2, LCAT, PRDX2, PRCP, TFF3, CST3, CRP, COL6A3; or selected from the group consisting of: IGFALS, MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, and PRCP.

Further it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from:

a) the group consisting of: IGFALS, MCAM, ENG, ADAM12, PIGF, MMRN2, SPINT1, QSOX1, SEPP1 , ECM1 , ROB04, LNPEP, ALDOA, MAP RE 1/3, ENPP2, LCAT, PRDX2, PRCP, BP, *alcoh,* bmi, *fihd, fhpet, vagbl, pbwgt, gest, age* and *mothpet*; or

b) the group consisting of: IGFALS, MCAM, ENG, ADAM12, PIGF, MMRN2, SPINT1, QSOX1, SEPP1 , ECM1 , ROB04, LNPEP, ALDOA, MAPRE1/3, ENPP2, LCAT, PRDX2, PRCP, BP and bmi; or

c) the group consisting of: IGFALS, MCAM, ENG, ADAM12, PIGF, MMRN2, SPINT1, QSOX1, SEPP1 , ECM1 , ROB04, LNPEP, ALDOA, MAPRE1/3, ENPP2, LCAT, PRDX2 and PRCP.

Exemplary, non-limiting test panels embodying the principles as herein disclosed include those individualised in rows 1 to 1008 of Table 4A, as well as test panels as defined herein which comprise those individualised in rows 1 to 1008 of Table 4A.

It shall be appreciated throughout this specification that while certain test panels of Tables 4A, 5Ma, 5Na, and 12 may include either BP15 or BP20, this not only individualises said panels, but is also meant to individualise otherwise identical panels containing the measurement of blood pressure (BP) in general (i.e., without distinction between BP15 and BP20).

Further exemplary, non-limiting test panels embodying the principles as herein disclosed include the 2-constituent panels individualised in the rows of Table 13F, as well as 3- or more-constituent panels as defined herein which comprise the so-individualised panels.

Further exemplary, non-limiting test panels embodying the principles as herein disclosed include the 3-constituent panels individualised in the rows of Table 13G, as well as 4- or more-constituent panels as defined herein which comprise the so-individualised panels.

Further exemplary, non-limiting test panels embodying the principles as herein disclosed include the 3-constituent panels individualised in the rows of Table 13H, as well as 4- or more-constituent panels as defined herein which comprise the so-individualised panels.

It is further disclosed exemplary, non-limiting test panels as defined in any one of (i.i) to (i.xvi) as set forth below:

(i.i) A test panel for the prediction of hypertensive disorders of pregnancy (HDP), preferably preeclampsia (PE), in a subject, the test panel comprising or consisting of:

-    measurement of the level of placental growth factor (PIGF) in the subject; and
-    measurement of the level of insulin-like growth factor-binding protein complex acid labile subunit (IGFALS) in the subject.

(i.ii) The test panel as set forth in (i.i) above, wherein the test panel further comprises:

-    measurement of the level of cell surface glycoprotein MUC18 (MCAM) in the subject; and/or
-    measurement of blood pressure (BP) of the subject.

(i.iii) The test panel as set forth in (i.ii) above, wherein blood pressure is measured at either about 15 or about 20 weeks of gestation.

(i.iv) The test panel as set forth in any one of (i.i) to (i.iii) above, wherein the test panel further comprises:

-    measurement of the level of endoglin (ENG) in the subject; and/or
-    measurement of the level of Kunitz-type protease inhibitor 1 (SPINT1) in the subject.

(i.v) The test panel as set forth in any one of (i.i) to (i.iv) above, wherein the test panel further comprises:

- measurement of the level of multimerin-2 (MMRN2) in the subject; and/or
- measurement of the level of disintegrin and metalloproteinase domain containing protein 12 (ADAM 12); and/or
- measurement of a value for the gestational age at blood sampling calculated from the date of the last menstrual period and/or from an ultrasound dating scan ("*gest*").

(i.vi) A test panel for the prediction of hypertensive disorders of pregnancy (HDP), preferably preeclampsia (PE), in a subject, the test panel comprising or consisting of:

- measurement of the level of disintegrin and metalloproteinase domain containing protein 12 (ADAM 12) in the subject; and
- - measurement of the level of insulin-like growth factor-binding protein complex acid labile subunit (IGFALS) in the subject; and
- - measurement of the level of placental growth factor (PIGF) in the subject.

(i.vii) The test panel as set forth in (i.vi), wherein the test panel further comprises:

- - measurement of the level of multimerin-2 (MMRN2) in the subject; and/or
- - measurement of blood pressure (BP) of the subject.

(i.viii) The test panel as set forth in (i.vii) above, wherein blood pressure is measured at either about 15 or about 20 weeks of gestation.

(i.ix) The test panel as set forth in any one of (i.vi) to (i.viii) above, wherein the test panel further comprises:

- measurement of the level of cell surface glycoprotein MUC18 (MCAM) in the subject; and/or
- measurement of the level of endoglin (ENG) in the subject; and/or
- measurement of the level of (SPINT1) in the subject.

(i.x) A test panel for the prediction of hypertensive disorders of pregnancy (HDP), preferably preeclampsia (PE), in a subject, the test panel comprising or consisting of:

- measurement of the level of endoglin (ENG) in the subject; and
- measurement of the level of insulin-like growth factor-binding protein complex acid labile subunit (IGFALS) in the subject; and
- measurement of the level of cell surface glycoprotein MUC18 (MCAM) in the subject.

(i.xi) The test panel as set forth in (i.x) above, wherein the test panel further comprises:

- measurement of the level of placental growth factor (PIGF) in the subject;and/or
- measurement of the level of Kunitz-type protease inhibitor 1 (SPINT1) in the subject; and/or
- measurement of the level of multimerin-2 (MMRN2) in the subject; and/or
- measurement of blood pressure (BP) of the subject.

(i.xii) The test panel as set forth in (i.xi) above, wherein blood pressure is measured at either about 15 or about 20 weeks of gestation.

(i.xiii) The test panel as set forth in any one of (i.x) to (i.xii) above, wherein the test panel further comprises:

measurement of the level of disintegrin and metalloproteinase domain containing protein 12 (ADAM 12) in the subject; and/or

- - measurement of a value for the gestational age at blood sampling calculated from the date of the last menstrual period and/or from an ultrasound dating scan ("*gest*").

(i.xiv) A test panel for the prediction of hypertensive disorders of pregnancy (HDP), preferably preeclampsia (PE), in a subject, the test panel comprising or consisting of:

- - measurement of the level of trefoil factor 3 (TFF3) in the subject; and

- - measurement of the level in the subject of two or more constituents selected from the group consisting of: cell surface glycoprotein MUC18 (MCAM), disintegrin and metalloproteinase domain containing protein 12 (ADAM 12), extracellular matrix protein 1 (ECM1), insulin-like growth factor-binding protein complex acid labile subunit (IGFALS), placental growth factor (PIGF), multimerin-2 (MMRN2), peroxiredoxin-2 (PRDX2), sulfhydryl oxidase 1 (QSOX1) and blood pressure (BP).

(i.xv) The test panel as set forth in (i.xiv) above, wherein blood pressure is measured at either about 15 or about 20 weeks of gestation.

(i.xvi) The test panel as set forth in (i.xiv) or (i.xv) above, wherein the test panel further comprises measurement of the level in the subject of one or more constituents selected from the group consisting of: collagen alpha-3(VI) chain (COL6A3), endoglin (ENG), Kunitz-type protease inhibitor 1 (SPINT1), leucyl-cystinyl aminopeptidase (LNPEP), C-reactive protein (CRP), phosphatidylcholine-sterol acyltransferase (LCAT), ectonucleotide pyrophosphatase/phosphodiesterase family member 2 (ENPP2), microtubule-associated protein RP (MAPRE1/3), fructose-bisphosphate aldolase A (ALDOA) and body mass index (bmi).

Based on analysis of exemplary panels embodying the principles herein disclosed, it has been observed that certain markers and/or clinical parameters tend to be comparatively more prevalent or recurrent in the exemplary panels (see Table 4C), and their inclusion in the panels herein disclosed may thus be particularly desired.

For example, a marker or clinical parameter may be preferably included in test panels as disclosed herein, if the marker or clinical parameter is present in 25% or more, more preferably in 50% or more, or even more preferably in 75% or more, of the exemplary panels as set forth in Table 4C (see columns AP and AS of Table 4C for markers and clinicals, respectively; note that frequencies of B15 and BP20 in column AS of Table 4C are added up to produce frequency of BP).

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ENG, ADAM 12, PIGF, MMRN2, SPINT1 , QSOX1 , SEPP1 , ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age* and *mothpet,* and wherein the panel contains a) any one, any two, any three, any four, any five or any six of IGFALS, MCAM, ENG, ADAM12, PIGF, MMRN2, SPINT1 and BP, or preferably b) any one, any two or all three of IGFALS, MCAM and BP, or more preferably c) any one or both of IGFALS and BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of ENG, IGFALS, MCAM, SPINT1, MMRN2, ADAM12, PIGF, SEPP1, QSOX1, ECM1 , ROB04, LNPEP, LCAT, PRCP, ENPP2, BP, *fihd, alcoh,* bmi, *fhpet, vagbl, gest, age.* Preferably, the panel may contain a)any one, any two, any three, any four, any five or any six of ENG, IGFALS, MCAM, SPINT1, MMRN2, ADAM 12, PIGF and BP, or more preferably b)any one, any two, any three, any four or all five of ENG, IGFALS, MCAM, SPINT1 and BP, or more preferably c)any one, any two, any three or all four of ENG, IGFALS, MCAM and BP. Such panels may be particularly but without limitation useful for predicting PE in "rule-in" tests, even more specifically for predicting PE without distinction between preterm and term PE. See also the illustrative information in **Table 5A.** Particularly preferred are panels as individualised in **Table 4A,** for which the values in columns C and D of **Table 4A** are both equal to or greater than 0.495, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, ADAM12, MMRN2, PIGF, MCAM, QSOX1, SEPP1 , ENPP2, MAPRE1/3, ALDOA, LNPEP, *alcoh,* BP, *fhpet,* bmi, *pbwgt, vagbl.* Preferably, the panel may contain a) any one, any two, any three, any four, any five or any six of IGFALS, ADAM12, MMRN2, PIGF, MCAM, *alcoh,* BP or more preferably b) any one, any two, any three, or all four of IGFALS, ADAM12, MMRN2, BP, or more preferably c) any one or both of IGFALS, ADAM 12. Such panels may be particularly but without limitation useful for predicting PE in "rule-out" tests, even more specifically for predicting PE without distinction between preterm and term PE. See also the illustrative information in **Table 5B.** Particularly preferred are panels as individualised in **Table 4A,** for which the values in columns E and F of **Table 4A** are both equal to or greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of MMRN2, ADAM 12, IGFALS, MCAM, PlGF, BP. Such panels may be particularly but without limitation useful for predicting PE in "rule-in" and "rule-out" tests, even more specifically for predicting PE without distinction between preterm and term PE. See also the illustrative information in **Table 5C.** Particularly preferred are panels as individualised in **Table 4A,** for which the values in columns C and D of **Table 4A** are both equal to or greater than 0.495 and the values in columns E and F of **Table 4A** are both equal to greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ENG, ADAM 12, MMRN2, PlGF, SPINT1 , QSOX1, SEPP1, ECM1, ROB04, LNPEP, ENPP2, ALDOA, MAPRE1/3, LCAT, PRDX2, PRCP, BP, *alcoh,* bmi, *fihd, fhpet, vagbl, pbwgt, gest, age, mothpet.* Preferably, the panel may contain a) any one, any two, any three, any four, any five or any six of IGFALS, MCAM, ENG, ADAM 12, MMRN2, PlGF, SPINT1, BP, or more preferably b) any one, any two, any three, or all four of IGFALS, MCAM, ENG, BP, or more preferably c) any one or both of IGFALS and BP. Such panels may be particularly but without limitation useful for predicting PE, even more specifically for predicting PE without distinction between preterm and term PE. See also the illustrative information in **Table 5D.** Particularly preferred are panels as individualised in **Table 4A,** for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, MMRN2, ADAM 12, ENG, PlGF, SPINT1 , ECM1 , LNPEP, QSOX1, SEPP1 , BP, *alcoh, vagbl,* bmi. Preferably, the panel may contain a) any one, any two, any three, any four, any five or any six of IGFALS, MCAM, MMRN2, ADAM12, ENG, PlGF, BP, or more preferably b) any one, any two, any three, any four, or all five of IGFALS, MCAM, MMRN2, ADAM12, BP, or more preferably c) any one, or both of IGFALS, BP. Such panels may be particularly but without limitation useful for predicting PE, even more specifically for predicting PE without distinction between preterm and term PE. See also the illustrative information in **Table 5E.** Particularly preferred are panels as individualised in **Table 4A,** for which the values in columns A and B of **Table 4A** are both equal to or greater than 0.775, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ENG, ADAM 12, PlGF, MMRN2, SPINT1, QSOX1, SEPP1 , ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age, mothpet.* Preferably, the panel may contain a) any one, any two, any three, any four, any five or any six of IGFALS, MCAM, ENG, ADAM 12, PlGF, MMRN2, SPINT1, BP, or more preferably b) any one, any two, or all three of IGFALS, MCAM, BP, or more preferably c) any one or both of IGFALS, BP. Such panels may be particularly but without limitation useful for predicting preterm PE. See also the illustrative information in Table 5F. Particularly preferred are panels as individualised in Table 4A, for which any one of the values in columns J, S or AD of Table 4B is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of SPINT1 , ENG, IGFALS, MCAM, MMRN2, ADAM 12, PlGF, ECM1 , QSOX1, ROB04, PRCP, LNPEP, ENPP2, BP, *alcoh,* bmi, *vagbl, gest.* Preferably, the panel may contain a) any one, any two, any three, any four, any five or any six of SPINT1, ENG, IGFALS, MCAM, MMRN2, ADAM12, PlGF, ECM1 , BP, or more preferably b) any one, any two, any three, any four, any five or all six of SPINT1 , ENG, IGFALS, MCAM, MMRN2, BP, or more preferably c) SPINT1 . Such panels may be particularly but without limitation useful for predicting preterm PE, even more specifically in European ancestry patients. See also the illustrative information in **Table 5G.** Particularly preferred are panels as individualised in **Table 4A,** for which the values in column J of Table 4B is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, ENG, SPINT1, MCAM, MMRN2, ADAM12, PIGF, QSOX1, SEPP1, ROB04, LNPEP, BP, *alcoh, fihd,* bmi, *gest, fhpet, age.* Preferably, the panel may contain a) any one, any two, any three, any four, any five or any six of IGFALS, ENG, SPINT1, MCAM, MMRN2, ADAM12, BP, or more preferably b) any one, any two, any three, any four, or all five of IGFALS, ENG, SPINT1 , MCAM, BP, or more preferably c) any one, any two, any three, or all four of IGFALS, ENG, SPINT1, BP. Such panels may be particularly but without limitation useful for predicting preterm PE, even more specifically in Australasian ancestry patients. See also the illustrative information in **Table 5H.** Particularly preferred are panels as individualised in **Table 4A,** for which the value in column S of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of SPINT1, ENG, IGFALS, MCAM , MMRN2, ADAM12 , PIGF, QSOX1, SEPP1 , ROB04, LNPEP, ENPP2, BP, bmi, *alcoh, fihd, fhpet,gest, vagbl, age.* Preferably, the panel may contain a) any one, any two, any three, any four, any five or any six of SPINT1, ENG, IGFALS, MCAM, MMRN2, ADAM12, BP, or more preferably b)any one, any two, any three, any four, or all five of SPINT1 , ENG, IGFALS, MCAM, BP, or more preferably c)any one, any two, or all three of SPINT1, ENG, IGFALS. Such panels may be particularly but without limitation useful for predicting preterm PE, even more specifically regardless of ancestry of the patients. See also the illustrative information in **Table 5I.** Particularly preferred are panels as individualised in **Table 4A,** for which the value in column AD of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ADAM 12, ENG, PIGF, MMRN2, SEPP1 , QSOX1, SPINT1, LNPEP, ECM1 , ALDOA, MAPRE1/3, BP, *alcoh,* bmi, *vagbl, fhpet, fihd.* Preferably, the panel may contain a) any one, any two, any three, any four, any five or any six of IGFALS, MCAM, ADAM12, ENG, PIGF, MMRN2, BP, or more preferably b) any one, any two, any three, or all four of IGFALS, MCAM, ADAM 12, BP, or more preferably c) any one or both of IGFALS, BP. Such panels may be particularly but without limitation useful for predicting term PE, even more specifically in European ancestry patients. See also the illustrative information in **Table 5J.** Particularly preferred are panels as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ADAM 12, PIGF, ENG, SPINT1, MMRN2, SEPP1 , QSOX1, ECM1 , ROB04, ALDOA, LNPEP, ENPP2, MAPRE1/3, LCAT, BP, *alcoh,* bmi, *fihd, fhpet, vagbl, pbwgt, gest, mothpet.* Preferably, the panel may contain a) any one, any two, any three, any four, any five or any six of IGFALS, MCAM, ADAM12, PIGF, ENG, SPINT1, BP, or more preferably b) any one, any two, any three, any four, or all five of IGFALS, MCAM, ADAM 12, PIGF, BP, or more preferably c) any one or both of IGFALS, BP. Such panels may be particularly but without limitation useful for predicting term PE, even more specifically in Australasian ancestry patients. See also the illustrative information in **Table 5K.** Particularly preferred are panels as individualised in **Table 4A,** for which the values in column V of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, MCAM, ENG, PIGF, ADAM12, SPINT1, MMRN2, QSOX1, SEPP1, ROB04, ECM1 , ALDOA, LNPEP, MAPRE1/3, ENPP2, BP,*alcoh*, bmi, *fihd, fhpet, vagbl, pbwgt, gest, mothpet, age.* Preferably, the panel may contain a)any one, any two, any three, any four, any five or any six of IGFALS, MCAM, ENG, PIGF, ADAM12, SPINT1, MMRN2, BP, or more preferably b)any one, any two, any three, or all four of IGFALS, MCAM, ENG, BP, or more preferably c)any one or both of IGFALS, BP. Such panels may be particularly but without limitation useful for predicting term PE, even more specifically regardless of ancestry of the patients. See also the illustrative information in **Table 5L.**

Particularly preferred are panels as individualised in **Table 4A,** for which the value in column AG of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, SPINT1, ENG, MCAM, PIGF, ADAM12, MMRN2, BP, gest. Preferably, the panel may contain a) any one, any two, any three, any four, or all five of IGFALS, SPINT1, ENG, MCAM, BP, or more preferably b)any one, any two, any three, or all four of IGFALS, SPINT1, ENG, BP, or more preferably c)IGFALS. Such panels may be particularly but without limitation useful for prediction of PE in rule-in and/or rule-out tests. See also the illustrative information in **Table 5M** and the individualised preferred panels in **Table 5Ma,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, SPINT1 , ENG, MCAM, PIGF, ADAM 12, MMRN2, BP. Preferably, the panel may contain a) any one, any two, any three, any four, any five, or all six of IGFALS, SPINT1 , ENG, MCAM, PIGF, BP, or more preferably b) any one, any two, any three, or all four of IGFALS, SPINT1 , ENG, BP, or more preferably c) IGFALS. Such panels may be particularly but without limitation useful for prediction of PE in rule-in and/or rule-out tests. See also the illustrative information in **Table 5N** and the exemplary preferred panels individualised in **Table 5Na,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said constituents selected from the group consisting of IGFALS, ENG, ADAM 12, SPINT1, MCAM, SEPP1 , MMRN2, ECM1 , MAPRE1/3, ALDOA, PIGF and BP. See the exemplary preferred panels individualised in **Table 14,** as well as test panels as defined herein which comprise so individualised panels.

The inventors further realised that many particularly well-performing test panels contain the measurement of the level of IGFALS.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 1 of said constituents being IGFALS, the other constituents selected from the group consisting of MCAM,ENG, ADAM12, PIGF, MMRN2, SPINT1, QSOX1, SEPP1, ECM1, ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of MCAM, ENG, ADAM 12, PIGF, MMRN2, SPINT1 , QSOX1, SEPP1 , ECM1 , ROB04, LNPEP, ALDOA, MAP RE 1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* Preferably the panel may contain a) any one, any two, any three, any four, or any five of ENG, MCAM, ADAM 12, MMRN2, PIGF, SPINT1 and BP, or preferably b) any one, any two or all three of ENG, MCAM and BP, or more preferably c) BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 1 of said constituents being IGFALS, the other constituents selected from the group consisting of MCAM, ENG, ADAM12, PIGF, MMRN2, SPINT1, QSOX1,SEPP1, ECM1, ROB04, LNPEP, ALDOA, MAP RE 1/3, ENPP2, LCAT, PRDX2, PRCP, BP, *alcoh,* bmi, *fihd, fhpet, vagbl, pbwgt, gest, age* and *mothpet.* Preferably the panel may contain a) any one, any two, any three, any four, or any five of ENG, MCAM, ADAM 12, MMRN2, PIGF, SPINT1 and BP, or preferably b) any one, any two or all three of ENG, MCAM and BP, or more preferably c) BP.

Particularly preferred though exemplary and non-limiting IGFALS-containing test panels embodying the principles herein disclosed include the IGFALS-containing, 2- or more-constituent panels individualised in the rows of Table 13A, as well as panels as defined herein which comprise the so-individualised panels.

The inventors further realised that many particularly well-performing test panels contain the measurement of the level of BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 1 of said constituents being BP, the other constituents selected from the group consisting of IGFALS, MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, *alcoh,* bmi, *fihd, fhpet, vagbl, pbwgt, gest, age, mothpet, sispet,* and *waist;* or selected from the group consisting of IGFALS, MCAM, ENG, ADAM 12, PIGF, MMRN2, SPINT1 , QSOX1, SEPP1 , ECM1 , ROB04, LNPEP, ALDOA, MAPRE1 MAPRE1/3, ENPP2, LCAT, PRDX2, PRCP, TFF3, CST3, CRP, COL6A3, *alcoh,* bmi, *fihd, fhpet, vagbl, pbwgt, gest, age, mothpet, sispet,* and *waist.*

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 1 of said constituents being BP, the other constituents selected from the group consisting of IGFALS, MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, *alcoh,* bmi, *fihd, fhpet, vagbl, pbwgt, gest, age* and *mothpet.*

Some other test panels do not contain the measurement of the level of IGFALS.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, wherein none of the constituents is IGFALS, the other constituents selected from the group consisting of MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* Such panels may or may not contain BP as one of their constituents.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, wherein none of the constituents is IGFALS, the other constituents selected from the group consisting of MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age* and *mothpet.*

Some other test panels do not contain the measurement of BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, wherein none of the constituents is BP, the other constituents selected from the group consisting of IGFALS, MCAM, ENG, ADAM 12, PIGF, MMRN2, SPINT1 , QSOX1, SEPP1, ECM1 , ROB04, LNPEP, ALDOA, MAPRE1/3, ENPP2, LCAT, PRDX2, PRCP, TFF3, CST3, CRP, COL6A3, IL6ST, PROC, PCDH12, *alcoh,* bmi, *fihd, fhpet, vagbl, pbwgt, gest, age, mothpet, sispet,* and *waist;* or selected from the group consisting of IGFALS, MCAM, ENG, ADAM12, PIGF, MMRN2, SPINT1, QSOX1, SEPP1 , ECM1 , ROB04, LNPEP, ALDOA, MAP RE 1/3, ENPP2, LCAT, PRDX2, PRCP, TFF3, CST3, CRP, COL6A3, *alcoh,* bmi, *fihd, fhpet, vagbl, pbwgt, gest, age, mothpet, sispet,* and *waist.* Such panels may or may not contain IGFALS as one of their constituents.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, wherein none of the constituents is BP, the other constituents selected from the group consisting of IGFALS, MCAM, ENG, ADAM 12, PIGF, MMRN2, SPINT1 , QSOX1, SEPP1, ECM1 , ROB04, LNPEP, ALDOA, MAPRE1/3, ENPP2, LCAT, PRDX2, PRCP, *alcoh,* bmi, *fihd, fhpet, vagbl, pbwgt, gest, age* and *mothpet.* Such panels may or may not contain IGFALS as one of their constituents.

The inventors further realised that many particularly well-performing test panels contain the measurement of the level of IGFALS and the measurement of blood pressure (BP) in the subject.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ENG, ADAM 12, PIGF, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, *alcoh,* bmi, *fihd, fhpet, vagbl, pbwgt, gest, age, mothpet, sispet,* and *waist;* or selected from the group consisting of MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, PRCP, TFF3, CST3, CRP, COL6A3, *alcoh,* bmi, *fihd, fhpet, vagbl, pbwgt, gest, age, mothpet, sispet,* and *waist.* In certain embodiments, the panel may have the following alternative features: aa) it contains PIGF and optionally and preferably does not contain ENG and ADAM12, or ab) it contains ENG and optionally and preferably does not contain PIGF and ADAM12, or ac) it contains ADAM12 and optionally and preferably does not contain PIGF and ENG, or ad) it contains PIGF and ENG and optionally and preferably does not contain ADAM12, or ae) it contains PIGF and ADAM12 and optionally and preferably does not contain ENG, or af) it does not contain PIGF and ENG and ADAM12.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ENG, ADAM 12, PIGF, MMRN2, SPINT1, QSOX1, SEPP1 , ECM1 , ROB04, LNPEP, ALDOA, MAPRE1/3, ENPP2, LCAT, PRDX2, PRCP, TFF3, *alcoh,* bmi, *fihd, fhpet, vagbl, pbwgt, gest, age* and *mothpet.* In certain embodiments, the panel may have the alternative features aa) to af) as defined above.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of ENG, MCAM, SPINT1, MMRN2, ADAM12, SEPP1, PIGF, ROB04, QSOX1, LNPEP, ECM1, *alcoh, fihd, fhpet, vagbl, gest,* bmi, *age.* Preferably, the panel may contain a) any one, any two, any three, or all four of ENG, MCAM, SPINT1 , MMRN2, or more preferably b) any one, any two, or all three of ENG, MCAM, SPINT1 , or more preferably c) any one or both of ENG, MCAM. The panel may have the alternative features aa) to af) as defined above. Such panels may be particularly but without limitation useful for predicting PE in "rule-in" tests, even more specifically for predicting PE without distinction between preterm and term PE. See also the illustrative information in **Table 6A.** Particularly preferred are panels containing IGFALS and BP as individualised in **Table 4A,** for which the values in columns Cand D of **Table 4A** are both equal to or greater than 0.495, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of ADAM 12, MMRN2, PIGF, MCAM, SEPP1 , LNPEP, *alcoh, fhpet, vagbl.* Preferably, the panel may contain a) any one, any two, any three, or any four of ADAM 12, MMRN2, PIGF, MCAM, *alcoh,* or more preferably b) any one, any two, any three, or all four of ADAM 12, MMRN2, PIGF, MCAM. The panel may have the alternative features aa) to af) as defined above. Such panels may be particularly but without limitation useful for predicting PE in "rule-out" tests, even more specifically for predicting PE without distinction between preterm and term PE. See also the illustrative information in **Table 6B.** Particularly preferred are panels containing IGFALS and BP as individualised in **Table 4A,** for which the values in columns E and F of **Table 4A** are both equal to or greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MMRN2, ADAM12, MCAM, PIGF. The panel may have the alternative features aa) to af) as defined above. Such panels may be particularly but without limitation useful for predicting PE in "rule-in"and"rule-out" tests, even more specifically for predicting PE without distinction between preterm and term PE. See also the illustrative information in **Table 6C.** Particularly preferred are panels containing IGFALS and BP as individualised in **Table 4A,** for which the values in columns C and D of **Table 4A** are both equal to or greater than 0.495 and the values in columns E and F of **Table 4A** are both equal to greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of ENG, MCAM, MMRN2, ADAM12, SPINT1 , PIGF, SEPP1, QSOX1, ROB04, LNPEP, ALDOA, ECM1 , MAPRE1/3, ENPP2, PRDX2, *alcoh, fihd, vagbl,* bmi, *fhpet, gest, age.* Preferably, the panel may contain a) any one, any two, any three, or any four of ENG, MCAM, MMRN2, ADAM12, SPINT1, PIGF or more preferably b) any one or both of ENG, MCAM, or more preferably c) ENG. The panel may have the alternative features aa) to af) as defined above. Such panels may be particularly but without limitation useful for predicting PE, even more specifically for predicting PE without distinction between preterm and term PE. See also the illustrative information in **Table 6D.** Particularly preferred are panels containing IGFALS and BP as individualised in **Table 4A,** for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ADAM12, MMRN2, PIGF, ENG, SPINT1, SEPP1 , ECM1 , LNPEP, QSOX1, *alcoh, vagbl.* Preferably, the panel may contain a) any one, any two, any three, or any four of MCAM, ADAM12, MMRN2, PIGF, ENG, or more preferably b) any one, any two, any three, or all four of MCAM, ADAM12, MMRN2, PIGF, or more preferably c) MCAM. The panel may have the alternative features aa) to af) as defined above. Such panels may be particularly but without limitation useful for predicting PE, even more specifically for predicting PE without distinction between preterm and term PE. See also the illustrative information in **Table 6E.** Particularly preferred are panels containing IGFALS and BP as individualised in **Table 4A,** for which the values in columns A and B of **Table 4A** are both equal to or greater than 0.775, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of ENG, MCAM, ADAM 12, MMRN2, PIGF, SPINT1 , SEPP1 , QSOX1, ROB04, LNPEP, ALDOA, ECM1, ENPP2, PRDX2, *alcoh, fihd, vagbl,* bmi, *fhpet, gest, age.* Preferably, the panel may contain any one, any two, any three, or any four of ENG, MCAM, ADAM12, MMRN2, PIGF, SPINT1. The panel may have the alternative features aa) to af) as defined above. Such panels may be particularly but without limitation useful for predicting preterm PE. See also the illustrative information in **Table 6F.** Particularly preferred are panels containing IGFALS and BP as individualised in **Table 4A,** for which any one of the values in columns J, S or AD of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of SPINT1 , ENG, MMRN2, MCAM, ADAM12, ROB04, PIGF, *alcoh.* Preferably, the panel may contain a) any one, any two, any three, or any four of SPINT1 , ENG, MMRN2, MCAM, ADAM12, *alcoh,* or more preferably b) any one, any two, any three, or all four of SPINT , ENG, MMRN2, MCAM, or more preferably c) any one or both of SPINT1, ENG. The panel may have the alternative features aa) to af) as defined above. Such panels may be particularly but without limitation useful for predicting preterm PE, even more specifically in European ancestry patients. See also the illustrative information in **Table 6G.** Particularly preferred are panels containing IGFALS and BP as individualised in **Table 4A,** for which the values in column J of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of ENG, SPINT1 , MCAM, MMRN2, ADAM12, PIGF, QSOX1, SEPP1, ROB04, LNPEP, *alcoh, fihd,* bmi, *gest, age.* Preferably, the panel may contain a) any one, any two, any three, or all four of ENG, SPINT1, MCAM, MMRN2, or more preferably b)any one, any two, or all three of ENG, SPINT1, MCAM, or more preferably c) any one or both of ENG, SPINT1. The panel may have the alternative features aa) to af) as defined above. Such panels may be particularly but without limitation useful for predicting preterm PE, even more specifically in Australasian ancestry patients. See also the illustrative information in **Table 6H.** Particularly preferred are panels containing IGFALS and BP as individualised in **Table 4A,** for which the value in column S of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of SPINT1 , ENG, MCAM, MMRN2, ADAM12, PIGF, QSOX1, SEPP1 , ROB04, LNPEP, *alcoh, fihd,* bmi, *gest, age.* Preferably, the panel may contain a) any one, any two, any three, or all four of SPINT1, ENG, MCAM, MMRN2, or more preferably b) any one, any two, or all three of SPINT1, ENG, MCAM, or more preferably c) any one or both of SPINT1, ENG. The panel may have the alternative features aa) to af) as defined above. Such panels may be particularly but without limitation useful for predicting preterm PE, even more specifically regardless of ancestry of the patients. See also the illustrative information in **Table 6I.** Particularly preferred are panels containing IGFALS and BP as individualised in **Table 4A,** for which the value in column AD of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ADAM 12, PIGF, MMRN2, ENG, SEPP1 , QSOX1, SPINT1 , LNPEP, ECM1 , ALDOA, MAPRE1/3, *alcoh,* bmi, *vagbl, fhpet, fihd.* Preferably, the panel may contain a) any one, any two, any three, or any four of MCAM, ADAM12, PIGF, MMRN2, ENG, or more preferably b) any one or both of MCAM, ADAM12. The panel may have the alternative features aa) to af) as defined above. Such panels may be particularly but without limitation useful for predicting term PE, even more specifically in European ancestry patients. See also the illustrative information in **Table 6J.** Particularly preferred are panels containing IGFALS and BP as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ADAM 12, PIGF, ENG, MMRN2, SPINT1, SEPP1 , QSOX1, ROB04, ECM1 , ALDOA, LNPEP, MAPRE1/3, ENPP2, *alcoh, fihd,* bmi, *vagbl, fhpet.* Preferably, the panel may contain a) any one, any two, any three, or any four of MCAM, ADAM 12, PIGF, ENG, MMRN2, SPINT1, or more preferably b) MCAM. The panel may have the alternative features aa) to af) as defined above. Such panels may be particularly but without limitation useful for predicting term PE, even more specifically in Australasian ancestry patients. See also the illustrative information in **Table 6K.** Particularly preferred are panels containing IGFALS and BP as individualised in **Table 4A,** for which the values in column V of Table 4B is equal to or greater than 0.745, as well as test panels as defined herein which comprise so individualised panels.

It is herein disclosed ca test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, ENG, PIGF, ADAM12, MMRN2, SPINT1, QSOX1, SEPP1 , ROB04, ALDOA, LNPEP, MAPRE1/3, ECM1 , ENPP2, *alcoh, fihd,* bmi, *vagbl, fhpet, gest, age.* Preferably, the panel may contain a) any one, any two, any three, or any four of MCAM, ENG, PIGF, ADAM 12, MMRN2, SPINT1, *alcoh,* or more preferably b) any one or both of MCAM, ENG. The panel may have the alternative features aa) to af) as defined above. Such panels may be particularly but without limitation useful for predicting term PE, even more specifically regardless of ancestry of the patients. See also the illustrative information in **Table 6L.** Particularly preferred are panels containing IGFALS and BP as individualised in **Table 4A,** for which the value in column AG of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of SPINT1 , ENG, MCAM, ADAM 12, PIGF, MMRN2, gest. Preferably, the panel may contain a) any one, any two, any three, or any four of SPINT1 , ENG, MCAM, ADAM 12, PIGF, MMRN2, or more preferably b) any one, any two, or all three of SPINT1 , ENG, MCAM. The panel may have the alternative features aa) to af) as defined above. Such panels may be particularly but without limitation useful for prediction of PE in rule-in and/or rule-out tests. See also the illustrative information in **Table 6M.** Particularly preferred are panels containing IGFALS and BP as individualised in **Table 5Ma,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being IGFALS and BP, the other constituents selected from the group consisting of MCAM, PIGF, SPINT1, ENG, ADAM12, MMRN2. Preferably, the panel may contain a) any one or both of MCAM, PIGF. The panel may have the alternative features aa) to af) as defined above. Such panels may be particularly but without limitation useful for prediction of PE in rule-in and/or rule-out tests. See also the illustrative information in **Table 6N.** Particularly preferred are panels containing IGFALS and BP as individualised in **Table 5Na,** as well as test panels as defined herein which comprise so individualised panels.

The inventors further realised that many particularly well-performing test panels contain the measurement of the level of PIGF.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, MCAM, ENG, ADAM12, MMRN2, SPINT1, QSOX1, SEPP1, ECM1 , ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of IGFALS, MCAM, ENG, ADAM12, MMRN2, SPINT1, QSOX1, SEPP1, ECM1 , ROB04, LNPEP, ALDOA, MAP RE 1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* The panel may have the following alternative features: ba) it contains ENG and optionally and preferably does not contain ADAM12, or bb) it contains ADAM12 and optionally and preferably does not contain ENG, or be) it does not contain ENG and ADAM12, or bd) it contains IGFALS and optionally and preferably does not contain ENG and ADAM12, or be) it contains IGFALS and ENG and optionally and preferably does not contain ADAM 12, or bf) it contains IGFALS and ADAM 12 and optionally and preferably does not contain ENG.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, MCAM, ENG, ADAM12, MMRN2, SPINT1, QSOX1, SEPP1, ECM1, ROB04, LNPEP, ALDOA, MAPRE1/3, ENPP2, LCAT, PRDX2, PRCP, TFF3, BP, *alcoh,* bmi, *fihd, fhpet, vagbl, pbwgt, gest, age* and *mothpet.* In certain embodiments, the panel may have the alternative features ba) to bf) as defined above.

Particularly preferred though exemplary and non-limiting PIGF-containing test panels embodying the principles herein disclosed include the PIGF-containing, 2- or more-constituent panels individualised in the rows of Table 13C, as well as panels as defined herein which comprise the so-individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of MCAM, ENG, IGFALS, SPINT1 , ADAM 12, ECM1 , MMRN2, SEPP1 , LNPEP, PRCP, QSOX1, LCAT, BP, alcoh, bmi, fihd, gest, fhpet. Preferably, the panel may contain a) any one, any two, any three, any four, or any five of MCAM, ENG, IGFALS, SPINT1 , ADAM12, BP, or more preferably b) any one, any two, any three, any four or all five of MCAM, ENG, IGFALS, SPINT1 , BP, or more preferably c) any one or both of MCAM, BP. The panel may have the alternative features ba) to bf) as defined above. Such panels may be particularly but without limitation useful for predicting PE in "rule-in" tests, even more specifically for predicting PE without distinction between preterm and term PE. See also the illustrative information in **Table 7A.** Particularly preferred are panels containing PIGF as individualised in **Table 4A,** for which the values in columns C and D of **Table 4A** are both equal to or greater than 0.495, as well as test panels as defined herein which comprise so individualised panels.

Preferably, when an PIGF-containing panel does not contain ENG and does not contain ADAM12, it may preferably contain a) any one, any two, any three, any four or all five of IGFALS, MCAM, SPINT1, LNPEP, BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, ADAM 12, MCAM, MMRN2, QSOX1, LNPEP, BP, bmi, *pbwgt, alcoh, fhpet.* Preferably, the panel may contain a) any one, any two, any three, any four, or any five of IGFALS, ADAM 12, MCAM, MMRN2, BP, bmi, pbwgt, or more preferably b) any one, any two, any three, or all four of IGFALS, ADAM 12, MCAM, BP, or more preferably c) any one or both of IGFALS, ADAM12., The panel may have the alternative features ba) to bf) as defined above. Such panels may be particularly but without limitation useful for predicting PE in "rule-out" tests, even more specifically for predicting PE without distinction between preterm and term PE. See also the illustrative information in **Table 7B.** Particularly preferred are panels containing PIGF as individualised in **Table 4A,** for which the values in columns E and F of **Table 4A** are both equal to or greater than 0.395, as well as test panels as defined herein which comprise so individualised panels.

Preferably, when an PIGF-containing panel does not contain ENG and does not contain ADAM12, it may preferably contain a) any one, any two, any three, or all four of IGFALS, MCAM, LNPEP, BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of MMRN2, ADAM 12, IGFALS, MCAM, PIGF, BP. The panel may have the alternative features ba) to bf) as defined above. Such panels may be particularly but without limitation useful for predicting PE in "rule-in" and "rule-out" tests, even more specifically for predicting PE without distinction between preterm and term PE. See also the illustrative information in **Table 7C.** Particularly preferred are panels containing PIGF as individualised in **Table 4A,** for which the values in columns C and D of **Table 4A** are both equal to or greater than 0.495 and the values in columns E and F of **Table 4A** are both equal to greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, ADAM12, MCAM, ENG, MMRN2, SPINT1, ECM1, SEPP1 , LNPEP, ALDOA, ROB04, QSOX1, ENPP2, MAPRE1/3, LCAT, PRCP, BP, *alcoh,* bmi, *fhpet, fihd, pbwgt, vagbl.* Preferably, the panel may contain a) any one, any two, any three, or all four of IGFALS, ADAM12, MCAM, BP or more preferably b) any one or both of IGFALS, BP. The panel may have the alternative features ba) to bf) as defined above. Such panels may be particularly but without limitation useful for predicting PE, even more specifically for predicting PE without distinction between preterm and term PE. See also the illustrative information in **Table 7D.** Particularly preferred are panels containing PIGF as individualised in Table 4A, for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, ADAM 12, MCAM, MMRN2, SEPP1 , LNPEP, BP, *alcoh.* Preferably, the panel may contain a) any one, any two, any three, any four, or all five of IGFALS, ADAM 12, MCAM, MMRN2, BP, or more preferably b) any one, any two, any three, or all four of IGFALS, ADAM 12, MCAM, BP or more preferably c) any one, any two or all three of IGFALS, ADAM12, BP. The panel may have the alternative features ba) to bf) as defined above. Such panels may be particularly but without limitation useful for predicting PE, even more specifically for predicting PE without distinction between preterm and term PE. See also the illustrative information in **Table 7E.** Particularly preferred are panels containing PIGF as individualised in **Table 4A,** for which the values in columns A and B of **Table 4A** are both equal to or greater than 0.775, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, ADAM12, MCAM, ENG, SPINT1, MMRN2, ECM1, SEPP1, LNPEP, ALDOA, ROB04, MAPRE1/3, QSOX1, ENPP2, LCAT, PRCP, BP, *alcoh, fihd,* bmi, *fhpet, pbwgt, gest.* Preferably, the panel may contain a)any one, any two, any three, or all four of IGFALS, ADAM12, MCAM, BP, or more preferably b)any one, any two, or all three of IGFALS, ADAM12, BP, or more preferably c)BP. The panel may have the alternative features ba) to bf) as defined above. Such panels may be particularly but without limitation useful for predicting preterm PE. See also the illustrative information in **Table 7F.** Particularly preferred are panels containing PIGF as individualised in **Table 4A,** for which any one of the values in columns J,S or AD of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of MCAM, ADAM12, ECM1 , SPINT1, IGFALS, MMRN2, ENG, PRCP, LNPEP, BP, *alcoh.* Preferably, the panel may contain a) any one, any two, any three, any four, or any five of MCAM, ADAM12, ECM1 , SPINT1, IGFALS, MMRN2, ENG, BP, or more preferably b) any one, any two, any three, any four, or all five of MCAM, ADAM12, ECM1, SPINT1, BP. The panel may have the alternative features ba) to bf) as defined above. Such panels may be particularly but without limitation useful for predicting preterm PE, even more specifically in European ancestry patients. See also the illustrative information in **Table 7G.** Particularly preferred are panels containing PIGF as individualised in **Table 4A,** for which the values in column J of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of IGFALS, ENG, SPINT1 , MCAM, ADAM12, MMRN2, SEPP1, BP, *alcoh, fhpet.* Preferably, the panel may contain a) any one, any two, any three, any four, or any five of IGFALS, ENG, SPINT1, MCAM, ADAM12, MMRN2, BP, or more preferably b) any one, any two, any three, any four, or all five of IGFALS, ENG, SPINT1, MCAM, BP, or more preferably c) any one, any two, any three, or all four of IGFALS, ENG, SPINT1, BP. The panel may have the alternative features ba) to bf) as defined above. Such panels may be particularly but without limitation useful for predicting preterm PE, even more specifically in Australasian ancestry patients. See also the illustrative information in **Table 7H.** Particularly preferred are panels containing PIGF as individualised in **Table 4A,** for which the value in column S of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of SPINT1, IGFALS, ENG, MCAM, ADAM12, SEPP1, MMRN2, BP, bmi, *alcoh, fhpet, gest.* Preferably, the panel may contain a) any one, any two, any three, any four, or any five of SPINT1, IGFALS, ENG, MCAM, ADAM12, BP, or more preferably b) any one, any two, any three, any four, or all five of SPINT1, IGFALS, ENG, MCAM, BP, or more preferably c) any one, any two, or all three of SPINT1, IGFALS, ENG. The panel may have the alternative features ba) to bf) as defined above. Such panels may be particularly but without limitation useful for predicting preterm PE, even more specifically regardless of ancestry of the patients. See also the illustrative information in **Table 7I.** Particularly preferred are panels containing PIGF as individualised in **Table 4A,** for which the value in column AD of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of IGFALS, ADAM12, MCAM, MMRN2, SEPP1, LNPEP, QSOX1, ENG, ALDOA, BP, *alcoh,* bmi, *fhpet, fihd.* Preferably, the panel may contain a) any one, any two, any three, any four, or any five of IGFALS, ADAM12, MCAM, MMRN2, SEPP1, BP, or more preferably b) any one, any two, any three, or all four of IGFALS, ADAM12, BP. The panel may have the alternative features ba) to bf) as defined above. Such panels may be particularly but without limitation useful for predicting term PE, even more specifically in European ancestry patients. See also the illustrative information in **Table 7J.** Particularly preferred are panels containing PIGF as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of IGFALS, ADAM12, MCAM, ENG, SPINT1, MMRN2, ECM1, SEPP1, ALDOA, LNPEP, MAP RE 1/3, QSOX1, ENPP2, ROB04, LCAT, BP, *alcoh,* bmi, *fihd, fhpet, pbwgt, gest.* Preferably, the panel may contain a) any one, any two, any three, or all four of IGFALS, ADAM12, MCAM, BP, or more preferably b) any one, or both IGFALS, BP. The panel may have the alternative features ba) to bf) as defined above. Such panels may be particularly but without limitation useful for predicting term PE, even more specifically in Australasian ancestry patients. See also the illustrative information in **Table 7K.** Particularly preferred are panels containing PIGF as individualised in **Table 4A,** for which the values in column V of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being PIGF, the other constituents selected from the group consisting of IGFALS, ADAM12, MCAM, ENG, MMRN2, SPINT1, SEPP1, ECM1, ALDOA, LNPEP, MAP RE 1/3, QSOX1, ROB04, ENPP2, BP, *alcoh,* bmi, *fihd, fhpet, pbwgt, gest.* Preferably, the panel may contain a)any one, any two, any three, any four, or all five of IGFALS, ADAM12, MCAM, ENG, BP, or more preferably b)any one, any two, or all three of IGFALS, ADAM 12, BP, or more preferably c)any one or both of IGFALS, BP. The panel may have the alternative features ba) to bf) as defined above. Such panels may be particularly but without limitation useful for predicting term PE, even more specifically regardless of ancestry of the patients. See also the illustrative information in **Table 7L.** Particularly preferred are panels containing PIGF as individualised in **Table 4A,** for which the value in column AG of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, SPINT1, MCAM, ENG, ADAM12, MMRN2, BP, *gest.* Preferably, the panel may contain a) any one, any two, any three, any four, or any five of IGFALS, SPINT1, MCAM, ENG, ADAM12, BP, or more preferably b) any one, any two, any three, or all four of IGFALS, SPINT1 , MCAM, BP, or more preferably c) any one or both of IGFALS, BP. The panel may have the alternative features ba) to bf) as defined above. Such panels may be particularly but without limitation useful for prediction of PE in rule-in and/or rule-out tests. See also the illustrative information in **Table 7M.** Particularly preferred are panels containing PIGF as individualised in **Table 5Ma,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being PIGF, the other constituents selected from the group consisting of IGFALS, MCAM, ADAM 12, MMRN2, SPINT1 , ENG, BP. Preferably, the panel may contain a) any one, any two, any three, any four, or all five of IGFALS, MCAM, ADAM 12, MMRN2, BP, or more preferably b) any one, any two, any three, or all four of IGFALS, MCAM, ADAM 12, BP, or more preferably c) IGFALS. The panel may have the alternative features ba) to bf) as defined above. Such panels may be particularly but without limitation useful for prediction of PE in rule-in and/or rule-out tests. See also the illustrative information in **Table 7N.** Particularly preferred are panels containing PIGF as individualised in **Table 5Na,** as well as test panels as defined herein which comprise so individualised panels.

It is herein disclosed a test panel comprising or consisting of: measurement of the level of the biomarker PIGF and measurement of the level of the biomarker IGFALS. Such a test panel significantly improves the predictive value of PIGF alone in predicting early onset PE. Furthermore, such a test panel allows the prediction of both early onset preeclampsia, in particular PE having onset before 34 weeks of gestation, and late onset pre-eclampsia, in particular PE having onset on or after 34 weeks of gestation.

Satisfactory and even more accurate evaluation of HDP and particularly PE, more particularly early onset PE and/or late onset PE, such as both early and late onset PE, may be achieved when the test panel comprising PIGF and IGFALS is supplemented with one or more of selenoprotein P (SEPP1), Xaa-Pro aminopeptidase 2 (XPNPEP2), tenascin-X (TNXB),prenylcysteine oxidase 1 (PCYOX1), multimerin-2 (MMRN2), endoglin (ENG), vascular endothelial growth factor receptor 3 (FLT4), peroxiredoxin-1 (PRDX1), disintegrin and metalloproteinase domain-containing protein 12 (ADAM 12), cell surface glycoprotein MUC18 (MCAM), leucyl-cystinyl aminopeptidase (LNPEP), ectonucleotide pyrophosphatase/phosphodiesterase family member 2 (ENPP2), basement membrane-specific heparan sulfate proteoglycan core protein (HSPG2) and sulfhydryloxidase 1 (QSOX1).

Hence, it is herein disclosed a test panel comprising or consisting of PIGF; IGFALS; and measurement of the level of any one or more biomarkers selected from the group consisting of SEPP1 , XPNPEP2, TNXB, PCYOX1, MMRN2, ENG, FLT4, PRDX1, ADAM 12, MCAM, LNPEP, ENPP2, HSPG2 and QSOX1.

It is herein disclosed a test panel comprising or consisting of: PIGF, IGFALS, and only one biomarker selected from the group consisting of SEPP1, XPNPEP2, TNXB, PCYOX1, MMRN2, ENG, FLT4, PRDX1, ADAM 12, MCAM, LNPEP, ENPP2, HSPG2 and QSOX1. For example, a test panel may comprise or consist of: PIGF; IGFALS; and SEPP1. Another test panel may comprise or consist of: PIGF; IGFALS; and XPNPEP2. A further test panel may comprise or consist of: PIGF; IGFALS; and TNXB. A yet another test panel may comprise or consist of: PIGF; IGFALS; and PCYOX1. Particularly preferred panels of this type may comprise or consist of markers and parameters as included in any one of the exemplary panels shown in Table 1 1.

It is herein disclosed a test panel comprising or consisting of: PIGF; IGFALS; and measurement of the level of any two or more biomarkers, such as three, four, five, six, seven or eight biomarkers, selected from the group consisting of SEPP1, XPNPEP2, TNXB, PCYOX1, MMRN2, ENG, FLT4, PRDX1, ADAM 12, MCAM, LNPEP, ENPP2, HSPG2 and QSOX1.

As herein disclosed, a test panel is provided comprising or consisting of: PIGF and any one or more biomarkers selected from the group consisting of IGFALS, SEPP1, XPNPEP2, TNXB, PCYOX1, MMRN2, ENG, FLT4, PRDX1, ADAM 12, MCAM, LNPEP, ENPP2, HSPG2 and QSOX1. Preferably, a test panel is provided comprising or consisting of: PIGF and only one biomarker selected from the group consisting of IGFALS, SEPP1, XPNPEP2, TNXB, PCYOX1, MMRN2, ENG, FLT4, PRDX1, ADAM 12, MCAM, LNPEP, ENPP2, HSPG2 and QSOX1. Preferably, a test panel is provided comprising or consisting of: PIGF and any two or more biomarkers, such as three, four, five, six, seven or eight biomarkers, selected from the group consisting of IGFALS, SEPP1, XPNPEP2, TNXB, PCYOX1, MMRN2, ENG, FLT4, PRDX1, ADAM 12, MCAM, LNPEP, ENPP2, HSPG2 and QSOX1. Preferably, a test panel is provided comprising or consisting of: PIGF and only two biomarkers selected from the group consisting of IGFALS, SEPP1, XPNPEP2, TNXB, PCYOX1, MMRN2, ENG, FLT4, PRDX1, ADAM12, MCAM, LNPEP, ENPP2, HSPG2 and QSOX1. Such test panels allow the prediction of PE, more particularly early onset PE and/or late onset PE, such as both early and late onset PE, and hence, can provide guidance to the medical practitioner such as to choose the appropriate treatment or to monitor the female during pregnancy and/or post partum.

The inventors further realised that many particularly well-performing test panels contain ENG.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, MCAM, ADAM12, PIGF, MMRN2, SPINT1, QSOX1, SEPP1, ECM1, ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of IGFALS, MCAM, ADAM12, PIGF, MMRN2, SPINT1, QSOX1, SEPP1, ECM1, ROB04, LNPEP, ALDOA, MAPRE1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* The panel may have the following alternative features: ca) it does not contain ADAM 12, or cb) it does not contain PIGF, or cc) it does not contain ADAM12 and PIGF, or cd) it contains PIGF and optionally and preferably does not contain ADAM12, or ce) it contains IGFALS and is otherwise as defined in any one of ca to cd).

Also, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, MCAM, ADAM12, PIGF, MMRN2, SPINT1, QSOX1, SEPP1, ECM1, ROB04, LNPEP, ALDOA, MAP RE 1/3, ENPP2, LCAT, PRDX2, PRCP, BP, *alcoh,* bmi, *fihd, fhpet, vagbl, pbwgt, gest, age* and *mothpet.* The panel may have the alternative features ca) to ce) as defined above.

Particularly preferred though exemplary and non-limiting ENG-containing test panels embodying the principles herein disclosed include the ENG-containing, 2- or more-constituent panels individualised in the rows of Table 13D, as well as panels as defined herein which comprise the so-individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, MCAM, SPINT1, MMRN2, ADAM 12, PIGF, SEPP1, QSOX1, ROB04, ECM1, LNPEP, LCAT, ENPP2, BP, *fihd, alcoh,* bmi, *fhpet, vagbl, gest, age.* Preferably, the panel may contain a)any one, any two, any three, any four, or any five of IGFALS, MCAM, SPINT1, MMRN2, ADAM12, BP, or more preferably b)any one, any two, any three, or all four of IGFALS, MCAM, SPINT1, BP, or more preferably c)any one, any two, or all three of IGFALS, MCAM, BP. The panel may have the alternative features ca) to ce) as defined above. Such panels may be particularly but without limitation useful for predicting PE in "rule-in" tests, even more specifically for predicting PE without distinction between preterm and term PE. See also the illustrative information in **Table 8A.** Particularly preferred are panels containing ENG as individualised in **Table 4A,** for which the values in columns C and D of **Table 4A** are both equal to or greater than 0.495, as well as test panels as defined herein which comprise so individualised panels.

Preferably, when an ENG-containing panel does not contain PIGF and ADAM12, it may preferably contain a) any one, any two, any three, any four or any five of IGFALS, MCAM, SPINT1, QSOX1, MMRN2, BP and *alcoh,* or more preferably contain b) any one, any two, any three, or all four of IGFALS, MCAM, SPINT1, BP, or more preferably contain c) any one, any two, or all three of IGFALS, MCAM, BP.

Preferably, when an ENG-containing panel does not contain PIGF and ADAM12, it may preferably contain a) any one, any two, any three, any four or any five of IGFALS, MCAM, SPINT1 , SEPP1, ROB04, QSOX1, MMRN2, LNPEP, ENPP2, BP, BMI, *age, gest, fhpet, fihd, vagbl, alcoh,* or more preferably contain b) any one, any two, any three, any four or all five of IGFALS, MCAM, SPINT1, SEPP1, BP, or more preferably contain c) any one, any two, or all three of IGFALS, MCAM, BP.

Preferably, when an ENG-containing panel contains PIGF and does not contain ADAM12, it may preferably contain a) any one, any two, or all three of IGFALS, MCAM, SPINT1.

Preferably, when an ENG-containing panel contains PIGF and does not contain ADAM12, it may preferably contain a)any one, any two, any three, any four or any five of IGFALS, MCAM, SPINT1, SEPP1, MMRN2, BP, *gest,* or more preferably contain b)any one, any two, any three, or all four of IGFALS, MCAM, SPINT1, BP, or more preferably contain c)any one, any two, or all three of IGFALS, MCAM, SPINT1.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS,MCAM, SPINT1, MMRN2, ADAM12, QSOX1, PIGF, SEPP1, ECM1, ROB04, ENPP2, LNPEP, BP, bmi, *alcoh, fihd, vagbl, fhpet, gest, age, mothpet.* Preferably, the panel may contain a) any one, any two, any three, any four, or all five of IGFALS, MCAM, SPINT1, MMRN2, BP, or more preferably b) any one, any two, any three, or all four of IGFALS, MCAM, SPINT1 , BP, or more preferably c) any one or both of IGFALS, BP. The panel may have the alternative features ca) to ce) as defined above. Such panels may be particularly but without limitation useful for predicting PE, even more specifically for predicting PE without distinction between preterm and term PE. See also the illustrative information in **Table 8B.** Particularly preferred are panels containing ENG as individualised in **Table 4A,** for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, MCAM, MMRN2, SPINT1 , ADAM12, ECM1 , QSOX1, SEPP1, BP, *vagbl, alcoh,* bmi. Preferably, the panel may contain a)any one, any two, any three, any four, or all five of IGFALS, MCAM, MMRN2, SPINT1, BP, or more preferably b)any one, any two, any three, or all four of IGFALS, MCAM, MMRN2, BP, or more preferably c)any one or both of IGFALS, MCAM., The panel may have the alternative features ca) to ce) as defined above. Such panels may be particularly but without limitation useful for predicting PE, even more specifically for predicting PE without distinction between preterm and term PE. See also the illustrative information in **Table 8C.** Particularly preferred are panels containing ENG as individualised in **Table 4A,** for which the values in columns A and B of **Table 4A** are both equal to or greater than 0.775, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS,MCAM, SPINT1, MMRN2, ADAM12, QSOX1, PIGF, SEPP1, ROB04, ECM1 , LNPEP, ENPP2, LCAT, BP, bmi, *alcoh, fihd, fhpet, vagbl, gest, mothpet, pbwgt, age.* Preferably, the panel may contain a) any one, any two, any three, any four, or any five of IGFALS, MCAM, SPINT1, MMRN2, ADAM12, BP, or more preferably b) any one, any two, any three, or all four of IGFALS, MCAM, SPINT1, BP, or more preferably c) any one or both of IGFALS, BP. the panel may have the alternative features ca) to ce) as defined above. Such panels may be particularly but without limitation useful for predicting preterm PE. See also the illustrative information in **Table 8D.** Particularly preferred are panels containing ENG as individualised in **Table 4A,** for which any one of the values in columns J, S or AD of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of SPINT1, IGFALS, MMRN2, MCAM, ADAM12, PIGF, ECM1, QSOX1, ROB04, ENPP2, BP, *alcoh,* bmi, *vagbl, gest.* Preferably, the panel may contain a) any one, any two, any three, any four, or any five of SPINT1, IGFALS, MMRN2, MCAM, ADAM12, BP, or more preferably b) any one, any two, any three, any four, or all five of SPINT1, IGFALS, MMRN2, MCAM, BP or more preferably c) any one or both of SPINT1, IGFALS. The panel may have the alternative features ca) to ce) as defined above. Such panels may be particularly but without limitation useful for predicting preterm PE, even more specifically in European ancestry patients. See also the illustrative information in **Table 8E.** Particularly preferred are panels containing ENG as individualised in **Table 4A,** for which the values in column J of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS,SPINT1, MCAM, MMRN2, ADAM12, QSOX1, PIGF, SEPP1, ROB04, LNPEP, BP, *alcoh, fihd,* bmi, *gest, fhpet, age.* Preferably, the panel may contain a)any one, any two, any three, any four, or any five of IGFALS, SPINT1, MCAM, MMRN2, ADAM12, BP, or more preferably b) any one, any two, any three, or all four of IGFALS, SPINT1, MCAM, BP, or more preferably c) any one, any two, or all of IGFALS, SPINT1, BP. The panel may have the alternative features ca) to ce) as defined above. Such panels may be particularly but without limitation useful for predicting preterm PE, even more specifically in Australasian ancestry patients. See also the illustrative information in *Table 8F.* Particularly preferred are panels containing ENG as individualised in Table 4A, for which the value in column S of Table 4B is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of SPINT1, IGFALS, MCAM, MMRN2, ADAM 12, QSOX1, PIGF, SEPP1, ROB04, LNPEP, ENPP2, BP, bmi, *alcoh, fihd, fhpet, gest, vagbl, age.* Preferably, the panel may contain a) any one, any two, any three, any four, or any five of SPINT1, IGFALS, MCAM, MMRN2, ADAM12, BP, or more preferably b) any one, any two, any three, or all four of SPINT1, IGFALS, MCAM, BP, or more preferably c) any one or both of SPINT1, IGFALS. The panel may have the alternative features ca) to ce) as defined above. Such panels may be particularly but without limitation useful for predicting preterm PE, even more specifically regardless of ancestry of the patients. See also the illustrative information in **Table 8G.** Particularly preferred are panels containing ENG as individualised in **Table 4A,** for which the value in column AD of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of MCAM, IGFALS, MMRN2, ADAM12, QSOX1, SPINT1, ECM1, PIGF, SEPP1, BP, bmi, *alcoh, vagbl, fhpet.* Preferably, the panel may contain a) any one, any two, any three, any four, or any five of MCAM, IGFALS, MMRN2, ADAM 12, QSOX1, BP, or more preferably b) any one, any two, or all three of MCAM, IGFALS, BP. The panel may have the alternative features ca) to ce) as defined above. Such panels may be particularly but without limitation useful for predicting term PE, even more specifically in European ancestry patients. See also the illustrative information in **Table 8H.** Particularly preferred are panels containing ENG as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of MCAM,IGFALS, SPINT1, ADAM12, MMRN2, PIGF, QSOX1, SEPP1, ROB04, ECM1, LNPEP, bmi, BP, *alcoh, fihd, fhpet, vagbl, pbwgt, gest, mothpet.* Preferably, the panel may contain a)any one, any two, any three, any four, or any five of MCAM, IGFALS, SPINT1, ADAM12, MMRN2, bmi, BP, or more preferably b)any one, any two, or all three of MCAM, IGFALS, BP. The panel may have the alternative features ca) to ce) as defined above. Such panels may be particularly but without limitation useful for predicting term PE, even more specifically in Australasian ancestry patients. See also the illustrative information in **Table 8I.** Particularly preferred are panels containing ENG as individualised in **Table 4A,** for which the values in column V of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, MCAM, SPINT1, MMRN2, ADAM12, PIGF, QSOX1, SEPP1, ROB04, ECM1 , LNPEP, ENPP2, BP, bmi, *alcoh, fihd, fhpet, vagbl, gest, mothpet, pbwgt, age.* Preferably, the panel may contain a)any one, any two, any three, any four, or any five of IGFALS, MCAM, SPINT1, MMRN2, BP, bmi, or more preferably b)any one, any two, or all three of IGFALS, MCAM, BP. The panel may have the alternative features ca) to ce) as defined above. Such panels may be particularly but without limitation useful for predicting term PE, even more specifically regardless of ancestry of the patients. See also the illustrative information in **Table 8J.** Particularly preferred are panels containing ENG as individualised in **Table 4A,** for which the value in column AG of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, SPINT1, MCAM, MMRN2, PIGF, ADAM12, BP, *gest.* Preferably, the panel may contain a) any one, any two, any three, any four, or any five of IGFALS, SPINT1 , MCAM, MMRN2, PIGF, ADAM12, BP, or more preferably b) any one, any two, any three, or all four of IGFALS, SPINT1, MCAM, BP, or more preferably c) any one, any two, or all three of IGFALS, SPINT1, BP. The panel may have the alternative features ca) to ce) as defined above. Such panels may be particularly but without limitation useful for prediction of PE in rule-in and/or rule-out tests. See also the illustrative information in **Table 8K.** Particularly preferred are panels containing ENG as individualised in **Table 5Ma,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ENG, the other constituents selected from the group consisting of IGFALS, SPINT1 , MCAM, PIGF, MMRN2, BP. Preferably, the panel may contain any one, any two, any three, or all four of IGFALS, SPINT1 , MCAM, BP. The panel may have the alternative features ca) to ce) as defined above. Such panels may be particularly but without limitation useful for prediction of PE in rule-in and/or rule-out tests. See also the illustrative information in **Table 8L.** Particularly preferred are panels containing ENG as individualised in **Table 5Na,** as well as test panels as defined herein which comprise so individualised panels.

The inventors further realised that many particularly well-performing test panels contain ADAM 12.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, MCAM, ENG, PIGF, MMRN2, SPINT1, QSOX1, SEPP1, ECM1,ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of IGFALS, MCAM, ENG, PIGF, MMRN2, SPINT1, QSOX1, SEPP1 , ECM1 , ROB04, LNPEP, ALDOA, MAPRE1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* The panel may have the following alternative features: da) it does not contain ENG, or db) it does not contain PIGF, or dc) it does not contain ENG and PIGF, or dd) it contains PIGF and optionally and preferably does not contain ENG, or de) it contains IGFALS and is otherwise as defined in any one of da to dd).

Also, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, MCAM, ENG, PIGF, MMRN2, SPINT1, QSOX1, SEPP1, ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age* and *mothpet.* In certain embodiments, the panel may have the alternative features da) to de) as defined above.

Particularly preferred though exemplary and non-limiting ADAM12-containing test panels embodying the principles herein disclosed include the ADAM12-containing, 2- or more-constituent panels individualised in the rows of Table 13B, as well as panels as defined herein which comprise the so-individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of ENG, SPINT1, IGFALS, MCAM, PIGF, MMRN2, QSOX1, SEPP1, ECM1 , ROB04, BP, bmi, *fihd, alcoh, fhpet.* Preferably, the panel may contain a) any one, any two, any three, any four, or any five of ENG, SPINT1, IGFALS, MCAM, PIGF, MMRN2, BP, bmi, *fihd,* or more preferably b) any one, any two, any three, any four or all five of ENG, SPINT1, IGFALS, MCAM, BP, or more preferably c) any one or both of ENG, BP. The panel may have the alternative features da) to de) as defined above. Such panels may be particularly but without limitation useful for predicting PE in "rule-in" tests, even more specifically for predicting PE without distinction between preterm and term PE. See also the illustrative information in **Table 9A.** Particularly preferred are panels containing ADAM12 as individualised in **Table 4A,** for which the values in columns C and D of **Table 4A** are both equal to or greater than 0.495, as well as test panels as defined herein which comprise so individualised panels.

Preferably, when an ADAM12-containing panel does not contain PIGF and does not contain ENG, it may preferably contain a) any one, any two, any three, any four or any five of IGFALS, ADAM12, MCAM, SEPP1, ROB04, MMRN2, ECM1, BP, *fihd, alcoh,* or more preferably b) any one, any two, or all three of IGFALS, ROB04, BP, *fihd,* or more preferably c) any one or both IGFALS, BP.

Preferably, when an ADAM12-containing panel contains PIGF and does not contain ENG, it may preferably contain a)any one, any two, any three, any four or any five of IGFALS, ADAM12, MCAM, SPINT1, SEPP1, MMRN2, BP, or more preferably b)any one, any two, any three, or any four of IGFALS, ADAM12, MCAM, SPINT1, BP, or more preferably c)any one, any two, or all three of IGFALS, ADAM12, BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of IGFALS, PIGF, MMRN2, MCAM, QSOX1, ENPP2, SEPP1, MAPRE1/3, ALDOA, *alcoh,* BP, *fhpet,* bmi, *pbwgt.* Preferably, the panel may contain a) any one, any two, any three, any four, or any five of IGFALS, PIGF, MMRN2, MCAM, *alcoh,* BP, or more preferably b) any one, any two, or all three of IGFALS, PIGF, BP, or more preferably c) IGFALS. The panel may have the alternative features da) to de) as defined above. Such panels may be particularly but without limitation useful for predicting PE in "rule-out" tests, even more specifically for predicting PE without distinction between preterm and term PE. See also the illustrative information in **Table 9B.** Particularly preferred are panels containing ADAM12 as individualised in **Table 4A,** for which the values in columns E and F of **Table 4A** are both equal to or greater than 0.395, as well as test panels as defined herein which comprise so individualised panels.

Preferably, when an ADAM12-containing panel contains PIGF and does not contain ENG, it may preferably contain IGFALS.

Preferably, when an ADAM12-containing panel does not contain PIGF and does not contain ENG, it may preferably contain a) any one, any two, any three, any four or any five of IGFALS, SEPP1, QSOX1, MMRN2, ENPP2, BP, *fhpet, alcoh,* or more preferably b) any one, any two, or all three of IGFALS, MMRN2, *alcoh,* or more preferably c) IGFALS.

Preferably, when an ADAM12-containing panel contains PIGF and does not contain ENG, it may preferably contain a) any one, any two, or all three of IGFALS, MCAM, BP.

Preferably, when an ADAM12-containing panel contains PIGF and does not contain ENG, it may preferably contain a) any one, any two, any three, any four, or any five of IGFALS, MCAM, QSOX1, MMRN2, BP, BMI, *fhpet, pbwgt, alcoh,* or more preferably b) any one, any two, any three, any four or all five of IGFALS, MCAM, BP, BMI, *pbwgt,* or more preferably c) IGFALS.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of MMRN2, ADAM12, IGFALS, MCAM, PIGF, BP. The panel may have the alternative features da) to de) as defined above. Such panels may be particularly but without limitation useful for predicting PE in "rule-in" and "rule-out" tests, even more specifically for predicting PE without distinction between preterm and term PE. See also the illustrative information in **Table 9C.** Particularly preferred are panels containing ADAM12 as individualised in **Table 4A,** for which the values in columns C and D of **Table 4A** are both equal to or greater than 0.495 and the values in columns E and F of **Table 4A** are both equal to greater than 0.395, as well as test panels as defined herein which comprise so individualised panels.

Preferably, when an ADAM12-containing panel contains PIGF and does not contain ENG, it may preferably contain any one, any two, any three, or all four of IGFALS, MCAM, MMRN2, BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of IGFALS, MCAM, PIGF, ENG, MMRN2, SPINT1, QSOX1, SEPP1, ECM1, ROB04, ENPP2, ALDOA, MAPRE1/3, LCAT, PRDX2, BP, *alcoh,* bmi, *fihd, fhpet, pbwgt.* Preferably, the panel may contain a) any one, any two, any three, any four, or all five of IGFALS, MCAM, PIGF, ENG, BP or more preferably b) any one or both of IGFALS, BP. The panel may have the alternative features da) to de) as defined above. Such panels may be particularly but without limitation useful for predicting PE, even more specifically for predicting PE without distinction between preterm and term PE. See also the illustrative information in **Table 9D.** Particularly preferred are panels containing ADAM12 as individualised in Table 4A, for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of IGFALS, PIGF, MCAM, MMRN2, SEPP1, ENG, ECM1, BP, *alcoh.* Preferably, the panel may contain a) any one, any two, any three, any four, or all five of IGFALS, PIGF, MCAM, MMRN2, BP, or more preferably b) any one, any two, or all three of IGFALS, PIGF, BP, or more preferably c) any one or both IGFALS, BP. The panel may have the alternative features da) to de) as defined above. Such panels may be particularly but without limitation useful for predicting PE, even more specifically for predicting PE without distinction between preterm and term PE. See also the illustrative information in **Table 9E.** Particularly preferred are panels containing ADAM12 as individualised in **Table 4A,** for which the values in columns A and B of Table 4A are both equal to or greater than 0.775, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of IGFALS, MCAM, PIGF, ENG, SPINT1, MMRN2, QSOX1, SEPP1, ECM1, ENPP2, ROB04, ALDOA, MAPRE1/3, LCAT, PRDX2, BP, *alcoh,* bmi, *fihd, fhpet, pbwgt, mothpet.* Preferably, the panel may contain a) any one, any two, any three, any four, or all five of IGFALS, MCAM, PIGF, ENG, BP, or more preferably b) BP. The panel may have the alternative features da) to de) as defined above. Such panels may be particularly but without limitation useful for predicting preterm PE. See also the illustrative information in **Table 9F.** Particularly preferred are panels containing ADAM12 as individualised in **Table 4A,** for which any one of the values in columns J, S or AD of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of IGFALS, SPINT1, MCAM, MMRN2, ENG, PIGF, ECM1, QSOX1, BP, bmi. Preferably, the panel may contain a) any one, any two, any three, any four, or any five of IGFALS, SPINT1, MCAM, MMRN2, ENG, PIGF, ECM1, BP, or more preferably b) any one, any two, any three, any four, or any five of IGFALS, SPINT1, MCAM, MMRN2, ENG, BP. The panel may have the alternative features da) to de) as defined above. Such panels may be particularly but without limitation useful for predicting preterm PE, even more specifically in European ancestry patients. See also the illustrative information in **Table 9G.** Particularly preferred are panels containing ADAM12 as individualised in **Table 4A,** for which the values in column J of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being ADAM12, the other constituents selected from the group consisting of SPINT1, IGFALS, ENG, MCAM, MMRN2, PIGF, QSOX1, SEPP1, BP, *fihd,* bmi, *alcoh.* Preferably, the panel may contain a) any one, any two, any three, any four, or any five of SPINT1, IGFALS, ENG, MCAM, MMRN2, PIGF, BP, or more preferably b) any one, any two, any three, or all four of SPINT1, IGFALS, ENG, BP, or more preferably c) any one, any two, or all three of SPINT1 , IGFALS, ENG. The panel may have the alternative features da) to de) as defined above. Such panels may be particularly but without limitation useful for predicting preterm PE, even more specifically in Australasian ancestry patients. See also the illustrative information in **Table 9H.** Particularly preferred are panels containing ADAM12 as individualised in **Table 4A,** for which the value in column S of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of SPINT1, ENG, IGFALS, MCAM, PIGF, MMRN2, QSOX1, SEPP1, ENPP2, BP, bmi, fihd, alcoh, fhpet. Preferably, the panel may contain a) any one, any two, any three, any four, or any five of SPINT1 , ENG, IGFALS, MCAM, PIGF, BP, bmi, or more preferably b) any one, any two, any three, any four, or all five of SPINT1 , ENG, IGFALS, MCAM, BP, or more preferably c) any one or both of SPINT1 , ENG. The panel may have the alternative features da) to de) as defined above. Such panels may be particularly but without limitation useful for predicting preterm PE, even more specifically regardless of ancestry of the patients. See also the illustrative information in **Table 9I.** Particularly preferred are panels containing ADAM12 as individualised in **Table 4A,** for which the value in column AD of **Table 4B** is equal to or greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, MCAM, PIGF, MMRN2, ENG, SEPP1, QSOX1, ALDOA, BP, *alcoh,* bmi, *fihd.* Preferably, the panel may contain a) any one, any two, any three, any four, or any five of IGFALS, MCAM, PIGF, MMRN2, ENG, BP, or more preferably b) any one, any two, any three, or all four of IGFALS, MCAM, PIGF, BP, or more preferably c)any one or both of IGFALS, BP. The panel may have the alternative features da) to de) as defined above. Such panels may be particularly but without limitation useful for predicting term PE, even more specifically in European ancestry patients. See also the illustrative information in **Table 9J.** Particularly preferred are panels containing ADAM12 as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, PIGF, MCAM, ENG, SPINT1, MMRN2, SEPP1, QSOX1, ECM1, ENPP2, ROB04, ALDOA, BP, bmi, *alcoh, fihd, fhpet, pbwgt, mothpet.* Preferably, the panel may contain a)any one, any two, any three, any four, or all five of IGFALS, PIGF, MCAM, ENG, BP, or more preferably b)BP. The panel may have the alternative features da)to de)as defined above. Such panels may be particularly but without limitation useful for predicting term PE, even more specifically in Australasian ancestry patients. See also the illustrative information in **Table 9K.** Particularly preferred are panels containing ADAM12 as individualised in **Table 4A,** for which the values in column V of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, PIGF, MCAM, ENG, SPINT1, MMRN2, QSOX1, SEPP1, ECM1 , ROB04, ENPP2, ALDOA, MAPRE1/3, BP, *alcoh,* bmi, *fihd, fhpet, pbwgt, mothpet.* Preferably, the panel may contain a) any one, any two, any three, any four, or all five of IGFALS, PIGF, MCAM, ENG, BP, or more preferably b) any one, any two, any three, or all four of IGFALS, PIGF, MCAM, BP, or more preferably c) any one or both of IGFALS, BP. The panel may have the alternative features da) to de) as defined above. Such panels may be particularly but without limitation useful for predicting term PE, even more specifically regardless of ancestry of the patients. See also the illustrative information in **Table 9L.** Particularly preferred are panels containing ADAM12 as individualised in **Table 4A,** for which the value in column AG of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, SPINT1, ENG, MCAM, PIGF, MMRN2, BP. Preferably, the panel may contain a)any one, any two, any three, any four, or all five of IGFALS, SPINT1, ENG, MCAM, BP, or more preferably b) any one, any two, or all three of IGFALS, SPINT1, BP. The panel may have the alternative features da) to de) as defined above. Such panels may be particularly but without limitation useful for prediction of PE in rule-in and/or rule-out tests. See also the illustrative information in **Table 9M.** Particularly preferred are panels containing ADAM12 as individualised in **Table 5Ma,** as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being ADAM12, the other constituents selected from the group consisting of IGFALS, PIGF, MCAM, MMRN2, BP. Preferably, the panel may contain a) any one or both of IGFALS, PIGF. The panel may have the alternative features da) to de) as defined above. Such panels may be particularly but without limitation useful for prediction of PE in rule-in and/or rule-out tests. See also the illustrative information in **Table 9N.** Particularly preferred are panels containing ADAM12 as individualised in **Table 5Na,** as well as test panels as defined herein which comprise so individualised panels.

The inventors further realised that many particularly well-performing test panels contain the measurement of the level of PIGF and ENG.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being PIGF and ENG, the other constituents selected from the group consisting of IGFALS, MCAM, ADAM 12, MMRN2, SPINT1 , QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of IGFALS, MCAM, ADAM12, MMRN2, SPINT1, QSOX1, SEPP1, ECM1, ROB04, LNPEP, ALDOA, MAPRE1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.* Preferably, such panel does not contain ADAM12, even more preferably such panel contains IGFALS, also preferably such panel contains IGFALS and BP, still more preferably such panel does not contain ADAM12 and contains IGFALS, also very preferably such panel does not contain ADAM12 and contains IGFALS and BP.

Also, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being PIGF and ENG, the other constituents selected from the group consisting of IGFALS, MCAM, ADAM 12, MMRN2, SPINT1 , QSOX1, SEPP1 , ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age and mothpet.* Preferably, such panel does not contain ADAM12, even more preferably such panel contains IGFALS, also preferably such panel contains IGFALS and BP, still more preferably such panel does not contain ADAM12 and contains IGFALS, also very preferably such panel does not contain ADAM12 and contains IGFALS and BP.

The inventors further realised that many particularly well-performing test panels contain the measurement of the level of PIGF and ADAM 12.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being PIGF and ADAM12, the other constituents selected from the group consisting of IGFALS, MCAM, ENG, MMRN2, SPINT1, QSOX1 , SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet* and *waist;* or selected from the group consisting of IGFALS, MCAM, ENG, MMRN2, SPINT1, QSOX1, SEPP1 , ECM1 , ROB04, LNPEP, ALDOA, MAP RE 1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet* and *waist.* Preferably, such panel does not contain ENG, even more preferably such panel contains IGFALS, also preferably such panel contains IGFALS and BP, still more preferably such panel does not contain ENG and contains IGFALS, also very preferably such panel does not contain ENG and contains IGFALS and BP.

Also, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, 2 of said constituents being PIGF and ADAM12, the other constituents selected from the group consisting of IGFALS, MCAM, ENG, MMRN2, SPINT1 , QSOX1, SEPP1 , ECM1 , ROB04, LNPEP, ALDOA, MAPRE1/3, ENPP2, LCAT, PRDX2, PRCP, BP, *alcoh,* bmi, *fihd, fhpet, vagbl, pbwgt, gest, age* and *mothpet.* Preferably, such panel does not contain ENG,even more preferably such panel contains IGFALS, also preferably such panel contains IGFALS and BP, still more preferably such panel does not contain ENG and contains IGFALS, also very preferably such panel does not contain ENG and contains IGFALS and BP.

The inventors further realised that many useful test panels do not contain PIGF, ENG and ADAM 12.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MCAM, MMRN2, SPINT1, QSOX1, SEPP1 , ECM1 , ROB04, LNPEP, ALDOA, MAPRE1/3, ENPP2, LCAT, PRDX2, PRCP, TFF3, CST3, CRP, COL6A3, IL6ST, PROC, PCDH12, BP, *alcoh,* bmi, *fihd, fhpet, vagbl, pbwgt, gest, age, mothpet, sispet,* and *waist;* or selected from the group consisting of IGFALS, MCAM, MMRN2, SPINT1, QSOX1, SEPP1 , ECM1 , ROB04, LNPEP, ALDOA, MAPRE1/3, ENPP2, LCAT, PRDX2, PRCP, TFF3, CST3, CRP, COL6A3, BP, *alcoh,* bmi, *fihd, fhpet, vagbl, pbwgt, gest, age, mothpet, sispet,* and *waist.*

Also, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MCAM, MMRN2, SPINT1 , QSOX1, SEPP1, ECM1, ROB04, LNPEP, ALDOA, MAPRE1/3, ENPP2, LCAT, PRDX2, PRCP, BP, *alcoh,* bmi, *fihd, fhpet, vagbl, pbwgt, gest, age* and *mothpet.*

Particularly preferred though exemplary and non-limiting test panels not containing PIGF, ENG and ADAM12 embodying the principles herein disclosed include the 2- or more-constituent panels individualised in the rows of Table 13E, as well as panels as defined herein which comprise the so-individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MMRN2, SEPP1 , *alcoh,* BP, *fhpet, vagbl.* Preferably, the panel may contain any one, any two, any three, or all four of IGFALS, MMRN2, *alcoh,* BP. Such panels may be particularly but without limitation useful for predicting PE in "rule-out" tests, even more specifically for predicting PE without distinction between preterm and term PE. See also the illustrative information in **Table 10A.** Particularly preferred are panels not including PIGF, ENG and ADAM 12 as individualised in **Table 4A,** for which the values in columns E and F of **Table 4A** are both equal to or greater than 0.395, as well as test panels as defined herein which comprise so individualised panels.

Preferably, a panel not containing PIGF, ENG and ADAM12 may preferably contain a) any one, any two, any three, any four, any five or any six of IGFALS, SEPP1 , MMRN2, BP, *fhpet, vagbl, alcoh,* or more preferably a) any one, any two, any three, or all four of IGFALS, MMRN2, BP, *alcoh.*

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MMRN2, SEPP1 , LNPEP, ALDOA, MAPRE1/3, MCAM, ECM1, ROB04, QSOX1, ENPP2, PRDX2, BP, *alcoh, vagbl, fhpet, fihd.* Preferably, the panel may contain a) any one, any two, any three, any four, or all five of IGFALS, MMRN2, SEPP1 , BP, *alcoh,* or more preferably b) any one, any two, or all three of IGFALS, MMRN2, BP. Such panels may be particularly but without limitation useful for predicting PE, even more specifically for predicting PE without distinction between preterm and term PE. See also the illustrative information in **Table 10B.** Particularly preferred are panels not including PIGF, ENG and ADAM12 as individualised in **Table 4A,** for which the values in columns A and B of **Table 4A** are both equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS,MMRN2, SEPP1, LNPEP, MCAM, ALDOA, MAPRE1/3, ECM1 , ROB04, QSOX1 , ENPP2, PRDX2, BP, *alcoh, vagbl, fhpet, fihd.* Preferably, the panel may contain a) any one, any two, any three, or all four of IGFALS, MMRN2, BP, *alcoh,* or more preferably b) any one, any two, or all three of IGFALS, MMRN2, BP, or more preferably c) any one or both of IGFALS, BP. Such panels may be particularly but without limitation useful for predicting preterm PE. See also the illustrative information in **Table 10C.** Particularly preferred are panels not including PIGF, ENG and ADAM12 as individualised in **Table 4A,** for which any one of the values in columns J, S or AD of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MMRN2, SEPP1, QSOX1, LNPEP, ECM1, ALDOA, MAPRE1/3, MCAM, BP, *vagbl.* Preferably, the panel may contain a)any one, any two, any three, any four, or all five of IGFALS, MMRN2, SEPP1 , BP, *vagbl,* or more preferably b)any one, any two, any three, or all four of IGFALS, MMRN2, BP, *vagbl.* Such panels may be particularly but without limitation useful for predicting term PE, even more specifically in European ancestry patients. See also the illustrative information in **Table 10D.** Particularly preferred are panels not including PIGF, ENG and ADAM12 as individualised in **Table 4A,** for which the value in column M of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MMRN2, MCAM, ECM1, SEPP1, ROB04, ALDOA, LNPEP, MAPRE1/3, BP, *alcoh, vagbl, fihd, fhpet.* Preferably, the panel may contain a) any one, any two, any three, any four, any five or any six of IGFALS, MMRN2, MCAM, ECM1 , BP, *alcoh, vagbl,* or more preferably b) any one, any two, any three, or all four of IGFALS, MMRN2, BP, alcoh, or more preferably c) any one, any two or all three of IGFALS, MMRN2, BP. Such panels may be particularly but without limitation useful for predicting term PE, even more specifically in Australasian ancestry patients. See also the illustrative information in **Table 10E.** Particularly preferred are panels not including PIGF, ENG and ADAM12 as individualised in **Table 4A,** for which the values in column V of **Table 4B** is equal to or greater than 0.745, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, said panel not including PIGF, ENG and ADAM12, and said constituents selected from the group consisting of IGFALS, MMRN2, ALDOA, MAPRE1/3, MCAM, LNPEP, QSOX1, SEPP1, ROB04, ENPP2, BP, *alcoh, vagbl, fhpet, fihd.* Preferably, the panel may contain a)any one, any two, any three, any four, any five or any six of IGFALS, MMRN2, ALDOA, MAPRE1/3, BP, *alcoh, vagbl,* or more preferably b)any one, any two, any three, or all four of IGFALS, MMRN2, BP, *alcoh,* or more preferably c)any one or both of IGFALS, BP. Such panels may be particularly but without limitation useful for predicting term PE,even more specifically regardless of ancestry of the patients. See also the illustrative information in **Table 10F.**

Particularly preferred are panels not including PIGF, ENG and ADAM12 as individualised in **Table 4A,** for which the value in column AG of **Table 4B** is equal to or greater than 0.745, as well as test panels as defined herein which comprise so individualised panels.

In only certain cases, and only insofar such cases would otherwise subsume or overlap with, preferably subsume, the scope defined by the following proviso, the proviso might potentially apply that the test panel is not one comprising IGFALS, a score for the maternal history parameter 'mother or sister with previous PE and/or father with ischemic heart disease' (henceforth *"fh_petxcardio"*), and BP.

In only certain cases, and only insofar such cases would otherwise subsume or overlap with, preferably subsume, the scope defined by the following proviso, the proviso might potentially apply that the test panel is not one comprising, IGFALS, *fihd,* and BP.

In only certain cases, and only insofar such cases would otherwise subsume or overlap with, preferably subsume, the scope defined by the following proviso, the proviso might potentially apply that the test panel is not one comprising, IGFALS, *fh_petxcardio* or *fihd,* and BP and further comprising at least one, more preferably at least two or even at least three of measurement of the level of SEPP1, measurement of the level of s-Endoglin (ENG or s-ENG), measurement of the level of quiescin Q6 (QSOX1), measurement of the level of peroxiredoxin-2 (PRDX2), measurement of blood glucose level, measurement of body mass index (BMI), a score for the maternal history parameter 'father of subject has/had ischemic heart disease' *"father_any_ihd"*), a score for the maternal history parameter 'mother or sister of subject has/had preeclampsia' (*"fh_pet"*), a value for the parameter 'high density lipoprotein level' *"bb-hdf"*)*,* a value for the parameter 'ratio of total cholesterol to high density lipoprotein' *"bb_total_hdl_ratio"*), a score for the parameter metabolic syndrome, a value for the parameter triglycerides level *"bb_trig"*), measurement of the level of vascular endothelial growth factor receptor 3 (FLT4), measurement of the level of lysosomal Pro-X carboxypeptidase (PRCP), measurement of the level of peroxiredoxin-1 (PRDX1), measurement of the level of leucyl-cystinyl aminopeptidase (LNPEP),measurement of the level of tenascin-X (TNXB), measurement of the level of basement membrane-specific heparan sulfate proteoglycan core protein (HSPG2), measurement of the level of cell surface glycoprotein (CD146, MUC18, MCAM), measurement of the level of phosphatidylinositol-glycan-specific phospholipase D (GPLD1), measurement of the level of collagen alpha-3(VI) chain (COL6A3),measurement of the level of Kunitz-type protease inhibitor 1 (SPINT1), measurement of the level of hepatocyte growth factor-like protein (MST1), measurement of the level of probable G-protein coupled receptor 126 (GPR126), measurement of the level of intercellular adhesion molecule 3 (ICAM3), measurement of the level of C-reactive protein (CRP), measurement of the level of disintegrin and metalloproteinase domain-containing protein 12 (ADAM 12), measurement of the level of phosphatidylcholine-sterol acyltransferase (LCAT), measurement of the level of roundabout homolog 4 (ROB04), measurement of the level of ectonucleotide pyrophosphatase/phosphodiesterase family member 2 (ENPP2), and measurement of the level of protein S100-A9 (S100A9).

In only certain casese, and only insofar such cases would otherwise subsume or overlap with, preferably subsume, the scope defined by the following proviso, the proviso might potentially apply that the test panel is not one comprising IGFALS and BP.

In only certain cases, and only insofar such cases would otherwise subsume or overlap with, preferably subsume, the scope defined by the following proviso, the proviso might potentially apply that the test panel is not one comprising IGFALS and BP and further comprising at least one, more preferably at least two or even at least three of measurement of the level of SEPP1 , measurement of the level of s-Endoglin, measurement of the level of QSOX1, measurement of the level of PRDX2, measurement of blood glucose level, measurement of BMI, a score for *fh_pet,* a value for *bb_hdl,* a value for *bb_total_hdl_*ratio, a score for metabolic syndrome, a value for the parameter triglycerides level *"bb_trig"*), measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3,measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROB04, measurement of the level of ENPP2, and measurement of the level of S100A9.

The inventors further realised that many particularly well-performing test panels contain TFF3.

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being TFF3, the other constituents selected from the group consisting of ECM1, MMRN2, ADAM12, MCAM, PIGF, QSOX1, IGFALS, COL6A3, ENG, PRDX2, SPINT1, LNPEP, CRP, LCAT, ENPP2, MAPRE1/3, ALDOA, BP, bmi.

Particularly preferred though exemplary and non-limiting TFF3-containing test panels embodying the principles herein disclosed include the TFF3-containing, 2- or more-constituent panels individualised in the rows of Table 12G, as well as panels as defined herein which comprise the so-individualised panels.

Exemplary, non-limiting test panels embodying the principles herein disclosed include those individualised in Table 12, as well as test panels as defined herein which comprise those individualised in Table 12.

It shall be appreciated that while Table 12 makes distinction between IGFALS (measured by mass spectrometry) and IGFALS-e (measured by ELISA), this not only individualises said panels, but is also meant to individualise otherwise identical panels containing the measurement of IGFALS by any suitable means.

Based on analysis of exemplary panels embodying the principles herein disclosed, it has been observed that certain markers and/or clinical parameters tend to be comparatively more prevalent or recurrent in the exemplary panels (see Table 12A), and their inclusion in the panels herein disclosed may thus be particularly desired.

For example, a marker or clinical parameter may be preferably included in test panels as intended herein, if the marker or clinical parameter is present in 25% or more, more preferably in 50% or more, or even more preferably in 75% or more, of the exemplary panels as set forth in Table 12A (see columns AP and AS of Table 12A for markers and clinicals, respectively; note that frequencies of B15 and BP20 in column AS of Table 12A should be added up to produce frequency of BP).

Accordingly, it is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being TFF3, the other constituents selected from the group consisting of ECM1, MMRN2, ADAM12, MCAM, PIGF, QSOX1, IGFALS, COL6A3, ENG, PRDX2, SPINT1, LNPEP, CRP, LCAT, ENPP2, MAPRE1/3, ALDOA, BP, bmi, and wherein the panel contains a) any one, any two, any three, any four, or any five of ECM1 , MMRN2, ADAM 12, MCAM, PIGF, BP, or more preferably b) any one, any two or all three of ECM1 , MMRN2, BP.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being TFF3, the other constituents selected from the group consisting of MMRN2, ECM1, MCAM, PIGF, ADAM12, COL6A3, IGFALS, ENG, LNPEP, PRDX2, QSOX1,SPINT1, CRP, LCAT, ALDOA, MAPRE1/3, BP, bmi. Preferably, the panel may contain a)any one, any two, any three, any four, or any five of MMRN2, ECM1, MCAM, PIGF, ADAM12, BP, or more preferably b)any one, any two, any three, or all four of MMRN2, ECM1, MCAM, BP, or more preferably c)any one, any two or all three of MMRN2, ECM1, BP. Such panels may be particularly but without limitation useful for predicting PE in "rule-in" tests, even more specifically for predicting PE without distinction between preterm and term PE. See also the illustrative information in **Table 12B.** Particularly preferred are panels as individualised in **Table 12,** for which the values in column **BB** of **Table 12** is greater than 0.495, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being TFF3, the other constituents selected from the group consisting of PIGF, ADAM12, ECM1, MMRN2, IGFALS, QSOX1, MCAM, ENG, SPINT1, COL6A3, PRDX2, LNPEP, LCAT, CRP, ENPP2, MAPRE1/3, BP, bmi. Preferably, the panel may contain a)any one, any two, any three, any four, or any five of PIGF, ADAM12, ECM1, MMRN2, IGFALS, QSOX1, BP, or more preferably b)any one, any two, or all three of PIGF, ADAM12, BP. Such panels may be particularly but without limitation useful for predicting PE in "rule-out" tests, even more specifically for predicting PE without distinction between preterm and term PE. See also the illustrative information in **Table 12C.** Particularly preferred are panels as individualised in **Table 12,** for which the values in column **BC** of **Table 12** is greater than 0.395, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being TFF3, the other constituents selected from the group consisting of PIGF, ADAM 12, IGFALS,MMRN2, ECM1, MCAM, QSOX1, LNPEP, LCAT, COL6A3, PRDX2, CRP, BP, and bmi. Preferably, the panel may contain a)any one,any two, any three, any four, or any five of PIGF, ADAM12, IGFALS, MMRN2, ECM1, MCAM, BP or more preferably b) any one, any two, any three, any four or all five of PIGF, ADAM12, IGFALS, MMRN2, BP, or even more preferably b) any one, any two, or all three of PIGF, ADAM12, and BP. Such panels may be particularly but without limitation useful for predicting PE, even more specifically for predicting PE without distinction between preterm and term PE. See also the illustrative information in **Table 12D.** Particularly preferred are panels as individualised in **Table 12,** for which the values in column **BA** of **Table 12** is greater than 0.795.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents,one of said constituents being TFF3, the other constituents selected from the group consisting of MMRN2, ECM1, PIGF, ADAM12, MCAM, COL6A3, IGFALS, QSOX1, SPINT1, PRDX2, ENG, LNPEP, CRP, ENPP2, MAPRE1/3, ALDOA, BP, bmi. Preferably, the panel may contain a) any one, any two, any three, any four, or any five of MMRN2, ECM1 , PIGF, ADAM 12, MCAM, COL6A3, BP, bmi, or more preferably b) any one, any two,or all three of MMRN2, ECM1, PIGF, BP, or more preferably c) MMRN2. Such panels may be particularly but without limitation useful for predicting preterm PE. See also the illustrative information in **Table 12E.** Particularly preferred are panels as individualised in **Table 12,** for which the values in column **BD** of **Table 12** is greater than 0.895, as well as test panels as defined herein which comprise so individualised panels.

It is herein disclosed a test panel, particularly for diagnosis, prediction, prognosis and/or monitoring of HDP or more particularly PE in a subject, more preferably for prediction of HDP or particularly PE in the subject, said test panel comprising or consisting of two or more constituents, or preferably three or more constituents, such as preferably between 3 and 6 constituents, for example 3, 4, 5 or 6 constituents, one of said constituents being TFF3, the other constituents selected from the group consisting of ADAM 12, MCAM, PIGF, IGFALS, BP. Preferably, the panel may contain a) any one, any two, any three, any four, or all five of ADAM12, MCAM, PIGF, IGFALS, BP. See also the illustrative information in **Table 12F.** Particularly preferred are panels as individualised in **Table 12,** for which the values in column **BG** of **Table 12** is greater than 0.795, as well as test panels as defined herein which comprise so individualised panels.

The present panels may display their diagnostic, predictive, prognostic and/or monitoring value for HDP or PE substantially throughout pregnancy and/or postpartum, or when evaluated within one or more sections of pregnancy (e.g., within 1st, 2nd and/or 3rd trimesters) or postpartum, or only when evaluated within one or more comparably short periods (e.g., about 10, 8, 6, 4 or 2 weeks) within pregnancy or postpartum. All such panels are useful and suitable herein.

The present panels can particularly advantageously allow the prediction of a subsequent / later incidence of HDP or PE in a subject which is considered healthy at the time of testing, i.e., in a subject not having clinically manifest (active) HDP or PE at the time of testing. Advantageously, the present panels can thus be particularly evaluated in subjects between about 10 and about 24 weeks of gestation, preferably between about 13 and about 22 weeks of gestation, more preferably between 14 and 21 weeks of gestation, more preferably between 15 and 20 weeks of gestation; such as between about 12 and about 18 weeks (i.e., 15 +/- about 3 weeks), preferably between about 13 and about 17 weeks (i.e., 15 +/- about 2 weeks), preferably between about 14 and about 16 weeks (i.e., 15 +/- about 1 week) or more preferably at about 15 weeks of gestation; or such as between about 17 and about 23 weeks (i.e., 20 +/- about 3 weeks), preferably between about 18 and about 22 weeks (i.e., 20 +/- about 2 weeks), preferably between about 19 and about 21 weeks (i.e., 20 +/- about 1 week) or more preferably at about 20 weeks of gestation (with reference to human female gestation). Such prediction may preferably indicate a probability, chance or risk that a tested subject will develop clinically manifest HDP or PE, optionally also allowing to predict onset within a certain time period or onset at a given age of gestation or postpartum, such as, for example, early onset preeclampsia (i.e., clinical manifestation <34 weeks of gestation) vs. preterm PE (i.e., clinical manifestation <37 weeks of gestation) vs. term PE (i.e., clinical manifestation >37 weeks of gestation). Preterm PE diagnosis commonly leads to delivery before 37 weeks of gestation.

Particularly preferably, the present panels can allow the prediction of a subsequent / later incidence of HDP or PE in a subject tested at between about 17 and about 23 weeks (i.e., 20 +/- about 3 weeks), preferably between about 18 and about 22 weeks (i.e., 20 +/about 2 weeks), preferably between about 19 and about 21 weeks (i.e., 20 +/- about 1 week) or more preferably at about 20 weeks of gestation (with reference to human female gestation).

Particularly preferably, the present panels can allow the prediction of a subsequent / later incidence of HDP or PE in a subject tested at about 20 weeks of gestation (with reference to human female gestation).

A further aspect herein disclosed relates to the use of any one test panel as specified herein for the diagnosis, prediction, prognosis and/or monitoring HDP or PE, preferably for the prediction of HDP or PE, more preferably for the prediction of PE. The present uses may be adequately qualified as in vitro or ex vivo uses, in that they apply particular *in vitro* or *ex vivo* processing and analysis on a sample obtained from a subject.

A further aspect herein disclosed relates to a method for the diagnosis, prediction, prognosis and/or monitoring of HDP or PE, preferably for the prediction of HDP or PE, more preferably for the prediction of PE, in a subject comprising testing or evaluating in said subject any one test panel as specified herein. The present methods may be adequately qualified as in vitro or ex vivo uses, in that they apply particular *in vitro* or *ex vivo* processing and analysis steps on a sample obtained from a subject.

To test or evaluate a test panel in a subject, the present methods, and particularly the examination phase of such methods in which data is collected from and/or about the subject, comprise measuring the level (i.e., quantity, amount) of the biomarker(s) comprised in said test panel in a sample from the subject and measuring or scoring the parameter(s) comprised in said test panel.

Hence, a method for the diagnosis, prediction and/or prognosis of HDP or PE in a subject, preferably for the prediction of HDP or PE, more preferably for the prediction of PE, using a test panel as taught herein may comprise steps: (i) measuring the quantity of the biomarker or biomarkers comprised in said test panel in the sample from the subject and measuring or scoring the parameter or parameters comprised in said test panel in the subject; (ii) comparing the quantity of the biomarker or biomarkers and the measurement or score of the parameter or parameters as measured or scored in (i) with a reference value representing a known diagnosis, prediction and/or prognosis of HDP or PE; (iii) finding a deviation or no deviation of the quantity of the biomarker or biomarkers and/or the measurement or score of the parameter or parameters as measured or scored in (i) from the reference value; and (iv) attributing said finding of deviation or no deviation to a particular diagnosis, prediction and/or prognosis of HDP or PE in the subject. The method may be performed for a subject at two or more successive time points and the respective outcomes at said successive time points may be compared, whereby the presence or absence of a change between the diagnosis, prediction and/or prognosis of HDP or PE at said successive time points is determined. When so applied, the method can monitor a change in the diagnosis, prediction and/or prognosis of HDP or PE in the subject over time.

For example, a deviation of the quantity of the biomarker(s) in a sample from a subject and the measurement or score of parameter(s) in the subject compared to a reference value representing the prediction or diagnosis of no HDP or PE (i.e., healthy state) or representing a good prognosis for HDP or PE can indicate respectively that the subject has or is at risk of having HDP or PE or can indicate a poor prognosis for HDP or PE in the subject (such as, e.g., a prognosis that PE will worsen or progress to HELLP syndrome or eclampsia). In another example, the absence of a deviation from a reference value representing the prediction or diagnosis of no HDP or PE or representing a good prognosis for HDP or PE can indicate respectively that the subject does not have or is not at risk of having HDP or PE or can indicate a good prognosis for HDP or PE in the subject. In yet another example, the absence of a deviation from a reference value representing the prediction or diagnosis of HDP or PE (i.e., disease state) or representing a poor prognosis for HDP or PE can indicate respectively that the subject has or is at risk of having HDP or PE or can indicate a poor prognosis for HDP or PE in the subject.

The quantity of biomarker(s) and the measurement or score of parameter(s) may vary during pregnancy and/or postpartum. To improve the accuracy of the present methods and uses, the quantity of biomarker(s) and the measurement or score of parameter(s) measured or scored at a given age of gestation or postpartum in the subject under examination are preferably compared to a reference value established at the same or substantially the same age of gestation or postpartum, e.g., within +/- about 3 weeks, preferably within +/- about 2 weeks, more preferably within +/- about 1 week, yet more preferably within +/- about 0.5 week.

Preferably, a method for monitoring HDP or PE or for monitoring the probability of developing HDP or PE using a test panel as taught herein comprises the steps of: (i) measuring the quantity of the biomarker or biomarkers comprised in said test panel in the sample from the subject and measuring or scoring the parameter or parameters comprised in said test panel in the subject at two or more successive time points; (ii) comparing the quantity of the biomarker or biomarkers and the measurement or score of the parameter or parameters as measured or scored in (i) between said two or more successive time points; (iii) finding a deviation or no deviation of the quantity of the biomarker or biomarkers and/or the measurement or score of the parameter or parameters as measured or scored in (i) between said two or more successive time points; (iv) attributing said finding of deviation or no deviation to a change in HDP or PE to a change in the probability of developing HDP or PE in the subject between the two or more successive time points.

Also disclosed is a method to determine whether a subject is or is not (such as, e.g., still is, or is no longer) in need of a therapeutic or prophylactic (preventative) treatment of HDP or PE using a test panel as taught herein, comprising: (i) measuring the quantity of the biomarker or biomarkers comprised in said test panel in the sample from the subject and measuring or scoring the parameter or parameters comprised in said test panel in the subject; (ii) comparing the quantity of the biomarker or biomarkers and the measurement or score of the parameter or parameters as measured or scored in (i) with a reference value representing a known diagnosis, prediction and/or prognosis of HDP or PE; (iii) finding a deviation or no deviation of the quantity of the biomarker or biomarkers and/or the measurement or score of the parameter or parameters as measured or scored in (i) from the reference value; (iv) inferring from said finding the presence or absence of a need for a therapeutic or prophylactic treatment of HDP or PE.

A treatment may be particularly indicated where the method allows for a conclusion that the subject has or is at risk of having HDP or PE or has a poor prognosis for HDP or PE. For example, a patient having HDP or PE upon admission to or during stay in a medical care centre may be tested as taught herein for the necessity of continuing the treatment of said HDP or PE, and may be discharged when such treatment is no longer needed or is needed only to a given limited extent.

Illustrative therapeutic and prophylactic treatments of HDP or PE encompass inter alia anti-hypertensive treatments (using inter alia beta-blockers, calcium channel blockers, vasodilators and/or DOPA decarboxylase inhibitors, such as, e.g., methyldopa, labetalol, acebutolol, metoprolol, pindolol, propranolol, nifedipine, isradipine and/or hydralazine, MgS04 treatment and/or aspirin (see, e.g., Bujold et al., Obstet Gynecol 2010, vol. 116, 402-14)), abortion, and delivery such as by labour induction or Caesarean section.

It is herein disclosed a system comprising:

- a computer data repository that comprises a reference value of the quantity of biomarkers comprised in a test panel as defined herein and, where the test panel comprises a clinical parameter or parameters, of measurement or score for said clinical parameter or parameters, said reference value representing a known diagnosis, prediction and/or prognosis of HDP or PE, preferably prediction of HDP or PE, more preferably prediction of PE; and
- a computer system programmed to access the data repository and to use information from the data repository in combination with information on the quantity of biomarkers comprised in the test panel in a sample from a subject and, where the test panel comprises a clinical parameter or parameters, on measurement or score for said clinical parameter or parameters in the subject, to make a diagnosis, prediction and/or prognosis of HDP or PE, preferably prediction of HDP or PE, more preferably prediction of PE, in the subject.

It is herein disclosed a method for making diagnosis, prediction and/or prognosis of HDP or PE, preferably prediction of HDP or PE, more preferably prediction of PE, in a subject comprising:

(i) receiving data representative of values of the quantity of biomarkers comprised in a test panel as defined herein in a sample from a subject and, where the test panel comprises a clinical parameter or parameters, on measurement or score for said clinical parameter or parameters in the subject;

(ii) accessing a data repository on a computer, said data repository comprising a reference value of the quantity of biomarkers comprised in the test panel and, where the test panel comprises a clinical parameter or parameters, of measurement or score for said clinical parameter or parameters, said reference value representing a known diagnosis, prediction and/or prognosis of HDP or PE, preferably prediction of HDP or PE, more preferably prediction of PE; and

(iii) comparing the data as received in (i) with the reference value in the data repository on the computer, thereby making a diagnosis, prediction and/or prognosis of HDP or PE, preferably prediction of HDP or PE, more preferably prediction of PE, in the subject.

Preferably, the determination of what action is to be taken, e.g., by a clinician, in view of said diagnosis, prediction and/or prognosis is performed by a (the) computer. Preferably, a (the) computer reports (i.e., generates an electronic report of) the action to be taken, preferably substantially in real time.

Preferably,an algorithm can be developed, based on the sum of the individual scores between 0 and 1 attributed to each specific biomarker level measured in the sample of the subject. HDP or PE can then be predicted if said sum reaches a certain threshold. Preferably, the weight of each individual biomarker score can be adjusted in order to improve the performance of the algorithm.

It is herein further disclosed a method for treating HDP or PE, preferably PE, in a subject in need of said treatment, the method comprising the steps of:

(i) measuring the quantity of the biomarkers comprised in a test panel as defined herein in a sample from the subject and, where the test panel comprises a clinical parameter or parameters, measuring or scoring said clinical parameter or parameters in the subject;

(ii) comparing the quantity of the biomarkers as measured in (i) and, where the test panel comprises a clinical parameter or parameters, the measurement or score of said parameter or parameters as measured or scored in (i) with a reference value of the quantity of the biomarkers comprised in the test panel and, where the test panel comprises a clinical parameter or parameters, of measurement or score for said clinical parameter or parameters, said reference value representing a known diagnosis, prediction and/or prognosis of HDP or PE, preferably prediction of HDP or PE, more preferably prediction of PE;

(iii) finding a deviation or no deviation of the quantity of the biomarkers as measured in (i) and, where the test panel comprises a clinical parameter or parameters, of the measurement or score of said parameter or parameters as measured or scored in (i) from the reference value;

(iv) attributing said finding of deviation or no deviation to a particular diagnosis, prediction and/or prognosis of HDP or PE, preferably prediction of HDP or PE, more preferably prediction of PE, in the subject;

(v) inferring from said particular diagnosis, prediction and/or prognosis of HDP or PE, preferably prediction of HDP or PE, more preferably prediction of PE, in the subject the presence or absence of a need for a therapeutic or prophylactic treatment of the HDP or PE, preferably PE, in the subject; and

(vi) subjecting the subject to a therapeutic or prophylactic treatment of the HDP or PE, preferably PE, when the subject is in need of said treatment, such as for example, administering a therapeutically or prophylactically effective amount of an active pharmaceutical ingredient capable of treating the HDP or PE, preferably PE, to said subject, when the subject is in need of said treatment;

(vi') Alternatively, the subject can be subjected to close monitoring in the hospital or at home, and restriction of activities to reduce the risks of early (pre-term) delivery of the baby. In said event, certain pregnancy prolonging drugs can be administered to the subject, or corticosteroids can be administered to accelerate the baby's lung development;

(vi") Further alternatively, if the baby is sufficiently at the end of term, early delivery of the baby can be induced, or the baby can be delivered by Caesarean section, in order to reduce the risks involved with hypertension for the mother;

(vi"') Yet further alternatively, if the mother's life is at a too high risk, abortion could be considered in order to safeguard the mother's life.

Illustrative therapeutic and prophylactic treatments of HDP or PE encompass *inter alia* anti-hypertensive treatments (using *inter alia* beta-blockers, calcium channel blockers, vasodilators and/or DOPA decarboxylase inhibitors, such as, e.g., methyldopa, labetalol, acebutolol, metoprolol, pindolol, propranolol, nifedipine, isradipine and/or hydralazine, MgS04 treatment and/or aspirin (see, e.g., Bujold et al., Obstet Gynecol 2010, vol. 116, 402-14)), regulation of fluid intake, abortion, and delivery such as by labour induction or Caesarean section.

In the aforementioned uses and methods, the test panels as defined herein may preferably be tested or evaluated, at 20 +/- about 3 weeks of gestation, more preferably 20 +/- about 2 weeks of gestation, even more preferably 20 +/- about 1 week of gestation or still more preferably at about 20 weeks of gestation. Particularly preferably, testing at such times allows to predict (the risk of) later development of HDP or PE in the subject.

Preferably, the aforementioned uses and methods may be performed for the prediction of preeclampsia without distinction between preterm and term PE.

Preferably, the aforementioned uses and methods may be performed specifically for the prediction of preterm preeclampsia.

Preferably, the aforementioned uses and methods may be performed specifically for the prediction of preterm preeclampsia.

Preferably, the aforementioned uses and methods may be performed for the prediction of early onset and/or late onset preeclampsia, i.e., for the prediction of only early onset pre-eclampsia, for the prediction of only late onset pre-eclampsia, or for the prediction of both early onset and late onset pre-eclampsia (e.g., for the prediction of each early onset PE and late onset PE, or for the prediction of PE without discrimination between early onset or late onset).

Preferably, the uses and methods as taught herein may be performed for the prediction of early onset and late onset pre-eclampsia, i.e., PE developing during the course of pregnancy or post partum. Also preferred, the uses and methods as taught herein may be performed for the prediction of early pre-eclampsia, i.e., pre-eclampsia developing before 34 weeks of gestation.

As noted above, PE may be associated with foetal complications such as intrauterine growth retardation (IUGR) and small for gestational age (SGA). Moreover, a biological relationship involving common pathological pathways seems to be suspected between these conditions.

Hence, preferably, the aforementioned uses and methods may be performed for the prediction of preeclampsia not accompanied by IUGR/SGA (i.e., PE without IUGR/SGA).

Preferably, the aforementioned uses and methods may be performed for the prediction of preeclampsia accompanied by IUGR/SGA (i.e., PE with IUGR/SGA).

Preferably, the aforementioned uses and methods may be envisaged for the prediction of IUGR/SGA without preeclampsia.

Preferably, panels as taught herein may be indicated for "rule-in" tests for predicting PE, as defined elsewhere in the specification. Such tests can identify a "high risk" population, that would be eligible for higher care level, more frequent visits, follow up testing / monitoring. Such rule-in tests aim to limit false positive referrals and thereby health care costs and preferably enrich of preterm pre-eclampsia cases. Preferred test criteria are that per pre-eclampsia case there are maximally 4 referrals of false positives (PPV ≥20%) and the so-identified "high risk" group contains at least 50% of all future pre-eclampsia cases (sensitivity ≥50%).

Preferably, panels as taught herein may be indicated for "rule-out" tests for predicting PE, as defined elsewhere in the specification. Such tests can identify a "low risk" population, that would be eligible for lower care level and less frequent visits. Such rule-out tests aim to limit false negative referrals, such that virtually no future preterm preeclampsia cases are classified as "low risk". Preferred test criteria are that per 99 controls maximally 1 referral of a false negative (NPV ≥ 99%) and the "low risk" group contains at least 40% of all true low risks (specificity ≥ 40%).

The herein disclosed test panels, methods and uses may be particularly useful in subjects known or expected to be at risk of developing HDP or PE, e.g., having one or more risk factors for HDP or PE. Without limitation risk factors associated with HDP and preferably PE include nulliparity, multiple gestation, prolonged interval between pregnancies, history of HDP or PE in a prior pregnancy or family history of HDP or PE, extremes in age (<20 years and >40 years), obesity, chronic hypertension, history of hypertension, chronic renal disease, migraine, headaches, (gestational) diabetes mellitus, history of diabetes mellitus, polycystic ovarian syndrome, autoimmune disorders such as lupus, rheumatoid arthritis, sarcoidosis or MS, vascular or connective tissue diseases, vitamin D insufficiency, antiphospholipid antibody syndrome or inherited thrombophilia, male partner whose previous partner had HDP or PE , hydrops fetalis and unexplained foetal intrauterine growth restriction.

Preferably, the present test panels, methods and uses may be complemented or combined with determination of the presence or absence and/or level of one or more risk factors for HDP or PE in the subject. By means of example, a risk factor may be included as a constituent in a panel.

In general clinical practice obese subjects (BMI ≥ 30 pre-pregnancy or in 1 st trimester) are considered at risk for a number pregnancy complications, including for example gestational diabetes, pre-eclampsia, etc., and therefore already subject to increased antenatal care (NHS National Institute for Health and Clinical Excellence (NICE) clinical guideline 62: Antenatal Care - Routine Care for the Healthy Pregnant woman, March 2008). Therefore, preferably the panels as taught herein may be used in non-obese subjects (BMI < 30), more particularly in nulliparous non-obese women. In other embodiments panels as taught herein may be used in obese subjects (BMI ≥ 30).

Preferably, the panels as taught herein may be used in nulliparous subjects.

The present test panels, methods and uses may also benefit from being further complemented or combined with the assessment of one or more other biomarkers and/or clinical parameters relevant for the respective diseases and conditions.

By means of example and not limitation, other biomarkers useful in evaluating HDP or PE include soluble fms-like tyrosine kinase-1 (sFlt-1, sVEGFR-1) (Maynard et al. 2003, J Clin Invest 111(5): 649-58), and vascular endothelial growth factor (VEGF) (Polliotti et al. 2003; Obstet Gynecol 101 : 1266-74), and biomarkers disclosed in WO2009/097584A1 to Proteogenix Inc. and WO2009/108073A1 to Auckland Uniservices Ltd.. By means of example, such additional biomarker may be included as a constituent in a panel.

Preferably, the measurement of the level of s-Flt-1 may considered, and may particularly advantageously be combined with the measurement of the level of PlGF, such as to obtain a s-Flt1/PlGF ratio. By means of example, s-Flt1 or s- Flt1/PlGF ratio may be included as a constituent in a panel.

Additional useful clinical parameters for the pregnant female subject may include without limitation, ethnicity, smoking status (esp. at 15 weeks visit), etc. One additional clinical parameter of particular interest may be a score for the parameter *metabolic syndrome.* The condition metabolic syndrome is known per se (see, e.g., Alberti et al. Diabetic Medicine, 2006, vol. 23, 469-480) and any subject diagnosed as having metabolic syndrome according to art-established definitions and methods would be scored as, e.g., "1 " or "yes" or "positive" for the parameter metabolic syndrome as intended herein. In preferred embodiments, a subject can be qualified as being metabolic syndrome positive (e.g., score = "1 " or "yes" or "positive") when she fulfilled at least 2 of the following 4 conditions: 1) BMI >=30, 2) *bb_trig* >1.7 (mmol/L)(the parameter *"bb_trig"* denotes the triglycerides level, for example, in the experimental section this parameter may denote the triglycerides level as obtained from the subject and stored in the SCOPE biobank), 3) *bb_hdl* <1 .29 (mmol/L) and 4) 1 st_vst_sbp_2nd > 130 (mm Hg) or 1 st_vst_dbp_2nd > 85 (mm Hg). A potential surrogate or proxy for the parameter metabolic syndrome may be waist circumference at 15 weeks (*waist* as used in the panels disclosed herein), which is an assessment of truncal obesity (Lancet, 366,1059-1062). By means of example, such clinical parameters may be included as a constituent in a panel.

Any one test panel, method or use as taught herein may preferably allow for sensitivity and/or specificity (preferably, sensitivity and specificity) of at least 50%, at least 60%, at least 70% or at least 80%, *e.g.*, ≥ 85% or ≥ 90% or ≥95%, *e.g.,* between about 80% and 100% or between about 85% and 95%, for a desired outcome, such as prediction of PE, prediction of preterm PE or prediction of term PE, in a desired patient population.

Preferably, any one test panel, method or use as taught herein may allow for AUC value equal to or greater than 0.750, more preferably equal to or greater than 0.775, even more preferably equal to or greater than 0.800, yet more preferably equal to or greater than 0.850, and most preferably equal to or greater than 0.900, for a desired outcome, such as prediction of PE, prediction of preterm PE or prediction of term PE, in a desired patient population.

Reference throughout this specification to "diseases and/or conditions" encompasses any such diseases and conditions as disclosed herein insofar consistent with the context of a particular recitation, more specifically but without limitation including hypertensive disorders of pregnancy (HDP) and preferably preeclampsia (PE).

The present test panels, methods and uses may be applied to subjects who have not yet been diagnosed as having the respective diseases and conditions (for example, preventative screening), or who have been diagnosed as having such, or who are suspected of having such (for example, display one or more characteristic signs and/or symptoms), or who are at risk of developing such (for example, genetic predisposition; presence of one or more developmental, environmental or behavioural risk factors). The test panels, methods and uses may also be used to detect various stages of progression or severity of the diseases and conditions. The test panels, methods and uses may also be used to detect response of the diseases and conditions to prophylactic or therapeutic treatments or other interventions. The test panels, methods and uses can furthermore be used to help the medical practitioner in deciding upon worsening, status-quo, partial recovery, or complete recovery of the subject from the diseases and conditions, resulting in either further treatment or observation or in discharge of the patient from a medical care centre. Also, the test panels, methods and uses as taught herein may be employed for population screening, such as, *e.g.,* screening in a general population or in a population stratified based on one or more criteria, *e.g., age,* ancestry, occupation, presence or absence of risk factors of the respective diseases and conditions, *etc.*

The respective quantities, measurements or scores for the biomarker(s) and parameter(s) in the present test panels may be evaluated separately and individually, i.e., each compared with its corresponding reference value. More advantageously, the quantities, measurements or scores for the biomarker(s) and parameter(s) may be used to establish a biomarker-and-parameter profile, which can be suitably compared with a corresponding multi-parameter reference value. In yet another alternative, the quantities, measurements or scores for the biomarker(s) and parameter(s) may each be modulated by an appropriate weighing factor and added up to yield a single value, which can then be suitably compared with a corresponding reference value obtained accordingly. One shall appreciate that such weighing factors may depend on the methodology used to quantify biomarkers and measure or score parameters, and for each particular experimental setting may be determined and comprised in a model suitable for diagnosis, prediction and/or prognosis of the diseases and conditions as taught herein. Various methods can be used for the purpose of establishing such models, e.g., support vector machine, Bayes classifiers, logistic regression, etc. (Cruz et al. Applications of Machine Learning in Cancer Prediction and Prognosis. Cancer Informatics 2007; 2; 59-77).

Reference values as employed herein may be established according to known procedures previously employed for other test panels comprising biomarkers and/or clinical parameters. Reference values may be established either within (i.e., constituting a step of) or external to (i.e., not constituting a step of) the methods and uses as taught herein.

Accordingly, any one of the methods or uses taught herein may comprise a step of establishing a requisite reference value.

Hence, it is herein disclosed a method for establishing a reference value for a test panel as taught herein, said reference value representing:

(a) a prediction or diagnosis of the absence of the diseases or conditions as taught herein or a good prognosis thereof, or

(b) a prediction or diagnosis of the diseases or conditions as taught herein or a poor prognosis thereof,

comprising:

(i) measuring the quantity of the biomarker or biomarkers comprised in said test panel in a sample from, and measuring or scoring the parameter or parameters comprised in said test panel in:

(i a) one or more subjects not having the respective diseases or conditions or not being at risk of having such or having a good prognosis for such, or

(i b) one or more subjects having the respective diseases or conditions or being at risk of having such or having a poor prognosis for such, and

(ii a) establishing from the quantity of the biomarker or biomarkers and measurement or score of the parameter or parameters as measured in (i a) the reference value representing the prediction or diagnosis of the absence of the respective diseases or conditions or representing the good prognosis therefore, or

(ii b) establishing from the quantity of the biomarker or biomarkers and measurement or score of the parameter or parameters as measured in (i b) the reference value representing the prediction or diagnosis of the respective diseases or conditions or representing the poor prognosis therefore.

Further disclosed is a method for establishing a base-line reference value for a test panel as taught herein in a subject, comprising: (i) measuring the quantity of the biomarker or biomarkers comprised in said test panel in a sample from the subject, and measuring or scoring the parameter or parameters comprised in said test panel in the subject at one or more time points when the subject is not suffering from the diseases or conditions as taught herein, and (ii) establishing from the quantity of the biomarker or biomarkers and measurement or score of the parameter or parameters as measured in (i) a range or mean reference value for the subject, which is the base-line reference value for said subject.

The quantity of biomarker(s) may be measured by any suitable technique such as may be known in the art.

For example, one may employ binding agents capable of specifically binding to the respective biomarkers. Binding agent may be *inter alia* an antibody, aptamer, photoaptamer, protein, peptide, peptidomimetic or a small molecule. For instance, one may employ an immunoassay technology or a mass spectrometry analysis method or a chromatography method, or a combination of said methods.

Preferably, in the methods as taught herein, the quantity of any one or more markers as taught herein is measured using an immunoassay technology, in preferred but non-limiting examples, using enzyme-linked immunosorbent assay (ELISA), radioimmunoassay (RIA), or ELISPOT technologies, preferably using ELISA.

Preferably, in the methods as taught herein, the quantity of any one or more markers as taught herein is measured using a binding agent capable of specifically binding to the respective markers, in preferred but non-limiting examples, using an aptamer, antibody, photoaptamer, protein, peptide, peptidomimetic, or a small molecule, preferably using an aptamer or antibody, more preferably using an antibody.

Further disclosed is a kit, particularly a kit for the diagnosis, prediction, prognosis and/or monitoring of the diseases or conditions as taught herein in a subject, the kit comprising (i) means for measuring the biomarker or biomarkers comprised in a test panel as taught herein, particularly in a sample from the subject, (ii) optionally means for measuring or scoring the parameter or parameters comprised in the test panel (however, said parameter(s) may be determined independently using devices other than the kit), particularly in the subject, and (iii) optionally and preferably a reference value for the test panel or means for establishing said reference value, wherein said reference value represents a known diagnosis, prediction and/or prognosis of the respective diseases or conditions.

The means for measuring the quantity of the biomarker(s) in the present kits may comprise, respectively, one or more binding agents capable of specifically binding to said biomarker(s). Binding agent may be *inter alia* an antibody, aptamer, photoaptamer, protein, peptide, peptidomimetic or a small molecule. Preferably, the present kits comprise one or more binding agents capable of specifically binding to said one or more markers as taught herein, such as one or more aptamers, antibodies, photoaptamers, proteins, peptides, peptidomimetics or small molecules, preferably one or more aptamers or antibodies, more preferably one or more aptamers capable of specifically binding to said one or more markers as taught herein. A binding agent may be advantageously immobilised on a solid phase or support. The present kits may employ an immunoassay technology or mass spectrometry analysis technology or chromatography technology, or a combination of said technologies, preferably the present kits employ an immunoassay technology, in preferred but non-limiting examples, enzyme-linked immunosorbent assay (ELISA), radioimmunoassay (RIA), or ELISPOT technologies, preferably using ELISA. Hence, the means for measuring the quantity of marker(s) may be an immunoassay, e.g., an immunoassay employing antibody(ies) and/or aptamers, e.g., ELISA, RIA, or ELISPOT assay.

Disclosed is thus also a kit, particularly a kit for the diagnosis, prediction, prognosis and/or monitoring the diseases or conditions as taught herein in a subject, the kit comprising: (i) one or more binding agents capable of specifically binding to the biomarker or biomarkers comprised in a test panel as taught herein, particularly in a sample from the subject, (ii) preferably, a known quantity or concentration of said biomarker or biomarkers (*e.g.,* for use as controls, standards and/or calibrators), (iii) optionally means for measuring or scoring the parameter or parameters comprised in the test panel, particularly in the subject (however, said parameter(s) may be determined independently using devices other than the kit), (iv) optionally and preferably a reference value for the test panel or means for establishing said reference value, wherein said reference value represents a known diagnosis, prediction and/or prognosis of the respective diseases or conditions. Said components under (i) and/or (ii) may be suitably labelled as taught elsewhere in this specification.

Further disclosed is the use of any one kit as described herein for the diagnosis, prediction, prognosis and/or monitoring the diseases or conditions as taught herein.

Also disclosed are reagents and tools useful for measuring biomarker(s) comprised in test panels as taught herein. Hence, disclosed is a protein, polypeptide or peptide array or microarray comprising the biomarker or biomarkers comprised in a test panel as taught herein. Also disclosed is a binding agent array or microarray comprising one or more binding agents capable of specifically binding to the biomarker or biomarkers comprised in a test panel as taught herein, preferably a known quantity of, or concentration of said binding agents. Such binding agents may be as detailed elsewhere in this specification. Also disclosed are kits as taught here above configured as portable devices, such as, for example, bed-side devices, for use at home or in clinical settings.

A related aspect herein disclosed relates to a portable testing device capable of measuring the quantity of the biomarker or biomarkers comprised in a test panel as taught herein in a sample from a subject comprising : (i) means for obtaining a sample from the subject, (ii) means for measuring the quantity of the biomarker or biomarkers comprised in the test panel in said sample, and (iii) means for visualising the quantity of said biomarker or biomarkers in the sample. The testing device may optionally further comprise (iv) means for measuring or scoring the parameter or parameters comprised in the test panel in the subject (however, said parameter(s) may be determined independently using devices other than the kit), and/or (v) means for visualising the measurement or score of said parameter or parameters in the subject. The means of parts (ii) and (iii) may be the same. The means of parts (iii) and (v) may be the same.

Preferably, said visualising means is capable of indicating whether the quantity of the biomarker or biomarkers and the measurement or score of the parameter or parameters in the subject deviates from (e.g., is below or above) a certain reference or base-line value as taught herein. Hence, the portable testing device may suitably also comprise said reference or base-line value or means for establishing the same.

The above and further aspects and preferred embodiments of the invention are described in the following sections and in the appended claims. The subject matter of appended claims is hereby specifically incorporated in this specification.

**BRIEF DESCRIPTION OF FIGURES**

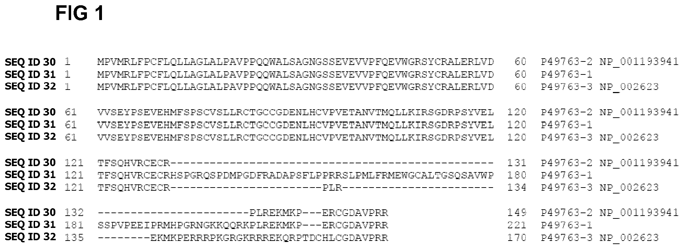

Figure 1 represents the amino acid sequence alignment of the three isoforms of human placental growth factor: P49763-2 (SEQ ID NO: 30), P49763-1 (SEQ ID NO: 31) and P49763-3 (SEQ ID NO: 32).

**DETAILED DESCRIPTION**

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less , more preferably or less, and still more preferably +/-0.1 % or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any >3, >4, >5, >6 or >7 etc. of said members, and up to all said members.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The inventors identified test panels comprising biomarker(s) and clinical parameter(s) useful in diagnosis, prognosis, prediction and/or monitoring hypertensive disorders of pregnancy (HDP), and more specifically preeclampsia (PE).

The term "panel" or "test panel" as used herein broadly refers to combinations, sets or groupings of biomarkers and/or parameters, particularly where the testing or evaluation of such panels in subjects is predictive and/or informative as regards the subject's status, disease or condition. Without limitation, a panel as intended herein may comprise or consist of between 3 and 10, preferably between 4 and 8, more preferably 5 or 6 biomarkers and parameters.

The term "biomarker" is widespread in the art and may broadly denote a biological molecule and/or a detectable portion thereof whose qualitative and/or quantitative evaluation in a subject is, alone or combined with other data, predictive and/or informative (e.g., predictive, diagnostic and/or prognostic) with respect to one or more aspects of the subject's phenotype and/or genotype, such as, for example, with respect to the status of the subject as to a given disease or condition. Particularly, biomarkers as intended herein may be metabolite-, RNA- (esp. mRNA-), peptide-, polypeptide- or protein-based, preferably peptide-, polypeptide- or protein-based.

The term "parameter" or "clinical parameter" is widespread in the art and may broadly denote information about a subject that is obtained in a clinical setting that may be relevant to a disease or condition of the subject. Particularly, parameters may encompass non-sample and/or non-analyte information. By means of illustration, clinical parameters common in medical practice may including *inter alia* basic subject characteristics such as, e.g., *age,* gender, weight, height, BMI, body type, ethnicity; biophysical parameters (e.g., diastolic blood pressure, systolic blood pressure, heart rate); imaging information (e.g., MRI); anamnesis information (e.g., medical history of the subject or its relatives); environmental factors etc.

As intended herein, the measurement of blood pressure may refer to any relevant blood pressure parameter, such as without limitation 1st or 2nd measurement, diastolic pressure, systolic pressure and/or mean arterial pressure. Mean arterial pressure (MAP) may be preferred, such as MAP at 15 weeks visit or MAP at 20 weeks visit. Mean arterial pressure at 15 weeks visit calculated from 2nd measurement blood pressures (henceforth *"1st_vst_map_2nd"*) may be preferred in test panels for 15 +/- 2 or 1 weeks. Mean arterial pressure calculated at 20 weeks visit from 1 st measurement blood pressures (henceforth *"2nd_vst_map_1st"*) may be preferred in test panels for 20 +/- 2 or 1 weeks. Further, blood pressure measurements may encompass the parameters "*1 st_vst_dbp_2nd"*, i.e., diastolic blood pressure as obtained from the 2nd measurement at the 15 weeks visit, *"1st_vst_sbp_2nd",* i.e., systolic blood pressure as obtained from the 2nd measurement at the 15 weeks visit and/or *"2nd_vst_map_2nd",*i.e., the mean arterial pressure calculated at 20 weeks visit from 2nd measurement blood pressures. The measurement of any one or more blood pressure parameters *1st_vst_dbp_2nd, 1st_vst_sbp_2nd, 1st_vst_map_2nd,* and *2nd_vst_map_2nd* may be particularly useful in panels herein disclosed. Blood pressure measurement may be abbreviated BP. Blood pressure measurement at 15 weeks of pregnancy may be abbreviated BP15 and blood pressure measurement at 20 weeks of pregnancy may be abbreviated BP20. BP15 and BP20 are encompassed by the term BP.

Hypertensive disorders of pregnancy (HDP) include a heterogeneous collection of diseases and conditions associated with hypertension during pregnancy and/or post partum (e.g., up to 12 weeks postpartum).

HDP may be conveniently classified as follows:

    I. Hypertension induced by pregnancy

        a. without proteinuria or (generalised) oedema
        b. with proteinuria or (generalised) oedema (i.e., preeclampsia)

            i. mild
            ii. severe

        c. eclampsia

    II. Coincidental hypertension (chronic hypertension)
    III. Hypertension worsened by pregnancy (pregnancy aggravated hypertension)

        a. superimposed preeclampsia
        b. superimposed eclampsia

Recent studies may no longer classify PE as mild or severe, but may instead identify PE groups based on gestation time, preferably: a. early onset (i.e., clinical manifestation <34 weeks of gestation); b. preterm (i.e., clinical manifestation at <37 weeks of gestation such as for example at >34 and <37 weeks of gestation); c. term (i.e., clinical manifestation ≥37 weeks of gestation).

HPD may otherwise be categorised as pre-existing or gestational, optionally adding "with preeclampsia" to either category if maternal or foetal symptoms, signs or test results necessitate this.

Non-proteinuric hypertension of pregnancy may be conveniently defined as blood pressure of systolic BP>140 mmHg and/or a diastolic BP>90 mmHg measured on two separate occasions over 4 hours apart, e.g., about 4 hours to about 168 hours apart. When the hypertension was measured before pregnancy or is measured before 20 weeks of gestation, one may commonly denote such as chronic hypertension. When the hypertension is measured in a previously normotensive woman after 20 weeks of gestation, one may denote such as pregnancy-induced hypertension. Typically, pregnancy-induced hypertension will resolve within 12 weeks postpartum. When blood pressure of at least 140/90 mmHg is measured but does not persist for more than 6 hours, one may denote such as transient hypertension.

Proteinuric hypertension of pregnancy may be as defined in the previous paragraph, further accompanied by >300 mg of total protein in a 24-hour urine collection.

HDP also encompasses diseases and conditions commonly denoted in the art as gestational hypertension, mild preeclampsia, pregnancy-induced hypertension, specific hypertension of pregnancy, toxaemia of pregnancy, etc.

The terms "gestational age", "age of gestation" and similar are widespread in the art and commonly denote the time as measured in weeks from the 1st day of a female's last menstrual period. A human pregnancy of normal gestation is between about 38 and 42 weeks, preferably about 40 weeks.

"Preeclampsia" (PE or pre-eclampsia) generally denotes a pregnancy-associated disease or condition characterised by hypertension with proteinuria or oedema or both. PE may also be accompanied by glomerular dysfunction, brain oedema, liver oedema, coagulation abnormalities and/or other complications.

PE may be conveniently defined as some combination of the following signs and symptoms:

(1) a systolic blood pressure (BP)>140 mmHg and/or a diastolic BP>90 mmHg after 20 weeks gestation (generally measured on two occasions over 4 hours apart, e.g., about 4 to about 168 hours apart),
(2) new onset proteinuria (1 + by dipstick on urinanalysis, >300 mg of protein in a 24-hour urine collection, or a single random urine sample having a protein/creatinine ratio $\geq$0.3) after 20 weeks gestation, and
(3) resolution of hypertension and proteinuria by 12 weeks postpartum,

such as in particular a combination of hypertension and proteinuria.

Severe PE may be conveniently defined as:

(1) a systolic BP $\geq$160 mmHg or diastolic BP$\geq$1 10 mmHg (generally measured on two occasions over 4 hours apart, e.g., about 4 to about 168 hours apart) or

(2) proteinuria characterised by a measurement of $\geq$3.5 g in a 24-hour urine collection or two random urine specimens with at least 3+ protein by dipstick.

In PE, hypertension and proteinuria generally occur within seven days of each other. In severe PE, severe hypertension, severe proteinuria or HELLP syndrome (haemolysis, elevated liver enzymes, low platelets) or eclampsia can occur simultaneously or only one symptom at a time.

Occasionally, severe PE can lead to the development of seizures, i.e., to eclampsia. Eclampsia can also include dysfunction or damage to several organs or tissues such as the liver (e.g., hepatocellular damage, periportal necrosis) and the central nervous system (e.g., cerebral oedema and cerebral haemorrhage).

Hence, HDP also encompasses diseases and conditions commonly denoted in the art as PE, including *inter alia* mild PE, severe PE and PE with further complications, eclampsia and HELLP syndrome.

The term "preterm pre-eclampsia" in particular denotes pre-eclampsia that warrants for delivery of the child before 37 weeks of gestation (<37 weeks).

The terms "predicting" or "prediction", "diagnosing" or "diagnosis" and "prognosticating" or "prognosis" are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

By means of further explanation and without limitation, "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated inter alia as an absolute value, range or statistics, or may be indicated relative to a suitable control subject or subject population (such as, *e.g.*, relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, i.e., that the subject is at risk of having such (*e.g.*, the risk is significantly increased vis-a-vis a control subject or subject population). The term "prediction of no" diseases or conditions as taught herein as described herein in a subject may particularly mean that the subject has a 'negative' prediction of such, i.e., that the subject's risk of having such is not significantly increased vis-a-vis a control subject or subject population.

Preferably prediction of HDP in particular PE in the context of the present invention may take form of "rule-in" tests, whereby panels are employed that can adequately predict the HDP preferably PE without identifying too many false positives. The test is thus designed to have maximum sensitivity for ruling patients into a certain treatment regimen or high risk group. In particularly preferred embodiments, the panels as used herein can provide for a Positive Predictive Value (PPV) above or equal to 0.2 (i.e., 20%). Such PPV value is deemed clinically in low prevalence diseases, such as HDP particularly PE.

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition and the prospect (e.g., the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

Hence, prediction or prognosis of a disease or condition can inter alia allow to predict or make a prognosis of the occurrence of the disease or condition, or to predict or make a prognosis of the progression, aggravation, alleviation or recurrence of the disease or condition or response to treatment or to other external or internal factors, situations or stressors, *etc.*

Further, monitoring a disease or condition can inter alia allow to predict the occurrence of the disease or condition, or to monitor the progression, aggravation, alleviation or recurrence of the disease or condition, or response to treatment or to other external or internal factors, situations or stressors, etc. Advantageously, monitoring may be applied in the course of a medical treatment of a subject, preferably medical treatment aimed at alleviating the so-monitored disease or condition. Such monitoring may be comprised, e.g., in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

The term "subject" or "patient" as used herein typically denotes humans, but may also encompass reference to non-human animals, preferably warm-blooded animals, more preferably viviparous animals, even more preferably mammals, such as, e.g., non-human primates, rodents, canines, felines, equines, ovines, porcines, and the like. Particularly intended are female subjects, more particularly pregnant or postpartum female subjects. The present test panels, methods and uses may be carried out as from any age of gestation (e.g., from about 5 or from about 8 weeks of gestation) and up to about 12 weeks postpartum (e.g., up to about 6 weeks or about 3 weeks post partum), and preferably between about 10 weeks and about 24 weeks of gestation.

The terms "sample" or "biological sample" as used herein include any biological specimen obtained from a subject. Samples may include, without limitation, whole blood, plasma, serum, red blood cells, white blood cells (*e.g.*, peripheral blood mononuclear cells), saliva, urine, stool (*i.e.*, faeces), tears, sweat, sebum, nipple aspirate, ductal lavage, tumour exudates, synovial fluid, cerebrospinal fluid, lymph, fine needle aspirate, amniotic fluid, any other bodily fluid, nail clippings, cell lysates, cellular secretion products, inflammation fluid, vaginal secretions, or biopsies such as preferably placental biopsies. Preferred samples may include ones comprising any one or more biomarkers as taught herein in detectable quantities. Preferably, the sample may be whole blood or a fractional component thereof such as, e.g., plasma, serum, or a cell pellet. Preferably the sample is readily obtainable by minimally invasive methods, allowing to remove or isolate said sample from the subject. Samples may also include tissue samples and biopsies, tissue homogenates and the like.

Preferably, the sample is blood plasma. The term "plasma" generally denotes the substantially colourless watery fluid of the blood that contains no cells, but in which the blood cells (erythrocytes, leukocytes, thrombocytes, etc.) are normally suspended, containing nutrients, sugars, proteins, minerals, enzymes, etc. Also preferably, said sample may be urine.

Preferably, the sample may be a placental biopsy, which can be taken during pregnancy using known techniques that are not or barely posing a risk for the pregnancy, or can in case of abortion or delivery be taken after the pregnancy is aborted or completed, e.g., for pathological or diagnostic purposes or for acquiring information regarding risk of occurrence of HDP such as PE in a future pregnancy of said subject.

A molecule or analyte such as a metabolite, nucleic acid, RNA, DNA or cDNA, protein, polypeptide or peptide, is "measured" in a sample when the presence or absence and/or quantity of said molecule or analyte or of said group of molecules or analytes is detected or determined in the sample, preferably substantially to the exclusion of other molecules and analytes. For example, a biomarker may be measured by measuring the mRNA encoding the same, or by measuring the encoded protein or polypeptide or a peptide thereof. For example, a metabolite (e.g., blood glucose) may be measured by standard laboratory tests. For example, a chemical element or compound (e.g., selenium) may be measured by standard laboratory tests (e.g., as taught in Rayman et al. 2003, Am J Obstet Gynecol 189: 1343).

A parameter is "scored" or "measured" for or in a patient when the presence or absence and/or quantity of said parameter is detected or determined for or in the subject. For example, a biophysical parameter (e.g., blood pressure) can be measured using standard tests and apparatus. For example, anamnesis parameters (e.g., maternal history parameters such as *fh_petxcardio, father_any_ihd,* and *fh_pet*) may be scored by reviewing relevant medical records or preferably by asking the respective question to a subject under examination and obtaining the answer as a "yes" or "no" (or potentially "unknown") statement.

The terms "quantity", "amount" and "level" are synonymous and generally well-understood in the art. With respect to molecules or analytes, the terms may particularly refer to an absolute quantification of the molecule or analyte in a sample, or to a relative quantification of the molecule or analyte in the sample, i.e., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g. weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second variables (e.g., first and second quantities) may but need not require to first determine the absolute values of said first and second variables. For example, a measurement method can produce quantifiable readouts (such as, *e.g.*, signal intensities) for said first and second variables, wherein said readouts are a function of the value of said variables, and wherein said readouts can be directly compared to produce a relative value for the first variable vs. the second variable, without the actual need to first convert the readouts to absolute values of the respective variables.

As used herein, the reference to any one biomarker, nucleic acid, peptide, polypeptide or protein corresponds to the biomarker, nucleic acid, peptide, polypeptide or protein commonly known under the respective designations in the art. The terms encompass such markers, nucleic acids, proteins and polypeptides of any organism where found, and particularly of animals, preferably warm-blooded animals, more preferably vertebrates, yet more preferably mammals, including humans and non-human mammals, still more preferably of humans. The terms particularly encompass such biomarkers, nucleic acids, proteins and polypeptides with a native sequence, i.e., ones of which the primary sequence is the same as that of the biomarkers, nucleic acids, proteins and polypeptides found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of biomarkers, nucleic acids, proteins and polypeptides are intended herein. Accordingly, all sequences of biomarkers, nucleic acids, proteins and polypeptides found in or derived from nature are considered "native". The terms encompass the biomarkers, nucleic acids, proteins and polypeptides when forming a part of a living organism, organ, tissue or cell, when forming a part of a biological sample, as well as when at least partly isolated from such sources. The terms also encompass the biomarkers, nucleic acids, proteins and polypeptides when produced by recombinant or synthetic means.

Exemplary human biomarkers, nucleic acids, proteins or polypeptides as taught herein may be as annotated under NCBI Genbank (http://www.ncbi.nlm.nih.gov/) accession numbers given in **Table 1** below. A skilled person can also appreciate that in some instances said sequences may be of precursors (e.g., preproteins) of the of biomarkers, nucleic acids, proteins or polypeptides as taught herein and may include parts which are processed away from the mature biomarkers, nucleic acids, proteins or polypeptides. A skilled person can further appreciate that although only one or more isoforms may be listed below, all isoforms are intended.

**Table 1**

| Swissprot name | Refseq Protein ID* | Refseq mRNA ID* | Description | HGNC.name |
|---|---|---|---|---|
| MMRN2_HUMAN | NP_079032.2 | NM_024756.2 | multimerin-2 | MMRN2 |
| ADA12_HUMAN | NP_003465.3 NP_067673.2. | NM_003474.4 NM_021641.3. | disintegrin and metalloproteinase domain-containing protein 12 | ADAM12 |
| ECM1_HUMAN | NPV_004416.2 NP_ 001189787.1. NP_073155.2 | NM_004425.3 NM_ 001202858.1 NM_022664.2. | extracellular matrix protein 1 | ECM1 |
| EGLN_HUMAN | NP_ 001108225.1 | NM_ 001114753.1 | endoglin isoform 1 | ENG |
| LCAT_HUMAN | NP_000220.1 | NM_000229.1 | phosphatidylcholine-sterol acyltransferase precursor | LCAT |
| LCAP_HUMAN | NP_005566.2, NP_787116.2 | NM_005575.2, NM_175920.3. | leucyl-cystinyl aminopeptidase | LNPEP |
| PRDX2_HUMAN | NP_005800.3 | NM_005809.4 | peroxiredoxin-2 | PRDX2 |
| CRP_HUMAN | NP_000558.2 | NM_000567.2 | C-reactive protein | CRP |
| MARE1_HUMAN | NP_036457.1 | NM_012325.2 | microtubule-associated protein RP/EB family member 1 | MAPRE1 |
| MARE3_HUMAN | NP_036458.2 | NM_012326.2 | microtubule-associated protein RP/EB family member 3 | MAPRE3 |
| PCP_HUMAN | NP_005031.1 NP_955450.2 | NM_005040.2 NM_199418.2 | lysosomal Pro-X carboxypeptidase | PRCP |
| C06A3_HUMAN | NP_004360.2 | NM_004369.3 | collagen alpha-3(VI) chain | COL6A3 |
| SPIT1_HUMAN | NP_857593.1 NP_ 001027539.1 NP_003701.1. | NM_181642.2 NM_ 001032367.1 NM_003710.3 | kunitz-type protease inhibitor 1 | SPINT1 |
| SEPP1_HUMAN | NP_005401.3, NP_ 001078955.1 | NM_005410.2, NM_ 001085486.1 | selenoprotein P | SEPP1 |
| QSOX1_HUMAN | NP_002817.2 NP_ 001004128.1 | NM_002826.4 NM_ 001004128.2 | sulfhydryl oxidase 1 | QSOX1 |
| ALS_HUMAN | NP_004961.1 NP_001139478 | NM_004970.2 NM_ 001146006.1 | insulin-like growth factor-binding protein complex acid labile subunit | IGFALS |
| ALDOA_HUMAN | NP_000025.1. NP_ 001121089.1. NP_ 001230106.1. NP_908930.1. NP_908932.1. NM_184043.2. | NM_000034.3 NM_ 001127617.2 NM_ 001243177.1 NM_184041.2 NM_184043.2, | fructose-bisphosphate aldolase A | ALDOA |
| MUC18_HUMAN | NP_006491.2 | NM_006500.2 | cell surface glycoprotein MUC18 | MCAM |

(continued)

| Swissprot name | Refseq Protein ID* | Refseq mRNA ID* | Description | HGNC.name |
|---|---|---|---|---|
| TFF3_HUMAN | NPV_003217.3 | NM_003226.3 | Trefoil factor 3 (intestinal) | TFF3 |
| ROBO4_HUMAN | NP_061928.4 | NM_019055.5 | roundabout homolog 4 | ROBO4 |
| ENPP2_HUMAN | NP_001035181.1 NP_001124335.1 NP 006200.3 | NM_001040092.2 NM_001130863.2 NM_006209.4 | ectonucleotide pyrophosphatase/ phosphodiesterase family member 2 | ENPP2 |
| PIGF_HUMAN | NP_001193941.1, NP_002623.2 | NM_001207012.1, NM_002632.5 | Placental Growth factor Isoform PIGF-3, Isoform PIGF-1 (PIGF-131), Isoform PIGF-2(PIGF-152) | PGF/PIGF |
| CYTC_HUMAN | NP_000090.1 | NM_000099.2 | Cystatin-C | CST3 |
| XPP2_HUMAN | NP_003390.4 | NM_003399.5 | Xaa-Pro aminopeptidase 2 | XPNPEP2 |
| PGBM_HUMAN | NP_005520.4 | NM_005529.5 | Basement membrane-specific heparan sulfate proteoglycan core protein; endorepellin; LG3 peptide | HSPG2 |
| TENX_HUMAN | NP_061978.6 NP_115859.2 | NM_019105.6 NM_032470.3 | Tenascin-X | TNXB |
| PCYOX_HUMAN | NP_057381.3 | NM_016297.3 | Prenylcysteine oxidase 1 | PCYOX1 |
| VGFR3_HUMAN | NP_002011.2 NP 891555.2 | NM_002020.4 NM 182925.4 | Vascular endothelial growth factor receptor 3; fms-related tyrosine kinase 4 | FLT4 |
| PRDX1_HUMAN | NP_001189360.1. NP 002565.1 NP 859047.1. | NM_001202431.1 NM 002574.3 NM 181696.2 | Peroxiredoxin-1 | PRDX1 |
| | NP 859048.1. | NM 181697.2. | | |
| IL6RB_HUMAN | NP_001177910.1 NP 002175.2 | NM_001190981.1 NM_002184.3 | Interleukin-6 receptor subunit beta | IL6ST |
| PROC_HUMAN | NP 000303.1 | NM_000312.3 | Vitamin K-dependent protein C | PROC |
| PCD12_HUMAN | NP 057664.1 | NM_016580.2 | Protocadherin-12 | PCDH12 |

*Genbank accession number for one or more representative amino acid and mRNA sequences (e.g., isoforms). The number following the period denotes the respective the Genbank sequence version.

Exemplary placental growth factor (PIGF) includes, without limitation, human PIGF having primary amino acid sequence as annotated under NCBI Genbank accession number NP_002623 (sequence version 2). Exemplary PIGF includes all isoforms of placental growth factor such as the human placenta growth factor isoform 1 precursor having primary amino acid sequence as annotated under NCBI Genbank accession number NP_002623 (sequence version 2) or UniProt accession number P49763-2, the human placenta growth factor isoform 2 precursor having primary amino acid sequence as annotated under NCBI Genbank accession number NP_001 13941 (sequence version 1) or UniProt accession number P49763-3, or the human placenta growth factor isoform 3 precursor having UniProt accession number P49763-1. The protein sequences of the three isoforms of human placenta growth factor are shown in Figure 1.

The reference herein to any biomarker, nucleic acid, protein or polypeptide may also encompass fragments thereof. Hence, the reference herein to measuring (or measuring the quantity of) any one biomarker, nucleic acid, protein or polypeptide may encompass measuring the biomarker, nucleic acid, protein or polypeptide, such as, e.g., measuring the mature and/or the processed soluble/secreted form (e.g. plasma circulating form) thereof and/or measuring one or more fragments thereof.

For example, any biomarker, nucleic acid, protein or polypeptide and/or one or more fragments thereof may be measured collectively, such that the measured quantity corresponds to the sum amounts of the collectively measured species. In another example, any biomarker, nucleic acid, protein or polypeptide and/or one or more fragments thereof may be measured each individually. Preferably, said fragment may be a plasma circulating (i.e., not cell- or membrane-bound) form. Without being bound by any theory, such circulating forms can be derived from full-length biomarkers, nucleic acids, proteins or polypeptides through natural processing, or can be resulting from known degradation processes occurring in a sample. In certain situations, the circulating form can also be the full-length biomarker, nucleic acid, protein or polypeptide, which is found to be circulating in the plasma. Said "circulating form" can thus be any biomarker, nucleic acid, protein or polypeptide or any processed soluble form thereof or fragments of either one, that is circulating in the sample, i.e. which is not bound to a cell- or membrane fraction of said sample.

Unless otherwise apparent from the context, reference herein to any biomarker, nucleic acid, protein or polypeptide and fragments thereof may generally also encompass modified forms of said biomarker, nucleic acid, protein or polypeptide and fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

Preferably, any biomarker, nucleic acid, protein or polypeptide and fragments thereof may be human, i.e., their primary sequence may be the same as a corresponding primary sequence of or present in a naturally occurring human biomarker, nucleic acid, protein or polypeptide. Hence, the qualifier "human" in this connection relates to the primary sequence of the respective biomarker, nucleic acid, protein or polypeptide, rather than to its origin or source. For example, such biomarker, nucleic acid, protein or polypeptide and fragments may be present in or isolated from samples of human subjects or may be obtained by other means (e.g., by recombinant expression, cell-free translation or non-biological peptide synthesis).

The term "fragment" of a protein, polypeptide or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said peptide, polypeptide or protein, such as, for example, in vivo or in vitro, such as, for example, by physical, chemical and/or enzymatic proteolysis. Without limitation, a fragment of a protein, polypeptide or peptide may represent at least about 5%, or at least about 10%, e.g., $\geq$ 20%, $\geq$ 30% or $\geq$ 40%, such as $\geq$ 50%, e.g. $\geq$ 60%, $\geq$ 70% or $\geq$ 80%, or even $\geq$ 90% or $\geq$ 95% of the amino acid sequence of said protein, polypeptide or peptide.

For example, a fragment may include a sequence of > 5 consecutive amino acids, or > 10 consecutive amino acids, or > 20 consecutive amino acids, or > 30 consecutive amino acids, e.g., >40 consecutive amino acids, such as for example > 50 consecutive amino acids, e.g., $\geq$ 60, > 70, > 80, > 90, > 100, > 200, > 300, > 400, > 500 or > 600 consecutive amino acids of the corresponding full length protein.

Preferably, a fragment may be N-terminally and/or C-terminally truncated by between 1 and about 20 amino acids, such as, e.g., by between 1 and about 15 amino acids, or by between 1 and about 10 amino acids, or by between 1 and about 5 amino acids, compared to the corresponding mature, full-length protein or its soluble or plasma circulating form.

Preferably, fragments of a given protein, polypeptide or peptide may be achieved by in vitro proteolysis of said protein, polypeptide or peptide to obtain advantageously detectable peptide(s) from a sample. For example, such proteolysis may be effected by suitable physical, chemical and/or enzymatic agents, e.g., proteinases, preferably endoproteinases, *i.e.*, protease cleaving internally within a protein, polypeptide or peptide chain. A non-limiting list of suitable endoproteinases includes serine proteinases (EC 3.4.21), threonine proteinases (EC 3.4.25), cysteine proteinases (EC 3.4.22), aspartic acid proteinases (EC 3.4.23), metalloproteinases (EC 3.4.24) and glutamic acid proteinases. Exemplary non-limiting endoproteinases include trypsin, chymotrypsin, elastase, *Lysobacter enzymogenes* endoproteinase Lys-C, *Staphylococcus aureus* endoproteinase Glu-C (endopeptidase V8) or *Clostridium histolyticum* endoproteinase Arg-C (clostripain). Further known or yet to be identified enzymes may be used; a skilled person can choose suitable protease(s) on the basis of their cleavage specificity and frequency to achieve desired peptide forms. Preferably, the proteolysis may be effected by endopeptidases of the trypsin type (EC 3.4.21.4), preferably trypsin, such as, without limitation, preparations of trypsin from bovine pancreas, human pancreas, porcine pancreas, recombinant trypsin, Lys-acetylated trypsin, trypsin in solution, trypsin immobilised to a solid support, *etc.* Trypsin is particularly useful, *inter alia* due to high specificity and efficiency of cleavage. It is further herein disclosed the use of any trypsin-like protease, *i.e.*, with a similar specificity to that of trypsin. Otherwise, chemical reagents may be used for proteolysis. For example, CNBr can cleave at Met; BNPS-skatole can cleave at Trp. The conditions for treatment, e.g., protein concentration, enzyme or chemical reagent concentration, pH, buffer, temperature, time, can be determined by the skilled person depending on the enzyme or chemical reagent employed.

The term "isolated" with reference to a particular component (such as for instance, nucleic acid, protein, polypeptide, peptide or fragment thereof) generally denotes that such component exists in separation from - for example, has been separated from or prepared in separation from - one or more other components of its natural environment. For instance, an isolated human or animal nucleic acid, protein, polypeptide, peptide or fragment exists in separation from a human or animal body where it occurs naturally.

The term "isolated" as used herein may preferably also encompass the qualifier "purified". As used herein, the term "purified" with reference to nucleic acid(s), protein(s), polypeptide(s), peptide(s) and/or fragment(s) thereof does not require absolute purity. Instead, it denotes that such nucleic acid(s), protein(s), polypeptide(s), peptide(s) and/or fragment(s) is (are) in a discrete environment in which their abundance (conveniently expressed in terms of mass or weight or concentration) relative to other proteins is greater than in a biological sample. A discrete environment denotes a single medium, such as for example a single solution, gel, precipitate, lyophilisate, etc. Purified nucleic acids, peptides, polypeptides or fragments may be obtained by known methods including, for example, laboratory or recombinant synthesis, chromatography, preparative electrophoresis, centrifugation, precipitation, affinity purification, etc.

Purified protein(s), polypeptide(s), peptide(s) and/or fragment(s) may preferably constitute by weight > 10%, more preferably $\geq$ 50%, such as $\geq$ 60%, yet more preferably $\geq$ 70%, such as $\geq$ 80%, and still more preferably $\geq$ 90%, such as $\geq$ 95%, $\geq$ 96%, $\geq$ 97%, $\geq$ 98%, $\geq$ 99% or even 100%, of the protein content of the discrete environment. Protein content may be determined, e.g., by the Lowry method (Lowry et al. 1951. J Biol Chem 193: 265), optionally as described by Hartree 1972 (Anal Biochem 48: 422-427). Also, purity of peptides or polypeptides may be determined by SDS-PAGE under reducing or nonreducing conditions using Coomassie blue or, preferably, silver stain.

Preferably, reagents disclosed herein may comprise a detectable label. The term "label" refers to any atom, molecule, moiety or biomolecule that can be used to provide a detectable and preferably quantifiable read-out or property, and that can be attached to or made part of an entity of interest, such as a peptide or polypeptide or a specific-binding agent. Labels may be suitably detectable by mass spectrometric, spectroscopic, optical, colourimetric, magnetic, photochemical, biochemical, immunochemical or chemical means. Labels include without limitation dyes; radiolabels such as $^{32}P$, $^{33}P$, $^{35}S$, $^{125}I$, $^{131}I$; electron-dense reagents; enzymes (e.g. , horse-radish phosphatise or alkaline phosphatise as commonly used in immunoassays); binding moieties such as biotin-streptavidin; haptens such as digoxigenin; luminogenic, phosphorescent or fluorogenic moieties; mass tags; and fluorescent dyes alone or in combination with moieties that can suppress or shift emission spectra by fluorescence resonance energy transfer (FRET).

For example, the label may be a mass-altering label. Preferably, a mass-altering label may involve the presence of a distinct stable isotope in one or more amino acids of the peptide vis-a-vis its corresponding non-labelled peptide. Mass-labelled peptides are particularly useful as positive controls, standards and calibrators in mass spectrometry applications. In particular, peptides including one or more distinct isotopes are chemically alike, separate chromatographically and electrophoretically in the same manner and also ionise and fragment in the same way. However, in a suitable mass analyser such peptides and optionally select fragmentation ions thereof will display distinguishable m/z ratios and can thus be discriminated. Examples of pairs of distinguishable stable isotopes include H and D, $^{12}C$ and $^{13}C$, $^{14}N$ and $^{15}N$ or $^{16}O$ and $^{18}O$. Usually, peptides and proteins of biological samples analysed in the present invention may substantially only contain common isotopes having high prevalence in nature, such as for example H, $^{12}C$, $^{14}N$ and $^{16}O$. In such case, the mass-labelled peptide may be labelled with one or more uncommon isotopes having low prevalence in nature, such as for instance D, $^{13}C$, $^{15}N$ and/or $^{18}O$. It is also conceivable that in cases where the peptides or proteins of a biological sample would include one or more uncommon isotopes, the mass-labelled peptide may comprise the respective common isotope(s).

Isotopically-labelled synthetic peptides may be obtained inter alia by synthesising or recombinantly producing such peptides using one or more isotopically-labelled amino acid substrates, or by chemically or enzymatically modifying unlabelled peptides to introduce thereto one or more distinct isotopes. By means of example and not limitation, D-labelled peptides may be synthesised or recombinantly produced in the presence of commercially available deuterated L-methionine $CH_3$-S-$CD_2CD_2$-CH(NH$_2$)-COOH or deuterated arginine H$_2$NC(=NH)-NH-(CD$_2$)$_3$-CD(NH$_2$)-COOH. It shall be appreciated that any amino acid of which deuterated or $^{15}N$- or $^{13}C$-containing forms exist may be considered for synthesis or recombinant production of labelled peptides. In another non-limiting example, a peptide may be treated with trypsin in H$_2$$^{16}O$ or H$_2$$^{18}O$, leading to incorporation of two oxygens ($^{16}O$ or $^{18}O$, respectively) at the COOH-termini of said peptide (e.g., US 2006/105415).

Also contemplated is the use of biomarkers, peptides, polypeptides or proteins and fragments thereof as taught herein, optionally comprising a detectable label, as (positive) controls, standards or calibrators in qualitative or quantitative detection assays (measurement methods) of said biomarkers, peptides, polypeptides or proteins and fragments thereof, and particularly in such methods for the diagnosis, prediction, prognosis and/or monitoring the diseases or conditions as taught herein in subjects. The biomarkers, proteins, polypeptides or peptides may be supplied in any form, inter alia as precipitate, vacuum-dried, lyophilisate, in solution as liquid or frozen, or covalently or non-covalently immobilised on solid phase, such as for example, on solid chromatographic matrix or on glass or plastic or other suitable surfaces (e.g., as a part of peptide arrays and microarrays). The peptides may be readily prepared, for example, isolated from natural sources, or prepared recombinantly or synthetically.

Further disclosed are binding agents capable of specifically binding to biomarkers, peptides, polypeptides or proteins and fragments thereof as taught herein. Binding agents as intended throughout this specification may include inter alia an antibody, aptamer, photoaptamer, protein, peptide, peptidomimetic or a small molecule.

The term "specifically bind" as used throughout this specification means that an agent (denoted herein also as "specific-binding agent") binds to one or more desired molecules or analytes substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to target(s) of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

Preferably, the agent may bind to its intended target(s) with affinity constant ($K_A$) of such binding $K_A \geq 1\times10^6$ $M^{-1}$, more preferably $K_A \geq 1\times10^7$ $M^{-1}$, yet more preferably $K_A \geq 1\times10^8$ $M^{-1}$, even more preferably $K_A \geq 1\times10^9$ $M^{-1}$, and still more preferably $K_A \geq 1\times10^{10}$ $M^{-1}$ or $K_A \geq 1\times10^{11}$ $M^{-1}$, wherein $K_A$ = [SBA_T]/[SBA][T], SBA denotes the specific-binding agent, T denotes the intended target. Determination of $K_A$ can be carried out by methods known in the art, such as for example, using equilibrium dialysis and Scatchard plot analysis.

As used herein, the term "antibody" is used in its broadest sense and generally refers to any immunologic binding agent. The term specifically encompasses intact monoclonal antibodies, polyclonal antibodies, multivalent (e.g., 2-, 3- or more-valent) and/or multi-specific antibodies (*e.g.*, bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced in vitro or in vivo.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581 -597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), e.g., dibodies, tribodies, and multibodies. The above designations Fab, Fab', F(ab')2, Fv, scFv etc. are intended to have their art-established meaning.

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation , the antibodies may be human, murine (e.g., mouse, rat, etc.), donkey, rabbit, goat, sheep, guinea pig, camel (e.g., *Camelus bactrianus* and *Camelus dromaderius*), llama (e.g., *Lama paccos, Lama glama* or *Lama vicugna*) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art,as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA RNA or any analogue thereof, that can specifically bind to a target molecule such as a peptide. Advantageously, aptamers can display fairly high specificity and affinity (e.g., KA in the order 1x10<9> M<"1>) for their targets. Aptamer production is described inter alia in US 5,270,163; Ellington & Szostak 1990 (Nature 346:818-822); Tuerk & Gold 1990 (Science 249:

505-510); or "The Aptamer Handbook: Functional Oligonucleotides and Their Applications", by Klussmann, ed., Wiley-VCH 2006, ISBN 3527310592. The term "photoaptamer" refers to an aptamer that contains one or more photoreactive functional groups that can covalently bind to or crosslink with a target molecule. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33 , and of two peptidomimetics based on the 11 -mer peptide Substance P, and related peptidomimetic design principles, are described in Horwell 1995 (Trends Biotechnol 13: 132-134).

The term "small molecule" refers to compounds, preferably organic compounds, with a size comparable to those organic molecules generally used in pharmaceuticals. The term excludes biological macromolecules (e.g., proteins, nucleic acids, etc.). Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da.

Hence, also disclosed are methods for immunising animals, e.g., non-human animals such as laboratory or farm, animals using (i.e., using as the immunising antigen) any one or more (isolated) markers, peptides, polypeptides or proteins and fragments thereof as taught herein, optionally attached to a presenting carrier. Immunisation and preparation of antibody reagents from immune sera is well-known per se and described in documents referred to elsewhere in this specification. The animals to be immunised may include any animal species, preferably warm-blooded species, more preferably vertebrate species, including, e.g., birds, fish, and mammals. Without limitation, the antibodies may be chicken, turkey, goose, duck, guinea fowl, shark, quail or pheasant. Also without limitation, the antibodies may be human, murine (e.g., mouse, rat, etc.), donkey, rabbit, goat, sheep, guinea pig, shark, camel, llama or horse. The term "presenting carrier" or "carrier" generally denotes an immunogenic molecule which, when bound to a second molecule, augments immune responses to the latter, usually through the provision of additional T cell epitopes. The presenting carrier may be a (poly)peptidic structure or a non-peptidic structure, such as inter alia glycans, polyethylene glycols, peptide mimetics, synthetic polymers, etc. Exemplary non-limiting carriers include human Hepatitis B virus core protein, multiple C3d domains, tetanus toxin fragment C or yeast Ty particles.

Immune sera obtained or obtainable by immunisation as taught herein may be particularly useful for generating antibody reagents that specifically bind to any one or more biomarkers, peptides, polypeptides or proteins and fragments thereof disclosed herein.

The binding molecule may labelled with a tag that permits detection with another agent (e.g. with a probe binding partner). Such tags can be, for example, biotin, streptavidin, histag, myc tag, maltose, maltose binding protein or any other kind of tag known in the art that has a binding partner. Example of associations which can be utilised in the probe:binding partner arrangement may be any, and includes, for example biotin:streptavidin, his-tag:metal ion (e.g. $Ni^{2+}$), maltose:maltose binding protein.

The binding molecule conjugate may be associated with or attached to a detection agent to facilitate detection. Examples of lab detection agents include, but are not limited to, luminescent labels; colourimetric labels, such as dyes; fluorescent labels; or chemical labels, such as electroactive agents (e.g., ferrocyanide); enzymes; radioactive labels; or radiofrequency labels. More commonly, the detection agent is a particle. Examples of particles useful in the practice of the invention include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles are colloidal gold particles. Colloidal gold may be made by any conventional means, such as the methods outlined in G. Frens, 1973 Nature Physical Science, 241:20 (1973). Alternative methods may be described in U.S. Pat. Nos. 5,578,577, 5,141,850; 4,775,636; 4,853,335; 4,859,612; 5,079,172; 5,202,267; 5,514,602; 5,616,467; 5,681,775.

Any existing, available or conventional separation, detection and quantification methods can be used herein to measure the presence or absence (e.g., readout being present vs. absent; or detectable amount vs. undetectable amount) and/or quantity (e.g., readout being an absolute or relative quantity, such as, for example, absolute or relative concentration) of biomarkers, peptides, polypeptides, proteins and/or fragments thereof in samples (any molecules or analytes of interest to be so-measured in samples, including any one or more biomarkers, peptides, polypeptides, proteins and fragments thereof as taught herein, may be herein below referred to collectively as biomarkers).

For example, such methods may include biochemical assay methods, immunoassay methods, mass spectrometry analysis methods, or chromatography methods, or combinations thereof.

The term " immunoassay " generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1st ed., Humana Press 2000, ISBN 0896037282.

By means of further explanation and not limitation, direct ELISA employs a labelled primary antibody to bind to and thereby quantify target antigen in a sample immobilised on a solid support such as a microwell plate. Indirect ELISA uses a non-labelled primary antibody which binds to the target antigen and a secondary labelled antibody that recognises and allows to quantify the antigen-bound primary antibody. In sandwich ELISA the target antigen is captured from a sample using an immobilised 'capture' antibody which binds to one antigenic site within the antigen, and subsequent to removal of non-bound analytes the so-captured antigen is detected using a 'detection' antibody which binds to another antigenic site within said antigen, where the detection antibody may be directly labelled or indirectly detectable as above. Competitive ELISA uses a labelled 'competitor' that may either be the primary antibody or the target antigen. In an example, non-labelled immobilised primary antibody is incubated with a sample, this reaction is allowed to reach equilibrium, and then labelled target antigen is added. The latter will bind to the primary antibody wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colourimetric, chemiluminescent or fluorescent, magnetic, piezo electric, pyroelectric and other.

Radioimmunoassay (RIA) is a competition-based technique and involves mixing known quantities of radioactively-labelled (e.g., <125>I- or <131>I-labelled) target antigen with antibody to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821 198 for guidance).

Generally, any mass spectrometric (MS) techniques that can obtain precise information on the mass of peptides, and preferably also on fragmentation and/or (partial) amino acid sequence of selected peptides (e.g., in tandem mass spectrometry, MS/MS; or in post source decay, TOF MS), are useful herein. Suitable peptide MS and MS/MS techniques and systems are well-known per se (see, e.g., Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)$^n$ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)$^n$; atmospheric pressure photoionization mass spectrometry (APPI-MS); APPI-MS/MS; and APPI- (MS)$^n$. Peptide ion fragmentation in tandem MS (MS/MS) arrangements may be achieved using manners established in the art, such as, *e.g.*, collision induced dissociation (CID). Detection and quantification of biomarkers by mass spectrometry may involve multiple reaction monitoring (MRM), such as described among others by Kuhn et al. 2004 (Proteomics 4: 1 175-86). MS peptide analysis methods may be advantageously combined with upstream peptide or protein separation or fractionation methods, such as for example with the chromatographic and other methods described herein below.

Chromatography can also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, *etc.*

Chromatography as used herein may be preferably columnar (i.e., wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like.

Chromatography, including single-, two- or more-dimensional chromatography, may be used as a peptide fractionation method in conjunction with a further peptide analysis method, such as for example, with a downstream mass spectrometry analysis as described elsewhere in this specification.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The level of biomarkers at the RNA level may be established using RNA analysis of placental tissue obtained e.g. using transcervical placental biopsy during early pregnancy or similar methods not endangering the pregnancy. This test involves the removal of a small amount of placental tissue between the tenth and twelfth week of pregnancy. Under ultrasound guidance via the vagina, a narrow tube is inserted into the placenta and a small biopsy is taken. Alternatively, the placental biopsy can be obtained from subjects with natural abortion of the pregnancy in order to establish the cause of said premature abortion. This information is an important predictive tool in view of future pregnancies.

The RNA level can be detected using standard quantitative RNA measurement tools known in the art. Non-limiting examples include hybridization-based analysis, microarray expression analysis, digital gene expression (DGE), RNA-in-situ hybridization (RISH), Northern-blot analysis and the like; PCR, RT-PCR, RT-qPCR, end-point PCR, digital PCR or the like; supported oligonucleotide detection, pyrosequencing, polony cyclic sequencing by synthesis, simultaneous bi-directional sequencing, single-molecule sequencing, single molecule real time sequencing, true single molecule sequencing, hybridization-assisted nanopore sequencing and sequencing by synthesis.

Biomarker presence can also be detected on placental biopsies obtained as indicated above using standard immunohistochemistry techniques, wherein the presence, absence, or quantity of biomarker proteins is detected directly in the placental tissue. The bioptic tissue can be fixed following routine procedures well known in the art.

The various aspects taught herein may further rely on comparing the quantity of biomarkers measured in samples and the measurement or score of parameters in patients with reference values, wherein said reference values represent known predictions, diagnoses and/or prognoses of diseases or conditions as taught herein.

For example, distinct reference values may represent the prediction of a risk (e.g., an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values can represent the diagnosis of a given disease or condition as taught herein vs. the diagnosis of no such disease or condition (such as, e.g., the diagnosis of healthy, or recovered from said disease or condition, eic). In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such difference between values being compared. A comparison may include a visual inspection, an arithmetical or statistical comparison of measurements. Such statistical comparisons include, but are not limited to, applying a rule.

Reference values may be established according to known procedures previously employed for other biomarkers and parameters. For example, a reference value may be established in an individual or a population of individuals characterised by a particular diagnosis, prediction and/or prognosis of said disease or condition (i.e., for whom said diagnosis, prediction and/or prognosis of the disease or condition holds true). Such population may comprise without limitation $\geq 2$, $\geq 10$, $\geq 100$, or even several hundreds or more individuals.

A "deviation" of a first value from a second value may generally encompass any direction (e.g., increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1 -fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., $\pm 1$ xSD or $\pm 2$xSD, or $\pm 1$ xSE or $\pm 2$xSE). Deviation may also refer to a value falling outside of a reference range defined by values in a given population (for example, outside of a range which comprises $\geq 40\%$, $\geq 50\%$, $\geq 60\%$, $\geq 70\%$, $\geq 75\%$ or $\geq 80\%$ or $\geq 85\%$ or $\geq 90\%$ or $\geq 95\%$ or even $\geq 100\%$ of values in said population).

Preferably, a deviation may be concluded if an observed alteration is beyond a given threshold or cut-off. Such threshold or cut-off may be selected as generally known in the art to provide for a chosen sensitivity and/or specificity of the diagnosis, prediction and/or prognosis methods, e.g., sensitivity and/or specificity of at least 50%, or at least 60%, or at least 70%, or at least 80%, or at least 85%, or at least 90%, or at least 95%.

It is herein disclosed kits or devices as set forth above for the diagnosis, prediction, prognosis and/or monitoring of any one disease or condition as taught herein comprising means for detecting the level of biomarker(s) comprised in test panels as taught herein in a sample of the patient. In a preferred embodiment, such a kit or kits can be used in clinical settings or at home. The kit can be used for diagnosing said disease or condition, for monitoring the effectiveness of treatment of a subject suffering from said disease or condition with an agent, or for preventive screening of subjects for the occurrence of said disease or condition in said subject.

In a clinical setting, the kit or device can be in the form of a bed-side device or in an emergency team setting, e.g. as part of the equipment of an ambulance or other moving emergency vehicle or team equipment or as part of a first-aid kit. The diagnostic kit or device can assist a medical practitioner, a first aid helper, or nurse to decide whether the patient under observation is developing a disease or condition as taught herein, after which appropriate action or treatment can be performed.

A home-test kit gives the patient a readout which she can communicate to a medicinal practitioner, a first aid helper or to the emergency department of a hospital, after which appropriate action can be taken. Such a home-test device is of particular interest for people having either a history of, or are at risk of suffering from any one disease or condition as taught herein.

Non-limiting examples are: systems comprising specific binding molecules for the requisite biomarker(s) attached to a solid phase, e.g. lateral flow strips or dipstick devices and the like well known in the art. One non-limiting example to perform a biochemical assay is to use a test-strip and labelled antibodies which combination does not require any washing of the membrane. The test strip is well known, for example, in the field of pregnancy testing kits where an anti-hCG antibody is present on the support, and is carried complexed with hCG by the flow of urine onto an immobilised second antibody that permits visualisation. Other non-limiting examples of such home test devices, systems or kits can be found for example in the following U.S. patents: 6,107,045, 6,974,706, 5,108,889, 6,027,944, 6,482,156, 6,511,814, 5,824,268, 5,726,010, 6,001,658 or U.S. patent applications: 2008/0090305 or 2003/0109067. It is herein further disclosed a lateral flow device or dipstick. Such dipstick comprises a test strip allowing migration of a sample by capillary flow from one end of the strip where the sample is applied to the other end of such strip where presence of an analyte in said sample is measured. It is herein further disclosed a device comprising a reagent strip. Such reagent strip comprises one or more test pads which when wetted with the sample, provide a colour change in the presence of an analyte and/or indicate the concentration of the protein in said sample.

In order to obtain a semi-quantitative test strip in which only a signal is formed once the level of the requisite biomarker(s) in the sample is higher than a certain predetermined threshold level or value, a predetermined amount of fixed capture antibodies for the biomarker(s) can be present on the test strip. This enables the capture of a certain amount of the biomarker(s) present in the sample, corresponding to the threshold level or value as predetermined. The remaining amount of biomarker(s) (if any) bound by e.g. a conjugated or labelled binding molecules can then be allowed to migrate to a detection zone which subsequently only produces a signal if the level of the biomarker(s) in the sample is higher than the predetermined threshold level or value.

Another possibility to determine whether the amount of any the requisite biomarker(s) in the sample is below or above a certain threshold level or value, is to use a primary capturing antibody capturing all said biomarker(s) present in the sample, in combination with a labelled secondary antibody, developing a certain signal or colour when bound to the solid phase. The intensity of the colour or signal can then either be compared to a reference colour or signal chart indicating that when the intensity of the signal is above a certain threshold signal, the test is positive. Alternatively, the amount or intensity of the colour or signal can be measured with an electronic device comprising e.g. a light absorbance sensor or light emission meter, resulting in a numerical value of signal intensity or colour absorbance formed, which can then be displayed to the subject in the form of a negative result if said numerical value is below the threshold value or a positive result if said numerical value is above the threshold value. It is of particular relevance in monitoring the level of said biomarker(s) in a patient over a period of time.

The reference value or range can e.g. be determined using the home device in a period wherein the subject is free of a given disease or condition, giving the patient an indication of her base-line level of the biomarker(s). Regularly using the home test device will thus enable the subject to notice a sudden change in levels of said biomarker(s) as compared to the base-line level, which can enable her to contact a medical practitioner.

Alternatively, the reference value can be determined in the subject suffering from a given disease or condition as taught herein, which then indicates her personal "risk level" for the biomarker(s), i.e. the level of the biomarker(s) which indicates she is or will soon be exposed to said disease or condition. This risk level is interesting for monitoring the disease progression or for evaluating the effect of the treatment.

Furthermore, the reference value or level can be established through combined measurement results in subjects with highly similar disease states or phenotypes (e.g. all having no disease or condition as taught herein or having said disease or condition).

Non-limiting examples of semi-quantitative tests known in the art, the principle of which could be used for the home test device according to the present invention are the HIV/AIDS test or Prostate Cancer tests sold by Sanitoets. The home prostate test is a rapid test intended as an initial semi-quantitative test to detect PSA blood levels higher than 4 ng/ml in whole blood. The typical home self-test kit comprises the following components: a test device to which the blood sample is to be administered and which results in a signal when the protein level is above a certain threshold level, an amount of diluent e.g. in dropper pipette to help the transfer of the analytes (i.e. the protein of interest) from the sample application zone to the signal detection zone, optionally an empty pipette for blood specimen collection, a finger pricking device, optionally a sterile swab to clean the area of pricking and instructions of use of the kit.

Similar tests are also known for e.g. breast cancer detection and CRP-protein level detection in view of cardiac risk home tests. The latter test encompasses the sending of the test result to a laboratory, where the result is interpreted by a technical or medical expert. Such telephone or internet based diagnosis of the patient's condition is of course possible and advisable with most of the kits, since interpretation of the test result is often more important than conducting the test. When using an electronic device as mentioned above which gives a numerical value of the level of protein present in the sample, this value can of course easily be communicated through telephone, mobile telephone, satellite phone, E-mail, internet or other communication means, warning a hospital, a medicinal practitioner or a first aid team that a person is, or may be at risk of, suffering from the disease or condition as taught herein. A non-limiting example of such a system is disclosed in U.S. patent 6,482,156.

The presence and/or concentration of biomarker(s) in a sample can be measured by surface plasmon resonance (SPR) using a chip having binding molecule for said biomarker(s) immobilized thereon, fluorescence resonance energy transfer (FRET), bioluminescence resonance energy transfer (BRET), fluorescence quenching, fluorescence polarization measurement or other means known in the art. Any of the binding assays described can be used to determine the presence and/or concentration of any biomarker(s) in a sample. To do so, binding molecules for the biomarker(s) are reacted with a sample, and the concentration of the biomarker(s) is measured as appropriate for the binding assay being used. To validate and calibrate an assay, control reactions using different concentrations of standard biomarker(s) and/or binding molecule therefore can be performed. Where solid phase assays are employed, after incubation, a washing step is performed to remove unbound markers. Bound biomarker is measured as appropriate for the given label (e.g., scintillation counting, fluorescence, antibody-dye etc.). If a qualitative result is desired, controls and different concentrations may not be necessary. Of course, the roles of said biomarker(s) and binding molecule may be switched; the skilled person may adapt the method so binding molecule is applied to sample, at various concentrations of sample.

The above aspects and embodiments are further supported by the following non-limiting examples.

## EXAMPLES

### Example 1 : Patient and control cohorts

Prospective clinical samples were collected from pregnant women with a singleton pregnancy at 15+/-1 and 20 +/-1 weeks' gestation and which were either diagnosed with pre-eclampsia (cases) or not diagnosed with pre-eclampsia (controls) in the further course of their pregnancy. All samples were obtained from participants in the SCOPE (Screening fOr Pregnancy Endpoints) prospective screening study of nulliparous women.

Written consent was obtained from each participant. The inclusion criteria applied for the study were nulliparity, singleton pregnancy, gestation age between 14 weeks 0 days and 16 weeks 6 days gestation and informed consent to participate. The exclusion criteria applied were: Unsure of last menstrual period (LMP) and unwilling to have ultrasound scan at <= 20 weeks, >=3 miscarriages, >=3 terminations, major fetal anomaly/abnormal karyotype, essential hypertension treated pre-pregnancy, moderate-severe hypertension at booking >=160/100 mmHg, diabetes, renal disease, systemic lupus erythematosus, anti-phospholipid syndrome, sickle cell disease, HIV positive, major uterine anomaly, cervical suture, knife cone biopsy, ruptured membranes now, long term steroids, treatment low-dose aspirin, treatment calcium (>1 g/24h), treatment eicosopentanoic acid (fish oil), treatment vitamin C >=1000mg & Vit E >=400iu, treatment heparin/low molecular weight heparin.

Preeclampsia defined as gestational hypertension (systolic blood pressure (BP) >= 140 mmHg and/or diastolic BP >= 90mmHg (Korotkoff V) on at least 2 occasions 4 hours apart after 20 weeks gestation but before the onset of labour) or postpartum systolic BP >= 140 mmHg and/or diastolic BP >= 90mmHg postpartum on at least 2 occasions 4 hours apart with proteinuria >= 300 mg/24h or spot urine protein: creatinine ratio >=30 mg/mmol creatinine or urine dipstick protein >= 2 or any multi-system complication of preeclampsia. Multisystem complications include any of the following: 1 . Acute renal insufficiency defined as a new increase in serum creatinine >=100 umol/L antepartum or >130 umol/L postpartum 2. Liver disease defined as raised aspartate transaminase and/or alanine transaminase >45 IU/L and/or severe right upper quadrant or epigastric pain or liver rupture 3. Neurological problems defined as eclampsia or imminent eclampsia (severe headache with hyperreflexia and persistent visual disturbance) or cerebral haemorrhage 4. Haematological including thrombocytopenia (platelets <100 x 10<9>/L), disseminated intravascular coagulation or haemolysis, diagnosed by features on blood film (e.g., fragmented cells, helmet cells) and reduced haptoglobin. Preeclampsia could be diagnosed at any stage during pregnancy after recruitment until delivery or in the first 2 weeks after delivery.

Spontaneous preterm birth is defined as spontaneous preterm labour or preterm premature rupture of the membranes (PPROM) resulting in preterm birth at <37.0 weeks. Preterm preeclampsia is defined as preeclampsia resulting in delivery at <37.0 weeks.

Early onset preeclampsia is defined as preeclampsia resulting in delivery at <34.0 weeks.

Small for Gestational Age is defined as a birthweight <10th% using customized centiles, adjusted for maternal weight, height, parity, ethnicity and infant sex. The weight is determined within 24 hours after the baby's birth.

Clinical data on known risk factors for pre-eclampsia (Zhong et al, Prenatal Diagnosis, 30, p. 293-308, 2010; Sibai et al, 365, p. 785-799, 2005) was collected at 15+/-1 and 20 +/-1 weeks' gestation by interview and examination of the women. Ultrasound data were obtained at 20 weeks on fetal measurements, anatomy, uterine and umbilical artery Doppler and cervical length. Fetal growth, uterine and umbilical Dopplers are measured at 24 weeks. Pregnancy outcome was tracked and the woman seen within 48 hours of delivery. Baby measurements are obtained within 48 hours of delivery.

In Table 2A an overview of baseline characteristics of cohorts from New-Zealand and Australia (Australasian cohort) (100 cases and 200 control), and from UK and Ireland (European cohort) (50 cases and 250 controls) are given together with some clinical parameters as obtained at the 15 and 20 weeks interviews and examinations. Blood pressure measurements were performed twice. The mean arterial pressure is calculated as follows: (1/3 <*> systolic blood pressure + 2/3 <*> diastolic blood pressure). In Table 2B the baseline characteristics of the combined cohort, constituting both the Australasian and the European cohort (150 cases and 450 controls), are given together with some clinical parameters as obtained at the 15 and 20 weeks interviews and examinations.

**Table 2A.** Material characteristics including information about family history of disease, clinical parameters obtained during visits at 15 weeks and 20 weeks of gestation and some maternal and fetal characteristics as collected at pregnancy outcome. Results are N, number of patients, or mean (Standard deviation).

| Parameter | Code | Test (New Zealand + Australia) | | Test (UK+Ireland) | |
|---|---|---|---|---|---|
| | | Controls (200) | Cases (100) | Controls (250) | Cases (50) |
| Age mother | | 26.83 (6.36) | 26.56 (5.98) | 28.90 (5.29) | 29.70 (5.53) |

(continued)

| Parameter | Code | Test (New Zealand + Australia) | | Test (UK+Ireland) | |
|-----------|------|------------------------------|--|-------------------|--|
| | | Controls (200) | Cases (100) | Controls (250) | Cases (50) |
| Ethnicity | | Asian = 6 Caucasian = 179 Indian = 5 Maori = 4 Pacific Islander = 2 Other (including African) = 4 | Asian = 7 Caucasian = 84 Indian = 3 Maori = 1 Pacific Islander = 3 Other (including African) = 2 | Asian = 2 Caucasian =224 Indian = 9 Maori = 0 Pacific Islander = 0 Other (including African) = 15 | Asian = 0 Caucasian = 45 Indian = 2 Maori = 0 Pacific Islander = 0 Other (including African) = 3 |
| Mother of patient had preeclampsia (yes/no) | | yes = 17 no = 183 | yes =15 no = 85 | yes =13 no = 237 | yes =5 no = 45 |
| Any sister of patient had preeclampsia (yes/no) | | yes = 7 no = 193 | yes = 6 no = 94 | yes =3 no = 247 | yes =4 no = 46 |
| Father of patient has ischemic heart disease (yes/no) | father_any_hd | yes = 16 no = 184 | yes = 21 no = 79 | yes = 28 no = 222 | yes = 6 no = 44 |
| Mother or sister of patient had preeclampsia (yes/no) | fh_pet | yes = 21 no = 179 | yes = 20 no = 80 | yes = 16 no = 234 | yes = 9 no = 41 |
| Mother or sister of patient had | fh_petxcardio | yes = 35 no = 165 | yes = 39 no = 61 | yes = 41 no = 209 | yes = 15 no = 35 |
| preeclampsia and/or father of patient has ischemic heart disease (yes/no) | | | | | |
| BMI at 15 weeks | 1st_vst_bmi | 25.83 (5.77) | 28.28 (7.39) | 24.92 (4.64) | 27.18 (5.01) |
| diastolic blood pressure at 15 weeks visit - 1st measurement (mm Hg) | 1st_vst_1st_dbp | 64.72 (7.95) | 68.97 (8.32) | 64.43 (7.46) | 67.14 (7.98) |
| Systolic blood pressure at 15 weeks visit - 1st measurement (mm Hg) | 1st_vst_st_sbp | 108.75 (10.42) | 114.31 (11.41) | 105.1 (11.03) | 109.88 (11.73) |
| diastolic blood pressure at 15 weeks visit - 2nd measurement (mm Hg) | 1st_vst_2nd_dbp | 64.40 (7.69) | 68.60 (10.39) | 64.93 (7.58) | 67.46 (7.35) |
| Systolic blood pressure at 15 weeks visit - 2nd measurement (mm Hg) | 1st_vst_2rtd_sbp | 107.29 (9.73) | 113.51 (10.79) | 105.34 (10.62) | 110.20 (11.87) |
| Mean arterial pressure calculated at 15 weeks visit | 1st_vst_map_1st | 79.39 (7.92) | 84.08 (8.42) | 77.99 (7.91) | 81.39 (8.64) |
| from 1st measurement blood pressures | | | | | |

(continued)

| Parameter | Code | Test (New Zealand + Australia) | | Test (UK+Ireland) | |
|---|---|---|---|---|---|
| | | Controls (200) | Cases (100) | Controls (250) | Cases (50) |
| Mean arterial pressure at 15 weeks visit calculated from 2nd measurement blood pressures | 1st_vst_map_2nd | 78.69 (7.49) | 83.57 (8.49) | 78.40 (7.93) | 81.71 (8.07) |
| Random blood glucose level (mmol/L) at 15 weeks visit | 1st_vst_random_glucose | 5.43 (0.87) | 5.47 (0.99) | 5.09 (1.04) | 5.01 (1.09) |
| Metabolic syndrome* | Metabolic_syndrome | yes = 22 no = 178 | yes = 31 no = 69 | Na | Na |
| diastolic blood pressure at 20 weeks visit - 1st measurement (mm Hg) | 2nd_vst_1st_dbp | 64.83 (7.69) | 68.82 (8.75) | 65.50 (7.12) | 69.84 (10.32) |
| Systolic blood pressure at 20 weeks visit - 1st measurement (mm Hg) | 2nd_vst_1st_sbp | 110.55 (10.64) | 115.31 (9.80) | 107.06 (10.07) | 113.68 (12.35) |
| diastolic blood pressure at 20 weeks visit - 2nd measurement (mm | 2nd_vst_2nd_dbp | 64.80 (7.46) | 68.48 (8.52) | 65.71 (7.52) | 70.90 (10.18) |
| Systolic blood pressure at 20 weeks visit - 2nd measurement (mm Hg) | 2nd_vst_2nd_sbp | 109.79 (10.46) | 114.48 (9.74) | 107.42 (9.93) | 114.42 (12.72) |
| Mean arterial pressure calculated at 20 weeks visit from 1st measurement blood pressures | 2nd_vst_map_1st | 80.07 (7.40) | 84.32 (7.85) | 79.35 (7.32) | 84.45 (10.42) |
| Mean arterial pressure at 20 weeks visit calculated from 2nd measurement blood pressures | 2nd_vst_map_2nd | 79.79 (7.94) | 83.81 (7.82) | 79.62 (7.46) | 85.41 (10.49) |
| Random blood glucose level (mmol/L) at 20 weeks visit | 2nd_vst_random_glucose | 5.40 (0.94) | 5.63 (1.11) | 5.25 (1.07) | 5.38 (1.15) |
| birth weight of newborn (g) | | 3561 (478) | 3016 (782) | 3368 (552) | 2984 (855) |
| Highest diastolic blood pressure measured during | highest_dbp | 74.06 (9.59) | 103.02 (9.57) | 79.23 (10.55) | 107.42 (15.44) |
| Highest systolic blood pressure measured during pregnancy | highest_sbp | 122.80 (12.93) | 163.43 (18.13) | 125.21 (13.48) | 166.74 (20.79) |

(continued)

| Parameter | Code | Test (New Zealand + Australia) | | Test (UK+Ireland) | |
|---|---|---|---|---|---|
| | | Controls (200) | Cases (100) | Controls (250) | Cases (50) |
| **Maximal read out for dipstick proteinurea (number of patients)** | | dipstick = 1: 94<br>dipstick =2: 7<br>No data: 99 | dipstick = 1: 11<br>dipstick = 2: 12<br>dipstick = 3: 33<br>dipstick = 4: 28<br>no data: 16 | dipstick = 1: 213<br>dipstick = 2: 26<br>dipstick = 3: 5<br>dipstick = 4: 1<br>no data: 5 | dipstick = 1: 2<br>dipstick = 2: 7<br>dipstick = 3: 16<br>dipstick = 4: 25<br>no data: |
| **Newborn is Small for Gestational *Age* (number of patients)** | | 16 | 22 | 33 | 17 |
| **Preeclampsia (number of patients)** | | - | 100<br>Early onset preeclampsia: 10<br>Preterm preeclampsia: 30<br>Multisystem complications: 34 | - | 50<br>Early onset preeclampsia: 4<br>Preterm preeclampsia: 12<br>Multisystem complications: 15 |

\* A patient was defined as having a metabolic syndrome if she fulfilled at least 2 of the following 4 conditions: a) f11_bmi greater than or equal to 30, b) bb_trig >1.7, c) bb_hdl <1.29, and d) (f11_2nd_sbp > 130) or (f11_2nd_dbp>85).

**Table 2B.** Maternal characteristics including information about family history of disease, clinical parameters obtained during visits at 15 weeks and 20 weeks of gestation and some maternal and fetal characteristics as collected at pregnancy outcome. Results are N, number of patients, or mean (Standard deviation).

| Parameter | Code | Cohort (New Zealand + Australia+ Europe) | |
|---|---|---|---|
| | | Controls (450) | Cases (150) |
| **Age mother** | | 27.98 (5.87) | 27.61 (6.00) |
| **Ethnicity** | | Asian = 8<br>Caucasian = 403<br>Indian = 14<br>Maori = 4<br>Pacific Islander = 2<br>Other (including African) = 19 | Asian = 7<br>Caucasian = 129<br>Indian = 5<br>Maori = 1<br>Pacific Islander = 3<br>Other (including African) = 5 |
| **Mother of patient had preeclampsia (yes/no)** | | yes = 30<br>no = 420 | yes =20<br>no = 130 |
| **Any sister of patient had preeclampsia (yes/no)** | | yes = 10<br>no = 440 | yes =10<br>no = 140 |
| **Father of patient has ischemic heart disease (yes/no)** | father_any_ihd | yes = 44<br>no = 406 | yes = 27<br>no = 123 |
| **Mother or sister of patient had preeclampsia (yes/no)** | fh_pet | yes = 37<br>no = 413 | yes = 29<br>no = 121 |

(continued)

| Parameter | Code | Cohort (New Zealand + Australia+ Europe) | |
| --- | --- | --- | --- |
| | | Controls (450) | Cases (150) |
| Mother or sister of patient had preeclampsia and/or father of patient has ischemic heart disease (yes/no) | fh_petxcardio | yes = 76<br>no = 374 | yes = 54<br>no = 96 |
| BMI at 15 weeks | 1st_vst_bmi | 25.33 (5.18) | 27.61 (6.00) |
| diastolic blood pressure at 15 weeks visit - 1st | 1st_vst_1st_dbp | 64.55 (7.67) | 68.36 (8.23) |
| measurement (mm Hg) | | | |
| Systolic blood pressure at 15 weeks visit - 1st measurement (mm Hg) | 1st_vst_1st_sbp | 106.73 (10.90) | 112.83(11.67) |
| diastolic blood pressure at 15 weeks visit - 2nd measurement (mm Hg) | 1st_vst_2nd_db p | 64.69 (7.62) | 68.22 (8.23) |
| Systolic blood pressure at 15 weeks visit - 2nd measurement (mm Hg) | 1st_vst_2nd_sb p | 106.21 (10.27) | 112.41 (11.23) |
| Mean arterial pressure calculated at 15 weeks visit from 1 st measurement blood pressures | 1st_vst_map_1s t | 78.61 (7.94) | 83.18 (8.56) |
| Mean arterial pressure at 15 weeks visit calculated from 2nd measurement blood pressures | 1st_vst_map_2n d | 78.53 (7.73) | 82.95 (8.37) |
| Random blood glucose level (mmol/L) at 15 weeks visit | 1st_vst_random _glucose | 5.21 (0.97) | 5.32 (1.04) |
| diastolic blood pressure at 20 weeks visit-1st measurement (mm Hg) | 2nd_vst_1st_db p | 65.20 (7.38) | 69.16 (9.28) |
| Systolic blood pressure at 20 weeks visit - 1st measurement (mm Hg) | 2nd_vst_1st_sb p | 108.61 (10.46) | 114.77 (10.70) |
| diastolic blood pressure at 20 weeks visit - 2nd measurement (mm Hg) | 2nd_vst_2nd_d bp | 65.30 (7.49) | 69.29 (9.14) |
| Systolic blood pressure at 20 weeks visit - 2nd measurement (mm Hg) | 2nd_vst_2nd_sb p | 108.48 (10.23) | 114.46 (10.78) |
| Mean arterial pressure calculated at 20 weeks visit from 1st measurement blood pressures | 2nd_vst_map_1 st | 79.67 (7.36) | 84.36 (8.75) |
| Mean arterial pressure at 20 weeks visit calculated from 2nd measurement blood pressures | 2nd_vst_map_2 nd | 79.69 (7.31) | 84.34 (8.80) |
| Random blood glucose level (mmol/L) at 20 weeks visit | 2nd_vst_rando m_ glucose | 5.31 (1.02) | 5.55 (1.13) |
| birth weight of newborn (g) | | 3420 (563) | 3005 (804) |
| Gender of newborn | | Female: 217<br>Male: 233 | Female: 74<br>Male: 76 |
| Highest diastolic blood pressure measured | highest_dbp | 76.92 (10.44) | 104.77 (11.86) |
| during pregnancy | | | |

(continued)

| Parameter | Code | Cohort (New Zealand + Australia+ Europe) | |
| --- | --- | --- | --- |
| | | Controls (450) | Cases (150) |
| Highest systolic blood pressure measured during pregnancy | híghest_sbp | 124.13 (13.28) | 165.00 (19.08) |
| Maximal read out for dipstick proteinurea (number of patients) | | dipstick = 1: 307 dipstick =2: 33 dipstick=3: 5 dipstick=4: 1 No data: 104 | dipstick = 1: 13 dipstick = 2: 19 dipstick = 3: 49 dipstick = 4: 53 no data: 16 |
| Newborn is Small for Gestational Age (number of patients) | | 49 | 39 |
| Preeclampsia (number of patients) | | - | 150 Early onset preeclampsia: 14 Preterm preeclampsia: 42 Multisystem complications: 39 |

## Example 2: MASSterclass® targeted protein quantitation

The following describes one exemplary and preferred way of targeted protein quantification in samples, particularly as also used in the present examples.

## MASSTERCLASS® experimental setup

MASSterclass® assays use targeted tandem mass spectrometry with stable isotope dilution as an end-stage peptide quantitation system (also called Multiple Reaction Monitoring (MRM) and Single Reaction Monitoring (SRM). The targeted peptide is specific {i.e., proteotypic) for the specific protein of interest, i.e., the amount of peptide measured is directly related to the amount of protein in the original sample. To reach the specificity and sensitivity needed for biomarker quantitation in complex samples, peptide fractionation precedes the end-stage quantitation step.
A suitable MASSTERCLASS® assay may include the following steps:

- Plasma/serum sample

- Depletion of human albumin and IgG (complexity reduction on protein level) using affinity capture with anti-albumin and anti-IgG antibodies using ProteoPrep spin columns (Sigma Aldrich)

- Spiking of known amounts of isotopically labelled peptides. These peptides has the same amino acid sequence as the proteotypic peptides of interest, typically with one isotopically labelled amino acid built in to generate a mass difference. During the entire process, the labelled peptide has identical chemical and chromatographic behaviour as the endogenous peptide, except during the end-stage quantitation step which is based on molecular mass.

- Tryptic digest. The proteins in the depleted serum/plasma sample are digested into peptides using trypsin. This enzyme cleaves proteins C-terminally from lysine and arginine, except when a proline is present C-terminally of the lysine or arginine. Before digestion, proteins are denatured by boiling, which renders the protein molecule more accessible for the trypsin activity during the 16h incubation at 37°C.

- Peptide-based fractionation: The charged peptide molecules are separated based on their specific isoelectric property. As there is no pi difference between the endogenous peptide and the isotopically labelled variant, they co-elute. Only those fractions containing the monitored peptides, or pools thereof, are selected and proceed to the next level of fractionation.

- LC-MS/MS based quantitation, including further separation on reversed phase (C18) nanoLC (PepMap C18; Dionex) and MS/MS: tandem mass spectrometry using MRM (4000 QTRAP; ABI) or SRM (Vantage TSQ; Thermo Scientific) mode. The LC column is connected to an electrospray needle connected to the source head of the mass spectrometer. As material elutes from the column, molecules are ionized and enter the mass spectrometer in the gas phase. The peptide that is monitored is specifically selected to pass the first quadrupole (Q1), based on its mass to charge ratio (m/z). The selected peptide is then fragmented in a second quadrupole (Q2) which is used as a collision cell. The resulting fragments then enter the third quadrupole (Q3). Depending on the instrument settings (determined during the assay development phase) only a specific peptide fragment or specific peptide fragments (or so called transitions) are selected for detection.

- The combination of the m/z of the monitored peptide and the m/z of the monitored fragment of this peptide is called a transition. This process can be performed for multiple transitions during one experiment. Both the endogenous peptide (analyte) and its corresponding isotopically labelled synthetic peptide (internal standard) elute at the same retention time, and are measured in the same LC-MS/MS experiment.

- The MASSterclass® readout is defined by the ratio between the area under the peak specific for the analyte and the area under the peak specific for the synthetic isotopically labelled analogue (internal standard). MASSterclass® readouts are directly related to the original concentration of the protein in the sample. MASSterclass readouts can therefore be compared between different samples and groups of samples.

A typical MASSTERCLASS® protocol followed in the present study:

- 25μl of plasma is subjected to a depletion of human albumin and IgG (ProteoPrep spin columns; Sigma Aldrich) according to the manufacturer's protocol, except that 20mM NH4HCO3 was used as the binding/equilibration buffer.

- The depleted sample (225μl) is denatured for 15min at 95°C and immediately cooled on ice

- 2 pmol of each isotopically labeled peptide (custom made 'Heavy AQUA' peptide; Thermo Scientific) is spiked in the sample

- 2C^g trypsin is added to the sample and digestion is allowed for 16h at 37°C

- Half of the resulting sample is applied to pl-based separation. Fractions containing the peptides of interest are pooled together, dried and resuspended in 0.1 % formic acid.

- 20μl of the final solution is separated using reverse-phase NanoLC with on-line MS/MS in SRM mode:

- Column: PepMap C18, 75μnι I.D. x 25cm L, 100 A pore diameter, 5μnι particle size

- Solvent A: 0.1 % formic acid

- Solvent B: 80% acetonitrile, 0.1 % formic acid

- Gradient: 30 min; 2%-55% Solvent B

- MS/MS in SRM mode: method contains the transitions for the analyte as well as for the synthetic, labeled peptide.

- The used transitions were experimentally determined and selected during protein assay development.

Table 3. The peptides used for different MASSterclass® assays. For some proteins more than one peptides were used for the assay.

| Protein | Peptide sequence | SEQ ID number |
|---------|------------------|---------------|
| MMRN2 | EAEPLVDIR | 1 |
| ADAM12 | ELIINLER | 2 |
| ADAM12 | ADEWSASVR | 3 |

(continued)

| Protein | Peptide sequence | SEQ ID number |
|---|---|---|
| ECM1 | NVALVSGDTENAK | 4 |
| ECM1 | EVGPPLPQEAVPLQK | 5 |
| ENG | LPDTPQGLLGEAR | 6 |
| LCAT | TYSVEYLDSSK | 7 |
| LCAT | LEPGQQEEYYR | 8 |
| LNPEP | YISIGSEAEK | 9 |
| PRDX2 | EGGLGPLNIPLLADVTR | 10 |
| CRP | ESDTSYVSLK | 11 |
| MAPRE1/3 MAPRE3 | FFDANYDGK | 12 |
| PRCP | YYGESLPFGDNSFK | 13 |
| COL6A3 | SLDEISQPAQELK | 14 |
| SPINT1 | YTSGFDELQR | 15 |
| SEPP1 | LPTDSELAPR | 16 |
| QSOX1 | LAGAPSEDPQFPK | 17 |
| IGFALS | LAELPADALGPLQR | 18 |
| ALDOA | GILAADESTGSIAK | 19 |
| MCAM | GATLALTQVTPQDER | 20 |
| ROBO4 | EDFQIQPR | 21 |
| ENPP2 | DIENLTSLDFFR | 22 |
| CST3 | ALDFAVGEYNK | 23 |
| XPNPEP2 | GTVDEFSGAEIVDK | 24 |
| HSPG2 | GSIQVDGEELVSGR | 25 |
| TNXB | TVTVEDLEPGK | 26 |
| PCYOX1 | SDFYDIVLVATPLNR | 27 |
| FLT4 | GPILEATAGDELVK | 28 |
| PRDX1 | ATAWDGAFK | 29 |
| IL6ST* | ILDYEVTLTR | 33 |
| PROC | GDSPWQVVLLDSK | 34 |
| PCDH12 | NPAYEVDVQAR | 35 |
| * The MC peptide in particular allows identification of IL6ST isoforms 1 and 3, while isoform 2 (NP_786943.1, NM_175767.2) may not be detected using this peptide. | | |

For purposes of Example 11 , two different peptides are taken into account for IGFALS (SEQ ID NO: 18 as above, and VAGLLEDTFPGLLGLR (SEQ ID NO: 36)); for PRDX1 (SEQ ID NO: 29 as above, and ADEGISFR (SEQ ID NO: 37)); and for ADAM 12 (SEQ ID NO: 2 as above, and DLETSLEK (SEQ ID NO: 38)).

The level of placental growth factor (PIGF) was determined via Delfia assay (PerkinElmer, Turku, Finland), which is a solid-phase, two-site fluoroimmunometric assay based on the direct sandwich technique in which monoclonal antibodies and polyclonal antibodies are directed against the unbound PIGF molecule. Likewise diagnostically relevant PIGF levels can be obtained with assays that target PIGF bound to its receptor s-Flt1 (e.g., assays from Roche Diagnostics, Switzerland; and R&D systems, Minnesota, USA), assays that target the free PIGF in circulation (PerkinElmer, Finland and Alere, Ireland) and assays that specifically target specific isoforms of PIGF (PerkinElmer, Finland and Alere, Ireland).

Alternatively, the level of IGFALS, specifically in the European cohort, was determined by ELISA, Mediagnost, Germany. The ELISA-measured values proved to be able to interchange the MASSterclass read outs, giving rise to similar or even better performances for the algorithms.

The level of TFF3 was determined by ELISA assay, BioVendor - Laboratorni medicina a.s., Czech Republic.

All marker levees were measured in subjects at 20 weeks gestation.

**Example 3: Statistical analysis**

Logistic regression was used to define multivariate classifier models (test panels) that predict the outcome (pre-eclampsia / no pre-eclampsia) (Royston et al. 2009, Prognosis and prognostic research: Developing a prognostic model, BMJ 2009: 338:b604).

The predictors (biomarkers and parameters) were normalised. The binary variables were coded 0/1, the analyte concentrations and relative concentrations (Masterclass measurements) were log-transformed. For feature selection, all parameters were normalised (Z-normalisation).

Feature selection was performed using the shrinkage and selection method Lasso (Tibshirani 1996, Regression shrinkage and selection via the lasso, J. Royal. Statist. Soc B. 58(1): 267-288). The performance of the models (test panels) was estimated using the apparent area under the receiver-operating curve (AUC). The prediction error for the classifiers was estimated using cross-validation. The classifiers were ranked based on their performance and prediction error.

Where indicated, and specifically for the outcomes a) all preeclampsia AUC value, b) all preeclampsia "rule-in" criterion and c) all preeclampsia "rule-out" criterion, algorithms were developed in the training set, i.e., the Australasian cohort, and evaluated upon their predictive performance in the testing set, i.e., the independent European cohort, without changing the weighing factors in the algorithms.

For each algorithm developed in the training set giving rise to a C-statistic AUO0.7, the according performance was also established in the test set. The number of covariates in an algorithm was limited to a maximum of 6 to avoid "overfitting"; all algorithms with a p value for the Wald test for any of the covariates above 0.10 were ignored.

Where indicated, the test panels were also evaluated for their performance as "rule-in" tests. To this aim, the panels were assessed for their ability to adequately predict preeclampsia without identifying too many false positives. Within the context of a low prevalence disease, such as PE, a Positive Predictive Value (PPV) above or equal to 0.20 (i.e., 20%) is found clinically desirable (PPV = # True Positives / (#True Positives + False Positives). Whereas the threshold for PPV may be set at a different value, the illustrative cut-off of 0.20 was found to work adequately in the examples.

To enable a quantitative assessment of the above PPV criterion, PPV-values are calculated for a population of 1000 pregnancies, taking into account the prevalence as relevant to the population studied. For all pre-eclampsia, prevalence of 5.3% has been previously reported in literature (BMJ 2011, vol. 342, d1875, doi: 10.1 136/bmj.d1875) and may be used for this calculation. PPV data can be transformed to sensitivity and specificity values to allow plotting of the PPV threshold on the receiving-operating curve (ROC). For this transformation, the True Positive (TP), False Positive (FP), False Negative (FN) and True Negative (TN) values were calculated as follows:

TP is set in increments of 1 from 0 to # diseased (i.e., 1000 x prevalence, herein 53);

$$FP = (TP-(TP<*>PPV))/PPV)$$

$$FN = \text{\# diseased (i.e., 1000 x prevalence, herein 53) - TP}$$

$$TN = \text{\# non-diseased (i.e., 1000-(1000 x prevalence), herein 947) - FP}$$

Sensitivity and 1 -Specificity were conventionally calculated based on the above TP, FP, FN, and TN values.

The above calculations can be readily applied to patient subpopulations and/or outcomes characterised by different prevalence. For example, prevalence of preeclampsia in non-obese subjects has been documented to be 4.3% (BMJ 201 1 , vol. 342, d1875, supra). Algorithms were classified as particularly good rule-in panels when both in training and test sets sensitivities (= detection rates) of ≥0.50 (≥50% case detection rate) were achieved.

Rule-in tests were not applied to prediction of preterm PE and term PE, due to a limited number of cases.

Where indicated, the test panels were also evaluated for their performance as "rule-out" tests.

To this aim, the panels were assessed for their ability to adequately rule-out preeclampsia well without the burden of missing too many cases (false negatives). Within the context of a low prevalence disease, a Negative Predictive Value (NPV) above or equal to 0.99 or 99% is found clinically viable; NPV = # True Negatives / (#True Negatives + False Negatives). Whereas the threshold for NPV may be set at a different value, the illustrative cut-off of 0.99 was found to work adequately in the examples.

To enable a quantitative assessment of the above NPV criterion, NPV-values are calculated for a population of 1000 pregnancies, taking into account the prevalence as relevant to the population studied. For all pre-eclampsia, prevalence of 5.3% has been previously reported in literature (BMJ 2011 , vol. 342, d1875, doi: 10.1 136/bmj.d1875) and may be used for this calculation. NPV data can be transformed to sensitivity and specificity values to allow plotting of the NPV threshold on the receiving-operating curve (ROC). For this transformation, the True Positive (TP), False Positive (FP), False Negative (FN) and True Negative (TN) values were calculated as follows:

$$TP = \# \text{ diseased (i.e., 1000 x prevalence, herein 53) - FN}$$

$$FP = \# \text{ non-diseased (i.e., 1000-(1000 x prevalence), herein 947) - TN}$$

$$FN = (TN-(TN<*>NPV)) / NPV$$

TN is set in decrements of 1 from # non-diseased to 0 (i.e., #non-diseased equals (1000 - (1000 x prevalence)), herein 947)

Sensitivity and 1 -Specificity were conventionally calculated based on the above TP, FP, FN, and TN values.

The above calculations can be readily applied to patient subpopulations and/or outcomes characterised by different prevalence. For example, prevalence of preeclampsia in non-obese subjects has been documented to be 4.3% (BMJ 201 1 , vol. 342, d1875, supra). Algorithms were classified as particularly good rule-out panels when both in training and test sets specificities of≥0.40 (≥40% control detection rate) were achieved.

Rule-out tests were not applied to prediction of preterm PE and term PE, due to a limited number of cases.

Algorithms were classified as particularly good rule-in and rule-out panels when both in training and test sets sensitivities (= detection rates) of ≥0.50 (≥50% case detection rate) were achieved, and when both in training and test sets specificities of ≥0.40 (≥40% control detection rate) were achieved.

### Example 4: Illustrative test panels for the prediction of PE

The data and analyses in this example have been obtained using the case-control sets as captured in Table 2, and applying the statistical analysis methods as elucidated in Example 3. Panels or combinations of markers and/or clinical parameters were obtained to develop models that estimate the probability of contracting preeclampsia.

Whereas the outcome of the test panels exemplified herein is the prediction of preeclampsia at 20 weeks of gestation, the test panels are useful throughout the second trimester, such as between 13 and 28 weeks of gestation, e.g., at 20 +/- 2, 20 +/- 1, 15 +/- 2 or 15 +/- 1 weeks of gestation, and can even be applied with success in the first trimester.

**Tables 4A and 4B** capture relevant statistics pertinent to performance of panels useful for predicting PE, which illustrate various embodiments of the present invention. The following abbreviations are used in the tables: **AUC:** area under the ROC curve; **lCI:** lower confidence interval; **uCI:** upper confidence interval. The following column denotations are used in the tables: **No:** sequential number given to a panel (arbitrary but denoting identical panels between Tables 4A and 4B); **Panel composition:** constituents forming up a panel (i.e., the panel consists of the recited constituents); **A:** AUC for predicting all PE (i.e., without distinction between preterm and term PE) at 20 weeks - training set (Australasian cohort); **B:** AUC for predicting all PE at 20 weeks - testing set (European cohort); **C:** Sensitivity at 20% PPV for predicting all PE at 20 weeks - training set (Australasian cohort); **D:** Sensitivity at 20% PPV for predicting all PE at 20 weeks - testing set (European cohort); **E:** Specificity at 99% NPV for predicting all PE at 20 weeks - training set (Australasian cohort); **F:** Specificity at 99% NPV for predicting all PE at 20 weeks - testing set (European cohort); **G:** AUC for predicting all PE at 20 weeks - testing set (European cohort); **H:** lCI AUC for predicting all PE at 20 weeks - testing set (European cohort); **I:** uCI AUC for predicting all PE at 20 weeks - testing set (European cohort); **J:** AUC for predicting preterm PE at 20 weeks - testing set (European cohort); **K:** lCI AUC for predicting preterm PE at 20 weeks - testing set (European cohort); **L:** uCI AUC for predicting preterm PE at 20 weeks - testing set (European cohort); **M:** AUC for predicting term PE at 20 weeks - testing set (European cohort); **N:** lCI AUC for predicting term PE at 20 weeks - testing set (European cohort); **O**: uCI AUC for predicting term PE at 20 weeks - testing set (European cohort); **P:** AUC - for predicting all PE at 20 weeks training set (Australasian cohort); **Q**: lCI AUC for predicting all PE at 20 weeks - training set (Australasian cohort); **R:** uCI AUC for predicting all PE at 20 weeks - training set (Australasian cohort); **S:** AUC for predicting preterm PE at 20 weeks - training set (Australasian cohort); **T:** lCI AUC for predicting preterm PE at 20 weeks - training set (Australasian cohort); **U:** uCI AUC for predicting preterm PE at 20 weeks - training set (Australasian cohort); **V:** AUC for predicting term PE at 20 weeks - training set (Australasian cohort); **W:** lCI AUC for predicting term PE at 20 weeks - training set (Australasian cohort); **Z:** uCI AUC for predicting term PE at 20 weeks - training set (Australasian cohort); **AA:** AUC for predicting all PE at 20 weeks - combined set (combined Australasian and European cohorts); **AB:** lCI AUC for predicting all PE at 20 weeks - combined set (combined Australasian and European cohorts); **AC:** uCI AUC for predicting all PE at 20 weeks - combined set (combined Australasian and European cohorts); **AD:** AUC for predicting preterm PE at 20 weeks - combined set (combined Australasian and European cohorts); **AE:** lCI AUC for predicting preterm PE at 20 weeks - combined set (combined Australasian and European cohorts); **AF:** uCI AUC for predicting preterm PE at 20 weeks - combined set (combined Australasian and European cohorts); **AG:** AUC for predicting term PE at 20 weeks - combined set (combined Australasian and European cohorts); **AH:** lCI AUC for predicting term PE at 20 weeks - combined set (combined Australasian and European cohorts); **AI**: uCI AUC for predicting term PE at 20 weeks - combined set (combined Australasian and European cohorts).

In Tables 4A and 4B, some redundancy is apparent among the panels, i.e., Tables 4A and 4B may list a test panel of a given composition more than once. One cause of the redundancy is when a given biomarker was measured in two or more distinct ways. For example, as set forth in Table 3, each ADAM12, ECM1 , and LCAT, could be measured by MASSTERCLASS® assays using two distinct peptides. Another cause of the redundancy is that the clinical parameter blood pressure (BP) or even the more specific parameters BP at 15 weeks (BP15) and BP at 20 weeks (BP20), cover a multiplicity of distinct blood pressure measurements, such as the measurement of systolic, diastolic or mean arterial blood pressure, as well as 1st or 2nd measurements (see Table 2).

**Table 4A**

| No. | Panel composition | A | B | C | D | E | F |
|---|---|---|---|---|---|---|---|
| 1 | bmi;ADAM12;ENG;SPINT1;QSOX1;IGFALS | 0.83 | 0.75 | 0.56 | 0.47 | 0.53 | 0.19 |
| 2 | bmi;ADAM12;ENG;MCAM;pbwqt;PIGF | 0.83 | 0.74 | 0.63 | 0.44 | 0.58 | 0.02 |
| 3 | bmi;*fhpet*;ADAM12;IGFALS;pbwgt;PIGF | 0.82 | 0.75 | 0.67 | 0.30 | 0.43 | 0.44 |
| 4 | bmi;ADAM12;ENG;SPINT1;QSOX1;IGFALS | 0.82 | 0.75 | 0.52 | 0.49 | 0.32 | 0.27 |
| 5 | bmi;ADAM12;QSOX1;IGFALS;pbwgt;PIGF | 0.82 | 0.76 | 0.64 | 0.34 | 0.49 | 0.51 |
| 6 | BP15;ADAM12;SPINT1;IGFALS;MCAM;PIGF | 0.82 | 0.76 | 0.59 | 0.50 | 0.43 | 0.32 |
| 7 | BP15;ADAM12;SPINT1;IGFALS;MCAM;PIGF | 0.82 | 0.76 | 0.59 | 0.55 | 0.40 | 0.30 |
| 8 | BP15;ADAM12;SPINT1;IGFALS;MCAM;PIGF | 0.81 | 0.76 | 0.59 | 0.45 | 0.46 | 0.30 |
| 9 | bmi;ADAM12;IGFALS;pbwgt;PIGF | 0.81 | 0.75 | 0.41 | 0.18 | 0.46 | 0.27 |
| 10 | bmi;BPI5;ADAM12;ENG;SPINT1;MCAM | 0.81 | 0.76 | 0.53 | 0.61 | 0.50 | 0.14 |
| 11 | bmi;ADAM12;ENG;SPINT1;SEPP1;IGFALS | 0.81 | 0.74 | 0.51 | 0.51 | 0.47 | 0.05 |
| 12 | bmi;frhd;ADAM12;ENG;SPINTI;IGFALS | 0.81 | 0.72 | 0.55 | 0.51 | 0.47 | 0.14 |
| 13 | BP15;ADAM12;SPINT1;IGFALS;MCAM;PIGF | 0.81 | 0.76 | 0.56 | 0.59 | 0.27 | 0.31 |
| 14 | BP15;MMRN2;ADAM12;IGFALS;MCAM;PIGF | 0.81 | 0.79 | 0.55 | 0.56 | 0.47 | 0.41 |
| 15 | BP15;MMRN2;ENG;SPINT1;IGFALS;MCAM | 0.81 | 0.77 | 0.59 | 0.57 | 0.46 | 0.22 |
| 16 | BP15;ADAM12;SPINT1;IGFALS;MCAM;PIGF | 0.81 | 0.75 | 0.56 | 0.32 | 0.44 | 0.30 |
| 17 | BP15;MMRN2;ENG;IGFALS;MCAM;PIGF | 0.81 | 0.75 | 0.62 | 0.44 | 0.52 | 0.27 |
| 18 | BP15;ADAM12;ENG;SPINT1;IGFALS;MCAM | 0.81 | 0.76 | 0.50 | 0.57 | 0.41 | 0.20 |
| 19 | BP15;MMRN2;ENG;SPINT1;IGFALS;MCAM | 0.81 | 0.77 | 0.51 | 0.57 | 0.40 | 0.26 |
| 20 | BP15;MMRN2;ENG;IGFALS;MCAM;PIGF | 0.81 | 0.75 | 0.63 | 0.46 | 0.46 | 0.15 |
| 21 | BP15;ENG;SPINT1;SEPP1 ;IGFALS;MCAM | 0.81 | 0.76 | 0.55 | 0.55 | 0.25 | 0.18 |
| 22 | BP15;ADAM12;ENG;SPINT1;IGFALS;MCAM | 0.81 | 0.76 | 0.52 | 0.59 | 0.50 | 0.21 |
| 23 | BP15;*fihd*;ADAM12;IGFALS;MCAM;PIGF | 0.81 | 0.76 | 0.57 | 0.23 | 0.37 | 0.36 |

(continued)

| No. | Panel composition | A | B | C | D | E | F |
|---|---|---|---|---|---|---|---|
| 24 | BP20;fihd;ADAM12;IGFALS;MCAM;PIGF | 0.81 | 0.77 | 0.60 | 0.42 | 0.40 | 0.28 |
| 25 | BP15;ENG;SPINT1;IGFALS;MCAM;PIGF | 0.81 | 0.76 | 0.62 | 0.48 | 0.26 | 0.22 |
| 26 | BP15;MMRN2;ENG;SPINT1;IGFALS;MCAM | 0.81 | 0.77 | 0.52 | 0.57 | 0.46 | 0.28 |
| 27 | BP15;ADAM12;ENG;SPINT1;IGFALS;MCAM | 0.81 | 0.75 | 0.50 | 0.52 | 0.40 | 0.27 |
| 28 | bmi;BP15;ADAM12;ENG;SPINT1;MCAM | 0.81 | 0.77 | 0.52 | 0.52 | 0.44 | 0.14 |
| 29 | *alcoh*;BP15;ENG;SPINT1;IGFALS;MCAM | 0.81 | 0.76 | 0.57 | 0.59 | 0.48 | 0.27 |
| 30 | BP15;ADAM12;IGFALS;MCAM;PIGF | 0.81 | 0.77 | 0.52 | 0.25 | 0.45 | 0.27 |
| 31 | *alcoh*;BP15;ADAM12;ENG;SPiNT1;IGFALS | 0.81 | 0.73 | 0.55 | 0.53 | 0.40 | 0.14 |
| 32 | BP15;*fihd*;ENG;SPINT1;IGFALS;MCAM | 0.81 | 0.75 | 0.58 | 0.57 | 0.32 | 0.19 |
| 33 | BP20;*fihd*;ADAM12;IGFALS;MCAM;PIGF | 0.81 | 0.76 | 0.65 | 0.33 | 0.40 | 0.38 |
| 34 | BP15;ENG;SPINT1;IGFALS;MCAM;PIGF | 0.81 | 0.75 | 0.56 | 0.50 | 0.40 | 0.28 |
| 35 | BP15;frhd;MMRN2;AQAM12;IGFALS;MCAM | 0.81 | 0.75 | 0.55 | 0.46 | 0.45 | 0.36 |
| 36 | BP15;MMRN2;ENG;SPINT1;IGFALS;MCAM | 0.81 | 0.78 | 0.51 | 0.55 | 0.48 | 0.28 |
| 37 | BP15;MMRN2;ENG;IGFALS;MCAM;PIGF | 0.81 | 0.75 | 0.61 | 0.50 | 0.47 | 0.30 |
| 38 | BP15;fihd;ENG;SPINT1;IGFALS;MCAM | 0.81 | 0.75 | 0.65 | 0.50 | 0.30 | 0.29 |
| 39 | bmi;BP15;ADAM12;ENG;SPINT1;MCAM | 0.81 | 0.76 | 0.51 | 0.55 | 0.43 | 0.14 |
| 40 | bmi;BP15;ADAM12;ENG;MCAM;PIGF | 0.81 | 0.75 | 0.51 | 0.50 | 0.43 | 0.03 |
| 41 | BP15;ENG;SPINT1;QSOX1;IGFALS;MCAM | 0.81 | 0.76 | 0.52 | 0.57 | 0.41 | 0.28 |
| 42 | *alcoh*;BP15;ADAM12;IGFALS;MCAM;PIGF | 0.81 | 0.78 | 0.56 | 0.46 | 0.47 | 0.47 |
| 43 | BP15;At7AM12;IGFALS;MCAM;PIGF | 0.81 | 0.77 | 0.52 | 0.31 | 0.40 | 0.30 |
| 44 | BP15;ENG;SPINT1;SEPP1;IGFALS;MCAM | 0.81 | 0.76 | 0.55 | 0.57 | 0.37 | 0.17 |
| 45 | *alcoh*;BP15;ADAM12;SPINT1;IGFALS;PIGF | 0.80 | 0.74 | 0.62 | 0.44 | 0.33 | 0.31 |
| 46 | BP15;ADAM12;SPINT1;IGFALS;MCAM;PIGF | 0.80 | 0.76 | 0.57 | 0.55 | 0.40 | 0.32 |
| 47 | BP15;ENG;SPINT1;IGFALS;MCAM;PIGF | 0.80 | 0.75 | 0.60 | 0.55 | 0.30 | 0.16 |
| 48 | BP15;ADAM12;IGFALS;MCAM;PIGF | 0.80 | 0.77 | 0.53 | 0.38 | 0.46 | 0.29 |
| 49 | BP15;MMRN2;ENG;IGFALS;MCAM;PIGF | 0.80 | 0.75 | 0.59 | 0.44 | 0.43 | 0.24 |
| 50 | bmi;*find*;ADAM12;ENG;SPINT1;MCAM | 0.80 | 0.75 | 0.53 | 0.55 | 0.43 | 0.07 |
| 51 | BP95;MMRN2;ADAM12;SEPP1;IGFALS;PIGF | 0.80 | 0.77 | 0.61 | 0.37 | 0.62 | 0.31 |
| 52 | BP15;ENG;SPINT1;IGFALS;MCAM;PIGF | 0.80 | 0.76 | 0.54 | 0.55 | 0.36 | 0.21 |
| 53 | bmi;ADAM12;ENG;SPINT1;MCAM;PIGF | 0.80 | 0.75 | 0.64 | 0.45 | 0.23 | 0.18 |
| 54 | BP20;ADAM12;SEPP1;IGFALS;MCAM;PIGF | 0.80 | 0.79 | 0.60 | 0.48 | 0.43 | 0.36 |
| 55 | *alcoh*;BP15;ENG;SPINT1;IGFALS;MCAM | 0.80 | 0.76 | 0.59 | 0.64 | 0.45 | 0.24 |
| 56 | BP15;ENG;SPINT1;QSOX1;IGFALS;MCAM | 0.80 | 0.76 | 0.48 | 0.55 | 0.43 | 0.31 |
| 57 | *alcoh*;BP15;ENG;SPINT1;IGFALS;MCAM | 0.80 | 0.76 | 0.53 | 0.50 | 0.52 | 0.33 |
| 58 | BP15;ADAM12;SPINT1;SEPP1;IGFALS;PIGF | 0.80 | 0.75 | 0.54 | 0.56 | 0.20 | 0.26 |
| 59 | bmi;BP20;ADAM12;ENG;QSOX1;IGFALS | 0.80 | 0.74 | 0.62 | 0.39 | 0.34 | 0.16 |
| 60 | *alcoh*;BP15;ADAM12;SPINT1;IGFALS;PIGF | 0.80 | 0,75 | 0.60 | 0.49 | 0.24 | 0.32 |
| 61 | BP15;ENG;SPINT1;SEPP1;IGFALS;MCAM | 0.80 | 0.76 | 0.53 | 0.57 | 0.30 | 0.29 |

(continued)

| No. | Panel composition | A | B | C | D | E | F |
|---|---|---|---|---|---|---|---|
| 62 | bmi;ADAM12;ENG;IGFALS;MCAM;PIGF | 0.80 | 0.76 | 0.55 | 0.42 | 0.39 | 0.24 |
| 63 | BP15;LNPEP;IGFALS;MCAM;PIGF | 0.80 | 0.74 | 0.54 | 0.29 | 0.52 | 0.33 |
| 64 | *fihd*;MMRN2;ADAM12;ENG;SPINT1;IGFALS | 0.80 | 0.72 | 0.57 | 0.51 | 0.29 | 0.17 |
| 65 | *alcoh*;BP15;ENG;SPINT1;IGFALS;MCAM | 0.80 | 0.76 | 0.54 | 0.57 | 0.56 | 0.28 |
| 66 | BP15;ENG;SPINT1;IGFALS;MCAM;PIGF | 0.80 | 0.76 | 0.62 | 0.50 | 0.32 | 0.21 |
| 67 | BP15;ADAM12;ENG;SPINT1;IGFALS;MCAM | 0.80 | 0.76 | 0.50 | 0.55 | 0.33 | 0.19 |
| 68 | BP15;ENG;SPINT1;IGFALS;MCAM;ROBO4 | 0.80 | 0.75 | 0.54 | 0.50 | 0.38 | 0.28 |
| 69 | BP15;MMRN2;ENG;IGFALS;MCAM;PIGF | 0.80 | 0.75 | 0.62 | 0.40 | 0.21 | 0.19 |
| 70 | BP15;ADAM12;ENG;SPINT1;MCAM;PIGF | 0.80 | 0.75 | 0.53 | 0.45 | 0.34 | 0.16 |
| 71 | *alcoh*;BP15;MMRN2;ENG;IGFALS;MCAM | 0.80 | 0.74 | 0.57 | 0.44 | 0.45 | 0.28 |
| 72 | ADAM12;ENG;SPINT1;IGFALS;MCAM;PIGF | 0.80 | 0.75 | 0.57 | 0.45 | 0.29 | 0.27 |
| 73 | *alcoh*;BP15;ADAM12;ENG;SPINT1;IGFALS | 0.80 | 0.73 | 0.50 | 0.51 | 0.48 | 0.14 |
| 74 | BPIS;*fihd*;ENG;SPINT1;IGFALS;MCAM | 0.80 | 0.75 | 0.58 | 0.50 | 0.32 | 0.17 |
| 75 | *alcoh*;BP20;ADAM12;IGFALS;MCAM;PIGF | 0.80 | 0.79 | 0.58 | 0.48 | 0.40 | 0.26 |
| 76 | bmi;BP15;ADAM12;ENG;SPINT1;PIGF | 0.80 | 0.73 | 0.54 | 0.53 | 0.44 | 0.14 |
| 77 | *alcoh*;BP20;*fihd*;MMRN2;ADAM12;IGFALS | 0.80 | 0.76 | 0.58 | 0.45 | 0.43 | 0.19 |
| 78 | BP15;ENG;SPINT1;QSOX1;IGFALS;MCAM | 0.80 | 0.76 | 0.49 | 0.57 | 0.40 | 0.27 |
| 79 | *alcoh*;BP15;ENG;SPINT1;IGFALS;MGAM | 0.80 | 0.75 | 0.57 | 0.55 | 0.51 | 0.30 |
| 80 | BP15;*fidh*;IGFALS;MCAM;PIGF | 0.80 | 0.73 | 0.51 | 0.27 | 0.19 | 0.30 |
| 81 | BP15;*fihd*;ENG;SPINT1;IGFALS;MCAM | 0.80 | 0.74 | 0.64 | 0.52 | 0.12 | 0.29 |
| 82 | BP15;*fihd*;ENG;SPINT1;IGFALS;MCAM | 0.80 | 0.75 | 0.51 | 0.59 | 0.51 | 0.15 |
| 83 | BP15;MMRN2;ENG;SPINT1;IGFALS;MCAM | 0.80 | 0.77 | 0.51 | 0.55 | 0.36 | 0.28 |
| 84 | BP15;ENG;SPINT1;QSOX1;IGFALS;MCAM | 0.80 | 0.76 | 0.43 | 0.59 | 0.47 | 0.25 |
| 85 | BP15;ADAM12;ENG;SPINT1;IGFALS;PIGF | 0.80 | 0.74 | 0.52 | 0.51 | 0.13 | 0.26 |
| 86 | BP15;ADAM12;IGFALS;MCAM;PIGF | 0.80 | 0.77 | 0.49 | 0.23 | 0.43 | 0.29 |
| 87 | BP15;*fihd*;ENG;SPINT1;IGFALS;MCAM | 0.80 | 0.74 | 0.62 | 0.52 | 0.18 | 0.17 |
| 88 | bmi;MMRN2;ADAM12;ENG;OSOX1;IGFALS | 0.80 | 0.74 | 0.57 | 0.43 | 0.34 | 0.28 |
| 89 | BP15;ENG;SPINT1;QSOX1;IGFALS;MCAM | 0.80 | 0.76 | 0.53 | 0.57 | 0.37 | 0.29 |
| 90 | bmi;BP15;ADAM12;ENG;MCAM;PIGF | 0.80 | 0.75 | 0.56 | 0.44 | 0.47 | 0.02 |
| 91 | BP15;ADAM12;ENG;SPINT1;IGFALS;PIGF | 0.80 | 0.74 | 0.54 | 0.49 | 0.22 | 0.37 |
| 92 | *alcoh*;BP15;ENG;SPINT1;IGFALS;MCAM | 0.80 | 0.76 | 0.55 | 0.59 | 0.55 | 0.38 |
| 93 | MMRN2;ADAM12;ENG;SPINT1;IGFALS;MCAM | 0.80 | 0.74 | 0.60 | 0.52 | 0.31 | 0.23 |
| 94 | BP15;MMRN2;ADAM12;ENG;SPINT1;IGFALS | 0.80 | 0.75 | 0.50 | 0.51 | 0.39 | 0.22 |
| 95 | BP15:ENG;SPINT1:IGFALS;MCAM;ROBO4 | 0.80 | 0.75 | 0.53 | 0.52 | 0.47 | 0.18 |
| 96 | BP15;ENG;SPINT1;QSOX1;IGFALS;MCAM | 0.80 | 0.76 | 0.46 | 0.57 | 0.46 | 0.28 |
| 97 | BP15;ADAM12;SPINT1;SEPP1;IGFALS;PIGF | 0.80 | 0.75 | 0.56 | 0.27 | 0.22 | 0.28 |
| 98 | BP15;MMRN2;ADAM12;ENG;SPINT1;IGFALS | 0.80 | 0.74 | 0.55 | 0.51 | 0.25 | 0.26 |
| 99 | BP15;ADAM12;IGFALS;ALDOA;MCAM;PIGF | 0.80 | 0.77 | 0.55 | 0.46 | 0.37 | 0.39 |

(continued)

| No. | Panel composition | A | B | C | D | E | F |
|-----|-------------------|------|------|------|------|------|------|
| 100 | BP15;ENG;SPINT1;SEPP1;IGFALS;MCAM | 0.80 | 0.76 | 0.57 | 0.57 | 0.44 | 0.27 |
| 101 | BP15;MMRN2;ADAM12;IGFALS;PlGF | 0.80 | 0.78 | 0.58 | 0.20 | 0.26 | 0.46 |
| 102 | BP15;MMRN2;ADAM12;ENG;SPINT1;IGFALS | 0.80 | 0.74 | 0.52 | 0.53 | 0.37 | 0.21 |
| 103 | BP15;ENG;SPINT1;SEPP1;IGFALS;MCAM | 0.80 | 0.76 | 0.53 | 0.55 | 0.25 | 0.16 |
| 104 | BP15;*fihd*;ENG;IGFALS;MCAM;ROBO4 | 0.80 | 0.70 | 0.57 | 0.50 | 0.21 | 0.17 |
| 105 | BP15;*fihd*;ADAM12;IGFALS;PlGF | 0.80 | 0.74 | 0.45 | 0.22 | 0.20 | 0.27 |
| 106 | bmi;BP15;ENG;SPINT1;IGFALS;MCAM | 0.80 | 0.77 | 0.42 | 0.57 | 0.23 | 0.29 |
| 107 | BP15;MMRN2;ECM1;ENG;IGFALS;MCAM | 0.80 | 0.74 | 0.56 | 0.47 | 0.13 | 0.24 |
| 108 | *alcoh*;BP15;MAPRE1/3;IGFALS;ALDOA;PlGF | 0.80 | 0.75 | 0.63 | 0.10 | 0.34 | 0.33 |
| 109 | BP15;MMRN2;MAPRE1/3;IGFALS;ALDOA;PlGF | 0.80 | 0.77 | 0.58 | 0.48 | 0.02 | 0.29 |
| 110 | bmi;BP15;ADAM12;ENG;SPINT1;MCAM | 0.80 | 0.76 | 0.52 | 0.55 | 0.34 | 0.13 |
| 111 | BP15;ADAM12;IGFALS;MCAM;PlGF | 0.80 | 0.78 | 0.49 | 0.42 | 0.38 | 0.33 |
| 112 | BP15;ENG;SPINT1;IGFALS;MCAM;PlGF | 0.80 | 0.76 | 0.56 | 0.52 | 0.42 | 0.31 |
| 113 | BP15;*fihd*;ADAM12;ECM1;MCAM;PlGF | 0.80 | 0.74 | 0.58 | 0.21 | 0.35 | 0.25 |
| 114 | BP15;MMRN2;ECM1;ENG;IGFALS;MCAM | 0.80 | 0.75 | 0.53 | 0.49 | 0.07 | 0.25 |
| 115 | BP15;*fihd*;MMRN2;ENG;IGFALS;MCAM | 0.80 | 0.74 | 0.56 | 0.48 | 0.28 | 0.12 |
| 116 | BP20;ADAM12;SEPP1;IGFALS;MCAM;PlGF | 0.80 | 0.78 | 0.59 | 0.54 | 0.46 | 0.38 |
| 117 | bmi;BP15;ADAM12;ENG;IGFALS;MCAM | 0.80 | 0.76 | 0.46 | 0.54 | 0.37 | 0.12 |
| 118 | BP15;*fihd*;ADAM12;IGFALS;PlGF | 0.80 | 0.74 | 0.46 | 0.20 | 0.27 | 0.28 |
| 119 | BP15;ENG;SPINT1;SEPP1;IGFALS;MCAM | 0.80 | 0.76 | 0.58 | 0.52 | 0.39 | 0.18 |
| 120 | BP15;ADAM12;SPINT1;SEPP1;IGFALS;PlGF | 0.80 | 0.74 | 0.49 | 0.58 | 0.20 | 0.26 |
| 121 | MMRN2;ADAM12;IGFALS;MCAM;PlGF | 0.80 | 0.78 | 0.40 | 0.40 | 0.44 | 0.30 |
| 122 | BP15;MMRN2;ADAM12;IGFALS;PlGF | 0.80 | 0.77 | 0.50 | 0.24 | 0.22 | 0.37 |
| 123 | MMRN2;ENG;SPINT1;IGFALS;MCAM;PlGF | 0.80 | 0.76 | 0.57 | 0.50 | 0.34 | 0.27 |
| 124 | *fihd*;MMRN2;ENG;SPINT1;IGFALS;MCAM | 0,80 | 0.74 | 0.60 | 0.50 | 0.41 | 0.19 |
| 125 | bmi;*fhpet*;ADAM12;ENG;MCAM;PlGF | 0.80 | 0.74 | 0.51 | 0.56 | 0.30 | 0.01 |
| 126 | BP15;ADAM12;ECM1;SPINT1;MCAM;PlGF | 0.80 | 0.74 | 0.56 | 0.45 | 0.32 | 0.17 |
| 127 | MMRN2;ADAM12;ENG;SPINT1;IGFALS;PlGF | 0.80 | 0.74 | 0.54 | 0.47 | 0.24 | 0.26 |
| 128 | BP15;ENG;SPINT1;IGFALS;MCAM | 0.80 | 0.75 | 0.57 | 0.57 | 0.38 | 0.27 |
| 129 | BP15;MMRN2;ENG;IGFALS;MCAM;*vagbl* | 0.80 | 0.74 | 0.51 | 0.52 | 0.21 | 0.30 |
| 130 | BP15;*fihd*;ADAM12;IGFALS;PlGF | 0.80 | 0.73 | 0.47 | 0.20 | 0.21 | 0.24 |
| 131 | BP15;ENG;SPINT1;IGFALS;MCAM;ROBO4 | 0.80 | 0.75 | 0.59 | 0.50 | 0.43 | 0.25 |
| 132 | *fihd*;ADAM12;IGFALS;MCAM;PlGF | 0.80 | 0.74 | 0.51 | 0.23 | 0.38 | 0.30 |
| 133 | *fihd*;ADAM12;ENG;SPINT1;IGFALS;MCAM | 0.80 | 0.73 | 0.53 | 0.52 | 0.18 | 0.18 |
| 134 | BP15;ENG;LNPEP;SPINT1;IGFALS;MCAM | 0.80 | 0.75 | 0.55 | 0.55 | 0.47 | 0.28 |
| 135 | *alcoh*;BP15;ADAM12;QSOX1;IGFALS;PlGF | 0.80 | 0.76 | 0.60 | 0.10 | 0.32 | 0.31 |
| 136 | BP20;ADAM12;SPINT1;SEPP1;IGFALS;PlGF | 0.80 | 0.76 | 0.57 | 0.49 | 0.30 | 0.14 |
| 137 | BP15;ADAM12;SPINT1;SEPP1;IGFALS;PlGF | 0.80 | 0.76 | 0.55 | 0.27 | 0.22 | 0.31 |

(continued)

| No. | Panel composition | A | B | C | D | E | F |
|---|---|---|---|---|---|---|---|
| 138 | ADAM12;SPINT1;IGFALS;MCAM;PlGF | 0.80 | 0.74 | 0.51 | 0.34 | 0.27 | 0.30 |
| 139 | bmi;BP20;ADAM12;ENG;SPINT1;QSOX1 | 0.80 | 0.74 | 0.55 | 0.51 | 0.35 | 0.09 |
| 140 | BP15;MMRN2;ADAM12;IGFALS;PlGF | 0.80 | 0.78 | 0.53 | 0.31 | 0.25 | 0.39 |
| 141 | BP15;ENG;SPINT1;QSOX1:IGFALS;MCAM | 0.80 | 0.76 | 0.48 | 0.52 | 0.47 | 0.27 |
| 142 | BP20;MMRN2;ADAM12;ENG;SPINT1;IGFALS | 0.80 | 0.75 | 0.59 | 0.44 | 0.15 | 0.26 |
| 143 | alcoh;BP15;MMRN2;ENG;IGFALS;MCAM | 0.80 | 0.75 | 0.58 | 0.50 | 0.47 | 0.15 |
| 144 | BP15;ADAM12;ENG;SPINT1;QSOX1;IGFALS | 0.80 | 0.75 | 0.50 | 0.51 | 0.43 | 0.13 |
| 145 | BP20;ECM1;ENG;IGFALS;MCAM;PlGF | 0.80 | 0.75 | 0.55 | 0.47 | 0.31 | 0.04 |
| 146 | BP15;ADAM12;SEPP1;IGFALS;PlGF | 0.80 | 0.76 | 0.45 | 0.18 | 0.20 | 0.27 |
| 147 | bmi;BP15;ADAM12;ENG;SPINT1;PlGF | 0.80 | 0.74 | 0.51 | 0.51 | 0.27 | 0.13 |
| 148 | BP15;MMRN2;ADAM12;ENG;SPINT1;IGFALS | 0.79 | 0.73 | 0.53 | 0.51 | 0.31 | 0.21 |
| 149 | BP15;fihd;ADAM12;ENG;SPINT1;IGFALS | 0.79 | 0.72 | 0.50 | 0.51 | 0.20 | 0.16 |
| 150 | bmi;BP15;ENG;QSOX1;IGFALS;MCAM | 0.79 | 0.74 | 0.49 | 0.52 | 0.43 | 0.01 |
| 151 | BP20;ENG;SPINT1;SEPP1;IGFALS;MCAM | 0.79 | 0.78 | 0.51 | 0.57 | 0.26 | 0.09 |
| 152 | BP15;ENG;SPINT1;QSOX1;IGFALS;MCAM | 0.79 | 0.77 | 0.42 | 0.59 | 0.52 | 0.16 |
| 153 | BP20;fihd;ADAM12;IGFALS;PlGF | 0.79 | 0.75 | 0.45 | 0.14 | 0.23 | 0.37 |
| 154 | BP15;ADAM12;ECM1;ENG;SPINT1;MCAM | 0.79 | 0.75 | 0.56 | 0.33 | 0.40 | 0.23 |
| 155 | BP20;ADAM12;ECM1;SPRINT1;MCAM;PlGF | 0.79 | 0.75 | 0.48 | 0.53 | 0.38 | 0.14 |
| 156 | BP20;fihd;ENG;SPINT1;IGFALS;MCAM | 0.79 | 0.76 | 0.50 | 0.57 | 0.22 | 0.18 |
| 157 | BP15;ENG;SPINT1;IGFALS;MCAM;ROBO4 | 0.79 | 0.75 | 0.52 | 0.57 | 0.33 | 0.22 |
| 158 | alcoh;BP20;fihd;MMRN2;ENG;IGFALS | 0.79 | 0.74 | 0.57 | 0.42 | 0.31 | 0.16 |
| 159 | BP15;ADAM12;ENG;SPINT1;IGFALS;PlGF | 0.79 | 0.74 | 0.54 | 0.51 | 0.19 | 0.37 |
| 160 | BP15;ADAM12;ENG;SEPP1;IGFALS;MCAM | 0.79 | 0.73 | 0.54 | 0.52 | 0.27 | 0.15 |
| 161 | BP20;MMRN2;ENG;SPINT1;IGFALS;MCAM | 0.79 | 0.78 | 0.49 | 0.57 | 0.47 | 0.17 |
| 162 | alcoh;BP15;MMRN2;ENG;SPINT1;IGFALS | 0.79 | 0.75 | 0.53 | 0.51 | 0.36 | 0.27 |
| 163 | BP15;MMRN2;ADAM12;ECM1;IGFALS;MCAM | 0.79 | 0.78 | 0.55 | 0.51 | 0.30 | 0.38 |
| 164 | BP20;ENG;SPINT1;IGFALS;MCAM;PlGF | 0.79 | 0.77 | 0.61 | 0.57 | 0.27 | 0.19 |
| 165 | BP15;ADAM12;ECM1;SPINT1;MCAM;PlGF | 0.79 | 0.74 | 0.57 | 0.30 | 0.37 | 0.17 |
| 166 | BP15;fihd;ADAM12;ENG;SPINT1;IGFALS | 0.79 | 0.72 | 0.51 | 0.51 | 0.26 | 0.15 |
| 167 | BP15;ADAM12;SPINT1;IGFALS;PlGF | 0.79 | 0.74 | 0.51 | 0.38 | 0.21 | 0.30 |
| 168 | bmi;BP15;ADAM12;pbwgt;PlGF | 0.79 | 0.74 | 0.49 | 0.25 | 0.27 | 0.16 |
| 169 | BP15;ENG;SPINT1;IGFALS;MCAM | 0.79 | 0.75 | 0.54 | 0.59 | 0.40 | 0.20 |
| 170 | bmi;fhpet;ENG;SPINT1;SEPP1;IGFALS | 0.79 | 0.76 | 0.58 | 0.42 | 0.28 | 0.25 |
| 171 | alcoh;MMRN2;ADAM12;IGFALS;PlGF | 0.79 | 0.76 | 0.54 | 0.29 | 0.52 | 0.38 |
| 172 | alcoh;BP20;fihd;ENG;SPINT1;IGFALS | 0.79 | 0.74 | 0.49 | 0.51 | 0.30 | 0.17 |
| 173 | MMRN2;ADAM12;ENG;SPINT1;G2SOX1;IGFALS | 0.79 | 0.73 | 0.54 | 0.51 | 0.27 | 0.23 |
| 174 | fhpet;ENG;SPINT1;IGFALS;MCAM;PlGF | 0.79 | 0.74 | 0.56 | 0.45 | 0.34 | 0.27 |
| 175 | alcoh;BP15;MMRN2;ENG;SPINT1;IGFALS | 0.79 | 0.76 | 0.47 | 0.58 | 0.40 | 0.38 |

(continued)

| No. | Panel composition | A | B | C | D | E | F |
|-----|-------------------|---|---|---|---|---|---|
| 176 | BP20;ADAM12;ENG;SPINT1;IGFALS;MCAM | 0.79 | 0.75 | 0.49 | 0.57 | 0.33 | 0.26 |
| 177 | *fihd*;MMRN2;ADAM12;IGFALS;PIGF | 0.79 | 0.74 | 0.40 | 0.29 | 0.36 | 0.39 |
| 178 | BP15;ADAM12;SPINT1;IGFALS;MCAM | 0.79 | 0.74 | 0.43 | 0.39 | 0.36 | 0.29 |
| 179 | BP15;ENG;SPINT1;IGFALS;MCAM;ROBO4 | 0.79 | 0.75 | 0.50 | 0.57 | 0.39 | 0.28 |
| 180 | BP15;ENG;SPINT1;QSOX1;IGFALS;MCAM | 0.79 | 0.76 | 0.52 | 0.50 | 0.46 | 0.27 |
| 181 | BP15;ENG;SPINT1;QSOX1;IGFALS;MCAM | 0.79 | 0.77 | 0.42 | 0.57 | 0.48 | 0.20 |
| 182 | BP15;ADAM12;IGFALS;MCAM;PIGF | 0.79 | 0.78 | 0.52 | 0.46 | 0.40 | 0.50 |
| 183 | BP15;MMR,N2;ADAM12;IGFALS;PIGF | 0.79 | 0.78 | 0.52 | 0.37 | 0.33 | 0.52 |
| 184 | bmi;*fhpet*;ADAM12;ENG;SPINT1;MCAM | 0.79 | 0.76 | 0.55 | 0.48 | 0.40 | 0.07 |
| 185 | BP15;*fihd*;MMRN2;IGFALS;PIGF | 0.79 | 0.73 | 0.54 | 0.34 | 0.00 | 0.29 |
| 186 | bmi;BP15;ENG;SPINT1;IGFALS;MCAM | 0.79 | 0.77 | 0.45 | 0.59 | 0.28 | 0.17 |
| 187 | BP15;MMRN2;ADAM12;ENG;IGFALS;MCAM | 0.79 | 0.77 | 0.53 | 0.50 | 0.27 | 0.35 |
| 188 | bmi;*fhpet*;ENG;SPINT1;QSOX1;IGFALS | 0.79 | 0.76 | 0.55 | 0.47 | 0.48 | 0.28 |
| 189 | BP20;MMRN2;ENG;IGFALS;MCAM;PIGF | 0.79 | 0.77 | 0.53 | 0.42 | 0.42 | 0.12 |
| 190 | BP20;*fihd*;IGFALS;MCAM;PIGF | 0.79 | 0.74 | 0,49 | 0.40 | 0.01 | 0.29 |
| 191 | BP15;*fihd*;MMRN2;ENG;SPINT1;IGFALS | 0.79 | 0.74 | 0.54 | 0.49 | 0.41 | 0.21 |
| 192 | BP15;MMRN2;MAPRE1/3;IGFALS;ALDOA;PIGF | 0.79 | 0.77 | 0.60 | 0.40 | 0.02 | 0.28 |
| 193 | BP15;ADAM12;ENG;SPINT1;QSOX1;IGFALS | 0.79 | 0.74 | 0.49 | 0.51 | 0.54 | 0.19 |
| 194 | BP20;*fihd*;ENG;SPINT1;IGFALS;MCAM | 0.79 | 0.75 | 0.55 | 0.52 | 0.25 | 0.18 |
| 195 | BP20;*fihd*;IGFALS;MGAM;PIGF | 0.79 | 0.74 | 0.48 | 0.29 | 0.01 | 0.30 |
| 196 | BP20;ADAM12;IGFALS;MCAM;PIGF | 0.79 | 0.79 | 0.53 | 0.42 | 0.39 | 0.37 |
| 197 | *alcoh*;BP15;ADAM12;IGFALS;PIGF | 0.79 | 0.74 | 0.55 | 0.16 | 0.33 | 0.35 |
| 198 | BP15;ADAM12;SPINT1;IGFALS;PIGF | 0.79 | 0.74 | 0.48 | 0.44 | 0.19 | 0.29 |
| 199 | BP15;ADAM12;SEPP1;IGFALS;PIGF | 0.79 | 0.75 | 0.51 | 0.16 | 0.16 | 0.27 |
| 200 | *alcoh*;MMRN2;ENG;SPINT1;IGFALS;MCAM | 0.79 | 0.76 | 0.52 | 0.55 | 0.34 | 0.30 |
| 201 | BP15;ADAM12;SPINT1;IGFALS;PIGF | 0.79 | 0.74 | 0.54 | 0.40 | 0.23 | 0.22 |
| 202 | *gest*;ENG;SPINT1;IGFALS;MCAM;PIGF | 0.79 | 0.74 | 0.54 | 0.50 | 0.31 | 0.20 |
| 203 | BP15;MMRN2;ENG;IGFALS;MCAM | 0.79 | 0.74 | 0.43 | 0.40 | 0.32 | 0.15 |
| 204 | *alcoh*;*fhpet*;ADAM12;QSOX1;IGFALS;PIGF | 0.79 | 0.74 | 0.64 | 0.33 | 0.25 | 0.35 |
| 205 | BP15;ADAM12;ECM1;MCAM;PIGF | 0.79 | 0.74 | 0.51 | 0.30 | 0.25 | 0.27 |
| 206 | BP15;ADAM12;SPINT1;IGFALS;PIGF | 0.79 | 0.73 | 0.51 | 0.22 | 0.24 | 0.23 |
| 207 | BP20;*fihd*;MMRN2;ENG;IGFALS;MCAM | 0.79 | 0.75 | 0.58 | 0.50 | 0.07 | 0.15 |
| 208 | BP20;MMRN2;ENG;SPINT1;IGFALS;MCAM | 0.79 | 0.78 | 0.54 | 0.61 | 0.18 | 0.20 |
| 209 | BP15;ADAM12;ENG;SPINT1;MCAM | 0.79 | 0.75 | 0.40 | 0.23 | 0.38 | 0.21 |
| 210 | *fhpet*;MMRN2;ENG;SPINT1;IGFALS;MCAM | 0.79 | 0.75 | 0.52 | 0.48 | 0.46 | 0.32 |
| 211 | *alcoh*;BP20;ENG;SPINT1;IGFALS;MCAM | 0.79 | 0.78 | 0.46 | 0.61 | 0.24 | 0.27 |
| 212 | BP15;MMRN2;ENG;IGFALS;MCAM | 0.79 | 0.74 | 0.39 | 0.44 | 0.02 | 0.28 |
| 213 | *fihd*;ENG;SPINT1;QSOX1;IGFALS;MCAM | 0.79 | 0.74 | 0.54 | 0.50 | 0.31 | 0.18 |

(continued)

| No. | Panel composition | A | B | C | D | E | F |
|-----|-------------------|---|---|---|---|---|---|
| 214 | BP15;MMRN2;ADAM12;ENG;SPINT1;IGFALS | 0.79 | 0.73 | 0.54 | 0.51 | 0.33 | 0.21 |
| 215 | *alcoh*;BP15;ADAM12;IGFALS;PIGF | 0.79 | 0.74 | 0.55 | 0.18 | 0.37 | 0.26 |
| 216 | BP20;*fihd*;MMRN2;ENG;SPINT1;IGFALS | 0.79 | 0.76 | 0.60 | 0.49 | 0.15 | 0.18 |
| 217 | BP15;ADAM12;MCAM;ENPP2;PIGF | 0.79 | 0.76 | 0.46 | 0.27 | 0.37 | 0.36 |
| 218 | MMRN2;ADAM12;ENG;SPINT1;QSOX1;IGFALS | 0.79 | 0.74 | 0.57 | 0.53 | 0.24 | 0.29 |
| 219 | BP20;*fihd*;MMRN2;ENG;IGFALS;MCAM | 0.79 | 0.74 | 0.53 | 0.52 | 0.06 | 0.14 |
| 220 | BP15;IGFALS;ALDOA;MCAM;PIGF | 0.79 | 0.74 | 0.59 | 0.17 | 0.39 | 0.40 |
| 221 | BP20;*fihd*;ENG;SEPP1;IGFALS;MCAM | 0.79 | 0.73 | 0.58 | 0.54 | 0.33 | 0.12 |
| 222 | *alcoh*;BP20;ADAM12;QSOX1;IGFALS;PIGF | 0.79 | 0.77 | 0.54 | 0.49 | 0.48 | 0.38 |
| 223 | BP15;ENG;SPINT1;IGFALS;MCAM | 0.79 | 0.75 | 0.58 | 0.57 | 0.37 | 0.18 |
| 224 | BP15;ENG;SPINT1;IGFALS;MCAM | 0.79 | 0.75 | 0.46 | 0.55 | 0.48 | 0.16 |
| 225 | BP15;MMRN2;ADAM12;IGFALS;MCAM | 0.79 | 0.77 | 0.48 | 0.35 | 0.46 | 0.37 |
| 226 | BP15;ADAM12;ECM1;MCAM;PIGF | 0.79 | 0.75 | 0.49 | 0.34 | 0.47 | 0.25 |
| 227 | *alcoh*;BP20;MMRN2;ENG;SPINT1;IGFALS | 0.79 | 0.77 | 0.50 | 0.58 | 0.38 | 0.30 |
| 228 | BP20;ADAM12;ENG;SPINT1;IGFALS;MCAM | 0.79 | 0.75 | 0.51 | 0.57 | 0.31 | 0.21 |
| 229 | *alcoh*;BP15;ADAM12;IGFALS;MCAM | 0.79 | 0.75 | 0.52 | 0.27 | 0.25 | 0.37 |
| 230 | *alcoh*;BP15;MMRN2;ENG;IGFALS;MCAM | 0.79 | 0.75 | 0.51 | 0.52 | 0.47 | 0.31 |
| 231 | ADAM12;ENG;SPINT1;QSOX1;IGFALS;MCAM | 0.79 | 0.74 | 0.53 | 0.50 | 0.25 | 0.21 |
| 232 | *alcoh*;BP20;ENG;SPINT1;QSOX1;IGFALS | 0.79 | 0.76 | 0.54 | 0.47 | 0.42 | 0.33 |
| 233 | BP20;*fihd*;MMRN2;ENG;SPINT1;IGFALS | 0.79 | 0.75 | 0.60 | 0.47 | 0.32 | 0.19 |
| 234 | BP15;ADAM12;SPINT1;IGFALS;MCAM | 0.79 | 0.74 | 0.43 | 0.36 | 0.38 | 0.30 |
| 235 | *alcoh*;BP15;ADAM12;IGFALS;PIGF | 0.79 | 0.75 | 0.55 | 0.14 | 0.36 | 0.38 |
| 236 | BP20;MMRN2;ADAM12;IGFALS;PIGF | 0.79 | 0.79 | 0.48 | 0.39 | 0.19 | 0.35 |
| 237 | BP15;MMRN2;ADAM12;IGFALS;PIGF | 0.79 | 0.77 | 0.45 | 0.39 | 0.20 | 0.35 |
| 238 | BP20;MMRN2;ADAM12;IGFALS;PIGF | 0.79 | 0.79 | 0.46 | 0.43 | 0.31 | 0.42 |
| 239 | bmi;BP15;*fhpet*;ADAM12;ENG;SPINT1 | 0.79 | 0.73 | 0.51 | 0.51 | 0.40 | 0.12 |
| 240 | BP15;ADAM12;SPINT1;MCAM;PIGF | 0.79 | 0.74 | 0.49 | 0.14 | 0.34 | 0.16 |
| 241 | MMRN2;ENG;SPINT1;IGFALS;MCAM;ENPP2 | 0.79 | 0.76 | 0.54 | 0.48 | 0.44 | 0.25 |
| 242 | BP15;ADAM12;ENG;SPINT1;MCAM | 0.79 | 0.74 | 0.46 | 0.27 | 0.20 | 0.13 |
| 243 | BP15;*fihd*;ADAM12;IGFALS;MCAM | 0.79 | 0.73 | 0.53 | 0.33 | 0.34 | 0.29 |
| 244 | *alcoh*;BP15;ADAM12;IGFALS;PIGF | 0.79 | 0.73 | 0.55 | 0.20 | 0.34 | 0.27 |
| 245 | *alcoh*;BP20;ENG;SPINT1;QSOX1;IGFALS | 0.79 | 0.76 | 0.59 | 0.42 | 0.32 | 0.36 |
| 246 | BP15;*fihd*;ENG;IGFALS;MCAM;ROBO4 | 0.79 | 0.72 | 0.52 | 0.50 | 0.26 | 0.15 |
| 247 | BP20;*fihd*;ADAM12;IGFALS;PIGF | 0.79 | 0.74 | 0.49 | 0.18 | 0.32 | 0.38 |
| 248 | BP20;MMRN2;ENG;SPINT1;IGFALS;MCAM | 0.79 | 0.77 | 0.51 | 0.55 | 0.46 | 0.19 |
| 249 | BP15;ADAM12;ECM1;MCAM;PIGF | 0.79 | 0.75 | 0.51 | 0.29 | 0.28 | 0.17 |
| 250 | BP15;ADAM12;SPINT1;IGFALS;MCAM | 0.79 | 0.73 | 0.43 | 0.36 | 0.36 | 0.27 |
| 251 | BP15;ENG;SPINT1;IGFALS;MCAM;ROBO4 | 0.79 | 0.76 | 0.49 | 0.55 | 0.46 | 0.20 |

(continued)

| No. | Panel composition | A | B | C | D | E | F |
|---|---|---|---|---|---|---|---|
| 252 | BP15;*fihd*;ADAM12;IGFALS;PIGF | 0.79 | 0.73 | 0.39 | 0.18 | 0.24 | 0.24 |
| 253 | *alcoh*;BP20;*fihd*;IGFALS;PIGF | 0.79 | 0.74 | 0.50 | 0.14 | 0.37 | 0.39 |
| 254 | *alcoh*;BP20;MMRN2;ENG;SPINT1;IGFALS | 0.79 | 0.77 | 0.53 | 0.51 | 0.33 | 0.36 |
| 255 | BP20;ADAM12;SEPP1;IGFALS;PIGF | 0.79 | 0.78 | 0.44 | 0.35 | 0.36 | 0.13 |
| 256 | *alcoh*;BP15;ADAM12;IGFALS;MCAM | 0.79 | 0.74 | 0.37 | 0.21 | 0.20 | 0.39 |
| 257 | ADAM12;ENG;SPINT1;QSOX1;IGFALS;MCAM | 0.79 | 0.75 | 0.53 | 0.50 | 0.20 | 0.24 |
| 258 | bmi;BP15;*fhpet*;ENG;IGFALS;MCAM | 0.79 | 0.73 | 0.52 | 0.50 | 0.30 | 0.01 |
| 259 | BP15;ADAM12;ECM1;MCAM;PIGF | 0.79 | 0.74 | 0.56 | 0.30 | 0.45 | 0.25 |
| 260 | BP15;ENG;LNPEP;SPINT1;IGFALS;MCAM | 0.79 | 0.75 | 0.49 | 0.57 | 0.46 | 0.27 |
| 261 | BP20;ADAM12;SPINT1;SEPP1;IGFALS;PIGF | 0.79 | 0.75 | 0.54 | 0.49 | 0.25 | 0.14 |
| 262 | BP20;ADAM12;IGFALS;MCAM;PIGF | 0.79 | 0.78 | 0.58 | 0.42 | 0.43 | 0.41 |
| 263 | *alcoh*;BP15;*fhpet*;ENG;IGFALS;MCAM | 0.79 | 0.74 | 0.59 | 0.52 | 0.32 | 0.15 |
| 264 | BP15;MMRN2;ENG;1GFALS;MCAM;*vagbl* | 0.79 | 0.76 | 0.52 | 0.52 | 0.33 | 0.17 |
| 265 | bmi;BP20;ADAM12;ENG;MCAM;PIGF | 0.79 | 0.76 | 0.49 | 0.52 | 0.35 | 0.20 |
| 266 | BP15;ADAM12;SEPP1;IGFALS;PIGF | 0.79 | 0.75 | 0.36 | 0.24 | 0.12 | 0.28 |
| 267 | *alcoh*;BP20;*fihd*;ADAM12;IGFALS;ROBO4 | 0.79 | 0.75 | 0.52 | 0.51 | 0.31 | 0.19 |
| 268 | BP20;ENG;SPINT1;IGFALS;MCAM;PIGF | 0.79 | 0.76 | 0.55 | 0.48 | 0.34 | 0.17 |
| 269 | BP15;MMRN2;ENG;IGFALS;MCAM | 0.79 | 0.74 | 0.48 | 0.50 | 0.20 | 0.24 |
| 270 | BP20;ENG;SPINT1;IGFALS;MCAM;PIGF | 0.79 | 0.76 | 0.60 | 0.52 | 0.28 | 0.19 |
| 271 | BP15;MMRN2;ADAM12;SPINT1;IGFALS | 0.79 | 0.73 | 0.47 | 0.31 | 0.37 | 0.28 |
| 272 | bmi;*fhpet*;ENG;SPINT1;IGFALS;MCAM | 0.79 | 0.77 | 0.48 | 0.52 | 0.46 | 0.21 |
| 273 | BP15;ADAM12;ECM1;MCAM;PIGF | 0.79 | 0.75 | 0.51 | 0.38 | 0.40 | 0.26 |
| 274 | *alcoh*;ADAM12;IGFALS;MCAM;PIGF | 0.79 | 0.75 | 0.48 | 0.21 | 0.43 | 0.30 |
| 275 | *alcoh*;BP20;BNG;SEPP1;IGFALS;MCAM | 0.79 | 0.75 | 0.49 | 0.54 | 0.37 | 0.04 |
| 276 | BP15;ECM1;IGFALS;MCAM;PIGF | 0.79 | 0.74 | 0.49 | 0.23 | 0.20 | 0.38 |
| 277 | bmi;*fhpet*;ENG;SPINT1;IGFALS;ROBO4 | 0.79 | 0.74 | 0.55 | 0.47 | 0.27 | 0.27 |
| 278 | *alcoh*;BP20;MMRN2;ENG;QSOX1;IGFALS | 0.79 | 0.76 | 0.52 | 0.55 | 0.48 | 0.02 |
| 279 | BP15;ENG;SPINT1;IGFALS;MCAM | 0.79 | 0.75 | 0.55 | 0.55 | 0.26 | 0.18 |
| 280 | *alcoh*;BP20;ADAM12;IGFALS;PIGF | 0.79 | 0.77 | 0.49 | 0.37 | 0.38 | 0.36 |
| 281 | *alcoh*;ENG;SPINT1;SEPP1;IGFALS;MCAM | 0.79 | 0.75 | 0.55 | 0.45 | 0.25 | 0.17 |
| 282 | BP15;ADAM12;ENG;SPINT1;QSOX1;IGFALS | 0.79 | 0.74 | 0.49 | 0.51 | 0.34 | 0.31 |
| 283 | BP15;*fihd*;MMRN2;ADAM12;IGFALS | 0.79 | 0.74 | 0.53 | 0.22 | 0.33 | 0.37 |
| 284 | bmi;*fihd*;MMRN2;ADAM12;ENG;MCAM | 0.79 | 0.72 | 0.54 | 0.50 | 0.43 | 0.01 |
| 285 | BP20;ENG;SPINT1;SEPP1;IGFALS;MCAM | 0.79 | 0.77 | 0.55 | 0.55 | 0.27 | 0.09 |
| 286 | BP20;ENG;SPINT1;SEPP1;IGFALS;MCAM | 0.79 | 0.77 | 0.59 | 0.57 | 0.28 | 0.17 |
| 287 | BP15;ADAM12;SEPP1;IGFALS;MCAM | 0.79 | 0.75 | 0.52 | 0.19 | 0.42 | 0.30 |
| 288 | BP15;ADAM12;ECM1;MCAM;PIGF | 0.79 | 0.74 | 0.58 | 0.29 | 0.31 | 0.18 |
| 289 | bmi;MMRN2;ENG;SPINT1;IGFALS;MCAM | 0.79 | 0.78 | 0.47 | 0.55 | 0.37 | 0.30 |

(continued)

| No. | Panel composition | A | B | C | D | E | F |
|---|---|---|---|---|---|---|---|
| 290 | *alcoh*;BP20;*fihd*;MMRN2;BGFALS | 0.79 | 0.75 | 0.48 | 0.36 | 0.47 | 0.36 |
| 291 | BP20;LNPEP;SPINT1;IGFALS;MCAM;PIGF | 0.79 | 0.76 | 0.56 | 0.52 | 0.31 | 0.51 |
| 292 | BP15;ENG;SPINT1;IGFALS;MCAM | 0.79 | 0.75 | 0.52 | 0.52 | 0.45 | 0.31 |
| 293 | BP15;ADAM12;MCAM;ENPP2;PIGF | 0.79 | 0.76 | 0.46 | 0.23 | 0.44 | 0.32 |
| 294 | BP20;*fihd*;MMRN2;ENG;SPINT1;IGFALS | 0.79 | 0.75 | 0.58 | 0.49 | 0.21 | 0.25 |
| 295 | BP15;MMRN2;ADAM12;SPINT1;IGFALS | 0.79 | 0.74 | 0.53 | 0.29 | 0.30 | 0.39 |
| 296 | *alcoh*;BP15;ADAM12;IGFALS;MCAM | 0.79 | 0.75 | 0.38 | 0.25 | 0.24 | 0.26 |
| 297 | *alcoh*;ENG;SPINT1;QSOX1;IGFALS;MCAM | 0.79 | 0.76 | 0.46 | 0.55 | 0.40 | 0.25 |
| 298 | BP15;ADAM12;SPINT1;IGFALS;PIGF | 0.79 | 0.74 | 0.53 | 0.44 | 0.27 | 0.21 |
| 299 | *alcoh*;BP20;ADAM12;ECM1;MCAM;PIGF | 0.79 | 0.77 | 0.54 | 0.49 | 0.25 | 0.11 |
| 300 | BP15;ADAM12;SPINT1;MCAM;PIGF | 0.79 | 0.74 | 0.49 | 0.27 | 0.32 | 0.16 |
| 301 | *gest*;MMRN2;ENG;SPINT1;IGFALS;MCAM | 0.79 | 0.75 | 0.47 | 0.55 | 0.41 | 0.24 |
| 302 | BP15;MMRN2;ENG;SPINT1;IGFALS;PIGF | 0.79 | 0.76 | 0.51 | 0.56 | 0.01 | 0.32 |
| 303 | *alcoh*;BP20;ENG;SPINT1;IGFALS;MCAM | 0.79 | 0.77 | 0.53 | 0.61 | 0.27 | 0.25 |
| 304 | *alcoh*;BP20;MMRN2;ENG;IGFALS;MCAM | 0.79 | 0.77 | 0.53 | 0.63 | 0.25 | 0.13 |
| 305 | bmi;BP15;ADAM12;ENG;QSOX1;MCAM | 0.79 | 0.75 | 0.48 | 0.52 | 0.34 | 0.12 |
| 306 | *alcoh*;BP20;MMRN2;ENG;SEPP1;IGFALS | 0.79 | 0.76 | 0.51 | 0.52 | 0.36 | 0.07 |
| 307 | *alcoh*;BP20;*fihd*;ADAM12;IGFALS | 0.79 | 0.73 | 0.54 | 0.22 | 0.34 | 0.14 |
| 308 | BP15;ADAM12;ENG;SPINT1;MCAM;ENPP2 | 0.79 | 0.75 | 0.52 | 0.48 | 0.36 | 0.26 |
| 309 | bmi;ENG;SPINT1;IGFALS;MCAM;PIGF | 0.79 | 0.77 | 0.53 | 0.43 | 0.21 | 0.26 |
| 310 | BP15;LNPEP;IGFALS;MCAM;PIGF | 0.79 | 0.75 | 0.48 | 0.40 | 0.40 | 0.36 |
| 311 | BP15;IGFALS;MCAM;PIGF | 0.79 | 0.73 | 0.42 | 0.27 | 0.39 | 0.29 |
| 312 | BP20;ADAM12;ECM1;ENG;SPINT1;MCAM | 0.79 | 0.75 | 0.47 | 0.60 | 0.40 | 0.27 |
| 313 | *alcoh*;BP20;MMRN2;ENG;SPINT1;IGFALS | 0.79 | 0.76 | 0.50 | 0.56 | 0.43 | 0.31 |
| 314 | *alcoh*;BP20;ENG;SPINT1;IGFALS;MCAM | 0.79 | 0.77 | 0.48 | 0.59 | 0.21 | 0.27 |
| 315 | ENG;SPINT1;SEPP1;IGFALS;MCAM;PIGF | 0.79 | 0.75 | 0.58 | 0.50 | 0.26 | 0.18 |
| 316 | BP15;IGFALS;MCAM;PIGF | 0.79 | 0.73 | 0.41 | 0.27 | 0.31 | 0.27 |
| 317 | *alcoh*;BP15;IGFALS;MCAM;PIGF | 0.79 | 0.74 | 0.57 | 0.13 | 0.32 | 0.37 |
| 318 | bmi;*fhpet*;ADAM12;IGFALS;PIGF | 0.79 | 0.75 | 0.45 | 0.29 | 0.36 | 0.36 |
| 319 | ADAM12;SEPP1;IGFALS;MCAM;PIGF | 0.79 | 0.76 | 0.49 | 0.44 | 0.39 | 0.27 |
| 320 | *age*;BP15;MMRN2;ENG;SPINT1;IGFALS | 0.79 | 0.75 | 0.50 | 0.51 | 0.45 | 0.30 |
| 321 | BP20;MMRN2;ECM1;ENG;IGFALS;MCAM | 0.79 | 0.78 | 0.49 | 0.55 | 0.20 | 0.12 |
| 322 | *alcoh*;BP20;*fihd*;MMRN2;IGFALS | 0.79 | 0.74 | 0.48 | 0.02 | 0.47 | 0.18 |
| 323 | *alcoh*;BP20;ADAM12;ECM1;MCAM;PIGF | 0.79 | 0.77 | 0.54 | 0.50 | 0.24 | 0.05 |
| 324 | bmi;BP15;ADAM12;ENG;MCAM | 0.79 | 0.74 | 0.35 | 0.33 | 0.36 | 0.01 |
| 325 | MMRN2;ENG;SEPP1;IGFALS;MCAM;PIGF | 0.79 | 0.74 | 0.51 | 0.50 | 0.27 | 0.18 |
| 326 | BP15;ADAM12;ENG;IGFALS;MCAM;ROBO4 | 0.79 | 0.74 | 0.54 | 0.46 | 0.42 | 0.15 |
| 327 | MMRN2;ENG;SPINT1;IGFALS;MCAM;*vagbl* | 0.79 | 0.76 | 0.51 | 0.52 | 0.43 | 0.32 |

(continued)

| No. | Panel composition | A | B | C | D | E | F |
|---|---|---|---|---|---|---|---|
| 328 | bmi;ADAM12;QSOX1;IGFALS;PIGF | 0.79 | 0.76 | 0.48 | 0.22 | 0.32 | 0.45 |
| 329 | *alcoh*;BP20;ADAM12;ECM1;MCAM;PIGF | 0.79 | 0.77 | 0.45 | 0.58 | 0.32 | 0.12 |
| 330 | *alcoh*;BP15;MMRN2;ADAM12;IGFALS | 0.79 | 0.75 | 0.39 | 0.12 | 0.41 | 0.33 |
| 331 | BP15;MMRN2;ECM1;LNPEP;IGFALS;MCAM | 0.79 | 0.75 | 0.58 | 0.40 | 0.20 | 0.34 |
| 332 | *alcoh*;BP20;MMRN2;ENG;QSOX1;IGFALS | 0.79 | 0.76 | 0.56 | 0.47 | 0.43 | 0.03 |
| 333 | BP15;ADAM12;ECM1;MCAM;PIGF | 0.79 | 0.74 | 0.53 | 0.26 | 0.45 | 0.24 |
| 334 | *alcoh*;BP15;ADAM12;IGFALS;MCAM | 0.79 | 0.74 | 0.43 | 0.40 | 0.28 | 0.30 |
| 335 | BP15;ADAM12;ECM1;MCAM;PIGF | 0.79 | 0.75 | 0.53 | 0.32 | 0.21 | 0.24 |
| 336 | BP20;ENG;SPINT1;QSOX1;IGFALS;MCAM | 0.79 | 0.78 | 0.42 | 0.59 | 0.26 | 0.15 |
| 337 | *alcoh*;BP20;ADAM12;IGFALS;PIGF | 0.79 | 0.76 | 0.43 | 0.33 | 0.49 | 0.26 |
| 338 | BP15;ENG;SPINT1;SEPP1;IGFALS;PIGF | 0.79 | 0.75 | 0.54 | 0.47 | 0.07 | 0.12 |
| 339 | bmi;BP15;ENG;SPINT1;IGFALS;MCAM | 0.79 | 0.77 | 0.45 | 0.59 | 0.44 | 0.13 |
| 340 | *alcoh*;BP15;ADAM12;IGFALS;PIGF | 0.79 | 0.74 | 0.54 | 0.12 | 0.34 | 0.36 |
| 341 | BP15;MAPRE1/3;IGFALS;ALDOA;PIGF | 0.79 | 0.75 | 0.42 | 0.18 | 0.04 | 0.21 |
| 342 | BP20;ADAM12;SPINT1;IGFALS;PIGF | 0.79 | 0.74 | 0.49 | 0.31 | 0.24 | 0.24 |
| 343 | BP20;ADAM12;ECM1;MCAM;PIGF | 0.79 | 0.77 | 0.51 | 0.53 | 0.27 | 0.04 |
| 344 | BP20;*fihd*;MMRN2;ENG;IGFALS;MCAM | 0.79 | 0.75 | 0.52 | 0.52 | 0.14 | 0.16 |
| 345 | *alcoh*;MMRN2;ENG;SPINT1;IGFALS;PIGF | 0.79 | 0.74 | 0.49 | 0.51 | 0.25 | 0.25 |
| 346 | *alcoh*;BP15;ADAM12;QSOX1;IGFALS | 0.79 | 0.73 | 0.39 | 0.02 | 0.30 | 0.39 |
| 347 | BP15;MMRN2;ENG;IGFALS;MCAM | 0.79 | 0.75 | 0.50 | 0.50 | 0.33 | 0.16 |
| 348 | BP15;MMRN2;ADAM12;SPINT1;IGFALS | 0.79 | 0.74 | 0.51 | 0.36 | 0.18 | 0.29 |
| 349 | BP20;MMRN2;ENG;SPINT1;SEPP1;IGFALS | 0.78 | 0.77 | 0.55 | 0.51 | 0.13 | 0.17 |
| 350 | *alcoh*;BP15;MMRN2;ADAM12;IGFALS | 0.78 | 0.74 | 0.43 | 0.10 | 0.42 | 0.36 |
| 351 | BP20;ADAM12;SEPP1;IGFALS;PIGF | 0.78 | 0.77 | 0.33 | 0.22 | 0.33 | 0.18 |
| 352 | BP15;ADAM12;ENG;SPINT1;MCAM | 0.78 | 0.75 | 0.52 | 0.32 | 0.28 | 0.16 |
| 353 | BP20;*fihd*;LNPEP;IGFALS;PIGF | 0.78 | 0.74 | 0.48 | 0.24 | 0.25 | 0.37 |
| 354 | *alcoh*;BP15;MMRN2;ADAM12;IGFALS | 0.78 | 0.75 | 0.44 | 0.04 | 0.35 | 0.38 |
| 355 | BP15;IGFALS;MCAM;PIGF | 0.78 | 0.74 | 0.43 | 0.25 | 0.32 | 0.36 |
| 356 | BP20;*fihd*;ADAM12;ENG;IGFALS;MCAM | 0.78 | 0.74 | 0.41 | 0.54 | 0.27 | 0.25 |
| 357 | BP20;*fihd*;MMRN2;ADAM12;IGFALS | 0.78 | 0.76 | 0.52 | 0.45 | 0.25 | 0.27 |
| 358 | *gest*;MMRN2;ENG;SPINT1;SEPP1;IGFALS | 0.78 | 0.74 | 0.60 | 0.42 | 0.25 | 0.17 |
| 359 | *alcoh*;BP20;ENG;QSOX1;IGFALS;MCAM | 0.78 | 0.76 | 0.45 | 0.63 | 0.45 | 0.03 |
| 360 | BP15;ADAM12;ENG;IGFALS;MCAM | 0.78 | 0.74 | 0.40 | 0.33 | 0.28 | 0.18 |
| 361 | bmi;ADAM12;ENG;SPINT1;MCAM | 0.78 | 0.75 | 0.46 | 0.45 | 0.35 | 0.13 |
| 362 | MMRN2;ENG;IGFALS;MCAM;PIGF | 0.78 | 0.74 | 0.56 | 0.40 | 0.26 | 0.16 |
| 363 | BP15;MMRN2;ADAM12;MAPRE1/3;IGFALS;ALDO A | 0.78 | 0.76 | 0.53 | 0.48 | 0.18 | 0.36 |
| 364 | BP15;ADAM12;IGFALS;PIGF | 0.78 | 0.75 | 0.41 | 0.14 | 0.22 | 0.29 |
| 365 | bmi;*fhpet*;ADAM12;ECM1;MCAM;PIGF | 0.78 | 0.74 | 0.64 | 0.36 | 0.07 | 0.16 |

(continued)

| No. | Panel composition | A | B | C | D | E | F |
|---|---|---|---|---|---|---|---|
| 366 | BP15;*gest*;ENG;SPINT1;IGFALS;PIGF | 0.78 | 0.74 | 0.49 | 0.53 | 0.13 | 0.17 |
| 367 | BP15;MMRN2;LNPEP;IGFALS;PIGF | 0.78 | 0.76 | 0.41 | 0.22 | 0.03 | 0.37 |
| 368 | *alcoh*;BP20;MMRN2;ENG;QSOX1;IGFALS | 0.78 | 0.76 | 0.56 | 0.55 | 0.40 | 0.03 |
| 369 | BP20;ENG;SPINT1;IGFALS;MCAM;ROBO4 | 0.78 | 0.77 | 0.37 | 0.61 | 0.37 | 0.18 |
| 370 | BP15;ADAM12;ALDOA;MCAM;PIGF | 0.78 | 0.74 | 0.52 | 0.27 | 0.44 | 0.39 |
| 371 | bmi;BP15;ENG;LNPEP;SPINT1;MCAM | 0.78 | 0.74 | 0.46 | 0.55 | 0.25 | 0.03 |
| 372 | bmi;BP15;ADAM12;ENG;MCAM | 0.78 | 0.74 | 0.37 | 0.29 | 0.36 | 0.02 |
| 373 | *alcoh*;BP20;ADAM12;IGFALS;PIGF | 0.78 | 0.75 | 0.45 | 0.22 | 0.46 | 0.39 |
| 374 | *alcoh*;BP20;ENG;QSOX1;IGFALS;MCAM | 0.78 | 0.77 | 0.46 | 0.63 | 0.46 | 0.03 |
| 375 | bmi;BP20;MMRN2;ADAM12;ENG;SPINT1 | 0.78 | 0.74 | 0.57 | 0.44 | 0.21 | 0.15 |
| 376 | BP15;ADAM12;ENG;1GFALS;MCAM | 0.78 | 0.74 | 0.46 | 0.42 | 0.25 | 0.25 |
| 377 | *alcoh*;BP35;MMRN2;ADAM12;IGFALS | 0.78 | 0.76 | 0.45 | 0.10 | 0.37 | 0.41 |
| 378 | BP15;IGFALS;MCAM;PIGF | 0.78 | 0.74 | 0.37 | 0.23 | 0.29 | 0.31 |
| 379 | BP15;*fihd*;ADAM12;MCAM;PIGF | 0.78 | 0.74 | 0.51 | 0.15 | 0.28 | 0.30 |
| 380 | MMRN2;ADAM12;SEPP1;IGFALS;PIGF | 0.78 | 0.76 | 0.40 | 0.45 | 0.33 | 0.35 |
| 381 | bmi;BP20;ADAM12;ENG;IGFALS;MCAM | 0.78 | 0.76 | 0.45 | 0.54 | 0.40 | 0.30 |
| 382 | BP15;ADAM12;ENG;IGFALS;MCAM | 0.78 | 0.74 | 0.48 | 0.42 | 0.23 | 0.25 |
| 383 | BP15;ADAM12;MCAM;ENPP2;PIGF | 0.78 | 0.76 | 0.52 | 0.27 | 0.16 | 0.30 |
| 384 | BP15;ECM1;IGFALS;MCAM;PIGF | 0.78 | 0.75 | 0.54 | 0.21 | 0.26 | 0.29 |
| 385 | *alcoh*;BP20;ENG;SPINT1;IGFALS;PIGF | 0.78 | 0.74 | 0.57 | 0.49 | 0.31 | 0.15 |
| 386 | ENG;SPINT1;IGFALS;MCAM;PIGF | 0.78 | 0.74 | 0.58 | 0.50 | 0.34 | 0.28 |
| 387 | BP15;ECM1;IGFALS;MCAM;PIGF | 0.78 | 0.74 | 0.53 | 0.21 | 0.22 | 0.19 |
| 388 | BP15;ADAM12;MCAM;ENPP2;PIGF | 0.78 | 0.76 | 0.45 | 0.19 | 0.43 | 0.39 |
| 389 | BP15;ECM1;IGFALS;MCAM;PIGF | 0.78 | 0.74 | 0.53 | 0.28 | 0.20 | 0.24 |
| 390 | BP20;MMRN2;ENG;SEPP1;IGFALS;MCAM | 0.78 | 0.76 | 0.49 | 0.54 | 0.19 | 0.03 |
| 391 | BP20;ADAM12;ECM1;MCAM;PIGF | 0.78 | 0.77 | 0.45 | 0.52 | 0.31 | 0.15 |
| 392 | BP15;ADAM12;ECM1;MCAM:PIGF | 0.78 | 0.75 | 0.52 | 0.35 | 0.19 | 0.25 |
| 393 | BP15;ENG;SPINT1;SEPP1;IGFALS | 0.78 | 0.74 | 0.45 | 0.33 | 0.26 | 0.06 |
| 394 | *alcoh*;BP20;ADAM12;QSOX1;IGFALS | 0.78 | 0.76 | 0.46 | 0.39 | 0.45 | 0.23 |
| 395 | BP20;*fihd*;ADAM12;QSOX1;IGFALS | 0.78 | 0.73 | 0.42 | 0.35 | 0.28 | 0.23 |
| 396 | *alcoh*;BP20;SEPP1;IGFALS;PIGF | 0.78 | 0.76 | 0.39 | 0.32 | 0.38 | 0.48 |
| 397 | MAPRE1/3;IGFALS;ALDOA;MCAM;PIGF | 0.78 | 0.75 | 0.36 | 0.35 | 0.33 | 0.31 |
| 398 | BP20;LNPEP;IGFALS;MCAM;PIGF | 0.78 | 0.78 | 0.49 | 0.46 | 0.32 | 0.49 |
| 399 | BP15;MMRN2;ENG;IGFALS;MCAM | 0.78 | 0.73 | 0.32 | 0.50 | 0.14 | 0.26 |
| 400 | BP20;ADAM12;ECM1;MCAM;PIGF | 0.78 | 0.77 | 0.47 | 0.54 | 0.25 | 0.06 |
| 401 | BP15;ADAM12;SPINT1;MCAM;PIGF | 0.78 | 0.74 | 0.49 | 0.41 | 0.17 | 0.12 |
| 402 | BP15;MAPRE1/3;IGFALS;ALDOA;PIGF | 0.78 | 0.74 | 0.38 | 0.20 | 0.03 | 0.21 |
| 403 | bmi;BP15;ADAM12;ENG;MCAM | 0.78 | 0.74 | 0.48 | 0.25 | 0.24 | 0.02 |

(continued)

| No. | Panel composition | A | B | C | D | E | F |
|---|---|---|---|---|---|---|---|
| 404 | BP20;ADAM12;ECM1;MCAM;PIGF | 0.78 | 0.77 | 0.42 | 0.53 | 0.37 | 0.14 |
| 405 | BP15;MMRN2;ENG;SPINT1;IGFALS | 0.78 | 0.74 | 0.39 | 0.47 | 0.49 | 0.26 |
| 406 | *alcoh*;BP20;MMRN2;ENG;IGFALS;MCAM | 0.78 | 0.77 | 0.56 | 0.54 | 0.26 | 0.21 |
| 407 | BP15;MMRN2;ENG;SPINT1;SEPP1;IGFALS | 0.78 | 0.75 | 0.50 | 0.51 | 0.16 | 0.18 |
| 408 | BP20;MMRN2;ENG;SPINT1;QSOX1;IGFALS | 0.78 | 0.77 | 0.47 | 0.53 | 0.13 | 0.16 |
| 409 | bmi;ADAM12;ENG;QSOX1;MCAM;PIGF | 0.78 | 0.75 | 0.53 | 0.52 | 0.23 | 0.01 |
| 410 | BP20;*gest*;MMRN2;ENG;SPINT1;IGFALS | 0.78 | 0.76 | 0.53 | 0.51 | 0.30 | 0.19 |
| 411 | BP15;ADAM12;ALDOA;MCAM;PIGF | 0.78 | 0.74 | 0.53 | 0.23 | 0.37 | 0.27 |
| 412 | BP15:ADAM12;ECM1;MCAM;PIGF | 0.78 | 0.74 | 0.53 | 0.32 | 0.16 | 0.31 |
| 413 | BP20;MMRN2;ADAM12;SPINT1;IGFALS | 0.78 | 0.74 | 0.54 | 0.33 | 0.18 | 0.29 |
| 414 | BP15;*fhpet*;MMRN2;IGFALS;PIGF | 0.78 | 0.75 | 0.51 | 0.30 | 0.00 | 0.29 |
| 415 | *alcoh*;BP20;IGFALS;MCAM;PIGF | 0.78 | 0.76 | 0.42 | 0.44 | 0.27 | 0.40 |
| 416 | BP20;ADAM12;SEPP1;IGFALS;PIGF | 0.78 | 0.77 | 0.45 | 0.20 | 0.37 | 0.14 |
| 417 | BP15;ADAM12;IGFALS;MGAM;ENPP2 | 0.78 | 0.75 | 0.43 | 0.35 | 0.30 | 0.39 |
| 418 | bmi;BP15;ADAM12;MCAM;PIGF | 0.78 | 0.76 | 0.51 | 0.27 | 0.34 | 0.26 |
| 419 | BP15;ADAM12;ENG;IGFALS;MCAM;ROBO4 | 0.78 | 0.75 | 0.48 | 0.54 | 0.23 | 0.05 |
| 420 | BP15;*fihd*;MMRN2;ADAM12;IGFALS | 0.78 | 0.74 | 0.57 | 0.27 | 0.42 | 0.33 |
| 421 | BP20;ENG;SPINT1;IGFALS;MCAM;ROBO4 | 0.78 | 0.76 | 0.40 | 0.57 | 0.44 | 0.18 |
| 422 | *alcoh*;BP20;ENG;SEPP1;IGFALS;MCAM | 0.78 | 0.75 | 0.53 | 0.52 | 0.29 | 0.15 |
| 423 | BP20;*fihd*;MMRN2;ADAM12;IGFALS | 0.78 | 0.75 | 0.52 | 0.43 | 0.30 | 0.23 |
| 424 | BP15;ADAM12;IGFALS;MCAM;ENPP2 | 0.78 | 0.75 | 0.43 | 0.27 | 0.29 | 0.36 |
| 425 | *alcoh*;BP20;*fihd*;MMRN2;IGFALS | 0.78 | 0.74 | 0.49 | 0.34 | 0.36 | 0.35 |
| 426 | BP20;MMRN2;ENG;SPINT1;SEPP1;IGFALS | 0.78 | 0.77 | 0.49 | 0.51 | 0.20 | 0.19 |
| 427 | BP15;MMRN2;ADAM12;IGFALS;MCAM | 0.78 | 0.78 | 0.43 | 0.40 | 0.26 | 0.42 |
| 428 | bmi;MMRN2;ADAM12;ENG;IGFALS | 0.78 | 0.74 | 0.46 | 0.47 | 0.42 | 0.13 |
| 429 | *gest*;MMRN2;ENG;SPINT1;QSOX1;IGFALS | 0.78 | 0.75 | 0.53 | 0.49 | 0.32 | 0.25 |
| 430 | BP20;MMRN2;ADAM12;SPINT1;IGFALS | 0.78 | 0.74 | 0.48 | 0.38 | 0.30 | 0.30 |
| 431 | BP20;ENG;IGFALS;MCAM;PIGF | 0.78 | 0.75 | 0.44 | 0.40 | 0.37 | 0.04 |
| 432 | *alcoh*;BP15;ENG;IGFALS;MCAM | 0.78 | 0.73 | 0.52 | 0.50 | 0.30 | 0.15 |
| 433 | bmi;ADAM12;ENG;MCAM;PIGF | 0.78 | 0.74 | 0.56 | 0.52 | 0.23 | 0.01 |
| 434 | BP20;MMRN2;ECM1;ENG;IGFALS;MCAM | 0.78 | 0.76 | 0.49 | 0.53 | 0.13 | 0.13 |
| 435 | BP15;MMRN2;IGFALS;ALDOA;PIGF | 0.78 | 0.74 | 0.45 | 0.16 | 0.08 | 0.37 |
| 436 | bmi;BP15;MMRN2;ENG;SPINT1;IGFALS | 0.78 | 0.76 | 0.45 | 0.56 | 0.07 | 0.38 |
| 437 | *alcoh*;BP20;MMRN2;ENG;IGFALS;MCAM | 0.78 | 0.76 | 0.44 | 0.58 | 0.31 | 0.16 |
| 438 | BP20;*fihd*;SEPP1;IGFALS;PIGF | 0.78 | 0.75 | 0.40 | 0.34 | 0.23 | 0.45 |
| 439 | BP20;ENG;SEPP1;QSOX1;IGFALS;MCAM | 0.78 | 0.76 | 0.42 | 0.54 | 0.31 | 0.02 |
| 440 | BP15;ADAM12;SEPP1;IGFALS;PIGF | 0.78 | 0.76 | 0.45 | 0.08 | 0.30 | 0.25 |
| 441 | ENG;SPINT1;SEPP1;IGFALS;MCAM;ROBO4 | 0.78 | 0.75 | 0.52 | 0.50 | 0.21 | 0.15 |

(continued)

| No. | Panel composition | A | B | C | D | E | F |
|---|---|---|---|---|---|---|---|
| 442 | bmi;BP15;MMRN2;ENG;SPINT1;IGFALS | 0.78 | 0.75 | 0.42 | 0.56 | 0.01 | 0.33 |
| 443 | BP20;*fihd*;ENG;IGFALS;MCAM;ROBO4 | 0.78 | 0.74 | 0.55 | 0.58 | 0.18 | 0.13 |
| 444 | *alcoh*;BP20;ADAM12;SEPP1;IGFALS | 0.78 | 0.75 | 0.48 | 0.37 | 0.40 | 0.13 |
| 445 | *alcoh*;BP15;ADAM12;MCAM;PlGF | 0.78 | 0.74 | 0.53 | 0.44 | 0.37 | 0.22 |
| 446 | MMRN2;ENG;SPINT1;IGFALS;MCAM | 0.78 | 0.75 | 0.52 | 0.50 | 0.43 | 0.27 |
| 447 | BP15;ADAM12;IGFALS;MCAM;ENPP2 | 0.78 | 0.74 | 0.42 | 0.23 | 0.33 | 0.39 |
| 448 | *alcoh*;BP20;MMRN2;ADAM12;IGFALS | 0.78 | 0.78 | 0.49 | 0.39 | 0.47 | 0.50 |
| 449 | bmi;ENG;SPINT1;QSOX1;IGFALS | 0.78 | 0.75 | 0.47 | 0.38 | 0.48 | 0.26 |
| 450 | BP20;MMRN2;ADAM12;ENG;IGFALS;MCAM | 0.78 | 0.79 | 0.49 | 0.54 | 0.09 | 0.29 |
| 451 | BP15;ADAM12;IGFALS;PlGF | 0.78 | 0.75 | 0.44 | 0.14 | 0.24 | 0.26 |
| 452 | *alcoh*;BP20;MMRN2;IGFALS;*vagbl* | 0.78 | 0.76 | 0.45 | 0.32 | 0.52 | 0.40 |
| 453 | BP15;MMRN2;ADAM12;SEPP1;IGFALS | 0.78 | 0.74 | 0.48 | 0.27 | 0.26 | 0.45 |
| 454 | BP20;ENG;SPINT1;IGFALS;MCAM | 0.78 | 0.77 | 0.48 | 0.59 | 0.31 | 0.18 |
| 455 | BP20;ECM1;LCAT;LNPEP;MCAM;PlGF | 0.78 | 0.75 | 0.52 | 0.51 | 0.29 | 0.03 |
| 456 | BP15;ADAM12;IGFALS;ROBO4;PlGF | 0.78 | 0.76 | 0.51 | 0.29 | 0.32 | 0.39 |
| 457 | BP20;ADAM12;QSOX1;IGFALS;PlGF | 0.78 | 0.77 | 0.41 | 0.39 | 0.20 | 0.38 |
| 458 | BP15;ADAM12;ECM1;MCAM;PlGF | 0.78 | 0.75 | 0.53 | 0.33 | 0.16 | 0.17 |
| 459 | ADAM12;IGFALS;MCAM;PlGF | 0.78 | 0.75 | 0.45 | 0.40 | 0.37 | 0.34 |
| 460 | BP15;IGFALS;MCAM;PlGF | 0.78 | 0.74 | 0.38 | 0.19 | 0.23 | 0.33 |
| 461 | BP20;*fihd*;ADAM12;SEPP1;IGFALS | 0.78 | 0.74 | 0.42 | 0.16 | 0.31 | 0.17 |
| 462 | BP20;*fihd*;ENG;SEPP1;IGFALS;MCAM | 0.78 | 0.72 | 0.53 | 0.52 | 0.16 | 0.14 |
| 463 | *alcoh*;ADAM12;QSOX1;IGFALS;PlGF | 0.78 | 0.74 | 0.60 | 0.24 | 0.42 | 0.37 |
| 464 | BP15;*gest*;ENG;SPINT1;IGFALS;PlGF | 0.78 | 0.74 | 0.54 | 0.53 | 0.09 | 0.20 |
| 465 | *alcoh*;BP15;MMRN2;ADAM12;IGFALS | 0.78 | 0.74 | 0.39 | 0.33 | 0.41 | 0.25 |
| 466 | *alcoh*;BP20;MMRN2;ENG;IGFALS;*vagbl* | 0.78 | 0.75 | 0.58 | 0.46 | 0.30 | 0.26 |
| 467 | BP20;MMRN2;ENG;SPINT1;QSOX1;IGFALS | 0.78 | 0.77 | 0.48 | 0.53 | 0.21 | 0.19 |
| 468 | BP15;MMRN2;LNPEP;IGFALS;PlGF | 0.78 | 0.76 | 0.32 | 0.12 | 0.19 | 0.42 |
| 469 | BP20;*fihd*;MMRN2;ADAM12;IGFALS | 0.78 | 0.74 | 0.47 | 0.33 | 0.19 | 0.30 |
| 470 | BP15;ADAM12;IGFALS;PlGF | 0.78 | 0.75 | 0.54 | 0.12 | 0.31 | 0.30 |
| 471 | BP20;MMRN2;ADAM12;ENG;IGFALS;MCAM | 0.78 | 0.78 | 0.47 | 0.56 | 0.19 | 0.25 |
| 472 | BP20;ECM1;IGFALS;MCAM;PlGF | 0.78 | 0.76 | 0.47 | 0.38 | 0.26 | 0.41 |
| 473 | BP15;ADAM12;IGFALS;PlGF | 0.78 | 0.74 | 0.37 | 0.14 | 0.25 | 0.29 |
| 474 | BP20;*fhpet*;ECM1;LNPEP;MCAM;PlGF | 0.78 | 0.77 | 0.44 | 0.60 | 0.27 | 0.02 |
| 475 | *alcoh*;MMRN2;ENG;SPINT1;IGFALS | 0.78 | 0.74 | 0.45 | 0.36 | 0.38 | 0.31 |
| 476 | BP20;MAPRE1/3;IGFALS;ALDOA;PlGF | 0.78 | 0.75 | 0.36 | 0.45 | 0.36 | 0.30 |
| 477 | *alcoh*;ENG;SPINT1;1GFALS;MCAM | 0.78 | 0.74 | 0.53 | 0.48 | 0.26 | 0.20 |
| 478 | BP20;MMRN2;ENG;SPINT1;SEPP1;IGFALS | 0.78 | 0.76 | 0.54 | 0.49 | 0.36 | 0.17 |
| 479 | BP20;ENG;SEPP1;IGFALS;MCAM;*vagbl* | 0.78 | 0.75 | 0.52 | 0.56 | 0.37 | 0.03 |

(continued)

| No. | Panel composition | A | B | C | D | E | F |
|-----|-------------------|------|------|------|------|------|------|
| 480 | *alcoh*;BP20;ENG;SPINT1;IGFALS | 0.78 | 0.74 | 0.35 | 0.44 | 0.33 | 0.18 |
| 481 | bmi;BP20;MMRN2;ENG;IGFALS;MCAM | 0.78 | 0.76 | 0.49 | 0.60 | 0.41 | 0.01 |
| 482 | BP20;MMRN2;ENG;IGFALS;MCAM;*vagbl* | 0.78 | 0.78 | 0.46 | 0.58 | 0.10 | 0.16 |
| 483 | BP15;MMRN2;ENG;IGFALS;MCAM | 0.78 | 0.75 | 0.50 | 0.44 | 0.30 | 0.14 |
| 484 | BP20;ENG;SPINT1;SEPP1;IGFALS;PIGF | 0.78 | 0.77 | 0.53 | 0.53 | 0.13 | 0.07 |
| 485 | *alcoh*;BP15;ADAM12;QSOX1;IGFALS | 0.78 | 0.74 | 0.38 | 0.04 | 0.30 | 0.48 |
| 486 | BP20;ENG;SPINT1;IGFALS;MCAM;ROBO4 | 0.78 | 0.77 | 0.49 | 0.61 | 0.38 | 0.26 |
| 487 | BP15;MAPRE1/3;IGFALS;ALDOA;PIGF | 0.78 | 0.74 | 0.40 | 0.38 | 0.21 | 0.21 |
| 488 | *alcoh*;BP15;MMRN2;ADAM12;IGFALS | 0.78 | 0.76 | 0.41 | 0.14 | 0.35 | 0.38 |
| 489 | *alcoh*;BP20;*fhpet*;MMRN2;IGFALS | 0.78 | 0.76 | 0.47 | 0.30 | 0.53 | 0.41 |
| 490 | BP15;ADAM12;ENG;SPINT1;MCAM;ENPP2 | 0.78 | 0.75 | 0.52 | 0.48 | 0.31 | 0.17 |
| 491 | bmi;ADAM12;IGFALS;PIGF | 0.78 | 0.75 | 0.35 | 0.22 | 0.40 | 0.37 |
| 492 | BP15;MAPRE1/3;IGFALS;ALDOA;PIGF | 0.78 | 0.74 | 0.47 | 0.20 | 0.19 | 0.18 |
| 493 | *alcoh*;MMRN2;ENG;IGFALS;MCAM;vagbl | 0.78 | 0.74 | 0.53 | 0.50 | 0.32 | 0.24 |
| 494 | BP20;LNPEP;IGFALS;MCAM;PIGF | 0.78 | 0.77 | 0.44 | 0.48 | 0.43 | 0.50 |
| 495 | BP15;MMRN2;LNPEP;IGFALS;PIGF | 0.78 | 0.76 | 0.42 | 0.35 | 0.27 | 0.37 |
| 496 | BP15;ADAM12;IGFALS;MCAM | 0.78 | 0.75 | 0.39 | 0.33 | 0.21 | 0.27 |
| 497 | BP20;*fihd*;ECM1;ENG;MCAM;PIGF | 0.78 | 0.71 | 0.53 | 0.52 | 0.02 | 0.01 |
| 498 | BP15;ADAM12;IGFALS;MCAM;ROBO4 | 0.78 | 0.75 | 0.48 | 0.40 | 0.43 | 0.25 |
| 499 | BP20;*fihd*;ENG;IGFALS;MCAM;ROBO4 | 0.78 | 0.73 | 0.53 | 0.54 | 0.14 | 0.14 |
| 500 | BP20;*fihd*;ADAM12;IGFALS;MCAM | 0.78 | 0.75 | 0.49 | 0.50 | 0.24 | 0.24 |
| 501 | BP20;MMRN2;ENG;QSOX1;IGFALS;MCAM | 0.78 | 0.77 | 0.41 | 0.60 | 0.19 | 0.14 |
| 502 | BP15;ADAM12;SPINT1;QSOX1;IGFALS | 0.78 | 0.74 | 0.51 | 0.36 | 0.13 | 0.25 |
| 503 | BP15;ADAM12;ALDOA;MCAM;PIGF | 0.78 | 0.74 | 0.42 | 0.23 | 0.26 | 0.36 |
| 504 | BP15;MAPRE1/3;IGFALS;ALDOA;PIGF | 0.78 | 0.75 | 0.49 | 0.18 | 0.03 | 0.17 |
| 505 | BP15;ADAM12;IGFALS;PIGF | 0.78 | 0.74 | 0.35 | 0.16 | 0.12 | 0.29 |
| 506 | BP20;*fihd*;ADAM12;SEPP1;IGFALS;ROBO4 | 0.78 | 0.74 | 0.53 | 0.51 | 0.38 | 0.25 |
| 507 | BP15;MMRN2;IGFALS;MCAM;*vagbl* | 0.78 | 0.74 | 0.48 | 0.35 | 0.18 | 0.39 |
| 508 | BP15;ENG;SPINT1;QSOX1;IGFALS | 0.78 | 0.74 | 0.45 | 0.42 | 0.29 | 0.15 |
| 509 | BP15;ECM1;ENG;SPINT1;MCAM | 0.78 | 0.74 | 0.49 | 0.40 | 0.14 | 0.18 |
| 510 | *alcoh*;BP20;ADAM12;ENG;IGFALS;MCAM | 0.78 | 0.77 | 0.49 | 0.54 | 0.28 | 0.25 |
| 511 | BP15;ADAM12;IGFALS;MCAM;ENPP2 | 0.78 | 0.74 | 0.45 | 0.27 | 0.29 | 0.41 |
| 512 | *alcoh*;BP2D;ECM1;LNPEP;MCAM;PIGF | 0.78 | 0.77 | 0.57 | 0.51 | 0.20 | 0.01 |
| 513 | BP20;ADAM12;IGFALS;ROBO4;PIGF | 0.78 | 0.77 | 0.40 | 0.33 | 0.32 | 0.23 |
| 514 | *alcoh*;BP20;ENG;QSOX1;IGFALS | 0.78 | 0.74 | 0.45 | 0.43 | 0.45 | 0.03 |
| 515 | BP15;ENG;SEPP1;IGFALS;MCAM;ROBO4 | 0.78 | 0.73 | 0.50 | 0.52 | 0.32 | 0.20 |
| 516 | BP15;ENG;SPINT1;SEPP1;IGFALS | 0.78 | 0.74 | 0.46 | 0.29 | 0.18 | 0.40 |
| 517 | *fhpet*;LNPEP;IGFALS;MCAM;PIGF | 0.78 | 0.74 | 0.41 | 0.35 | 0.32 | 0.29 |

(continued)

| No. | Panel composition | A | B | C | D | E | F |
|---|---|---|---|---|---|---|---|
| 518 | *alcoh*;BP15;ADAM12;QSOX1;IGFALS | 0.78 | 0.74 | 0.43 | 0.02 | 0.29 | 0.23 |
| 519 | BP15;MMRN2;ADAM12;IGFALS;ENPP2 | 0.78 | 0.74 | 0.45 | 0.18 | 0.29 | 0.45 |
| 520 | MMRN2;ADAM12;IGFALS;PIGF | 0.78 | 0.76 | 0.36 | 0.27 | 0.41 | 0.44 |
| 521 | *alcoh*;BP20;ADAM12;QSOX1;IGFALS | 0.78 | 0.76 | 0.44 | 0.39 | 0.37 | 0.24 |
| 522 | BP20;ENG;IGFALS;MCAM;PIGF | 0.78 | 0.74 | 0.48 | 0.35 | 0.38 | 0.03 |
| 523 | BP95;M1NRN2;ENG;SPINT1;IGFALS | 0.78 | 0.75 | 0.48 | 0.47 | 0.44 | 0.26 |
| 524 | *alcoh*;BP20;ADAM12;QSOX1;IGFALS | 0.78 | 0.75 | 0.40 | 0.04 | 0.37 | 0.36 |
| 525 | BP15;IGFALS;MCAM;PIGF | 0.78 | 0.74 | 0.37 | 0.23 | 0.19 | 0.32 |
| 526 | bmi;BP20;MMRN2;ADAM12;ENG;PIGF | 0.78 | 0.75 | 0.49 | 0.57 | 0.09 | 0.03 |
| 527 | *alcoh*;BP20;ENG;QSOX1;IGFALS | 0.78 | 0.74 | 0.46 | 0.39 | 0.43 | 0.04 |
| 528 | ECM1;LNPEP;IGFALS;MCAM;PIGF | 0.78 | 0.74 | 0.40 | 0.28 | 0.27 | 0.32 |
| 529 | BP20;ADAM12;ECM1;MCAM;PIGF | 0.78 | 0.76 | 0.50 | 0.55 | 0.14 | 0.05 |
| 530 | BP15;ENG;SPINT1;QSOX1;IGFALS | 0.78 | 0.74 | 0.45 | 0.42 | 0.30 | 0.27 |
| 531 | bmi;BP20;MMRN2;ENG;IGFALS;MCAM | 0.78 | 0.75 | 0.48 | 0.54 | 0.42 | 0.01 |
| 532 | bmi;*fhpet*;MMRN2;ADAM12;ENG;MCAM | 0.78 | 0.75 | 0.46 | 0.58 | 0.15 | 0.01 |
| 533 | *alcoh*;BP20;ENG;QSOX1;IGFALS;MCAM | 0.78 | 0.77 | 0.47 | 0.60 | 0.35 | 0.03 |
| 534 | *alcoh*;BP15;ADAM12;IGFALS;MCAM | 0.78 | 0.76 | 0.52 | 0.23 | 0.30 | 0.39 |
| 535 | BP15;MMRN2;ENG;SPINT1;IGFALS | 0.78 | 0.75 | 0.46 | 0.49 | 0.43 | 0.29 |
| 536 | BP20;*fihd*;ADAM12;IGFALS;MCAM | 0.78 | 0.74 | 0.49 | 0.44 | 0.35 | 0.24 |
| 537 | *alcoh*;BP20;MMRN2;ENG;IGFALS | 0.78 | 0.75 | 0.47 | 0.50 | 0.37 | 0.16 |
| 538 | BP15;ADAM12;ENG;IGFALS;MCAM | 0.78 | 0.75 | 0.40 | 0.40 | 0.31 | 0.12 |
| 539 | BP15;MMRN2;ENG;IGFALS;PIGF | 0.78 | 0.74 | 0.42 | 0.36 | 0.02 | 0.24 |
| 540 | BP15;ADAM12;ENG;SPINT1;MCAM | 0.78 | 0.75 | 0.48 | 0.39 | 0.30 | 0.14 |
| 541 | *alcoh*;BP20;*fhpet*;SEPP1;IGFALS | 0.78 | 0.74 | 0.45 | 0.18 | 0.41 | 0.11 |
| 542 | BP15;MMRN2;ADAM12;IGFALS;ENPP2 | 0.78 | 0.74 | 0.42 | 0.16 | 0.35 | 0.27 |
| 543 | BP15;MMRN2;ENG;SPINT1;IGFALS | 0.78 | 0.74 | 0.43 | 0.49 | 0.00 | 0.31 |
| 544 | *alcoh*;BP15;ADAM12;QSOX1;IGFALS | 0.78 | 0.74 | 0.50 | 0.08 | 0.32 | 0.19 |
| 545 | bmi;ENG;QSOX1;IGFALS;MCAM;*vagbl* | 0.78 | 0.75 | 0.46 | 0.56 | 0.34 | 0.01 |
| 546 | ENG;SPINT1;SEPP1;IGFALS;MCAM | 0.78 | 0.75 | 0.48 | 0.41 | 0.20 | 0.11 |
| 547 | alcoh;fhpet;MMRN2;ADAM12;IGFALS | 0.78 | 0.74 | 0.45 | 0.22 | 0.41 | 0.46 |
| 548 | BP15;MMRN2;IGFALS;PIGF | 0.78 | 0.75 | 0.38 | 0.30 | 0.00 | 0.37 |
| 549 | BP20;ADAM12;IGFALS;PIGF | 0.78 | 0.77 | 0.32 | 0.33 | 0.26 | 0.41 |
| 550 | *alcoh*;BP20;MMRN2;IGFALS;*vagbl* | 0.78 | 0.75 | 0.46 | 0.30 | 0.25 | 0.42 |
| 551 | BP20;ENG;SPINT1;SEPP1;IGFALS;PIGF | 0.78 | 0.76 | 0.55 | 0.49 | 0.16 | 0.08 |
| 552 | BP20;ENG;SPINT1;IGFALS;MCAM | 0.78 | 0.76 | 0.47 | 0.57 | 0.37 | 0.20 |
| 553 | *alcoh*;BP15;*fhpet*;MMRN2;IGFALS | 0.78 | 0.74 | 0.46 | 0.22 | 0.36 | 0.40 |
| 554 | BP15;MMRN2;ENG;IGFALS;PIGF | 0.78 | 0.74 | 0.44 | 0.42 | 0.00 | 0.30 |
| 555 | BP15;ADAM12;IGFALS;MCAM | 0.78 | 0.75 | 0.42 | 0.29 | 0.16 | 0.32 |

(continued)

| No. | Panel composition | A | B | C | D | E | F |
|---|---|---|---|---|---|---|---|
| 556 | *alcoh*;BP20;*fhpet*;ENG;IGFALS;MCAM | 0.78 | 0.75 | 0.50 | 0.52 | 0.34 | 0.15 |
| 557 | BP15;MAPRE1/3;IGFALS;ALDOA;MCAM | 0.78 | 0.74 | 0.47 | 0.20 | 0.03 | 0.33 |
| 558 | BP20;MMRN2;LNPEP;IGFALS;PIGF | 0.78 | 0.78 | 0.36 | 0.35 | 0.34 | 0.53 |
| 559 | *alcoh*;BP20;MMRN2;ADAM12;IGFALS | 0.78 | 0.77 | 0.50 | 0.24 | 0.41 | 0.49 |
| 560 | BP15;MMRN2;ADAM12;IGFALS;ENPP2 | 0.78 | 0.75 | 0.46 | 0.12 | 0.37 | 0.37 |
| 561 | *alcoh*;BP15;ADAM12;MCAM;PIGF | 0.78 | 0.74 | 0.49 | 0.44 | 0.24 | 0.28 |
| 562 | BP15;ADAM12;MAPRE1/3;ALDOA;MCAM | 0.78 | 0.71 | 0.45 | 0.23 | 0.46 | 0.47 |
| 563 | BP20;MMRN2;ENG;SPINT1;IGFALS | 0.78 | 0.76 | 0.48 | 0.49 | 0.16 | 0.19 |
| 564 | BP20;MMRN2;ENG;IGFALS;MCAM;*vagbl* | 0.78 | 0.78 | 0.46 | 0.56 | 0.14 | 0.24 |
| 565 | BP15;ADAM12;IGFALS;MCAM | 0.78 | 0.75 | 0.41 | 0.33 | 0.29 | 0.39 |
| 566 | *fhpet*;ENG;SPINT1;IGFALS;MCAM | 0.78 | 0.74 | 0.40 | 0.43 | 0.43 | 0.21 |
| 567 | *alcoh*;BP20;ADAM12;SEPP1;IGFALS | 0.78 | 0.74 | 0.43 | 0.04 | 0.46 | 0.24 |
| 568 | *alcoh*;BP20;ADAM12;SEPP1;IGFALS | 0.78 | 0.74 | 0.39 | 0.12 | 0.41 | 0.14 |
| 569 | BP20;*fihd*;ENG;SEPP1;IGFALS;ROBO4 | 0.78 | 0.72 | 0.53 | 0.50 | 0.12 | 0.02 |
| 570 | MMRN2;ADAM12;ECM1;IGFALS;MCAM | 0.78 | 0.76 | 0.41 | 0.40 | 0.01 | 0.21 |
| 571 | BP15;MMRN2;IGFALS;PIGF | 0.78 | 0.75 | 0.38 | 0.18 | 0.06 | 0.35 |
| 572 | BP20;ENG;IGFALS;MCAM;PIGF | 0.78 | 0.74 | 0.47 | 0.44 | 0.32 | 0.19 |
| 573 | bmi;BP20;*fhpet*;ADAM12;ENG;MCAM | 0.78 | 0.75 | 0.52 | 0.50 | 0.36 | 0.01 |
| 574 | *alcoh*;BP20;*fhpet*;ENG;IGFALS;MCAM | 0.77 | 0.75 | 0.45 | 0.56 | 0.31 | 0.24 |
| 575 | bmi;BP20;MMRN2;ADAM12;ENG;MCAM | 0.77 | 0.76 | 0.52 | 0.54 | 0.37 | 0.12 |
| 576 | BP15;MMRN2;ENG;IGFALS;PIGF | 0.77 | 0.74 | 0.40 | 0.38 | 0.01 | 0.27 |
| 577 | bmi;ADAM12;ECM1;MCAM;PIGF | 0.77 | 0.74 | 0.52 | 0.34 | 0.07 | 0.24 |
| 578 | *alcoh*;BP20;ENG;IGFALS;MCAM | 0.77 | 0.75 | 0.45 | 0.58 | 0.35 | 0.14 |
| 579 | BP15;MMRN2;ENG;IGFALS;PIGF | 0.77 | 0.74 | 0.43 | 0.38 | 0.16 | 0.36 |
| 580 | BP20;ECM1;SEPP1;IGFALS;PIGF | 0.77 | 0.76 | 0.41 | 0.29 | 0.34 | 0.23 |
| 581 | bmi;MMRN2;ENG;SPINT1;IGFALS | 0.77 | 0.75 | 0.40 | 0.40 | 0.47 | 0.29 |
| 582 | BP15;MMRN2;ECM1;ENG;SPINT1;MCAM | 0.77 | 0.76 | 0.53 | 0.49 | 0.17 | 0.11 |
| 583 | *alcoh*;BP20;MMRN2;SEPP1;IGFALS | 0.77 | 0.76 | 0.50 | 0.20 | 0.43 | 0.45 |
| 584 | bmi;ENG;SPINT1;SEPP1;IGFALS | 0.77 | 0.75 | 0.44 | 0.42 | 0.27 | 0.27 |
| 585 | BP20;MMRN2;ENG;SEPP1;IGFALS;MCAM | 0.77 | 0.76 | 0.52 | 0.56 | 0.18 | 0.17 |
| 586 | BP20;ENG;SPINT1;SEPP1;IGFALS;PIGF | 0.77 | 0.76 | 0.54 | 0.49 | 0.13 | 0.07 |
| 587 | BP15.ENG;SPINT1;QSOX1;IGFALS | 0.77 | 0.74 | 0.50 | 0.40 | 0.30 | 0.29 |
| 588 | BP20;*fihd*;IGFALS;PIGF | 0.77 | 0.74 | 0.44 | 0.28 | 0.02 | 0.29 |
| 589 | *alcoh*;BP20;*fhpet*;MMRN2;IGFALS | 0.77 | 0.75 | 0.48 | 0.20 | 0.42 | 0.35 |
| 590 | *alcoh*;BP20;ENG;SPINT1;IGFALS | 0.77 | 0.74 | 0.37 | 0.40 | 0.30 | 0.21 |
| 591 | BP20;ENG;SPINT1;SEPP1;IGFALS | 0.77 | 0.76 | 0.40 | 0.44 | 0.20 | 0.07 |
| 592 | ADAM12;SEPP1;IGFALS;ENPP2;PIGF | 0.77 | 0.74 | 0.50 | 0.12 | 0.28 | 0.35 |
| 593 | *alcoh*;BP20;MMRN2;ADAM12;IGFALS | 0.77 | 0.76 | 0.41 | 0.20 | 0.38 | 0.27 |

(continued)

| No. | Panel composition | A | B | C | D | E | F |
|-----|------------------|---|---|---|---|---|---|
| 594 | MMRN2;MAPRE1/3;IGFALS;ALDOA;PIGF | 0.77 | 0.77 | 0.46 | 0.35 | 0.25 | 0.36 |
| 595 | BP15;ENG;SPINT1;QSOX1;IGFALS | 0.77 | 0.74 | 0.50 | 0.42 | 0.28 | 0.29 |
| 596 | BP15;MMRN2;ENG;IGFALS;PIGF | 0.77 | 0.74 | 0.39 | 0.44 | 0.14 | 0.37 |
| 597 | bmi;MMRN2;ADAM12;QSOX1;IGFALS | 0.77 | 0.75 | 0.48 | 0.27 | 0.35 | 0.41 |
| 598 | BP20;ENG;SPINT1;IGFALS;MCAM | 0.77 | 0.77 | 0.38 | 0.59 | 0.20 | 0.22 |
| 599 | alcoh;MMRN2;IGFALS;PIGF | 0.77 | 0.74 | 0.42 | 0.24 | 0.37 | 0.37 |
| 600 | ENG;SPINT1;QSOX1;IGFALS;MCAM | 0.77 | 0.75 | 0.47 | 0.48 | 0.28 | 0.20 |
| 601 | BP20;ECM1;ENG;SPINT1;MCAM;PIGF | 0.77 | 0.76 | 0.48 | 0.58 | 0.20 | 0.02 |
| 602 | alcoh;BP20;ADAM12;QSOX1;IGFALS | 0.77 | 0.75 | 0.41 | 0.04 | 0.36 | 0.28 |
| 603 | BP15;MMRN2;IGFALS;PIGF | 0.77 | 0.75 | 0.45 | 0.26 | 0.01 | 0.34 |
| 604 | bmi;ENG;SPINT1;IGFALS;MCAM | 0.77 | 0.77 | 0.39 | 0.52 | 0.34 | 0.15 |
| 605 | BP20;MMRN2;ADAM12;ENG;IGFALS;MCAM | 0.77 | 0.77 | 0.49 | 0.56 | 0.19 | 0.19 |
| 606 | alcoh;BP20;ADAM12;IGFALS;MCAM | 0.77 | 0.77 | 0.42 | 0.48 | 0.29 | 0.37 |
| 607 | BP15;MMRN2;ENG;LCAT;SPINT1;MCAM | 0.77 | 0.73 | 0.53 | 0.52 | 0.20 | 0.06 |
| 608 | alcoh;BP20;MMRN2;IGFALS;vagbl | 0.77 | 0.75 | 0.45 | 0.18 | 0.37 | 0.36 |
| 609 | BP15;ADAM12;MCAM;PIGF | 0.77 | 0.74 | 0.50 | 0.23 | 0.40 | 0.25 |
| 610 | BP20;ECM1;LNPEP:MCAM;PIGF | 0.77 | 0.77 | 0.42 | 0.55 | 0.26 | 0.01 |
| 611 | BP15;ADAM12;MCAM;PIGF | 0.77 | 0.74 | 0.46 | 0.25 | 0.37 | 0.27 |
| 612 | alcoh;bmi;BP20;ENG;IGFALS;MCAM | 0.77 | 0.75 | 0.40 | 0.54 | 0.43 | 0.14 |
| 613 | alcoh;BP15;ADAM12;QSOX1;IGFALS | 0.77 | 0.74 | 0.45 | 0.02 | 0.35 | 0.32 |
| 614 | ENG;LNPEP;SPINT1;IGFALS;MCAM;ENPP2 | 0.77 | 0.74 | 0.51 | 0.50 | 0.37 | 0.22 |
| 615 | BP20;ADAM12;QSOX1;IGFALS;PIGF | 0.77 | 0.78 | 0.43 | 0.39 | 0.19 | 0.26 |
| 616 | BP15;ADAM12;MCAM;PIGF | 0.77 | 0.74 | 0.51 | 0.27 | 0.33 | 0.27 |
| 617 | BP15;MMRN2;ECM1;IGFALS;MCAM | 0.77 | 0.74 | 0.41 | 0.34 | 0.21 | 0.35 |
| 618 | BP15;MMRN2;IGFALS;PIGF | 0.77 | 0.76 | 0.37 | 0.28 | 0.16 | 0.43 |
| 619 | BP20;MMRN2;ENG;SPINT1;IGFALS | 0.77 | 0.76 | 0.48 | 0.53 | 0.20 | 0.25 |
| 620 | BP15;ADAM12;MCAM;ENPP2;PIGF | 0.77 | 0.76 | 0.47 | 0.17 | 0.17 | 0.35 |
| 621 | BP20;ADAM12;IGFALS;PIGF | 0.77 | 0.77 | 0.22 | 0.24 | 0.21 | 0.30 |
| 622 | BP20;fihd;ADAM12;MCAM;PIGF | 0.77 | 0.76 | 0.49 | 0.40 | 0.19 | 0.25 |
| 623 | bmi;BP20;ADAM12;ENG;QSOX1;MCAM | 0.77 | 0.76 | 0.41 | 0.54 | 0.24 | 0.01 |
| 624 | BP15;ADAM12;IGFALS;PIGF | 0.77 | 0.75 | 0.46 | 0.14 | 0.25 | 0.25 |
| 625 | BP15;MMRN2;IGFALS;PIGF | 0.77 | 0.75 | 0.36 | 0.36 | 0.15 | 0.41 |
| 626 | BP20;ADAM12;SEPP1;IGFALS;MCAM | 0.77 | 0.77 | 0.39 | 0.50 | 0.33 | 0.17 |
| 627 | bmi;BP15;MMRN2;ADAM12;ENG;MCAM | 0.77 | 0.75 | 0.56 | 0.38 | 0.28 | 0.01 |
| 628 | BP20;MMRN2;MAPRE1/3;IGFALS;ALDOA;ENPP2 | 0.77 | 0.75 | 0.45 | 0.58 | 0.28 | 0.09 |
| 629 | BP20;MMRN2;ENG;IGFALS;MCAM | 0.77 | 0.77 | 0.41 | 0.56 | 0.08 | 0.17 |
| 630 | BP15;MMRN2;ECM1;IGFALS;MCAM | 0.77 | 0.74 | 0.51 | 0.36 | 0.19 | 0.37 |
| 631 | MMRN2;ADAM12;QSOX1;IGFALS;ENPP2 | 0.77 | 0.73 | 0.46 | 0.14 | 0.41 | 0.47 |

(continued)

| No. | Panel composition | A | B | C | D | E | F |
|-----|-------------------|------|------|------|------|------|------|
| 632 | BP15;ENG;SPINT1;SEPP1;IGFALS | 0.77 | 0.75 | 0.46 | 0.42 | 0.26 | 0.29 |
| 633 | BP20;*fihd*;ADAM12;ENG;SPINT1;MCAM | 0.77 | 0.75 | 0.53 | 0.48 | 0.36 | 0.23 |
| 634 | BP15;MMRN2;ENG;IGFALS;PIGF | 0.77 | 0.74 | 0.42 | 0.38 | 0.00 | 0.24 |
| 635 | ENG;SPINT1;OSOX1;IGFALS;MCAM | 0.77 | 0.75 | 0.48 | 0.43 | 0.26 | 0.20 |
| 636 | MMRN2;ENG;SEPP1;IGFALS;MCAM;*vagbl* | 0.77 | 0.73 | 0.51 | 0.50 | 0.25 | 0.26 |
| 637 | BP20;MMRN2;ENG;QSOX1;IGFALS;MCAM | 0.77 | 0.77 | 0.49 | 0.58 | 0.16 | 0.15 |
| 638 | BP20;ADAM12;SEPP1;IGFALS;ROBO4 | 0.77 | 0.75 | 0.35 | 0.51 | 0.31 | 0.25 |
| 639 | bmi;ADAM12;QSOX1;IGFALS;pbwgt | 0.77 | 0.75 | 0.54 | 0.16 | 0.26 | 0.27 |
| 640 | BP15;MMRN2;ENG;SPINT1;IGFALS | 0.77 | 0.74 | 0.45 | 0.47 | 0.00 | 0.28 |
| 641 | bmi;ADAM12;ENG;IGFALS;MCAM | 0.77 | 0.74 | 0.43 | 0.46 | 0.43 | 0.19 |
| 642 | MMRN2;ENG;SPINT1;SEPP1;IGFALS | 0.77 | 0.74 | 0.45 | 0.42 | 0.13 | 0.18 |
| 643 | BP15;ENG;QSOX1;IGFALS;MCAM | 0.77 | 0.74 | 0.43 | 0.50 | 0.28 | 0.01 |
| 644 | MMRN2;IGFALS;ENPP2;PIGF | 0.77 | 0.73 | 0.45 | 0.31 | 0.23 | 0.35 |
| 645 | *alcoh*;BP15;ADAM12;QSOX1;IGFALS | 0.77 | 0.74 | 0.47 | 0.06 | 0.31 | 0.25 |
| 646 | BP20;ENG;SEPP1;IGFALS;MCAM | 0.77 | 0.75 | 0.47 | 0.52 | 0.25 | 0.03 |
| 647 | BP20;ADAM12;ENG;QSOX1;IGFALS;MCAM | 0.77 | 0.78 | 0.43 | 0.56 | 0.21 | 0.21 |
| 648 | BP15;ADAM12;IGFALS;MCAM | 0.77 | 0.74 | 0.43 | 0.35 | 0.18 | 0.37 |
| 649 | BP15;ENG;SPINT1;QSOX1;IGFALS | 0.77 | 0.74 | 0.37 | 0.42 | 0.35 | 0.10 |
| 650 | *alcoh*;BP20;ECM1;ENG;MCAM;PIGF | 0.77 | 0.73 | 0.56 | 0.53 | 0.04 | 0.02 |
| 651 | *alcoh*;BP20;ADAM12;IGFALS;ROBO4 | 0.77 | 0.76 | 0.42 | 0.45 | 0.34 | 0.23 |
| 652 | BP15;MMRN2;ADAM12;IGFALS;ENPP2 | 0.77 | 0.75 | 0.45 | 0.18 | 0.39 | 0.47 |
| 653 | BP20;LNPEP;SPINT1;IGFALS;PIGF | 0.77 | 0.74 | 0.40 | 0.33 | 0.14 | 0.15 |
| 654 | BP20;MMRN2;ENG;SPINT1;IGFALS | 0.77 | 0.75 | 0.46 | 0.49 | 0.33 | 0.21 |
| 655 | BP20;ADAM12;MAPRE1/3;IGFALS;ALDOA | 0.77 | 0.74 | 0.30 | 0.45 | 0.37 | 0.17 |
| 656 | BP15;ENG;SPINT1;SEPP1;IGFALS | 0.77 | 0.74 | 0.49 | 0.36 | 0.39 | 0.31 |
| 657 | ENG;SPINT1;IGFALS;MCAM;ENPP2 | 0.77 | 0.74 | 0.51 | 0.45 | 0.38 | 0.18 |
| 658 | BP20;MMRN2;ADAM12;IGFALS;ENPP2 | 0.77 | 0.77 | 0.49 | 0.20 | 0.33 | 0.36 |
| 659 | BP20;*fihd*;MMRN2;LNPEP;IGFALS | 0.77 | 0.74 | 0.49 | 0.39 | 0.32 | 0.23 |
| 660 | BP20;ENG;SPINT1;SEPP1;IGFALS | 0.77 | 0.75 | 0.31 | 0.38 | 0.25 | 0.08 |
| 661 | bmi;*fhpet*;ENG;SPINT1;IGFALS | 0.77 | 0.74 | 0.47 | 0.42 | 0.49 | 0.25 |
| 662 | MMRN2;ECM1;ENG;IGFALS;MCAM | 0.77 | 0.74 | 0.36 | 0.47 | 0.16 | 0.15 |
| 663 | bmi;ENG;QSOX1;IGFALS;MCAM | 0.77 | 0.74 | 0.43 | 0.46 | 0.35 | 0.01 |
| 664 | BP15;ADAM12;SPINT1;MCAM;PIGF | 0.77 | 0.74 | 0.55 | 0.41 | 0.33 | 0.18 |
| 665 | BP15;ENG;SPINT1;QSOX1;IGFALS | 0.77 | 0.74 | 0.38 | 0.42 | 0.42 | 0.10 |
| 666 | BP20;ENG;SEPP1;GSOX1;IGFALS;MCAM | 0.77 | 0.75 | 0.50 | 0.56 | 0.25 | 0.13 |
| 667 | BP20;ENG;SPINT1;QSOX1;IGFALS | 0.77 | 0.75 | 0.36 | 0.38 | 0.36 | 0.15 |
| 668 | bmi;ENG;SPINT1;QSOX1;IGFALS | 0.77 | 0.75 | 0.48 | 0.40 | 0.48 | 0.32 |
| 669 | *alcoh*;BP20;ADAM12;QSOX1;IGFALS | 0.77 | 0.76 | 0.43 | 0.39 | 0.30 | 0.35 |

(continued)

| No. | Panel composition | A | B | C | D | E | F |
|---|---|---|---|---|---|---|---|
| 670 | SP15;ADAM12;ALDOA;MCAM;PlGF | 0.77 | 0.74 | 0.46 | 0.25 | 0.25 | 0.25 |
| 671 | BP20;ADAM12;MAPRE1/3;ALDOA;PlGF | 0.77 | 0.74 | 0.23 | 0.43 | 0.26 | 0.04 |
| 672 | *alcoh*;BP20;IGFALS;PlGF | 0.77 | 0.75 | 0.37 | 0.40 | 0.38 | 0.40 |
| 673 | *alcoh*;BP20;LNPEP;QSOX1;IGFALS | 0.77 | 0.75 | 0.45 | 0.02 | 0.27 | 0.23 |
| 674 | MMRN2;ECM1;IGFALS;PlGF | 0.77 | 0.74 | 0.44 | 0.29 | 0.20 | 0.30 |
| 675 | BP20;MMRN2;ADAM12;IGFALS;MCAM | 0.77 | 0.78 | 0.43 | 0.50 | 0.20 | 0.30 |
| 676 | BP20;ADAM12;IGFALS;PlGF | 0.77 | 0.76 | 0.37 | 0.35 | 0.34 | 0.41 |
| 677 | BP15;MMRN2;ENG;SPINT1;IGFALS | 0.77 | 0.74 | 0.42 | 0.47 | 0.08 | 0.25 |
| 678 | bmi;MMRN2;ENG;IGFALS;MCAM | 0.77 | 0.74 | 0.46 | 0.46 | 0.40 | 0.01 |
| 679 | bmi;*fhpet*;ADAM12;IGFALS;pbwgt | 0.77 | 0.75 | 0.52 | 0.23 | 0.36 | 0.28 |
| 680 | BP20;MMRN2;ADAM12;IGFALS;ENPP2 | 0.77 | 0.76 | 0.44 | 0.22 | 0.31 | 0.32 |
| 681 | BP15;ENG;SPINT1;QSOX1;IGFALS | 0.77 | 0.74 | 0.44 | 0.40 | 0.39 | 0.26 |
| 682 | BP15;MMRN2;IGFALS;PlGF | 0.77 | 0.75 | 0.39 | 0.24 | 0.00 | 0.27 |
| 683 | *alcoh*;BP20;MMRN2;ENG;IGFALS | 0.77 | 0.74 | 0.42 | 0.34 | 0.32 | 0.18 |
| 684 | MMRN2;ENG;SPINT1;IGFALS;PlGF | 0.77 | 0.75 | 0.51 | 0.42 | 0.32 | 0.26 |
| 685 | BP20;ECM1;ENG;LNPEP;SPINT1;MCAM | 0.77 | 0.77 | 0.48 | 0.53 | 0.27 | 0.28 |
| 686 | *fhpet*;MMRN2;IGFALS;PlGF | 0.77 | 0.75 | 0.48 | 0.30 | 0.21 | 0.30 |
| 687 | BP20;MMRN2;MAPRE1/3;IGFALS;ALDOA;*vagbl* | 0.77 | 0.78 | 0.45 | 0.58 | 0.11 | 0.14 |
| 688 | ADAM12;IGFALS;ROBO4;ENPP2;PlGF | 0.77 | 0.75 | 0.40 | 0.16 | 0.21 | 0.43 |
| 689 | BP20;MMRN2;ENG;IGFALS;PlGF | 0.77 | 0.76 | 0.42 | 0.44 | 0.34 | 0.13 |
| 690 | MMRN2;ENG;SPINT1;QSOX1;IGFALS | 0.77 | 0.74 | 0.43 | 0.51 | 0.12 | 0.27 |
| 691 | *alcoh*;BP20;ENG;IGFALS;MCAM | 0.77 | 0.75 | 0.40 | 0.58 | 0.29 | 0.24 |
| 692 | BP20;MMRN2;ECM1;LNPEP;QSOX1;IGFALS | 0.77 | 0.77 | 0.43 | 0.54 | 0.10 | 0.39 |
| 693 | BP20;*fihd*;ECM1;ENG;SPINT1;MCAM | 0.77 | 0.76 | 0.54 | 0.51 | 0.25 | 0.22 |
| 694 | MMRN2;ENG;SPINT1;QSOX1;IGFALS | 0.77 | 0.74 | 0.43 | 0.47 | 0.14 | 0.28 |
| 695 | BP15;SEPP1;IGFALS;PlGF | 0.77 | 0.74 | 0.43 | 0.20 | 0.06 | 0.23 |
| 696 | *alcoh*;BP20;SEPP1;IGFALS;ROBO4 | 0.77 | 0.75 | 0.43 | 0.30 | 0.40 | 0.07 |
| 697 | BP15;MMRN2;ADAM12;IGFALS | 0.77 | 0.74 | 0.50 | 0.35 | 0.27 | 0.39 |
| 698 | BP20;ADAM12;SPINT1;MCAM;PlGF | 0.77 | 0.74 | 0.46 | 0.43 | 0.19 | 0.16 |
| 699 | SP20;MMRN2;ENG;IGFALS;MCAM | 0.77 | 0.76 | 0.42 | 0.54 | 0.09 | 0.15 |
| 700 | BP20;ENG;SPINT1;QSOX1;IGFALS | 0.77 | 0.75 | 0.35 | 0.44 | 0.15 | 0.15 |
| 701 | ADAM12;SEPP1;IGFALS;PlGF | 0.77 | 0.74 | 0.30 | 0.29 | 0.25 | 0.30 |
| 702 | BP20;ADAM12;ENG;IGFALS;MCAM;ROBO4 | 0.77 | 0.76 | 0.43 | 0.54 | 0.31 | 0.17 |
| 703 | BP20;ADAM12;SEPP1;IGFALS;MCAM | 0.77 | 0.76 | 0.41 | 0.40 | 0.24 | 0.18 |
| 704 | BP20;ENG;SEPP1;QSOX1;IGFALS;MCAM | 0.77 | 0.76 | 0.51 | 0.50 | 0.14 | 0.13 |
| 705 | BP15;ADAM12;MCAM;PlGF | 0.77 | 0.74 | 0.47 | 0.38 | 0.27 | 0.31 |
| 706 | BP20;ENG;SPINT1;SEPP1;IGFALS | 0.77 | 0.75 | 0.43 | 0.36 | 0.18 | 0.07 |
| 707 | *alcoh*;BP20;*fhpet*;IGFALS;ROBO4 | 0.77 | 0.74 | 0.44 | 0.32 | 0.41 | 0.12 |

(continued)

| No. | Panel composition | A | B | C | D | E | F |
|---|---|---|---|---|---|---|---|
| 708 | BP20;IGFALS;MCAM;PIGF | 0.77 | 0.76 | 0.40 | 0.35 | 0.37 | 0.36 |
| 709 | BP15;MMRN2;ADAM12;IGFALS | 0.77 | 0.75 | 0.49 | 0.22 | 0.35 | 0.40 |
| 710 | *alcoh*;BP20;ENG;QSOX1;IGFALS | 0.77 | 0.74 | 0.46 | 0.43 | 0.44 | 0.04 |
| 711 | BP15;SEPP1;IGFALS;PIGF | 0.77 | 0.74 | 0.37 | 0.20 | 0.12 | 0.24 |
| 712 | bmi;MMRN2;ADAM12;ENG;MCAM;mothpet | 0.77 | 0.75 | 0.48 | 0.52 | 0.16 | 0.01 |
| 713 | BP15;MMRN2;MAPRE1/3;IGFALS;ALDOA | 0.77 | 0.74 | 0.45 | 0.23 | 0.07 | 0.28 |
| 714 | *alcoh*;BP15;ADAM12;QSOX1;IGFALS | 0.77 | 0.74 | 0.44 | 0.02 | 0.34 | 0.35 |
| 715 | BP15;MMRN2;ECM1;IGFALS;MCAM | 0.77 | 0.74 | 0.41 | 0.38 | 0.13 | 0.26 |
| 716 | bmi;BP15;ENG;SPINT1;IGFALS | 0.77 | 0.74 | 0.32 | 0.53 | 0.36 | 0.25 |
| 717 | *alcoh*;BP20;LNPEP;QSOX1;IGFALS | 0.77 | 0.75 | 0.44 | 0.02 | 0.31 | 0.24 |
| 718 | *alcoh*;BP20;*fihd*;ENG;MCAM;PIGF | 0.77 | 0.71 | 0.54 | 0.52 | 0.17 | 0.01 |
| 719 | BP20;ENG:QSOX1:IGFALS;MCAM | 0.77 | 0.75 | 0.28 | 0.56 | 0.28 | 0.01 |
| 720 | BP15;MMRN2;MAPRE1/3;IGFALS;ALDOA | 0.77 | 0.74 | 0.47 | 0.38 | 0.11 | 0.31 |
| 721 | BP20;MMRN2;ENG;IGFALS;MCAM | 0.77 | 0.77 | 0.46 | 0.56 | 0.14 | 0.25 |
| 722 | BP15:ENG:QSOX1:IGFALS;MCAM | 0.77 | 0.74 | 0.37 | 0.48 | 0.21 | 0.01 |
| 723 | BP20;ENG;SEPP1;QSOX1;IGFALS | 0.77 | 0.74 | 0.42 | 0.39 | 0.33 | 0.03 |
| 724 | *alcoh*;BP20;ENG;IGFALS;MCAM | 0.77 | 0.75 | 0.43 | 0.50 | 0.38 | 0.16 |
| 725 | BP15;LNPEP;IGFALS;PIGF | 0.77 | 0.74 | 0.46 | 0.10 | 0.29 | 0.30 |
| 726 | BP15;ADAM12;MCAM;PIGF | 0.77 | 0.73 | 0.41 | 0.23 | 0.43 | 0.25 |
| 727 | ENG;SPINT1;IGFALS;MCAM | 0.77 | 0.74 | 0.36 | 0.55 | 0.19 | 0.19 |
| 728 | BP20;ENG;SEPP1;IGFALS;PIGF | 0.77 | 0.75 | 0.33 | 0.32 | 0.23 | 0.08 |
| 729 | BP15;SEPP1;IGFALS;PIGF | 0.77 | 0.73 | 0.45 | 0.18 | 0.06 | 0.25 |
| 730 | BP20;ENG;QSOX1;IGFALS;MCAM | 0.77 | 0.76 | 0.52 | 0.60 | 0.26 | 0.02 |
| 731 | BP15;ENG;SPINT1;QSOX1;IGFALS | 0.77 | 0.74 | 0.39 | 0.42 | 0.39 | 0.14 |
| 732 | ADAM12;QSOX1;IGFALS;PIGF | 0.77 | 0.74 | 0.41 | 0.18 | 0.29 | 0.42 |
| 733 | BP20;*fhpet*;SEPP1;IGFALS;PIGF | 0.77 | 0.77 | 0.39 | 0.34 | 0.24 | 0.53 |
| 734 | *alcoh*;BP20;IGFALS;PIGF | 0.77 | 0.74 | 0.36 | 0.32 | 0.37 | 0.47 |
| 735 | *alcoh*;MMRN2;ENG;IGFALS;MCAM | 0.77 | 0.74 | 0.46 | 0.46 | 0.31 | 0.24 |
| 736 | *alcoh*;BP20;MMRN2;ENG;IGFALS | 0.77 | 0.75 | 0.55 | 0.46 | 0.27 | 0.26 |
| 737 | *alcoh*;BP20;MMRN2;IGFALS | 0.77 | 0.76 | 0.42 | 0.26 | 0.29 | 0.41 |
| 738 | bmi;BP15;ENG;SPINT1;IGFALS | 0.77 | 0.74 | 0.32 | 0.40 | 0.32 | 0.30 |
| 739 | BP20;*fihd*;ADAM12;MCAM;PIGF | 0.77 | 0.74 | 0.45 | 0.31 | 0.22 | 0.06 |
| 740 | BP15;MMRN2;ADAM12;IGFALS | 0.77 | 0.74 | 0.43 | 0.22 | 0.29 | 0.32 |
| 741 | *alcoh*;BP20;SEPP1;IGFALS | 0.77 | 0.74 | 0.41 | 0.08 | 0.35 | 0.44 |
| 742 | BP20;MMRN2;ENG;QSOX1;IGFALS | 0.77 | 0.75 | 0.43 | 0.53 | 0.12 | 0.11 |
| 743 | BP20;ADAM12;SEPP1;IGFALS;ROBO4 | 0.77 | 0.75 | 0.32 | 0.51 | 0.26 | 0.25 |
| 744 | BP20;ENG;SPINT1;QSOX1;IGFALS | 0.77 | 0.75 | 0.32 | 0.40 | 0.31 | 0.20 |
| 745 | BP20;MMRN2;ENG;IGFALS;PIGF | 0.77 | 0.76 | 0.40 | 0.36 | 0.23 | 0.17 |

(continued)

| No. | Panel composition | A | B | C | D | E | F |
|-----|-------------------|---|---|---|---|---|---|
| 746 | LNPEP;IGFALS;MCAM;PIGF | 0.77 | 0.74 | 0.36 | 0.33 | 0.30 | 0.31 |
| 747 | BP20;IGFALS;MCAM;PIGF | 0.77 | 0.75 | 0.44 | 0.35 | 0.01 | 0.37 |
| 748 | BP20;ENG;SEPP1;QSOX1;IGFALS | 0.77 | 0.74 | 0.43 | 0.37 | 0.37 | 0.04 |
| 749 | *alcoh*;BP20;IGFALS;ROBO4;*vagbl* | 0.77 | 0.74 | 0.45 | 0.38 | 0.32 | 0.12 |
| 750 | *alcoh*;BP20;ADAM12;IGFALS;ROBO4 | 0.77 | 0.76 | 0.41 | 0.41 | 0.31 | 0.35 |
| 751 | *alcoh*;BP20;IGFALS;PIGF | 0.77 | 0.73 | 0.36 | 0.36 | 0.32 | 0.41 |
| 752 | BP15;MMRN2;ADAM12;IGFALS | 0.77 | 0.75 | 0.46 | 0.16 | 0.35 | 0.40 |
| 753 | MMRN2;ADAM12;ENG;QSOX1;IGFALS;MCAM | 0.77 | 0.76 | 0.49 | 0.54 | 0.21 | 0.15 |
| 754 | bmi;ENG;SPINT1;IGFALS;mothpet | 0.77 | 0.74 | 0.45 | 0.42 | 0.23 | 0.12 |
| 755 | BP15;MMRN2;ECM1;IGFALS;MCAM | 0.77 | 0.75 | 0.45 | 0.40 | 0.33 | 0.36 |
| 756 | *alcoh*;BP20;ADAM12;MCAM;PIGF | 0.77 | 0.76 | 0.42 | 0.48 | 0.21 | 0.05 |
| 757 | BP20;ENG;SEPP1;IGFALS;MCAM | 0.77 | 0.74 | 0.42 | 0.52 | 0.12 | 0.12 |
| 758 | BP15;ADAM12;IGFALS;MCAM | 0.77 | 0.76 | 0.41 | 0.27 | 0.21 | 0.32 |
| 759 | bmi;BP15;ENG;SPINT1;MCAM | 0.77 | 0.74 | 0.30 | 0.43 | 0.28 | 0.25 |
| 760 | BP20;MMRN2;ENG;SEPP1;IGFALS | 0.77 | 0.76 | 0.41 | 0.44 | 0.03 | 0.12 |
| 761 | *alcoh*;BP20;ECM1;ENG;MCAM;PIGF | 0.77 | 0.72 | 0.51 | 0.52 | 0.13 | 0.01 |
| 762 | BP20;ECM1;IGFALS;ROBO4;PIGF | 0.77 | 0.75 | 0.39 | 0.29 | 0.23 | 0.41 |
| 763 | BP20;ENG;SPINT1;QSOX1;IGFALS | 0.77 | 0.75 | 0.33 | 0.40 | 0.35 | 0.20 |
| 764 | BP15;SEPP1;IGFALS;PIGF | 0.77 | 0.74 | 0.47 | 0.24 | 0.03 | 0.18 |
| 765 | bmi;BP15;ENG;SPINT1;MCAM | 0.77 | 0.75 | 0.35 | 0.41 | 0.20 | 0.28 |
| 766 | BP20;*fihd*;MMRN2;IGFALS | 0.77 | 0.74 | 0.45 | 0.32 | 0.27 | 0.16 |
| 767 | *alcoh*;BP20;ADAM12;IGFALS | 0.77 | 0.75 | 0.38 | 0.16 | 0.31 | 0.15 |
| 768 | BP20;*fihd*;MMRN2;ADAM12;PRDX2;IGFALS | 0.77 | 0.77 | 0.56 | 0.47 | 0.20 | 0.22 |
| 769 | BP15;MMRN2;MAPRE1/3;IGFALS;ALDOA | 0.77 | 0.75 | 0.48 | 0.43 | 0.18 | 0.31 |
| 770 | *alcoh*;BP20;LNPEP;IGFALS;MCAM | 0.77 | 0.76 | 0.39 | 0.29 | 0.20 | 0.38 |
| 771 | BP20;IGFALS;MCAM;PIGF | 0.76 | 0.75 | 0.36 | 0.33 | 0.22 | 0.31 |
| 772 | BP15;ADAM12;ENG;IGFALS;MCAM | 0.76 | 0.74 | 0.49 | 0.40 | 0.25 | 0.27 |
| 773 | BP20;ENG;SPINT1;IGFALS;PIGF | 0.76 | 0.74 | 0.36 | 0.44 | 0.13 | 0.17 |
| 774 | MMRN2;ADAM12;MAPRE1/3;IGFALS;ALDOA | 0.76 | 0.75 | 0.40 | 0.38 | 0.13 | 0.29 |
| 775 | BP15;ADAM12;IGFALS;MCAM;ENPP2 | 0.76 | 0.75 | 0.43 | 0.21 | 0.26 | 0.40 |
| 776 | BP20;MMRN2;IGFALS;PIGF | 0.76 | 0.77 | 0.40 | 0.36 | 0.21 | 0.47 |
| 777 | bmi;MMRN2;ADAM12;IGFALS | 0.76 | 0.74 | 0.45 | 0.22 | 0.40 | 0.36 |
| 778 | BP20;LNPEP;IGFALS;ROBO4:PIGF | 0.76 | 0.77 | 0.39 | 0.24 | 0.30 | 0.51 |
| 779 | BP20;MMRN2;ECM1;SEPP1;IGFALS | 0.76 | 0.76 | 0.44 | 0.42 | 0.20 | 0.17 |
| 780 | BP20;ENG;SPINT1;QSOX1;IGFALS | 0.76 | 0.75 | 0.46 | 0.44 | 0.18 | 0.20 |
| 781 | *alcoh*;BP20;ADAM12;IGFALS;ROBO4 | 0.76 | 0.75 | 0.39 | 0.20 | 0.32 | 0.28 |
| 782 | BP20;ECM1;LNPEP;MCAM;PIGF | 0.76 | 0.76 | 0.35 | 0.52 | 0.27 | 0.01 |
| 783 | BP20;ENG;SPINT1;IGFALS;ROBO4 | 0.76 | 0.75 | 0.40 | 0.38 | 0.30 | 0.17 |

(continued)

| No. | Panel composition | A | B | C | D | E | F |
|-----|-------------------|-----|-----|-----|-----|-----|-----|
| 784 | BP20;MMRN2;IGFALS;PIGF | 0.76 | 0.77 | 0.43 | 0.32 | 0.01 | 0.45 |
| 785 | BP15;ENG;QSOX1;IGFALS;MCAM | 0.76 | 0.74 | 0.37 | 0.52 | 0.20 | 0.02 |
| 786 | BP20;ADAM12;ENG;IGFALS;MCAM | 0.76 | 0.76 | 0.46 | 0.56 | 0.31 | 0.28 |
| 787 | BP20;ENG;SEPP1;IGFALS;MCAM | 0.76 | 0.74 | 0.45 | 0.48 | 0.18 | 0.16 |
| 788 | *alcoh*;BP20;MMRN2;IGFALS | 0.76 | 0.75 | 0.41 | 0.02 | 0.37 | 0.38 |
| 789 | BP20;ADAM12;ENG;QSOX1;IGFALS | 0.76 | 0.74 | 0.32 | 0.39 | 0.19 | 0.20 |
| 790 | BP20;ADAM12;SEPP1;IGFALS;ENPP2 | 0.76 | 0.74 | 0.32 | 0.37 | 0.37 | 0.18 |
| 791 | bmi;BP15;ENG;SPINT1;MCAM | 0.76 | 0.74 | 0.33 | 0.48 | 0.29 | 0.15 |
| 792 | bmi;ADAM12;IGFALS;pbwgt | 0.76 | 0.74 | 0.33 | 0.16 | 0.36 | 0.13 |
| 793 | BP15;IGFALS;ROBO4;PIGF | 0.76 | 0.74 | 0.38 | 0.32 | 0.06 | 0.38 |
| 794 | *alcoh*;BP20;*fhpet*;IGFALS | 0.76 | 0.74 | 0.35 | 0.28 | 0.25 | 0.24 |
| 795 | BP20;MMRN2;MAPRE1/3;IGFALS;ALDOA;*vagbl* | 0.76 | 0.77 | 0.47 | 0.60 | 0.01 | 0.09 |
| 796 | BP20;ADAM12;ENG;QSOX1;IGFALS;MCAM | 0.76 | 0.76 | 0.40 | 0.60 | 0.21 | 0.27 |
| 797 | BP20;MMRN2;ADAM12;IGFALS | 0.76 | 0.77 | 0.44 | 0.39 | 0.26 | 0.28 |
| 798 | BP20;MMRN2;SEPP1;IGFALS;ENPP2 | 0.76 | 0.75 | 0.36 | 0.22 | 0.35 | 0.15 |
| 799 | BP20;ENG;QSOX1;IGFALS;MCAM | 0.76 | 0.75 | 0.35 | 0.56 | 0.16 | 0.02 |
| 800 | bmi;BP20;ENG;IGFALS;MCAM | 0.76 | 0.75 | 0.43 | 0.56 | 0.40 | 0.16 |
| 801 | BP20;MMRN2;MAPRE1/3;IGFALS;ALDOA | 0.76 | 0.76 | 0.38 | 0.53 | 0.01 | 0.14 |
| 802 | BP20;ADAM12;QSOX1;IGFACS;ROBO4 | 0.76 | 0.76 | 0.41 | 0.49 | 0.19 | 0.24 |
| 803 | BP15;MMRN2;MAPRE1/3;IGFALS;ALDOA | 0.76 | 0.75 | 0.41 | 0.43 | 0.08 | 0.25 |
| 804 | BP15;SEPP1;IGFALS;PIGF | 0.76 | 0.74 | 0.40 | 0.18 | 0.19 | 0.22 |
| 805 | MMRN2:ENG;IGFALS;MCAM;*vagbl* | 0.76 | 0.74 | 0.39 | 0.42 | 0.08 | 0.15 |
| 806 | BP20;ADAM12;ENG;SEPP1;IGFALS | 0.76 | 0.74 | 0.42 | 0.47 | 0.32 | 0.13 |
| 807 | BP15;IGFALS;PIGF | 0.76 | 0.73 | 0.40 | 0.18 | 0.09 | 0.28 |
| 808 | BP20;ADAM12;SEPP1;IGFALS;ROBO4 | 0.76 | 0.75 | 0.43 | 0.53 | 0.37 | 0.23 |
| 809 | *alcoh*;BP20;LNPEP;QSOX1;IGFALS | 0.76 | 0.74 | 0.45 | 0.02 | 0.07 | 0.36 |
| 810 | bmi;BP20;ADAM12;ENG;MCAM | 0.76 | 0.75 | 0.43 | 0.42 | 0.36 | 0.16 |
| 811 | BP20;ADAM12;ENG;SPINT1;MCAM | 0.76 | 0.75 | 0.47 | 0.45 | 0.38 | 0.27 |
| 812 | SP20;ECM1;ENG;SPINT1;MCAM;PIGF | 0.76 | 0.75 | 0.53 | 0.58 | 0.09 | 0.01 |
| 813 | bmi;BP20;ADAM12;ENG;MCAM | 0.76 | 0.74 | 0.45 | 0.44 | 0.37 | 0.01 |
| 814 | BP20;LNPEP;IGFALS;PIGF | 0.76 | 0.76 | 0.37 | 0.22 | 0.30 | 0.47 |
| 815 | BP15;SEPP1;IGFALS;PIGF | 0.76 | 0.74 | 0.48 | 0.20 | 0.24 | 0.26 |
| 816 | bmi;MMRN2;ADAM12;ENG;MCAM | 0.76 | 0.74 | 0.46 | 0.48 | 0.17 | 0.01 |
| 817 | BP15;IGFALS;ROBO4;PIGF | 0.76 | 0.73 | 0.41 | 0.34 | 0.03 | 0.32 |
| 818 | BP20;ADAM12;ENG;SPINT1;MCAM | 0.76 | 0.74 | 0.41 | 0.57 | 0.25 | 0.19 |
| 819 | ADAM12;QSOX1;IGFALS;PIGF | 0.76 | 0.75 | 0.43 | 0.20 | 0.30 | 0.44 |
| 820 | BP20;ADAM12;MAPRE1/3;ALDOA;PIGF | 0.76 | 0.74 | 0.39 | 0.40 | 0.38 | 0.31 |
| 821 | BP20;ENG;SEPP1;IGFALS;PIGF | 0.76 | 0.74 | 0.29 | 0.44 | 0.09 | 0.09 |

(continued)

| No. | Panel composition | A | B | C | D | E | F |
|-----|-------------------|------|------|------|------|------|------|
| 822 | *alcoh*;BP20;QSOX1;IGFALS | 0.76 | 0.74 | 0.40 | 0.06 | 0.30 | 0.24 |
| 823 | BP15;LNPEP;IGFALS;PIGF | 0.76 | 0.74 | 0.41 | 0.10 | 0.29 | 0.26 |
| 824 | BP20;ADAM12;MAPRE1/3;IGFALS;ALDOA | 0.76 | 0.74 | 0.19 | 0.28 | 0.38 | 0.31 |
| 825 | BP20;ECM1;LNPEP;MCAM;PIGF | 0.76 | 0.76 | 0.24 | 0.43 | 0.23 | 0.06 |
| 826 | BP20;MMRN2;ADAM12;IGFALS | 0.76 | 0.77 | 0.46 | 0.35 | 0.31 | 0.29 |
| 827 | BP20;ADAM12;ENG;QSOX1;IGFALS | 0.76 | 0.75 | 0.42 | 0.35 | 0.14 | 0.25 |
| 828 | *alcoh*;BP20;ADAM12;IGFALS | 0.76 | 0.74 | 0.33 | 0.16 | 0.27 | 0.28 |
| 829 | BP20;ADAM12;IGFALS;MCAM;ENPP2 | 0.76 | 0.76 | 0.40 | 0.27 | 0.27 | 0.37 |
| 830 | *alcoh*;BP20;MMRN2;IGFALS | 0.76 | 0.75 | 0.44 | 0.18 | 0.25 | 0.42 |
| 831 | *fhpet*;MMRN2;MAPRE1/3;IGFALS;ALDOA | 0.76 | 0.75 | 0.46 | 0.38 | 0.09 | 0.38 |
| 832 | BP20;ECM1;LNPEP;MCAM;PIGF | 0.76 | 0.76 | 0.42 | 0.40 | 0.13 | 0.03 |
| 833 | BP20;ADAM12;ENG;IGFALS;MCAM;ROBO4 | 0.76 | 0.76 | 0.46 | 0.54 | 0.26 | 0.14 |
| 834 | BP20;MMRN2;ENG;QSOX1;IGFALS | 0.76 | 0.76 | 0.44 | 0.53 | 0.13 | 0.11 |
| 835 | BP20;SEPP1;IGFALS;PIGF | 0.76 | 0.77 | 0.34 | 0.30 | 0.26 | 0.55 |
| 836 | BP15;ADAM12;SEPP1;IGFALS | 0.76 | 0.73 | 0.42 | 0.12 | 0.23 | 0.14 |
| 837 | BP20;MMRN2;ENG;IGFALS;*vagbl* | 0.76 | 0.76 | 0.41 | 0.42 | 0.07 | 0.17 |
| 838 | BP20;MMRN2;ENG;QSOX1;IGFALS | 0.76 | 0.75 | 0.45 | 0.41 | 0.21 | 0.11 |
| 839 | bmi;BP20;ENG;IGFALS;MCAM | 0.76 | 0.74 | 0.39 | 0.50 | 0.42 | 0.13 |
| 840 | BP20;ENG;QSOX1;IGFALS;MCAM;ROBO4 | 0.76 | 0.76 | 0.39 | 0.56 | 0.14 | 0.13 |
| 841 | BP20;MMRN2;ENG;SEPP1;IGFALS | 0.76 | 0.75 | 0.38 | 0.46 | 0.19 | 0.23 |
| 842 | BP20;ENG;SEPP1;IGFALS;PIGF | 0.76 | 0.74 | 0.32 | 0.42 | 0.26 | 0.13 |
| 843 | BP20;MMRN2;ENG;QSOX1;IGFALS | 0.76 | 0.75 | 0.41 | 0.47 | 0.01 | 0.02 |
| 844 | BP20;ADAM12;SEPP1;IGFALS | 0.76 | 0.75 | 0.31 | 0.49 | 0.32 | 0.17 |
| 845 | *alcoh*;BP20;ADAM12;MCAM;PIGF | 0.76 | 0.76 | 0.42 | 0.50 | 0.20 | 0.10 |
| 846 | BP20;ENG;QSOX1;IGFALS;MCAM | 0.76 | 0.75 | 0.34 | 0.60 | 0.16 | 0.02 |
| 847 | BP20;ECM1;SEPP1;IGFALS;ROBO4 | 0.76 | 0.74 | 0.34 | 0.38 | 0.35 | 0.05 |
| 848 | BP20;ENG;QSOX1;IGFALS;MCAM;ROBO4 | 0.76 | 0.76 | 0.41 | 0.58 | 0.21 | 0.13 |
| 849 | BP15;MMRN2;SEPP1;IGFALS;*vagbl* | 0.76 | 0.74 | 0.36 | 0.18 | 0.18 | 0.34 |
| 850 | BP20;*fihd*;ENG;SPINT1;MCAM;PIGF | 0.76 | 0.73 | 0.56 | 0.59 | 0.08 | 0.01 |
| 851 | BP15;MMRN2;ADAM12;IGFALS | 0.76 | 0.74 | 0.44 | 0.31 | 0.25 | 0.29 |
| 852 | *alcoh*;BP20;ADAM12;IGFALS | 0.76 | 0.74 | 0.35 | 0.18 | 0.34 | 0.24 |
| 853 | BP20;ADAM12;QSOX1;IGFALS | 0.76 | 0.75 | 0.38 | 0.35 | 0.30 | 0.26 |
| 854 | ADAM12;IGFALS;ROBO4;PIGF | 0.76 | 0.74 | 0.34 | 0.12 | 0.27 | 0.31 |
| 855 | *alcoh*;ADAM12;SEPP1;IGFALS;ENPP2 | 0.76 | 0.69 | 0.34 | 0.04 | 0.42 | 0.43 |
| 856 | BP15;ADAM12;QSOX1;IGFALS | 0.76 | 0.74 | 0.49 | 0.12 | 0.17 | 0.28 |
| 857 | bmi;ADAM12;QSOX1;IGFALS | 0.76 | 0.73 | 0.53 | 0.12 | 0.35 | 0.33 |
| 858 | *alcoh*;BP20;LNPEP;QSOX1;IGFALS | 0.76 | 0.74 | 0.47 | 0.02 | 0.27 | 0.26 |
| 859 | BP15;MMRN2;ADAM12;IGFALS | 0.76 | 0.76 | 0.46 | 0.20 | 0.32 | 0.40 |

(continued)

| No. | Panel composition | A | B | C | D | E | F |
|---|---|---|---|---|---|---|---|
| 860 | BP20;ADAM12;QSOX1;IGFALS;ROBO4 | 0.76 | 0.75 | 0.34 | 0.33 | 0.16 | 0.25 |
| 861 | *alcoh*;BP20;LNPEP;IGFALS;MCAM | 0.76 | 0.75 | 0.38 | 0.23 | 0.24 | 0.38 |
| 862 | BP15;IGFALS;ROBO4;PIGF | 0.76 | 0.74 | 0.39 | 0.28 | 0.04 | 0.28 |
| 863 | BP20;MMRN2;ECM1;IGFALS;MCAM | 0.76 | 0.77 | 0.44 | 0.47 | 0.33 | 0.30 |
| 864 | BP20;MMRN2;ECM1;LNPEP;PRCP;PIGF | 0.76 | 0.77 | 0.50 | 0.51 | 0.13 | 0.01 |
| 865 | BP20;ENG;QSOX1;IGFALS;MCAM | 0.76 | 0.75 | 0.36 | 0.54 | 0.18 | 0.03 |
| 866 | BP20;ENG;IGFALS;MCAM;ROBO4 | 0.76 | 0.75 | 0.41 | 0.54 | 0.21 | 0.14 |
| 867 | BP20;ADAM12;MCAM;PIGF | 0.76 | 0.76 | 0.41 | 0.50 | 0.19 | 0.06 |
| 868 | BP20;ADAM12;ENG;IGFALS;MCAM | 0.76 | 0.76 | 0.34 | 0.52 | 0.19 | 0.25 |
| 869 | BP20;ADAM12;LCAT;PIGF | 0.76 | 0.74 | 0.33 | 0.35 | 0.21 | 0.37 |
| 870 | BP20;MMRN2;ENG;SEPP1;IGFALS | 0.76 | 0.75 | 0.37 | 0.42 | 0.01 | 0.13 |
| 871 | BP20;ADAM12;QSOX1;IGFALS;ROBO4 | 0.76 | 0.75 | 0.47 | 0.43 | 0.18 | 0.37 |
| 872 | BP20;ADAM12;IGFALS;MCAM | 0.76 | 0.77 | 0.41 | 0.50 | 0.21 | 0.27 |
| 873 | bmi;BP20;ENG;LNPEP;SPINT1;MCAM | 0.76 | 0.76 | 0.41 | 0.59 | 0.29 | 0.13 |
| 874 | BP20;MMRN2;SEPP1;IGFALS;*vagbl* | 0.76 | 0.77 | 0.33 | 0.42 | 0.29 | 0.16 |
| 875 | BP20;MMRN2;MAPRE1/3;IGFALS;ALDOA | 0.76 | 0.77 | 0.41 | 0.50 | 0.12 | 0.14 |
| 876 | BP20;SEPP1;IGFALS;PIGF | 0.76 | 0.76 | 0.35 | 0.26 | 0.26 | 0.47 |
| 877 | BP15;IGFALS;ROBO4;PIGF | 0.76 | 0.74 | 0.37 | 0.34 | 0.24 | 0.38 |
| 878 | BP20;ENG;IGFALS;MCAM | 0.76 | 0.75 | 0.35 | 0.56 | 0.20 | 0.18 |
| 879 | BP20;MMRN2;ECM1;SEPP1;IGFALS | 0.76 | 0.75 | 0.45 | 0.27 | 0.13 | 0.12 |
| 880 | BP15;IGFALS;ROBO4;PIGF | 0.76 | 0.74 | 0.35 | 0.32 | 0.20 | 0.36 |
| 881 | *alcoh*;BP20;LNPEP;QSOX1;IGFALS | 0.76 | 0.74 | 0.41 | 0.02 | 0.18 | 0.28 |
| 882 | BP20;ENG;IGFALS;MCAM;*vagbl* | 0.76 | 0.75 | 0.35 | 0.54 | 0.25 | 0.19 |
| 883 | BP20;ENG;QSOX1;IGFALS;MCAM | 0.76 | 0.76 | 0.37 | 0.54 | 0.13 | 0.03 |
| 884 | BP20;MMRN2;ECM1;ENG;SPINT1;MCAM | 0.76 | 0.79 | 0.52 | 0.56 | 0.21 | 0.20 |
| 885 | ADAM12;IGFALS;PIGF | 0.76 | 0.73 | 0.32 | 0.10 | 0.28 | 0.27 |
| 886 | BP20;MMRN2;MAPRE1/3;IGFALS;ALDOA | 0.76 | 0.76 | 0.45 | 0.45 | 0.01 | 0.08 |
| 887 | BP20;MMRN2;ENG;QSOX1;IGFALS | 0.76 | 0.76 | 0.41 | 0.53 | 0.12 | 0.12 |
| 888 | BP20;ADAM12;ALDOA;MCAM;PIGF | 0.76 | 0.77 | 0.41 | 0.48 | 0.26 | 0.18 |
| 889 | MMRN2;MAPRE1/3;IGFALS;ALDOA;*vagbl* | 0.76 | 0.76 | 0.43 | 0.28 | 0.12 | 0.19 |
| 890 | BP20;SEPP1;IGFALS;ROBO4;*vagbl* | 0.76 | 0.75 | 0.28 | 0.44 | 0.27 | 0.08 |
| 891 | BP20;ADAM12;ECM1;PIGF | 0.76 | 0.74 | 0.34 | 0.27 | 0.20 | 0.02 |
| 892 | MMRN2;IGFALS;PIGF | 0.76 | 0.74 | 0.43 | 0.32 | 0.19 | 0.32 |
| 893 | BP20;ADAM12;ECM1;ENG;MCAM | 0.76 | 0.75 | 0.31 | 0.45 | 0.21 | 0.12 |
| 894 | BP15;MMRN2;IGFALS;*vagbl* | 0.76 | 0.74 | 0.33 | 0.34 | 0.14 | 0.40 |
| 895 | BP20;ENG;IGFALS;MCAM;*vagbl* | 0.76 | 0.75 | 0.32 | 0.56 | 0.18 | 0.21 |
| 896 | BP24;ADAM12;ENG;IGFALS;MCAM | 0.76 | 0.76 | 0.41 | 0.52 | 0.22 | 0.33 |
| 897 | BP20;MMRN2;ADAM12;IGFALS | 0.76 | 0.76 | 0.40 | 0.24 | 0.21 | 0.34 |

(continued)

| No. | Panel composition | A | B | C | D | E | F |
|---|---|---|---|---|---|---|---|
| 898 | BP20;MMRN2;LNPEP;IGFALS;ENPP2 | 0.76 | 0.75 | 0.39 | 0.43 | 0.33 | 0.21 |
| 899 | MMRN2;ADAM12;QSOX1;IGFALS | 0.76 | 0.74 | 0.46 | 0.20 | 0.14 | 0.23 |
| 900 | BP20;ENG;SPINT1;IGFALS | 0.76 | 0.74 | 0.38 | 0.38 | 0.15 | 0.18 |
| 901 | BP15;ADAM12;MCAM;PIGF | 0.76 | 0.74 | 0.49 | 0.35 | 0.27 | 0.26 |
| 902 | BP15;ADAM12;IGFALS;MCAM | 0.76 | 0.75 | 0.39 | 0.31 | 0.13 | 0.30 |
| 903 | BP20;*fhpet*SEPP1;IGFALS;ROBO4 | 0.76 | 0.75 | 0.33 | 0.40 | 0.29 | 0.22 |
| 904 | BP20;MMRN2;ENG;IGFALS;*vagbl* | 0.76 | 0.76 | 0.33 | 0.40 | 0.13 | 0.27 |
| 905 | BP20;ADAM12;SEPP1;IGFALS | 0.76 | 0.74 | 0.33 | 0.35 | 0.27 | 0.17 |
| 906 | BP20;MMRN2;ENG;QSOX1;IGFALS | 0.76 | 0.75 | 0.42 | 0.51 | 0.00 | 0.02 |
| 907 | BP20;MMRN2;ENG;IGFALS | 0.76 | 0.75 | 0.40 | 0.40 | 0.30 | 0.17 |
| 908 | BP20;*fhpet*,IGFALS;PIGF | 0.76 | 0.75 | 0.43 | 0.32 | 0.13 | 0.46 |
| 909 | *alcoh*;BP20;IGFALS;ROBO4 | 0.76 | 0.74 | 0.42 | 0.26 | 0.35 | 0.12 |
| 910 | BP20;ADAM12;IGFALS;ROBO4 | 0.76 | 0.74 | 0.40 | 0.47 | 0.20 | 0.27 |
| 911 | BP20;LNPEP;SEPP1;IGFALS;ROBO4 | 0.76 | 0.75 | 0.29 | 0.45 | 0.32 | 0.11 |
| 912 | bmi;BP15;ADAM12;MCAM | 0.76 | 0.74 | 0.36 | 0.25 | 0.22 | 0.36 |
| 913 | BP20;MMRN2;SEPP1;IGFALS;*vagbl* | 0.76 | 0.76 | 0.33 | 0.32 | 0.25 | 0.15 |
| 914 | MAPRE1/3;IGFALS;ALDOA;PIGF | 0.76 | 0.74 | 0.29 | 0.28 | 0.32 | 0.32 |
| 915 | BP15;IGFALS;PIGF | 0.76 | 0.73 | 0.36 | 0.16 | 0.16 | 0.28 |
| 916 | BP20;SEPP1;IGFALS;PIGF | 0.76 | 0.76 | 0.24 | 0.28 | 0.30 | 0.17 |
| 917 | MMRN2;ADAM12;IGFALS;MCAM | 0.76 | 0.75 | 0.43 | 0.46 | 0.16 | 0.23 |
| 918 | BP20;ADAM12;QSOX1;IGFALS | 0.76 | 0.76 | 0.36 | 0.33 | 0.13 | 0.26 |
| 919 | BP15;IGFALS;PIGF | 0.76 | 0.73 | 0.39 | 0.20 | 0.18 | 0.28 |
| 920 | BP15;ADAM12;QSOX1;IGFALS | 0.76 | 0.74 | 0.46 | 0.12 | 0.17 | 0.29 |
| 921 | BP20;ADAM12;SEPP1;IGFALS | 0.76 | 0.74 | 0.36 | 0.45 | 0.26 | 0.21 |
| 922 | BP20;MMRN2;ECM1;ENG;SPINT1;MCAM | 0.76 | 0.77 | 0.53 | 0.55 | 0.23 | 0.03 |
| 923 | BP20;MMRN2;ENG;IGFALS;*vagbl* | 0.75 | 0.75 | 0.40 | 0.38 | 0.00 | 0.16 |
| 924 | *alcoh*;BP20;LNPEP;IGFALS;MCAM | 0.75 | 0.75 | 0.42 | 0.15 | 0.21 | 0.30 |
| 925 | BP20;ENG;IGFALS;ROBO4;PIGF | 0.75 | 0.75 | 0.41 | 0.42 | 0.32 | 0.11 |
| 926 | *alcoh*;BP20;IGFALS | 0.75 | 0.73 | 0.34 | 0.18 | 0.25 | 0.24 |
| 927 | BP20;ENG;IGFALS;MCAM;ROBO4 | 0.75 | 0.75 | 0.39 | 0.44 | 0.25 | 0.15 |
| 928 | BP20;ADAM12;QSOX1;IGFALS | 0.75 | 0.75 | 0.40 | 0.27 | 0.24 | 0.29 |
| 929 | bmi;BP20;ADAM12;PIGF | 0.75 | 0.75 | 0.35 | 0.29 | 0.20 | 0.19 |
| 930 | BP20;ECM1;ENG;SPINT1;MCAM | 0.75 | 0.77 | 0.48 | 0.49 | 0.27 | 0.17 |
| 931 | BP20;ADAM12;OSOX1;IGFALS;ROBO4 | 0.75 | 0.76 | 0.49 | 0.45 | 0.13 | 0.39 |
| 932 | BP20;ADAM12;IGFALS;MCAM | 0.75 | 0.76 | 0.39 | 0.46 | 0.16 | 0.28 |
| 933 | BP20;ADAM12;MCAM;PIGF | 0.75 | 0.76 | 0.38 | 0.58 | 0.14 | 0.14 |
| 934 | BP15;MMRN2;IGFALS;*vagbl* | 0.75 | 0.74 | 0.35 | 0.22 | 0.13 | 0.34 |
| 935 | *alcoh*;BP20;IGFALS;MCAM | 0.75 | 0.75 | 0.35 | 0.06 | 0.26 | 0.32 |

(continued)

| No. | Panel composition | A | B | C | D | E | F |
|---|---|---|---|---|---|---|---|
| 936 | BP20;ECM1:LNPEP;MCAM;PIGF | 0.75 | 0.75 | 0.39 | 0.33 | 0.16 | 0.02 |
| 937 | BP20;ENG;IGFALS;MCAM | 0.75 | 0.74 | 0.32 | 0.48 | 0.25 | 0.21 |
| 938 | BP20;ADAM12;IGFALS;MCAM | 0.75 | 0.77 | 0.38 | 0.48 | 0.21 | 0.32 |
| 939 | BP20;MMRN2;SEPP1;IGFALS | 0.75 | 0.76 | 0.34 | 0.44 | 0.24 | 0.18 |
| 940 | BP20;ADAM12;OSOX1;IGFALS | 0.75 | 0.75 | 0.45 | 0.41 | 0.13 | 0.27 |
| 941 | BP20;IGFALS;ROBO4;PIGF | 0.75 | 0.76 | 0.38 | 0.30 | 0.30 | 0.45 |
| 942 | BP20;ADAM12;IGFALS;ROBO4 | 0.75 | 0.74 | 0.44 | 0.41 | 0.21 | 0.24 |
| 943 | BP20;ADAM12;ECM1;PIGF | 0.75 | 0.74 | 0.30 | 0.42 | 0.31 | 0.12 |
| 944 | BP20;ADAM12;QSOX1;IGFALS | 0.75 | 0.75 | 0.36 | 0.33 | 0.25 | 0.27 |
| 945 | BP20;ENG;IGFALS;MCAM | 0.75 | 0.75 | 0.29 | 0.52 | 0.19 | 0.21 |
| 946 | BP20;ADAM12;QSOX1;IGFALS | 0.75 | 0.75 | 0.39 | 0.33 | 0.12 | 0.38 |
| 947 | BP20;IGFALS;ROBO4;PIGF | 0.75 | 0.75 | 0.37 | 0.34 | 0.26 | 0.40 |
| 948 | BP15;MMRN2;IGFALS;*vagbl* | 0.75 | 0.74 | 0.44 | 0.30 | 0.15 | 0.38 |
| 949 | BP20;IGFALS;PIGF | 0.75 | 0.75 | 0.39 | 0.30 | 0.24 | 0.36 |
| 950 | BP20;ADAM12;MCAM;PIGF | 0.75 | 0.75 | 0.36 | 0.46 | 0.22 | 0.07 |
| 951 | BP20;ADAM12;ENPP2;PIGF | 0.75 | 0.75 | 0.29 | 0.24 | 0.21 | 0.25 |
| 952 | bmi;BP20;ADAM12;PIGF | 0.75 | 0.74 | 0.33 | 0.33 | 0.21 | 0.12 |
| 953 | BP20;MMRN2;SEPP1;IGFALS | 0.75 | 0.76 | 0.32 | 0.30 | 0.25 | 0.16 |
| 954 | BP20;MMRN2;LNPEP;IGFALS | 0.75 | 0.76 | 0.41 | 0.43 | 0.18 | 0.25 |
| 955 | bmi;BP15;ADAM12;MCAM | 0.75 | 0.75 | 0.43 | 0.23 | 0.20 | 0.48 |
| 956 | BP20;MMRN2;IGFALS;*vagbl* | 0.75 | 0.76 | 0.35 | 0.34 | 0.16 | 0.45 |
| 957 | BP20;MMRN2;SEPP1;IGFALS | 0.75 | 0.75 | 0.40 | 0.38 | 0.16 | 0.30 |
| 958 | BP20:*fihd*;MMRN2;PRDX2;IGFALS | 0.75 | 0.75 | 0.47 | 0.34 | 0.00 | 0.19 |
| 959 | BP20;*fhpet*,LNPEP;MCAM;PIGF | 0.75 | 0.75 | 0.34 | 0.54 | 0.23 | 0.03 |
| 960 | BP20;SEPP1;IGFALS;*vagbl* | 0.75 | 0.75 | 0.33 | 0.38 | 0.31 | 0.14 |
| 961 | BP20;MMRN2;ENG;IGFALS | 0.75 | 0.74 | 0.38 | 0.42 | 0.00 | 0.17 |
| 962 | BP20;LNPEP;SEPP1;IGFALS | 0.75 | 0.74 | 0.31 | 0.37 | 0.26 | 0.15 |
| 963 | BP20;MMRN2;ENG;IGFALS | 0.75 | 0.75 | 0.31 | 0.38 | 0.13 | 0.27 |
| 964 | BP20;SEPP1;IGFALS;ROBO4 | 0.75 | 0.75 | 0.27 | 0.42 | 0.28 | 0.09 |
| 965 | BP20;IGFALS;PIGF | 0.75 | 0.75 | 0.32 | 0.32 | 0.22 | 0.31 |
| 966 | BP15;MMRN2;IGFALS;*vagbl* | 0.75 | 0.74 | 0.36 | 0.24 | 0.19 | 0.42 |
| 967 | BP20;*fhpet*;ECM1;MCAM;PIGF | 0.75 | 0.75 | 0.34 | 0.49 | 0.11 | 0.01 |
| 968 | MMRN2;ADAM12;IGFALS | 0.75 | 0.73 | 0.45 | 0.18 | 0.22 | 0.27 |
| 969 | MMRN2;MAPRE1/3;IGFALS;ALDOA | 0.75 | 0.74 | 0.48 | 0.30 | 0.12 | 0.20 |
| 970 | BP20;LNPEP;IGFALS;MCAM | 0.75 | 0.76 | 0.42 | 0.42 | 0.24 | 0.38 |
| 971 | BP20;ECM1;ENG;SPINT1;MCAM | 0.75 | 0.77 | 0.31 | 0.44 | 0.19 | 0.25 |
| 972 | BP20;IGFALS;ROBO4;PIGF | 0.75 | 0.75 | 0.40 | 0.28 | 0.03 | 0.38 |
| 973 | BP20;LNPEP;QSOX1;IGFALS | 0.75 | 0.74 | 0.34 | 0.39 | 0.19 | 0.30 |

(continued)

| No. | Panel composition | A | B | C | D | E | F |
|---|---|---|---|---|---|---|---|
| 974 | BP20;MMRN2;IGFALS;*vagbl* | 0.75 | 0.76 | 0.40 | 0.34 | 0.13 | 0.38 |
| 975 | BP20;*fhpet*;MMRN2;IGFALS | 0.75 | 0.75 | 0.35 | 0.28 | 0.18 | 0.35 |
| 976 | BP20;IGFALS;PIGF | 0.75 | 0.74 | 0.41 | 0.26 | 0.34 | 0.36 |
| 977 | BP20;*fhpet*;SEPP1;IGFALS | 0.75 | 0.75 | 0.29 | 0.36 | 0.21 | 0.15 |
| 978 | *alcoh*;BP20;ADAM12;ECM1;MCAM | 0.75 | 0.75 | 0.35 | 0.43 | 0.27 | 0.26 |
| 979 | BP20;ECM1;ENG;SPINT1;MCAM | 0.75 | 0.75 | 0.33 | 0.49 | 0.13 | 0.06 |
| 980 | BP20;ADAM12;IGFALS | 0.75 | 0.74 | 0.31 | 0.45 | 0.25 | 0.30 |
| 981 | *alcoh*;BP20;ENG;SPINT1;MCAM | 0.74 | 0.76 | 0.41 | 0.48 | 0.18 | 0.05 |
| 982 | BF20;ADAM12;ECM1;MCAM | 0.74 | 0.75 | 0.35 | 0.51 | 0.19 | 0.15 |
| 983 | BP20;MMRN2;IGFALS;*vagbl* | 0.74 | 0.76 | 0.38 | 0.26 | 0.18 | 0.25 |
| 984 | BP20;MMRN2;LNPEP;IGFALS | 0.74 | 0.75 | 0.33 | 0.39 | 0.15 | 0.37 |
| 985 | BP20;LNPEP;IGFALS;MCAM | 0.74 | 0.75 | 0.41 | 0.31 | 0.15 | 0.40 |
| 986 | BP20;MMRN2;IGFALS | 0.74 | 0.76 | 0.34 | 0.34 | 0.15 | 0.45 |
| 987 | BP20;LNPEP;MCAM;PIGF | 0.74 | 0.75 | 0.16 | 0.48 | 0.18 | 0.03 |
| 988 | *alcoh*;BP20;MMRN2;ECM1;PRCP;PIGF | 0.74 | 0.73 | 0.50 | 0.51 | 0.08 | 0.01 |
| 989 | BP20;MMRN2;ADAM12;ENG;MCAM | 0.74 | 0.77 | 0.38 | 0.46 | 0.06 | 0.31 |
| 990 | BP20;SEPP1;IGFALS | 0.74 | 0.74 | 0.33 | 0.40 | 0.29 | 0.15 |
| 991 | BP20;MMRN2;IGFALS | 0.74 | 0.75 | 0.36 | 0.24 | 0.13 | 0.26 |
| 992 | BP20;MMRN2;ECM1;ENG;MCAM | 0.74 | 0.76 | 0.28 | 0.43 | 0.18 | 0.02 |
| 993 | BP20;MMRN2;IGFALS | 0.74 | 0.75 | 0.38 | 0.38 | 0.13 | 0.40 |
| 994 | BP20;ADAM12;PIGF | 0.74 | 0.74 | 0.29 | 0.31 | 0.13 | 0.02 |
| 995 | BP20;IGFALS;MCAM | 0.74 | 0.74 | 0.40 | 0.44 | 0.29 | 0.44 |
| 996 | BP20;ADAM12;PIGF | 0.74 | 0.75 | 0.25 | 0.37 | 0.07 | 0.12 |
| 997 | BP20;IGFALS;MCAM | 0.74 | 0.74 | 0.41 | 0.35 | 0.12 | 0.37 |
| 998 | bmi;BP20;ENG;SPINT1;MCAM | 0.73 | 0.77 | 0.31 | 0.55 | 0.23 | 0.15 |
| 999 | BP20;ADAM12;ECM1;MCAM | 0.73 | 0.76 | 0.28 | 0.47 | 0.19 | 0.27 |
| 1000 | *alcoh*;BP20;ECM1;MCAM;PIGF | 0.75 | 0.75 | 0.49 | 0.32 | 0.14 | 0.00 |
| 1001 | bmi;*fhpet*;ENG;SPINT1;MCAM | 0.75 | 0.75 | 0.33 | 0.43 | 0.26 | 0.09 |
| 1002 | BP15;*fhpet*;ECM1;ENG;SPINT1;MCAM | 0.77 | 0.75 | 0.49 | 0.51 | 0.08 | 0.05 |
| 1003 | BP15;MMRN2;ENG;SPINT1;MCAM;ENPP2 | 0.76 | 0.75 | 0.43 | 0.52 | 0.01 | 0.04 |
| 1004 | bmi;BP20;MMRN2;ECM1;ENG;MCAM | 0.75 | 0.76 | 0.46 | 0.57 | 0.21 | 0.01 |
| 1005 | bmi;BP20;ENG;SPINT1;MCAM;PIGF | 0.75 | 0.76 | 0.43 | 0.59 | 0.13 | 0.09 |
| 1006 | BP20;ENG;SPINT1;MCAM;ENPP2;PIGF | 0.75 | 0.75 | 0.46 | 0.59 | 0.18 | 0.01 |
| 1007 | *alcoh*;BP20;*fhpet*,ADAM12;LCAT;MCAM | 0.75 | 0.75 | 0.39 | 0.60 | 0.25 | 0.16 |
| 1008 | *alcoh*;BP20;ENG;SPINT1;MCAM;PIGF | 0.75 | 0.75 | 0.39 | 0.64 | 0.13 | 0.01 |

Table 4B

| No. | G | H | I | J | K | L | M | N | O | P | Q | R | S | T | U | V | W | Z | AA | AB | AC | AD | AE | AF | AG | AH | AI |
|-----|------|------|------|------|------|------|------|------|------|------|------|------|------|------|------|------|------|------|------|------|------|------|------|------|------|------|------|
| 1 | 0.75 | 0.67 | 0.83 | 0.89 | 0.72 | 1.00 | 0.71 | 0.62 | 0.80 | 0.81 | 0.76 | 0.86 | 0.92 | 0.87 | 0.96 | 0.77 | 0.71 | 0.83 | 0.81 | 0.76 | 0.85 | 0.92 | 0.87 | 0.97 | 0.76 | 0.71 | 0.81 |
| 2 | 0.74 | 0.66 | 0.83 | 0.78 | 0.57 | 0.98 | 0.73 | 0.64 | 0.83 | 0.81 | 0.76 | 0.87 | 0.84 | 0.75 | 0.94 | 0.80 | 0.74 | 0.86 | 0.80 | 0.76 | 0.85 | 0.84 | 0.75 | 0.93 | 0.79 | 0.74 | 0.84 |
| 3 | 0.75 | 0.68 | 0.83 | 0.83 | 0.68 | 0.99 | 0.73 | 0.65 | 0.81 | 0.80 | 0.74 | 0.85 | 0.84 | 0.74 | 0.93 | 0.78 | 0.72 | 0.85 | 0.80 | 0.75 | 0.84 | 0.85 | 0.78 | 0.92 | 0.78 | 0.73 | 0.82 |
| 4 | 0.75 | 0.67 | 0.83 | 0.89 | 0.72 | 1.00 | 0.71 | 0.62 | 0.80 | 0.81 | 0.75 | 0.86 | 0.92 | 0.88 | 0.96 | 0.76 | 0.69 | 0.82 | 0.80 | 0.76 | 0.84 | 0.92 | 0.87 | 0.97 | 0.75 | 0.70 | 0.80 |
| 5 | 0.76 | 0.69 | 0.83 | 0.86 | 0.71 | 1.00 | 0.74 | 0.66 | 0.81 | 0.79 | 0.73 | 0.85 | 0.84 | 0.76 | 0.93 | 0.77 | 0.70 | 0.83 | 0.79 | 0.75 | 0.83 | 0.86 | 0.79 | 0.93 | 0.77 | 0.72 | 0.81 |
| 6 | 0.76 | 0.68 | 0.84 | 0.89 | 0.75 | 1.00 | 0.72 | 0.64 | 0.81 | 0.81 | 0.76 | 0.86 | 0.90 | 0.83 | 0.97 | 0.78 | 0.71 | 0.84 | 0.80 | 0.76 | 0.84 | 0.90 | 0.85 | 0.96 | 0.77 | 0.72 | 0.82 |
| 7 | 0.76 | 0.69 | 0.84 | 0.89 | 0.74 | 1.00 | 0.73 | 0.64 | 0.81 | 0.81 | 0.76 | 0.86 | 0.90 | 0.83 | 0.97 | 0.78 | 0.71 | 0.84 | 0.80 | 0.76 | 0.84 | 0.90 | 0.84 | 0.96 | 0.77 | 0.72 | 0.82 |
| 8 | 0.76 | 0.68 | 0.84 | 0.89 | 0.75 | 1.00 | 0.72 | 0.63 | 0.80 | 0.81 | 0.76 | 0.86 | 0.90 | 0.84 | 0.97 | 0.78 | 0.71 | 0.84 | 0.80 | 0.76 | 0.84 | 0.91 | 0.85 | 0.96 | 0.76 | 0.71 | 0.81 |
| 9 | 0.75 | 0.68 | 0.83 | 0.84 | 0.69 | 0.99 | 0.73 | 0.65 | 0.81 | 0.79 | 0.73 | 0.85 | 0.82 | 0.73 | 0.91 | 0.77 | 0.71 | 0.84 | 0.79 | 0.75 | 0.83 | 0.85 | 0.78 | 0.92 | 0.77 | 0.72 | 0.82 |
| 10 | 0.76 | 0.68 | 0.85 | 0.87 | 0.71 | 1.00 | 0.73 | 0.64 | 0.82 | 0.81 | 0.76 | 0.86 | 0.90 | 0.84 | 0.95 | 0.77 | 0.71 | 0.83 | 0.81 | 0.77 | 0.85 | 0.90 | 0.85 | 0.96 | 0.77 | 0.72 | 0.82 |
| 11 | 0.74 | 0.66 | 0.83 | 0.89 | 0.71 | 1.00 | 0.70 | 0.61 | 0.80 | 0.80 | 0.75 | 0.85 | 0.91 | 0.87 | 0.95 | 0.76 | 0.69 | 0.82 | 0.80 | 0.76 | 0.84 | 0.92 | 0.87 | 0.97 | 0.75 | 0.70 | 0.80 |
| 12 | 0.72 | 0.64 | 0.81 | 0.87 | 0.71 | 1.00 | 0.68 | 0.58 | 0.78 | 0.80 | 0.75 | 0.85 | 0.89 | 0.84 | 0.94 | 0.77 | 0.70 | 0.83 | 0.80 | 0.75 | 0.84 | 0.91 | 0.85 | 0.96 | 0.76 | 0.71 | 0.81 |
| 13 | 0.76 | 0.68 | 0.84 | 0.89 | 0.71 | 1.00 | 0.72 | 0.64 | 0.81 | 0.80 | 0.75 | 0.86 | 0.89 | 0.82 | 0.97 | 0.77 | 0.71 | 0.84 | 0.80 | 0.76 | 0.84 | 0.90 | 0.84 | 0.96 | 0.76 | 0.71 | 0.82 |
| 14 | 0.79 | 0.73 | 0.86 | 0.88 | 0.78 | 0.98 | 0.76 | 0.69 | 0.84 | 0.81 | 0.76 | 0.87 | 0.89 | 0.81 | 0.98 | 0.78 | 0.72 | 0.85 | 0.81 | 0.77 | 0.85 | 0.89 | 0.83 | 0.95 | 0.79 | 0.74 | 0.83 |
| 15 | 0.77 | 0.69 | 0.86 | 0.88 | 0.75 | 1.00 | 0.74 | 0.64 | 0.84 | 0.80 | 0.75 | 0.86 | 0.92 | 0.87 | 0.97 | 0.76 | 0.69 | 0.82 | 0.81 | 0.76 | 0.85 | 0.92 | 0.87 | 0.97 | 0.77 | 0.71 | 0.81 |
| 16 | 0.75 | 0.68 | 0.83 | 0.89 | 0.75 | 1.00 | 0.71 | 0.63 | 0.80 | 0.80 | 0.75 | 0.86 | 0.90 | 0.83 | 0.96 | 0.77 | 0.70 | 0.83 | 0.80 | 0.75 | 0.84 | 0.90 | 0.85 | 0.96 | 0.76 | 0.71 | 0.81 |
| 17 | 0.75 | 0.67 | 0.83 | 0.80 | 0.65 | 0.95 | 0.73 | 0.64 | 0.83 | 0.82 | 0.76 | 0.87 | 0.90 | 0.82 | 0.97 | 0.79 | 0.72 | 0.85 | 0.80 | 0.76 | 0.84 | 0.87 | 0.79 | 0.94 | 0.78 | 0.72 | 0.83 |
| 18 | 0.76 | 0.67 | 0.84 | 0.89 | 0.74 | 1.00 | 0.72 | 0.62 | 0.81 | 0.80 | 0.75 | 0.86 | 0.91 | 0.85 | 0.96 | 0.76 | 0.69 | 0.82 | 0.80 | 0.76 | 0.85 | 0.91 | 0.86 | 0.97 | 0.76 | 0.71 | 0.82 |
| 19 | 0.77 | 0.68 | 0.85 | 0.88 | 0.74 | 1.00 | 0.73 | 0.64 | 0.83 | 0.80 | 0.75 | 0.86 | 0.92 | 0.87 | 0.97 | 0.78 | 0.72 | 0.85 | 0.80 | 0.76 | 0.85 | 0.92 | 0.87 | 0.97 | 0.76 | 0.71 | 0.82 |
| 20 | 0.75 | 0.66 | 0.83 | 0.80 | 0.65 | 0.95 | 0.73 | 0.63 | 0.82 | 0.82 | 0.76 | 0.87 | 0.90 | 0.83 | 0.97 | 0.76 | 0.69 | 0.83 | 0.80 | 0.75 | 0.84 | 0.87 | 0.80 | 0.94 | 0.77 | 0.72 | 0.83 |
| 21 | 0.76 | 0.68 | 0.85 | 0.86 | 0.69 | 1.00 | 0.74 | 0.65 | 0.83 | 0.80 | 0.75 | 0.86 | 0.91 | 0.87 | 0.96 | 0.76 | 0.69 | 0.82 | 0.80 | 0.76 | 0.84 | 0.90 | 0.85 | 0.96 | 0.76 | 0.71 | 0.81 |
| 22 | 0.76 | 0.67 | 0.84 | 0.89 | 0.73 | 1.00 | 0.72 | 0.63 | 0.81 | 0.80 | 0.75 | 0.85 | 0.91 | 0.86 | 0.96 | 0.76 | 0.69 | 0.84 | 0.80 | 0.76 | 0.85 | 0.91 | 0.86 | 0.97 | 0.77 | 0.72 | 0.82 |
| 23 | 0.76 | 0.69 | 0.83 | 0.83 | 0.70 | 0.96 | 0.74 | 0.66 | 0.82 | 0.81 | 0.76 | 0.86 | 0.89 | 0.81 | 0.97 | 0.78 | 0.72 | 0.84 | 0.80 | 0.76 | 0.84 | 0.87 | 0.81 | 0.94 | 0.77 | 0.73 | 0.82 |
| 24 | 0.77 | 0.70 | 0.85 | 0.86 | 0.74 | 0.98 | 0.75 | 0.66 | 0.83 | 0.81 | 0.75 | 0.86 | 0.89 | 0.82 | 0.96 | 0.78 | 0.70 | 0.83 | 0.80 | 0.76 | 0.85 | 0.89 | 0.83 | 0.94 | 0.77 | 0.71 | 0.82 |
| 25 | 0.76 | 0.68 | 0.84 | 0.88 | 0.72 | 1.00 | 0.73 | 0.64 | 0.82 | 0.80 | 0.75 | 0.86 | 0.91 | 0.85 | 0.97 | 0.76 | 0.69 | 0.82 | 0.81 | 0.76 | 0.85 | 0.91 | 0.85 | 0.96 | 0.76 | 0.71 | 0.82 |
| 26 | 0.77 | 0.69 | 0.85 | 0.88 | 0.74 | 1.00 | 0.74 | 0.64 | 0.83 | 0.80 | 0.75 | 0.86 | 0.92 | 0.87 | 0.97 | 0.76 | 0.69 | 0.82 | 0.80 | 0.76 | 0.85 | 0.91 | 0.86 | 0.97 | 0.76 | 0.70 | 0.81 |
| 27 | 0.75 | 0.66 | 0.83 | 0.89 | 0.73 | 1.00 | 0.71 | 0.61 | 0.80 | 0.80 | 0.75 | 0.85 | 0.91 | 0.86 | 0.96 | 0.76 | 0.69 | 0.82 | 0.80 | 0.76 | 0.84 | 0.91 | 0.86 | 0.96 | 0.75 | 0.72 | 0.82 |
| 28 | 0.77 | 0.68 | 0.85 | 0.88 | 0.72 | 1.00 | 0.73 | 0.64 | 0.83 | 0.81 | 0.76 | 0.86 | 0.89 | 0.83 | 0.95 | 0.77 | 0.71 | 0.83 | 0.81 | 0.77 | 0.85 | 0.90 | 0.80 | 0.94 | 0.72 | 0.71 | 0.82 |
| 29 | 0.76 | 0.68 | 0.84 | 0.88 | 0.72 | 0.97 | 0.73 | 0.63 | 0.82 | 0.80 | 0.75 | 0.86 | 0.90 | 0.80 | 0.96 | 0.76 | 0.69 | 0.84 | 0.80 | 0.76 | 0.84 | 0.88 | 0.81 | 0.96 | 0.77 | 0.71 | 0.82 |
| 30 | 0.77 | 0.69 | 0.84 | 0.86 | 0.74 | 1.00 | 0.74 | 0.65 | 0.82 | 0.81 | 0.75 | 0.86 | 0.90 | 0.84 | 0.96 | 0.78 | 0.72 | 0.84 | 0.80 | 0.76 | 0.84 | 0.90 | 0.85 | 0.94 | 0.77 | 0.72 | 0.82 |
| 31 | 0.73 | 0.64 | 0.81 | 0.86 | 0.71 | 1.00 | 0.69 | 0.59 | 0.78 | 0.80 | 0.75 | 0.85 | 0.90 | 0.84 | 0.96 | 0.76 | 0.69 | 0.82 | 0.79 | 0.75 | 0.84 | 0.90 | 0.85 | 0.96 | 0.75 | 0.70 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 32 | 0.75 | 0.66 | 0.83 | 0.85 | 0.69 | 1.00 | 0.72 | 0.62 | 0.82 | 0.80 | 0.75 | 0.85 | 0.90 | 0.83 | 0.96 | 0.76 | 0.69 | 0.83 | 0.80 | 0.75 | 0.84 | 0.89 | 0.83 | 0.95 | 0.76 | 0.71 | 0.82 |
| 33 | 0.76 | 0.69 | 0.84 | 0.86 | 0.74 | 0.98 | 0.73 | 0.65 | 0.82 | 0.81 | 0.75 | 0.86 | 0.89 | 0.82 | 0.96 | 0.78 | 0.71 | 0.84 | 0.80 | 0.76 | 0.84 | 0.88 | 0.83 | 0.94 | 0.77 | 0.72 | 0.82 |
| 34 | 0.75 | 0.67 | 0.84 | 0.86 | 0.70 | 1.00 | 0.72 | 0.63 | 0.82 | 0.80 | 0.75 | 0.86 | 0.91 | 0.85 | 0.97 | 0.76 | 0.69 | 0.83 | 0.80 | 0.76 | 0.85 | 0.91 | 0.85 | 0.96 | 0.77 | 0.71 | 0.82 |
| 35 | 0.75 | 0.68 | 0.83 | 0.83 | 0.70 | 0.95 | 0.73 | 0.65 | 0.82 | 0.81 | 0.75 | 0.86 | 0.88 | 0.81 | 0.95 | 0.78 | 0.71 | 0.84 | 0.79 | 0.75 | 0.83 | 0.86 | 0.80 | 0.93 | 0.76 | 0.71 | 0.81 |
| 36 | 0.78 | 0.69 | 0.86 | 0.89 | 0.75 | 1.00 | 0.74 | 0.65 | 0.84 | 0.80 | 0.74 | 0.85 | 0.92 | 0.87 | 0.96 | 0.75 | 0.68 | 0.82 | 0.81 | 0.76 | 0.85 | 0.92 | 0.87 | 0.97 | 0.77 | 0.71 | 0.82 |
| 37 | 0.75 | 0.67 | 0.83 | 0.80 | 0.64 | 0.95 | 0.74 | 0.64 | 0.83 | 0.81 | 0.76 | 0.87 | 0.89 | 0.81 | 0.97 | 0.78 | 0.72 | 0.85 | 0.80 | 0.75 | 0.84 | 0.86 | 0.79 | 0.94 | 0.78 | 0.72 | 0.83 |
| 38 | 0.75 | 0.66 | 0.83 | 0.84 | 0.68 | 1.00 | 0.72 | 0.62 | 0.82 | 0.80 | 0.74 | 0.86 | 0.89 | 0.83 | 0.96 | 0.76 | 0.69 | 0.83 | 0.80 | 0.75 | 0.84 | 0.89 | 0.83 | 0.95 | 0.76 | 0.71 | 0.82 |
| 39 | 0.76 | 0.68 | 0.85 | 0.88 | 0.72 | 1.00 | 0.73 | 0.64 | 0.82 | 0.80 | 0.75 | 0.86 | 0.89 | 0.83 | 0.95 | 0.77 | 0.71 | 0.83 | 0.81 | 0.77 | 0.85 | 0.90 | 0.85 | 0.96 | 0.77 | 0.72 | 0.82 |
| 40 | 0.75 | 0.67 | 0.83 | 0.79 | 0.62 | 0.96 | 0.74 | 0.65 | 0.83 | 0.82 | 0.76 | 0.87 | 0.86 | 0.77 | 0.95 | 0.80 | 0.74 | 0.86 | 0.80 | 0.76 | 0.84 | 0.84 | 0.76 | 0.92 | 0.79 | 0.74 | 0.84 |
| 41 | 0.76 | 0.68 | 0.84 | 0.86 | 0.70 | 1.00 | 0.73 | 0.64 | 0.83 | 0.80 | 0.75 | 0.85 | 0.90 | 0.85 | 0.95 | 0.76 | 0.69 | 0.82 | 0.80 | 0.76 | 0.84 | 0.90 | 0.84 | 0.95 | 0.76 | 0.71 | 0.82 |
| 42 | 0.78 | 0.71 | 0.84 | 0.86 | 0.74 | 0.98 | 0.75 | 0.67 | 0.82 | 0.81 | 0.75 | 0.86 | 0.87 | 0.78 | 0.96 | 0.78 | 0.72 | 0.84 | 0.81 | 0.77 | 0.85 | 0.88 | 0.81 | 0.94 | 0.78 | 0.73 | 0.83 |
| 43 | 0.77 | 0.71 | 0.84 | 0.87 | 0.76 | 0.98 | 0.74 | 0.67 | 0.82 | 0.81 | 0.75 | 0.86 | 0.88 | 0.80 | 0.96 | 0.78 | 0.72 | 0.84 | 0.80 | 0.76 | 0.84 | 0.88 | 0.82 | 0.94 | 0.77 | 0.73 | 0.82 |
| 44 | 0.76 | 0.68 | 0.84 | 0.85 | 0.68 | 1.00 | 0.73 | 0.64 | 0.83 | 0.80 | 0.75 | 0.85 | 0.91 | 0.86 | 0.96 | 0.75 | 0.69 | 0.82 | 0.80 | 0.76 | 0.84 | 0.90 | 0.85 | 0.96 | 0.76 | 0.71 | 0.81 |
| 45 | 0.74 | 0.66 | 0.82 | 0.87 | 0.72 | 1.00 | 0.70 | 0.62 | 0.79 | 0.80 | 0.75 | 0.85 | 0.89 | 0.80 | 0.97 | 0.77 | 0.70 | 0.83 | 0.79 | 0.75 | 0.84 | 0.89 | 0.83 | 0.96 | 0.76 | 0.71 | 0.81 |
| 46 | 0.76 | 0.68 | 0.84 | 0.89 | 0.74 | 1.00 | 0.73 | 0.64 | 0.81 | 0.80 | 0.74 | 0.85 | 0.89 | 0.82 | 0.96 | 0.76 | 0.70 | 0.83 | 0.80 | 0.75 | 0.84 | 0.90 | 0.84 | 0.96 | 0.76 | 0.71 | 0.81 |
| 47 | 0.75 | 0.67 | 0.83 | 0.87 | 0.70 | 1.00 | 0.72 | 0.62 | 0.81 | 0.80 | 0.75 | 0.86 | 0.91 | 0.85 | 0.97 | 0.76 | 0.69 | 0.84 | 0.80 | 0.75 | 0.84 | 0.91 | 0.85 | 0.97 | 0.77 | 0.72 | 0.82 |
| 48 | 0.77 | 0.70 | 0.84 | 0.85 | 0.73 | 0.96 | 0.75 | 0.66 | 0.83 | 0.81 | 0.75 | 0.86 | 0.88 | 0.79 | 0.96 | 0.78 | 0.72 | 0.84 | 0.80 | 0.76 | 0.84 | 0.87 | 0.81 | 0.94 | 0.78 | 0.73 | 0.82 |
| 49 | 0.75 | 0.67 | 0.83 | 0.83 | 0.70 | 0.96 | 0.73 | 0.63 | 0.82 | 0.81 | 0.75 | 0.87 | 0.90 | 0.82 | 0.98 | 0.78 | 0.71 | 0.82 | 0.80 | 0.75 | 0.84 | 0.88 | 0.81 | 0.94 | 0.77 | 0.71 | 0.82 |
| 50 | 0.75 | 0.66 | 0.83 | 0.89 | 0.72 | 1.00 | 0.70 | 0.60 | 0.81 | 0.80 | 0.75 | 0.85 | 0.88 | 0.81 | 0.95 | 0.78 | 0.71 | 0.83 | 0.80 | 0.76 | 0.84 | 0.90 | 0.84 | 0.96 | 0.76 | 0.71 | 0.82 |
| 51 | 0.77 | 0.70 | 0.84 | 0.84 | 0.72 | 0.97 | 0.75 | 0.67 | 0.83 | 0.81 | 0.75 | 0.86 | 0.89 | 0.80 | 0.97 | 0.77 | 0.71 | 0.84 | 0.80 | 0.75 | 0.84 | 0.87 | 0.80 | 0.94 | 0.77 | 0.72 | 0.82 |
| 52 | 0.76 | 0.68 | 0.84 | 0.87 | 0.72 | 1.00 | 0.73 | 0.63 | 0.82 | 0.80 | 0.74 | 0.85 | 0.91 | 0.85 | 0.97 | 0.76 | 0.69 | 0.83 | 0.80 | 0.75 | 0.85 | 0.91 | 0.85 | 0.96 | 0.76 | 0.71 | 0.82 |
| 53 | 0.76 | 0.67 | 0.84 | 0.89 | 0.72 | 1.00 | 0.71 | 0.62 | 0.81 | 0.80 | 0.75 | 0.86 | 0.88 | 0.80 | 0.96 | 0.78 | 0.71 | 0.84 | 0.80 | 0.76 | 0.85 | 0.91 | 0.84 | 0.97 | 0.77 | 0.72 | 0.82 |
| 54 | 0.79 | 0.72 | 0.86 | 0.88 | 0.74 | 1.00 | 0.76 | 0.68 | 0.85 | 0.81 | 0.75 | 0.86 | 0.89 | 0.82 | 0.96 | 0.77 | 0.71 | 0.84 | 0.80 | 0.76 | 0.84 | 0.89 | 0.83 | 0.95 | 0.79 | 0.74 | 0.82 |
| 55 | 0.76 | 0.67 | 0.85 | 0.87 | 0.72 | 1.00 | 0.73 | 0.63 | 0.83 | 0.80 | 0.74 | 0.85 | 0.90 | 0.84 | 0.96 | 0.76 | 0.69 | 0.82 | 0.80 | 0.76 | 0.85 | 0.90 | 0.85 | 0.96 | 0.76 | 0.71 | 0.81 |
| 56 | 0.76 | 0.68 | 0.84 | 0.85 | 0.69 | 1.00 | 0.74 | 0.64 | 0.83 | 0.80 | 0.74 | 0.85 | 0.90 | 0.85 | 0.95 | 0.75 | 0.68 | 0.82 | 0.80 | 0.75 | 0.84 | 0.90 | 0.84 | 0.95 | 0.76 | 0.71 | 0.82 |
| 57 | 0.76 | 0.68 | 0.84 | 0.87 | 0.71 | 1.00 | 0.73 | 0.63 | 0.82 | 0.80 | 0.74 | 0.85 | 0.89 | 0.84 | 0.95 | 0.76 | 0.69 | 0.82 | 0.80 | 0.76 | 0.85 | 0.90 | 0.85 | 0.96 | 0.76 | 0.71 | 0.82 |
| 58 | 0.75 | 0.67 | 0.83 | 0.87 | 0.71 | 1.00 | 0.71 | 0.63 | 0.80 | 0.80 | 0.74 | 0.85 | 0.89 | 0.82 | 0.97 | 0.76 | 0.69 | 0.83 | 0.79 | 0.74 | 0.83 | 0.89 | 0.83 | 0.96 | 0.75 | 0.70 | 0.80 |
| 59 | 0.74 | 0.66 | 0.82 | 0.81 | 0.63 | 0.98 | 0.72 | 0.63 | 0.80 | 0.80 | 0.75 | 0.85 | 0.88 | 0.81 | 0.94 | 0.77 | 0.70 | 0.83 | 0.79 | 0.75 | 0.83 | 0.87 | 0.80 | 0.93 | 0.76 | 0.71 | 0.81 |
| 60 | 0.75 | 0.67 | 0.82 | 0.87 | 0.72 | 1.00 | 0.71 | 0.62 | 0.80 | 0.80 | 0.74 | 0.85 | 0.88 | 0.80 | 0.96 | 0.76 | 0.70 | 0.83 | 0.79 | 0.75 | 0.84 | 0.89 | 0.83 | 0.96 | 0.76 | 0.72 | 0.81 |
| 61 | 0.76 | 0.68 | 0.84 | 0.86 | 0.69 | 1.00 | 0.73 | 0.64 | 0.82 | 0.81 | 0.76 | 0.85 | 0.90 | 0.86 | 0.95 | 0.75 | 0.69 | 0.82 | 0.80 | 0.76 | 0.85 | 0.90 | 0.85 | 0.93 | 0.76 | 0.71 | 0.81 |
| 62 | 0.76 | 0.68 | 0.84 | 0.79 | 0.61 | 0.98 | 0.75 | 0.66 | 0.83 | 0.80 | 0.76 | 0.87 | 0.86 | 0.78 | 0.94 | 0.79 | 0.74 | 0.85 | 0.81 | 0.76 | 0.83 | 0.86 | 0.78 | 0.93 | 0.79 | 0.74 | 0.84 |
| 63 | 0.74 | 0.67 | 0.82 | 0.81 | 0.68 | 0.94 | 0.72 | 0.64 | 0.81 | 0.80 | 0.75 | 0.86 | 0.88 | 0.81 | 0.96 | 0.77 | 0.71 | 0.84 | 0.79 | 0.74 | 0.83 | 0.86 | 0.80 | 0.93 | 0.76 | 0.71 | 0.81 |
| 64 | 0.72 | 0.63 | 0.81 | 0.89 | 0.76 | 1.00 | 0.67 | 0.57 | 0.77 | 0.80 | 0.74 | 0.85 | 0.92 | 0.88 | 0.96 | 0.75 | 0.67 | 0.82 | 0.78 | 0.74 | 0.83 | 0.92 | 0.87 | 0.96 | 0.73 | 0.67 | 0.79 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 65 | 0.76 | 0.68 | 0.84 | 0.88 | 0.72 | 1.00 | 0.72 | 0.63 | 0.82 | 0.80 | 0.74 | 0.85 | 0.90 | 0.84 | 0.95 | 0.76 | 0.69 | 0.82 | 0.80 | 0.76 | 0.85 | 0.91 | 0.86 | 0.96 | 0.76 | 0.71 | 0.82 |
| 66 | 0.76 | 0.68 | 0.84 | 0.88 | 0.72 | 1.00 | 0.72 | 0.63 | 0.81 | 0.80 | 0.74 | 0.86 | 0.91 | 0.85 | 0.97 | 0.76 | 0.69 | 0.83 | 0.80 | 0.76 | 0.84 | 0.91 | 0.85 | 0.97 | 0.76 | 0.71 | 0.81 |
| 67 | 0.76 | 0.68 | 0.84 | 0.89 | 0.74 | 1.00 | 0.72 | 0.63 | 0.81 | 0.80 | 0.74 | 0.85 | 0.90 | 0.85 | 0.95 | 0.75 | 0.68 | 0.82 | 0.80 | 0.76 | 0.84 | 0.91 | 0.86 | 0.97 | 0.76 | 0.71 | 0.81 |
| 68 | 0.75 | 0.67 | 0.84 | 0.86 | 0.72 | 1.00 | 0.72 | 0.62 | 0.82 | 0.80 | 0.74 | 0.85 | 0.90 | 0.84 | 0.96 | 0.75 | 0.69 | 0.82 | 0.80 | 0.75 | 0.84 | 0.90 | 0.85 | 0.95 | 0.76 | 0.70 | 0.81 |
| 69 | 0.75 | 0.67 | 0.83 | 0.81 | 0.65 | 0.96 | 0.73 | 0.64 | 0.82 | 0.81 | 0.75 | 0.86 | 0.90 | 0.83 | 0.97 | 0.77 | 0.71 | 0.84 | 0.80 | 0.75 | 0.84 | 0.87 | 0.80 | 0.94 | 0.77 | 0.72 | 0.82 |
| 70 | 0.75 | 0.66 | 0.83 | 0.88 | 0.72 | 1.00 | 0.71 | 0.61 | 0.80 | 0.79 | 0.74 | 0.85 | 0.90 | 0.83 | 0.97 | 0.75 | 0.69 | 0.82 | 0.79 | 0.74 | 0.84 | 0.90 | 0.84 | 0.96 | 0.75 | 0.70 | 0.80 |
| 71 | 0.74 | 0.66 | 0.82 | 0.78 | 0.63 | 0.92 | 0.73 | 0.64 | 0.83 | 0.81 | 0.75 | 0.86 | 0.90 | 0.83 | 0.96 | 0.77 | 0.70 | 0.83 | 0.80 | 0.75 | 0.84 | 0.87 | 0.80 | 0.93 | 0.77 | 0.72 | 0.82 |
| 72 | 0.75 | 0.67 | 0.83 | 0.90 | 0.74 | 1.00 | 0.70 | 0.61 | 0.79 | 0.80 | 0.74 | 0.85 | 0.91 | 0.86 | 0.97 | 0.75 | 0.69 | 0.82 | 0.79 | 0.75 | 0.84 | 0.92 | 0.87 | 0.98 | 0.75 | 0.70 | 0.80 |
| 73 | 0.73 | 0.65 | 0.81 | 0.87 | 0.71 | 1.00 | 0.69 | 0.60 | 0.78 | 0.80 | 0.75 | 0.85 | 0.90 | 0.84 | 0.95 | 0.75 | 0.69 | 0.81 | 0.79 | 0.75 | 0.84 | 0.91 | 0.85 | 0.96 | 0.75 | 0.70 | 0.80 |
| 74 | 0.75 | 0.66 | 0.83 | 0.83 | 0.66 | 1.00 | 0.72 | 0.62 | 0.82 | 0.80 | 0.74 | 0.85 | 0.89 | 0.83 | 0.95 | 0.76 | 0.69 | 0.83 | 0.80 | 0.75 | 0.84 | 0.89 | 0.83 | 0.95 | 0.76 | 0.71 | 0.82 |
| 75 | 0.79 | 0.71 | 0.86 | 0.87 | 0.75 | 1.00 | 0.76 | 0.68 | 0.84 | 0.80 | 0.75 | 0.86 | 0.88 | 0.80 | 0.96 | 0.78 | 0.71 | 0.84 | 0.81 | 0.77 | 0.85 | 0.89 | 0.82 | 0.95 | 0.79 | 0.74 | 0.83 |
| 76 | 0.73 | 0.65 | 0.82 | 0.89 | 0.72 | 1.00 | 0.69 | 0.60 | 0.78 | 0.79 | 0.74 | 0.85 | 0.88 | 0.80 | 0.96 | 0.76 | 0.70 | 0.83 | 0.79 | 0.75 | 0.84 | 0.90 | 0.83 | 0.96 | 0.75 | 0.70 | 0.81 |
| 77 | 0.76 | 0.69 | 0.84 | 0.85 | 0.76 | 0.94 | 0.74 | 0.65 | 0.82 | 0.80 | 0.75 | 0.86 | 0.89 | 0.82 | 0.95 | 0.77 | 0.70 | 0.83 | 0.80 | 0.76 | 0.84 | 0.88 | 0.83 | 0.93 | 0.77 | 0.71 | 0.82 |
| 78 | 0.76 | 0.68 | 0.85 | 0.85 | 0.69 | 1.00 | 0.74 | 0.64 | 0.83 | 0.79 | 0.74 | 0.85 | 0.90 | 0.84 | 0.95 | 0.75 | 0.69 | 0.82 | 0.80 | 0.76 | 0.84 | 0.90 | 0.84 | 0.95 | 0.76 | 0.70 | 0.82 |
| 79 | 0.75 | 0.67 | 0.83 | 0.87 | 0.71 | 1.00 | 0.72 | 0.62 | 0.81 | 0.80 | 0.74 | 0.85 | 0.89 | 0.84 | 0.95 | 0.76 | 0.69 | 0.82 | 0.80 | 0.76 | 0.84 | 0.90 | 0.85 | 0.96 | 0.76 | 0.71 | 0.81 |
| 80 | 0.73 | 0.66 | 0.81 | 0.81 | 0.69 | 0.93 | 0.71 | 0.62 | 0.79 | 0.80 | 0.74 | 0.86 | 0.88 | 0.81 | 0.96 | 0.77 | 0.70 | 0.84 | 0.78 | 0.74 | 0.83 | 0.86 | 0.79 | 0.92 | 0.76 | 0.70 | 0.81 |
| 81 | 0.74 | 0.65 | 0.83 | 0.86 | 0.69 | 1.00 | 0.71 | 0.61 | 0.81 | 0.80 | 0.75 | 0.85 | 0.89 | 0.83 | 0.95 | 0.76 | 0.69 | 0.83 | 0.79 | 0.75 | 0.84 | 0.89 | 0.83 | 0.95 | 0.76 | 0.70 | 0.81 |
| 82 | 0.75 | 0.66 | 0.84 | 0.85 | 0.68 | 1.00 | 0.72 | 0.62 | 0.82 | 0.79 | 0.74 | 0.85 | 0.89 | 0.84 | 0.96 | 0.76 | 0.69 | 0.82 | 0.80 | 0.75 | 0.84 | 0.89 | 0.83 | 0.96 | 0.76 | 0.71 | 0.82 |
| 83 | 0.77 | 0.68 | 0.85 | 0.89 | 0.74 | 1.00 | 0.73 | 0.63 | 0.82 | 0.80 | 0.74 | 0.85 | 0.91 | 0.86 | 0.96 | 0.75 | 0.68 | 0.82 | 0.80 | 0.76 | 0.84 | 0.91 | 0.86 | 0.95 | 0.76 | 0.70 | 0.81 |
| 84 | 0.76 | 0.68 | 0.85 | 0.85 | 0.68 | 1.00 | 0.74 | 0.65 | 0.83 | 0.79 | 0.74 | 0.85 | 0.90 | 0.85 | 0.95 | 0.75 | 0.69 | 0.82 | 0.80 | 0.76 | 0.84 | 0.90 | 0.84 | 0.95 | 0.76 | 0.71 | 0.82 |
| 85 | 0.74 | 0.66 | 0.82 | 0.88 | 0.72 | 1.00 | 0.70 | 0.61 | 0.79 | 0.80 | 0.74 | 0.85 | 0.90 | 0.83 | 0.97 | 0.75 | 0.69 | 0.82 | 0.79 | 0.75 | 0.83 | 0.90 | 0.84 | 0.97 | 0.75 | 0.70 | 0.80 |
| 86 | 0.77 | 0.70 | 0.84 | 0.87 | 0.76 | 0.98 | 0.74 | 0.65 | 0.82 | 0.80 | 0.75 | 0.86 | 0.88 | 0.80 | 0.96 | 0.77 | 0.71 | 0.84 | 0.80 | 0.76 | 0.84 | 0.88 | 0.82 | 0.94 | 0.77 | 0.72 | 0.82 |
| 87 | 0.74 | 0.65 | 0.83 | 0.84 | 0.67 | 1.00 | 0.71 | 0.61 | 0.81 | 0.80 | 0.74 | 0.85 | 0.89 | 0.82 | 0.95 | 0.76 | 0.69 | 0.83 | 0.79 | 0.75 | 0.84 | 0.89 | 0.83 | 0.95 | 0.76 | 0.70 | 0.81 |
| 88 | 0.74 | 0.66 | 0.82 | 0.82 | 0.65 | 0.99 | 0.71 | 0.63 | 0.80 | 0.80 | 0.74 | 0.85 | 0.89 | 0.83 | 0.95 | 0.76 | 0.68 | 0.82 | 0.78 | 0.74 | 0.83 | 0.88 | 0.81 | 0.94 | 0.75 | 0.70 | 0.80 |
| 89 | 0.76 | 0.68 | 0.84 | 0.85 | 0.69 | 1.00 | 0.73 | 0.64 | 0.83 | 0.79 | 0.74 | 0.85 | 0.90 | 0.84 | 0.95 | 0.75 | 0.69 | 0.81 | 0.80 | 0.75 | 0.84 | 0.90 | 0.84 | 0.95 | 0.76 | 0.71 | 0.81 |
| 90 | 0.75 | 0.67 | 0.83 | 0.81 | 0.65 | 0.96 | 0.73 | 0.64 | 0.82 | 0.81 | 0.75 | 0.86 | 0.85 | 0.76 | 0.95 | 0.79 | 0.73 | 0.85 | 0.80 | 0.75 | 0.84 | 0.85 | 0.77 | 0.92 | 0.78 | 0.73 | 0.83 |
| 91 | 0.74 | 0.66 | 0.82 | 0.88 | 0.72 | 1.00 | 0.70 | 0.61 | 0.79 | 0.80 | 0.74 | 0.85 | 0.90 | 0.83 | 0.97 | 0.75 | 0.69 | 0.82 | 0.79 | 0.75 | 0.84 | 0.90 | 0.84 | 0.97 | 0.75 | 0.70 | 0.80 |
| 92 | 0.76 | 0.68 | 0.85 | 0.87 | 0.71 | 1.00 | 0.73 | 0.64 | 0.83 | 0.79 | 0.74 | 0.85 | 0.89 | 0.84 | 0.95 | 0.74 | 0.67 | 0.81 | 0.80 | 0.76 | 0.85 | 0.93 | 0.88 | 0.95 | 0.77 | 0.68 | 0.79 |
| 93 | 0.74 | 0.65 | 0.83 | 0.91 | 0.78 | 1.00 | 0.69 | 0.59 | 0.79 | 0.79 | 0.74 | 0.85 | 0.92 | 0.87 | 0.96 | 0.74 | 0.67 | 0.80 | 0.79 | 0.74 | 0.84 | 0.92 | 0.88 | 0.97 | 0.74 | 0.69 | 0.79 |
| 94 | 0.75 | 0.66 | 0.83 | 0.89 | 0.76 | 1.00 | 0.70 | 0.61 | 0.80 | 0.80 | 0.74 | 0.85 | 0.93 | 0.88 | 0.97 | 0.74 | 0.67 | 0.80 | 0.79 | 0.75 | 0.84 | 0.90 | 0.88 | 0.97 | 0.74 | 0.71 | 0.82 |
| 95 | 0.75 | 0.67 | 0.84 | 0.86 | 0.72 | 1.00 | 0.72 | 0.62 | 0.83 | 0.79 | 0.74 | 0.85 | 0.90 | 0.84 | 0.95 | 0.75 | 0.68 | 0.82 | 0.80 | 0.75 | 0.84 | 0.90 | 0.84 | 0.95 | 0.76 | 0.71 | 0.81 |
| 96 | 0.76 | 0.68 | 0.84 | 0.85 | 0.68 | 1.00 | 0.74 | 0.64 | 0.83 | 0.79 | 0.74 | 0.85 | 0.90 | 0.84 | 0.95 | 0.75 | 0.68 | 0.81 | 0.80 | 0.75 | 0.84 | 0.90 | 0.84 | 0.95 | 0.76 | 0.71 | 0.81 |
| 97 | 0.75 | 0.67 | 0.83 | 0.87 | 0.70 | 1.00 | 0.71 | 0.62 | 0.80 | 0.79 | 0.74 | 0.85 | 0.90 | 0.82 | 0.97 | 0.75 | 0.69 | 0.82 | 0.78 | 0.74 | 0.83 | 0.89 | 0.83 | 0.96 | 0.75 | 0.69 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 98 | 0.74 | 0.66 | 0.83 | 0.90 | 0.77 | 1.00 | 0.70 | 0.60 | 0.79 | 0.79 | 0.74 | 0.85 | 0.92 | 0.87 | 0.97 | 0.74 | 0.67 | 0.81 | 0.79 | 0.74 | 0.83 | 0.92 | 0.87 | 0.97 | 0.74 | 0.68 | 0.79 |
| 99 | 0.77 | 0.70 | 0.84 | 0.82 | 0.70 | 0.95 | 0.75 | 0.68 | 0.83 | 0.80 | 0.75 | 0.85 | 0.87 | 0.78 | 0.96 | 0.78 | 0.71 | 0.84 | 0.80 | 0.76 | 0.84 | 0.86 | 0.79 | 0.93 | 0.78 | 0.73 | 0.82 |
| 100 | 0.76 | 0.68 | 0.85 | 0.87 | 0.69 | 1.00 | 0.74 | 0.64 | 0.83 | 0.79 | 0.74 | 0.85 | 0.90 | 0.85 | 0.95 | 0.75 | 0.68 | 0.81 | 0.80 | 0.75 | 0.84 | 0.90 | 0.85 | 0.96 | 0.76 | 0.71 | 0.81 |
| 101 | 0.78 | 0.71 | 0.84 | 0.85 | 0.74 | 0.95 | 0.75 | 0.68 | 0.83 | 0.80 | 0.74 | 0.85 | 0.87 | 0.78 | 0.97 | 0.77 | 0.70 | 0.83 | 0.80 | 0.76 | 0.84 | 0.87 | 0.80 | 0.94 | 0.77 | 0.72 | 0.82 |
| 102 | 0.74 | 0.66 | 0.83 | 0.89 | 0.76 | 1.00 | 0.70 | 0.61 | 0.80 | 0.79 | 0.74 | 0.85 | 0.92 | 0.87 | 0.97 | 0.74 | 0.67 | 0.81 | 0.79 | 0.75 | 0.83 | 0.92 | 0.87 | 0.97 | 0.74 | 0.69 | 0.79 |
| 103 | 0.76 | 0.68 | 0.84 | 0.87 | 0.70 | 1.00 | 0.73 | 0.64 | 0.82 | 0.79 | 0.74 | 0.85 | 0.90 | 0.85 | 0.95 | 0.75 | 0.68 | 0.82 | 0.79 | 0.75 | 0.84 | 0.90 | 0.85 | 0.96 | 0.75 | 0.70 | 0.81 |
| 104 | 0.70 | 0.61 | 0.79 | 0.74 | 0.59 | 0.89 | 0.69 | 0.59 | 0.80 | 0.80 | 0.75 | 0.86 | 0.88 | 0.80 | 0.95 | 0.77 | 0.70 | 0.84 | 0.78 | 0.73 | 0.83 | 0.84 | 0.77 | 0.91 | 0.75 | 0.70 | 0.81 |
| 105 | 0.74 | 0.67 | 0.81 | 0.80 | 0.67 | 0.93 | 0.72 | 0.64 | 0.80 | 0.80 | 0.74 | 0.85 | 0.86 | 0.77 | 0.95 | 0.77 | 0.71 | 0.84 | 0.78 | 0.74 | 0.83 | 0.85 | 0.77 | 0.92 | 0.76 | 0.71 | 0.81 |
| 106 | 0.77 | 0.69 | 0.85 | 0.86 | 0.70 | 1.00 | 0.74 | 0.65 | 0.83 | 0.80 | 0.75 | 0.86 | 0.89 | 0.84 | 0.95 | 0.77 | 0.70 | 0.83 | 0.81 | 0.77 | 0.85 | 0.90 | 0.85 | 0.95 | 0.77 | 0.72 | 0.83 |
| 107 | 0.74 | 0.66 | 0.82 | 0.80 | 0.67 | 0.94 | 0.72 | 0.62 | 0.82 | 0.79 | 0.74 | 0.85 | 0.86 | 0.78 | 0.94 | 0.76 | 0.70 | 0.83 | 0.78 | 0.74 | 0.83 | 0.85 | 0.78 | 0.92 | 0.76 | 0.70 | 0.81 |
| 108 | 0.75 | 0.67 | 0.83 | 0.81 | 0.66 | 0.96 | 0.73 | 0.64 | 0.82 | 0.80 | 0.74 | 0.85 | 0.88 | 0.80 | 0.96 | 0.76 | 0.70 | 0.83 | 0.79 | 0.75 | 0.84 | 0.87 | 0.80 | 0.94 | 0.77 | 0.72 | 0.82 |
| 109 | 0.77 | 0.69 | 0.85 | 0.86 | 0.73 | 0.98 | 0.74 | 0.65 | 0.83 | 0.80 | 0.74 | 0.86 | 0.88 | 0.80 | 0.97 | 0.77 | 0.70 | 0.84 | 0.80 | 0.76 | 0.85 | 0.88 | 0.82 | 0.95 | 0.77 | 0.72 | 0.82 |
| 110 | 0.76 | 0.68 | 0.85 | 0.88 | 0.71 | 1.00 | 0.73 | 0.63 | 0.82 | 0.80 | 0.75 | 0.85 | 0.89 | 0.83 | 0.95 | 0.76 | 0.70 | 0.83 | 0.80 | 0.76 | 0.85 | 0.90 | 0.85 | 0.96 | 0.77 | 0.72 | 0.82 |
| 111 | 0.78 | 0.71 | 0.84 | 0.87 | 0.76 | 0.98 | 0.74 | 0.67 | 0.82 | 0.80 | 0.74 | 0.85 | 0.87 | 0.78 | 0.96 | 0.77 | 0.71 | 0.84 | 0.80 | 0.76 | 0.84 | 0.87 | 0.81 | 0.94 | 0.77 | 0.73 | 0.82 |
| 112 | 0.76 | 0.67 | 0.84 | 0.86 | 0.70 | 1.00 | 0.73 | 0.63 | 0.82 | 0.79 | 0.74 | 0.85 | 0.91 | 0.85 | 0.96 | 0.75 | 0.68 | 0.82 | 0.80 | 0.75 | 0.84 | 0.90 | 0.85 | 0.96 | 0.76 | 0.71 | 0.82 |
| 113 | 0.74 | 0.66 | 0.82 | 0.86 | 0.76 | 0.96 | 0.70 | 0.61 | 0.79 | 0.78 | 0.72 | 0.84 | 0.84 | 0.74 | 0.95 | 0.76 | 0.69 | 0.83 | 0.77 | 0.73 | 0.82 | 0.85 | 0.78 | 0.92 | 0.74 | 0.69 | 0.80 |
| 114 | 0.75 | 0.67 | 0.83 | 0.82 | 0.69 | 0.94 | 0.72 | 0.63 | 0.82 | 0.79 | 0.74 | 0.85 | 0.87 | 0.79 | 0.95 | 0.76 | 0.70 | 0.83 | 0.79 | 0.74 | 0.83 | 0.86 | 0.79 | 0.93 | 0.76 | 0.71 | 0.82 |
| 115 | 0.74 | 0.65 | 0.82 | 0.77 | 0.61 | 0.92 | 0.73 | 0.63 | 0.83 | 0.80 | 0.75 | 0.86 | 0.88 | 0.80 | 0.96 | 0.77 | 0.70 | 0.84 | 0.79 | 0.74 | 0.83 | 0.85 | 0.77 | 0.92 | 0.77 | 0.71 | 0.82 |
| 116 | 0.78 | 0.71 | 0.86 | 0.88 | 0.74 | 1.00 | 0.75 | 0.67 | 0.84 | 0.80 | 0.75 | 0.85 | 0.88 | 0.81 | 0.95 | 0.77 | 0.70 | 0.83 | 0.80 | 0.75 | 0.84 | 0.88 | 0.82 | 0.94 | 0.76 | 0.72 | 0.81 |
| 117 | 0.76 | 0.68 | 0.84 | 0.79 | 0.64 | 0.94 | 0.75 | 0.66 | 0.84 | 0.80 | 0.75 | 0.86 | 0.85 | 0.77 | 0.94 | 0.78 | 0.72 | 0.84 | 0.80 | 0.76 | 0.84 | 0.85 | 0.77 | 0.92 | 0.78 | 0.73 | 0.83 |
| 118 | 0.74 | 0.67 | 0.81 | 0.81 | 0.68 | 0.93 | 0.72 | 0.64 | 0.80 | 0.80 | 0.74 | 0.85 | 0.87 | 0.78 | 0.95 | 0.77 | 0.70 | 0.83 | 0.78 | 0.74 | 0.83 | 0.85 | 0.78 | 0.92 | 0.76 | 0.71 | 0.81 |
| 119 | 0.76 | 0.68 | 0.84 | 0.87 | 0.71 | 1.00 | 0.73 | 0.64 | 0.82 | 0.79 | 0.74 | 0.85 | 0.90 | 0.86 | 0.95 | 0.75 | 0.68 | 0.81 | 0.79 | 0.75 | 0.84 | 0.90 | 0.85 | 0.96 | 0.75 | 0.70 | 0.80 |
| 120 | 0.74 | 0.66 | 0.82 | 0.88 | 0.71 | 1.00 | 0.71 | 0.62 | 0.79 | 0.79 | 0.74 | 0.85 | 0.89 | 0.82 | 0.96 | 0.75 | 0.69 | 0.82 | 0.78 | 0.74 | 0.83 | 0.89 | 0.83 | 0.96 | 0.74 | 0.69 | 0.79 |
| 121 | 0.78 | 0.71 | 0.85 | 0.90 | 0.80 | 0.99 | 0.74 | 0.65 | 0.82 | 0.80 | 0.74 | 0.85 | 0.88 | 0.80 | 0.96 | 0.77 | 0.71 | 0.83 | 0.80 | 0.76 | 0.84 | 0.89 | 0.84 | 0.95 | 0.77 | 0.72 | 0.82 |
| 122 | 0.77 | 0.70 | 0.84 | 0.85 | 0.74 | 0.96 | 0.74 | 0.66 | 0.82 | 0.80 | 0.74 | 0.85 | 0.88 | 0.79 | 0.97 | 0.77 | 0.70 | 0.83 | 0.79 | 0.75 | 0.84 | 0.87 | 0.80 | 0.94 | 0.77 | 0.72 | 0.82 |
| 123 | 0.76 | 0.68 | 0.85 | 0.90 | 0.76 | 1.00 | 0.72 | 0.63 | 0.82 | 0.79 | 0.74 | 0.85 | 0.90 | 0.85 | 0.96 | 0.75 | 0.68 | 0.83 | 0.80 | 0.75 | 0.85 | 0.92 | 0.86 | 0.97 | 0.76 | 0.71 | 0.81 |
| 124 | 0.74 | 0.66 | 0.83 | 0.89 | 0.75 | 1.00 | 0.70 | 0.60 | 0.80 | 0.79 | 0.73 | 0.85 | 0.89 | 0.83 | 0.95 | 0.75 | 0.68 | 0.82 | 0.79 | 0.74 | 0.84 | 0.90 | 0.85 | 0.96 | 0.75 | 0.69 | 0.80 |
| 125 | 0.74 | 0.66 | 0.83 | 0.78 | 0.57 | 0.98 | 0.73 | 0.64 | 0.83 | 0.81 | 0.75 | 0.86 | 0.85 | 0.76 | 0.94 | 0.79 | 0.73 | 0.86 | 0.80 | 0.75 | 0.85 | 0.84 | 0.76 | 0.93 | 0.78 | 0.73 | 0.84 |
| 126 | 0.74 | 0.66 | 0.82 | 0.89 | 0.76 | 1.00 | 0.70 | 0.60 | 0.79 | 0.78 | 0.72 | 0.84 | 0.85 | 0.75 | 0.96 | 0.75 | 0.68 | 0.82 | 0.77 | 0.73 | 0.82 | 0.87 | 0.79 | 0.94 | 0.74 | 0.69 | 0.79 |
| 127 | 0.74 | 0.66 | 0.82 | 0.90 | 0.76 | 1.00 | 0.69 | 0.60 | 0.78 | 0.79 | 0.74 | 0.85 | 0.91 | 0.85 | 0.98 | 0.75 | 0.68 | 0.82 | 0.79 | 0.74 | 0.83 | 0.92 | 0.86 | 0.97 | 0.74 | 0.69 | 0.79 |
| 128 | 0.75 | 0.67 | 0.84 | 0.85 | 0.70 | 1.00 | 0.72 | 0.63 | 0.82 | 0.79 | 0.74 | 0.85 | 0.90 | 0.84 | 0.95 | 0.75 | 0.68 | 0.81 | 0.80 | 0.75 | 0.84 | 0.90 | 0.84 | 0.95 | 0.76 | 0.70 | 0.81 |
| 129 | 0.74 | 0.66 | 0.83 | 0.78 | 0.64 | 0.92 | 0.73 | 0.64 | 0.83 | 0.80 | 0.75 | 0.86 | 0.88 | 0.82 | 0.95 | 0.77 | 0.70 | 0.83 | 0.79 | 0.74 | 0.83 | 0.86 | 0.79 | 0.92 | 0.76 | 0.71 | 0.82 |
| 130 | 0.73 | 0.66 | 0.81 | 0.78 | 0.64 | 0.92 | 0.72 | 0.63 | 0.80 | 0.80 | 0.74 | 0.85 | 0.87 | 0.78 | 0.96 | 0.77 | 0.70 | 0.83 | 0.78 | 0.74 | 0.83 | 0.84 | 0.77 | 0.92 | 0.76 | 0.71 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 131 | 0.75 | 0.67 | 0.84 | 0.84 | 0.69 | 0.99 | 0.73 | 0.63 | 0.83 | 0.79 | 0.74 | 0.85 | 0.90 | 0.84 | 0.95 | 0.75 | 0.68 | 0.81 | 0.80 | 0.75 | 0.84 | 0.89 | 0.84 | 0.95 | 0.76 | 0.71 | 0.81 |
| 132 | 0.74 | 0.66 | 0.81 | 0.84 | 0.71 | 0.98 | 0.70 | 0.61 | 0.79 | 0.80 | 0.74 | 0.85 | 0.87 | 0.80 | 0.95 | 0.77 | 0.70 | 0.83 | 0.78 | 0.74 | 0.83 | 0.87 | 0.81 | 0.94 | 0.75 | 0.70 | 0.80 |
| 133 | 0.73 | 0.63 | 0.82 | 0.89 | 0.73 | 1.00 | 0.68 | 0.57 | 0.78 | 0.79 | 0.74 | 0.85 | 0.90 | 0.85 | 0.95 | 0.75 | 0.68 | 0.81 | 0.79 | 0.74 | 0.83 | 0.91 | 0.86 | 0.96 | 0.74 | 0.68 | 0.79 |
| 134 | 0.75 | 0.66 | 0.83 | 0.85 | 0.68 | 1.00 | 0.72 | 0.62 | 0.81 | 0.79 | 0.74 | 0.84 | 0.90 | 0.85 | 0.95 | 0.74 | 0.68 | 0.81 | 0.79 | 0.75 | 0.84 | 0.90 | 0.84 | 0.95 | 0.75 | 0.70 | 0.81 |
| 135 | 0.76 | 0.69 | 0.82 | 0.82 | 0.68 | 0.96 | 0.73 | 0.66 | 0.81 | 0.80 | 0.74 | 0.85 | 0.87 | 0.78 | 0.96 | 0.77 | 0.71 | 0.83 | 0.79 | 0.75 | 0.83 | 0.86 | 0.79 | 0.93 | 0.77 | 0.72 | 0.82 |
| 136 | 0.76 | 0.68 | 0.84 | 0.88 | 0.72 | 1.00 | 0.73 | 0.64 | 0.82 | 0.79 | 0.74 | 0.85 | 0.89 | 0.82 | 0.96 | 0.75 | 0.68 | 0.82 | 0.79 | 0.74 | 0.83 | 0.90 | 0.83 | 0.96 | 0.75 | 0.70 | 0.80 |
| 137 | 0.76 | 0.68 | 0.83 | 0.87 | 0.71 | 1.00 | 0.72 | 0.64 | 0.80 | 0.79 | 0.73 | 0.85 | 0.89 | 0.82 | 0.96 | 0.75 | 0.68 | 0.82 | 0.79 | 0.75 | 0.83 | 0.89 | 0.83 | 0.96 | 0.75 | 0.70 | 0.80 |
| 138 | 0.74 | 0.66 | 0.82 | 0.90 | 0.76 | 1.00 | 0.70 | 0.61 | 0.78 | 0.79 | 0.73 | 0.85 | 0.89 | 0.82 | 0.96 | 0.75 | 0.68 | 0.82 | 0.78 | 0.74 | 0.83 | 0.90 | 0.85 | 0.96 | 0.74 | 0.69 | 0.79 |
| 139 | 0.74 | 0.65 | 0.82 | 0.88 | 0.71 | 1.00 | 0.70 | 0.60 | 0.79 | 0.79 | 0.73 | 0.84 | 0.87 | 0.81 | 0.94 | 0.75 | 0.68 | 0.81 | 0.79 | 0.74 | 0.83 | 0.89 | 0.83 | 0.95 | 0.75 | 0.69 | 0.80 |
| 140 | 0.78 | 0.71 | 0.84 | 0.87 | 0.78 | 0.96 | 0.75 | 0.68 | 0.83 | 0.79 | 0.74 | 0.85 | 0.88 | 0.78 | 0.97 | 0.76 | 0.70 | 0.83 | 0.80 | 0.76 | 0.84 | 0.88 | 0.81 | 0.94 | 0.77 | 0.72 | 0.82 |
| 141 | 0.76 | 0.68 | 0.84 | 0.86 | 0.70 | 1.00 | 0.73 | 0.64 | 0.82 | 0.79 | 0.74 | 0.85 | 0.90 | 0.84 | 0.95 | 0.75 | 0.68 | 0.81 | 0.80 | 0.75 | 0.84 | 0.90 | 0.85 | 0.95 | 0.76 | 0.70 | 0.81 |
| 142 | 0.75 | 0.67 | 0.83 | 0.91 | 0.78 | 1.00 | 0.71 | 0.61 | 0.80 | 0.79 | 0.73 | 0.85 | 0.93 | 0.88 | 0.97 | 0.73 | 0.66 | 0.80 | 0.79 | 0.75 | 0.83 | 0.93 | 0.89 | 0.97 | 0.74 | 0.68 | 0.79 |
| 143 | 0.75 | 0.67 | 0.83 | 0.82 | 0.69 | 0.95 | 0.73 | 0.63 | 0.83 | 0.80 | 0.74 | 0.85 | 0.89 | 0.83 | 0.96 | 0.76 | 0.69 | 0.83 | 0.80 | 0.75 | 0.84 | 0.87 | 0.81 | 0.94 | 0.77 | 0.71 | 0.82 |
| 144 | 0.74 | 0.66 | 0.83 | 0.87 | 0.70 | 1.00 | 0.71 | 0.62 | 0.80 | 0.79 | 0.74 | 0.85 | 0.91 | 0.86 | 0.96 | 0.74 | 0.67 | 0.80 | 0.79 | 0.75 | 0.83 | 0.91 | 0.86 | 0.96 | 0.74 | 0.69 | 0.80 |
| 145 | 0.75 | 0.67 | 0.83 | 0.83 | 0.68 | 0.97 | 0.72 | 0.63 | 0.82 | 0.78 | 0.73 | 0.84 | 0.85 | 0.76 | 0.94 | 0.76 | 0.69 | 0.83 | 0.79 | 0.74 | 0.83 | 0.85 | 0.78 | 0.93 | 0.76 | 0.71 | 0.82 |
| 146 | 0.76 | 0.69 | 0.83 | 0.81 | 0.67 | 0.95 | 0.74 | 0.66 | 0.82 | 0.80 | 0.74 | 0.85 | 0.87 | 0.78 | 0.95 | 0.77 | 0.70 | 0.83 | 0.79 | 0.74 | 0.83 | 0.85 | 0.78 | 0.92 | 0.76 | 0.71 | 0.81 |
| 147 | 0.74 | 0.65 | 0.82 | 0.89 | 0.71 | 1.00 | 0.69 | 0.60 | 0.79 | 0.79 | 0.74 | 0.85 | 0.88 | 0.80 | 0.96 | 0.76 | 0.69 | 0.82 | 0.79 | 0.75 | 0.84 | 0.90 | 0.83 | 0.96 | 0.75 | 0.70 | 0.80 |
| 148 | 0.73 | 0.65 | 0.82 | 0.89 | 0.76 | 1.00 | 0.69 | 0.59 | 0.79 | 0.79 | 0.73 | 0.85 | 0.92 | 0.87 | 0.97 | 0.73 | 0.66 | 0.80 | 0.78 | 0.74 | 0.83 | 0.92 | 0.87 | 0.97 | 0.73 | 0.68 | 0.79 |
| 149 | 0.72 | 0.64 | 0.81 | 0.85 | 0.69 | 1.00 | 0.69 | 0.59 | 0.79 | 0.79 | 0.74 | 0.85 | 0.90 | 0.84 | 0.95 | 0.75 | 0.68 | 0.81 | 0.78 | 0.74 | 0.83 | 0.89 | 0.84 | 0.95 | 0.74 | 0.69 | 0.80 |
| 150 | 0.74 | 0.66 | 0.83 | 0.75 | 0.56 | 0.94 | 0.74 | 0.64 | 0.83 | 0.80 | 0.75 | 0.86 | 0.87 | 0.80 | 0.94 | 0.77 | 0.71 | 0.84 | 0.79 | 0.75 | 0.84 | 0.85 | 0.77 | 0.92 | 0.77 | 0.72 | 0.82 |
| 151 | 0.78 | 0.69 | 0.86 | 0.88 | 0.71 | 1.00 | 0.75 | 0.65 | 0.84 | 0.79 | 0.73 | 0.85 | 0.91 | 0.86 | 0.95 | 0.74 | 0.67 | 0.81 | 0.80 | 0.75 | 0.84 | 0.91 | 0.86 | 0.96 | 0.76 | 0.70 | 0.81 |
| 152 | 0.77 | 0.69 | 0.85 | 0.86 | 0.69 | 1.00 | 0.74 | 0.65 | 0.84 | 0.79 | 0.73 | 0.84 | 0.89 | 0.84 | 0.94 | 0.75 | 0.68 | 0.81 | 0.80 | 0.76 | 0.84 | 0.90 | 0.84 | 0.95 | 0.76 | 0.71 | 0.82 |
| 153 | 0.75 | 0.68 | 0.83 | 0.83 | 0.71 | 0.95 | 0.73 | 0.65 | 0.81 | 0.79 | 0.74 | 0.85 | 0.87 | 0.79 | 0.95 | 0.77 | 0.70 | 0.83 | 0.79 | 0.75 | 0.83 | 0.86 | 0.80 | 0.92 | 0.76 | 0.71 | 0.81 |
| 154 | 0.75 | 0.67 | 0.84 | 0.88 | 0.74 | 1.00 | 0.71 | 0.62 | 0.81 | 0.78 | 0.72 | 0.83 | 0.87 | 0.79 | 0.94 | 0.74 | 0.67 | 0.81 | 0.79 | 0.74 | 0.83 | 0.89 | 0.83 | 0.94 | 0.75 | 0.70 | 0.80 |
| 155 | 0.75 | 0.66 | 0.83 | 0.90 | 0.80 | 1.00 | 0.70 | 0.60 | 0.80 | 0.77 | 0.71 | 0.83 | 0.85 | 0.74 | 0.96 | 0.74 | 0.67 | 0.81 | 0.77 | 0.73 | 0.82 | 0.87 | 0.79 | 0.95 | 0.74 | 0.69 | 0.80 |
| 156 | 0.76 | 0.67 | 0.85 | 0.88 | 0.72 | 1.00 | 0.73 | 0.63 | 0.83 | 0.79 | 0.73 | 0.85 | 0.89 | 0.84 | 0.95 | 0.75 | 0.68 | 0.82 | 0.80 | 0.75 | 0.84 | 0.90 | 0.85 | 0.96 | 0.76 | 0.70 | 0.81 |
| 157 | 0.75 | 0.66 | 0.83 | 0.86 | 0.70 | 1.00 | 0.72 | 0.62 | 0.82 | 0.79 | 0.73 | 0.84 | 0.89 | 0.84 | 0.95 | 0.74 | 0.68 | 0.81 | 0.79 | 0.75 | 0.84 | 0.90 | 0.84 | 0.95 | 0.75 | 0.70 | 0.81 |
| 158 | 0.74 | 0.66 | 0.82 | 0.80 | 0.66 | 0.94 | 0.72 | 0.63 | 0.81 | 0.80 | 0.74 | 0.85 | 0.89 | 0.83 | 0.95 | 0.76 | 0.69 | 0.83 | 0.79 | 0.74 | 0.83 | 0.87 | 0.81 | 0.93 | 0.76 | 0.70 | 0.80 |
| 159 | 0.74 | 0.66 | 0.82 | 0.87 | 0.71 | 1.00 | 0.70 | 0.61 | 0.79 | 0.79 | 0.73 | 0.85 | 0.90 | 0.83 | 0.97 | 0.75 | 0.68 | 0.82 | 0.79 | 0.75 | 0.83 | 0.91 | 0.85 | 0.97 | 0.75 | 0.70 | 0.80 |
| 160 | 0.73 | 0.65 | 0.81 | 0.78 | 0.62 | 0.94 | 0.72 | 0.63 | 0.81 | 0.80 | 0.74 | 0.85 | 0.89 | 0.82 | 0.95 | 0.76 | 0.68 | 0.83 | 0.78 | 0.73 | 0.82 | 0.86 | 0.79 | 0.92 | 0.76 | 0.70 | 0.81 |
| 161 | 0.78 | 0.70 | 0.87 | 0.91 | 0.78 | 1.00 | 0.75 | 0.65 | 0.84 | 0.79 | 0.73 | 0.85 | 0.91 | 0.87 | 0.96 | 0.74 | 0.67 | 0.81 | 0.80 | 0.76 | 0.85 | 0.93 | 0.88 | 0.97 | 0.75 | 0.70 | 0.80 |
| 162 | 0.75 | 0.67 | 0.83 | 0.87 | 0.73 | 1.00 | 0.71 | 0.62 | 0.81 | 0.79 | 0.73 | 0.84 | 0.92 | 0.87 | 0.96 | 0.73 | 0.67 | 0.80 | 0.79 | 0.75 | 0.84 | 0.91 | 0.86 | 0.96 | 0.75 | 0.70 | 0.80 |
| 163 | 0.78 | 0.71 | 0.85 | 0.88 | 0.77 | 0.99 | 0.74 | 0.66 | 0.83 | 0.78 | 0.73 | 0.84 | 0.85 | 0.76 | 0.93 | 0.76 | 0.69 | 0.83 | 0.79 | 0.75 | 0.83 | 0.86 | 0.80 | 0.93 | 0.76 | 0.71 | 0.81 |

| No. | G | H | I | J | K | L | M | N | O | P | Q | R | S | T | U | V | W | Z | AA | AB | AC | AD | AE | AF | AG | AH | AI |
|-----|---|---|---|---|---|---|---|---|---|---|---|---|---|---|---|---|---|---|----|----|----|----|----|----|----|----|----|
| 164 | 0.77 | 0.68 | 0.85 | 0.89 | 0.74 | 1.00 | 0.73 | 0.63 | 0.83 | 0.79 | 0.73 | 0.85 | 0.91 | 0.86 | 0.96 | 0.75 | 0.68 | 0.82 | 0.80 | 0.76 | 0.85 | 0.92 | 0.86 | 0.97 | 0.76 | 0.71 | 0.82 |
| 165 | 0.74 | 0.66 | 0.82 | 0.89 | 0.77 | 1.00 | 0.69 | 0.60 | 0.79 | 0.77 | 0.71 | 0.83 | 0.84 | 0.73 | 0.95 | 0.74 | 0.67 | 0.81 | 0.77 | 0.72 | 0.82 | 0.86 | 0.78 | 0.94 | 0.74 | 0.68 | 0.79 |
| 166 | 0.72 | 0.64 | 0.81 | 0.85 | 0.69 | 1.00 | 0.69 | 0.59 | 0.79 | 0.79 | 0.74 | 0.84 | 0.90 | 0.84 | 0.95 | 0.74 | 0.68 | 0.81 | 0.78 | 0.74 | 0.83 | 0.90 | 0.84 | 0.95 | 0.74 | 0.69 | 0.80 |
| 167 | 0.74 | 0.66 | 0.82 | 0.87 | 0.73 | 1.00 | 0.70 | 0.61 | 0.79 | 0.79 | 0.73 | 0.84 | 0.88 | 0.80 | 0.96 | 0.75 | 0.68 | 0.82 | 0.78 | 0.74 | 0.83 | 0.89 | 0.82 | 0.95 | 0.75 | 0.69 | 0.80 |
| 168 | 0.74 | 0.66 | 0.82 | 0.83 | 0.68 | 0.99 | 0.71 | 0.62 | 0.80 | 0.77 | 0.71 | 0.83 | 0.81 | 0.71 | 0.91 | 0.75 | 0.69 | 0.82 | 0.77 | 0.73 | 0.82 | 0.83 | 0.75 | 0.91 | 0.75 | 0.70 | 0.80 |
| 169 | 0.75 | 0.67 | 0.84 | 0.85 | 0.70 | 1.00 | 0.72 | 0.62 | 0.82 | 0.79 | 0.73 | 0.84 | 0.89 | 0.83 | 0.95 | 0.74 | 0.68 | 0.81 | 0.80 | 0.75 | 0.84 | 0.89 | 0.84 | 0.95 | 0.76 | 0.70 | 0.81 |
| 170 | 0.76 | 0.68 | 0.84 | 0.85 | 0.70 | 1.00 | 0.73 | 0.65 | 0.82 | 0.79 | 0.74 | 0.85 | 0.89 | 0.84 | 0.95 | 0.75 | 0.68 | 0.82 | 0.80 | 0.75 | 0.84 | 0.90 | 0.84 | 0.95 | 0.76 | 0.71 | 0.81 |
| 171 | 0.76 | 0.69 | 0.83 | 0.86 | 0.67 | 0.97 | 0.72 | 0.65 | 0.80 | 0.79 | 0.74 | 0.85 | 0.89 | 0.82 | 0.96 | 0.76 | 0.69 | 0.82 | 0.79 | 0.75 | 0.83 | 0.89 | 0.83 | 0.94 | 0.76 | 0.71 | 0.81 |
| 172 | 0.74 | 0.66 | 0.82 | 0.85 | 0.75 | 1.00 | 0.71 | 0.62 | 0.80 | 0.79 | 0.74 | 0.85 | 0.89 | 0.84 | 0.95 | 0.75 | 0.68 | 0.82 | 0.79 | 0.75 | 0.84 | 0.89 | 0.84 | 0.95 | 0.75 | 0.70 | 0.81 |
| 173 | 0.73 | 0.65 | 0.82 | 0.90 | 0.68 | 1.00 | 0.69 | 0.59 | 0.78 | 0.79 | 0.73 | 0.85 | 0.94 | 0.91 | 0.97 | 0.72 | 0.65 | 0.80 | 0.78 | 0.73 | 0.83 | 0.93 | 0.89 | 0.98 | 0.72 | 0.66 | 0.78 |
| 174 | 0.74 | 0.66 | 0.83 | 0.88 | 0.75 | 1.00 | 0.70 | 0.61 | 0.80 | 0.79 | 0.73 | 0.85 | 0.92 | 0.86 | 0.97 | 0.74 | 0.67 | 0.81 | 0.79 | 0.75 | 0.84 | 0.92 | 0.86 | 0.97 | 0.75 | 0.69 | 0.80 |
| 175 | 0.76 | 0.68 | 0.83 | 0.88 | 0.72 | 1.00 | 0.72 | 0.63 | 0.81 | 0.79 | 0.73 | 0.84 | 0.91 | 0.86 | 0.96 | 0.74 | 0.67 | 0.80 | 0.80 | 0.75 | 0.84 | 0.91 | 0.86 | 0.96 | 0.75 | 0.70 | 0.80 |
| 176 | 0.75 | 0.67 | 0.84 | 0.90 | 0.75 | 1.00 | 0.71 | 0.61 | 0.81 | 0.79 | 0.73 | 0.84 | 0.90 | 0.85 | 0.96 | 0.74 | 0.67 | 0.81 | 0.80 | 0.75 | 0.84 | 0.92 | 0.87 | 0.97 | 0.75 | 0.70 | 0.80 |
| 177 | 0.74 | 0.66 | 0.81 | 0.85 | 0.74 | 0.96 | 0.70 | 0.62 | 0.79 | 0.79 | 0.74 | 0.85 | 0.87 | 0.79 | 0.95 | 0.76 | 0.70 | 0.83 | 0.78 | 0.74 | 0.82 | 0.87 | 0.81 | 0.93 | 0.75 | 0.69 | 0.80 |
| 178 | 0.74 | 0.66 | 0.82 | 0.85 | 0.74 | 0.98 | 0.71 | 0.62 | 0.79 | 0.78 | 0.73 | 0.84 | 0.87 | 0.80 | 0.94 | 0.75 | 0.68 | 0.81 | 0.78 | 0.73 | 0.82 | 0.87 | 0.81 | 0.93 | 0.74 | 0.70 | 0.79 |
| 179 | 0.75 | 0.66 | 0.84 | 0.87 | 0.72 | 1.00 | 0.71 | 0.62 | 0.81 | 0.79 | 0.73 | 0.84 | 0.89 | 0.84 | 0.95 | 0.74 | 0.68 | 0.81 | 0.79 | 0.75 | 0.84 | 0.90 | 0.85 | 0.95 | 0.75 | 0.70 | 0.80 |
| 180 | 0.76 | 0.68 | 0.84 | 0.87 | 0.73 | 1.00 | 0.73 | 0.64 | 0.82 | 0.79 | 0.73 | 0.84 | 0.90 | 0.84 | 0.95 | 0.74 | 0.67 | 0.81 | 0.79 | 0.75 | 0.84 | 0.90 | 0.85 | 0.95 | 0.75 | 0.70 | 0.81 |
| 181 | 0.77 | 0.68 | 0.85 | 0.85 | 0.70 | 1.00 | 0.74 | 0.65 | 0.83 | 0.79 | 0.73 | 0.84 | 0.89 | 0.84 | 0.95 | 0.74 | 0.67 | 0.81 | 0.80 | 0.75 | 0.84 | 0.90 | 0.84 | 0.95 | 0.76 | 0.71 | 0.81 |
| 182 | 0.78 | 0.71 | 0.84 | 0.86 | 0.69 | 0.98 | 0.75 | 0.67 | 0.83 | 0.80 | 0.74 | 0.85 | 0.87 | 0.78 | 0.96 | 0.77 | 0.70 | 0.83 | 0.80 | 0.76 | 0.84 | 0.87 | 0.80 | 0.94 | 0.77 | 0.72 | 0.82 |
| 183 | 0.78 | 0.72 | 0.85 | 0.86 | 0.73 | 0.96 | 0.76 | 0.68 | 0.83 | 0.79 | 0.74 | 0.85 | 0.87 | 0.78 | 0.97 | 0.76 | 0.70 | 0.83 | 0.80 | 0.76 | 0.84 | 0.87 | 0.81 | 0.94 | 0.77 | 0.72 | 0.82 |
| 184 | 0.76 | 0.67 | 0.85 | 0.89 | 0.76 | 1.00 | 0.72 | 0.62 | 0.82 | 0.79 | 0.74 | 0.85 | 0.88 | 0.81 | 0.95 | 0.76 | 0.70 | 0.82 | 0.80 | 0.76 | 0.85 | 0.90 | 0.84 | 0.96 | 0.77 | 0.71 | 0.82 |
| 185 | 0.73 | 0.66 | 0.81 | 0.79 | 0.72 | 0.91 | 0.72 | 0.63 | 0.80 | 0.79 | 0.73 | 0.85 | 0.86 | 0.77 | 0.95 | 0.77 | 0.70 | 0.84 | 0.78 | 0.73 | 0.82 | 0.84 | 0.77 | 0.91 | 0.76 | 0.70 | 0.81 |
| 186 | 0.77 | 0.68 | 0.85 | 0.86 | 0.67 | 1.00 | 0.74 | 0.65 | 0.83 | 0.80 | 0.75 | 0.85 | 0.89 | 0.83 | 0.94 | 0.76 | 0.70 | 0.83 | 0.81 | 0.76 | 0.85 | 0.89 | 0.84 | 0.95 | 0.77 | 0.72 | 0.82 |
| 187 | 0.77 | 0.69 | 0.85 | 0.86 | 0.69 | 0.96 | 0.74 | 0.65 | 0.84 | 0.80 | 0.74 | 0.85 | 0.89 | 0.81 | 0.96 | 0.76 | 0.69 | 0.82 | 0.80 | 0.75 | 0.84 | 0.88 | 0.83 | 0.94 | 0.76 | 0.71 | 0.81 |
| 188 | 0.76 | 0.68 | 0.84 | 0.85 | 0.77 | 1.00 | 0.73 | 0.65 | 0.82 | 0.79 | 0.74 | 0.85 | 0.89 | 0.84 | 0.95 | 0.75 | 0.68 | 0.81 | 0.80 | 0.76 | 0.84 | 0.90 | 0.85 | 0.95 | 0.76 | 0.71 | 0.81 |
| 189 | 0.77 | 0.69 | 0.85 | 0.84 | 0.67 | 0.98 | 0.74 | 0.65 | 0.84 | 0.80 | 0.74 | 0.86 | 0.90 | 0.83 | 0.96 | 0.76 | 0.70 | 0.83 | 0.80 | 0.76 | 0.84 | 0.89 | 0.82 | 0.95 | 0.77 | 0.72 | 0.82 |
| 190 | 0.74 | 0.67 | 0.82 | 0.83 | 0.71 | 0.96 | 0.71 | 0.63 | 0.80 | 0.79 | 0.73 | 0.85 | 0.87 | 0.80 | 0.94 | 0.76 | 0.69 | 0.83 | 0.78 | 0.74 | 0.83 | 0.86 | 0.80 | 0.92 | 0.75 | 0.70 | 0.80 |
| 191 | 0.74 | 0.66 | 0.83 | 0.85 | 0.71 | 0.99 | 0.71 | 0.62 | 0.81 | 0.79 | 0.73 | 0.84 | 0.90 | 0.85 | 0.95 | 0.74 | 0.67 | 0.81 | 0.79 | 0.74 | 0.83 | 0.90 | 0.85 | 0.95 | 0.75 | 0.69 | 0.82 |
| 192 | 0.77 | 0.69 | 0.85 | 0.86 | 0.71 | 0.98 | 0.74 | 0.65 | 0.83 | 0.79 | 0.74 | 0.85 | 0.88 | 0.80 | 0.97 | 0.76 | 0.69 | 0.83 | 0.80 | 0.75 | 0.84 | 0.88 | 0.81 | 0.95 | 0.77 | 0.72 | 0.81 |
| 193 | 0.74 | 0.66 | 0.82 | 0.87 | 0.74 | 1.00 | 0.71 | 0.62 | 0.80 | 0.79 | 0.73 | 0.85 | 0.91 | 0.87 | 0.96 | 0.73 | 0.66 | 0.80 | 0.79 | 0.73 | 0.82 | 0.91 | 0.86 | 0.96 | 0.74 | 0.68 | 0.80 |
| 194 | 0.75 | 0.66 | 0.84 | 0.88 | 0.71 | 1.00 | 0.70 | 0.61 | 0.82 | 0.79 | 0.73 | 0.85 | 0.89 | 0.83 | 0.95 | 0.75 | 0.68 | 0.82 | 0.79 | 0.75 | 0.84 | 0.90 | 0.85 | 0.95 | 0.75 | 0.70 | 0.82 |
| 195 | 0.74 | 0.66 | 0.81 | 0.83 | 0.72 | 0.95 | 0.70 | 0.61 | 0.79 | 0.79 | 0.73 | 0.85 | 0.87 | 0.80 | 0.94 | 0.76 | 0.69 | 0.83 | 0.78 | 0.73 | 0.82 | 0.86 | 0.80 | 0.92 | 0.75 | 0.70 | 0.80 |
| 196 | 0.79 | 0.71 | 0.86 | 0.88 | 0.77 | 1.00 | 0.75 | 0.67 | 0.84 | 0.79 | 0.74 | 0.85 | 0.87 | 0.79 | 0.95 | 0.77 | 0.70 | 0.83 | 0.80 | 0.76 | 0.84 | 0.88 | 0.82 | 0.94 | 0.77 | 0.72 | 0.82 |

| No. | G | H | I | J | K | L | M | N | O | P | Q | R | S | T | U | V | W | Z | AA | AB | AC | AD | AE | AF | AG | AH | AI |
|-----|---|---|---|---|---|---|---|---|---|---|---|---|---|---|---|---|---|---|----|----|----|----|----|----|----|----|----|
| 197 | 0.74 | 0.67 | 0.81 | 0.81 | 0.67 | 0.94 | 0.72 | 0.64 | 0.80 | 0.79 | 0.74 | 0.85 | 0.86 | 0.77 | 0.95 | 0.76 | 0.70 | 0.83 | 0.79 | 0.74 | 0.83 | 0.85 | 0.78 | 0.92 | 0.76 | 0.71 | 0.81 |
| 198 | 0.74 | 0.67 | 0.82 | 0.87 | 0.73 | 1.00 | 0.71 | 0.62 | 0.79 | 0.79 | 0.73 | 0.84 | 0.87 | 0.79 | 0.96 | 0.75 | 0.68 | 0.82 | 0.78 | 0.74 | 0.83 | 0.88 | 0.82 | 0.95 | 0.75 | 0.70 | 0.80 |
| 199 | 0.75 | 0.68 | 0.82 | 0.80 | 0.66 | 0.95 | 0.73 | 0.65 | 0.81 | 0.79 | 0.74 | 0.85 | 0.87 | 0.79 | 0.95 | 0.76 | 0.70 | 0.83 | 0.78 | 0.74 | 0.83 | 0.85 | 0.78 | 0.92 | 0.76 | 0.71 | 0.80 |
| 200 | 0.76 | 0.68 | 0.84 | 0.91 | 0.77 | 1.00 | 0.72 | 0.62 | 0.81 | 0.79 | 0.73 | 0.84 | 0.90 | 0.85 | 0.95 | 0.74 | 0.67 | 0.81 | 0.80 | 0.75 | 0.84 | 0.92 | 0.87 | 0.97 | 0.75 | 0.70 | 0.81 |
| 201 | 0.74 | 0.67 | 0.82 | 0.87 | 0.72 | 1.00 | 0.71 | 0.62 | 0.80 | 0.79 | 0.73 | 0.84 | 0.88 | 0.80 | 0.96 | 0.75 | 0.68 | 0.82 | 0.78 | 0.74 | 0.83 | 0.88 | 0.82 | 0.95 | 0.75 | 0.70 | 0.80 |
| 202 | 0.74 | 0.66 | 0.83 | 0.89 | 0.71 | 1.00 | 0.70 | 0.60 | 0.79 | 0.79 | 0.73 | 0.85 | 0.89 | 0.83 | 0.96 | 0.75 | 0.68 | 0.82 | 0.79 | 0.74 | 0.83 | 0.91 | 0.85 | 0.97 | 0.75 | 0.69 | 0.80 |
| 203 | 0.74 | 0.66 | 0.82 | 0.77 | 0.63 | 0.92 | 0.73 | 0.64 | 0.83 | 0.80 | 0.74 | 0.85 | 0.89 | 0.82 | 0.96 | 0.76 | 0.69 | 0.83 | 0.79 | 0.74 | 0.83 | 0.86 | 0.79 | 0.93 | 0.76 | 0.71 | 0.81 |
| 204 | 0.74 | 0.67 | 0.81 | 0.83 | 0.69 | 0.97 | 0.71 | 0.63 | 0.79 | 0.80 | 0.74 | 0.85 | 0.89 | 0.81 | 0.96 | 0.76 | 0.69 | 0.83 | 0.79 | 0.74 | 0.83 | 0.88 | 0.81 | 0.94 | 0.75 | 0.70 | 0.80 |
| 205 | 0.74 | 0.67 | 0.82 | 0.84 | 0.73 | 0.95 | 0.71 | 0.62 | 0.80 | 0.78 | 0.72 | 0.84 | 0.82 | 0.70 | 0.95 | 0.76 | 0.69 | 0.82 | 0.77 | 0.73 | 0.82 | 0.84 | 0.75 | 0.92 | 0.75 | 0.70 | 0.80 |
| 206 | 0.73 | 0.65 | 0.81 | 0.87 | 0.73 | 1.00 | 0.69 | 0.61 | 0.78 | 0.79 | 0.73 | 0.84 | 0.88 | 0.80 | 0.96 | 0.75 | 0.68 | 0.82 | 0.78 | 0.74 | 0.82 | 0.89 | 0.82 | 0.95 | 0.74 | 0.69 | 0.79 |
| 207 | 0.75 | 0.66 | 0.83 | 0.82 | 0.68 | 0.95 | 0.73 | 0.62 | 0.83 | 0.80 | 0.74 | 0.85 | 0.89 | 0.82 | 0.95 | 0.76 | 0.69 | 0.83 | 0.79 | 0.74 | 0.84 | 0.87 | 0.81 | 0.93 | 0.76 | 0.70 | 0.82 |
| 208 | 0.78 | 0.70 | 0.87 | 0.91 | 0.79 | 1.00 | 0.75 | 0.65 | 0.84 | 0.79 | 0.73 | 0.84 | 0.91 | 0.86 | 0.96 | 0.74 | 0.66 | 0.81 | 0.80 | 0.76 | 0.85 | 0.92 | 0.88 | 0.97 | 0.76 | 0.70 | 0.81 |
| 209 | 0.75 | 0.67 | 0.83 | 0.87 | 0.72 | 1.00 | 0.71 | 0.62 | 0.80 | 0.78 | 0.73 | 0.84 | 0.90 | 0.84 | 0.96 | 0.73 | 0.67 | 0.80 | 0.78 | 0.74 | 0.82 | 0.90 | 0.84 | 0.95 | 0.74 | 0.69 | 0.79 |
| 210 | 0.75 | 0.67 | 0.83 | 0.89 | 0.76 | 1.00 | 0.71 | 0.61 | 0.81 | 0.79 | 0.73 | 0.84 | 0.91 | 0.86 | 0.96 | 0.74 | 0.67 | 0.81 | 0.79 | 0.75 | 0.84 | 0.92 | 0.87 | 0.96 | 0.74 | 0.69 | 0.80 |
| 211 | 0.78 | 0.69 | 0.86 | 0.90 | 0.73 | 1.00 | 0.74 | 0.65 | 0.83 | 0.79 | 0.73 | 0.84 | 0.89 | 0.83 | 0.94 | 0.75 | 0.68 | 0.81 | 0.80 | 0.76 | 0.85 | 0.91 | 0.86 | 0.96 | 0.76 | 0.71 | 0.82 |
| 212 | 0.74 | 0.66 | 0.82 | 0.80 | 0.67 | 0.93 | 0.72 | 0.62 | 0.82 | 0.80 | 0.74 | 0.85 | 0.89 | 0.83 | 0.96 | 0.76 | 0.69 | 0.82 | 0.78 | 0.74 | 0.83 | 0.87 | 0.81 | 0.93 | 0.75 | 0.70 | 0.81 |
| 213 | 0.74 | 0.66 | 0.83 | 0.86 | 0.70 | 1.00 | 0.71 | 0.61 | 0.81 | 0.78 | 0.73 | 0.84 | 0.89 | 0.83 | 0.95 | 0.74 | 0.67 | 0.81 | 0.78 | 0.74 | 0.83 | 0.89 | 0.83 | 0.95 | 0.74 | 0.69 | 0.80 |
| 214 | 0.73 | 0.64 | 0.82 | 0.90 | 0.76 | 1.00 | 0.68 | 0.59 | 0.78 | 0.79 | 0.73 | 0.84 | 0.92 | 0.87 | 0.97 | 0.73 | 0.66 | 0.80 | 0.78 | 0.74 | 0.83 | 0.92 | 0.87 | 0.97 | 0.73 | 0.67 | 0.78 |
| 215 | 0.74 | 0.67 | 0.81 | 0.79 | 0.64 | 0.94 | 0.72 | 0.64 | 0.80 | 0.79 | 0.74 | 0.85 | 0.86 | 0.77 | 0.95 | 0.76 | 0.70 | 0.83 | 0.79 | 0.74 | 0.83 | 0.84 | 0.77 | 0.92 | 0.76 | 0.72 | 0.81 |
| 216 | 0.76 | 0.67 | 0.84 | 0.87 | 0.74 | 1.00 | 0.72 | 0.62 | 0.82 | 0.79 | 0.73 | 0.85 | 0.90 | 0.85 | 0.95 | 0.74 | 0.67 | 0.81 | 0.79 | 0.75 | 0.84 | 0.91 | 0.86 | 0.95 | 0.75 | 0.69 | 0.80 |
| 217 | 0.76 | 0.69 | 0.83 | 0.83 | 0.71 | 0.95 | 0.73 | 0.65 | 0.81 | 0.79 | 0.74 | 0.85 | 0.85 | 0.74 | 0.95 | 0.77 | 0.71 | 0.83 | 0.78 | 0.74 | 0.82 | 0.84 | 0.77 | 0.92 | 0.76 | 0.71 | 0.81 |
| 218 | 0.74 | 0.65 | 0.82 | 0.90 | 0.75 | 1.00 | 0.69 | 0.60 | 0.79 | 0.79 | 0.74 | 0.84 | 0.94 | 0.91 | 0.97 | 0.72 | 0.65 | 0.79 | 0.78 | 0.73 | 0.82 | 0.93 | 0.89 | 0.98 | 0.72 | 0.66 | 0.78 |
| 219 | 0.74 | 0.65 | 0.82 | 0.81 | 0.67 | 0.96 | 0.71 | 0.61 | 0.82 | 0.80 | 0.73 | 0.85 | 0.88 | 0.82 | 0.94 | 0.76 | 0.69 | 0.83 | 0.78 | 0.74 | 0.83 | 0.86 | 0.80 | 0.93 | 0.75 | 0.69 | 0.81 |
| 220 | 0.74 | 0.67 | 0.81 | 0.79 | 0.68 | 0.90 | 0.72 | 0.64 | 0.80 | 0.79 | 0.74 | 0.85 | 0.87 | 0.80 | 0.95 | 0.76 | 0.69 | 0.83 | 0.78 | 0.74 | 0.82 | 0.85 | 0.78 | 0.91 | 0.76 | 0.70 | 0.81 |
| 221 | 0.73 | 0.64 | 0.82 | 0.76 | 0.58 | 0.94 | 0.72 | 0.62 | 0.83 | 0.80 | 0.74 | 0.85 | 0.88 | 0.82 | 0.94 | 0.76 | 0.69 | 0.83 | 0.78 | 0.73 | 0.83 | 0.85 | 0.78 | 0.92 | 0.75 | 0.72 | 0.81 |
| 222 | 0.77 | 0.70 | 0.84 | 0.85 | 0.72 | 0.99 | 0.75 | 0.67 | 0.83 | 0.79 | 0.74 | 0.85 | 0.88 | 0.80 | 0.96 | 0.76 | 0.70 | 0.82 | 0.80 | 0.76 | 0.84 | 0.88 | 0.82 | 0.95 | 0.77 | 0.70 | 0.82 |
| 223 | 0.75 | 0.67 | 0.84 | 0.85 | 0.69 | 1.00 | 0.73 | 0.63 | 0.82 | 0.79 | 0.73 | 0.84 | 0.89 | 0.83 | 0.95 | 0.76 | 0.67 | 0.81 | 0.79 | 0.75 | 0.84 | 0.89 | 0.84 | 0.95 | 0.76 | 0.72 | 0.81 |
| 224 | 0.75 | 0.67 | 0.84 | 0.85 | 0.69 | 1.00 | 0.73 | 0.63 | 0.82 | 0.79 | 0.73 | 0.84 | 0.89 | 0.83 | 0.94 | 0.74 | 0.67 | 0.81 | 0.79 | 0.75 | 0.84 | 0.90 | 0.84 | 0.95 | 0.76 | 0.70 | 0.81 |
| 225 | 0.77 | 0.70 | 0.84 | 0.84 | 0.71 | 0.96 | 0.75 | 0.67 | 0.83 | 0.79 | 0.74 | 0.85 | 0.88 | 0.81 | 0.95 | 0.75 | 0.69 | 0.82 | 0.79 | 0.75 | 0.83 | 0.87 | 0.81 | 0.93 | 0.76 | 0.71 | 0.81 |
| 226 | 0.75 | 0.67 | 0.82 | 0.86 | 0.77 | 0.96 | 0.70 | 0.61 | 0.80 | 0.78 | 0.72 | 0.83 | 0.82 | 0.71 | 0.94 | 0.76 | 0.69 | 0.82 | 0.77 | 0.73 | 0.82 | 0.84 | 0.77 | 0.92 | 0.75 | 0.70 | 0.80 |
| 227 | 0.77 | 0.69 | 0.85 | 0.90 | 0.77 | 1.00 | 0.73 | 0.64 | 0.82 | 0.79 | 0.73 | 0.84 | 0.91 | 0.87 | 0.95 | 0.74 | 0.67 | 0.80 | 0.80 | 0.76 | 0.84 | 0.92 | 0.88 | 0.97 | 0.75 | 0.70 | 0.81 |
| 228 | 0.75 | 0.67 | 0.84 | 0.89 | 0.74 | 1.00 | 0.71 | 0.62 | 0.81 | 0.79 | 0.73 | 0.84 | 0.90 | 0.85 | 0.96 | 0.74 | 0.67 | 0.81 | 0.80 | 0.75 | 0.84 | 0.92 | 0.87 | 0.96 | 0.75 | 0.70 | 0.80 |
| 229 | 0.75 | 0.68 | 0.82 | 0.82 | 0.71 | 0.94 | 0.73 | 0.65 | 0.81 | 0.79 | 0.74 | 0.85 | 0.85 | 0.77 | 0.94 | 0.76 | 0.70 | 0.83 | 0.79 | 0.75 | 0.83 | 0.85 | 0.78 | 0.92 | 0.76 | 0.71 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 230 | 0.75 | 0.67 | 0.83 | 0.79 | 0.64 | 0.94 | 0.74 | 0.65 | 0.83 | 0.79 | 0.74 | 0.85 | 0.89 | 0.82 | 0.95 | 0.76 | 0.69 | 0.82 | 0.79 | 0.75 | 0.84 | 0.86 | 0.79 | 0.93 | 0.77 | 0.72 | 0.82 |
| 231 | 0.74 | 0.66 | 0.83 | 0.89 | 0.73 | 1.00 | 0.70 | 0.60 | 0.79 | 0.79 | 0.73 | 0.84 | 0.91 | 0.87 | 0.96 | 0.73 | 0.66 | 0.80 | 0.79 | 0.74 | 0.83 | 0.92 | 0.87 | 0.97 | 0.74 | 0.68 | 0.79 |
| 232 | 0.76 | 0.68 | 0.84 | 0.87 | 0.70 | 1.00 | 0.73 | 0.65 | 0.82 | 0.79 | 0.73 | 0.84 | 0.90 | 0.85 | 0.96 | 0.74 | 0.67 | 0.81 | 0.80 | 0.76 | 0.84 | 0.91 | 0.85 | 0.96 | 0.76 | 0.71 | 0.81 |
| 233 | 0.75 | 0.66 | 0.83 | 0.87 | 0.73 | 1.00 | 0.71 | 0.61 | 0.81 | 0.79 | 0.73 | 0.85 | 0.90 | 0.85 | 0.95 | 0.74 | 0.67 | 0.81 | 0.79 | 0.74 | 0.83 | 0.90 | 0.85 | 0.95 | 0.74 | 0.69 | 0.80 |
| 234 | 0.74 | 0.66 | 0.82 | 0.85 | 0.71 | 0.99 | 0.71 | 0.62 | 0.80 | 0.78 | 0.73 | 0.84 | 0.87 | 0.80 | 0.94 | 0.75 | 0.68 | 0.81 | 0.78 | 0.73 | 0.82 | 0.87 | 0.81 | 0.93 | 0.74 | 0.69 | 0.79 |
| 235 | 0.75 | 0.68 | 0.82 | 0.81 | 0.68 | 0.95 | 0.73 | 0.65 | 0.81 | 0.79 | 0.74 | 0.85 | 0.86 | 0.76 | 0.95 | 0.76 | 0.70 | 0.83 | 0.79 | 0.75 | 0.83 | 0.85 | 0.78 | 0.92 | 0.76 | 0.72 | 0.81 |
| 236 | 0.79 | 0.73 | 0.86 | 0.90 | 0.82 | 0.98 | 0.76 | 0.68 | 0.84 | 0.79 | 0.73 | 0.85 | 0.88 | 0.80 | 0.96 | 0.75 | 0.69 | 0.82 | 0.80 | 0.76 | 0.84 | 0.89 | 0.84 | 0.95 | 0.77 | 0.72 | 0.82 |
| 237 | 0.77 | 0.70 | 0.83 | 0.86 | 0.76 | 0.96 | 0.74 | 0.66 | 0.81 | 0.79 | 0.73 | 0.85 | 0.88 | 0.79 | 0.96 | 0.76 | 0.69 | 0.82 | 0.79 | 0.75 | 0.83 | 0.87 | 0.81 | 0.94 | 0.76 | 0.71 | 0.81 |
| 238 | 0.79 | 0.72 | 0.86 | 0.89 | 0.81 | 0.98 | 0.76 | 0.68 | 0.84 | 0.79 | 0.74 | 0.85 | 0.88 | 0.80 | 0.96 | 0.76 | 0.69 | 0.82 | 0.80 | 0.76 | 0.84 | 0.89 | 0.83 | 0.95 | 0.77 | 0.72 | 0.82 |
| 239 | 0.73 | 0.64 | 0.82 | 0.87 | 0.70 | 1.00 | 0.69 | 0.59 | 0.79 | 0.79 | 0.73 | 0.84 | 0.87 | 0.80 | 0.94 | 0.75 | 0.69 | 0.81 | 0.79 | 0.75 | 0.83 | 0.89 | 0.83 | 0.95 | 0.75 | 0.70 | 0.80 |
| 240 | 0.74 | 0.65 | 0.82 | 0.88 | 0.75 | 1.00 | 0.69 | 0.60 | 0.79 | 0.78 | 0.72 | 0.84 | 0.88 | 0.81 | 0.96 | 0.74 | 0.68 | 0.81 | 0.77 | 0.72 | 0.82 | 0.88 | 0.82 | 0.95 | 0.73 | 0.68 | 0.78 |
| 241 | 0.76 | 0.67 | 0.84 | 0.90 | 0.77 | 1.00 | 0.72 | 0.62 | 0.82 | 0.78 | 0.73 | 0.84 | 0.89 | 0.84 | 0.95 | 0.74 | 0.67 | 0.81 | 0.79 | 0.75 | 0.84 | 0.91 | 0.86 | 0.96 | 0.75 | 0.69 | 0.80 |
| 242 | 0.74 | 0.66 | 0.83 | 0.86 | 0.70 | 1.00 | 0.71 | 0.61 | 0.81 | 0.78 | 0.73 | 0.84 | 0.90 | 0.84 | 0.96 | 0.73 | 0.67 | 0.80 | 0.78 | 0.74 | 0.83 | 0.90 | 0.84 | 0.95 | 0.74 | 0.69 | 0.80 |
| 243 | 0.73 | 0.66 | 0.81 | 0.78 | 0.63 | 0.93 | 0.72 | 0.63 | 0.80 | 0.79 | 0.73 | 0.85 | 0.85 | 0.77 | 0.93 | 0.76 | 0.70 | 0.83 | 0.78 | 0.73 | 0.82 | 0.84 | 0.77 | 0.91 | 0.76 | 0.70 | 0.81 |
| 244 | 0.73 | 0.66 | 0.81 | 0.80 | 0.65 | 0.94 | 0.71 | 0.63 | 0.79 | 0.79 | 0.73 | 0.85 | 0.86 | 0.78 | 0.95 | 0.76 | 0.70 | 0.83 | 0.78 | 0.74 | 0.83 | 0.85 | 0.78 | 0.92 | 0.76 | 0.71 | 0.81 |
| 245 | 0.76 | 0.68 | 0.84 | 0.87 | 0.70 | 1.00 | 0.73 | 0.65 | 0.82 | 0.79 | 0.73 | 0.84 | 0.90 | 0.85 | 0.96 | 0.73 | 0.67 | 0.80 | 0.80 | 0.76 | 0.84 | 0.91 | 0.86 | 0.96 | 0.75 | 0.70 | 0.80 |
| 246 | 0.72 | 0.63 | 0.81 | 0.76 | 0.62 | 0.91 | 0.70 | 0.60 | 0.81 | 0.79 | 0.74 | 0.85 | 0.87 | 0.79 | 0.95 | 0.76 | 0.69 | 0.83 | 0.78 | 0.73 | 0.83 | 0.84 | 0.77 | 0.91 | 0.76 | 0.70 | 0.81 |
| 247 | 0.74 | 0.67 | 0.81 | 0.83 | 0.70 | 0.95 | 0.71 | 0.63 | 0.80 | 0.79 | 0.73 | 0.85 | 0.86 | 0.78 | 0.94 | 0.76 | 0.69 | 0.83 | 0.78 | 0.74 | 0.82 | 0.86 | 0.79 | 0.92 | 0.75 | 0.70 | 0.80 |
| 248 | 0.77 | 0.69 | 0.86 | 0.91 | 0.78 | 1.00 | 0.73 | 0.63 | 0.83 | 0.79 | 0.73 | 0.84 | 0.91 | 0.87 | 0.96 | 0.73 | 0.66 | 0.80 | 0.80 | 0.75 | 0.84 | 0.92 | 0.88 | 0.97 | 0.75 | 0.70 | 0.81 |
| 249 | 0.75 | 0.67 | 0.82 | 0.84 | 0.74 | 0.95 | 0.72 | 0.63 | 0.81 | 0.77 | 0.71 | 0.84 | 0.83 | 0.71 | 0.95 | 0.75 | 0.69 | 0.82 | 0.77 | 0.73 | 0.82 | 0.84 | 0.76 | 0.92 | 0.75 | 0.70 | 0.80 |
| 250 | 0.73 | 0.65 | 0.81 | 0.85 | 0.71 | 0.99 | 0.70 | 0.61 | 0.79 | 0.78 | 0.73 | 0.84 | 0.87 | 0.80 | 0.94 | 0.74 | 0.68 | 0.81 | 0.77 | 0.73 | 0.82 | 0.87 | 0.81 | 0.93 | 0.74 | 0.68 | 0.79 |
| 251 | 0.76 | 0.68 | 0.85 | 0.86 | 0.71 | 1.00 | 0.73 | 0.63 | 0.83 | 0.78 | 0.73 | 0.84 | 0.89 | 0.83 | 0.94 | 0.74 | 0.67 | 0.81 | 0.80 | 0.75 | 0.84 | 0.89 | 0.84 | 0.95 | 0.76 | 0.71 | 0.81 |
| 252 | 0.73 | 0.66 | 0.81 | 0.78 | 0.65 | 0.92 | 0.72 | 0.64 | 0.80 | 0.79 | 0.73 | 0.85 | 0.86 | 0.78 | 0.95 | 0.76 | 0.69 | 0.83 | 0.78 | 0.74 | 0.82 | 0.84 | 0.77 | 0.91 | 0.76 | 0.71 | 0.81 |
| 253 | 0.74 | 0.66 | 0.81 | 0.79 | 0.65 | 0.93 | 0.72 | 0.64 | 0.80 | 0.79 | 0.73 | 0.85 | 0.85 | 0.78 | 0.93 | 0.76 | 0.70 | 0.83 | 0.78 | 0.74 | 0.83 | 0.85 | 0.78 | 0.91 | 0.76 | 0.71 | 0.81 |
| 254 | 0.77 | 0.69 | 0.85 | 0.90 | 0.78 | 1.00 | 0.73 | 0.64 | 0.82 | 0.79 | 0.73 | 0.84 | 0.91 | 0.87 | 0.96 | 0.73 | 0.66 | 0.80 | 0.80 | 0.76 | 0.84 | 0.92 | 0.88 | 0.96 | 0.75 | 0.70 | 0.80 |
| 255 | 0.78 | 0.71 | 0.85 | 0.85 | 0.71 | 0.99 | 0.76 | 0.67 | 0.84 | 0.79 | 0.74 | 0.85 | 0.88 | 0.80 | 0.95 | 0.76 | 0.69 | 0.82 | 0.79 | 0.75 | 0.83 | 0.87 | 0.81 | 0.93 | 0.76 | 0.71 | 0.81 |
| 256 | 0.74 | 0.67 | 0.82 | 0.80 | 0.67 | 0.93 | 0.72 | 0.64 | 0.81 | 0.79 | 0.73 | 0.85 | 0.85 | 0.77 | 0.94 | 0.76 | 0.70 | 0.83 | 0.78 | 0.74 | 0.83 | 0.85 | 0.78 | 0.91 | 0.76 | 0.71 | 0.81 |
| 257 | 0.75 | 0.66 | 0.83 | 0.89 | 0.73 | 1.00 | 0.71 | 0.61 | 0.80 | 0.78 | 0.73 | 0.84 | 0.91 | 0.87 | 0.96 | 0.73 | 0.66 | 0.80 | 0.79 | 0.74 | 0.83 | 0.92 | 0.87 | 0.97 | 0.74 | 0.68 | 0.79 |
| 258 | 0.73 | 0.64 | 0.82 | 0.72 | 0.54 | 0.90 | 0.73 | 0.63 | 0.83 | 0.80 | 0.75 | 0.85 | 0.86 | 0.79 | 0.94 | 0.77 | 0.71 | 0.84 | 0.79 | 0.75 | 0.84 | 0.83 | 0.75 | 0.91 | 0.78 | 0.73 | 0.83 |
| 259 | 0.74 | 0.66 | 0.82 | 0.86 | 0.75 | 0.96 | 0.70 | 0.60 | 0.79 | 0.77 | 0.71 | 0.83 | 0.82 | 0.71 | 0.94 | 0.76 | 0.69 | 0.82 | 0.77 | 0.72 | 0.81 | 0.84 | 0.76 | 0.92 | 0.74 | 0.69 | 0.79 |
| 260 | 0.75 | 0.66 | 0.84 | 0.86 | 0.70 | 1.00 | 0.72 | 0.62 | 0.82 | 0.78 | 0.73 | 0.84 | 0.90 | 0.85 | 0.95 | 0.74 | 0.67 | 0.80 | 0.79 | 0.75 | 0.84 | 0.90 | 0.85 | 0.95 | 0.75 | 0.70 | 0.81 |
| 261 | 0.75 | 0.67 | 0.83 | 0.89 | 0.72 | 1.00 | 0.72 | 0.63 | 0.80 | 0.79 | 0.73 | 0.84 | 0.89 | 0.82 | 0.96 | 0.74 | 0.68 | 0.81 | 0.78 | 0.74 | 0.83 | 0.90 | 0.83 | 0.96 | 0.74 | 0.69 | 0.79 |
| 262 | 0.78 | 0.71 | 0.85 | 0.88 | 0.77 | 1.00 | 0.74 | 0.66 | 0.83 | 0.79 | 0.74 | 0.85 | 0.87 | 0.79 | 0.95 | 0.76 | 0.70 | 0.83 | 0.80 | 0.76 | 0.84 | 0.88 | 0.82 | 0.94 | 0.77 | 0.72 | 0.81 |

EP 2 791 683 B1

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 263 | 0.74 | 0.65 | 0.82 | 0.77 | 0.62 | 0.92 | 0.73 | 0.63 | 0.83 | 0.79 | 0.74 | 0.85 | 0.87 | 0.79 | 0.95 | 0.76 | 0.70 | 0.82 | 0.79 | 0.74 | 0.83 | 0.84 | 0.76 | 0.91 | 0.77 | 0.72 | 0.82 |
| 264 | 0.76 | 0.67 | 0.84 | 0.81 | 0.70 | 0.93 | 0.74 | 0.64 | 0.84 | 0.79 | 0.74 | 0.85 | 0.88 | 0.81 | 0.96 | 0.76 | 0.69 | 0.83 | 0.79 | 0.74 | 0.84 | 0.87 | 0.80 | 0.93 | 0.76 | 0.71 | 0.82 |
| 265 | 0.76 | 0.68 | 0.84 | 0.81 | 0.63 | 0.98 | 0.74 | 0.65 | 0.84 | 0.80 | 0.75 | 0.86 | 0.85 | 0.76 | 0.95 | 0.79 | 0.72 | 0.85 | 0.80 | 0.76 | 0.84 | 0.85 | 0.77 | 0.93 | 0.78 | 0.73 | 0.83 |
| 266 | 0.75 | 0.68 | 0.82 | 0.81 | 0.65 | 0.96 | 0.73 | 0.65 | 0.81 | 0.79 | 0.73 | 0.85 | 0.87 | 0.79 | 0.95 | 0.76 | 0.69 | 0.83 | 0.78 | 0.74 | 0.82 | 0.85 | 0.78 | 0.92 | 0.75 | 0.70 | 0.80 |
| 267 | 0.75 | 0.67 | 0.83 | 0.82 | 0.71 | 0.93 | 0.72 | 0.63 | 0.82 | 0.79 | 0.74 | 0.84 | 0.85 | 0.77 | 0.93 | 0.76 | 0.70 | 0.83 | 0.79 | 0.75 | 0.83 | 0.85 | 0.79 | 0.91 | 0.76 | 0.71 | 0.81 |
| 268 | 0.76 | 0.68 | 0.84 | 0.89 | 0.73 | 1.00 | 0.72 | 0.63 | 0.82 | 0.79 | 0.73 | 0.85 | 0.91 | 0.85 | 0.96 | 0.74 | 0.67 | 0.81 | 0.80 | 0.75 | 0.84 | 0.92 | 0.86 | 0.97 | 0.76 | 0.70 | 0.81 |
| 269 | 0.74 | 0.65 | 0.82 | 0.79 | 0.65 | 0.93 | 0.72 | 0.62 | 0.82 | 0.79 | 0.74 | 0.85 | 0.89 | 0.82 | 0.96 | 0.76 | 0.69 | 0.82 | 0.78 | 0.74 | 0.83 | 0.86 | 0.80 | 0.93 | 0.75 | 0.70 | 0.81 |
| 270 | 0.76 | 0.68 | 0.84 | 0.89 | 0.73 | 1.00 | 0.72 | 0.63 | 0.82 | 0.79 | 0.73 | 0.84 | 0.90 | 0.84 | 0.96 | 0.74 | 0.67 | 0.81 | 0.80 | 0.75 | 0.84 | 0.91 | 0.86 | 0.97 | 0.76 | 0.70 | 0.81 |
| 271 | 0.73 | 0.66 | 0.81 | 0.86 | 0.74 | 0.98 | 0.70 | 0.61 | 0.79 | 0.78 | 0.73 | 0.84 | 0.89 | 0.83 | 0.95 | 0.74 | 0.67 | 0.81 | 0.77 | 0.73 | 0.82 | 0.89 | 0.84 | 0.94 | 0.73 | 0.68 | 0.78 |
| 272 | 0.77 | 0.69 | 0.85 | 0.88 | 0.71 | 1.00 | 0.74 | 0.65 | 0.82 | 0.80 | 0.74 | 0.85 | 0.89 | 0.83 | 0.94 | 0.76 | 0.69 | 0.82 | 0.81 | 0.76 | 0.85 | 0.90 | 0.85 | 0.96 | 0.77 | 0.72 | 0.82 |
| 273 | 0.75 | 0.67 | 0.82 | 0.87 | 0.78 | 0.96 | 0.71 | 0.62 | 0.80 | 0.78 | 0.72 | 0.83 | 0.83 | 0.71 | 0.94 | 0.76 | 0.69 | 0.82 | 0.77 | 0.73 | 0.82 | 0.85 | 0.77 | 0.92 | 0.74 | 0.69 | 0.80 |
| 274 | 0.75 | 0.68 | 0.82 | 0.86 | 0.71 | 1.00 | 0.72 | 0.63 | 0.80 | 0.79 | 0.74 | 0.85 | 0.86 | 0.79 | 0.94 | 0.77 | 0.70 | 0.83 | 0.79 | 0.75 | 0.83 | 0.88 | 0.81 | 0.94 | 0.76 | 0.71 | 0.81 |
| 275 | 0.75 | 0.67 | 0.84 | 0.78 | 0.60 | 0.96 | 0.74 | 0.65 | 0.84 | 0.79 | 0.74 | 0.85 | 0.87 | 0.81 | 0.94 | 0.76 | 0.69 | 0.83 | 0.79 | 0.75 | 0.84 | 0.86 | 0.79 | 0.93 | 0.77 | 0.71 | 0.82 |
| 276 | 0.74 | 0.67 | 0.82 | 0.83 | 0.74 | 0.93 | 0.71 | 0.62 | 0.80 | 0.78 | 0.72 | 0.84 | 0.83 | 0.73 | 0.92 | 0.76 | 0.70 | 0.83 | 0.78 | 0.74 | 0.82 | 0.84 | 0.78 | 0.90 | 0.76 | 0.70 | 0.81 |
| 277 | 0.74 | 0.66 | 0.82 | 0.86 | 0.72 | 1.00 | 0.71 | 0.61 | 0.80 | 0.79 | 0.74 | 0.85 | 0.89 | 0.84 | 0.94 | 0.75 | 0.68 | 0.81 | 0.79 | 0.75 | 0.84 | 0.90 | 0.85 | 0.95 | 0.75 | 0.70 | 0.81 |
| 278 | 0.76 | 0.68 | 0.84 | 0.82 | 0.68 | 0.97 | 0.74 | 0.65 | 0.83 | 0.79 | 0.74 | 0.85 | 0.92 | 0.87 | 0.96 | 0.74 | 0.67 | 0.81 | 0.79 | 0.75 | 0.84 | 0.89 | 0.84 | 0.95 | 0.76 | 0.70 | 0.81 |
| 279 | 0.75 | 0.66 | 0.83 | 0.85 | 0.69 | 1.00 | 0.72 | 0.62 | 0.81 | 0.78 | 0.73 | 0.84 | 0.89 | 0.83 | 0.94 | 0.74 | 0.67 | 0.81 | 0.79 | 0.75 | 0.84 | 0.89 | 0.84 | 0.95 | 0.75 | 0.70 | 0.81 |
| 280 | 0.77 | 0.69 | 0.84 | 0.84 | 0.71 | 0.97 | 0.74 | 0.66 | 0.82 | 0.79 | 0.74 | 0.84 | 0.86 | 0.78 | 0.95 | 0.76 | 0.70 | 0.82 | 0.80 | 0.76 | 0.84 | 0.87 | 0.80 | 0.93 | 0.77 | 0.72 | 0.82 |
| 281 | 0.75 | 0.67 | 0.83 | 0.89 | 0.72 | 1.00 | 0.71 | 0.62 | 0.80 | 0.78 | 0.73 | 0.84 | 0.89 | 0.83 | 0.95 | 0.74 | 0.67 | 0.81 | 0.79 | 0.75 | 0.83 | 0.91 | 0.85 | 0.96 | 0.75 | 0.69 | 0.80 |
| 282 | 0.74 | 0.66 | 0.83 | 0.87 | 0.70 | 1.00 | 0.71 | 0.62 | 0.80 | 0.78 | 0.73 | 0.84 | 0.91 | 0.87 | 0.96 | 0.73 | 0.66 | 0.80 | 0.79 | 0.74 | 0.83 | 0.91 | 0.86 | 0.96 | 0.74 | 0.68 | 0.79 |
| 283 | 0.74 | 0.66 | 0.81 | 0.82 | 0.72 | 0.92 | 0.71 | 0.62 | 0.80 | 0.79 | 0.73 | 0.84 | 0.87 | 0.79 | 0.94 | 0.75 | 0.69 | 0.82 | 0.78 | 0.73 | 0.82 | 0.85 | 0.80 | 0.91 | 0.74 | 0.69 | 0.80 |
| 284 | 0.72 | 0.63 | 0.81 | 0.78 | 0.59 | 0.96 | 0.71 | 0.60 | 0.81 | 0.80 | 0.74 | 0.85 | 0.84 | 0.75 | 0.93 | 0.78 | 0.72 | 0.84 | 0.78 | 0.74 | 0.83 | 0.83 | 0.75 | 0.91 | 0.76 | 0.71 | 0.81 |
| 285 | 0.77 | 0.68 | 0.86 | 0.89 | 0.71 | 1.00 | 0.74 | 0.64 | 0.83 | 0.78 | 0.73 | 0.84 | 0.91 | 0.86 | 0.95 | 0.73 | 0.66 | 0.80 | 0.79 | 0.75 | 0.84 | 0.91 | 0.86 | 0.97 | 0.75 | 0.69 | 0.80 |
| 286 | 0.77 | 0.68 | 0.85 | 0.88 | 0.70 | 1.00 | 0.74 | 0.64 | 0.83 | 0.78 | 0.73 | 0.84 | 0.90 | 0.85 | 0.95 | 0.74 | 0.67 | 0.81 | 0.79 | 0.75 | 0.84 | 0.90 | 0.85 | 0.96 | 0.75 | 0.70 | 0.81 |
| 287 | 0.75 | 0.67 | 0.82 | 0.81 | 0.67 | 0.95 | 0.72 | 0.64 | 0.81 | 0.79 | 0.73 | 0.84 | 0.87 | 0.80 | 0.94 | 0.76 | 0.69 | 0.82 | 0.77 | 0.73 | 0.81 | 0.85 | 0.78 | 0.91 | 0.74 | 0.69 | 0.79 |
| 288 | 0.74 | 0.66 | 0.82 | 0.86 | 0.76 | 0.97 | 0.70 | 0.61 | 0.79 | 0.77 | 0.71 | 0.83 | 0.83 | 0.72 | 0.94 | 0.75 | 0.68 | 0.82 | 0.77 | 0.72 | 0.81 | 0.85 | 0.77 | 0.92 | 0.74 | 0.69 | 0.79 |
| 289 | 0.78 | 0.70 | 0.86 | 0.90 | 0.77 | 1.00 | 0.74 | 0.65 | 0.83 | 0.79 | 0.74 | 0.85 | 0.89 | 0.84 | 0.95 | 0.75 | 0.69 | 0.82 | 0.81 | 0.76 | 0.85 | 0.91 | 0.87 | 0.96 | 0.77 | 0.71 | 0.82 |
| 290 | 0.75 | 0.68 | 0.82 | 0.81 | 0.71 | 0.90 | 0.73 | 0.65 | 0.82 | 0.79 | 0.74 | 0.85 | 0.86 | 0.80 | 0.92 | 0.76 | 0.69 | 0.83 | 0.79 | 0.74 | 0.83 | 0.85 | 0.80 | 0.90 | 0.76 | 0.71 | 0.81 |
| 291 | 0.76 | 0.68 | 0.84 | 0.88 | 0.74 | 1.00 | 0.73 | 0.64 | 0.81 | 0.79 | 0.73 | 0.84 | 0.89 | 0.83 | 0.95 | 0.75 | 0.68 | 0.82 | 0.79 | 0.74 | 0.83 | 0.89 | 0.84 | 0.95 | 0.75 | 0.70 | 0.80 |
| 292 | 0.75 | 0.67 | 0.84 | 0.87 | 0.70 | 1.00 | 0.72 | 0.62 | 0.81 | 0.78 | 0.73 | 0.84 | 0.89 | 0.84 | 0.95 | 0.74 | 0.67 | 0.81 | 0.79 | 0.75 | 0.84 | 0.90 | 0.84 | 0.96 | 0.75 | 0.70 | 0.80 |
| 293 | 0.76 | 0.69 | 0.83 | 0.86 | 0.75 | 0.96 | 0.73 | 0.65 | 0.81 | 0.79 | 0.73 | 0.84 | 0.85 | 0.75 | 0.94 | 0.76 | 0.70 | 0.83 | 0.78 | 0.74 | 0.82 | 0.85 | 0.78 | 0.92 | 0.75 | 0.71 | 0.80 |
| 294 | 0.75 | 0.67 | 0.83 | 0.87 | 0.75 | 1.00 | 0.72 | 0.62 | 0.81 | 0.78 | 0.73 | 0.84 | 0.90 | 0.85 | 0.95 | 0.73 | 0.66 | 0.81 | 0.79 | 0.74 | 0.83 | 0.91 | 0.86 | 0.95 | 0.74 | 0.69 | 0.80 |
| 295 | 0.74 | 0.66 | 0.81 | 0.86 | 0.75 | 0.97 | 0.70 | 0.61 | 0.79 | 0.78 | 0.72 | 0.84 | 0.89 | 0.83 | 0.95 | 0.73 | 0.66 | 0.80 | 0.77 | 0.73 | 0.82 | 0.89 | 0.84 | 0.94 | 0.73 | 0.68 | 0.78 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 296 | 0.75 | 0.68 | 0.82 | 0.79 | 0.65 | 0.93 | 0.74 | 0.66 | 0.82 | 0.79 | 0.73 | 0.84 | 0.85 | 0.77 | 0.94 | 0.76 | 0.70 | 0.83 | 0.79 | 0.75 | 0.83 | 0.84 | 0.77 | 0.91 | 0.77 | 0.72 | 0.81 |
| 297 | 0.76 | 0.68 | 0.84 | 0.88 | 0.72 | 1.00 | 0.72 | 0.64 | 0.81 | 0.78 | 0.72 | 0.84 | 0.89 | 0.83 | 0.94 | 0.74 | 0.67 | 0.80 | 0.79 | 0.75 | 0.84 | 0.91 | 0.85 | 0.96 | 0.75 | 0.70 | 0.80 |
| 298 | 0.74 | 0.67 | 0.82 | 0.87 | 0.73 | 1.00 | 0.71 | 0.62 | 0.79 | 0.78 | 0.72 | 0.84 | 0.88 | 0.80 | 0.96 | 0.74 | 0.67 | 0.81 | 0.78 | 0.74 | 0.83 | 0.89 | 0.82 | 0.95 | 0.75 | 0.69 | 0.80 |
| 299 | 0.77 | 0.68 | 0.85 | 0.88 | 0.78 | 0.98 | 0.73 | 0.63 | 0.83 | 0.77 | 0.71 | 0.83 | 0.83 | 0.71 | 0.94 | 0.75 | 0.68 | 0.81 | 0.79 | 0.74 | 0.83 | 0.86 | 0.78 | 0.94 | 0.76 | 0.71 | 0.81 |
| 300 | 0.74 | 0.66 | 0.82 | 0.87 | 0.72 | 1.00 | 0.70 | 0.61 | 0.79 | 0.78 | 0.72 | 0.84 | 0.88 | 0.79 | 0.96 | 0.74 | 0.68 | 0.81 | 0.77 | 0.73 | 0.82 | 0.88 | 0.81 | 0.95 | 0.73 | 0.68 | 0.79 |
| 301 | 0.75 | 0.67 | 0.84 | 0.90 | 0.75 | 1.00 | 0.71 | 0.61 | 0.81 | 0.78 | 0.73 | 0.84 | 0.90 | 0.84 | 0.95 | 0.74 | 0.67 | 0.81 | 0.79 | 0.74 | 0.83 | 0.91 | 0.85 | 0.96 | 0.74 | 0.69 | 0.79 |
| 302 | 0.76 | 0.68 | 0.84 | 0.87 | 0.74 | 1.00 | 0.72 | 0.63 | 0.82 | 0.78 | 0.73 | 0.84 | 0.90 | 0.83 | 0.97 | 0.74 | 0.67 | 0.81 | 0.79 | 0.75 | 0.84 | 0.90 | 0.85 | 0.96 | 0.75 | 0.70 | 0.81 |
| 303 | 0.77 | 0.69 | 0.85 | 0.89 | 0.73 | 1.00 | 0.74 | 0.64 | 0.83 | 0.78 | 0.73 | 0.84 | 0.88 | 0.83 | 0.94 | 0.74 | 0.67 | 0.81 | 0.79 | 0.76 | 0.84 | 0.91 | 0.86 | 0.96 | 0.76 | 0.71 | 0.81 |
| 304 | 0.77 | 0.69 | 0.86 | 0.84 | 0.71 | 0.97 | 0.75 | 0.65 | 0.85 | 0.79 | 0.74 | 0.85 | 0.90 | 0.84 | 0.95 | 0.75 | 0.68 | 0.82 | 0.80 | 0.76 | 0.85 | 0.89 | 0.83 | 0.94 | 0.77 | 0.71 | 0.82 |
| 305 | 0.75 | 0.67 | 0.84 | 0.77 | 0.60 | 0.95 | 0.75 | 0.65 | 0.84 | 0.79 | 0.74 | 0.85 | 0.85 | 0.77 | 0.93 | 0.77 | 0.71 | 0.83 | 0.79 | 0.75 | 0.83 | 0.83 | 0.76 | 0.91 | 0.77 | 0.72 | 0.82 |
| 306 | 0.76 | 0.68 | 0.84 | 0.80 | 0.64 | 0.96 | 0.74 | 0.65 | 0.83 | 0.79 | 0.74 | 0.85 | 0.90 | 0.84 | 0.96 | 0.75 | 0.68 | 0.82 | 0.79 | 0.74 | 0.83 | 0.87 | 0.81 | 0.94 | 0.76 | 0.70 | 0.81 |
| 307 | 0.73 | 0.66 | 0.81 | 0.79 | 0.66 | 0.92 | 0.72 | 0.63 | 0.80 | 0.78 | 0.73 | 0.84 | 0.85 | 0.78 | 0.93 | 0.76 | 0.70 | 0.83 | 0.78 | 0.74 | 0.83 | 0.84 | 0.78 | 0.91 | 0.76 | 0.71 | 0.81 |
| 308 | 0.75 | 0.67 | 0.83 | 0.87 | 0.71 | 1.00 | 0.71 | 0.62 | 0.80 | 0.78 | 0.73 | 0.84 | 0.89 | 0.82 | 0.96 | 0.74 | 0.67 | 0.80 | 0.78 | 0.74 | 0.83 | 0.89 | 0.83 | 0.95 | 0.74 | 0.69 | 0.79 |
| 309 | 0.77 | 0.69 | 0.85 | 0.89 | 0.72 | 1.00 | 0.73 | 0.64 | 0.82 | 0.79 | 0.74 | 0.85 | 0.89 | 0.84 | 0.95 | 0.76 | 0.69 | 0.83 | 0.80 | 0.76 | 0.85 | 0.91 | 0.86 | 0.97 | 0.77 | 0.72 | 0.82 |
| 310 | 0.75 | 0.68 | 0.82 | 0.83 | 0.71 | 0.95 | 0.73 | 0.64 | 0.81 | 0.79 | 0.73 | 0.84 | 0.87 | 0.79 | 0.95 | 0.76 | 0.69 | 0.82 | 0.78 | 0.74 | 0.83 | 0.86 | 0.80 | 0.93 | 0.76 | 0.71 | 0.81 |
| 311 | 0.73 | 0.66 | 0.81 | 0.80 | 0.68 | 0.92 | 0.71 | 0.62 | 0.79 | 0.79 | 0.73 | 0.84 | 0.87 | 0.79 | 0.95 | 0.76 | 0.69 | 0.83 | 0.78 | 0.73 | 0.82 | 0.85 | 0.78 | 0.91 | 0.75 | 0.70 | 0.80 |
| 312 | 0.75 | 0.67 | 0.84 | 0.91 | 0.77 | 1.00 | 0.71 | 0.61 | 0.81 | 0.77 | 0.71 | 0.83 | 0.86 | 0.77 | 0.95 | 0.73 | 0.66 | 0.80 | 0.78 | 0.73 | 0.83 | 0.89 | 0.82 | 0.96 | 0.74 | 0.69 | 0.80 |
| 313 | 0.76 | 0.68 | 0.84 | 0.90 | 0.77 | 1.00 | 0.72 | 0.63 | 0.81 | 0.79 | 0.73 | 0.84 | 0.91 | 0.86 | 0.95 | 0.73 | 0.66 | 0.80 | 0.79 | 0.75 | 0.84 | 0.92 | 0.88 | 0.97 | 0.74 | 0.69 | 0.80 |
| 314 | 0.77 | 0.68 | 0.85 | 0.90 | 0.74 | 1.00 | 0.73 | 0.63 | 0.82 | 0.78 | 0.73 | 0.84 | 0.89 | 0.83 | 0.94 | 0.74 | 0.67 | 0.81 | 0.80 | 0.76 | 0.84 | 0.91 | 0.86 | 0.96 | 0.76 | 0.70 | 0.81 |
| 315 | 0.75 | 0.67 | 0.84 | 0.88 | 0.71 | 1.00 | 0.71 | 0.62 | 0.80 | 0.79 | 0.73 | 0.84 | 0.90 | 0.85 | 0.96 | 0.74 | 0.67 | 0.81 | 0.79 | 0.74 | 0.84 | 0.91 | 0.86 | 0.97 | 0.75 | 0.69 | 0.80 |
| 316 | 0.73 | 0.66 | 0.81 | 0.81 | 0.69 | 0.93 | 0.70 | 0.62 | 0.79 | 0.77 | 0.73 | 0.84 | 0.87 | 0.79 | 0.94 | 0.76 | 0.69 | 0.83 | 0.78 | 0.73 | 0.82 | 0.85 | 0.79 | 0.91 | 0.75 | 0.70 | 0.80 |
| 317 | 0.74 | 0.66 | 0.81 | 0.83 | 0.71 | 0.95 | 0.71 | 0.62 | 0.79 | 0.79 | 0.73 | 0.84 | 0.87 | 0.79 | 0.95 | 0.76 | 0.69 | 0.82 | 0.78 | 0.74 | 0.83 | 0.86 | 0.79 | 0.93 | 0.76 | 0.70 | 0.81 |
| 318 | 0.75 | 0.68 | 0.82 | 0.84 | 0.71 | 0.96 | 0.73 | 0.65 | 0.80 | 0.79 | 0.73 | 0.84 | 0.85 | 0.76 | 0.94 | 0.76 | 0.70 | 0.83 | 0.79 | 0.75 | 0.83 | 0.86 | 0.79 | 0.92 | 0.76 | 0.70 | 0.81 |
| 319 | 0.76 | 0.68 | 0.83 | 0.86 | 0.71 | 1.00 | 0.72 | 0.64 | 0.81 | 0.79 | 0.74 | 0.85 | 0.87 | 0.80 | 0.94 | 0.76 | 0.70 | 0.83 | 0.78 | 0.74 | 0.83 | 0.87 | 0.81 | 0.94 | 0.75 | 0.72 | 0.80 |
| 320 | 0.75 | 0.67 | 0.83 | 0.85 | 0.71 | 0.99 | 0.72 | 0.62 | 0.81 | 0.78 | 0.73 | 0.84 | 0.90 | 0.85 | 0.95 | 0.73 | 0.66 | 0.80 | 0.79 | 0.74 | 0.83 | 0.90 | 0.85 | 0.95 | 0.76 | 0.70 | 0.80 |
| 321 | 0.78 | 0.69 | 0.86 | 0.86 | 0.74 | 0.97 | 0.75 | 0.65 | 0.85 | 0.79 | 0.72 | 0.84 | 0.86 | 0.78 | 0.94 | 0.74 | 0.67 | 0.81 | 0.79 | 0.74 | 0.84 | 0.87 | 0.81 | 0.93 | 0.75 | 0.69 | 0.82 |
| 322 | 0.74 | 0.67 | 0.81 | 0.80 | 0.70 | 0.90 | 0.72 | 0.63 | 0.81 | 0.79 | 0.73 | 0.84 | 0.85 | 0.78 | 0.92 | 0.76 | 0.69 | 0.83 | 0.78 | 0.74 | 0.82 | 0.84 | 0.79 | 0.90 | 0.76 | 0.70 | 0.81 |
| 323 | 0.77 | 0.69 | 0.85 | 0.89 | 0.79 | 0.99 | 0.73 | 0.63 | 0.83 | 0.77 | 0.71 | 0.83 | 0.83 | 0.72 | 0.95 | 0.75 | 0.68 | 0.81 | 0.78 | 0.74 | 0.83 | 0.86 | 0.78 | 0.94 | 0.75 | 0.70 | 0.81 |
| 324 | 0.74 | 0.66 | 0.82 | 0.75 | 0.57 | 0.93 | 0.74 | 0.65 | 0.83 | 0.79 | 0.74 | 0.85 | 0.88 | 0.77 | 0.93 | 0.77 | 0.71 | 0.83 | 0.79 | 0.75 | 0.83 | 0.83 | 0.75 | 0.90 | 0.77 | 0.72 | 0.82 |
| 325 | 0.74 | 0.66 | 0.83 | 0.81 | 0.63 | 0.98 | 0.72 | 0.63 | 0.81 | 0.80 | 0.74 | 0.85 | 0.86 | 0.81 | 0.95 | 0.77 | 0.70 | 0.82 | 0.78 | 0.74 | 0.83 | 0.86 | 0.79 | 0.94 | 0.76 | 0.70 | 0.81 |
| 326 | 0.74 | 0.66 | 0.82 | 0.78 | 0.63 | 0.93 | 0.73 | 0.63 | 0.82 | 0.79 | 0.73 | 0.84 | 0.89 | 0.78 | 0.95 | 0.76 | 0.69 | 0.81 | 0.78 | 0.74 | 0.83 | 0.85 | 0.78 | 0.92 | 0.76 | 0.71 | 0.81 |
| 327 | 0.76 | 0.68 | 0.84 | 0.90 | 0.77 | 1.00 | 0.72 | 0.63 | 0.82 | 0.78 | 0.72 | 0.84 | 0.89 | 0.84 | 0.94 | 0.74 | 0.67 | 0.81 | 0.79 | 0.74 | 0.84 | 0.91 | 0.86 | 0.95 | 0.75 | 0.69 | 0.80 |
| 328 | 0.76 | 0.69 | 0.83 | 0.86 | 0.74 | 0.98 | 0.73 | 0.65 | 0.80 | 0.78 | 0.73 | 0.84 | 0.86 | 0.77 | 0.94 | 0.75 | 0.69 | 0.82 | 0.78 | 0.74 | 0.82 | 0.86 | 0.80 | 0.93 | 0.75 | 0.70 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 329 | 0.77 | 0.69 | 0.85 | 0.89 | 0.78 | 0.99 | 0.73 | 0.64 | 0.83 | 0.77 | 0.71 | 0.83 | 0.83 | 0.72 | 0.95 | 0.74 | 0.68 | 0.81 | 0.79 | 0.74 | 0.83 | 0.86 | 0.78 | 0.94 | 0.76 | 0.71 | 0.81 |
| 330 | 0.75 | 0.68 | 0.82 | 0.81 | 0.72 | 0.91 | 0.73 | 0.65 | 0.81 | 0.79 | 0.73 | 0.84 | 0.88 | 0.81 | 0.95 | 0.75 | 0.68 | 0.81 | 0.78 | 0.74 | 0.83 | 0.86 | 0.81 | 0.92 | 0.75 | 0.71 | 0.80 |
| 331 | 0.75 | 0.68 | 0.82 | 0.83 | 0.70 | 0.95 | 0.72 | 0.64 | 0.81 | 0.78 | 0.72 | 0.84 | 0.84 | 0.76 | 0.92 | 0.75 | 0.68 | 0.82 | 0.77 | 0.73 | 0.81 | 0.84 | 0.77 | 0.90 | 0.74 | 0.69 | 0.80 |
| 332 | 0.76 | 0.68 | 0.84 | 0.83 | 0.69 | 0.97 | 0.73 | 0.64 | 0.83 | 0.79 | 0.73 | 0.84 | 0.91 | 0.86 | 0.96 | 0.73 | 0.67 | 0.80 | 0.79 | 0.75 | 0.84 | 0.89 | 0.84 | 0.95 | 0.75 | 0.70 | 0.80 |
| 333 | 0.74 | 0.66 | 0.81 | 0.87 | 0.78 | 0.96 | 0.69 | 0.60 | 0.78 | 0.77 | 0.71 | 0.83 | 0.82 | 0.70 | 0.93 | 0.75 | 0.69 | 0.82 | 0.76 | 0.72 | 0.81 | 0.84 | 0.76 | 0.92 | 0.74 | 0.68 | 0.79 |
| 334 | 0.74 | 0.66 | 0.81 | 0.82 | 0.70 | 0.93 | 0.71 | 0.63 | 0.80 | 0.79 | 0.73 | 0.84 | 0.85 | 0.76 | 0.93 | 0.76 | 0.70 | 0.82 | 0.78 | 0.74 | 0.82 | 0.85 | 0.78 | 0.91 | 0.75 | 0.70 | 0.81 |
| 335 | 0.75 | 0.67 | 0.83 | 0.88 | 0.78 | 0.97 | 0.71 | 0.62 | 0.80 | 0.77 | 0.71 | 0.83 | 0.81 | 0.68 | 0.93 | 0.76 | 0.69 | 0.83 | 0.77 | 0.73 | 0.82 | 0.84 | 0.76 | 0.92 | 0.75 | 0.70 | 0.80 |
| 336 | 0.78 | 0.69 | 0.86 | 0.88 | 0.72 | 1.00 | 0.75 | 0.65 | 0.84 | 0.78 | 0.72 | 0.84 | 0.90 | 0.85 | 0.95 | 0.73 | 0.66 | 0.82 | 0.80 | 0.75 | 0.84 | 0.91 | 0.86 | 0.96 | 0.76 | 0.70 | 0.81 |
| 337 | 0.76 | 0.69 | 0.83 | 0.84 | 0.71 | 0.97 | 0.74 | 0.66 | 0.82 | 0.79 | 0.73 | 0.84 | 0.86 | 0.78 | 0.94 | 0.76 | 0.69 | 0.80 | 0.79 | 0.75 | 0.84 | 0.87 | 0.81 | 0.93 | 0.77 | 0.72 | 0.81 |
| 338 | 0.75 | 0.67 | 0.83 | 0.84 | 0.66 | 1.00 | 0.73 | 0.64 | 0.81 | 0.78 | 0.73 | 0.84 | 0.90 | 0.83 | 0.97 | 0.74 | 0.67 | 0.82 | 0.79 | 0.74 | 0.83 | 0.89 | 0.83 | 0.96 | 0.75 | 0.70 | 0.80 |
| 339 | 0.77 | 0.69 | 0.86 | 0.87 | 0.70 | 1.00 | 0.75 | 0.65 | 0.84 | 0.79 | 0.74 | 0.85 | 0.89 | 0.83 | 0.94 | 0.75 | 0.69 | 0.81 | 0.81 | 0.76 | 0.85 | 0.90 | 0.85 | 0.95 | 0.77 | 0.72 | 0.82 |
| 340 | 0.74 | 0.67 | 0.82 | 0.79 | 0.64 | 0.94 | 0.73 | 0.65 | 0.81 | 0.79 | 0.73 | 0.84 | 0.86 | 0.77 | 0.95 | 0.76 | 0.69 | 0.82 | 0.79 | 0.74 | 0.83 | 0.85 | 0.77 | 0.92 | 0.76 | 0.72 | 0.81 |
| 341 | 0.75 | 0.67 | 0.83 | 0.83 | 0.70 | 0.97 | 0.73 | 0.63 | 0.82 | 0.78 | 0.73 | 0.84 | 0.87 | 0.79 | 0.95 | 0.75 | 0.68 | 0.82 | 0.79 | 0.74 | 0.83 | 0.87 | 0.80 | 0.94 | 0.76 | 0.71 | 0.81 |
| 342 | 0.74 | 0.67 | 0.82 | 0.89 | 0.74 | 1.00 | 0.70 | 0.62 | 0.79 | 0.78 | 0.72 | 0.84 | 0.88 | 0.80 | 0.95 | 0.74 | 0.68 | 0.82 | 0.78 | 0.74 | 0.83 | 0.89 | 0.83 | 0.95 | 0.74 | 0.69 | 0.79 |
| 343 | 0.77 | 0.68 | 0.85 | 0.90 | 0.81 | 0.98 | 0.72 | 0.62 | 0.83 | 0.77 | 0.71 | 0.83 | 0.82 | 0.71 | 0.94 | 0.75 | 0.68 | 0.81 | 0.78 | 0.73 | 0.82 | 0.85 | 0.77 | 0.93 | 0.75 | 0.69 | 0.80 |
| 344 | 0.74 | 0.66 | 0.83 | 0.82 | 0.69 | 0.95 | 0.72 | 0.62 | 0.83 | 0.79 | 0.73 | 0.85 | 0.87 | 0.80 | 0.95 | 0.76 | 0.68 | 0.81 | 0.78 | 0.74 | 0.83 | 0.86 | 0.80 | 0.93 | 0.75 | 0.70 | 0.81 |
| 345 | 0.75 | 0.67 | 0.82 | 0.90 | 0.75 | 1.00 | 0.72 | 0.62 | 0.79 | 0.78 | 0.73 | 0.84 | 0.91 | 0.85 | 0.96 | 0.74 | 0.67 | 0.83 | 0.79 | 0.74 | 0.83 | 0.92 | 0.87 | 0.97 | 0.74 | 0.69 | 0.80 |
| 346 | 0.74 | 0.67 | 0.80 | 0.79 | 0.66 | 0.92 | 0.70 | 0.61 | 0.80 | 0.79 | 0.73 | 0.84 | 0.86 | 0.78 | 0.94 | 0.75 | 0.69 | 0.81 | 0.78 | 0.74 | 0.82 | 0.84 | 0.78 | 0.91 | 0.75 | 0.70 | 0.80 |
| 347 | 0.73 | 0.66 | 0.83 | 0.82 | 0.70 | 0.93 | 0.74 | 0.64 | 0.83 | 0.79 | 0.73 | 0.85 | 0.89 | 0.82 | 0.96 | 0.75 | 0.68 | 0.82 | 0.79 | 0.74 | 0.83 | 0.87 | 0.81 | 0.93 | 0.75 | 0.70 | 0.81 |
| 348 | 0.75 | 0.66 | 0.81 | 0.87 | 0.76 | 0.98 | 0.72 | 0.62 | 0.79 | 0.78 | 0.72 | 0.83 | 0.89 | 0.83 | 0.95 | 0.73 | 0.69 | 0.82 | 0.77 | 0.73 | 0.82 | 0.89 | 0.84 | 0.94 | 0.73 | 0.67 | 0.78 |
| 349 | 0.74 | 0.69 | 0.85 | 0.88 | 0.72 | 1.00 | 0.75 | 0.61 | 0.83 | 0.78 | 0.72 | 0.84 | 0.91 | 0.87 | 0.96 | 0.72 | 0.69 | 0.82 | 0.79 | 0.74 | 0.83 | 0.91 | 0.86 | 0.97 | 0.74 | 0.69 | 0.80 |
| 350 | 0.77 | 0.67 | 0.81 | 0.82 | 0.72 | 0.92 | 0.71 | 0.64 | 0.80 | 0.78 | 0.73 | 0.84 | 0.88 | 0.81 | 0.94 | 0.75 | 0.66 | 0.80 | 0.78 | 0.74 | 0.82 | 0.86 | 0.81 | 0.92 | 0.75 | 0.70 | 0.80 |
| 351 | 0.74 | 0.70 | 0.84 | 0.84 | 0.70 | 0.98 | 0.73 | 0.64 | 0.83 | 0.79 | 0.73 | 0.84 | 0.87 | 0.80 | 0.94 | 0.75 | 0.65 | 0.80 | 0.79 | 0.75 | 0.83 | 0.87 | 0.81 | 0.93 | 0.76 | 0.71 | 0.81 |
| 352 | 0.77 | 0.66 | 0.83 | 0.86 | 0.70 | 1.00 | 0.72 | 0.66 | 0.80 | 0.78 | 0.72 | 0.83 | 0.89 | 0.83 | 0.95 | 0.73 | 0.68 | 0.81 | 0.78 | 0.74 | 0.82 | 0.89 | 0.84 | 0.95 | 0.74 | 0.68 | 0.79 |
| 353 | 0.75 | 0.67 | 0.81 | 0.79 | 0.66 | 0.92 | 0.71 | 0.62 | 0.81 | 0.78 | 0.73 | 0.84 | 0.86 | 0.78 | 0.94 | 0.76 | 0.69 | 0.82 | 0.78 | 0.74 | 0.82 | 0.84 | 0.78 | 0.91 | 0.76 | 0.70 | 0.81 |
| 354 | 0.74 | 0.68 | 0.82 | 0.82 | 0.73 | 0.91 | 0.72 | 0.64 | 0.81 | 0.79 | 0.73 | 0.84 | 0.88 | 0.80 | 0.95 | 0.75 | 0.68 | 0.80 | 0.78 | 0.74 | 0.82 | 0.87 | 0.81 | 0.92 | 0.75 | 0.70 | 0.80 |
| 355 | 0.75 | 0.67 | 0.81 | 0.82 | 0.71 | 0.93 | 0.72 | 0.64 | 0.80 | 0.78 | 0.73 | 0.84 | 0.86 | 0.78 | 0.94 | 0.76 | 0.69 | 0.82 | 0.78 | 0.73 | 0.82 | 0.86 | 0.79 | 0.92 | 0.75 | 0.70 | 0.80 |
| 356 | 0.74 | 0.66 | 0.83 | 0.81 | 0.66 | 0.95 | 0.70 | 0.63 | 0.82 | 0.79 | 0.73 | 0.85 | 0.86 | 0.79 | 0.94 | 0.75 | 0.68 | 0.81 | 0.78 | 0.74 | 0.82 | 0.85 | 0.79 | 0.92 | 0.76 | 0.70 | 0.81 |
| 357 | 0.74 | 0.68 | 0.83 | 0.86 | 0.77 | 0.95 | 0.75 | 0.62 | 0.81 | 0.78 | 0.73 | 0.84 | 0.86 | 0.79 | 0.94 | 0.76 | 0.69 | 0.82 | 0.77 | 0.73 | 0.83 | 0.86 | 0.81 | 0.92 | 0.74 | 0.69 | 0.80 |
| 358 | 0.76 | 0.66 | 0.83 | 0.88 | 0.71 | 1.00 | 0.72 | 0.63 | 0.80 | 0.79 | 0.74 | 0.85 | 0.91 | 0.87 | 0.96 | 0.76 | 0.69 | 0.83 | 0.80 | 0.75 | 0.82 | 0.90 | 0.85 | 0.96 | 0.72 | 0.67 | 0.78 |
| 359 | 0.74 | 0.68 | 0.84 | 0.81 | 0.64 | 0.97 | 0.71 | 0.61 | 0.84 | 0.79 | 0.73 | 0.84 | 0.87 | 0.80 | 0.94 | 0.75 | 0.69 | 0.82 | 0.78 | 0.74 | 0.84 | 0.87 | 0.80 | 0.93 | 0.77 | 0.72 | 0.82 |
| 360 | 0.74 | 0.66 | 0.82 | 0.80 | 0.67 | 0.93 | 0.72 | 0.65 | 0.81 | 0.79 | 0.73 | 0.84 | 0.87 | 0.79 | 0.95 | 0.73 | 0.69 | 0.80 | 0.78 | 0.74 | 0.83 | 0.85 | 0.78 | 0.92 | 0.75 | 0.70 | 0.81 |
| 361 | 0.75 | 0.66 | 0.84 | 0.89 | 0.72 | 1.00 | 0.71 | 0.61 | 0.81 | 0.79 | 0.73 | 0.84 | 0.87 | 0.80 | 0.94 | 0.75 | 0.69 | 0.82 | 0.80 | 0.75 | 0.84 | 0.90 | 0.84 | 0.96 | 0.76 | 0.70 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 362 | 0.74 | 0.66 | 0.82 | 0.82 | 0.66 | 0.98 | 0.72 | 0.62 | 0.81 | 0.79 | 0.73 | 0.85 | 0.87 | 0.80 | 0.95 | 0.76 | 0.69 | 0.83 | 0.79 | 0.74 | 0.83 | 0.87 | 0.79 | 0.94 | 0.76 | 0.70 | 0.81 |
| 363 | 0.76 | 0.67 | 0.84 | 0.85 | 0.76 | 0.94 | 0.73 | 0.63 | 0.82 | 0.78 | 0.73 | 0.84 | 0.88 | 0.81 | 0.95 | 0.74 | 0.67 | 0.82 | 0.78 | 0.73 | 0.83 | 0.87 | 0.82 | 0.93 | 0.74 | 0.69 | 0.80 |
| 364 | 0.75 | 0.68 | 0.82 | 0.82 | 0.69 | 0.94 | 0.73 | 0.65 | 0.81 | 0.78 | 0.73 | 0.84 | 0.85 | 0.76 | 0.94 | 0.76 | 0.69 | 0.82 | 0.78 | 0.74 | 0.82 | 0.85 | 0.78 | 0.92 | 0.76 | 0.71 | 0.81 |
| 365 | 0.74 | 0.66 | 0.83 | 0.87 | 0.77 | 0.98 | 0.70 | 0.60 | 0.80 | 0.78 | 0.72 | 0.84 | 0.83 | 0.71 | 0.94 | 0.76 | 0.69 | 0.83 | 0.78 | 0.73 | 0.83 | 0.85 | 0.77 | 0.93 | 0.75 | 0.70 | 0.81 |
| 366 | 0.74 | 0.66 | 0.83 | 0.86 | 0.69 | 1.00 | 0.71 | 0.61 | 0.80 | 0.78 | 0.73 | 0.84 | 0.88 | 0.82 | 0.95 | 0.74 | 0.68 | 0.81 | 0.79 | 0.74 | 0.83 | 0.89 | 0.83 | 0.95 | 0.75 | 0.69 | 0.80 |
| 367 | 0.76 | 0.69 | 0.83 | 0.81 | 0.71 | 0.92 | 0.74 | 0.66 | 0.82 | 0.79 | 0.73 | 0.84 | 0.87 | 0.78 | 0.95 | 0.75 | 0.69 | 0.82 | 0.78 | 0.74 | 0.83 | 0.85 | 0.79 | 0.92 | 0.76 | 0.71 | 0.81 |
| 368 | 0.76 | 0.68 | 0.84 | 0.83 | 0.69 | 0.96 | 0.74 | 0.65 | 0.83 | 0.78 | 0.73 | 0.84 | 0.91 | 0.86 | 0.96 | 0.73 | 0.66 | 0.80 | 0.79 | 0.75 | 0.83 | 0.89 | 0.84 | 0.95 | 0.75 | 0.70 | 0.80 |
| 369 | 0.77 | 0.68 | 0.86 | 0.90 | 0.76 | 1.00 | 0.73 | 0.63 | 0.84 | 0.78 | 0.72 | 0.84 | 0.90 | 0.85 | 0.94 | 0.73 | 0.66 | 0.80 | 0.80 | 0.75 | 0.84 | 0.91 | 0.86 | 0.96 | 0.75 | 0.70 | 0.81 |
| 370 | 0.74 | 0.67 | 0.81 | 0.83 | 0.72 | 0.93 | 0.71 | 0.63 | 0.80 | 0.78 | 0.73 | 0.84 | 0.86 | 0.77 | 0.95 | 0.75 | 0.69 | 0.82 | 0.77 | 0.73 | 0.82 | 0.85 | 0.79 | 0.92 | 0.74 | 0.70 | 0.79 |
| 371 | 0.74 | 0.65 | 0.83 | 0.82 | 0.65 | 0.98 | 0.72 | 0.62 | 0.82 | 0.79 | 0.73 | 0.84 | 0.89 | 0.83 | 0.94 | 0.75 | 0.68 | 0.81 | 0.79 | 0.74 | 0.83 | 0.88 | 0.82 | 0.94 | 0.75 | 0.70 | 0.81 |
| 372 | 0.74 | 0.66 | 0.82 | 0.73 | 0.55 | 0.92 | 0.74 | 0.65 | 0.83 | 0.80 | 0.74 | 0.85 | 0.85 | 0.76 | 0.93 | 0.77 | 0.71 | 0.84 | 0.79 | 0.74 | 0.83 | 0.82 | 0.74 | 0.90 | 0.77 | 0.72 | 0.82 |
| 373 | 0.75 | 0.68 | 0.82 | 0.83 | 0.70 | 0.97 | 0.73 | 0.64 | 0.81 | 0.78 | 0.73 | 0.84 | 0.86 | 0.78 | 0.94 | 0.75 | 0.69 | 0.82 | 0.79 | 0.75 | 0.83 | 0.86 | 0.80 | 0.93 | 0.76 | 0.71 | 0.81 |
| 374 | 0.77 | 0.68 | 0.85 | 0.81 | 0.64 | 0.98 | 0.75 | 0.66 | 0.85 | 0.79 | 0.73 | 0.84 | 0.87 | 0.80 | 0.94 | 0.75 | 0.69 | 0.82 | 0.80 | 0.76 | 0.84 | 0.87 | 0.80 | 0.93 | 0.77 | 0.72 | 0.83 |
| 375 | 0.74 | 0.66 | 0.83 | 0.90 | 0.76 | 1.00 | 0.70 | 0.60 | 0.80 | 0.78 | 0.72 | 0.83 | 0.88 | 0.81 | 0.95 | 0.73 | 0.67 | 0.80 | 0.79 | 0.74 | 0.83 | 0.90 | 0.85 | 0.96 | 0.74 | 0.69 | 0.79 |
| 376 | 0.74 | 0.66 | 0.82 | 0.79 | 0.64 | 0.94 | 0.72 | 0.63 | 0.81 | 0.79 | 0.73 | 0.84 | 0.86 | 0.79 | 0.94 | 0.75 | 0.69 | 0.82 | 0.78 | 0.74 | 0.83 | 0.85 | 0.78 | 0.92 | 0.75 | 0.70 | 0.81 |
| 377 | 0.76 | 0.69 | 0.82 | 0.83 | 0.75 | 0.92 | 0.73 | 0.65 | 0.81 | 0.78 | 0.73 | 0.84 | 0.88 | 0.81 | 0.94 | 0.74 | 0.68 | 0.81 | 0.79 | 0.74 | 0.83 | 0.87 | 0.82 | 0.92 | 0.75 | 0.70 | 0.80 |
| 378 | 0.74 | 0.67 | 0.81 | 0.83 | 0.72 | 0.94 | 0.71 | 0.63 | 0.80 | 0.78 | 0.73 | 0.84 | 0.86 | 0.78 | 0.94 | 0.76 | 0.69 | 0.82 | 0.78 | 0.73 | 0.82 | 0.85 | 0.79 | 0.92 | 0.75 | 0.70 | 0.80 |
| 379 | 0.74 | 0.66 | 0.81 | 0.84 | 0.73 | 0.96 | 0.70 | 0.61 | 0.79 | 0.78 | 0.72 | 0.84 | 0.87 | 0.79 | 0.95 | 0.75 | 0.68 | 0.82 | 0.77 | 0.72 | 0.81 | 0.86 | 0.80 | 0.93 | 0.73 | 0.68 | 0.79 |
| 380 | 0.76 | 0.69 | 0.83 | 0.86 | 0.74 | 0.98 | 0.73 | 0.64 | 0.81 | 0.79 | 0.73 | 0.84 | 0.87 | 0.80 | 0.95 | 0.75 | 0.68 | 0.82 | 0.78 | 0.74 | 0.82 | 0.87 | 0.81 | 0.93 | 0.75 | 0.70 | 0.80 |
| 381 | 0.76 | 0.68 | 0.84 | 0.79 | 0.63 | 0.95 | 0.75 | 0.66 | 0.84 | 0.80 | 0.74 | 0.85 | 0.85 | 0.77 | 0.93 | 0.77 | 0.71 | 0.83 | 0.80 | 0.76 | 0.84 | 0.85 | 0.78 | 0.92 | 0.78 | 0.73 | 0.83 |
| 382 | 0.74 | 0.66 | 0.82 | 0.77 | 0.62 | 0.93 | 0.73 | 0.64 | 0.82 | 0.79 | 0.73 | 0.84 | 0.86 | 0.79 | 0.94 | 0.75 | 0.69 | 0.82 | 0.78 | 0.74 | 0.83 | 0.84 | 0.77 | 0.92 | 0.76 | 0.71 | 0.81 |
| 383 | 0.76 | 0.69 | 0.83 | 0.86 | 0.76 | 0.97 | 0.72 | 0.64 | 0.81 | 0.78 | 0.72 | 0.84 | 0.84 | 0.73 | 0.94 | 0.76 | 0.70 | 0.83 | 0.78 | 0.73 | 0.82 | 0.85 | 0.77 | 0.92 | 0.75 | 0.70 | 0.80 |
| 384 | 0.75 | 0.67 | 0.82 | 0.84 | 0.75 | 0.94 | 0.72 | 0.63 | 0.80 | 0.77 | 0.71 | 0.83 | 0.82 | 0.72 | 0.91 | 0.76 | 0.69 | 0.83 | 0.78 | 0.73 | 0.82 | 0.84 | 0.77 | 0.90 | 0.76 | 0.70 | 0.81 |
| 385 | 0.75 | 0.66 | 0.83 | 0.87 | 0.71 | 1.00 | 0.71 | 0.62 | 0.80 | 0.78 | 0.73 | 0.84 | 0.89 | 0.83 | 0.96 | 0.74 | 0.67 | 0.81 | 0.79 | 0.75 | 0.83 | 0.90 | 0.85 | 0.96 | 0.75 | 0.70 | 0.80 |
| 386 | 0.74 | 0.66 | 0.83 | 0.89 | 0.73 | 1.00 | 0.70 | 0.61 | 0.80 | 0.78 | 0.72 | 0.84 | 0.89 | 0.83 | 0.95 | 0.74 | 0.67 | 0.81 | 0.79 | 0.74 | 0.83 | 0.91 | 0.85 | 0.97 | 0.74 | 0.69 | 0.80 |
| 387 | 0.74 | 0.66 | 0.82 | 0.84 | 0.74 | 0.94 | 0.71 | 0.61 | 0.80 | 0.77 | 0.72 | 0.83 | 0.82 | 0.73 | 0.91 | 0.76 | 0.69 | 0.83 | 0.77 | 0.73 | 0.82 | 0.84 | 0.77 | 0.90 | 0.75 | 0.70 | 0.80 |
| 388 | 0.76 | 0.69 | 0.82 | 0.86 | 0.76 | 0.96 | 0.72 | 0.64 | 0.80 | 0.78 | 0.73 | 0.84 | 0.84 | 0.75 | 0.94 | 0.76 | 0.70 | 0.82 | 0.77 | 0.73 | 0.82 | 0.85 | 0.78 | 0.92 | 0.75 | 0.70 | 0.79 |
| 389 | 0.74 | 0.67 | 0.82 | 0.83 | 0.72 | 0.94 | 0.71 | 0.62 | 0.80 | 0.77 | 0.71 | 0.83 | 0.81 | 0.71 | 0.91 | 0.75 | 0.68 | 0.83 | 0.77 | 0.73 | 0.82 | 0.83 | 0.76 | 0.90 | 0.75 | 0.70 | 0.81 |
| 390 | 0.76 | 0.68 | 0.85 | 0.82 | 0.67 | 0.98 | 0.74 | 0.65 | 0.84 | 0.79 | 0.73 | 0.85 | 0.89 | 0.83 | 0.95 | 0.75 | 0.68 | 0.82 | 0.78 | 0.74 | 0.83 | 0.87 | 0.81 | 0.93 | 0.75 | 0.69 | 0.81 |
| 391 | 0.77 | 0.69 | 0.85 | 0.90 | 0.80 | 0.99 | 0.73 | 0.63 | 0.82 | 0.76 | 0.70 | 0.82 | 0.83 | 0.72 | 0.94 | 0.74 | 0.67 | 0.81 | 0.78 | 0.73 | 0.82 | 0.86 | 0.78 | 0.94 | 0.75 | 0.70 | 0.80 |
| 392 | 0.75 | 0.67 | 0.82 | 0.88 | 0.79 | 0.97 | 0.70 | 0.61 | 0.79 | 0.77 | 0.71 | 0.83 | 0.81 | 0.69 | 0.94 | 0.75 | 0.69 | 0.82 | 0.77 | 0.72 | 0.81 | 0.84 | 0.76 | 0.92 | 0.74 | 0.69 | 0.79 |
| 393 | 0.74 | 0.66 | 0.82 | 0.81 | 0.64 | 0.98 | 0.72 | 0.63 | 0.81 | 0.78 | 0.73 | 0.84 | 0.90 | 0.85 | 0.95 | 0.73 | 0.66 | 0.80 | 0.78 | 0.74 | 0.82 | 0.89 | 0.83 | 0.94 | 0.74 | 0.69 | 0.79 |
| 394 | 0.76 | 0.68 | 0.83 | 0.85 | 0.72 | 0.98 | 0.73 | 0.64 | 0.81 | 0.78 | 0.73 | 0.84 | 0.87 | 0.80 | 0.95 | 0.75 | 0.68 | 0.81 | 0.79 | 0.74 | 0.83 | 0.87 | 0.81 | 0.94 | 0.75 | 0.70 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 395 | 0.73 | 0.66 | 0.81 | 0.82 | 0.69 | 0.95 | 0.71 | 0.62 | 0.80 | 0.78 | 0.73 | 0.84 | 0.85 | 0.78 | 0.92 | 0.76 | 0.69 | 0.82 | 0.77 | 0.72 | 0.81 | 0.84 | 0.78 | 0.90 | 0.74 | 0.69 | 0.79 |
| 396 | 0.76 | 0.69 | 0.83 | 0.80 | 0.66 | 0.95 | 0.74 | 0.66 | 0.82 | 0.78 | 0.73 | 0.84 | 0.86 | 0.79 | 0.93 | 0.75 | 0.69 | 0.82 | 0.79 | 0.74 | 0.83 | 0.85 | 0.79 | 0.92 | 0.76 | 0.71 | 0.81 |
| 397 | 0.75 | 0.67 | 0.83 | 0.83 | 0.66 | 1.00 | 0.73 | 0.64 | 0.82 | 0.78 | 0.73 | 0.84 | 0.88 | 0.81 | 0.94 | 0.75 | 0.68 | 0.82 | 0.79 | 0.74 | 0.83 | 0.88 | 0.81 | 0.94 | 0.76 | 0.71 | 0.81 |
| 398 | 0.78 | 0.71 | 0.85 | 0.85 | 0.73 | 0.98 | 0.75 | 0.67 | 0.83 | 0.79 | 0.73 | 0.84 | 0.87 | 0.80 | 0.94 | 0.75 | 0.69 | 0.82 | 0.79 | 0.75 | 0.83 | 0.87 | 0.81 | 0.93 | 0.76 | 0.71 | 0.81 |
| 399 | 0.73 | 0.65 | 0.82 | 0.80 | 0.66 | 0.94 | 0.71 | 0.61 | 0.81 | 0.79 | 0.73 | 0.85 | 0.88 | 0.81 | 0.95 | 0.75 | 0.68 | 0.82 | 0.78 | 0.73 | 0.83 | 0.87 | 0.80 | 0.93 | 0.75 | 0.69 | 0.80 |
| 400 | 0.77 | 0.69 | 0.85 | 0.90 | 0.81 | 0.99 | 0.73 | 0.63 | 0.83 | 0.76 | 0.70 | 0.83 | 0.83 | 0.72 | 0.95 | 0.74 | 0.67 | 0.81 | 0.77 | 0.73 | 0.82 | 0.86 | 0.78 | 0.94 | 0.74 | 0.69 | 0.80 |
| 401 | 0.74 | 0.65 | 0.82 | 0.88 | 0.74 | 1.00 | 0.69 | 0.60 | 0.79 | 0.77 | 0.71 | 0.83 | 0.87 | 0.79 | 0.96 | 0.73 | 0.66 | 0.80 | 0.77 | 0.72 | 0.81 | 0.88 | 0.81 | 0.95 | 0.73 | 0.67 | 0.78 |
| 402 | 0.74 | 0.66 | 0.83 | 0.82 | 0.67 | 0.97 | 0.72 | 0.62 | 0.82 | 0.78 | 0.72 | 0.84 | 0.86 | 0.78 | 0.95 | 0.75 | 0.68 | 0.82 | 0.79 | 0.74 | 0.83 | 0.86 | 0.79 | 0.93 | 0.76 | 0.71 | 0.81 |
| 403 | 0.74 | 0.66 | 0.82 | 0.77 | 0.61 | 0.92 | 0.73 | 0.64 | 0.82 | 0.79 | 0.74 | 0.85 | 0.84 | 0.75 | 0.92 | 0.77 | 0.71 | 0.84 | 0.79 | 0.74 | 0.82 | 0.83 | 0.75 | 0.90 | 0.77 | 0.72 | 0.82 |
| 404 | 0.77 | 0.69 | 0.85 | 0.89 | 0.80 | 0.98 | 0.73 | 0.63 | 0.82 | 0.76 | 0.70 | 0.82 | 0.83 | 0.71 | 0.94 | 0.74 | 0.67 | 0.81 | 0.78 | 0.73 | 0.83 | 0.85 | 0.77 | 0.93 | 0.75 | 0.70 | 0.80 |
| 405 | 0.74 | 0.66 | 0.83 | 0.86 | 0.73 | 0.98 | 0.71 | 0.62 | 0.81 | 0.78 | 0.72 | 0.83 | 0.91 | 0.86 | 0.96 | 0.72 | 0.65 | 0.79 | 0.78 | 0.74 | 0.83 | 0.90 | 0.86 | 0.95 | 0.74 | 0.68 | 0.79 |
| 406 | 0.77 | 0.69 | 0.85 | 0.84 | 0.70 | 0.97 | 0.75 | 0.65 | 0.84 | 0.79 | 0.73 | 0.84 | 0.89 | 0.83 | 0.95 | 0.74 | 0.67 | 0.82 | 0.80 | 0.75 | 0.84 | 0.88 | 0.83 | 0.94 | 0.76 | 0.71 | 0.82 |
| 407 | 0.75 | 0.66 | 0.83 | 0.86 | 0.71 | 1.00 | 0.71 | 0.62 | 0.81 | 0.78 | 0.72 | 0.84 | 0.91 | 0.86 | 0.96 | 0.73 | 0.66 | 0.80 | 0.78 | 0.73 | 0.82 | 0.90 | 0.85 | 0.96 | 0.73 | 0.68 | 0.79 |
| 408 | 0.77 | 0.69 | 0.85 | 0.88 | 0.74 | 1.00 | 0.74 | 0.65 | 0.83 | 0.78 | 0.72 | 0.83 | 0.92 | 0.89 | 0.96 | 0.71 | 0.64 | 0.79 | 0.79 | 0.74 | 0.83 | 0.92 | 0.88 | 0.97 | 0.74 | 0.68 | 0.79 |
| 409 | 0.75 | 0.66 | 0.83 | 0.80 | 0.60 | 0.99 | 0.73 | 0.64 | 0.82 | 0.78 | 0.74 | 0.85 | 0.83 | 0.73 | 0.93 | 0.78 | 0.71 | 0.85 | 0.79 | 0.74 | 0.83 | 0.84 | 0.75 | 0.92 | 0.77 | 0.72 | 0.82 |
| 410 | 0.76 | 0.68 | 0.84 | 0.89 | 0.76 | 1.00 | 0.72 | 0.63 | 0.82 | 0.78 | 0.72 | 0.83 | 0.91 | 0.87 | 0.95 | 0.72 | 0.65 | 0.79 | 0.79 | 0.74 | 0.83 | 0.92 | 0.87 | 0.96 | 0.74 | 0.68 | 0.80 |
| 411 | 0.74 | 0.67 | 0.81 | 0.81 | 0.68 | 0.93 | 0.72 | 0.63 | 0.80 | 0.78 | 0.72 | 0.84 | 0.86 | 0.77 | 0.95 | 0.75 | 0.69 | 0.82 | 0.77 | 0.73 | 0.82 | 0.84 | 0.77 | 0.92 | 0.75 | 0.70 | 0.80 |
| 412 | 0.74 | 0.67 | 0.82 | 0.84 | 0.73 | 0.95 | 0.71 | 0.62 | 0.80 | 0.76 | 0.70 | 0.83 | 0.81 | 0.68 | 0.94 | 0.73 | 0.68 | 0.82 | 0.77 | 0.72 | 0.81 | 0.82 | 0.74 | 0.91 | 0.72 | 0.67 | 0.78 |
| 413 | 0.74 | 0.66 | 0.82 | 0.88 | 0.78 | 0.98 | 0.70 | 0.60 | 0.79 | 0.77 | 0.72 | 0.83 | 0.89 | 0.83 | 0.94 | 0.75 | 0.65 | 0.80 | 0.77 | 0.73 | 0.82 | 0.89 | 0.85 | 0.94 | 0.75 | 0.70 | 0.80 |
| 414 | 0.75 | 0.68 | 0.82 | 0.80 | 0.68 | 0.91 | 0.73 | 0.65 | 0.81 | 0.78 | 0.72 | 0.84 | 0.87 | 0.79 | 0.96 | 0.75 | 0.68 | 0.82 | 0.78 | 0.74 | 0.82 | 0.85 | 0.79 | 0.92 | 0.72 | 0.67 | 0.82 |
| 415 | 0.76 | 0.68 | 0.83 | 0.85 | 0.72 | 0.98 | 0.73 | 0.64 | 0.81 | 0.78 | 0.73 | 0.84 | 0.86 | 0.79 | 0.94 | 0.75 | 0.69 | 0.82 | 0.79 | 0.75 | 0.83 | 0.87 | 0.81 | 0.93 | 0.75 | 0.70 | 0.79 |
| 416 | 0.77 | 0.69 | 0.84 | 0.85 | 0.71 | 0.99 | 0.74 | 0.66 | 0.83 | 0.78 | 0.73 | 0.84 | 0.87 | 0.80 | 0.94 | 0.75 | 0.68 | 0.82 | 0.78 | 0.74 | 0.83 | 0.86 | 0.80 | 0.90 | 0.76 | 0.71 | 0.80 |
| 417 | 0.75 | 0.68 | 0.82 | 0.82 | 0.69 | 0.94 | 0.72 | 0.64 | 0.80 | 0.78 | 0.73 | 0.84 | 0.84 | 0.75 | 0.93 | 0.76 | 0.69 | 0.82 | 0.77 | 0.73 | 0.82 | 0.84 | 0.77 | 0.91 | 0.75 | 0.70 | 0.80 |
| 418 | 0.76 | 0.68 | 0.83 | 0.84 | 0.72 | 0.96 | 0.73 | 0.64 | 0.81 | 0.79 | 0.73 | 0.85 | 0.86 | 0.76 | 0.96 | 0.77 | 0.70 | 0.83 | 0.78 | 0.74 | 0.83 | 0.86 | 0.78 | 0.93 | 0.76 | 0.71 | 0.81 |
| 419 | 0.75 | 0.66 | 0.83 | 0.82 | 0.69 | 0.95 | 0.72 | 0.62 | 0.82 | 0.78 | 0.73 | 0.84 | 0.85 | 0.77 | 0.94 | 0.75 | 0.69 | 0.82 | 0.78 | 0.74 | 0.83 | 0.85 | 0.78 | 0.92 | 0.76 | 0.70 | 0.81 |
| 420 | 0.74 | 0.66 | 0.81 | 0.81 | 0.70 | 0.91 | 0.71 | 0.63 | 0.80 | 0.78 | 0.72 | 0.84 | 0.86 | 0.78 | 0.93 | 0.75 | 0.68 | 0.82 | 0.77 | 0.73 | 0.81 | 0.85 | 0.79 | 0.91 | 0.74 | 0.69 | 0.79 |
| 421 | 0.76 | 0.67 | 0.85 | 0.90 | 0.75 | 1.00 | 0.72 | 0.62 | 0.82 | 0.78 | 0.72 | 0.83 | 0.89 | 0.84 | 0.94 | 0.73 | 0.66 | 0.80 | 0.79 | 0.75 | 0.84 | 0.91 | 0.86 | 0.96 | 0.75 | 0.69 | 0.80 |
| 422 | 0.75 | 0.66 | 0.83 | 0.77 | 0.60 | 0.95 | 0.74 | 0.64 | 0.83 | 0.79 | 0.73 | 0.84 | 0.86 | 0.79 | 0.93 | 0.76 | 0.69 | 0.82 | 0.79 | 0.73 | 0.83 | 0.85 | 0.78 | 0.92 | 0.76 | 0.71 | 0.82 |
| 423 | 0.75 | 0.68 | 0.83 | 0.85 | 0.76 | 0.95 | 0.72 | 0.63 | 0.81 | 0.78 | 0.72 | 0.84 | 0.86 | 0.79 | 0.94 | 0.75 | 0.68 | 0.82 | 0.78 | 0.73 | 0.82 | 0.86 | 0.81 | 0.92 | 0.74 | 0.69 | 0.79 |
| 424 | 0.76 | 0.68 | 0.81 | 0.79 | 0.64 | 0.93 | 0.73 | 0.66 | 0.81 | 0.78 | 0.73 | 0.84 | 0.84 | 0.76 | 0.93 | 0.76 | 0.69 | 0.82 | 0.78 | 0.73 | 0.82 | 0.83 | 0.76 | 0.90 | 0.75 | 0.71 | 0.80 |
| 425 | 0.74 | 0.67 | 0.81 | 0.80 | 0.70 | 0.90 | 0.72 | 0.64 | 0.81 | 0.78 | 0.72 | 0.84 | 0.85 | 0.79 | 0.92 | 0.75 | 0.68 | 0.82 | 0.78 | 0.74 | 0.82 | 0.85 | 0.80 | 0.90 | 0.75 | 0.70 | 0.80 |
| 426 | 0.77 | 0.68 | 0.85 | 0.87 | 0.72 | 1.00 | 0.73 | 0.64 | 0.83 | 0.78 | 0.72 | 0.84 | 0.91 | 0.86 | 0.96 | 0.72 | 0.65 | 0.79 | 0.79 | 0.74 | 0.83 | 0.91 | 0.86 | 0.96 | 0.74 | 0.69 | 0.80 |
| 427 | 0.78 | 0.71 | 0.85 | 0.86 | 0.76 | 0.97 | 0.75 | 0.67 | 0.83 | 0.78 | 0.73 | 0.84 | 0.87 | 0.79 | 0.95 | 0.75 | 0.68 | 0.82 | 0.79 | 0.74 | 0.83 | 0.87 | 0.81 | 0.93 | 0.75 | 0.70 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 428 | 0.74 | 0.65 | 0.82 | 0.81 | 0.66 | 0.97 | 0.71 | 0.62 | 0.80 | 0.79 | 0.73 | 0.84 | 0.86 | 0.80 | 0.93 | 0.75 | 0.69 | 0.82 | 0.78 | 0.74 | 0.82 | 0.86 | 0.80 | 0.92 | 0.75 | 0.70 | 0.80 |
| 429 | 0.75 | 0.67 | 0.83 | 0.88 | 0.73 | 1.00 | 0.71 | 0.62 | 0.80 | 0.77 | 0.72 | 0.83 | 0.92 | 0.87 | 0.96 | 0.71 | 0.64 | 0.79 | 0.77 | 0.73 | 0.82 | 0.91 | 0.86 | 0.96 | 0.72 | 0.67 | 0.78 |
| 430 | 0.74 | 0.66 | 0.82 | 0.89 | 0.77 | 1.00 | 0.69 | 0.60 | 0.79 | 0.77 | 0.72 | 0.83 | 0.89 | 0.83 | 0.94 | 0.73 | 0.66 | 0.80 | 0.77 | 0.72 | 0.81 | 0.89 | 0.84 | 0.94 | 0.72 | 0.67 | 0.78 |
| 431 | 0.75 | 0.66 | 0.83 | 0.81 | 0.65 | 0.97 | 0.72 | 0.63 | 0.82 | 0.79 | 0.73 | 0.84 | 0.88 | 0.81 | 0.94 | 0.76 | 0.69 | 0.82 | 0.79 | 0.74 | 0.83 | 0.87 | 0.80 | 0.93 | 0.76 | 0.71 | 0.81 |
| 432 | 0.73 | 0.65 | 0.82 | 0.78 | 0.64 | 0.93 | 0.72 | 0.62 | 0.81 | 0.78 | 0.73 | 0.84 | 0.85 | 0.77 | 0.94 | 0.76 | 0.69 | 0.82 | 0.78 | 0.74 | 0.83 | 0.84 | 0.76 | 0.91 | 0.76 | 0.71 | 0.81 |
| 433 | 0.74 | 0.65 | 0.83 | 0.79 | 0.59 | 0.99 | 0.73 | 0.63 | 0.82 | 0.80 | 0.74 | 0.85 | 0.84 | 0.74 | 0.93 | 0.78 | 0.72 | 0.85 | 0.79 | 0.74 | 0.84 | 0.84 | 0.75 | 0.92 | 0.77 | 0.72 | 0.82 |
| 434 | 0.76 | 0.68 | 0.85 | 0.85 | 0.73 | 0.97 | 0.73 | 0.63 | 0.83 | 0.77 | 0.71 | 0.83 | 0.85 | 0.77 | 0.93 | 0.74 | 0.67 | 0.81 | 0.78 | 0.73 | 0.83 | 0.86 | 0.79 | 0.93 | 0.75 | 0.69 | 0.81 |
| 435 | 0.74 | 0.67 | 0.81 | 0.77 | 0.65 | 0.89 | 0.73 | 0.65 | 0.81 | 0.78 | 0.72 | 0.84 | 0.87 | 0.79 | 0.95 | 0.75 | 0.68 | 0.82 | 0.77 | 0.73 | 0.82 | 0.84 | 0.77 | 0.91 | 0.75 | 0.70 | 0.80 |
| 436 | 0.76 | 0.68 | 0.84 | 0.88 | 0.74 | 1.00 | 0.72 | 0.63 | 0.82 | 0.79 | 0.73 | 0.84 | 0.90 | 0.84 | 0.95 | 0.74 | 0.67 | 0.81 | 0.79 | 0.75 | 0.84 | 0.91 | 0.86 | 0.96 | 0.75 | 0.70 | 0.81 |
| 437 | 0.76 | 0.68 | 0.84 | 0.83 | 0.69 | 0.98 | 0.74 | 0.64 | 0.84 | 0.79 | 0.73 | 0.84 | 0.89 | 0.83 | 0.94 | 0.75 | 0.68 | 0.82 | 0.79 | 0.75 | 0.84 | 0.88 | 0.82 | 0.94 | 0.76 | 0.71 | 0.81 |
| 438 | 0.75 | 0.68 | 0.83 | 0.80 | 0.65 | 0.94 | 0.74 | 0.66 | 0.82 | 0.78 | 0.72 | 0.84 | 0.85 | 0.78 | 0.92 | 0.75 | 0.69 | 0.82 | 0.78 | 0.73 | 0.82 | 0.84 | 0.77 | 0.90 | 0.76 | 0.70 | 0.81 |
| 439 | 0.76 | 0.67 | 0.84 | 0.80 | 0.63 | 0.98 | 0.74 | 0.64 | 0.84 | 0.79 | 0.73 | 0.84 | 0.88 | 0.83 | 0.94 | 0.75 | 0.68 | 0.82 | 0.78 | 0.73 | 0.83 | 0.86 | 0.80 | 0.93 | 0.75 | 0.69 | 0.80 |
| 440 | 0.76 | 0.69 | 0.83 | 0.81 | 0.66 | 0.95 | 0.75 | 0.67 | 0.82 | 0.78 | 0.73 | 0.84 | 0.86 | 0.78 | 0.94 | 0.75 | 0.69 | 0.82 | 0.78 | 0.74 | 0.83 | 0.85 | 0.78 | 0.92 | 0.76 | 0.71 | 0.81 |
| 441 | 0.75 | 0.66 | 0.83 | 0.88 | 0.72 | 1.00 | 0.71 | 0.61 | 0.81 | 0.78 | 0.72 | 0.83 | 0.89 | 0.83 | 0.95 | 0.73 | 0.66 | 0.80 | 0.78 | 0.74 | 0.83 | 0.90 | 0.85 | 0.95 | 0.74 | 0.68 | 0.79 |
| 442 | 0.75 | 0.67 | 0.83 | 0.88 | 0.75 | 1.00 | 0.72 | 0.62 | 0.81 | 0.79 | 0.73 | 0.84 | 0.90 | 0.84 | 0.95 | 0.74 | 0.67 | 0.81 | 0.79 | 0.75 | 0.84 | 0.91 | 0.86 | 0.96 | 0.75 | 0.69 | 0.80 |
| 443 | 0.74 | 0.65 | 0.82 | 0.80 | 0.67 | 0.94 | 0.71 | 0.61 | 0.82 | 0.79 | 0.73 | 0.84 | 0.87 | 0.80 | 0.94 | 0.75 | 0.68 | 0.82 | 0.78 | 0.74 | 0.83 | 0.86 | 0.79 | 0.92 | 0.75 | 0.70 | 0.81 |
| 444 | 0.75 | 0.68 | 0.83 | 0.81 | 0.67 | 0.95 | 0.73 | 0.65 | 0.82 | 0.78 | 0.73 | 0.83 | 0.86 | 0.78 | 0.93 | 0.75 | 0.68 | 0.81 | 0.78 | 0.74 | 0.82 | 0.85 | 0.79 | 0.91 | 0.75 | 0.70 | 0.80 |
| 445 | 0.74 | 0.66 | 0.81 | 0.85 | 0.73 | 0.97 | 0.70 | 0.61 | 0.79 | 0.78 | 0.72 | 0.84 | 0.86 | 0.77 | 0.95 | 0.75 | 0.68 | 0.82 | 0.77 | 0.73 | 0.82 | 0.86 | 0.79 | 0.93 | 0.74 | 0.69 | 0.79 |
| 446 | 0.75 | 0.67 | 0.84 | 0.90 | 0.77 | 1.00 | 0.71 | 0.61 | 0.81 | 0.78 | 0.72 | 0.83 | 0.90 | 0.84 | 0.95 | 0.73 | 0.66 | 0.80 | 0.79 | 0.74 | 0.83 | 0.91 | 0.86 | 0.96 | 0.74 | 0.68 | 0.80 |
| 447 | 0.74 | 0.67 | 0.81 | 0.80 | 0.67 | 0.94 | 0.72 | 0.65 | 0.80 | 0.78 | 0.72 | 0.84 | 0.84 | 0.76 | 0.93 | 0.75 | 0.69 | 0.82 | 0.77 | 0.73 | 0.82 | 0.83 | 0.76 | 0.90 | 0.75 | 0.70 | 0.80 |
| 448 | 0.78 | 0.71 | 0.84 | 0.87 | 0.79 | 0.95 | 0.74 | 0.66 | 0.83 | 0.78 | 0.73 | 0.84 | 0.88 | 0.82 | 0.94 | 0.74 | 0.67 | 0.81 | 0.79 | 0.75 | 0.83 | 0.89 | 0.84 | 0.93 | 0.76 | 0.71 | 0.80 |
| 449 | 0.75 | 0.67 | 0.83 | 0.86 | 0.68 | 1.00 | 0.72 | 0.64 | 0.81 | 0.78 | 0.73 | 0.84 | 0.88 | 0.83 | 0.94 | 0.74 | 0.67 | 0.80 | 0.79 | 0.75 | 0.83 | 0.90 | 0.84 | 0.95 | 0.75 | 0.70 | 0.80 |
| 450 | 0.79 | 0.71 | 0.86 | 0.89 | 0.81 | 0.97 | 0.75 | 0.66 | 0.85 | 0.78 | 0.73 | 0.84 | 0.89 | 0.82 | 0.96 | 0.74 | 0.67 | 0.81 | 0.79 | 0.75 | 0.84 | 0.89 | 0.84 | 0.94 | 0.76 | 0.70 | 0.81 |
| 451 | 0.75 | 0.68 | 0.82 | 0.80 | 0.67 | 0.93 | 0.73 | 0.65 | 0.81 | 0.78 | 0.72 | 0.84 | 0.85 | 0.76 | 0.95 | 0.75 | 0.69 | 0.82 | 0.78 | 0.74 | 0.82 | 0.84 | 0.77 | 0.91 | 0.76 | 0.71 | 0.81 |
| 452 | 0.76 | 0.69 | 0.83 | 0.83 | 0.74 | 0.92 | 0.73 | 0.65 | 0.82 | 0.78 | 0.73 | 0.84 | 0.85 | 0.79 | 0.91 | 0.75 | 0.68 | 0.82 | 0.78 | 0.74 | 0.83 | 0.86 | 0.81 | 0.90 | 0.76 | 0.71 | 0.81 |
| 453 | 0.74 | 0.67 | 0.81 | 0.81 | 0.69 | 0.92 | 0.72 | 0.64 | 0.81 | 0.78 | 0.72 | 0.84 | 0.88 | 0.81 | 0.94 | 0.74 | 0.67 | 0.81 | 0.76 | 0.72 | 0.81 | 0.85 | 0.79 | 0.91 | 0.73 | 0.68 | 0.78 |
| 454 | 0.77 | 0.68 | 0.86 | 0.89 | 0.73 | 1.00 | 0.73 | 0.63 | 0.83 | 0.78 | 0.72 | 0.83 | 0.89 | 0.84 | 0.94 | 0.73 | 0.66 | 0.80 | 0.80 | 0.75 | 0.84 | 0.91 | 0.86 | 0.96 | 0.75 | 0.70 | 0.81 |
| 455 | 0.75 | 0.67 | 0.84 | 0.85 | 0.74 | 0.96 | 0.72 | 0.62 | 0.82 | 0.76 | 0.70 | 0.82 | 0.80 | 0.69 | 0.92 | 0.75 | 0.68 | 0.82 | 0.76 | 0.72 | 0.81 | 0.82 | 0.74 | 0.90 | 0.74 | 0.69 | 0.80 |
| 456 | 0.76 | 0.69 | 0.83 | 0.84 | 0.73 | 0.95 | 0.73 | 0.65 | 0.82 | 0.78 | 0.72 | 0.84 | 0.84 | 0.74 | 0.94 | 0.76 | 0.69 | 0.82 | 0.78 | 0.74 | 0.83 | 0.85 | 0.78 | 0.92 | 0.76 | 0.71 | 0.81 |
| 457 | 0.77 | 0.70 | 0.84 | 0.87 | 0.74 | 1.00 | 0.74 | 0.66 | 0.82 | 0.78 | 0.73 | 0.84 | 0.87 | 0.79 | 0.95 | 0.75 | 0.68 | 0.81 | 0.79 | 0.74 | 0.83 | 0.87 | 0.81 | 0.94 | 0.75 | 0.70 | 0.80 |
| 458 | 0.75 | 0.67 | 0.82 | 0.85 | 0.74 | 0.95 | 0.71 | 0.62 | 0.80 | 0.76 | 0.70 | 0.83 | 0.81 | 0.68 | 0.94 | 0.75 | 0.68 | 0.81 | 0.77 | 0.72 | 0.81 | 0.83 | 0.74 | 0.91 | 0.74 | 0.69 | 0.80 |
| 459 | 0.75 | 0.68 | 0.83 | 0.87 | 0.74 | 0.99 | 0.72 | 0.63 | 0.80 | 0.78 | 0.73 | 0.84 | 0.86 | 0.78 | 0.94 | 0.76 | 0.69 | 0.82 | 0.78 | 0.74 | 0.83 | 0.87 | 0.81 | 0.94 | 0.75 | 0.70 | 0.80 |
| 460 | 0.74 | 0.67 | 0.81 | 0.83 | 0.72 | 0.94 | 0.71 | 0.63 | 0.80 | 0.78 | 0.72 | 0.84 | 0.86 | 0.77 | 0.94 | 0.75 | 0.68 | 0.82 | 0.78 | 0.73 | 0.82 | 0.85 | 0.79 | 0.92 | 0.75 | 0.70 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 461 | 0.74 | 0.65 | 0.82 | 0.80 | 0.66 | 0.94 | 0.72 | 0.62 | 0.81 | 0.78 | 0.72 | 0.84 | 0.85 | 0.78 | 0.92 | 0.75 | 0.68 | 0.82 | 0.77 | 0.72 | 0.81 | 0.84 | 0.77 | 0.90 | 0.74 | 0.69 | 0.80 |
| 462 | 0.72 | 0.63 | 0.81 | 0.76 | 0.57 | 0.94 | 0.71 | 0.61 | 0.81 | 0.79 | 0.73 | 0.84 | 0.86 | 0.79 | 0.93 | 0.76 | 0.69 | 0.83 | 0.77 | 0.72 | 0.82 | 0.83 | 0.76 | 0.91 | 0.75 | 0.69 | 0.80 |
| 463 | 0.74 | 0.67 | 0.81 | 0.84 | 0.71 | 0.98 | 0.70 | 0.63 | 0.78 | 0.78 | 0.73 | 0.84 | 0.87 | 0.80 | 0.95 | 0.75 | 0.68 | 0.81 | 0.78 | 0.74 | 0.82 | 0.87 | 0.81 | 0.94 | 0.74 | 0.69 | 0.79 |
| 464 | 0.74 | 0.66 | 0.83 | 0.86 | 0.69 | 1.00 | 0.71 | 0.62 | 0.80 | 0.78 | 0.72 | 0.84 | 0.88 | 0.82 | 0.95 | 0.74 | 0.67 | 0.81 | 0.79 | 0.74 | 0.83 | 0.89 | 0.83 | 0.95 | 0.75 | 0.69 | 0.80 |
| 465 | 0.74 | 0.67 | 0.81 | 0.83 | 0.73 | 0.92 | 0.71 | 0.63 | 0.80 | 0.78 | 0.72 | 0.84 | 0.87 | 0.80 | 0.94 | 0.74 | 0.67 | 0.81 | 0.78 | 0.73 | 0.82 | 0.86 | 0.81 | 0.92 | 0.74 | 0.69 | 0.79 |
| 466 | 0.75 | 0.68 | 0.83 | 0.81 | 0.69 | 0.94 | 0.73 | 0.64 | 0.82 | 0.78 | 0.73 | 0.84 | 0.88 | 0.83 | 0.94 | 0.74 | 0.64 | 0.78 | 0.79 | 0.74 | 0.83 | 0.87 | 0.82 | 0.93 | 0.75 | 0.70 | 0.81 |
| 467 | 0.77 | 0.69 | 0.85 | 0.88 | 0.74 | 1.00 | 0.74 | 0.65 | 0.83 | 0.77 | 0.71 | 0.83 | 0.92 | 0.88 | 0.96 | 0.71 | 0.68 | 0.81 | 0.78 | 0.74 | 0.83 | 0.92 | 0.87 | 0.96 | 0.74 | 0.68 | 0.79 |
| 468 | 0.76 | 0.70 | 0.83 | 0.82 | 0.71 | 0.92 | 0.75 | 0.67 | 0.82 | 0.78 | 0.72 | 0.84 | 0.87 | 0.79 | 0.95 | 0.75 | 0.68 | 0.82 | 0.77 | 0.72 | 0.81 | 0.86 | 0.79 | 0.92 | 0.76 | 0.71 | 0.81 |
| 469 | 0.74 | 0.66 | 0.82 | 0.85 | 0.76 | 0.93 | 0.71 | 0.61 | 0.80 | 0.78 | 0.72 | 0.84 | 0.86 | 0.78 | 0.93 | 0.75 | 0.68 | 0.82 | 0.78 | 0.74 | 0.81 | 0.86 | 0.80 | 0.91 | 0.74 | 0.68 | 0.79 |
| 470 | 0.75 | 0.69 | 0.82 | 0.83 | 0.71 | 0.94 | 0.73 | 0.65 | 0.81 | 0.78 | 0.72 | 0.84 | 0.85 | 0.75 | 0.95 | 0.75 | 0.69 | 0.82 | 0.79 | 0.74 | 0.82 | 0.85 | 0.78 | 0.92 | 0.76 | 0.71 | 0.81 |
| 471 | 0.78 | 0.70 | 0.86 | 0.89 | 0.81 | 0.98 | 0.75 | 0.65 | 0.84 | 0.79 | 0.73 | 0.84 | 0.89 | 0.82 | 0.96 | 0.74 | 0.67 | 0.81 | 0.78 | 0.75 | 0.84 | 0.90 | 0.85 | 0.95 | 0.75 | 0.70 | 0.81 |
| 472 | 0.76 | 0.68 | 0.84 | 0.87 | 0.77 | 0.97 | 0.72 | 0.63 | 0.82 | 0.77 | 0.71 | 0.83 | 0.82 | 0.72 | 0.91 | 0.75 | 0.68 | 0.81 | 0.78 | 0.73 | 0.82 | 0.85 | 0.78 | 0.91 | 0.75 | 0.70 | 0.81 |
| 473 | 0.74 | 0.67 | 0.81 | 0.81 | 0.68 | 0.93 | 0.72 | 0.64 | 0.80 | 0.78 | 0.72 | 0.84 | 0.85 | 0.76 | 0.94 | 0.75 | 0.68 | 0.82 | 0.77 | 0.74 | 0.82 | 0.84 | 0.77 | 0.92 | 0.75 | 0.69 | 0.80 |
| 474 | 0.77 | 0.69 | 0.86 | 0.86 | 0.76 | 0.96 | 0.74 | 0.64 | 0.84 | 0.76 | 0.70 | 0.82 | 0.82 | 0.71 | 0.93 | 0.74 | 0.67 | 0.81 | 0.77 | 0.72 | 0.82 | 0.84 | 0.76 | 0.92 | 0.73 | 0.68 | 0.80 |
| 475 | 0.74 | 0.66 | 0.82 | 0.89 | 0.76 | 1.00 | 0.69 | 0.60 | 0.78 | 0.78 | 0.72 | 0.83 | 0.90 | 0.85 | 0.95 | 0.72 | 0.65 | 0.79 | 0.78 | 0.74 | 0.83 | 0.91 | 0.87 | 0.96 | 0.75 | 0.68 | 0.78 |
| 476 | 0.75 | 0.67 | 0.84 | 0.84 | 0.69 | 0.98 | 0.73 | 0.63 | 0.83 | 0.78 | 0.72 | 0.84 | 0.87 | 0.79 | 0.94 | 0.75 | 0.68 | 0.81 | 0.79 | 0.75 | 0.83 | 0.87 | 0.80 | 0.94 | 0.76 | 0.71 | 0.81 |
| 477 | 0.74 | 0.66 | 0.83 | 0.90 | 0.74 | 1.00 | 0.70 | 0.61 | 0.79 | 0.77 | 0.72 | 0.83 | 0.88 | 0.82 | 0.93 | 0.73 | 0.66 | 0.80 | 0.79 | 0.74 | 0.83 | 0.91 | 0.85 | 0.96 | 0.74 | 0.69 | 0.80 |
| 478 | 0.76 | 0.68 | 0.84 | 0.88 | 0.72 | 1.00 | 0.73 | 0.63 | 0.82 | 0.78 | 0.72 | 0.83 | 0.91 | 0.87 | 0.96 | 0.72 | 0.65 | 0.79 | 0.78 | 0.74 | 0.83 | 0.91 | 0.86 | 0.96 | 0.73 | 0.68 | 0.79 |
| 479 | 0.75 | 0.66 | 0.84 | 0.78 | 0.61 | 0.96 | 0.74 | 0.64 | 0.84 | 0.79 | 0.73 | 0.84 | 0.87 | 0.81 | 0.93 | 0.75 | 0.68 | 0.82 | 0.78 | 0.73 | 0.82 | 0.85 | 0.78 | 0.91 | 0.75 | 0.70 | 0.81 |
| 480 | 0.74 | 0.66 | 0.83 | 0.86 | 0.70 | 1.00 | 0.71 | 0.62 | 0.80 | 0.78 | 0.72 | 0.83 | 0.89 | 0.83 | 0.95 | 0.73 | 0.66 | 0.80 | 0.79 | 0.75 | 0.83 | 0.90 | 0.84 | 0.95 | 0.75 | 0.69 | 0.80 |
| 481 | 0.76 | 0.68 | 0.85 | 0.80 | 0.62 | 0.98 | 0.75 | 0.65 | 0.84 | 0.79 | 0.74 | 0.85 | 0.88 | 0.82 | 0.95 | 0.76 | 0.69 | 0.83 | 0.80 | 0.75 | 0.84 | 0.87 | 0.80 | 0.94 | 0.77 | 0.72 | 0.82 |
| 482 | 0.78 | 0.70 | 0.86 | 0.85 | 0.73 | 0.97 | 0.75 | 0.65 | 0.86 | 0.78 | 0.73 | 0.84 | 0.88 | 0.82 | 0.94 | 0.75 | 0.67 | 0.82 | 0.79 | 0.75 | 0.84 | 0.88 | 0.82 | 0.93 | 0.76 | 0.70 | 0.82 |
| 483 | 0.75 | 0.67 | 0.83 | 0.79 | 0.64 | 0.93 | 0.74 | 0.64 | 0.84 | 0.78 | 0.73 | 0.84 | 0.88 | 0.80 | 0.95 | 0.73 | 0.68 | 0.82 | 0.78 | 0.74 | 0.83 | 0.86 | 0.79 | 0.92 | 0.75 | 0.70 | 0.81 |
| 484 | 0.77 | 0.69 | 0.85 | 0.87 | 0.69 | 1.00 | 0.74 | 0.65 | 0.83 | 0.78 | 0.72 | 0.84 | 0.90 | 0.85 | 0.96 | 0.75 | 0.66 | 0.80 | 0.79 | 0.75 | 0.84 | 0.91 | 0.85 | 0.96 | 0.75 | 0.70 | 0.80 |
| 485 | 0.74 | 0.67 | 0.81 | 0.78 | 0.66 | 0.90 | 0.73 | 0.65 | 0.81 | 0.78 | 0.73 | 0.84 | 0.86 | 0.78 | 0.94 | 0.73 | 0.68 | 0.81 | 0.78 | 0.74 | 0.82 | 0.84 | 0.77 | 0.91 | 0.75 | 0.70 | 0.80 |
| 486 | 0.77 | 0.68 | 0.85 | 0.89 | 0.75 | 1.00 | 0.73 | 0.63 | 0.83 | 0.77 | 0.72 | 0.83 | 0.88 | 0.83 | 0.94 | 0.75 | 0.66 | 0.80 | 0.79 | 0.75 | 0.84 | 0.90 | 0.86 | 0.95 | 0.75 | 0.70 | 0.81 |
| 487 | 0.74 | 0.66 | 0.83 | 0.81 | 0.65 | 0.97 | 0.72 | 0.63 | 0.82 | 0.78 | 0.72 | 0.84 | 0.86 | 0.78 | 0.94 | 0.73 | 0.68 | 0.81 | 0.79 | 0.74 | 0.83 | 0.86 | 0.79 | 0.93 | 0.76 | 0.71 | 0.81 |
| 488 | 0.76 | 0.69 | 0.82 | 0.82 | 0.73 | 0.91 | 0.74 | 0.66 | 0.81 | 0.78 | 0.73 | 0.83 | 0.87 | 0.80 | 0.93 | 0.75 | 0.67 | 0.81 | 0.78 | 0.75 | 0.82 | 0.86 | 0.81 | 0.91 | 0.76 | 0.70 | 0.80 |
| 489 | 0.76 | 0.69 | 0.83 | 0.82 | 0.73 | 0.91 | 0.74 | 0.66 | 0.82 | 0.78 | 0.73 | 0.84 | 0.88 | 0.81 | 0.93 | 0.74 | 0.68 | 0.81 | 0.79 | 0.74 | 0.83 | 0.86 | 0.81 | 0.91 | 0.77 | 0.72 | 0.81 |
| 490 | 0.75 | 0.67 | 0.82 | 0.88 | 0.72 | 1.00 | 0.71 | 0.62 | 0.81 | 0.77 | 0.72 | 0.83 | 0.83 | 0.82 | 0.95 | 0.74 | 0.66 | 0.80 | 0.78 | 0.75 | 0.82 | 0.90 | 0.84 | 0.91 | 0.76 | 0.70 | 0.79 |
| 491 | 0.75 | 0.68 | 0.82 | 0.84 | 0.72 | 0.96 | 0.72 | 0.64 | 0.80 | 0.78 | 0.72 | 0.83 | 0.86 | 0.75 | 0.92 | 0.76 | 0.70 | 0.82 | 0.79 | 0.74 | 0.83 | 0.85 | 0.78 | 0.91 | 0.75 | 0.70 | 0.81 |
| 492 | 0.74 | 0.66 | 0.82 | 0.82 | 0.67 | 0.97 | 0.72 | 0.62 | 0.81 | 0.78 | 0.72 | 0.84 | 0.86 | 0.77 | 0.94 | 0.75 | 0.68 | 0.82 | 0.78 | 0.74 | 0.83 | 0.86 | 0.79 | 0.93 | 0.76 | 0.70 | 0.81 |
| 493 | 0.74 | 0.66 | 0.82 | 0.81 | 0.66 | 0.97 | 0.72 | 0.62 | 0.81 | 0.78 | 0.73 | 0.84 | 0.85 | 0.78 | 0.92 | 0.76 | 0.69 | 0.82 | 0.78 | 0.74 | 0.83 | 0.86 | 0.79 | 0.92 | 0.75 | 0.70 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 494 | 0.77 | 0.70 | 0.84 | 0.85 | 0.72 | 0.98 | 0.74 | 0.66 | 0.82 | 0.78 | 0.73 | 0.84 | 0.87 | 0.80 | 0.93 | 0.75 | 0.68 | 0.82 | 0.78 | 0.74 | 0.83 | 0.87 | 0.81 | 0.93 | 0.75 | 0.70 | 0.80 |
| 495 | 0.76 | 0.69 | 0.83 | 0.83 | 0.73 | 0.93 | 0.74 | 0.66 | 0.82 | 0.78 | 0.72 | 0.84 | 0.87 | 0.78 | 0.95 | 0.74 | 0.68 | 0.81 | 0.78 | 0.74 | 0.82 | 0.86 | 0.80 | 0.92 | 0.75 | 0.70 | 0.80 |
| 496 | 0.75 | 0.67 | 0.82 | 0.81 | 0.67 | 0.95 | 0.73 | 0.64 | 0.81 | 0.78 | 0.72 | 0.83 | 0.85 | 0.77 | 0.93 | 0.75 | 0.68 | 0.81 | 0.77 | 0.73 | 0.82 | 0.84 | 0.77 | 0.91 | 0.75 | 0.70 | 0.80 |
| 497 | 0.71 | 0.62 | 0.80 | 0.78 | 0.63 | 0.94 | 0.69 | 0.58 | 0.79 | 0.76 | 0.70 | 0.83 | 0.86 | 0.75 | 0.96 | 0.73 | 0.66 | 0.81 | 0.76 | 0.71 | 0.81 | 0.83 | 0.75 | 0.92 | 0.73 | 0.67 | 0.79 |
| 498 | 0.75 | 0.67 | 0.83 | 0.81 | 0.66 | 0.96 | 0.73 | 0.64 | 0.82 | 0.78 | 0.72 | 0.83 | 0.85 | 0.76 | 0.93 | 0.75 | 0.68 | 0.81 | 0.78 | 0.73 | 0.82 | 0.84 | 0.77 | 0.91 | 0.75 | 0.70 | 0.80 |
| 499 | 0.73 | 0.64 | 0.82 | 0.80 | 0.65 | 0.94 | 0.70 | 0.60 | 0.81 | 0.78 | 0.73 | 0.84 | 0.86 | 0.79 | 0.93 | 0.75 | 0.68 | 0.82 | 0.77 | 0.73 | 0.82 | 0.85 | 0.78 | 0.91 | 0.75 | 0.69 | 0.80 |
| 500 | 0.75 | 0.67 | 0.83 | 0.83 | 0.70 | 0.96 | 0.72 | 0.63 | 0.82 | 0.78 | 0.72 | 0.84 | 0.84 | 0.76 | 0.92 | 0.76 | 0.69 | 0.82 | 0.78 | 0.73 | 0.82 | 0.84 | 0.78 | 0.91 | 0.75 | 0.70 | 0.81 |
| 501 | 0.77 | 0.69 | 0.86 | 0.85 | 0.72 | 0.98 | 0.74 | 0.64 | 0.85 | 0.78 | 0.73 | 0.84 | 0.90 | 0.84 | 0.95 | 0.74 | 0.67 | 0.81 | 0.79 | 0.74 | 0.83 | 0.89 | 0.84 | 0.94 | 0.75 | 0.69 | 0.81 |
| 502 | 0.74 | 0.66 | 0.81 | 0.84 | 0.69 | 0.98 | 0.71 | 0.62 | 0.79 | 0.77 | 0.71 | 0.83 | 0.87 | 0.80 | 0.94 | 0.73 | 0.66 | 0.80 | 0.77 | 0.72 | 0.81 | 0.87 | 0.80 | 0.93 | 0.73 | 0.67 | 0.78 |
| 503 | 0.74 | 0.67 | 0.81 | 0.83 | 0.72 | 0.93 | 0.71 | 0.63 | 0.80 | 0.78 | 0.72 | 0.84 | 0.85 | 0.75 | 0.96 | 0.75 | 0.68 | 0.82 | 0.77 | 0.73 | 0.82 | 0.85 | 0.78 | 0.92 | 0.75 | 0.70 | 0.80 |
| 504 | 0.75 | 0.67 | 0.83 | 0.83 | 0.69 | 0.97 | 0.73 | 0.64 | 0.82 | 0.78 | 0.72 | 0.84 | 0.87 | 0.78 | 0.95 | 0.74 | 0.68 | 0.81 | 0.79 | 0.74 | 0.83 | 0.87 | 0.80 | 0.94 | 0.76 | 0.70 | 0.81 |
| 505 | 0.74 | 0.67 | 0.81 | 0.81 | 0.69 | 0.94 | 0.72 | 0.64 | 0.80 | 0.78 | 0.72 | 0.84 | 0.85 | 0.76 | 0.94 | 0.75 | 0.68 | 0.82 | 0.78 | 0.73 | 0.82 | 0.85 | 0.78 | 0.92 | 0.75 | 0.70 | 0.80 |
| 506 | 0.74 | 0.66 | 0.82 | 0.81 | 0.68 | 0.95 | 0.71 | 0.62 | 0.81 | 0.78 | 0.72 | 0.83 | 0.84 | 0.77 | 0.92 | 0.75 | 0.68 | 0.81 | 0.77 | 0.72 | 0.81 | 0.84 | 0.78 | 0.90 | 0.74 | 0.68 | 0.79 |
| 507 | 0.74 | 0.67 | 0.81 | 0.78 | 0.66 | 0.89 | 0.73 | 0.64 | 0.81 | 0.78 | 0.72 | 0.84 | 0.84 | 0.77 | 0.92 | 0.75 | 0.68 | 0.82 | 0.77 | 0.72 | 0.81 | 0.82 | 0.76 | 0.89 | 0.75 | 0.69 | 0.80 |
| 508 | 0.74 | 0.66 | 0.82 | 0.81 | 0.64 | 0.98 | 0.72 | 0.63 | 0.81 | 0.77 | 0.72 | 0.83 | 0.89 | 0.84 | 0.95 | 0.72 | 0.65 | 0.79 | 0.78 | 0.74 | 0.83 | 0.88 | 0.83 | 0.94 | 0.74 | 0.69 | 0.80 |
| 509 | 0.74 | 0.65 | 0.82 | 0.82 | 0.68 | 0.97 | 0.71 | 0.62 | 0.81 | 0.77 | 0.71 | 0.82 | 0.85 | 0.77 | 0.93 | 0.73 | 0.66 | 0.80 | 0.77 | 0.73 | 0.82 | 0.86 | 0.79 | 0.92 | 0.74 | 0.68 | 0.79 |
| 510 | 0.77 | 0.69 | 0.85 | 0.84 | 0.70 | 0.97 | 0.75 | 0.65 | 0.84 | 0.78 | 0.73 | 0.84 | 0.86 | 0.78 | 0.94 | 0.75 | 0.69 | 0.82 | 0.80 | 0.75 | 0.84 | 0.87 | 0.80 | 0.93 | 0.77 | 0.72 | 0.82 |
| 511 | 0.74 | 0.67 | 0.81 | 0.81 | 0.70 | 0.93 | 0.71 | 0.63 | 0.80 | 0.78 | 0.72 | 0.83 | 0.83 | 0.75 | 0.92 | 0.76 | 0.69 | 0.82 | 0.77 | 0.73 | 0.81 | 0.83 | 0.76 | 0.90 | 0.74 | 0.69 | 0.79 |
| 512 | 0.77 | 0.68 | 0.85 | 0.86 | 0.74 | 0.98 | 0.73 | 0.63 | 0.84 | 0.76 | 0.70 | 0.82 | 0.81 | 0.70 | 0.93 | 0.74 | 0.67 | 0.81 | 0.77 | 0.72 | 0.82 | 0.84 | 0.75 | 0.92 | 0.75 | 0.69 | 0.81 |
| 513 | 0.77 | 0.70 | 0.85 | 0.88 | 0.79 | 0.98 | 0.74 | 0.65 | 0.82 | 0.78 | 0.72 | 0.84 | 0.85 | 0.76 | 0.94 | 0.75 | 0.69 | 0.82 | 0.79 | 0.75 | 0.83 | 0.87 | 0.81 | 0.93 | 0.76 | 0.71 | 0.81 |
| 514 | 0.74 | 0.66 | 0.82 | 0.79 | 0.63 | 0.96 | 0.72 | 0.63 | 0.81 | 0.78 | 0.73 | 0.84 | 0.88 | 0.81 | 0.95 | 0.74 | 0.68 | 0.81 | 0.78 | 0.74 | 0.83 | 0.86 | 0.80 | 0.93 | 0.75 | 0.70 | 0.80 |
| 515 | 0.73 | 0.64 | 0.81 | 0.74 | 0.57 | 0.91 | 0.72 | 0.62 | 0.82 | 0.78 | 0.72 | 0.84 | 0.86 | 0.78 | 0.93 | 0.75 | 0.68 | 0.82 | 0.77 | 0.72 | 0.82 | 0.83 | 0.75 | 0.90 | 0.75 | 0.69 | 0.80 |
| 516 | 0.74 | 0.66 | 0.82 | 0.81 | 0.64 | 0.99 | 0.72 | 0.63 | 0.81 | 0.78 | 0.72 | 0.83 | 0.89 | 0.84 | 0.95 | 0.72 | 0.65 | 0.79 | 0.78 | 0.73 | 0.82 | 0.88 | 0.83 | 0.94 | 0.74 | 0.68 | 0.79 |
| 517 | 0.74 | 0.67 | 0.81 | 0.83 | 0.68 | 0.97 | 0.71 | 0.63 | 0.79 | 0.78 | 0.72 | 0.84 | 0.88 | 0.81 | 0.95 | 0.74 | 0.68 | 0.81 | 0.78 | 0.73 | 0.82 | 0.87 | 0.80 | 0.93 | 0.74 | 0.69 | 0.79 |
| 518 | 0.74 | 0.67 | 0.81 | 0.79 | 0.68 | 0.91 | 0.72 | 0.64 | 0.80 | 0.78 | 0.72 | 0.84 | 0.86 | 0.77 | 0.94 | 0.75 | 0.68 | 0.81 | 0.77 | 0.73 | 0.82 | 0.84 | 0.78 | 0.91 | 0.75 | 0.70 | 0.80 |
| 519 | 0.74 | 0.68 | 0.81 | 0.80 | 0.70 | 0.91 | 0.73 | 0.65 | 0.80 | 0.78 | 0.72 | 0.83 | 0.86 | 0.78 | 0.94 | 0.74 | 0.67 | 0.81 | 0.77 | 0.73 | 0.81 | 0.84 | 0.78 | 0.91 | 0.74 | 0.69 | 0.79 |
| 520 | 0.76 | 0.69 | 0.83 | 0.88 | 0.79 | 0.97 | 0.72 | 0.64 | 0.80 | 0.78 | 0.72 | 0.84 | 0.87 | 0.78 | 0.95 | 0.74 | 0.68 | 0.81 | 0.78 | 0.74 | 0.82 | 0.88 | 0.82 | 0.94 | 0.75 | 0.70 | 0.80 |
| 521 | 0.76 | 0.69 | 0.83 | 0.85 | 0.73 | 0.97 | 0.73 | 0.65 | 0.82 | 0.78 | 0.72 | 0.84 | 0.87 | 0.79 | 0.94 | 0.74 | 0.68 | 0.81 | 0.79 | 0.74 | 0.83 | 0.87 | 0.81 | 0.93 | 0.75 | 0.70 | 0.80 |
| 522 | 0.74 | 0.66 | 0.82 | 0.81 | 0.64 | 0.97 | 0.72 | 0.62 | 0.81 | 0.78 | 0.73 | 0.84 | 0.88 | 0.81 | 0.94 | 0.75 | 0.68 | 0.82 | 0.78 | 0.74 | 0.83 | 0.86 | 0.79 | 0.93 | 0.75 | 0.70 | 0.81 |
| 523 | 0.75 | 0.67 | 0.83 | 0.86 | 0.73 | 0.99 | 0.72 | 0.62 | 0.81 | 0.77 | 0.72 | 0.83 | 0.91 | 0.86 | 0.95 | 0.72 | 0.65 | 0.79 | 0.78 | 0.74 | 0.83 | 0.90 | 0.86 | 0.95 | 0.74 | 0.68 | 0.79 |
| 524 | 0.75 | 0.68 | 0.82 | 0.85 | 0.72 | 0.97 | 0.72 | 0.63 | 0.80 | 0.78 | 0.72 | 0.83 | 0.87 | 0.79 | 0.94 | 0.74 | 0.67 | 0.81 | 0.78 | 0.74 | 0.82 | 0.87 | 0.81 | 0.93 | 0.74 | 0.69 | 0.79 |
| 525 | 0.74 | 0.67 | 0.81 | 0.81 | 0.69 | 0.94 | 0.72 | 0.63 | 0.80 | 0.78 | 0.72 | 0.84 | 0.85 | 0.77 | 0.94 | 0.75 | 0.68 | 0.82 | 0.77 | 0.73 | 0.82 | 0.85 | 0.78 | 0.91 | 0.75 | 0.70 | 0.80 |
| 526 | 0.75 | 0.67 | 0.83 | 0.83 | 0.67 | 1.00 | 0.72 | 0.63 | 0.82 | 0.78 | 0.73 | 0.84 | 0.84 | 0.74 | 0.94 | 0.76 | 0.70 | 0.83 | 0.79 | 0.74 | 0.83 | 0.85 | 0.77 | 0.93 | 0.76 | 0.71 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 527 | 0.74 | 0.66 | 0.82 | 0.79 | 0.63 | 0.95 | 0.73 | 0.63 | 0.82 | 0.78 | 0.73 | 0.84 | 0.88 | 0.81 | 0.95 | 0.74 | 0.67 | 0.81 | 0.78 | 0.74 | 0.83 | 0.86 | 0.79 | 0.93 | 0.75 | 0.70 | 0.81 |
| 528 | 0.74 | 0.67 | 0.82 | 0.85 | 0.72 | 0.98 | 0.70 | 0.62 | 0.79 | 0.77 | 0.71 | 0.83 | 0.82 | 0.73 | 0.91 | 0.75 | 0.68 | 0.82 | 0.77 | 0.72 | 0.81 | 0.84 | 0.77 | 0.91 | 0.74 | 0.69 | 0.79 |
| 529 | 0.76 | 0.67 | 0.84 | 0.91 | 0.82 | 0.99 | 0.70 | 0.60 | 0.81 | 0.76 | 0.70 | 0.82 | 0.81 | 0.69 | 0.93 | 0.74 | 0.67 | 0.81 | 0.77 | 0.72 | 0.81 | 0.85 | 0.77 | 0.93 | 0.74 | 0.68 | 0.79 |
| 530 | 0.74 | 0.66 | 0.82 | 0.81 | 0.64 | 0.98 | 0.72 | 0.63 | 0.81 | 0.77 | 0.72 | 0.83 | 0.89 | 0.84 | 0.94 | 0.72 | 0.65 | 0.79 | 0.78 | 0.74 | 0.82 | 0.89 | 0.83 | 0.94 | 0.74 | 0.69 | 0.79 |
| 531 | 0.75 | 0.67 | 0.84 | 0.79 | 0.61 | 0.98 | 0.74 | 0.65 | 0.84 | 0.79 | 0.74 | 0.85 | 0.88 | 0.82 | 0.95 | 0.76 | 0.69 | 0.83 | 0.79 | 0.75 | 0.84 | 0.86 | 0.79 | 0.94 | 0.77 | 0.71 | 0.82 |
| 532 | 0.75 | 0.66 | 0.84 | 0.79 | 0.61 | 0.97 | 0.74 | 0.63 | 0.84 | 0.79 | 0.73 | 0.85 | 0.83 | 0.74 | 0.92 | 0.77 | 0.71 | 0.84 | 0.79 | 0.75 | 0.84 | 0.83 | 0.76 | 0.91 | 0.77 | 0.72 | 0.83 |
| 533 | 0.77 | 0.68 | 0.85 | 0.80 | 0.64 | 0.96 | 0.76 | 0.66 | 0.85 | 0.78 | 0.72 | 0.84 | 0.85 | 0.78 | 0.93 | 0.75 | 0.68 | 0.82 | 0.79 | 0.75 | 0.84 | 0.86 | 0.79 | 0.93 | 0.77 | 0.72 | 0.82 |
| 534 | 0.76 | 0.69 | 0.83 | 0.82 | 0.69 | 0.94 | 0.74 | 0.66 | 0.82 | 0.78 | 0.72 | 0.83 | 0.84 | 0.75 | 0.93 | 0.75 | 0.69 | 0.82 | 0.78 | 0.74 | 0.83 | 0.84 | 0.77 | 0.91 | 0.76 | 0.71 | 0.81 |
| 535 | 0.75 | 0.67 | 0.83 | 0.85 | 0.72 | 0.99 | 0.72 | 0.66 | 0.85 | 0.77 | 0.72 | 0.83 | 0.90 | 0.85 | 0.96 | 0.72 | 0.65 | 0.79 | 0.78 | 0.74 | 0.83 | 0.90 | 0.85 | 0.95 | 0.74 | 0.68 | 0.79 |
| 536 | 0.74 | 0.66 | 0.82 | 0.83 | 0.70 | 0.95 | 0.71 | 0.62 | 0.82 | 0.78 | 0.72 | 0.83 | 0.83 | 0.75 | 0.91 | 0.75 | 0.68 | 0.82 | 0.77 | 0.73 | 0.82 | 0.84 | 0.77 | 0.90 | 0.74 | 0.69 | 0.80 |
| 537 | 0.75 | 0.68 | 0.83 | 0.82 | 0.69 | 0.95 | 0.73 | 0.64 | 0.82 | 0.78 | 0.73 | 0.84 | 0.89 | 0.84 | 0.95 | 0.73 | 0.66 | 0.80 | 0.79 | 0.74 | 0.83 | 0.88 | 0.82 | 0.93 | 0.75 | 0.70 | 0.80 |
| 538 | 0.74 | 0.66 | 0.83 | 0.81 | 0.67 | 0.95 | 0.72 | 0.63 | 0.82 | 0.78 | 0.72 | 0.84 | 0.85 | 0.76 | 0.94 | 0.75 | 0.68 | 0.82 | 0.78 | 0.74 | 0.83 | 0.85 | 0.78 | 0.92 | 0.76 | 0.70 | 0.81 |
| 539 | 0.74 | 0.67 | 0.82 | 0.80 | 0.66 | 0.94 | 0.73 | 0.64 | 0.81 | 0.78 | 0.72 | 0.84 | 0.87 | 0.78 | 0.96 | 0.75 | 0.68 | 0.82 | 0.78 | 0.73 | 0.82 | 0.85 | 0.78 | 0.93 | 0.75 | 0.70 | 0.80 |
| 540 | 0.75 | 0.66 | 0.83 | 0.86 | 0.70 | 1.00 | 0.71 | 0.62 | 0.81 | 0.77 | 0.71 | 0.83 | 0.90 | 0.84 | 0.95 | 0.72 | 0.65 | 0.79 | 0.78 | 0.73 | 0.82 | 0.90 | 0.84 | 0.95 | 0.73 | 0.68 | 0.79 |
| 541 | 0.74 | 0.67 | 0.82 | 0.76 | 0.62 | 0.90 | 0.74 | 0.65 | 0.82 | 0.78 | 0.72 | 0.83 | 0.83 | 0.76 | 0.91 | 0.75 | 0.68 | 0.82 | 0.77 | 0.73 | 0.82 | 0.82 | 0.76 | 0.89 | 0.76 | 0.71 | 0.81 |
| 542 | 0.74 | 0.67 | 0.81 | 0.81 | 0.72 | 0.91 | 0.72 | 0.64 | 0.80 | 0.77 | 0.72 | 0.83 | 0.86 | 0.77 | 0.94 | 0.74 | 0.67 | 0.81 | 0.77 | 0.72 | 0.81 | 0.84 | 0.78 | 0.91 | 0.74 | 0.68 | 0.79 |
| 543 | 0.74 | 0.66 | 0.82 | 0.86 | 0.73 | 0.99 | 0.71 | 0.61 | 0.80 | 0.77 | 0.72 | 0.83 | 0.90 | 0.85 | 0.96 | 0.72 | 0.65 | 0.79 | 0.78 | 0.73 | 0.82 | 0.90 | 0.85 | 0.95 | 0.73 | 0.68 | 0.80 |
| 544 | 0.74 | 0.67 | 0.81 | 0.80 | 0.68 | 0.92 | 0.72 | 0.64 | 0.80 | 0.78 | 0.72 | 0.83 | 0.85 | 0.76 | 0.93 | 0.75 | 0.68 | 0.81 | 0.78 | 0.73 | 0.82 | 0.84 | 0.78 | 0.91 | 0.75 | 0.70 | 0.83 |
| 545 | 0.75 | 0.66 | 0.84 | 0.75 | 0.53 | 0.96 | 0.75 | 0.66 | 0.84 | 0.79 | 0.73 | 0.84 | 0.84 | 0.76 | 0.91 | 0.77 | 0.70 | 0.83 | 0.79 | 0.75 | 0.84 | 0.83 | 0.75 | 0.91 | 0.78 | 0.72 | 0.83 |
| 546 | 0.75 | 0.66 | 0.83 | 0.88 | 0.71 | 1.00 | 0.71 | 0.62 | 0.80 | 0.77 | 0.71 | 0.83 | 0.89 | 0.83 | 0.94 | 0.72 | 0.65 | 0.79 | 0.78 | 0.73 | 0.82 | 0.90 | 0.84 | 0.95 | 0.73 | 0.68 | 0.79 |
| 547 | 0.74 | 0.67 | 0.81 | 0.84 | 0.75 | 0.93 | 0.70 | 0.62 | 0.79 | 0.77 | 0.72 | 0.83 | 0.87 | 0.80 | 0.93 | 0.74 | 0.67 | 0.80 | 0.78 | 0.73 | 0.82 | 0.87 | 0.82 | 0.92 | 0.74 | 0.69 | 0.79 |
| 548 | 0.75 | 0.68 | 0.82 | 0.81 | 0.70 | 0.91 | 0.73 | 0.65 | 0.81 | 0.78 | 0.72 | 0.84 | 0.86 | 0.77 | 0.95 | 0.75 | 0.68 | 0.81 | 0.78 | 0.73 | 0.82 | 0.85 | 0.78 | 0.91 | 0.75 | 0.70 | 0.80 |
| 549 | 0.77 | 0.70 | 0.84 | 0.85 | 0.74 | 0.97 | 0.74 | 0.66 | 0.82 | 0.78 | 0.72 | 0.83 | 0.85 | 0.77 | 0.94 | 0.75 | 0.68 | 0.81 | 0.79 | 0.74 | 0.83 | 0.86 | 0.80 | 0.93 | 0.76 | 0.71 | 0.81 |
| 550 | 0.75 | 0.69 | 0.82 | 0.82 | 0.73 | 0.91 | 0.73 | 0.65 | 0.81 | 0.78 | 0.72 | 0.83 | 0.85 | 0.79 | 0.91 | 0.75 | 0.68 | 0.82 | 0.78 | 0.74 | 0.82 | 0.85 | 0.81 | 0.90 | 0.75 | 0.70 | 0.80 |
| 551 | 0.76 | 0.68 | 0.85 | 0.86 | 0.68 | 1.00 | 0.74 | 0.65 | 0.83 | 0.78 | 0.72 | 0.83 | 0.90 | 0.84 | 0.95 | 0.73 | 0.66 | 0.80 | 0.79 | 0.75 | 0.83 | 0.90 | 0.84 | 0.96 | 0.75 | 0.70 | 0.80 |
| 552 | 0.76 | 0.67 | 0.85 | 0.89 | 0.73 | 1.00 | 0.72 | 0.62 | 0.82 | 0.77 | 0.72 | 0.83 | 0.89 | 0.84 | 0.94 | 0.72 | 0.65 | 0.79 | 0.79 | 0.75 | 0.84 | 0.91 | 0.86 | 0.96 | 0.75 | 0.69 | 0.80 |
| 553 | 0.74 | 0.67 | 0.81 | 0.76 | 0.66 | 0.87 | 0.73 | 0.65 | 0.81 | 0.78 | 0.72 | 0.83 | 0.86 | 0.79 | 0.93 | 0.74 | 0.67 | 0.81 | 0.78 | 0.73 | 0.82 | 0.84 | 0.78 | 0.90 | 0.75 | 0.70 | 0.80 |
| 554 | 0.74 | 0.66 | 0.82 | 0.78 | 0.63 | 0.92 | 0.73 | 0.64 | 0.82 | 0.78 | 0.72 | 0.84 | 0.87 | 0.78 | 0.96 | 0.75 | 0.68 | 0.82 | 0.78 | 0.73 | 0.82 | 0.85 | 0.77 | 0.92 | 0.75 | 0.70 | 0.81 |
| 555 | 0.75 | 0.68 | 0.82 | 0.80 | 0.65 | 0.94 | 0.74 | 0.66 | 0.81 | 0.78 | 0.73 | 0.83 | 0.85 | 0.76 | 0.93 | 0.75 | 0.68 | 0.81 | 0.78 | 0.73 | 0.82 | 0.84 | 0.77 | 0.91 | 0.75 | 0.70 | 0.80 |
| 556 | 0.75 | 0.67 | 0.82 | 0.79 | 0.62 | 0.96 | 0.74 | 0.64 | 0.83 | 0.78 | 0.73 | 0.84 | 0.86 | 0.79 | 0.94 | 0.75 | 0.68 | 0.82 | 0.79 | 0.74 | 0.83 | 0.85 | 0.78 | 0.92 | 0.76 | 0.71 | 0.81 |
| 557 | 0.74 | 0.66 | 0.83 | 0.78 | 0.67 | 0.90 | 0.73 | 0.64 | 0.82 | 0.78 | 0.72 | 0.83 | 0.84 | 0.76 | 0.92 | 0.74 | 0.67 | 0.82 | 0.77 | 0.73 | 0.82 | 0.83 | 0.77 | 0.89 | 0.75 | 0.70 | 0.80 |
| 558 | 0.78 | 0.72 | 0.85 | 0.86 | 0.77 | 0.95 | 0.76 | 0.68 | 0.83 | 0.78 | 0.72 | 0.84 | 0.87 | 0.80 | 0.95 | 0.75 | 0.66 | 0.81 | 0.79 | 0.75 | 0.83 | 0.88 | 0.82 | 0.93 | 0.76 | 0.71 | 0.81 |
| 559 | 0.77 | 0.71 | 0.84 | 0.87 | 0.79 | 0.95 | 0.74 | 0.66 | 0.82 | 0.78 | 0.72 | 0.83 | 0.88 | 0.82 | 0.94 | 0.73 | 0.66 | 0.80 | 0.79 | 0.75 | 0.83 | 0.89 | 0.84 | 0.93 | 0.75 | 0.70 | 0.80 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 560 | 0.75 | 0.68 | 0.81 | 0.82 | 0.73 | 0.92 | 0.72 | 0.64 | 0.80 | 0.77 | 0.72 | 0.83 | 0.85 | 0.77 | 0.93 | 0.74 | 0.67 | 0.81 | 0.77 | 0.73 | 0.81 | 0.85 | 0.79 | 0.91 | 0.74 | 0.69 | 0.79 |
| 561 | 0.74 | 0.66 | 0.82 | 0.86 | 0.74 | 0.98 | 0.70 | 0.61 | 0.79 | 0.77 | 0.72 | 0.83 | 0.85 | 0.74 | 0.95 | 0.75 | 0.68 | 0.81 | 0.77 | 0.73 | 0.82 | 0.86 | 0.78 | 0.93 | 0.74 | 0.69 | 0.79 |
| 562 | 0.71 | 0.63 | 0.79 | 0.80 | 0.68 | 0.92 | 0.69 | 0.59 | 0.78 | 0.78 | 0.72 | 0.83 | 0.89 | 0.82 | 0.95 | 0.73 | 0.66 | 0.80 | 0.76 | 0.71 | 0.80 | 0.86 | 0.80 | 0.91 | 0.72 | 0.67 | 0.77 |
| 563 | 0.76 | 0.68 | 0.84 | 0.89 | 0.76 | 1.00 | 0.73 | 0.63 | 0.82 | 0.77 | 0.72 | 0.83 | 0.91 | 0.86 | 0.95 | 0.71 | 0.64 | 0.79 | 0.79 | 0.74 | 0.83 | 0.92 | 0.87 | 0.96 | 0.74 | 0.68 | 0.79 |
| 564 | 0.78 | 0.69 | 0.86 | 0.84 | 0.73 | 0.96 | 0.75 | 0.65 | 0.85 | 0.78 | 0.72 | 0.84 | 0.87 | 0.80 | 0.94 | 0.74 | 0.67 | 0.82 | 0.79 | 0.74 | 0.84 | 0.87 | 0.81 | 0.93 | 0.76 | 0.70 | 0.82 |
| 565 | 0.75 | 0.68 | 0.82 | 0.82 | 0.69 | 0.95 | 0.73 | 0.64 | 0.81 | 0.78 | 0.72 | 0.83 | 0.84 | 0.76 | 0.93 | 0.75 | 0.68 | 0.81 | 0.77 | 0.73 | 0.82 | 0.84 | 0.77 | 0.91 | 0.75 | 0.70 | 0.80 |
| 566 | 0.74 | 0.65 | 0.82 | 0.87 | 0.71 | 1.00 | 0.70 | 0.60 | 0.80 | 0.77 | 0.71 | 0.83 | 0.89 | 0.84 | 0.94 | 0.72 | 0.65 | 0.79 | 0.78 | 0.74 | 0.83 | 0.90 | 0.85 | 0.95 | 0.74 | 0.68 | 0.79 |
| 567 | 0.74 | 0.67 | 0.82 | 0.79 | 0.65 | 0.93 | 0.73 | 0.64 | 0.81 | 0.78 | 0.72 | 0.83 | 0.85 | 0.78 | 0.92 | 0.74 | 0.68 | 0.81 | 0.78 | 0.73 | 0.82 | 0.84 | 0.78 | 0.90 | 0.75 | 0.70 | 0.80 |
| 568 | 0.74 | 0.66 | 0.81 | 0.79 | 0.65 | 0.93 | 0.72 | 0.64 | 0.81 | 0.77 | 0.72 | 0.83 | 0.85 | 0.78 | 0.92 | 0.74 | 0.67 | 0.81 | 0.77 | 0.73 | 0.81 | 0.84 | 0.78 | 0.90 | 0.74 | 0.69 | 0.79 |
| 569 | 0.72 | 0.63 | 0.80 | 0.76 | 0.60 | 0.92 | 0.70 | 0.60 | 0.80 | 0.78 | 0.72 | 0.84 | 0.86 | 0.80 | 0.93 | 0.74 | 0.67 | 0.82 | 0.76 | 0.71 | 0.81 | 0.83 | 0.76 | 0.90 | 0.73 | 0.67 | 0.79 |
| 570 | 0.76 | 0.68 | 0.83 | 0.90 | 0.82 | 0.98 | 0.71 | 0.62 | 0.80 | 0.76 | 0.70 | 0.82 | 0.80 | 0.71 | 0.90 | 0.74 | 0.67 | 0.81 | 0.77 | 0.72 | 0.81 | 0.84 | 0.77 | 0.91 | 0.74 | 0.68 | 0.79 |
| 571 | 0.75 | 0.68 | 0.82 | 0.82 | 0.71 | 0.92 | 0.73 | 0.65 | 0.81 | 0.78 | 0.72 | 0.84 | 0.86 | 0.77 | 0.95 | 0.74 | 0.67 | 0.81 | 0.78 | 0.73 | 0.82 | 0.85 | 0.79 | 0.92 | 0.75 | 0.70 | 0.80 |
| 572 | 0.74 | 0.66 | 0.82 | 0.81 | 0.65 | 0.97 | 0.72 | 0.63 | 0.81 | 0.78 | 0.73 | 0.84 | 0.87 | 0.80 | 0.94 | 0.75 | 0.68 | 0.82 | 0.78 | 0.74 | 0.83 | 0.86 | 0.79 | 0.93 | 0.76 | 0.70 | 0.81 |
| 573 | 0.75 | 0.66 | 0.84 | 0.76 | 0.57 | 0.96 | 0.74 | 0.65 | 0.84 | 0.79 | 0.74 | 0.84 | 0.84 | 0.75 | 0.92 | 0.77 | 0.71 | 0.83 | 0.79 | 0.75 | 0.84 | 0.83 | 0.75 | 0.91 | 0.78 | 0.73 | 0.83 |
| 574 | 0.75 | 0.67 | 0.84 | 0.78 | 0.62 | 0.94 | 0.75 | 0.65 | 0.84 | 0.78 | 0.72 | 0.84 | 0.86 | 0.78 | 0.93 | 0.75 | 0.68 | 0.81 | 0.79 | 0.75 | 0.83 | 0.85 | 0.78 | 0.92 | 0.77 | 0.72 | 0.82 |
| 575 | 0.76 | 0.68 | 0.85 | 0.82 | 0.66 | 0.98 | 0.74 | 0.65 | 0.84 | 0.79 | 0.73 | 0.85 | 0.86 | 0.77 | 0.95 | 0.76 | 0.69 | 0.83 | 0.79 | 0.75 | 0.84 | 0.86 | 0.78 | 0.93 | 0.77 | 0.71 | 0.82 |
| 576 | 0.74 | 0.66 | 0.81 | 0.79 | 0.64 | 0.93 | 0.72 | 0.63 | 0.81 | 0.78 | 0.72 | 0.84 | 0.87 | 0.78 | 0.95 | 0.75 | 0.68 | 0.82 | 0.78 | 0.73 | 0.82 | 0.85 | 0.77 | 0.92 | 0.75 | 0.70 | 0.80 |
| 577 | 0.74 | 0.66 | 0.82 | 0.88 | 0.78 | 0.98 | 0.69 | 0.60 | 0.78 | 0.77 | 0.71 | 0.83 | 0.80 | 0.69 | 0.92 | 0.76 | 0.69 | 0.83 | 0.77 | 0.72 | 0.82 | 0.84 | 0.76 | 0.92 | 0.74 | 0.69 | 0.80 |
| 578 | 0.75 | 0.67 | 0.84 | 0.80 | 0.64 | 0.96 | 0.74 | 0.64 | 0.84 | 0.78 | 0.72 | 0.84 | 0.86 | 0.79 | 0.93 | 0.75 | 0.68 | 0.82 | 0.79 | 0.75 | 0.84 | 0.86 | 0.79 | 0.93 | 0.76 | 0.71 | 0.82 |
| 579 | 0.74 | 0.67 | 0.82 | 0.80 | 0.68 | 0.92 | 0.72 | 0.64 | 0.81 | 0.78 | 0.72 | 0.84 | 0.87 | 0.78 | 0.96 | 0.74 | 0.67 | 0.81 | 0.78 | 0.73 | 0.82 | 0.85 | 0.78 | 0.92 | 0.75 | 0.70 | 0.80 |
| 580 | 0.76 | 0.68 | 0.83 | 0.82 | 0.70 | 0.95 | 0.74 | 0.65 | 0.82 | 0.76 | 0.70 | 0.82 | 0.82 | 0.73 | 0.90 | 0.74 | 0.67 | 0.81 | 0.77 | 0.73 | 0.82 | 0.83 | 0.76 | 0.89 | 0.75 | 0.70 | 0.80 |
| 581 | 0.75 | 0.67 | 0.83 | 0.89 | 0.76 | 1.00 | 0.71 | 0.62 | 0.80 | 0.78 | 0.73 | 0.84 | 0.89 | 0.84 | 0.94 | 0.73 | 0.66 | 0.80 | 0.79 | 0.75 | 0.83 | 0.91 | 0.86 | 0.96 | 0.74 | 0.69 | 0.80 |
| 582 | 0.76 | 0.68 | 0.84 | 0.84 | 0.72 | 0.96 | 0.73 | 0.64 | 0.83 | 0.76 | 0.70 | 0.82 | 0.84 | 0.75 | 0.93 | 0.72 | 0.65 | 0.80 | 0.77 | 0.73 | 0.82 | 0.85 | 0.78 | 0.92 | 0.74 | 0.69 | 0.80 |
| 583 | 0.76 | 0.69 | 0.83 | 0.81 | 0.70 | 0.92 | 0.74 | 0.66 | 0.83 | 0.78 | 0.72 | 0.83 | 0.85 | 0.79 | 0.92 | 0.74 | 0.67 | 0.81 | 0.78 | 0.74 | 0.82 | 0.85 | 0.80 | 0.90 | 0.75 | 0.70 | 0.80 |
| 584 | 0.75 | 0.67 | 0.83 | 0.86 | 0.68 | 1.00 | 0.72 | 0.64 | 0.81 | 0.78 | 0.72 | 0.83 | 0.88 | 0.82 | 0.94 | 0.73 | 0.67 | 0.80 | 0.79 | 0.75 | 0.83 | 0.89 | 0.84 | 0.95 | 0.75 | 0.70 | 0.80 |
| 585 | 0.76 | 0.68 | 0.84 | 0.82 | 0.67 | 0.97 | 0.74 | 0.64 | 0.84 | 0.78 | 0.72 | 0.84 | 0.88 | 0.81 | 0.95 | 0.74 | 0.67 | 0.81 | 0.78 | 0.73 | 0.83 | 0.87 | 0.80 | 0.93 | 0.75 | 0.69 | 0.80 |
| 586 | 0.76 | 0.68 | 0.84 | 0.87 | 0.69 | 1.00 | 0.73 | 0.64 | 0.82 | 0.78 | 0.72 | 0.83 | 0.90 | 0.85 | 0.96 | 0.72 | 0.65 | 0.80 | 0.79 | 0.74 | 0.83 | 0.91 | 0.85 | 0.97 | 0.74 | 0.69 | 0.80 |
| 587 | 0.74 | 0.66 | 0.82 | 0.81 | 0.64 | 0.98 | 0.72 | 0.63 | 0.81 | 0.77 | 0.71 | 0.83 | 0.89 | 0.84 | 0.95 | 0.72 | 0.65 | 0.79 | 0.78 | 0.73 | 0.82 | 0.89 | 0.83 | 0.94 | 0.73 | 0.68 | 0.79 |
| 588 | 0.74 | 0.66 | 0.81 | 0.79 | 0.67 | 0.92 | 0.72 | 0.63 | 0.80 | 0.77 | 0.72 | 0.83 | 0.84 | 0.76 | 0.92 | 0.75 | 0.68 | 0.82 | 0.77 | 0.73 | 0.82 | 0.84 | 0.77 | 0.90 | 0.75 | 0.70 | 0.80 |
| 589 | 0.75 | 0.68 | 0.82 | 0.81 | 0.72 | 0.91 | 0.73 | 0.65 | 0.81 | 0.78 | 0.72 | 0.83 | 0.86 | 0.80 | 0.92 | 0.74 | 0.67 | 0.81 | 0.78 | 0.74 | 0.82 | 0.86 | 0.81 | 0.91 | 0.75 | 0.70 | 0.80 |
| 590 | 0.74 | 0.65 | 0.82 | 0.85 | 0.69 | 1.00 | 0.70 | 0.61 | 0.79 | 0.77 | 0.72 | 0.83 | 0.88 | 0.82 | 0.94 | 0.72 | 0.66 | 0.79 | 0.78 | 0.74 | 0.83 | 0.89 | 0.84 | 0.95 | 0.74 | 0.69 | 0.79 |
| 591 | 0.76 | 0.68 | 0.85 | 0.86 | 0.68 | 1.00 | 0.73 | 0.64 | 0.83 | 0.77 | 0.72 | 0.83 | 0.90 | 0.85 | 0.95 | 0.72 | 0.65 | 0.79 | 0.78 | 0.74 | 0.83 | 0.90 | 0.85 | 0.95 | 0.74 | 0.69 | 0.79 |
| 592 | 0.74 | 0.67 | 0.81 | 0.81 | 0.67 | 0.96 | 0.72 | 0.65 | 0.80 | 0.78 | 0.72 | 0.83 | 0.86 | 0.78 | 0.93 | 0.75 | 0.68 | 0.81 | 0.77 | 0.73 | 0.81 | 0.85 | 0.78 | 0.91 | 0.74 | 0.69 | 0.79 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 593 | 0.76 | 0.69 | 0.83 | 0.87 | 0.79 | 0.95 | 0.73 | 0.65 | 0.81 | 0.78 | 0.72 | 0.83 | 0.87 | 0.81 | 0.94 | 0.73 | 0.66 | 0.80 | 0.78 | 0.74 | 0.83 | 0.88 | 0.84 | 0.93 | 0.75 | 0.70 | 0.80 |
| 594 | 0.77 | 0.69 | 0.85 | 0.87 | 0.73 | 1.00 | 0.74 | 0.65 | 0.83 | 0.78 | 0.72 | 0.84 | 0.87 | 0.79 | 0.95 | 0.74 | 0.67 | 0.81 | 0.79 | 0.75 | 0.83 | 0.88 | 0.82 | 0.94 | 0.76 | 0.71 | 0.81 |
| 595 | 0.74 | 0.66 | 0.82 | 0.80 | 0.64 | 0.97 | 0.72 | 0.63 | 0.81 | 0.77 | 0.71 | 0.83 | 0.89 | 0.84 | 0.95 | 0.71 | 0.64 | 0.78 | 0.78 | 0.73 | 0.82 | 0.88 | 0.82 | 0.94 | 0.74 | 0.69 | 0.79 |
| 596 | 0.74 | 0.67 | 0.82 | 0.78 | 0.63 | 0.93 | 0.73 | 0.64 | 0.82 | 0.78 | 0.72 | 0.84 | 0.86 | 0.77 | 0.95 | 0.74 | 0.68 | 0.81 | 0.78 | 0.73 | 0.82 | 0.84 | 0.77 | 0.92 | 0.76 | 0.70 | 0.81 |
| 597 | 0.75 | 0.68 | 0.82 | 0.86 | 0.78 | 0.95 | 0.72 | 0.63 | 0.80 | 0.77 | 0.71 | 0.83 | 0.86 | 0.79 | 0.92 | 0.73 | 0.66 | 0.80 | 0.76 | 0.72 | 0.81 | 0.86 | 0.80 | 0.91 | 0.73 | 0.67 | 0.78 |
| 598 | 0.77 | 0.68 | 0.85 | 0.88 | 0.73 | 1.00 | 0.73 | 0.63 | 0.83 | 0.77 | 0.71 | 0.83 | 0.88 | 0.83 | 0.94 | 0.72 | 0.65 | 0.79 | 0.79 | 0.75 | 0.84 | 0.90 | 0.85 | 0.95 | 0.75 | 0.69 | 0.80 |
| 599 | 0.74 | 0.67 | 0.81 | 0.83 | 0.70 | 0.95 | 0.71 | 0.63 | 0.79 | 0.78 | 0.72 | 0.83 | 0.87 | 0.80 | 0.94 | 0.74 | 0.67 | 0.81 | 0.78 | 0.73 | 0.82 | 0.87 | 0.81 | 0.93 | 0.74 | 0.69 | 0.79 |
| 600 | 0.75 | 0.67 | 0.84 | 0.87 | 0.71 | 1.00 | 0.72 | 0.62 | 0.82 | 0.77 | 0.71 | 0.83 | 0.89 | 0.83 | 0.94 | 0.72 | 0.65 | 0.79 | 0.78 | 0.74 | 0.83 | 0.90 | 0.84 | 0.95 | 0.74 | 0.68 | 0.79 |
| 601 | 0.76 | 0.67 | 0.85 | 0.90 | 0.77 | 1.00 | 0.72 | 0.61 | 0.83 | 0.76 | 0.69 | 0.82 | 0.85 | 0.74 | 0.95 | 0.72 | 0.65 | 0.80 | 0.78 | 0.73 | 0.83 | 0.88 | 0.80 | 0.95 | 0.75 | 0.69 | 0.80 |
| 602 | 0.75 | 0.68 | 0.83 | 0.85 | 0.73 | 0.96 | 0.72 | 0.64 | 0.81 | 0.78 | 0.72 | 0.83 | 0.86 | 0.79 | 0.94 | 0.74 | 0.67 | 0.80 | 0.78 | 0.74 | 0.82 | 0.86 | 0.80 | 0.93 | 0.75 | 0.70 | 0.80 |
| 603 | 0.75 | 0.68 | 0.82 | 0.81 | 0.70 | 0.92 | 0.73 | 0.64 | 0.81 | 0.77 | 0.72 | 0.83 | 0.86 | 0.77 | 0.94 | 0.74 | 0.67 | 0.81 | 0.77 | 0.73 | 0.82 | 0.85 | 0.78 | 0.91 | 0.75 | 0.70 | 0.80 |
| 604 | 0.77 | 0.69 | 0.85 | 0.88 | 0.72 | 1.00 | 0.74 | 0.64 | 0.83 | 0.78 | 0.73 | 0.84 | 0.87 | 0.81 | 0.93 | 0.75 | 0.68 | 0.81 | 0.80 | 0.76 | 0.84 | 0.90 | 0.85 | 0.95 | 0.76 | 0.71 | 0.82 |
| 605 | 0.77 | 0.70 | 0.85 | 0.88 | 0.79 | 0.98 | 0.74 | 0.64 | 0.83 | 0.78 | 0.72 | 0.84 | 0.88 | 0.81 | 0.94 | 0.74 | 0.67 | 0.81 | 0.79 | 0.74 | 0.83 | 0.89 | 0.84 | 0.94 | 0.75 | 0.69 | 0.80 |
| 606 | 0.77 | 0.70 | 0.85 | 0.86 | 0.74 | 0.98 | 0.75 | 0.66 | 0.83 | 0.78 | 0.72 | 0.83 | 0.84 | 0.75 | 0.93 | 0.75 | 0.68 | 0.81 | 0.79 | 0.75 | 0.83 | 0.86 | 0.79 | 0.92 | 0.76 | 0.71 | 0.81 |
| 607 | 0.73 | 0.64 | 0.82 | 0.81 | 0.65 | 0.97 | 0.71 | 0.60 | 0.81 | 0.76 | 0.70 | 0.82 | 0.87 | 0.79 | 0.95 | 0.72 | 0.64 | 0.79 | 0.77 | 0.72 | 0.82 | 0.86 | 0.79 | 0.93 | 0.73 | 0.68 | 0.79 |
| 608 | 0.75 | 0.68 | 0.82 | 0.82 | 0.73 | 0.91 | 0.72 | 0.64 | 0.81 | 0.78 | 0.72 | 0.83 | 0.84 | 0.77 | 0.91 | 0.75 | 0.68 | 0.82 | 0.78 | 0.73 | 0.82 | 0.85 | 0.80 | 0.90 | 0.75 | 0.70 | 0.80 |
| 609 | 0.73 | 0.66 | 0.81 | 0.85 | 0.73 | 0.97 | 0.70 | 0.61 | 0.79 | 0.77 | 0.71 | 0.83 | 0.86 | 0.76 | 0.95 | 0.74 | 0.67 | 0.81 | 0.76 | 0.72 | 0.81 | 0.86 | 0.79 | 0.93 | 0.73 | 0.68 | 0.78 |
| 610 | 0.77 | 0.68 | 0.85 | 0.86 | 0.76 | 0.97 | 0.73 | 0.63 | 0.83 | 0.76 | 0.69 | 0.82 | 0.81 | 0.70 | 0.92 | 0.74 | 0.66 | 0.81 | 0.76 | 0.72 | 0.81 | 0.83 | 0.75 | 0.91 | 0.74 | 0.68 | 0.80 |
| 611 | 0.74 | 0.67 | 0.82 | 0.85 | 0.74 | 0.96 | 0.71 | 0.62 | 0.79 | 0.77 | 0.71 | 0.83 | 0.85 | 0.76 | 0.94 | 0.74 | 0.68 | 0.81 | 0.77 | 0.72 | 0.81 | 0.86 | 0.79 | 0.92 | 0.73 | 0.68 | 0.78 |
| 612 | 0.75 | 0.66 | 0.84 | 0.76 | 0.56 | 0.96 | 0.75 | 0.65 | 0.84 | 0.79 | 0.74 | 0.85 | 0.86 | 0.79 | 0.93 | 0.76 | 0.70 | 0.83 | 0.80 | 0.75 | 0.84 | 0.85 | 0.77 | 0.92 | 0.78 | 0.73 | 0.83 |
| 613 | 0.74 | 0.67 | 0.81 | 0.79 | 0.67 | 0.92 | 0.72 | 0.65 | 0.80 | 0.77 | 0.72 | 0.83 | 0.85 | 0.77 | 0.93 | 0.74 | 0.68 | 0.81 | 0.77 | 0.73 | 0.82 | 0.84 | 0.77 | 0.90 | 0.75 | 0.70 | 0.80 |
| 614 | 0.74 | 0.65 | 0.82 | 0.87 | 0.72 | 1.00 | 0.69 | 0.60 | 0.79 | 0.77 | 0.71 | 0.83 | 0.88 | 0.82 | 0.94 | 0.72 | 0.65 | 0.79 | 0.78 | 0.73 | 0.82 | 0.90 | 0.84 | 0.95 | 0.73 | 0.68 | 0.79 |
| 615 | 0.78 | 0.71 | 0.84 | 0.86 | 0.75 | 0.98 | 0.75 | 0.67 | 0.83 | 0.78 | 0.72 | 0.83 | 0.86 | 0.78 | 0.95 | 0.74 | 0.67 | 0.81 | 0.79 | 0.74 | 0.83 | 0.87 | 0.81 | 0.93 | 0.75 | 0.71 | 0.80 |
| 616 | 0.74 | 0.67 | 0.82 | 0.83 | 0.71 | 0.95 | 0.71 | 0.62 | 0.80 | 0.77 | 0.71 | 0.83 | 0.85 | 0.75 | 0.95 | 0.74 | 0.68 | 0.81 | 0.77 | 0.72 | 0.81 | 0.85 | 0.78 | 0.92 | 0.74 | 0.69 | 0.79 |
| 617 | 0.74 | 0.67 | 0.82 | 0.83 | 0.72 | 0.93 | 0.71 | 0.62 | 0.80 | 0.77 | 0.71 | 0.83 | 0.82 | 0.73 | 0.90 | 0.75 | 0.68 | 0.82 | 0.77 | 0.72 | 0.81 | 0.83 | 0.76 | 0.89 | 0.74 | 0.69 | 0.80 |
| 618 | 0.76 | 0.69 | 0.83 | 0.83 | 0.73 | 0.92 | 0.73 | 0.66 | 0.81 | 0.77 | 0.71 | 0.83 | 0.86 | 0.77 | 0.95 | 0.74 | 0.67 | 0.81 | 0.78 | 0.73 | 0.82 | 0.86 | 0.79 | 0.92 | 0.75 | 0.70 | 0.80 |
| 619 | 0.76 | 0.68 | 0.84 | 0.89 | 0.77 | 1.00 | 0.72 | 0.63 | 0.82 | 0.77 | 0.71 | 0.83 | 0.91 | 0.86 | 0.95 | 0.71 | 0.64 | 0.78 | 0.79 | 0.74 | 0.83 | 0.91 | 0.87 | 0.96 | 0.74 | 0.68 | 0.79 |
| 620 | 0.76 | 0.69 | 0.82 | 0.84 | 0.72 | 0.95 | 0.73 | 0.65 | 0.81 | 0.77 | 0.71 | 0.83 | 0.83 | 0.71 | 0.94 | 0.75 | 0.69 | 0.82 | 0.77 | 0.73 | 0.82 | 0.83 | 0.75 | 0.92 | 0.75 | 0.70 | 0.80 |
| 621 | 0.77 | 0.69 | 0.84 | 0.85 | 0.73 | 0.97 | 0.74 | 0.66 | 0.82 | 0.77 | 0.72 | 0.83 | 0.85 | 0.77 | 0.93 | 0.74 | 0.68 | 0.81 | 0.79 | 0.74 | 0.83 | 0.86 | 0.80 | 0.93 | 0.76 | 0.71 | 0.81 |
| 622 | 0.76 | 0.68 | 0.83 | 0.86 | 0.74 | 0.99 | 0.72 | 0.63 | 0.81 | 0.77 | 0.71 | 0.83 | 0.88 | 0.79 | 0.97 | 0.73 | 0.66 | 0.80 | 0.77 | 0.72 | 0.81 | 0.87 | 0.80 | 0.94 | 0.73 | 0.68 | 0.79 |
| 623 | 0.76 | 0.68 | 0.84 | 0.80 | 0.62 | 0.98 | 0.75 | 0.66 | 0.84 | 0.79 | 0.73 | 0.84 | 0.84 | 0.75 | 0.93 | 0.76 | 0.70 | 0.83 | 0.79 | 0.74 | 0.83 | 0.84 | 0.76 | 0.91 | 0.77 | 0.72 | 0.82 |
| 624 | 0.75 | 0.68 | 0.82 | 0.80 | 0.67 | 0.94 | 0.74 | 0.66 | 0.81 | 0.77 | 0.72 | 0.83 | 0.85 | 0.75 | 0.94 | 0.74 | 0.68 | 0.81 | 0.78 | 0.74 | 0.82 | 0.84 | 0.77 | 0.91 | 0.76 | 0.71 | 0.81 |
| 625 | 0.75 | 0.69 | 0.82 | 0.82 | 0.71 | 0.92 | 0.74 | 0.65 | 0.82 | 0.77 | 0.71 | 0.83 | 0.85 | 0.77 | 0.94 | 0.74 | 0.67 | 0.81 | 0.78 | 0.73 | 0.82 | 0.85 | 0.78 | 0.91 | 0.75 | 0.70 | 0.80 |

108

| No. | 626 | 627 | 628 | 629 | 630 | 631 | 632 | 633 | 634 | 635 | 636 | 637 | 638 | 639 | 640 | 641 | 642 | 643 | 644 | 645 | 646 | 647 | 648 | 649 | 650 | 651 | 652 | 653 | 654 | 655 | 656 | 657 | 658 |
|---|---|---|---|---|---|---|---|---|---|---|---|---|---|---|---|---|---|---|---|---|---|---|---|---|---|---|---|---|---|---|---|---|---|
| G | 0.77 | 0.75 | 0.75 | 0.77 | 0.74 | 0.73 | 0.75 | 0.75 | 0.74 | 0.75 | 0.73 | 0.77 | 0.75 | 0.75 | 0.74 | 0.74 | 0.74 | 0.74 | 0.73 | 0.74 | 0.75 | 0.78 | 0.74 | 0.74 | 0.73 | 0.76 | 0.75 | 0.74 | 0.75 | 0.74 | 0.74 | 0.74 | 0.77 |
| H | 0.69 | 0.67 | 0.67 | 0.69 | 0.67 | 0.66 | 0.67 | 0.66 | 0.66 | 0.66 | 0.65 | 0.69 | 0.67 | 0.67 | 0.66 | 0.66 | 0.65 | 0.65 | 0.66 | 0.67 | 0.66 | 0.70 | 0.66 | 0.66 | 0.64 | 0.68 | 0.68 | 0.67 | 0.67 | 0.65 | 0.66 | 0.65 | 0.69 |
| I | 0.85 | 0.83 | 0.84 | 0.85 | 0.82 | 0.80 | 0.83 | 0.83 | 0.82 | 0.84 | 0.82 | 0.85 | 0.83 | 0.82 | 0.82 | 0.83 | 0.82 | 0.82 | 0.80 | 0.81 | 0.84 | 0.86 | 0.81 | 0.82 | 0.82 | 0.83 | 0.81 | 0.82 | 0.84 | 0.83 | 0.82 | 0.83 | 0.84 |
| J | 0.85 | 0.78 | 0.83 | 0.85 | 0.82 | 0.85 | 0.82 | 0.87 | 0.79 | 0.87 | 0.79 | 0.85 | 0.85 | 0.85 | 0.86 | 0.76 | 0.87 | 0.79 | 0.83 | 0.80 | 0.79 | 0.85 | 0.82 | 0.81 | 0.81 | 0.85 | 0.81 | 0.85 | 0.88 | 0.81 | 0.81 | 0.87 | 0.87 |
| K | 0.71 | 0.61 | 0.73 | 0.73 | 0.70 | 0.77 | 0.65 | 0.72 | 0.65 | 0.71 | 0.61 | 0.73 | 0.72 | 0.71 | 0.72 | 0.57 | 0.71 | 0.65 | 0.73 | 0.68 | 0.62 | 0.73 | 0.70 | 0.63 | 0.65 | 0.75 | 0.71 | 0.70 | 0.75 | 0.67 | 0.64 | 0.72 | 0.79 |
| L | 0.99 | 0.95 | 0.93 | 0.97 | 0.93 | 0.92 | 1.00 | 1.00 | 0.94 | 1.00 | 0.97 | 0.97 | 0.97 | 0.98 | 1.00 | 0.96 | 1.00 | 0.93 | 0.93 | 0.91 | 0.96 | 0.98 | 0.94 | 0.98 | 0.96 | 0.95 | 0.91 | 0.99 | 1.00 | 0.94 | 0.99 | 1.00 | 0.95 |
| M | 0.74 | 0.74 | 0.73 | 0.74 | 0.72 | 0.69 | 0.73 | 0.71 | 0.72 | 0.72 | 0.72 | 0.74 | 0.72 | 0.72 | 0.71 | 0.74 | 0.70 | 0.72 | 0.70 | 0.73 | 0.74 | 0.75 | 0.71 | 0.72 | 0.70 | 0.73 | 0.73 | 0.71 | 0.72 | 0.72 | 0.72 | 0.70 | 0.73 |
| N | 0.65 | 0.65 | 0.62 | 0.64 | 0.63 | 0.61 | 0.64 | 0.61 | 0.63 | 0.62 | 0.62 | 0.64 | 0.63 | 0.64 | 0.61 | 0.65 | 0.61 | 0.62 | 0.62 | 0.64 | 0.64 | 0.65 | 0.62 | 0.63 | 0.59 | 0.64 | 0.65 | 0.63 | 0.62 | 0.62 | 0.63 | 0.60 | 0.65 |
| O | 0.83 | 0.84 | 0.84 | 0.84 | 0.80 | 0.78 | 0.81 | 0.80 | 0.81 | 0.81 | 0.81 | 0.84 | 0.82 | 0.80 | 0.80 | 0.83 | 0.80 | 0.82 | 0.78 | 0.81 | 0.84 | 0.84 | 0.80 | 0.81 | 0.81 | 0.82 | 0.80 | 0.80 | 0.81 | 0.82 | 0.81 | 0.80 | 0.82 |
| P | 0.77 | 0.79 | 0.77 | 0.78 | 0.77 | 0.77 | 0.77 | 0.77 | 0.78 | 0.77 | 0.78 | 0.78 | 0.77 | 0.77 | 0.75 | 0.77 | 0.79 | 0.77 | 0.77 | 0.77 | 0.78 | 0.78 | 0.77 | 0.77 | 0.76 | 0.77 | 0.77 | 0.77 | 0.77 | 0.77 | 0.77 | 0.77 | 0.77 |
| Q | 0.72 | 0.73 | 0.71 | 0.72 | 0.71 | 0.71 | 0.71 | 0.71 | 0.72 | 0.71 | 0.72 | 0.72 | 0.71 | 0.69 | 0.71 | 0.73 | 0.71 | 0.72 | 0.71 | 0.72 | 0.72 | 0.72 | 0.71 | 0.71 | 0.69 | 0.72 | 0.71 | 0.71 | 0.72 | 0.71 | 0.71 | 0.71 | 0.71 |
| R | 0.83 | 0.84 | 0.83 | 0.84 | 0.83 | 0.83 | 0.83 | 0.82 | 0.84 | 0.83 | 0.84 | 0.84 | 0.82 | 0.81 | 0.83 | 0.84 | 0.83 | 0.83 | 0.83 | 0.83 | 0.84 | 0.84 | 0.83 | 0.83 | 0.82 | 0.83 | 0.83 | 0.83 | 0.83 | 0.83 | 0.83 | 0.83 | 0.83 |
| S | 0.85 | 0.85 | 0.84 | 0.89 | 0.81 | 0.85 | 0.89 | 0.88 | 0.87 | 0.89 | 0.85 | 0.88 | 0.85 | 0.81 | 0.90 | 0.83 | 0.90 | 0.85 | 0.84 | 0.85 | 0.87 | 0.87 | 0.84 | 0.89 | 0.82 | 0.84 | 0.85 | 0.87 | 0.90 | 0.86 | 0.89 | 0.87 | 0.85 |
| T | 0.78 | 0.76 | 0.76 | 0.83 | 0.73 | 0.78 | 0.84 | 0.81 | 0.78 | 0.83 | 0.78 | 0.82 | 0.77 | 0.72 | 0.85 | 0.75 | 0.85 | 0.77 | 0.76 | 0.76 | 0.81 | 0.79 | 0.76 | 0.84 | 0.71 | 0.76 | 0.77 | 0.80 | 0.86 | 0.79 | 0.83 | 0.80 | 0.77 |
| U | 0.92 | 0.94 | 0.91 | 0.95 | 0.90 | 0.92 | 0.95 | 0.96 | 0.95 | 0.94 | 0.92 | 0.94 | 0.92 | 0.89 | 0.95 | 0.91 | 0.95 | 0.93 | 0.93 | 0.93 | 0.93 | 0.94 | 0.92 | 0.94 | 0.93 | 0.93 | 0.93 | 0.94 | 0.95 | 0.93 | 0.94 | 0.93 | 0.93 |
| V | 0.74 | 0.76 | 0.74 | 0.73 | 0.75 | 0.74 | 0.72 | 0.72 | 0.74 | 0.72 | 0.75 | 0.73 | 0.73 | 0.72 | 0.71 | 0.77 | 0.71 | 0.74 | 0.74 | 0.74 | 0.74 | 0.74 | 0.74 | 0.72 | 0.73 | 0.74 | 0.73 | 0.73 | 0.71 | 0.73 | 0.72 | 0.73 | 0.73 |
| W | 0.67 | 0.69 | 0.67 | 0.66 | 0.68 | 0.67 | 0.65 | 0.65 | 0.67 | 0.65 | 0.68 | 0.66 | 0.67 | 0.65 | 0.64 | 0.71 | 0.64 | 0.68 | 0.67 | 0.67 | 0.67 | 0.67 | 0.68 | 0.65 | 0.66 | 0.68 | 0.67 | 0.66 | 0.64 | 0.67 | 0.65 | 0.66 | 0.67 |
| Z | 0.81 | 0.83 | 0.82 | 0.81 | 0.82 | 0.81 | 0.79 | 0.78 | 0.81 | 0.79 | 0.82 | 0.81 | 0.80 | 0.79 | 0.79 | 0.83 | 0.78 | 0.81 | 0.81 | 0.81 | 0.81 | 0.81 | 0.81 | 0.79 | 0.81 | 0.81 | 0.80 | 0.80 | 0.78 | 0.80 | 0.79 | 0.80 | 0.80 |
| AA | 0.77 | 0.79 | 0.78 | 0.79 | 0.76 | 0.75 | 0.78 | 0.78 | 0.77 | 0.78 | 0.76 | 0.79 | 0.76 | 0.76 | 0.78 | 0.79 | 0.77 | 0.78 | 0.77 | 0.78 | 0.78 | 0.79 | 0.77 | 0.78 | 0.77 | 0.78 | 0.77 | 0.77 | 0.78 | 0.78 | 0.78 | 0.78 | 0.77 |
| AB | 0.72 | 0.74 | 0.73 | 0.74 | 0.72 | 0.71 | 0.74 | 0.73 | 0.73 | 0.74 | 0.72 | 0.74 | 0.72 | 0.71 | 0.73 | 0.75 | 0.73 | 0.73 | 0.72 | 0.73 | 0.73 | 0.74 | 0.72 | 0.74 | 0.72 | 0.74 | 0.73 | 0.73 | 0.74 | 0.73 | 0.73 | 0.73 | 0.73 |
| AC | 0.81 | 0.83 | 0.82 | 0.83 | 0.81 | 0.80 | 0.82 | 0.82 | 0.82 | 0.83 | 0.81 | 0.83 | 0.81 | 0.80 | 0.82 | 0.83 | 0.82 | 0.82 | 0.81 | 0.82 | 0.82 | 0.83 | 0.81 | 0.82 | 0.82 | 0.82 | 0.81 | 0.82 | 0.83 | 0.82 | 0.82 | 0.83 | 0.81 |
| AD | 0.84 | 0.84 | 0.84 | 0.88 | 0.82 | 0.84 | 0.89 | 0.89 | 0.85 | 0.90 | 0.83 | 0.88 | 0.84 | 0.83 | 0.90 | 0.83 | 0.90 | 0.84 | 0.84 | 0.84 | 0.85 | 0.87 | 0.84 | 0.88 | 0.83 | 0.86 | 0.84 | 0.88 | 0.91 | 0.86 | 0.88 | 0.89 | 0.86 |
| AE | 0.78 | 0.76 | 0.79 | 0.83 | 0.75 | 0.79 | 0.83 | 0.83 | 0.78 | 0.84 | 0.76 | 0.83 | 0.78 | 0.76 | 0.85 | 0.76 | 0.85 | 0.77 | 0.78 | 0.78 | 0.79 | 0.81 | 0.77 | 0.83 | 0.74 | 0.79 | 0.78 | 0.81 | 0.87 | 0.80 | 0.82 | 0.84 | 0.81 |
| AF | 0.91 | 0.92 | 0.90 | 0.94 | 0.89 | 0.90 | 0.94 | 0.96 | 0.93 | 0.95 | 0.90 | 0.94 | 0.90 | 0.89 | 0.95 | 0.91 | 0.96 | 0.91 | 0.91 | 0.91 | 0.92 | 0.93 | 0.90 | 0.94 | 0.92 | 0.92 | 0.90 | 0.94 | 0.96 | 0.92 | 0.94 | 0.95 | 0.92 |
| AG | 0.74 | 0.77 | 0.75 | 0.75 | 0.74 | 0.72 | 0.74 | 0.73 | 0.75 | 0.74 | 0.74 | 0.75 | 0.73 | 0.73 | 0.73 | 0.77 | 0.72 | 0.75 | 0.74 | 0.75 | 0.75 | 0.75 | 0.74 | 0.74 | 0.74 | 0.75 | 0.74 | 0.74 | 0.73 | 0.75 | 0.74 | 0.74 | 0.74 |
| AH | 0.69 | 0.71 | 0.70 | 0.69 | 0.69 | 0.66 | 0.69 | 0.68 | 0.69 | 0.68 | 0.68 | 0.69 | 0.67 | 0.67 | 0.68 | 0.72 | 0.67 | 0.70 | 0.69 | 0.70 | 0.69 | 0.70 | 0.69 | 0.69 | 0.70 | 0.69 | 0.68 | 0.67 | 0.69 | 0.68 | 0.68 | 0.68 | 0.68 |
| AI | 0.79 | 0.82 | 0.81 | 0.81 | 0.80 | 0.77 | 0.79 | 0.79 | 0.80 | 0.79 | 0.79 | 0.81 | 0.78 | 0.78 | 0.79 | 0.82 | 0.78 | 0.81 | 0.79 | 0.80 | 0.80 | 0.81 | 0.79 | 0.79 | 0.80 | 0.80 | 0.79 | 0.79 | 0.79 | 0.80 | 0.79 | 0.79 | 0.79 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 659 | 0.74 | 0.66 | 0.82 | 0.81 | 0.70 | 0.92 | 0.72 | 0.63 | 0.81 | 0.77 | 0.71 | 0.83 | 0.84 | 0.77 | 0.92 | 0.74 | 0.67 | 0.81 | 0.76 | 0.72 | 0.81 | 0.84 | 0.78 | 0.90 | 0.74 | 0.68 | 0.79 |
| 660 | 0.75 | 0.67 | 0.83 | 0.84 | 0.66 | 1.00 | 0.72 | 0.63 | 0.82 | 0.77 | 0.71 | 0.83 | 0.89 | 0.84 | 0.94 | 0.71 | 0.64 | 0.78 | 0.78 | 0.74 | 0.83 | 0.89 | 0.84 | 0.95 | 0.74 | 0.68 | 0.79 |
| 661 | 0.74 | 0.66 | 0.82 | 0.85 | 0.68 | 1.00 | 0.71 | 0.62 | 0.80 | 0.78 | 0.72 | 0.83 | 0.88 | 0.82 | 0.94 | 0.74 | 0.67 | 0.80 | 0.79 | 0.75 | 0.83 | 0.89 | 0.84 | 0.95 | 0.75 | 0.70 | 0.80 |
| 662 | 0.74 | 0.65 | 0.82 | 0.83 | 0.69 | 0.97 | 0.71 | 0.61 | 0.81 | 0.76 | 0.70 | 0.82 | 0.82 | 0.73 | 0.91 | 0.74 | 0.66 | 0.81 | 0.77 | 0.72 | 0.82 | 0.84 | 0.77 | 0.91 | 0.74 | 0.68 | 0.80 |
| 663 | 0.74 | 0.66 | 0.83 | 0.75 | 0.54 | 0.97 | 0.74 | 0.65 | 0.83 | 0.78 | 0.73 | 0.84 | 0.84 | 0.77 | 0.92 | 0.76 | 0.69 | 0.83 | 0.79 | 0.74 | 0.83 | 0.84 | 0.76 | 0.92 | 0.77 | 0.72 | 0.82 |
| 664 | 0.74 | 0.66 | 0.82 | 0.88 | 0.73 | 1.00 | 0.70 | 0.61 | 0.79 | 0.76 | 0.70 | 0.82 | 0.87 | 0.78 | 0.96 | 0.72 | 0.65 | 0.79 | 0.76 | 0.72 | 0.81 | 0.88 | 0.81 | 0.95 | 0.72 | 0.67 | 0.78 |
| 665 | 0.74 | 0.66 | 0.82 | 0.81 | 0.64 | 0.99 | 0.72 | 0.63 | 0.81 | 0.77 | 0.71 | 0.83 | 0.89 | 0.84 | 0.94 | 0.72 | 0.65 | 0.79 | 0.78 | 0.74 | 0.83 | 0.89 | 0.83 | 0.94 | 0.74 | 0.69 | 0.79 |
| 666 | 0.75 | 0.66 | 0.84 | 0.79 | 0.62 | 0.96 | 0.74 | 0.64 | 0.83 | 0.78 | 0.72 | 0.83 | 0.86 | 0.80 | 0.93 | 0.74 | 0.67 | 0.81 | 0.78 | 0.73 | 0.82 | 0.85 | 0.78 | 0.92 | 0.75 | 0.69 | 0.80 |
| 667 | 0.75 | 0.67 | 0.84 | 0.85 | 0.68 | 1.00 | 0.73 | 0.63 | 0.82 | 0.77 | 0.71 | 0.83 | 0.90 | 0.85 | 0.95 | 0.71 | 0.64 | 0.78 | 0.78 | 0.74 | 0.83 | 0.90 | 0.85 | 0.96 | 0.74 | 0.69 | 0.79 |
| 668 | 0.75 | 0.67 | 0.83 | 0.85 | 0.67 | 1.00 | 0.72 | 0.64 | 0.81 | 0.77 | 0.72 | 0.83 | 0.88 | 0.83 | 0.94 | 0.72 | 0.65 | 0.79 | 0.79 | 0.74 | 0.83 | 0.89 | 0.84 | 0.95 | 0.75 | 0.69 | 0.80 |
| 669 | 0.76 | 0.69 | 0.83 | 0.83 | 0.71 | 0.95 | 0.74 | 0.65 | 0.82 | 0.77 | 0.72 | 0.83 | 0.86 | 0.79 | 0.94 | 0.73 | 0.67 | 0.80 | 0.78 | 0.74 | 0.82 | 0.86 | 0.80 | 0.92 | 0.75 | 0.70 | 0.80 |
| 670 | 0.74 | 0.67 | 0.81 | 0.80 | 0.68 | 0.92 | 0.72 | 0.64 | 0.81 | 0.77 | 0.71 | 0.83 | 0.84 | 0.74 | 0.95 | 0.74 | 0.68 | 0.81 | 0.77 | 0.72 | 0.81 | 0.83 | 0.76 | 0.91 | 0.74 | 0.70 | 0.80 |
| 671 | 0.74 | 0.65 | 0.83 | 0.85 | 0.72 | 0.99 | 0.71 | 0.60 | 0.81 | 0.77 | 0.71 | 0.83 | 0.87 | 0.77 | 0.96 | 0.73 | 0.67 | 0.80 | 0.77 | 0.73 | 0.82 | 0.87 | 0.80 | 0.94 | 0.75 | 0.69 | 0.79 |
| 672 | 0.75 | 0.68 | 0.82 | 0.81 | 0.68 | 0.95 | 0.73 | 0.65 | 0.81 | 0.77 | 0.71 | 0.83 | 0.85 | 0.77 | 0.93 | 0.74 | 0.67 | 0.81 | 0.78 | 0.74 | 0.82 | 0.85 | 0.78 | 0.92 | 0.75 | 0.70 | 0.80 |
| 673 | 0.75 | 0.67 | 0.82 | 0.80 | 0.67 | 0.94 | 0.73 | 0.65 | 0.81 | 0.77 | 0.72 | 0.82 | 0.85 | 0.77 | 0.93 | 0.74 | 0.67 | 0.81 | 0.77 | 0.73 | 0.82 | 0.85 | 0.78 | 0.91 | 0.75 | 0.70 | 0.80 |
| 674 | 0.74 | 0.67 | 0.81 | 0.85 | 0.77 | 0.94 | 0.70 | 0.62 | 0.79 | 0.76 | 0.70 | 0.82 | 0.81 | 0.72 | 0.91 | 0.74 | 0.66 | 0.81 | 0.76 | 0.72 | 0.81 | 0.84 | 0.76 | 0.90 | 0.74 | 0.68 | 0.79 |
| 675 | 0.78 | 0.71 | 0.85 | 0.90 | 0.82 | 0.98 | 0.74 | 0.65 | 0.83 | 0.77 | 0.71 | 0.83 | 0.86 | 0.79 | 0.93 | 0.74 | 0.67 | 0.81 | 0.78 | 0.73 | 0.82 | 0.88 | 0.82 | 0.93 | 0.75 | 0.69 | 0.80 |
| 676 | 0.76 | 0.69 | 0.83 | 0.85 | 0.73 | 0.97 | 0.73 | 0.65 | 0.81 | 0.77 | 0.71 | 0.83 | 0.85 | 0.76 | 0.93 | 0.71 | 0.64 | 0.81 | 0.78 | 0.74 | 0.82 | 0.86 | 0.79 | 0.92 | 0.73 | 0.70 | 0.80 |
| 677 | 0.74 | 0.65 | 0.82 | 0.87 | 0.73 | 1.00 | 0.70 | 0.60 | 0.80 | 0.77 | 0.71 | 0.83 | 0.90 | 0.85 | 0.95 | 0.73 | 0.67 | 0.81 | 0.78 | 0.73 | 0.82 | 0.90 | 0.85 | 0.95 | 0.76 | 0.67 | 0.78 |
| 678 | 0.74 | 0.66 | 0.83 | 0.78 | 0.59 | 0.97 | 0.73 | 0.63 | 0.82 | 0.79 | 0.73 | 0.84 | 0.86 | 0.79 | 0.93 | 0.76 | 0.69 | 0.83 | 0.79 | 0.74 | 0.83 | 0.85 | 0.78 | 0.93 | 0.75 | 0.71 | 0.81 |
| 679 | 0.75 | 0.67 | 0.82 | 0.83 | 0.70 | 0.96 | 0.72 | 0.63 | 0.81 | 0.75 | 0.69 | 0.81 | 0.79 | 0.70 | 0.88 | 0.73 | 0.66 | 0.80 | 0.77 | 0.73 | 0.81 | 0.82 | 0.76 | 0.89 | 0.74 | 0.70 | 0.80 |
| 680 | 0.76 | 0.69 | 0.83 | 0.86 | 0.78 | 0.95 | 0.73 | 0.64 | 0.81 | 0.77 | 0.71 | 0.83 | 0.85 | 0.77 | 0.93 | 0.71 | 0.66 | 0.80 | 0.77 | 0.73 | 0.82 | 0.86 | 0.80 | 0.92 | 0.75 | 0.69 | 0.79 |
| 681 | 0.74 | 0.66 | 0.82 | 0.81 | 0.63 | 0.98 | 0.72 | 0.63 | 0.81 | 0.77 | 0.71 | 0.82 | 0.89 | 0.84 | 0.94 | 0.74 | 0.64 | 0.78 | 0.78 | 0.73 | 0.82 | 0.88 | 0.83 | 0.94 | 0.74 | 0.68 | 0.79 |
| 682 | 0.75 | 0.68 | 0.82 | 0.82 | 0.71 | 0.93 | 0.73 | 0.65 | 0.81 | 0.77 | 0.71 | 0.83 | 0.85 | 0.77 | 0.94 | 0.73 | 0.67 | 0.81 | 0.77 | 0.73 | 0.82 | 0.85 | 0.78 | 0.91 | 0.75 | 0.69 | 0.80 |
| 683 | 0.74 | 0.67 | 0.82 | 0.81 | 0.68 | 0.94 | 0.72 | 0.63 | 0.80 | 0.78 | 0.72 | 0.83 | 0.88 | 0.83 | 0.94 | 0.72 | 0.66 | 0.80 | 0.78 | 0.73 | 0.82 | 0.87 | 0.81 | 0.93 | 0.74 | 0.68 | 0.79 |
| 684 | 0.75 | 0.66 | 0.83 | 0.88 | 0.75 | 1.00 | 0.71 | 0.61 | 0.83 | 0.77 | 0.71 | 0.83 | 0.90 | 0.83 | 0.96 | 0.71 | 0.65 | 0.79 | 0.77 | 0.73 | 0.82 | 0.91 | 0.85 | 0.96 | 0.73 | 0.68 | 0.79 |
| 685 | 0.77 | 0.68 | 0.85 | 0.88 | 0.75 | 1.00 | 0.73 | 0.63 | 0.80 | 0.75 | 0.69 | 0.81 | 0.85 | 0.76 | 0.94 | 0.73 | 0.64 | 0.78 | 0.77 | 0.73 | 0.82 | 0.87 | 0.80 | 0.94 | 0.74 | 0.68 | 0.79 |
| 686 | 0.75 | 0.68 | 0.82 | 0.83 | 0.72 | 0.94 | 0.72 | 0.64 | 0.86 | 0.77 | 0.71 | 0.83 | 0.87 | 0.79 | 0.95 | 0.74 | 0.66 | 0.80 | 0.77 | 0.74 | 0.82 | 0.86 | 0.80 | 0.92 | 0.76 | 0.69 | 0.79 |
| 687 | 0.78 | 0.70 | 0.86 | 0.85 | 0.75 | 0.95 | 0.76 | 0.66 | 0.79 | 0.77 | 0.71 | 0.84 | 0.83 | 0.76 | 0.90 | 0.75 | 0.67 | 0.82 | 0.79 | 0.73 | 0.83 | 0.85 | 0.79 | 0.90 | 0.75 | 0.71 | 0.79 |
| 688 | 0.75 | 0.68 | 0.82 | 0.85 | 0.75 | 0.95 | 0.72 | 0.64 | 0.83 | 0.77 | 0.72 | 0.81 | 0.83 | 0.74 | 0.93 | 0.74 | 0.68 | 0.82 | 0.77 | 0.74 | 0.82 | 0.85 | 0.78 | 0.91 | 0.75 | 0.70 | 0.82 |
| 689 | 0.76 | 0.69 | 0.84 | 0.84 | 0.71 | 0.97 | 0.74 | 0.65 | 0.80 | 0.77 | 0.71 | 0.82 | 0.87 | 0.80 | 0.95 | 0.70 | 0.67 | 0.82 | 0.78 | 0.73 | 0.83 | 0.87 | 0.81 | 0.93 | 0.72 | 0.70 | 0.79 |
| 690 | 0.74 | 0.66 | 0.82 | 0.87 | 0.72 | 1.00 | 0.70 | 0.61 | 0.83 | 0.77 | 0.72 | 0.82 | 0.91 | 0.87 | 0.96 | 0.70 | 0.63 | 0.81 | 0.77 | 0.73 | 0.82 | 0.91 | 0.86 | 0.96 | 0.76 | 0.67 | 0.80 |
| 691 | 0.75 | 0.67 | 0.84 | 0.79 | 0.63 | 0.95 | 0.74 | 0.65 | 0.83 | 0.77 | 0.72 | 0.83 | 0.84 | 0.77 | 0.92 | 0.75 | 0.68 | 0.81 | 0.79 | 0.74 | 0.83 | 0.85 | 0.78 | 0.92 | 0.76 | 0.71 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 692 | 0.77 | 0.70 | 0.84 | 0.84 | 0.74 | 0.95 | 0.75 | 0.66 | 0.83 | 0.76 | 0.69 | 0.82 | 0.84 | 0.77 | 0.92 | 0.72 | 0.64 | 0.79 | 0.77 | 0.72 | 0.81 | 0.85 | 0.79 | 0.90 | 0.73 | 0.68 | 0.79 |
| 693 | 0.76 | 0.67 | 0.85 | 0.86 | 0.72 | 1.00 | 0.73 | 0.62 | 0.83 | 0.75 | 0.69 | 0.82 | 0.84 | 0.74 | 0.94 | 0.72 | 0.64 | 0.79 | 0.77 | 0.72 | 0.82 | 0.86 | 0.78 | 0.93 | 0.74 | 0.68 | 0.80 |
| 694 | 0.74 | 0.66 | 0.82 | 0.87 | 0.72 | 1.00 | 0.71 | 0.61 | 0.80 | 0.76 | 0.70 | 0.82 | 0.91 | 0.87 | 0.96 | 0.70 | 0.63 | 0.77 | 0.77 | 0.73 | 0.82 | 0.91 | 0.86 | 0.96 | 0.72 | 0.66 | 0.78 |
| 695 | 0.74 | 0.67 | 0.81 | 0.77 | 0.63 | 0.91 | 0.74 | 0.66 | 0.82 | 0.77 | 0.71 | 0.83 | 0.85 | 0.76 | 0.93 | 0.74 | 0.67 | 0.81 | 0.77 | 0.72 | 0.81 | 0.83 | 0.76 | 0.90 | 0.74 | 0.69 | 0.80 |
| 696 | 0.75 | 0.67 | 0.82 | 0.81 | 0.70 | 0.92 | 0.72 | 0.64 | 0.81 | 0.77 | 0.71 | 0.82 | 0.83 | 0.75 | 0.90 | 0.74 | 0.67 | 0.81 | 0.77 | 0.72 | 0.81 | 0.83 | 0.77 | 0.89 | 0.74 | 0.69 | 0.80 |
| 697 | 0.74 | 0.67 | 0.81 | 0.82 | 0.72 | 0.92 | 0.72 | 0.63 | 0.80 | 0.77 | 0.71 | 0.83 | 0.87 | 0.79 | 0.94 | 0.73 | 0.66 | 0.80 | 0.76 | 0.72 | 0.81 | 0.86 | 0.80 | 0.91 | 0.73 | 0.68 | 0.78 |
| 698 | 0.74 | 0.66 | 0.83 | 0.88 | 0.74 | 1.00 | 0.70 | 0.60 | 0.80 | 0.76 | 0.70 | 0.82 | 0.87 | 0.78 | 0.97 | 0.72 | 0.65 | 0.79 | 0.76 | 0.72 | 0.81 | 0.88 | 0.81 | 0.96 | 0.72 | 0.67 | 0.78 |
| 699 | 0.76 | 0.68 | 0.84 | 0.85 | 0.72 | 0.98 | 0.73 | 0.63 | 0.83 | 0.78 | 0.72 | 0.83 | 0.88 | 0.82 | 0.94 | 0.73 | 0.66 | 0.81 | 0.78 | 0.73 | 0.83 | 0.88 | 0.82 | 0.93 | 0.74 | 0.69 | 0.80 |
| 700 | 0.75 | 0.67 | 0.84 | 0.85 | 0.68 | 1.00 | 0.73 | 0.64 | 0.82 | 0.77 | 0.71 | 0.82 | 0.90 | 0.85 | 0.95 | 0.71 | 0.64 | 0.78 | 0.78 | 0.74 | 0.83 | 0.90 | 0.85 | 0.96 | 0.74 | 0.68 | 0.79 |
| 701 | 0.74 | 0.66 | 0.81 | 0.82 | 0.67 | 0.97 | 0.71 | 0.63 | 0.79 | 0.77 | 0.72 | 0.83 | 0.86 | 0.79 | 0.93 | 0.74 | 0.67 | 0.81 | 0.77 | 0.72 | 0.81 | 0.85 | 0.79 | 0.92 | 0.74 | 0.68 | 0.79 |
| 702 | 0.76 | 0.68 | 0.84 | 0.86 | 0.75 | 0.98 | 0.73 | 0.63 | 0.83 | 0.77 | 0.72 | 0.83 | 0.85 | 0.77 | 0.93 | 0.74 | 0.67 | 0.81 | 0.78 | 0.74 | 0.83 | 0.87 | 0.81 | 0.93 | 0.75 | 0.70 | 0.81 |
| 703 | 0.76 | 0.68 | 0.84 | 0.85 | 0.71 | 0.98 | 0.73 | 0.64 | 0.82 | 0.77 | 0.71 | 0.83 | 0.85 | 0.78 | 0.91 | 0.74 | 0.67 | 0.80 | 0.76 | 0.72 | 0.81 | 0.84 | 0.78 | 0.90 | 0.73 | 0.68 | 0.78 |
| 704 | 0.76 | 0.67 | 0.84 | 0.79 | 0.62 | 0.97 | 0.74 | 0.64 | 0.84 | 0.77 | 0.72 | 0.83 | 0.86 | 0.79 | 0.93 | 0.74 | 0.67 | 0.81 | 0.78 | 0.73 | 0.82 | 0.85 | 0.78 | 0.92 | 0.75 | 0.69 | 0.80 |
| 705 | 0.74 | 0.67 | 0.82 | 0.86 | 0.75 | 0.97 | 0.70 | 0.61 | 0.79 | 0.77 | 0.71 | 0.83 | 0.84 | 0.74 | 0.95 | 0.74 | 0.67 | 0.81 | 0.76 | 0.72 | 0.81 | 0.85 | 0.78 | 0.93 | 0.73 | 0.68 | 0.78 |
| 706 | 0.75 | 0.67 | 0.84 | 0.86 | 0.68 | 1.00 | 0.72 | 0.63 | 0.81 | 0.77 | 0.71 | 0.82 | 0.90 | 0.85 | 0.95 | 0.71 | 0.64 | 0.78 | 0.78 | 0.73 | 0.82 | 0.90 | 0.85 | 0.95 | 0.73 | 0.68 | 0.78 |
| 707 | 0.74 | 0.67 | 0.82 | 0.81 | 0.71 | 0.91 | 0.72 | 0.63 | 0.81 | 0.77 | 0.71 | 0.83 | 0.84 | 0.76 | 0.92 | 0.74 | 0.67 | 0.81 | 0.78 | 0.73 | 0.82 | 0.84 | 0.78 | 0.90 | 0.75 | 0.70 | 0.80 |
| 708 | 0.76 | 0.68 | 0.83 | 0.86 | 0.74 | 0.97 | 0.72 | 0.64 | 0.81 | 0.77 | 0.71 | 0.83 | 0.85 | 0.78 | 0.93 | 0.74 | 0.67 | 0.81 | 0.78 | 0.73 | 0.82 | 0.86 | 0.81 | 0.92 | 0.75 | 0.69 | 0.80 |
| 709 | 0.75 | 0.68 | 0.82 | 0.81 | 0.71 | 0.92 | 0.73 | 0.65 | 0.81 | 0.77 | 0.71 | 0.83 | 0.87 | 0.79 | 0.94 | 0.73 | 0.66 | 0.80 | 0.77 | 0.73 | 0.81 | 0.85 | 0.79 | 0.91 | 0.73 | 0.68 | 0.79 |
| 710 | 0.74 | 0.66 | 0.82 | 0.78 | 0.62 | 0.94 | 0.73 | 0.64 | 0.82 | 0.77 | 0.71 | 0.83 | 0.86 | 0.79 | 0.94 | 0.73 | 0.66 | 0.80 | 0.78 | 0.73 | 0.82 | 0.85 | 0.78 | 0.92 | 0.75 | 0.70 | 0.80 |
| 711 | 0.74 | 0.67 | 0.81 | 0.75 | 0.61 | 0.90 | 0.73 | 0.65 | 0.81 | 0.77 | 0.71 | 0.83 | 0.84 | 0.76 | 0.93 | 0.74 | 0.67 | 0.81 | 0.77 | 0.72 | 0.81 | 0.82 | 0.75 | 0.89 | 0.75 | 0.69 | 0.80 |
| 712 | 0.75 | 0.66 | 0.83 | 0.79 | 0.61 | 0.98 | 0.73 | 0.63 | 0.83 | 0.78 | 0.73 | 0.84 | 0.81 | 0.71 | 0.91 | 0.77 | 0.71 | 0.83 | 0.78 | 0.74 | 0.83 | 0.82 | 0.74 | 0.90 | 0.77 | 0.72 | 0.82 |
| 713 | 0.74 | 0.67 | 0.82 | 0.81 | 0.71 | 0.90 | 0.73 | 0.63 | 0.82 | 0.77 | 0.71 | 0.83 | 0.85 | 0.76 | 0.93 | 0.74 | 0.66 | 0.81 | 0.77 | 0.72 | 0.82 | 0.84 | 0.78 | 0.91 | 0.74 | 0.69 | 0.80 |
| 714 | 0.74 | 0.68 | 0.81 | 0.79 | 0.67 | 0.91 | 0.73 | 0.65 | 0.81 | 0.77 | 0.71 | 0.83 | 0.84 | 0.76 | 0.93 | 0.74 | 0.67 | 0.80 | 0.77 | 0.73 | 0.81 | 0.83 | 0.77 | 0.90 | 0.75 | 0.70 | 0.80 |
| 715 | 0.74 | 0.67 | 0.82 | 0.84 | 0.74 | 0.93 | 0.71 | 0.62 | 0.80 | 0.76 | 0.70 | 0.82 | 0.80 | 0.71 | 0.89 | 0.75 | 0.68 | 0.82 | 0.76 | 0.71 | 0.81 | 0.81 | 0.75 | 0.88 | 0.74 | 0.68 | 0.80 |
| 716 | 0.74 | 0.66 | 0.82 | 0.83 | 0.66 | 1.00 | 0.72 | 0.62 | 0.81 | 0.78 | 0.72 | 0.83 | 0.87 | 0.81 | 0.93 | 0.73 | 0.67 | 0.80 | 0.79 | 0.74 | 0.83 | 0.88 | 0.82 | 0.94 | 0.75 | 0.70 | 0.80 |
| 717 | 0.75 | 0.68 | 0.82 | 0.81 | 0.68 | 0.93 | 0.74 | 0.65 | 0.82 | 0.77 | 0.71 | 0.83 | 0.85 | 0.77 | 0.93 | 0.73 | 0.67 | 0.80 | 0.78 | 0.73 | 0.82 | 0.84 | 0.78 | 0.91 | 0.75 | 0.70 | 0.80 |
| 718 | 0.71 | 0.61 | 0.80 | 0.75 | 0.57 | 0.94 | 0.69 | 0.59 | 0.80 | 0.77 | 0.71 | 0.83 | 0.87 | 0.79 | 0.96 | 0.73 | 0.66 | 0.81 | 0.76 | 0.71 | 0.81 | 0.84 | 0.75 | 0.92 | 0.73 | 0.68 | 0.79 |
| 719 | 0.75 | 0.67 | 0.84 | 0.82 | 0.67 | 0.97 | 0.73 | 0.63 | 0.83 | 0.77 | 0.72 | 0.83 | 0.87 | 0.80 | 0.93 | 0.74 | 0.67 | 0.81 | 0.78 | 0.74 | 0.83 | 0.86 | 0.80 | 0.92 | 0.75 | 0.69 | 0.80 |
| 720 | 0.74 | 0.67 | 0.82 | 0.80 | 0.69 | 0.92 | 0.73 | 0.64 | 0.82 | 0.77 | 0.71 | 0.83 | 0.84 | 0.76 | 0.92 | 0.74 | 0.67 | 0.81 | 0.77 | 0.73 | 0.82 | 0.84 | 0.78 | 0.90 | 0.75 | 0.69 | 0.80 |
| 721 | 0.77 | 0.68 | 0.85 | 0.85 | 0.73 | 0.96 | 0.74 | 0.64 | 0.84 | 0.77 | 0.71 | 0.83 | 0.88 | 0.81 | 0.95 | 0.73 | 0.66 | 0.80 | 0.78 | 0.74 | 0.83 | 0.88 | 0.82 | 0.93 | 0.75 | 0.69 | 0.81 |
| 722 | 0.74 | 0.65 | 0.83 | 0.76 | 0.60 | 0.93 | 0.73 | 0.63 | 0.83 | 0.77 | 0.71 | 0.83 | 0.85 | 0.77 | 0.92 | 0.74 | 0.67 | 0.81 | 0.78 | 0.73 | 0.82 | 0.83 | 0.76 | 0.91 | 0.75 | 0.70 | 0.81 |
| 723 | 0.74 | 0.65 | 0.83 | 0.78 | 0.61 | 0.96 | 0.73 | 0.63 | 0.82 | 0.77 | 0.72 | 0.83 | 0.88 | 0.83 | 0.94 | 0.73 | 0.66 | 0.80 | 0.77 | 0.72 | 0.81 | 0.86 | 0.79 | 0.92 | 0.73 | 0.68 | 0.79 |
| 724 | 0.75 | 0.66 | 0.83 | 0.80 | 0.63 | 0.97 | 0.73 | 0.63 | 0.82 | 0.77 | 0.72 | 0.83 | 0.85 | 0.78 | 0.92 | 0.74 | 0.67 | 0.81 | 0.78 | 0.74 | 0.83 | 0.85 | 0.79 | 0.92 | 0.76 | 0.70 | 0.81 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 725 | 0.74 | 0.67 | 0.81 | 0.79 | 0.67 | 0.90 | 0.72 | 0.64 | 0.80 | 0.77 | 0.71 | 0.83 | 0.84 | 0.75 | 0.93 | 0.74 | 0.67 | 0.80 | 0.77 | 0.73 | 0.81 | 0.83 | 0.76 | 0.90 | 0.75 | 0.70 | 0.80 |
| 726 | 0.73 | 0.66 | 0.81 | 0.86 | 0.75 | 0.97 | 0.69 | 0.61 | 0.78 | 0.77 | 0.71 | 0.82 | 0.85 | 0.76 | 0.94 | 0.74 | 0.67 | 0.80 | 0.76 | 0.71 | 0.80 | 0.85 | 0.78 | 0.92 | 0.72 | 0.67 | 0.77 |
| 727 | 0.74 | 0.65 | 0.83 | 0.88 | 0.72 | 1.00 | 0.70 | 0.60 | 0.80 | 0.76 | 0.70 | 0.82 | 0.87 | 0.81 | 0.93 | 0.72 | 0.65 | 0.79 | 0.78 | 0.73 | 0.82 | 0.90 | 0.84 | 0.95 | 0.73 | 0.68 | 0.79 |
| 728 | 0.75 | 0.67 | 0.83 | 0.78 | 0.61 | 0.96 | 0.74 | 0.65 | 0.83 | 0.77 | 0.72 | 0.83 | 0.87 | 0.80 | 0.93 | 0.74 | 0.67 | 0.81 | 0.78 | 0.73 | 0.82 | 0.85 | 0.78 | 0.92 | 0.75 | 0.70 | 0.80 |
| 729 | 0.73 | 0.66 | 0.81 | 0.76 | 0.62 | 0.91 | 0.72 | 0.64 | 0.81 | 0.77 | 0.71 | 0.83 | 0.84 | 0.76 | 0.93 | 0.74 | 0.67 | 0.81 | 0.76 | 0.72 | 0.81 | 0.82 | 0.75 | 0.89 | 0.74 | 0.69 | 0.79 |
| 730 | 0.76 | 0.67 | 0.85 | 0.82 | 0.67 | 0.97 | 0.74 | 0.64 | 0.84 | 0.77 | 0.72 | 0.83 | 0.87 | 0.80 | 0.93 | 0.73 | 0.66 | 0.81 | 0.78 | 0.74 | 0.83 | 0.86 | 0.80 | 0.92 | 0.75 | 0.70 | 0.81 |
| 731 | 0.74 | 0.66 | 0.82 | 0.81 | 0.64 | 0.98 | 0.72 | 0.63 | 0.81 | 0.76 | 0.71 | 0.82 | 0.89 | 0.84 | 0.94 | 0.71 | 0.64 | 0.78 | 0.78 | 0.73 | 0.82 | 0.89 | 0.83 | 0.94 | 0.74 | 0.68 | 0.79 |
| 732 | 0.74 | 0.67 | 0.81 | 0.86 | 0.73 | 0.98 | 0.70 | 0.63 | 0.78 | 0.77 | 0.71 | 0.83 | 0.86 | 0.79 | 0.94 | 0.73 | 0.66 | 0.80 | 0.77 | 0.72 | 0.81 | 0.86 | 0.80 | 0.93 | 0.73 | 0.68 | 0.78 |
| 733 | 0.77 | 0.70 | 0.84 | 0.81 | 0.67 | 0.95 | 0.75 | 0.67 | 0.83 | 0.77 | 0.71 | 0.83 | 0.86 | 0.78 | 0.93 | 0.73 | 0.66 | 0.80 | 0.78 | 0.73 | 0.82 | 0.85 | 0.78 | 0.91 | 0.75 | 0.70 | 0.80 |
| 734 | 0.74 | 0.67 | 0.81 | 0.81 | 0.67 | 0.94 | 0.72 | 0.64 | 0.80 | 0.77 | 0.71 | 0.83 | 0.84 | 0.76 | 0.92 | 0.74 | 0.67 | 0.81 | 0.78 | 0.73 | 0.82 | 0.85 | 0.78 | 0.91 | 0.75 | 0.70 | 0.80 |
| 735 | 0.74 | 0.66 | 0.82 | 0.82 | 0.67 | 0.98 | 0.71 | 0.62 | 0.80 | 0.77 | 0.71 | 0.83 | 0.86 | 0.79 | 0.93 | 0.72 | 0.65 | 0.81 | 0.78 | 0.73 | 0.82 | 0.86 | 0.80 | 0.93 | 0.74 | 0.69 | 0.80 |
| 736 | 0.75 | 0.68 | 0.83 | 0.82 | 0.70 | 0.94 | 0.73 | 0.64 | 0.82 | 0.77 | 0.71 | 0.83 | 0.89 | 0.84 | 0.94 | 0.73 | 0.66 | 0.79 | 0.78 | 0.74 | 0.82 | 0.88 | 0.82 | 0.93 | 0.74 | 0.69 | 0.80 |
| 737 | 0.76 | 0.69 | 0.83 | 0.83 | 0.75 | 0.92 | 0.74 | 0.66 | 0.82 | 0.77 | 0.71 | 0.83 | 0.86 | 0.80 | 0.92 | 0.73 | 0.66 | 0.80 | 0.78 | 0.74 | 0.82 | 0.86 | 0.81 | 0.91 | 0.75 | 0.70 | 0.80 |
| 738 | 0.74 | 0.66 | 0.82 | 0.84 | 0.67 | 1.00 | 0.71 | 0.62 | 0.80 | 0.77 | 0.72 | 0.83 | 0.87 | 0.81 | 0.93 | 0.73 | 0.66 | 0.80 | 0.79 | 0.74 | 0.83 | 0.88 | 0.82 | 0.94 | 0.75 | 0.70 | 0.80 |
| 739 | 0.74 | 0.66 | 0.82 | 0.86 | 0.75 | 0.98 | 0.70 | 0.60 | 0.79 | 0.77 | 0.71 | 0.83 | 0.87 | 0.78 | 0.97 | 0.73 | 0.66 | 0.79 | 0.76 | 0.71 | 0.80 | 0.87 | 0.80 | 0.94 | 0.72 | 0.66 | 0.77 |
| 740 | 0.74 | 0.67 | 0.82 | 0.83 | 0.73 | 0.92 | 0.72 | 0.63 | 0.80 | 0.77 | 0.71 | 0.83 | 0.86 | 0.78 | 0.94 | 0.73 | 0.66 | 0.80 | 0.76 | 0.72 | 0.81 | 0.85 | 0.79 | 0.94 | 0.73 | 0.68 | 0.78 |
| 741 | 0.74 | 0.67 | 0.81 | 0.77 | 0.64 | 0.90 | 0.73 | 0.65 | 0.81 | 0.77 | 0.71 | 0.82 | 0.82 | 0.75 | 0.90 | 0.74 | 0.67 | 0.81 | 0.77 | 0.72 | 0.81 | 0.82 | 0.76 | 0.88 | 0.75 | 0.70 | 0.80 |
| 742 | 0.75 | 0.67 | 0.84 | 0.83 | 0.70 | 0.97 | 0.73 | 0.63 | 0.83 | 0.77 | 0.71 | 0.83 | 0.90 | 0.85 | 0.94 | 0.72 | 0.65 | 0.79 | 0.77 | 0.73 | 0.82 | 0.88 | 0.83 | 0.94 | 0.73 | 0.67 | 0.79 |
| 743 | 0.75 | 0.67 | 0.83 | 0.84 | 0.72 | 0.96 | 0.72 | 0.63 | 0.81 | 0.76 | 0.71 | 0.82 | 0.84 | 0.77 | 0.91 | 0.73 | 0.66 | 0.80 | 0.75 | 0.71 | 0.80 | 0.83 | 0.77 | 0.89 | 0.72 | 0.67 | 0.77 |
| 744 | 0.75 | 0.66 | 0.83 | 0.85 | 0.68 | 1.00 | 0.72 | 0.62 | 0.81 | 0.77 | 0.71 | 0.82 | 0.90 | 0.86 | 0.95 | 0.71 | 0.64 | 0.78 | 0.78 | 0.73 | 0.82 | 0.90 | 0.85 | 0.96 | 0.73 | 0.68 | 0.78 |
| 745 | 0.76 | 0.69 | 0.84 | 0.84 | 0.72 | 0.96 | 0.74 | 0.65 | 0.83 | 0.77 | 0.71 | 0.83 | 0.87 | 0.80 | 0.95 | 0.73 | 0.66 | 0.80 | 0.78 | 0.74 | 0.83 | 0.87 | 0.81 | 0.93 | 0.75 | 0.70 | 0.80 |
| 746 | 0.74 | 0.66 | 0.81 | 0.83 | 0.69 | 0.98 | 0.71 | 0.63 | 0.79 | 0.77 | 0.71 | 0.83 | 0.85 | 0.78 | 0.92 | 0.74 | 0.67 | 0.81 | 0.77 | 0.73 | 0.81 | 0.86 | 0.79 | 0.92 | 0.74 | 0.69 | 0.79 |
| 747 | 0.75 | 0.67 | 0.82 | 0.85 | 0.74 | 0.97 | 0.71 | 0.62 | 0.80 | 0.77 | 0.71 | 0.83 | 0.85 | 0.77 | 0.92 | 0.74 | 0.67 | 0.81 | 0.77 | 0.73 | 0.82 | 0.86 | 0.80 | 0.92 | 0.74 | 0.69 | 0.79 |
| 748 | 0.74 | 0.66 | 0.83 | 0.78 | 0.60 | 0.95 | 0.73 | 0.64 | 0.83 | 0.77 | 0.72 | 0.83 | 0.88 | 0.82 | 0.94 | 0.72 | 0.65 | 0.80 | 0.77 | 0.72 | 0.81 | 0.85 | 0.79 | 0.92 | 0.73 | 0.68 | 0.79 |
| 749 | 0.74 | 0.67 | 0.82 | 0.81 | 0.71 | 0.91 | 0.72 | 0.63 | 0.81 | 0.77 | 0.71 | 0.82 | 0.81 | 0.73 | 0.90 | 0.75 | 0.68 | 0.81 | 0.77 | 0.73 | 0.82 | 0.83 | 0.77 | 0.89 | 0.75 | 0.70 | 0.80 |
| 750 | 0.76 | 0.68 | 0.83 | 0.84 | 0.74 | 0.94 | 0.73 | 0.64 | 0.81 | 0.77 | 0.71 | 0.82 | 0.84 | 0.76 | 0.92 | 0.74 | 0.67 | 0.80 | 0.78 | 0.74 | 0.82 | 0.85 | 0.79 | 0.91 | 0.75 | 0.70 | 0.80 |
| 751 | 0.73 | 0.66 | 0.81 | 0.80 | 0.66 | 0.94 | 0.71 | 0.63 | 0.80 | 0.77 | 0.71 | 0.83 | 0.85 | 0.77 | 0.93 | 0.72 | 0.67 | 0.80 | 0.77 | 0.73 | 0.82 | 0.85 | 0.78 | 0.91 | 0.73 | 0.66 | 0.80 |
| 752 | 0.75 | 0.68 | 0.82 | 0.83 | 0.74 | 0.93 | 0.73 | 0.65 | 0.81 | 0.77 | 0.71 | 0.82 | 0.86 | 0.79 | 0.94 | 0.73 | 0.65 | 0.79 | 0.77 | 0.73 | 0.81 | 0.86 | 0.80 | 0.92 | 0.73 | 0.68 | 0.79 |
| 753 | 0.76 | 0.68 | 0.84 | 0.87 | 0.76 | 0.98 | 0.72 | 0.62 | 0.82 | 0.77 | 0.71 | 0.83 | 0.87 | 0.80 | 0.94 | 0.74 | 0.67 | 0.80 | 0.77 | 0.72 | 0.82 | 0.87 | 0.81 | 0.93 | 0.73 | 0.67 | 0.79 |
| 754 | 0.74 | 0.66 | 0.82 | 0.85 | 0.68 | 1.00 | 0.71 | 0.62 | 0.80 | 0.78 | 0.72 | 0.82 | 0.86 | 0.80 | 0.94 | 0.74 | 0.68 | 0.81 | 0.79 | 0.74 | 0.83 | 0.88 | 0.83 | 0.94 | 0.75 | 0.70 | 0.80 |
| 755 | 0.75 | 0.67 | 0.82 | 0.84 | 0.74 | 0.94 | 0.72 | 0.63 | 0.81 | 0.76 | 0.70 | 0.82 | 0.80 | 0.71 | 0.92 | 0.73 | 0.66 | 0.80 | 0.77 | 0.72 | 0.81 | 0.82 | 0.76 | 0.89 | 0.74 | 0.69 | 0.80 |
| 756 | 0.76 | 0.68 | 0.84 | 0.88 | 0.75 | 1.00 | 0.72 | 0.62 | 0.82 | 0.76 | 0.71 | 0.82 | 0.85 | 0.75 | 0.95 | 0.73 | 0.66 | 0.80 | 0.78 | 0.73 | 0.82 | 0.87 | 0.79 | 0.94 | 0.74 | 0.69 | 0.80 |
| 757 | 0.74 | 0.66 | 0.83 | 0.79 | 0.61 | 0.97 | 0.73 | 0.63 | 0.83 | 0.77 | 0.72 | 0.83 | 0.87 | 0.81 | 0.93 | 0.73 | 0.66 | 0.80 | 0.77 | 0.72 | 0.81 | 0.85 | 0.78 | 0.92 | 0.74 | 0.68 | 0.79 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 758 | 0.76 | 0.68 | 0.83 | 0.82 | 0.69 | 0.95 | 0.73 | 0.65 | 0.82 | 0.77 | 0.71 | 0.82 | 0.83 | 0.74 | 0.92 | 0.74 | 0.67 | 0.81 | 0.77 | 0.73 | 0.82 | 0.83 | 0.76 | 0.91 | 0.75 | 0.70 | 0.80 |
| 759 | 0.74 | 0.66 | 0.83 | 0.80 | 0.62 | 0.99 | 0.73 | 0.63 | 0.82 | 0.78 | 0.72 | 0.83 | 0.86 | 0.80 | 0.93 | 0.74 | 0.67 | 0.81 | 0.78 | 0.74 | 0.83 | 0.86 | 0.80 | 0.93 | 0.75 | 0.70 | 0.81 |
| 760 | 0.76 | 0.67 | 0.84 | 0.81 | 0.66 | 0.97 | 0.74 | 0.64 | 0.83 | 0.77 | 0.71 | 0.83 | 0.88 | 0.82 | 0.94 | 0.72 | 0.65 | 0.80 | 0.77 | 0.72 | 0.82 | 0.86 | 0.80 | 0.92 | 0.73 | 0.68 | 0.79 |
| 761 | 0.72 | 0.63 | 0.81 | 0.81 | 0.65 | 0.96 | 0.69 | 0.58 | 0.80 | 0.75 | 0.69 | 0.82 | 0.83 | 0.72 | 0.93 | 0.73 | 0.65 | 0.80 | 0.76 | 0.71 | 0.81 | 0.83 | 0.75 | 0.92 | 0.74 | 0.68 | 0.79 |
| 762 | 0.75 | 0.68 | 0.83 | 0.86 | 0.78 | 0.94 | 0.72 | 0.63 | 0.81 | 0.75 | 0.69 | 0.81 | 0.80 | 0.70 | 0.89 | 0.73 | 0.66 | 0.81 | 0.77 | 0.73 | 0.82 | 0.84 | 0.77 | 0.90 | 0.75 | 0.69 | 0.80 |
| 763 | 0.75 | 0.66 | 0.83 | 0.84 | 0.67 | 1.00 | 0.72 | 0.63 | 0.81 | 0.76 | 0.70 | 0.82 | 0.90 | 0.86 | 0.95 | 0.70 | 0.63 | 0.77 | 0.78 | 0.73 | 0.82 | 0.90 | 0.85 | 0.95 | 0.73 | 0.67 | 0.78 |
| 764 | 0.74 | 0.67 | 0.81 | 0.77 | 0.62 | 0.92 | 0.73 | 0.65 | 0.81 | 0.77 | 0.71 | 0.83 | 0.84 | 0.76 | 0.93 | 0.74 | 0.67 | 0.81 | 0.76 | 0.72 | 0.81 | 0.82 | 0.75 | 0.90 | 0.74 | 0.69 | 0.79 |
| 765 | 0.75 | 0.67 | 0.83 | 0.82 | 0.65 | 0.99 | 0.72 | 0.64 | 0.81 | 0.77 | 0.71 | 0.83 | 0.87 | 0.81 | 0.93 | 0.73 | 0.66 | 0.80 | 0.78 | 0.74 | 0.83 | 0.87 | 0.81 | 0.93 | 0.75 | 0.70 | 0.80 |
| 766 | 0.74 | 0.66 | 0.82 | 0.81 | 0.70 | 0.91 | 0.72 | 0.63 | 0.81 | 0.77 | 0.71 | 0.82 | 0.83 | 0.75 | 0.90 | 0.74 | 0.67 | 0.81 | 0.76 | 0.72 | 0.81 | 0.83 | 0.77 | 0.88 | 0.74 | 0.68 | 0.79 |
| 767 | 0.75 | 0.68 | 0.82 | 0.82 | 0.69 | 0.94 | 0.73 | 0.64 | 0.81 | 0.77 | 0.71 | 0.82 | 0.84 | 0.76 | 0.92 | 0.73 | 0.67 | 0.80 | 0.78 | 0.74 | 0.82 | 0.85 | 0.78 | 0.91 | 0.75 | 0.70 | 0.80 |
| 768 | 0.77 | 0.69 | 0.84 | 0.88 | 0.81 | 0.95 | 0.73 | 0.64 | 0.82 | 0.76 | 0.69 | 0.83 | 0.86 | 0.75 | 0.96 | 0.73 | 0.65 | 0.82 | 0.78 | 0.73 | 0.82 | 0.87 | 0.81 | 0.94 | 0.74 | 0.68 | 0.80 |
| 769 | 0.75 | 0.67 | 0.83 | 0.82 | 0.71 | 0.92 | 0.73 | 0.63 | 0.82 | 0.77 | 0.71 | 0.83 | 0.84 | 0.75 | 0.92 | 0.74 | 0.66 | 0.81 | 0.77 | 0.72 | 0.82 | 0.84 | 0.78 | 0.90 | 0.74 | 0.69 | 0.80 |
| 770 | 0.76 | 0.69 | 0.83 | 0.82 | 0.69 | 0.95 | 0.74 | 0.66 | 0.83 | 0.77 | 0.71 | 0.82 | 0.83 | 0.74 | 0.92 | 0.74 | 0.67 | 0.81 | 0.78 | 0.73 | 0.82 | 0.84 | 0.77 | 0.91 | 0.75 | 0.70 | 0.80 |
| 771 | 0.75 | 0.68 | 0.83 | 0.85 | 0.73 | 0.96 | 0.72 | 0.64 | 0.81 | 0.77 | 0.71 | 0.83 | 0.85 | 0.77 | 0.92 | 0.74 | 0.67 | 0.81 | 0.78 | 0.73 | 0.82 | 0.86 | 0.80 | 0.92 | 0.74 | 0.69 | 0.80 |
| 772 | 0.74 | 0.66 | 0.83 | 0.78 | 0.61 | 0.94 | 0.73 | 0.64 | 0.83 | 0.77 | 0.71 | 0.83 | 0.84 | 0.76 | 0.93 | 0.74 | 0.67 | 0.81 | 0.78 | 0.73 | 0.82 | 0.83 | 0.76 | 0.91 | 0.75 | 0.70 | 0.81 |
| 773 | 0.74 | 0.66 | 0.83 | 0.86 | 0.70 | 1.00 | 0.71 | 0.62 | 0.81 | 0.77 | 0.71 | 0.82 | 0.89 | 0.82 | 0.95 | 0.72 | 0.65 | 0.79 | 0.78 | 0.74 | 0.83 | 0.90 | 0.84 | 0.96 | 0.74 | 0.69 | 0.79 |
| 774 | 0.75 | 0.66 | 0.83 | 0.85 | 0.76 | 0.94 | 0.72 | 0.62 | 0.82 | 0.76 | 0.70 | 0.82 | 0.86 | 0.79 | 0.92 | 0.72 | 0.65 | 0.80 | 0.77 | 0.72 | 0.81 | 0.86 | 0.81 | 0.91 | 0.73 | 0.68 | 0.79 |
| 775 | 0.75 | 0.68 | 0.82 | 0.79 | 0.64 | 0.93 | 0.73 | 0.66 | 0.81 | 0.77 | 0.71 | 0.82 | 0.81 | 0.72 | 0.91 | 0.74 | 0.67 | 0.81 | 0.77 | 0.72 | 0.81 | 0.82 | 0.74 | 0.89 | 0.75 | 0.70 | 0.80 |
| 776 | 0.77 | 0.70 | 0.84 | 0.86 | 0.77 | 0.95 | 0.74 | 0.66 | 0.82 | 0.77 | 0.71 | 0.82 | 0.86 | 0.78 | 0.94 | 0.73 | 0.66 | 0.80 | 0.78 | 0.74 | 0.82 | 0.87 | 0.81 | 0.92 | 0.75 | 0.70 | 0.80 |
| 777 | 0.74 | 0.67 | 0.81 | 0.85 | 0.76 | 0.94 | 0.71 | 0.62 | 0.79 | 0.76 | 0.70 | 0.82 | 0.83 | 0.76 | 0.91 | 0.73 | 0.66 | 0.80 | 0.76 | 0.72 | 0.81 | 0.85 | 0.79 | 0.90 | 0.73 | 0.68 | 0.78 |
| 778 | 0.77 | 0.70 | 0.84 | 0.86 | 0.76 | 0.95 | 0.74 | 0.66 | 0.82 | 0.77 | 0.71 | 0.82 | 0.84 | 0.76 | 0.92 | 0.74 | 0.67 | 0.81 | 0.78 | 0.74 | 0.82 | 0.85 | 0.80 | 0.91 | 0.75 | 0.70 | 0.80 |
| 779 | 0.76 | 0.69 | 0.84 | 0.83 | 0.73 | 0.94 | 0.74 | 0.65 | 0.83 | 0.75 | 0.69 | 0.81 | 0.80 | 0.72 | 0.89 | 0.73 | 0.66 | 0.81 | 0.76 | 0.71 | 0.80 | 0.81 | 0.75 | 0.88 | 0.73 | 0.68 | 0.79 |
| 780 | 0.75 | 0.67 | 0.83 | 0.84 | 0.66 | 1.00 | 0.72 | 0.63 | 0.82 | 0.76 | 0.70 | 0.82 | 0.89 | 0.84 | 0.94 | 0.70 | 0.63 | 0.77 | 0.78 | 0.74 | 0.82 | 0.90 | 0.84 | 0.95 | 0.73 | 0.68 | 0.79 |
| 781 | 0.75 | 0.67 | 0.82 | 0.84 | 0.74 | 0.94 | 0.72 | 0.63 | 0.80 | 0.77 | 0.71 | 0.82 | 0.83 | 0.75 | 0.92 | 0.74 | 0.67 | 0.80 | 0.77 | 0.73 | 0.82 | 0.85 | 0.79 | 0.91 | 0.74 | 0.69 | 0.79 |
| 782 | 0.76 | 0.67 | 0.84 | 0.86 | 0.76 | 0.97 | 0.72 | 0.62 | 0.82 | 0.75 | 0.68 | 0.81 | 0.81 | 0.70 | 0.92 | 0.72 | 0.65 | 0.80 | 0.75 | 0.71 | 0.80 | 0.83 | 0.75 | 0.91 | 0.73 | 0.67 | 0.78 |
| 783 | 0.75 | 0.66 | 0.83 | 0.87 | 0.74 | 1.00 | 0.71 | 0.61 | 0.81 | 0.76 | 0.71 | 0.82 | 0.89 | 0.83 | 0.94 | 0.71 | 0.64 | 0.78 | 0.78 | 0.74 | 0.82 | 0.90 | 0.85 | 0.94 | 0.73 | 0.68 | 0.79 |
| 784 | 0.77 | 0.70 | 0.84 | 0.86 | 0.77 | 0.95 | 0.74 | 0.66 | 0.83 | 0.77 | 0.71 | 0.83 | 0.86 | 0.78 | 0.94 | 0.73 | 0.66 | 0.80 | 0.78 | 0.74 | 0.82 | 0.87 | 0.81 | 0.92 | 0.75 | 0.70 | 0.80 |
| 785 | 0.74 | 0.66 | 0.83 | 0.76 | 0.59 | 0.93 | 0.74 | 0.64 | 0.84 | 0.77 | 0.71 | 0.83 | 0.84 | 0.76 | 0.92 | 0.74 | 0.67 | 0.81 | 0.78 | 0.73 | 0.82 | 0.83 | 0.76 | 0.91 | 0.75 | 0.70 | 0.81 |
| 786 | 0.76 | 0.68 | 0.84 | 0.84 | 0.70 | 0.97 | 0.74 | 0.64 | 0.83 | 0.77 | 0.71 | 0.83 | 0.85 | 0.78 | 0.93 | 0.74 | 0.67 | 0.81 | 0.78 | 0.74 | 0.83 | 0.86 | 0.80 | 0.92 | 0.75 | 0.70 | 0.81 |
| 787 | 0.74 | 0.66 | 0.83 | 0.78 | 0.61 | 0.95 | 0.73 | 0.63 | 0.83 | 0.77 | 0.71 | 0.83 | 0.85 | 0.78 | 0.92 | 0.74 | 0.67 | 0.81 | 0.77 | 0.73 | 0.82 | 0.84 | 0.77 | 0.91 | 0.75 | 0.69 | 0.80 |
| 788 | 0.75 | 0.68 | 0.82 | 0.83 | 0.74 | 0.91 | 0.73 | 0.65 | 0.81 | 0.77 | 0.71 | 0.82 | 0.85 | 0.78 | 0.91 | 0.73 | 0.66 | 0.80 | 0.77 | 0.73 | 0.81 | 0.85 | 0.80 | 0.90 | 0.74 | 0.69 | 0.79 |
| 789 | 0.74 | 0.67 | 0.82 | 0.83 | 0.69 | 0.96 | 0.72 | 0.62 | 0.81 | 0.77 | 0.71 | 0.83 | 0.87 | 0.81 | 0.94 | 0.73 | 0.66 | 0.80 | 0.77 | 0.73 | 0.81 | 0.87 | 0.81 | 0.92 | 0.73 | 0.68 | 0.79 |
| 790 | 0.74 | 0.67 | 0.82 | 0.80 | 0.67 | 0.94 | 0.73 | 0.63 | 0.82 | 0.76 | 0.71 | 0.82 | 0.83 | 0.76 | 0.91 | 0.73 | 0.67 | 0.80 | 0.76 | 0.71 | 0.80 | 0.82 | 0.76 | 0.89 | 0.73 | 0.68 | 0.78 |

| No. | G | H | I | J | K | L | M | N | O | P | Q | R | S | T | U | V | W | Z | AA | AB | AC | AD | AE | AF | AG | AH | AI |
|-----|---|---|---|---|---|---|---|---|---|---|---|---|---|---|---|---|---|---|----|----|----|----|----|----|----|----|----|
| 791 | 0.74 | 0.66 | 0.83 | 0.80 | 0.61 | 0.98 | 0.73 | 0.64 | 0.82 | 0.77 | 0.72 | 0.83 | 0.87 | 0.81 | 0.93 | 0.73 | 0.66 | 0.81 | 0.78 | 0.74 | 0.83 | 0.86 | 0.80 | 0.93 | 0.75 | 0.70 | 0.81 |
| 792 | 0.74 | 0.66 | 0.82 | 0.82 | 0.69 | 0.95 | 0.71 | 0.62 | 0.80 | 0.74 | 0.68 | 0.80 | 0.77 | 0.69 | 0.86 | 0.73 | 0.66 | 0.80 | 0.76 | 0.72 | 0.80 | 0.81 | 0.75 | 0.88 | 0.74 | 0.69 | 0.79 |
| 793 | 0.74 | 0.66 | 0.81 | 0.81 | 0.70 | 0.92 | 0.71 | 0.63 | 0.80 | 0.76 | 0.70 | 0.82 | 0.83 | 0.73 | 0.92 | 0.74 | 0.67 | 0.81 | 0.77 | 0.72 | 0.81 | 0.83 | 0.76 | 0.90 | 0.74 | 0.69 | 0.80 |
| 794 | 0.74 | 0.66 | 0.81 | 0.77 | 0.64 | 0.89 | 0.73 | 0.64 | 0.81 | 0.76 | 0.71 | 0.82 | 0.83 | 0.75 | 0.91 | 0.74 | 0.67 | 0.80 | 0.77 | 0.73 | 0.81 | 0.82 | 0.76 | 0.89 | 0.75 | 0.70 | 0.80 |
| 795 | 0.77 | 0.68 | 0.86 | 0.86 | 0.76 | 0.96 | 0.74 | 0.64 | 0.85 | 0.76 | 0.70 | 0.82 | 0.83 | 0.76 | 0.90 | 0.74 | 0.66 | 0.81 | 0.78 | 0.73 | 0.82 | 0.85 | 0.79 | 0.90 | 0.75 | 0.69 | 0.81 |
| 796 | 0.76 | 0.68 | 0.84 | 0.85 | 0.72 | 0.98 | 0.73 | 0.64 | 0.83 | 0.77 | 0.71 | 0.83 | 0.86 | 0.79 | 0.93 | 0.73 | 0.66 | 0.80 | 0.78 | 0.73 | 0.82 | 0.87 | 0.81 | 0.93 | 0.74 | 0.69 | 0.80 |
| 797 | 0.77 | 0.70 | 0.84 | 0.88 | 0.80 | 0.96 | 0.74 | 0.65 | 0.82 | 0.76 | 0.71 | 0.82 | 0.87 | 0.80 | 0.94 | 0.72 | 0.65 | 0.79 | 0.77 | 0.73 | 0.82 | 0.87 | 0.82 | 0.92 | 0.73 | 0.68 | 0.79 |
| 798 | 0.75 | 0.67 | 0.82 | 0.81 | 0.70 | 0.91 | 0.73 | 0.64 | 0.82 | 0.76 | 0.71 | 0.82 | 0.82 | 0.75 | 0.89 | 0.74 | 0.67 | 0.81 | 0.75 | 0.71 | 0.80 | 0.81 | 0.75 | 0.87 | 0.73 | 0.68 | 0.78 |
| 799 | 0.75 | 0.66 | 0.83 | 0.81 | 0.66 | 0.97 | 0.73 | 0.63 | 0.83 | 0.77 | 0.71 | 0.83 | 0.87 | 0.80 | 0.93 | 0.73 | 0.66 | 0.80 | 0.77 | 0.73 | 0.82 | 0.86 | 0.80 | 0.92 | 0.74 | 0.69 | 0.80 |
| 800 | 0.75 | 0.66 | 0.83 | 0.75 | 0.56 | 0.95 | 0.74 | 0.65 | 0.84 | 0.78 | 0.73 | 0.84 | 0.85 | 0.78 | 0.92 | 0.75 | 0.69 | 0.82 | 0.79 | 0.75 | 0.83 | 0.84 | 0.76 | 0.92 | 0.77 | 0.72 | 0.82 |
| 801 | 0.76 | 0.68 | 0.85 | 0.85 | 0.75 | 0.95 | 0.74 | 0.63 | 0.84 | 0.76 | 0.70 | 0.83 | 0.85 | 0.77 | 0.92 | 0.73 | 0.66 | 0.81 | 0.78 | 0.73 | 0.82 | 0.86 | 0.80 | 0.91 | 0.75 | 0.69 | 0.81 |
| 802 | 0.76 | 0.68 | 0.83 | 0.87 | 0.76 | 0.98 | 0.72 | 0.63 | 0.81 | 0.76 | 0.71 | 0.82 | 0.85 | 0.77 | 0.93 | 0.73 | 0.66 | 0.80 | 0.76 | 0.72 | 0.81 | 0.86 | 0.79 | 0.92 | 0.73 | 0.67 | 0.78 |
| 803 | 0.75 | 0.67 | 0.83 | 0.82 | 0.72 | 0.92 | 0.73 | 0.63 | 0.82 | 0.76 | 0.70 | 0.83 | 0.83 | 0.75 | 0.92 | 0.73 | 0.66 | 0.81 | 0.77 | 0.72 | 0.82 | 0.84 | 0.78 | 0.90 | 0.74 | 0.68 | 0.80 |
| 804 | 0.74 | 0.67 | 0.82 | 0.79 | 0.65 | 0.92 | 0.73 | 0.65 | 0.81 | 0.77 | 0.71 | 0.82 | 0.84 | 0.76 | 0.92 | 0.73 | 0.67 | 0.80 | 0.77 | 0.72 | 0.81 | 0.83 | 0.76 | 0.90 | 0.74 | 0.69 | 0.79 |
| 805 | 0.74 | 0.66 | 0.83 | 0.82 | 0.67 | 0.96 | 0.72 | 0.62 | 0.82 | 0.77 | 0.71 | 0.83 | 0.84 | 0.77 | 0.92 | 0.74 | 0.66 | 0.81 | 0.77 | 0.72 | 0.82 | 0.84 | 0.78 | 0.91 | 0.74 | 0.68 | 0.80 |
| 806 | 0.74 | 0.66 | 0.83 | 0.79 | 0.64 | 0.94 | 0.73 | 0.63 | 0.82 | 0.77 | 0.71 | 0.83 | 0.87 | 0.80 | 0.93 | 0.73 | 0.66 | 0.80 | 0.77 | 0.72 | 0.81 | 0.85 | 0.79 | 0.91 | 0.73 | 0.68 | 0.79 |
| 807 | 0.73 | 0.66 | 0.80 | 0.77 | 0.65 | 0.89 | 0.71 | 0.63 | 0.80 | 0.76 | 0.70 | 0.82 | 0.83 | 0.74 | 0.92 | 0.74 | 0.67 | 0.81 | 0.76 | 0.72 | 0.81 | 0.82 | 0.75 | 0.89 | 0.74 | 0.69 | 0.79 |
| 808 | 0.75 | 0.67 | 0.83 | 0.84 | 0.71 | 0.96 | 0.72 | 0.63 | 0.81 | 0.76 | 0.70 | 0.82 | 0.84 | 0.76 | 0.91 | 0.73 | 0.66 | 0.79 | 0.76 | 0.72 | 0.81 | 0.84 | 0.78 | 0.90 | 0.73 | 0.68 | 0.78 |
| 809 | 0.74 | 0.67 | 0.81 | 0.78 | 0.64 | 0.91 | 0.73 | 0.65 | 0.81 | 0.77 | 0.71 | 0.82 | 0.84 | 0.77 | 0.92 | 0.73 | 0.66 | 0.80 | 0.77 | 0.73 | 0.81 | 0.83 | 0.77 | 0.90 | 0.74 | 0.69 | 0.79 |
| 810 | 0.75 | 0.67 | 0.83 | 0.78 | 0.60 | 0.95 | 0.74 | 0.65 | 0.83 | 0.78 | 0.72 | 0.84 | 0.83 | 0.74 | 0.92 | 0.76 | 0.69 | 0.82 | 0.78 | 0.74 | 0.83 | 0.83 | 0.75 | 0.91 | 0.77 | 0.72 | 0.82 |
| 811 | 0.75 | 0.66 | 0.83 | 0.88 | 0.73 | 1.00 | 0.71 | 0.61 | 0.80 | 0.76 | 0.70 | 0.82 | 0.88 | 0.81 | 0.96 | 0.70 | 0.63 | 0.77 | 0.77 | 0.73 | 0.82 | 0.89 | 0.83 | 0.96 | 0.73 | 0.67 | 0.78 |
| 812 | 0.75 | 0.66 | 0.84 | 0.90 | 0.77 | 1.00 | 0.70 | 0.59 | 0.81 | 0.75 | 0.68 | 0.81 | 0.83 | 0.72 | 0.94 | 0.71 | 0.64 | 0.79 | 0.77 | 0.72 | 0.82 | 0.87 | 0.79 | 0.95 | 0.73 | 0.68 | 0.79 |
| 813 | 0.74 | 0.66 | 0.83 | 0.77 | 0.58 | 0.96 | 0.73 | 0.64 | 0.83 | 0.78 | 0.72 | 0.84 | 0.83 | 0.74 | 0.91 | 0.76 | 0.70 | 0.82 | 0.78 | 0.74 | 0.83 | 0.83 | 0.75 | 0.91 | 0.76 | 0.71 | 0.81 |
| 814 | 0.76 | 0.69 | 0.83 | 0.82 | 0.70 | 0.94 | 0.74 | 0.66 | 0.82 | 0.76 | 0.71 | 0.82 | 0.84 | 0.76 | 0.92 | 0.73 | 0.67 | 0.80 | 0.78 | 0.73 | 0.82 | 0.85 | 0.78 | 0.91 | 0.75 | 0.70 | 0.80 |
| 815 | 0.74 | 0.67 | 0.81 | 0.77 | 0.62 | 0.91 | 0.73 | 0.65 | 0.81 | 0.77 | 0.71 | 0.82 | 0.84 | 0.75 | 0.92 | 0.74 | 0.67 | 0.81 | 0.77 | 0.72 | 0.81 | 0.82 | 0.75 | 0.89 | 0.75 | 0.70 | 0.80 |
| 816 | 0.74 | 0.66 | 0.83 | 0.80 | 0.62 | 0.98 | 0.73 | 0.63 | 0.82 | 0.78 | 0.72 | 0.84 | 0.82 | 0.72 | 0.91 | 0.76 | 0.70 | 0.83 | 0.78 | 0.73 | 0.82 | 0.82 | 0.74 | 0.91 | 0.76 | 0.71 | 0.81 |
| 817 | 0.73 | 0.66 | 0.81 | 0.79 | 0.67 | 0.91 | 0.72 | 0.63 | 0.80 | 0.76 | 0.70 | 0.82 | 0.83 | 0.73 | 0.92 | 0.74 | 0.67 | 0.81 | 0.77 | 0.72 | 0.81 | 0.82 | 0.75 | 0.90 | 0.75 | 0.69 | 0.80 |
| 818 | 0.74 | 0.66 | 0.83 | 0.89 | 0.74 | 1.00 | 0.70 | 0.60 | 0.80 | 0.76 | 0.70 | 0.82 | 0.89 | 0.81 | 0.96 | 0.70 | 0.63 | 0.77 | 0.77 | 0.73 | 0.82 | 0.90 | 0.84 | 0.96 | 0.72 | 0.67 | 0.78 |
| 819 | 0.75 | 0.68 | 0.81 | 0.85 | 0.73 | 0.97 | 0.71 | 0.64 | 0.79 | 0.77 | 0.71 | 0.83 | 0.86 | 0.78 | 0.94 | 0.73 | 0.66 | 0.80 | 0.77 | 0.72 | 0.81 | 0.86 | 0.80 | 0.92 | 0.73 | 0.68 | 0.78 |
| 820 | 0.74 | 0.66 | 0.83 | 0.84 | 0.70 | 0.98 | 0.71 | 0.62 | 0.81 | 0.76 | 0.70 | 0.82 | 0.87 | 0.79 | 0.96 | 0.72 | 0.65 | 0.79 | 0.78 | 0.73 | 0.82 | 0.87 | 0.80 | 0.94 | 0.74 | 0.69 | 0.79 |
| 821 | 0.74 | 0.67 | 0.82 | 0.77 | 0.59 | 0.95 | 0.74 | 0.65 | 0.82 | 0.77 | 0.71 | 0.83 | 0.87 | 0.80 | 0.93 | 0.73 | 0.66 | 0.80 | 0.77 | 0.72 | 0.81 | 0.85 | 0.77 | 0.92 | 0.74 | 0.69 | 0.79 |
| 822 | 0.74 | 0.67 | 0.81 | 0.80 | 0.68 | 0.92 | 0.72 | 0.63 | 0.81 | 0.76 | 0.71 | 0.82 | 0.83 | 0.74 | 0.91 | 0.74 | 0.67 | 0.81 | 0.77 | 0.73 | 0.81 | 0.83 | 0.77 | 0.90 | 0.75 | 0.69 | 0.80 |
| 823 | 0.74 | 0.66 | 0.81 | 0.76 | 0.63 | 0.90 | 0.73 | 0.64 | 0.81 | 0.76 | 0.71 | 0.82 | 0.84 | 0.75 | 0.93 | 0.73 | 0.67 | 0.80 | 0.77 | 0.72 | 0.81 | 0.82 | 0.75 | 0.89 | 0.75 | 0.70 | 0.80 |

114

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 824 | 0.74 | 0.66 | 0.83 | 0.78 | 0.65 | 0.91 | 0.73 | 0.64 | 0.83 | 0.76 | 0.71 | 0.82 | 0.85 | 0.78 | 0.92 | 0.72 | 0.66 | 0.79 | 0.78 | 0.73 | 0.82 | 0.85 | 0.79 | 0.91 | 0.75 | 0.70 | 0.80 |
| 825 | 0.76 | 0.68 | 0.84 | 0.85 | 0.73 | 0.96 | 0.73 | 0.63 | 0.82 | 0.75 | 0.68 | 0.81 | 0.81 | 0.71 | 0.92 | 0.72 | 0.65 | 0.79 | 0.76 | 0.71 | 0.81 | 0.83 | 0.75 | 0.91 | 0.73 | 0.68 | 0.79 |
| 826 | 0.77 | 0.70 | 0.84 | 0.87 | 0.78 | 0.96 | 0.74 | 0.65 | 0.82 | 0.76 | 0.70 | 0.82 | 0.87 | 0.80 | 0.94 | 0.72 | 0.64 | 0.79 | 0.77 | 0.73 | 0.82 | 0.87 | 0.82 | 0.92 | 0.73 | 0.68 | 0.79 |
| 827 | 0.75 | 0.67 | 0.83 | 0.83 | 0.70 | 0.96 | 0.72 | 0.63 | 0.82 | 0.77 | 0.71 | 0.83 | 0.87 | 0.80 | 0.94 | 0.72 | 0.65 | 0.80 | 0.77 | 0.73 | 0.82 | 0.86 | 0.80 | 0.92 | 0.73 | 0.68 | 0.79 |
| 828 | 0.74 | 0.67 | 0.81 | 0.81 | 0.69 | 0.94 | 0.71 | 0.63 | 0.80 | 0.76 | 0.71 | 0.82 | 0.84 | 0.76 | 0.92 | 0.73 | 0.66 | 0.80 | 0.77 | 0.73 | 0.81 | 0.84 | 0.78 | 0.91 | 0.74 | 0.69 | 0.79 |
| 829 | 0.76 | 0.69 | 0.83 | 0.85 | 0.73 | 0.96 | 0.73 | 0.65 | 0.81 | 0.76 | 0.70 | 0.82 | 0.82 | 0.73 | 0.90 | 0.74 | 0.67 | 0.81 | 0.77 | 0.72 | 0.81 | 0.83 | 0.76 | 0.90 | 0.74 | 0.69 | 0.79 |
| 830 | 0.75 | 0.69 | 0.82 | 0.82 | 0.74 | 0.91 | 0.73 | 0.65 | 0.81 | 0.76 | 0.71 | 0.82 | 0.85 | 0.79 | 0.92 | 0.73 | 0.65 | 0.80 | 0.77 | 0.73 | 0.82 | 0.86 | 0.81 | 0.90 | 0.74 | 0.69 | 0.79 |
| 831 | 0.75 | 0.67 | 0.83 | 0.83 | 0.71 | 0.95 | 0.73 | 0.64 | 0.82 | 0.76 | 0.70 | 0.82 | 0.85 | 0.78 | 0.91 | 0.73 | 0.65 | 0.80 | 0.77 | 0.72 | 0.82 | 0.86 | 0.80 | 0.91 | 0.74 | 0.68 | 0.79 |
| 832 | 0.76 | 0.68 | 0.84 | 0.88 | 0.78 | 0.97 | 0.72 | 0.62 | 0.82 | 0.74 | 0.68 | 0.81 | 0.80 | 0.68 | 0.91 | 0.73 | 0.65 | 0.80 | 0.75 | 0.70 | 0.80 | 0.82 | 0.74 | 0.90 | 0.73 | 0.67 | 0.78 |
| 833 | 0.76 | 0.68 | 0.84 | 0.86 | 0.74 | 0.97 | 0.73 | 0.63 | 0.82 | 0.77 | 0.71 | 0.83 | 0.85 | 0.77 | 0.92 | 0.74 | 0.67 | 0.80 | 0.78 | 0.73 | 0.82 | 0.86 | 0.80 | 0.92 | 0.74 | 0.69 | 0.80 |
| 834 | 0.76 | 0.68 | 0.84 | 0.83 | 0.70 | 0.96 | 0.74 | 0.64 | 0.83 | 0.77 | 0.71 | 0.83 | 0.90 | 0.85 | 0.95 | 0.71 | 0.64 | 0.78 | 0.77 | 0.73 | 0.82 | 0.88 | 0.83 | 0.93 | 0.73 | 0.67 | 0.79 |
| 835 | 0.77 | 0.70 | 0.84 | 0.82 | 0.68 | 0.96 | 0.75 | 0.67 | 0.83 | 0.76 | 0.71 | 0.82 | 0.84 | 0.77 | 0.92 | 0.73 | 0.66 | 0.80 | 0.77 | 0.73 | 0.82 | 0.84 | 0.78 | 0.91 | 0.75 | 0.69 | 0.80 |
| 836 | 0.73 | 0.66 | 0.81 | 0.77 | 0.63 | 0.91 | 0.72 | 0.64 | 0.81 | 0.76 | 0.71 | 0.82 | 0.84 | 0.77 | 0.92 | 0.73 | 0.66 | 0.80 | 0.76 | 0.71 | 0.80 | 0.82 | 0.76 | 0.89 | 0.73 | 0.68 | 0.78 |
| 837 | 0.76 | 0.68 | 0.84 | 0.82 | 0.71 | 0.94 | 0.74 | 0.65 | 0.84 | 0.77 | 0.71 | 0.83 | 0.87 | 0.81 | 0.93 | 0.72 | 0.65 | 0.80 | 0.77 | 0.73 | 0.82 | 0.86 | 0.81 | 0.92 | 0.74 | 0.68 | 0.80 |
| 838 | 0.75 | 0.67 | 0.83 | 0.82 | 0.69 | 0.96 | 0.73 | 0.63 | 0.82 | 0.77 | 0.71 | 0.82 | 0.89 | 0.84 | 0.94 | 0.71 | 0.64 | 0.78 | 0.77 | 0.73 | 0.82 | 0.88 | 0.83 | 0.93 | 0.73 | 0.67 | 0.79 |
| 839 | 0.74 | 0.65 | 0.83 | 0.75 | 0.55 | 0.96 | 0.74 | 0.64 | 0.83 | 0.78 | 0.73 | 0.84 | 0.85 | 0.78 | 0.92 | 0.75 | 0.69 | 0.82 | 0.79 | 0.74 | 0.83 | 0.84 | 0.76 | 0.92 | 0.77 | 0.72 | 0.82 |
| 840 | 0.76 | 0.67 | 0.84 | 0.84 | 0.71 | 0.97 | 0.73 | 0.63 | 0.83 | 0.77 | 0.71 | 0.83 | 0.86 | 0.79 | 0.92 | 0.73 | 0.66 | 0.80 | 0.77 | 0.73 | 0.82 | 0.86 | 0.80 | 0.92 | 0.74 | 0.68 | 0.80 |
| 841 | 0.75 | 0.67 | 0.83 | 0.80 | 0.66 | 0.95 | 0.73 | 0.64 | 0.82 | 0.77 | 0.71 | 0.82 | 0.88 | 0.82 | 0.94 | 0.72 | 0.65 | 0.79 | 0.77 | 0.72 | 0.81 | 0.86 | 0.80 | 0.92 | 0.73 | 0.68 | 0.78 |
| 842 | 0.74 | 0.67 | 0.82 | 0.77 | 0.60 | 0.95 | 0.73 | 0.65 | 0.82 | 0.77 | 0.71 | 0.83 | 0.86 | 0.80 | 0.93 | 0.73 | 0.66 | 0.80 | 0.77 | 0.73 | 0.82 | 0.85 | 0.78 | 0.92 | 0.75 | 0.70 | 0.80 |
| 843 | 0.75 | 0.66 | 0.83 | 0.82 | 0.68 | 0.96 | 0.72 | 0.62 | 0.82 | 0.77 | 0.71 | 0.83 | 0.89 | 0.84 | 0.94 | 0.71 | 0.64 | 0.79 | 0.77 | 0.72 | 0.81 | 0.87 | 0.82 | 0.93 | 0.72 | 0.67 | 0.78 |
| 844 | 0.75 | 0.67 | 0.83 | 0.82 | 0.68 | 0.96 | 0.73 | 0.64 | 0.83 | 0.76 | 0.70 | 0.82 | 0.84 | 0.77 | 0.92 | 0.72 | 0.66 | 0.79 | 0.76 | 0.72 | 0.80 | 0.84 | 0.78 | 0.90 | 0.73 | 0.68 | 0.78 |
| 845 | 0.76 | 0.68 | 0.84 | 0.87 | 0.73 | 1.00 | 0.72 | 0.63 | 0.82 | 0.76 | 0.70 | 0.82 | 0.85 | 0.75 | 0.95 | 0.73 | 0.66 | 0.79 | 0.78 | 0.73 | 0.82 | 0.87 | 0.79 | 0.94 | 0.75 | 0.69 | 0.80 |
| 846 | 0.75 | 0.67 | 0.84 | 0.82 | 0.66 | 0.97 | 0.73 | 0.63 | 0.83 | 0.77 | 0.71 | 0.83 | 0.86 | 0.80 | 0.93 | 0.73 | 0.66 | 0.80 | 0.77 | 0.73 | 0.82 | 0.86 | 0.79 | 0.92 | 0.74 | 0.69 | 0.80 |
| 847 | 0.74 | 0.66 | 0.83 | 0.82 | 0.70 | 0.94 | 0.72 | 0.62 | 0.82 | 0.75 | 0.69 | 0.81 | 0.78 | 0.70 | 0.87 | 0.73 | 0.66 | 0.80 | 0.75 | 0.70 | 0.79 | 0.79 | 0.73 | 0.86 | 0.73 | 0.67 | 0.78 |
| 848 | 0.76 | 0.68 | 0.85 | 0.84 | 0.72 | 0.96 | 0.74 | 0.64 | 0.84 | 0.76 | 0.71 | 0.82 | 0.85 | 0.77 | 0.92 | 0.73 | 0.66 | 0.80 | 0.78 | 0.74 | 0.83 | 0.86 | 0.80 | 0.92 | 0.75 | 0.69 | 0.80 |
| 849 | 0.74 | 0.67 | 0.82 | 0.77 | 0.65 | 0.89 | 0.74 | 0.66 | 0.82 | 0.76 | 0.70 | 0.82 | 0.83 | 0.76 | 0.91 | 0.73 | 0.66 | 0.80 | 0.76 | 0.71 | 0.80 | 0.81 | 0.75 | 0.87 | 0.74 | 0.68 | 0.79 |
| 850 | 0.73 | 0.64 | 0.83 | 0.84 | 0.67 | 1.00 | 0.70 | 0.60 | 0.81 | 0.76 | 0.69 | 0.82 | 0.88 | 0.80 | 0.97 | 0.71 | 0.63 | 0.79 | 0.77 | 0.72 | 0.82 | 0.88 | 0.81 | 0.95 | 0.73 | 0.67 | 0.79 |
| 851 | 0.74 | 0.67 | 0.81 | 0.83 | 0.74 | 0.92 | 0.71 | 0.62 | 0.80 | 0.76 | 0.70 | 0.82 | 0.86 | 0.78 | 0.93 | 0.72 | 0.65 | 0.79 | 0.76 | 0.71 | 0.80 | 0.85 | 0.79 | 0.91 | 0.72 | 0.67 | 0.77 |
| 852 | 0.74 | 0.67 | 0.82 | 0.80 | 0.68 | 0.92 | 0.72 | 0.64 | 0.81 | 0.76 | 0.71 | 0.82 | 0.83 | 0.75 | 0.91 | 0.73 | 0.66 | 0.80 | 0.77 | 0.73 | 0.82 | 0.84 | 0.77 | 0.90 | 0.75 | 0.70 | 0.80 |
| 853 | 0.75 | 0.68 | 0.83 | 0.84 | 0.72 | 0.97 | 0.72 | 0.63 | 0.81 | 0.76 | 0.70 | 0.82 | 0.85 | 0.77 | 0.92 | 0.72 | 0.65 | 0.80 | 0.76 | 0.72 | 0.81 | 0.85 | 0.78 | 0.91 | 0.73 | 0.67 | 0.78 |
| 854 | 0.74 | 0.67 | 0.81 | 0.86 | 0.75 | 0.96 | 0.71 | 0.62 | 0.79 | 0.76 | 0.71 | 0.82 | 0.84 | 0.75 | 0.92 | 0.73 | 0.67 | 0.80 | 0.77 | 0.73 | 0.81 | 0.85 | 0.79 | 0.92 | 0.74 | 0.69 | 0.79 |
| 855 | 0.69 | 0.61 | 0.76 | 0.72 | 0.59 | 0.86 | 0.68 | 0.60 | 0.76 | 0.76 | 0.71 | 0.82 | 0.82 | 0.74 | 0.90 | 0.74 | 0.67 | 0.80 | 0.74 | 0.70 | 0.79 | 0.80 | 0.73 | 0.86 | 0.72 | 0.67 | 0.77 |
| 856 | 0.74 | 0.67 | 0.81 | 0.78 | 0.65 | 0.91 | 0.72 | 0.64 | 0.80 | 0.76 | 0.70 | 0.82 | 0.84 | 0.76 | 0.92 | 0.73 | 0.66 | 0.80 | 0.76 | 0.71 | 0.80 | 0.83 | 0.76 | 0.90 | 0.73 | 0.68 | 0.78 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 857 | 0.73 | 0.66 | 0.81 | 0.82 | 0.72 | 0.93 | 0.71 | 0.62 | 0.79 | 0.75 | 0.70 | 0.81 | 0.82 | 0.74 | 0.90 | 0.73 | 0.65 | 0.80 | 0.75 | 0.71 | 0.80 | 0.83 | 0.76 | 0.89 | 0.72 | 0.67 | 0.77 |
| 858 | 0.74 | 0.67 | 0.81 | 0.80 | 0.68 | 0.92 | 0.72 | 0.64 | 0.81 | 0.76 | 0.70 | 0.82 | 0.84 | 0.76 | 0.92 | 0.73 | 0.66 | 0.80 | 0.77 | 0.72 | 0.81 | 0.84 | 0.77 | 0.90 | 0.74 | 0.69 | 0.79 |
| 859 | 0.76 | 0.69 | 0.82 | 0.83 | 0.72 | 0.93 | 0.73 | 0.65 | 0.81 | 0.76 | 0.70 | 0.82 | 0.86 | 0.78 | 0.93 | 0.72 | 0.65 | 0.79 | 0.77 | 0.72 | 0.81 | 0.85 | 0.80 | 0.91 | 0.73 | 0.68 | 0.78 |
| 860 | 0.75 | 0.67 | 0.83 | 0.87 | 0.78 | 0.97 | 0.71 | 0.62 | 0.80 | 0.76 | 0.70 | 0.82 | 0.84 | 0.76 | 0.92 | 0.72 | 0.65 | 0.79 | 0.76 | 0.71 | 0.80 | 0.85 | 0.79 | 0.91 | 0.72 | 0.66 | 0.77 |
| 861 | 0.75 | 0.68 | 0.82 | 0.82 | 0.69 | 0.95 | 0.73 | 0.65 | 0.81 | 0.76 | 0.70 | 0.82 | 0.82 | 0.74 | 0.91 | 0.73 | 0.67 | 0.80 | 0.77 | 0.73 | 0.81 | 0.84 | 0.77 | 0.90 | 0.74 | 0.69 | 0.80 |
| 862 | 0.74 | 0.66 | 0.81 | 0.81 | 0.70 | 0.92 | 0.71 | 0.63 | 0.80 | 0.76 | 0.70 | 0.82 | 0.82 | 0.73 | 0.91 | 0.74 | 0.67 | 0.81 | 0.76 | 0.72 | 0.81 | 0.83 | 0.76 | 0.90 | 0.74 | 0.69 | 0.79 |
| 863 | 0.77 | 0.69 | 0.84 | 0.88 | 0.80 | 0.97 | 0.73 | 0.63 | 0.82 | 0.75 | 0.69 | 0.81 | 0.79 | 0.70 | 0.88 | 0.73 | 0.66 | 0.81 | 0.76 | 0.71 | 0.81 | 0.82 | 0.76 | 0.89 | 0.74 | 0.68 | 0.79 |
| 864 | 0.77 | 0.69 | 0.85 | 0.90 | 0.83 | 0.97 | 0.73 | 0.64 | 0.83 | 0.74 | 0.68 | 0.81 | 0.78 | 0.67 | 0.90 | 0.73 | 0.65 | 0.80 | 0.76 | 0.71 | 0.81 | 0.83 | 0.74 | 0.91 | 0.74 | 0.68 | 0.79 |
| 865 | 0.75 | 0.67 | 0.84 | 0.81 | 0.66 | 0.95 | 0.73 | 0.63 | 0.83 | 0.77 | 0.71 | 0.82 | 0.85 | 0.78 | 0.92 | 0.73 | 0.66 | 0.80 | 0.78 | 0.73 | 0.82 | 0.85 | 0.79 | 0.91 | 0.75 | 0.69 | 0.80 |
| 866 | 0.75 | 0.67 | 0.84 | 0.84 | 0.73 | 0.96 | 0.72 | 0.62 | 0.83 | 0.77 | 0.71 | 0.82 | 0.85 | 0.78 | 0.92 | 0.73 | 0.66 | 0.80 | 0.78 | 0.73 | 0.82 | 0.86 | 0.80 | 0.92 | 0.75 | 0.69 | 0.80 |
| 867 | 0.76 | 0.68 | 0.84 | 0.89 | 0.77 | 1.00 | 0.72 | 0.63 | 0.82 | 0.76 | 0.70 | 0.82 | 0.85 | 0.74 | 0.95 | 0.73 | 0.66 | 0.79 | 0.77 | 0.72 | 0.81 | 0.86 | 0.79 | 0.94 | 0.73 | 0.68 | 0.78 |
| 868 | 0.76 | 0.68 | 0.84 | 0.83 | 0.69 | 0.96 | 0.74 | 0.65 | 0.84 | 0.76 | 0.71 | 0.82 | 0.84 | 0.76 | 0.92 | 0.73 | 0.66 | 0.80 | 0.78 | 0.74 | 0.83 | 0.85 | 0.78 | 0.92 | 0.75 | 0.70 | 0.81 |
| 869 | 0.74 | 0.66 | 0.82 | 0.85 | 0.73 | 0.96 | 0.70 | 0.61 | 0.79 | 0.75 | 0.69 | 0.81 | 0.80 | 0.68 | 0.91 | 0.73 | 0.67 | 0.80 | 0.76 | 0.71 | 0.80 | 0.82 | 0.74 | 0.91 | 0.74 | 0.68 | 0.79 |
| 870 | 0.75 | 0.66 | 0.83 | 0.80 | 0.63 | 0.96 | 0.73 | 0.64 | 0.82 | 0.77 | 0.71 | 0.83 | 0.88 | 0.82 | 0.93 | 0.72 | 0.64 | 0.79 | 0.76 | 0.71 | 0.81 | 0.85 | 0.79 | 0.92 | 0.72 | 0.67 | 0.78 |
| 871 | 0.75 | 0.68 | 0.83 | 0.86 | 0.75 | 0.97 | 0.72 | 0.63 | 0.80 | 0.76 | 0.70 | 0.82 | 0.84 | 0.77 | 0.92 | 0.72 | 0.65 | 0.79 | 0.76 | 0.72 | 0.81 | 0.85 | 0.79 | 0.91 | 0.73 | 0.67 | 0.78 |
| 872 | 0.77 | 0.69 | 0.84 | 0.85 | 0.72 | 0.98 | 0.74 | 0.65 | 0.83 | 0.76 | 0.70 | 0.82 | 0.83 | 0.74 | 0.92 | 0.73 | 0.66 | 0.80 | 0.77 | 0.73 | 0.82 | 0.85 | 0.78 | 0.91 | 0.74 | 0.69 | 0.80 |
| 873 | 0.76 | 0.68 | 0.85 | 0.86 | 0.69 | 1.00 | 0.74 | 0.64 | 0.83 | 0.77 | 0.72 | 0.83 | 0.87 | 0.80 | 0.94 | 0.73 | 0.66 | 0.80 | 0.79 | 0.74 | 0.83 | 0.88 | 0.82 | 0.95 | 0.75 | 0.70 | 0.80 |
| 874 | 0.77 | 0.69 | 0.84 | 0.82 | 0.70 | 0.93 | 0.75 | 0.66 | 0.84 | 0.76 | 0.70 | 0.82 | 0.83 | 0.76 | 0.90 | 0.73 | 0.66 | 0.80 | 0.76 | 0.71 | 0.80 | 0.82 | 0.76 | 0.88 | 0.74 | 0.68 | 0.79 |
| 875 | 0.77 | 0.68 | 0.85 | 0.84 | 0.74 | 0.93 | 0.74 | 0.65 | 0.84 | 0.76 | 0.70 | 0.82 | 0.84 | 0.76 | 0.91 | 0.73 | 0.65 | 0.80 | 0.78 | 0.73 | 0.82 | 0.85 | 0.80 | 0.90 | 0.75 | 0.69 | 0.81 |
| 876 | 0.76 | 0.69 | 0.83 | 0.82 | 0.67 | 0.96 | 0.74 | 0.66 | 0.82 | 0.76 | 0.70 | 0.82 | 0.84 | 0.77 | 0.91 | 0.73 | 0.66 | 0.80 | 0.77 | 0.72 | 0.81 | 0.84 | 0.78 | 0.90 | 0.74 | 0.69 | 0.79 |
| 877 | 0.74 | 0.67 | 0.81 | 0.82 | 0.71 | 0.92 | 0.72 | 0.63 | 0.80 | 0.77 | 0.70 | 0.82 | 0.82 | 0.72 | 0.92 | 0.73 | 0.66 | 0.80 | 0.77 | 0.72 | 0.81 | 0.83 | 0.76 | 0.90 | 0.74 | 0.69 | 0.79 |
| 878 | 0.75 | 0.66 | 0.84 | 0.80 | 0.64 | 0.95 | 0.73 | 0.63 | 0.84 | 0.75 | 0.71 | 0.81 | 0.85 | 0.78 | 0.88 | 0.73 | 0.67 | 0.80 | 0.78 | 0.73 | 0.82 | 0.85 | 0.79 | 0.92 | 0.75 | 0.69 | 0.81 |
| 879 | 0.75 | 0.68 | 0.83 | 0.83 | 0.73 | 0.93 | 0.73 | 0.63 | 0.82 | 0.76 | 0.69 | 0.82 | 0.80 | 0.72 | 0.91 | 0.74 | 0.66 | 0.80 | 0.75 | 0.70 | 0.79 | 0.81 | 0.74 | 0.87 | 0.73 | 0.67 | 0.78 |
| 880 | 0.74 | 0.67 | 0.81 | 0.79 | 0.67 | 0.92 | 0.72 | 0.64 | 0.81 | 0.76 | 0.70 | 0.82 | 0.82 | 0.72 | 0.92 | 0.73 | 0.66 | 0.81 | 0.77 | 0.72 | 0.81 | 0.82 | 0.75 | 0.89 | 0.75 | 0.69 | 0.80 |
| 881 | 0.74 | 0.67 | 0.82 | 0.78 | 0.65 | 0.91 | 0.73 | 0.65 | 0.81 | 0.77 | 0.70 | 0.82 | 0.84 | 0.76 | 0.91 | 0.73 | 0.66 | 0.80 | 0.77 | 0.72 | 0.81 | 0.83 | 0.77 | 0.90 | 0.74 | 0.69 | 0.79 |
| 882 | 0.75 | 0.66 | 0.84 | 0.80 | 0.64 | 0.96 | 0.73 | 0.63 | 0.83 | 0.76 | 0.71 | 0.82 | 0.84 | 0.77 | 0.92 | 0.73 | 0.66 | 0.81 | 0.77 | 0.73 | 0.82 | 0.84 | 0.78 | 0.91 | 0.75 | 0.70 | 0.80 |
| 883 | 0.76 | 0.67 | 0.84 | 0.81 | 0.66 | 0.95 | 0.74 | 0.64 | 0.84 | 0.76 | 0.70 | 0.82 | 0.85 | 0.77 | 0.91 | 0.73 | 0.62 | 0.80 | 0.78 | 0.73 | 0.83 | 0.85 | 0.78 | 0.91 | 0.73 | 0.68 | 0.81 |
| 884 | 0.79 | 0.70 | 0.87 | 0.92 | 0.83 | 1.00 | 0.75 | 0.64 | 0.85 | 0.74 | 0.68 | 0.80 | 0.84 | 0.74 | 0.94 | 0.70 | 0.62 | 0.78 | 0.76 | 0.72 | 0.81 | 0.88 | 0.81 | 0.94 | 0.74 | 0.68 | 0.80 |
| 885 | 0.73 | 0.66 | 0.80 | 0.82 | 0.69 | 0.96 | 0.70 | 0.62 | 0.78 | 0.76 | 0.70 | 0.82 | 0.85 | 0.76 | 0.93 | 0.73 | 0.66 | 0.80 | 0.77 | 0.72 | 0.82 | 0.85 | 0.78 | 0.92 | 0.75 | 0.69 | 0.78 |
| 886 | 0.76 | 0.67 | 0.84 | 0.85 | 0.76 | 0.95 | 0.73 | 0.62 | 0.83 | 0.76 | 0.70 | 0.82 | 0.84 | 0.76 | 0.91 | 0.73 | 0.65 | 0.79 | 0.76 | 0.72 | 0.81 | 0.85 | 0.80 | 0.91 | 0.73 | 0.68 | 0.79 |
| 887 | 0.76 | 0.68 | 0.84 | 0.83 | 0.70 | 0.95 | 0.73 | 0.64 | 0.83 | 0.76 | 0.70 | 0.82 | 0.89 | 0.84 | 0.94 | 0.70 | 0.63 | 0.78 | 0.77 | 0.73 | 0.82 | 0.88 | 0.83 | 0.93 | 0.74 | 0.68 | 0.80 |
| 888 | 0.77 | 0.69 | 0.85 | 0.89 | 0.80 | 0.98 | 0.73 | 0.63 | 0.83 | 0.76 | 0.70 | 0.82 | 0.85 | 0.75 | 0.95 | 0.72 | 0.66 | 0.79 | 0.78 | 0.73 | 0.82 | 0.87 | 0.80 | 0.94 | 0.74 | 0.69 | 0.80 |
| 889 | 0.76 | 0.68 | 0.84 | 0.84 | 0.72 | 0.95 | 0.74 | 0.64 | 0.83 | 0.76 | 0.70 | 0.82 | 0.80 | 0.72 | 0.89 | 0.74 | 0.66 | 0.81 | 0.77 | 0.72 | 0.82 | 0.83 | 0.77 | 0.89 | 0.75 | 0.69 | 0.80 |

| No. | G | H | I | J | K | L | M | N | O | P | Q | R | S | T | U | V | W | Z | AA | AB | AC | AD | AE | AF | AG | AH | AI |
|-----|------|------|------|------|------|------|------|------|------|------|------|------|------|------|------|------|------|------|------|------|------|------|------|------|------|------|------|
| 890 | 0.75 | 0.67 | 0.83 | 0.81 | 0.69 | 0.94 | 0.73 | 0.64 | 0.83 | 0.75 | 0.70 | 0.81 | 0.80 | 0.73 | 0.88 | 0.73 | 0.66 | 0.80 | 0.75 | 0.70 | 0.80 | 0.80 | 0.74 | 0.87 | 0.73 | 0.67 | 0.78 |
| 891 | 0.74 | 0.66 | 0.82 | 0.85 | 0.74 | 0.97 | 0.71 | 0.61 | 0.80 | 0.74 | 0.68 | 0.80 | 0.80 | 0.69 | 0.92 | 0.72 | 0.65 | 0.79 | 0.75 | 0.71 | 0.80 | 0.83 | 0.74 | 0.91 | 0.73 | 0.67 | 0.78 |
| 892 | 0.74 | 0.67 | 0.81 | 0.84 | 0.73 | 0.94 | 0.71 | 0.63 | 0.80 | 0.76 | 0.70 | 0.82 | 0.84 | 0.76 | 0.93 | 0.73 | 0.66 | 0.80 | 0.76 | 0.72 | 0.81 | 0.85 | 0.79 | 0.91 | 0.73 | 0.68 | 0.79 |
| 893 | 0.75 | 0.66 | 0.83 | 0.84 | 0.71 | 0.96 | 0.72 | 0.62 | 0.81 | 0.75 | 0.69 | 0.81 | 0.81 | 0.71 | 0.92 | 0.72 | 0.65 | 0.79 | 0.76 | 0.71 | 0.81 | 0.83 | 0.75 | 0.91 | 0.73 | 0.68 | 0.79 |
| 894 | 0.74 | 0.67 | 0.80 | 0.75 | 0.63 | 0.86 | 0.73 | 0.65 | 0.81 | 0.76 | 0.70 | 0.82 | 0.83 | 0.75 | 0.91 | 0.73 | 0.66 | 0.80 | 0.76 | 0.71 | 0.80 | 0.81 | 0.74 | 0.87 | 0.74 | 0.68 | 0.79 |
| 895 | 0.75 | 0.67 | 0.84 | 0.79 | 0.63 | 0.94 | 0.74 | 0.64 | 0.84 | 0.76 | 0.70 | 0.82 | 0.83 | 0.75 | 0.90 | 0.74 | 0.66 | 0.81 | 0.78 | 0.73 | 0.82 | 0.84 | 0.77 | 0.90 | 0.76 | 0.70 | 0.81 |
| 896 | 0.76 | 0.68 | 0.84 | 0.84 | 0.71 | 0.97 | 0.73 | 0.64 | 0.82 | 0.76 | 0.71 | 0.82 | 0.85 | 0.77 | 0.92 | 0.73 | 0.66 | 0.80 | 0.77 | 0.73 | 0.82 | 0.86 | 0.79 | 0.92 | 0.74 | 0.69 | 0.80 |
| 897 | 0.76 | 0.69 | 0.83 | 0.88 | 0.81 | 0.95 | 0.72 | 0.64 | 0.81 | 0.76 | 0.70 | 0.82 | 0.86 | 0.79 | 0.93 | 0.71 | 0.64 | 0.79 | 0.76 | 0.72 | 0.81 | 0.87 | 0.82 | 0.92 | 0.72 | 0.67 | 0.78 |
| 898 | 0.75 | 0.68 | 0.82 | 0.83 | 0.74 | 0.92 | 0.73 | 0.64 | 0.81 | 0.76 | 0.70 | 0.82 | 0.84 | 0.77 | 0.91 | 0.72 | 0.65 | 0.79 | 0.76 | 0.71 | 0.80 | 0.84 | 0.78 | 0.90 | 0.73 | 0.67 | 0.78 |
| 899 | 0.74 | 0.66 | 0.81 | 0.86 | 0.78 | 0.94 | 0.70 | 0.61 | 0.78 | 0.76 | 0.70 | 0.81 | 0.86 | 0.79 | 0.92 | 0.71 | 0.64 | 0.79 | 0.75 | 0.70 | 0.79 | 0.85 | 0.80 | 0.90 | 0.71 | 0.65 | 0.76 |
| 900 | 0.74 | 0.65 | 0.83 | 0.85 | 0.69 | 1.00 | 0.71 | 0.61 | 0.81 | 0.76 | 0.70 | 0.82 | 0.88 | 0.83 | 0.93 | 0.70 | 0.63 | 0.77 | 0.78 | 0.73 | 0.82 | 0.89 | 0.84 | 0.94 | 0.73 | 0.68 | 0.79 |
| 901 | 0.74 | 0.67 | 0.82 | 0.82 | 0.70 | 0.95 | 0.72 | 0.63 | 0.80 | 0.76 | 0.69 | 0.81 | 0.84 | 0.72 | 0.95 | 0.73 | 0.66 | 0.80 | 0.76 | 0.71 | 0.81 | 0.84 | 0.76 | 0.92 | 0.73 | 0.68 | 0.78 |
| 902 | 0.75 | 0.68 | 0.83 | 0.80 | 0.65 | 0.95 | 0.74 | 0.65 | 0.82 | 0.76 | 0.70 | 0.82 | 0.82 | 0.73 | 0.91 | 0.73 | 0.66 | 0.80 | 0.77 | 0.72 | 0.81 | 0.83 | 0.75 | 0.90 | 0.74 | 0.69 | 0.80 |
| 903 | 0.75 | 0.67 | 0.82 | 0.81 | 0.69 | 0.93 | 0.73 | 0.64 | 0.82 | 0.76 | 0.69 | 0.82 | 0.83 | 0.76 | 0.90 | 0.72 | 0.65 | 0.79 | 0.75 | 0.71 | 0.80 | 0.82 | 0.76 | 0.88 | 0.72 | 0.67 | 0.78 |
| 904 | 0.76 | 0.68 | 0.83 | 0.82 | 0.71 | 0.93 | 0.74 | 0.65 | 0.83 | 0.75 | 0.70 | 0.81 | 0.87 | 0.80 | 0.93 | 0.71 | 0.64 | 0.79 | 0.77 | 0.73 | 0.82 | 0.86 | 0.81 | 0.91 | 0.74 | 0.68 | 0.79 |
| 905 | 0.74 | 0.66 | 0.82 | 0.81 | 0.67 | 0.94 | 0.72 | 0.63 | 0.81 | 0.76 | 0.70 | 0.82 | 0.84 | 0.78 | 0.91 | 0.72 | 0.65 | 0.79 | 0.75 | 0.71 | 0.80 | 0.83 | 0.77 | 0.89 | 0.72 | 0.66 | 0.77 |
| 906 | 0.75 | 0.67 | 0.83 | 0.82 | 0.68 | 0.96 | 0.73 | 0.63 | 0.83 | 0.76 | 0.70 | 0.81 | 0.89 | 0.84 | 0.94 | 0.71 | 0.63 | 0.78 | 0.76 | 0.72 | 0.81 | 0.87 | 0.82 | 0.93 | 0.72 | 0.66 | 0.78 |
| 907 | 0.75 | 0.67 | 0.83 | 0.83 | 0.71 | 0.94 | 0.73 | 0.63 | 0.82 | 0.76 | 0.70 | 0.82 | 0.88 | 0.82 | 0.93 | 0.71 | 0.64 | 0.79 | 0.77 | 0.72 | 0.82 | 0.87 | 0.82 | 0.92 | 0.73 | 0.67 | 0.79 |
| 908 | 0.75 | 0.68 | 0.82 | 0.81 | 0.69 | 0.93 | 0.73 | 0.65 | 0.81 | 0.76 | 0.70 | 0.82 | 0.85 | 0.76 | 0.93 | 0.72 | 0.65 | 0.79 | 0.77 | 0.73 | 0.81 | 0.85 | 0.78 | 0.91 | 0.74 | 0.69 | 0.79 |
| 909 | 0.74 | 0.67 | 0.81 | 0.82 | 0.73 | 0.91 | 0.72 | 0.63 | 0.80 | 0.76 | 0.70 | 0.82 | 0.82 | 0.73 | 0.90 | 0.73 | 0.66 | 0.80 | 0.77 | 0.72 | 0.81 | 0.83 | 0.77 | 0.89 | 0.74 | 0.69 | 0.79 |
| 910 | 0.74 | 0.66 | 0.82 | 0.85 | 0.73 | 0.97 | 0.71 | 0.61 | 0.80 | 0.75 | 0.70 | 0.81 | 0.83 | 0.74 | 0.91 | 0.72 | 0.65 | 0.79 | 0.76 | 0.72 | 0.81 | 0.84 | 0.78 | 0.91 | 0.73 | 0.69 | 0.78 |
| 911 | 0.75 | 0.67 | 0.82 | 0.82 | 0.69 | 0.94 | 0.72 | 0.63 | 0.80 | 0.75 | 0.70 | 0.81 | 0.83 | 0.75 | 0.90 | 0.72 | 0.65 | 0.79 | 0.75 | 0.70 | 0.79 | 0.82 | 0.76 | 0.88 | 0.72 | 0.68 | 0.77 |
| 912 | 0.74 | 0.67 | 0.82 | 0.80 | 0.64 | 0.95 | 0.72 | 0.64 | 0.82 | 0.76 | 0.70 | 0.82 | 0.84 | 0.74 | 0.91 | 0.73 | 0.66 | 0.80 | 0.75 | 0.71 | 0.80 | 0.82 | 0.75 | 0.89 | 0.73 | 0.66 | 0.78 |
| 913 | 0.76 | 0.69 | 0.84 | 0.81 | 0.70 | 0.93 | 0.75 | 0.66 | 0.83 | 0.76 | 0.70 | 0.82 | 0.82 | 0.75 | 0.89 | 0.73 | 0.66 | 0.80 | 0.75 | 0.73 | 0.82 | 0.81 | 0.75 | 0.87 | 0.73 | 0.68 | 0.79 |
| 914 | 0.74 | 0.66 | 0.82 | 0.83 | 0.66 | 0.99 | 0.72 | 0.63 | 0.80 | 0.76 | 0.70 | 0.82 | 0.85 | 0.77 | 0.93 | 0.72 | 0.65 | 0.80 | 0.77 | 0.72 | 0.82 | 0.86 | 0.79 | 0.93 | 0.74 | 0.69 | 0.80 |
| 915 | 0.73 | 0.66 | 0.80 | 0.78 | 0.66 | 0.90 | 0.72 | 0.64 | 0.80 | 0.76 | 0.70 | 0.82 | 0.83 | 0.73 | 0.92 | 0.73 | 0.66 | 0.80 | 0.76 | 0.72 | 0.81 | 0.82 | 0.75 | 0.89 | 0.74 | 0.69 | 0.79 |
| 916 | 0.76 | 0.69 | 0.83 | 0.81 | 0.67 | 0.95 | 0.74 | 0.66 | 0.82 | 0.76 | 0.70 | 0.82 | 0.84 | 0.76 | 0.91 | 0.73 | 0.65 | 0.80 | 0.77 | 0.71 | 0.81 | 0.84 | 0.77 | 0.90 | 0.74 | 0.69 | 0.79 |
| 917 | 0.75 | 0.68 | 0.83 | 0.88 | 0.81 | 0.96 | 0.71 | 0.62 | 0.80 | 0.76 | 0.69 | 0.82 | 0.83 | 0.75 | 0.91 | 0.73 | 0.65 | 0.80 | 0.76 | 0.72 | 0.81 | 0.85 | 0.79 | 0.91 | 0.73 | 0.67 | 0.78 |
| 918 | 0.76 | 0.68 | 0.83 | 0.84 | 0.72 | 0.96 | 0.73 | 0.64 | 0.82 | 0.76 | 0.70 | 0.82 | 0.84 | 0.76 | 0.92 | 0.72 | 0.65 | 0.79 | 0.76 | 0.72 | 0.81 | 0.85 | 0.78 | 0.91 | 0.73 | 0.68 | 0.78 |
| 919 | 0.73 | 0.66 | 0.80 | 0.77 | 0.64 | 0.90 | 0.72 | 0.63 | 0.80 | 0.76 | 0.69 | 0.82 | 0.83 | 0.74 | 0.91 | 0.73 | 0.66 | 0.80 | 0.76 | 0.71 | 0.80 | 0.82 | 0.75 | 0.89 | 0.74 | 0.69 | 0.79 |
| 920 | 0.74 | 0.67 | 0.81 | 0.77 | 0.64 | 0.90 | 0.73 | 0.65 | 0.81 | 0.76 | 0.70 | 0.82 | 0.84 | 0.75 | 0.91 | 0.72 | 0.65 | 0.79 | 0.76 | 0.71 | 0.80 | 0.82 | 0.76 | 0.89 | 0.73 | 0.68 | 0.78 |
| 921 | 0.74 | 0.66 | 0.82 | 0.80 | 0.65 | 0.94 | 0.73 | 0.63 | 0.82 | 0.76 | 0.70 | 0.81 | 0.84 | 0.77 | 0.91 | 0.72 | 0.65 | 0.77 | 0.76 | 0.71 | 0.80 | 0.83 | 0.77 | 0.89 | 0.73 | 0.68 | 0.78 |
| 922 | 0.77 | 0.69 | 0.86 | 0.92 | 0.83 | 1.00 | 0.73 | 0.63 | 0.84 | 0.74 | 0.67 | 0.80 | 0.85 | 0.75 | 0.94 | 0.69 | 0.61 | 0.77 | 0.77 | 0.72 | 0.82 | 0.88 | 0.81 | 0.95 | 0.73 | 0.67 | 0.79 |

117

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 923 | 0.75 | 0.67 | 0.83 | 0.81 | 0.69 | 0.93 | 0.73 | 0.64 | 0.83 | 0.76 | 0.70 | 0.82 | 0.86 | 0.80 | 0.92 | 0.72 | 0.64 | 0.79 | 0.77 | 0.72 | 0.81 | 0.85 | 0.80 | 0.91 | 0.73 | 0.68 | 0.79 |
| 924 | 0.75 | 0.68 | 0.82 | 0.79 | 0.65 | 0.93 | 0.74 | 0.66 | 0.82 | 0.76 | 0.70 | 0.82 | 0.82 | 0.73 | 0.90 | 0.73 | 0.66 | 0.80 | 0.77 | 0.73 | 0.81 | 0.83 | 0.76 | 0.90 | 0.75 | 0.70 | 0.80 |
| 925 | 0.75 | 0.67 | 0.82 | 0.84 | 0.73 | 0.94 | 0.72 | 0.63 | 0.81 | 0.76 | 0.70 | 0.82 | 0.84 | 0.76 | 0.92 | 0.73 | 0.66 | 0.80 | 0.78 | 0.73 | 0.82 | 0.85 | 0.79 | 0.91 | 0.75 | 0.70 | 0.80 |
| 926 | 0.73 | 0.66 | 0.81 | 0.78 | 0.66 | 0.89 | 0.72 | 0.63 | 0.81 | 0.76 | 0.70 | 0.81 | 0.81 | 0.73 | 0.90 | 0.73 | 0.66 | 0.80 | 0.76 | 0.72 | 0.81 | 0.82 | 0.75 | 0.88 | 0.74 | 0.69 | 0.79 |
| 927 | 0.75 | 0.67 | 0.84 | 0.83 | 0.72 | 0.95 | 0.73 | 0.63 | 0.83 | 0.76 | 0.70 | 0.82 | 0.84 | 0.76 | 0.91 | 0.73 | 0.66 | 0.80 | 0.78 | 0.73 | 0.82 | 0.85 | 0.79 | 0.91 | 0.75 | 0.70 | 0.80 |
| 928 | 0.75 | 0.67 | 0.82 | 0.85 | 0.73 | 0.96 | 0.71 | 0.63 | 0.80 | 0.76 | 0.70 | 0.81 | 0.84 | 0.77 | 0.92 | 0.72 | 0.65 | 0.79 | 0.75 | 0.71 | 0.80 | 0.84 | 0.79 | 0.90 | 0.72 | 0.66 | 0.77 |
| 929 | 0.75 | 0.67 | 0.82 | 0.85 | 0.72 | 0.98 | 0.71 | 0.62 | 0.80 | 0.76 | 0.70 | 0.82 | 0.82 | 0.71 | 0.92 | 0.74 | 0.67 | 0.80 | 0.76 | 0.72 | 0.81 | 0.84 | 0.76 | 0.91 | 0.74 | 0.69 | 0.79 |
| 930 | 0.77 | 0.68 | 0.86 | 0.88 | 0.74 | 1.00 | 0.74 | 0.63 | 0.84 | 0.74 | 0.67 | 0.80 | 0.83 | 0.73 | 0.92 | 0.70 | 0.62 | 0.77 | 0.77 | 0.72 | 0.82 | 0.86 | 0.79 | 0.93 | 0.74 | 0.68 | 0.79 |
| 931 | 0.76 | 0.68 | 0.83 | 0.86 | 0.75 | 0.97 | 0.73 | 0.64 | 0.81 | 0.75 | 0.69 | 0.81 | 0.84 | 0.76 | 0.92 | 0.72 | 0.64 | 0.79 | 0.76 | 0.72 | 0.81 | 0.85 | 0.79 | 0.91 | 0.73 | 0.67 | 0.78 |
| 932 | 0.76 | 0.69 | 0.84 | 0.86 | 0.74 | 0.98 | 0.73 | 0.64 | 0.82 | 0.75 | 0.70 | 0.81 | 0.82 | 0.74 | 0.91 | 0.73 | 0.66 | 0.80 | 0.77 | 0.72 | 0.81 | 0.84 | 0.78 | 0.91 | 0.74 | 0.68 | 0.79 |
| 933 | 0.76 | 0.68 | 0.85 | 0.88 | 0.77 | 1.00 | 0.73 | 0.63 | 0.82 | 0.75 | 0.69 | 0.81 | 0.85 | 0.74 | 0.95 | 0.72 | 0.65 | 0.79 | 0.77 | 0.72 | 0.81 | 0.86 | 0.79 | 0.94 | 0.73 | 0.68 | 0.79 |
| 934 | 0.74 | 0.67 | 0.81 | 0.76 | 0.66 | 0.87 | 0.73 | 0.65 | 0.81 | 0.75 | 0.69 | 0.82 | 0.82 | 0.74 | 0.91 | 0.73 | 0.65 | 0.80 | 0.75 | 0.71 | 0.80 | 0.81 | 0.75 | 0.88 | 0.73 | 0.68 | 0.79 |
| 935 | 0.75 | 0.67 | 0.82 | 0.82 | 0.70 | 0.94 | 0.72 | 0.64 | 0.81 | 0.75 | 0.70 | 0.81 | 0.80 | 0.72 | 0.89 | 0.73 | 0.67 | 0.80 | 0.77 | 0.72 | 0.81 | 0.82 | 0.76 | 0.89 | 0.74 | 0.69 | 0.80 |
| 936 | 0.75 | 0.66 | 0.83 | 0.88 | 0.79 | 0.97 | 0.70 | 0.60 | 0.81 | 0.74 | 0.67 | 0.80 | 0.80 | 0.69 | 0.91 | 0.71 | 0.64 | 0.79 | 0.74 | 0.69 | 0.79 | 0.82 | 0.75 | 0.90 | 0.71 | 0.65 | 0.77 |
| 937 | 0.74 | 0.66 | 0.83 | 0.80 | 0.64 | 0.96 | 0.72 | 0.62 | 0.82 | 0.76 | 0.70 | 0.82 | 0.85 | 0.78 | 0.92 | 0.72 | 0.65 | 0.80 | 0.77 | 0.72 | 0.82 | 0.85 | 0.78 | 0.91 | 0.74 | 0.68 | 0.80 |
| 938 | 0.77 | 0.69 | 0.84 | 0.83 | 0.70 | 0.97 | 0.74 | 0.66 | 0.83 | 0.75 | 0.69 | 0.81 | 0.81 | 0.73 | 0.90 | 0.73 | 0.66 | 0.80 | 0.77 | 0.73 | 0.82 | 0.84 | 0.77 | 0.90 | 0.75 | 0.69 | 0.80 |
| 939 | 0.76 | 0.69 | 0.84 | 0.82 | 0.71 | 0.94 | 0.75 | 0.66 | 0.83 | 0.75 | 0.69 | 0.81 | 0.83 | 0.76 | 0.90 | 0.72 | 0.65 | 0.79 | 0.76 | 0.71 | 0.80 | 0.83 | 0.77 | 0.88 | 0.73 | 0.67 | 0.78 |
| 940 | 0.75 | 0.67 | 0.82 | 0.82 | 0.70 | 0.95 | 0.72 | 0.64 | 0.81 | 0.75 | 0.69 | 0.81 | 0.84 | 0.76 | 0.92 | 0.72 | 0.64 | 0.79 | 0.76 | 0.72 | 0.80 | 0.84 | 0.78 | 0.90 | 0.73 | 0.67 | 0.78 |
| 941 | 0.76 | 0.68 | 0.83 | 0.86 | 0.77 | 0.95 | 0.72 | 0.64 | 0.81 | 0.75 | 0.69 | 0.81 | 0.82 | 0.74 | 0.91 | 0.73 | 0.66 | 0.80 | 0.77 | 0.72 | 0.81 | 0.85 | 0.79 | 0.91 | 0.74 | 0.69 | 0.79 |
| 942 | 0.74 | 0.67 | 0.82 | 0.84 | 0.72 | 0.96 | 0.71 | 0.62 | 0.81 | 0.75 | 0.69 | 0.81 | 0.83 | 0.74 | 0.91 | 0.72 | 0.65 | 0.79 | 0.76 | 0.72 | 0.81 | 0.84 | 0.78 | 0.91 | 0.73 | 0.68 | 0.78 |
| 943 | 0.74 | 0.66 | 0.82 | 0.84 | 0.72 | 0.97 | 0.70 | 0.61 | 0.80 | 0.74 | 0.67 | 0.80 | 0.81 | 0.70 | 0.92 | 0.71 | 0.64 | 0.77 | 0.76 | 0.71 | 0.80 | 0.83 | 0.75 | 0.91 | 0.73 | 0.68 | 0.78 |
| 944 | 0.75 | 0.68 | 0.82 | 0.84 | 0.73 | 0.95 | 0.72 | 0.63 | 0.81 | 0.75 | 0.69 | 0.81 | 0.84 | 0.76 | 0.92 | 0.72 | 0.64 | 0.79 | 0.75 | 0.71 | 0.80 | 0.84 | 0.78 | 0.90 | 0.72 | 0.66 | 0.77 |
| 945 | 0.75 | 0.66 | 0.83 | 0.79 | 0.64 | 0.95 | 0.73 | 0.63 | 0.83 | 0.76 | 0.70 | 0.82 | 0.84 | 0.76 | 0.91 | 0.72 | 0.65 | 0.79 | 0.77 | 0.73 | 0.82 | 0.84 | 0.78 | 0.91 | 0.75 | 0.69 | 0.80 |
| 946 | 0.75 | 0.68 | 0.83 | 0.82 | 0.70 | 0.95 | 0.73 | 0.65 | 0.81 | 0.75 | 0.69 | 0.81 | 0.84 | 0.76 | 0.92 | 0.72 | 0.64 | 0.79 | 0.76 | 0.72 | 0.81 | 0.84 | 0.78 | 0.90 | 0.73 | 0.68 | 0.78 |
| 947 | 0.75 | 0.68 | 0.83 | 0.85 | 0.76 | 0.94 | 0.72 | 0.64 | 0.81 | 0.75 | 0.69 | 0.81 | 0.82 | 0.74 | 0.91 | 0.72 | 0.65 | 0.79 | 0.77 | 0.73 | 0.81 | 0.85 | 0.79 | 0.90 | 0.74 | 0.69 | 0.79 |
| 948 | 0.74 | 0.67 | 0.81 | 0.78 | 0.67 | 0.88 | 0.73 | 0.65 | 0.81 | 0.75 | 0.69 | 0.81 | 0.82 | 0.74 | 0.91 | 0.72 | 0.65 | 0.79 | 0.76 | 0.71 | 0.80 | 0.82 | 0.75 | 0.88 | 0.73 | 0.68 | 0.79 |
| 949 | 0.75 | 0.68 | 0.82 | 0.82 | 0.71 | 0.94 | 0.73 | 0.64 | 0.81 | 0.75 | 0.69 | 0.81 | 0.83 | 0.74 | 0.91 | 0.72 | 0.65 | 0.79 | 0.77 | 0.72 | 0.81 | 0.84 | 0.78 | 0.90 | 0.74 | 0.69 | 0.79 |
| 950 | 0.75 | 0.67 | 0.83 | 0.89 | 0.78 | 1.00 | 0.70 | 0.60 | 0.80 | 0.75 | 0.69 | 0.81 | 0.84 | 0.74 | 0.95 | 0.72 | 0.65 | 0.79 | 0.75 | 0.70 | 0.80 | 0.86 | 0.78 | 0.94 | 0.71 | 0.66 | 0.77 |
| 951 | 0.75 | 0.68 | 0.82 | 0.85 | 0.73 | 0.97 | 0.71 | 0.63 | 0.80 | 0.75 | 0.69 | 0.81 | 0.82 | 0.72 | 0.92 | 0.72 | 0.65 | 0.79 | 0.76 | 0.71 | 0.80 | 0.84 | 0.76 | 0.91 | 0.73 | 0.68 | 0.78 |
| 952 | 0.74 | 0.67 | 0.82 | 0.84 | 0.71 | 0.98 | 0.71 | 0.62 | 0.80 | 0.76 | 0.70 | 0.82 | 0.81 | 0.71 | 0.92 | 0.73 | 0.67 | 0.80 | 0.76 | 0.72 | 0.81 | 0.83 | 0.75 | 0.91 | 0.74 | 0.69 | 0.79 |
| 953 | 0.76 | 0.68 | 0.83 | 0.82 | 0.71 | 0.93 | 0.74 | 0.65 | 0.83 | 0.75 | 0.69 | 0.81 | 0.83 | 0.76 | 0.90 | 0.72 | 0.64 | 0.79 | 0.75 | 0.70 | 0.80 | 0.82 | 0.76 | 0.88 | 0.72 | 0.67 | 0.78 |
| 954 | 0.76 | 0.69 | 0.83 | 0.84 | 0.74 | 0.93 | 0.73 | 0.65 | 0.82 | 0.75 | 0.69 | 0.81 | 0.85 | 0.78 | 0.92 | 0.71 | 0.63 | 0.78 | 0.76 | 0.71 | 0.80 | 0.85 | 0.80 | 0.90 | 0.72 | 0.67 | 0.78 |
| 955 | 0.75 | 0.67 | 0.82 | 0.79 | 0.63 | 0.94 | 0.73 | 0.65 | 0.81 | 0.75 | 0.70 | 0.81 | 0.83 | 0.74 | 0.92 | 0.73 | 0.66 | 0.79 | 0.75 | 0.71 | 0.80 | 0.82 | 0.74 | 0.89 | 0.73 | 0.68 | 0.78 |

118

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 956 | 0.76 | 0.69 | 0.84 | 0.83 | 0.74 | 0.93 | 0.74 | 0.66 | 0.83 | 0.75 | 0.69 | 0.81 | 0.82 | 0.75 | 0.89 | 0.72 | 0.64 | 0.79 | 0.76 | 0.72 | 0.81 | 0.83 | 0.78 | 0.89 | 0.73 | 0.68 | 0.79 |
| 957 | 0.75 | 0.68 | 0.83 | 0.81 | 0.69 | 0.93 | 0.73 | 0.65 | 0.82 | 0.75 | 0.69 | 0.81 | 0.83 | 0.75 | 0.90 | 0.72 | 0.64 | 0.79 | 0.75 | 0.71 | 0.80 | 0.82 | 0.76 | 0.88 | 0.73 | 0.67 | 0.78 |
| 958 | 0.75 | 0.68 | 0.83 | 0.85 | 0.78 | 0.93 | 0.72 | 0.63 | 0.81 | 0.75 | 0.68 | 0.82 | 0.82 | 0.70 | 0.93 | 0.73 | 0.64 | 0.81 | 0.76 | 0.71 | 0.81 | 0.84 | 0.77 | 0.91 | 0.73 | 0.68 | 0.79 |
| 959 | 0.75 | 0.66 | 0.84 | 0.83 | 0.69 | 0.97 | 0.72 | 0.62 | 0.83 | 0.75 | 0.69 | 0.81 | 0.85 | 0.76 | 0.94 | 0.71 | 0.64 | 0.78 | 0.75 | 0.70 | 0.80 | 0.84 | 0.77 | 0.92 | 0.72 | 0.66 | 0.77 |
| 960 | 0.75 | 0.67 | 0.83 | 0.77 | 0.62 | 0.92 | 0.74 | 0.64 | 0.83 | 0.75 | 0.69 | 0.81 | 0.80 | 0.72 | 0.87 | 0.73 | 0.66 | 0.80 | 0.75 | 0.70 | 0.79 | 0.79 | 0.73 | 0.86 | 0.73 | 0.68 | 0.79 |
| 961 | 0.74 | 0.66 | 0.82 | 0.82 | 0.70 | 0.94 | 0.72 | 0.62 | 0.82 | 0.76 | 0.70 | 0.81 | 0.87 | 0.81 | 0.93 | 0.71 | 0.63 | 0.78 | 0.76 | 0.72 | 0.81 | 0.86 | 0.81 | 0.91 | 0.72 | 0.67 | 0.78 |
| 962 | 0.74 | 0.66 | 0.82 | 0.78 | 0.63 | 0.92 | 0.73 | 0.64 | 0.82 | 0.75 | 0.69 | 0.81 | 0.82 | 0.75 | 0.90 | 0.72 | 0.65 | 0.79 | 0.75 | 0.70 | 0.79 | 0.81 | 0.75 | 0.87 | 0.72 | 0.67 | 0.78 |
| 963 | 0.75 | 0.67 | 0.83 | 0.83 | 0.72 | 0.94 | 0.72 | 0.63 | 0.82 | 0.75 | 0.69 | 0.81 | 0.87 | 0.81 | 0.93 | 0.70 | 0.63 | 0.78 | 0.77 | 0.72 | 0.81 | 0.87 | 0.81 | 0.92 | 0.73 | 0.67 | 0.78 |
| 964 | 0.75 | 0.67 | 0.83 | 0.82 | 0.70 | 0.94 | 0.72 | 0.63 | 0.82 | 0.75 | 0.69 | 0.80 | 0.81 | 0.73 | 0.89 | 0.72 | 0.65 | 0.79 | 0.74 | 0.70 | 0.79 | 0.81 | 0.75 | 0.87 | 0.72 | 0.66 | 0.77 |
| 965 | 0.75 | 0.67 | 0.82 | 0.81 | 0.69 | 0.93 | 0.72 | 0.64 | 0.81 | 0.75 | 0.69 | 0.81 | 0.83 | 0.74 | 0.91 | 0.72 | 0.65 | 0.79 | 0.77 | 0.72 | 0.81 | 0.84 | 0.78 | 0.90 | 0.74 | 0.69 | 0.79 |
| 966 | 0.74 | 0.67 | 0.81 | 0.77 | 0.66 | 0.88 | 0.73 | 0.65 | 0.81 | 0.75 | 0.69 | 0.81 | 0.81 | 0.73 | 0.89 | 0.72 | 0.65 | 0.79 | 0.75 | 0.71 | 0.80 | 0.81 | 0.75 | 0.87 | 0.73 | 0.68 | 0.79 |
| 967 | 0.75 | 0.67 | 0.84 | 0.86 | 0.77 | 0.95 | 0.72 | 0.61 | 0.83 | 0.73 | 0.67 | 0.80 | 0.80 | 0.68 | 0.92 | 0.71 | 0.63 | 0.78 | 0.75 | 0.70 | 0.80 | 0.83 | 0.74 | 0.91 | 0.73 | 0.67 | 0.78 |
| 968 | 0.73 | 0.66 | 0.81 | 0.85 | 0.78 | 0.92 | 0.70 | 0.61 | 0.78 | 0.75 | 0.69 | 0.81 | 0.84 | 0.77 | 0.91 | 0.71 | 0.63 | 0.78 | 0.75 | 0.70 | 0.79 | 0.85 | 0.80 | 0.90 | 0.71 | 0.65 | 0.76 |
| 969 | 0.74 | 0.66 | 0.82 | 0.82 | 0.70 | 0.93 | 0.72 | 0.62 | 0.82 | 0.75 | 0.68 | 0.81 | 0.81 | 0.73 | 0.89 | 0.72 | 0.64 | 0.80 | 0.76 | 0.71 | 0.81 | 0.83 | 0.77 | 0.89 | 0.73 | 0.67 | 0.79 |
| 970 | 0.76 | 0.68 | 0.83 | 0.81 | 0.67 | 0.95 | 0.74 | 0.66 | 0.82 | 0.75 | 0.69 | 0.81 | 0.81 | 0.73 | 0.90 | 0.72 | 0.65 | 0.79 | 0.76 | 0.71 | 0.80 | 0.82 | 0.75 | 0.89 | 0.73 | 0.68 | 0.79 |
| 971 | 0.77 | 0.68 | 0.85 | 0.86 | 0.73 | 1.00 | 0.74 | 0.64 | 0.84 | 0.73 | 0.67 | 0.79 | 0.82 | 0.72 | 0.92 | 0.69 | 0.62 | 0.76 | 0.77 | 0.72 | 0.81 | 0.85 | 0.78 | 0.92 | 0.73 | 0.68 | 0.79 |
| 972 | 0.75 | 0.67 | 0.82 | 0.85 | 0.75 | 0.94 | 0.71 | 0.63 | 0.80 | 0.75 | 0.69 | 0.81 | 0.82 | 0.73 | 0.91 | 0.72 | 0.65 | 0.79 | 0.76 | 0.72 | 0.81 | 0.84 | 0.78 | 0.90 | 0.72 | 0.68 | 0.78 |
| 973 | 0.74 | 0.67 | 0.82 | 0.80 | 0.66 | 0.93 | 0.73 | 0.64 | 0.81 | 0.75 | 0.69 | 0.81 | 0.83 | 0.75 | 0.91 | 0.71 | 0.64 | 0.78 | 0.75 | 0.70 | 0.80 | 0.82 | 0.76 | 0.89 | 0.73 | 0.67 | 0.79 |
| 974 | 0.76 | 0.69 | 0.83 | 0.82 | 0.71 | 0.91 | 0.74 | 0.66 | 0.82 | 0.75 | 0.69 | 0.81 | 0.82 | 0.74 | 0.89 | 0.72 | 0.64 | 0.79 | 0.76 | 0.71 | 0.80 | 0.83 | 0.77 | 0.89 | 0.72 | 0.68 | 0.79 |
| 975 | 0.75 | 0.68 | 0.82 | 0.83 | 0.74 | 0.91 | 0.73 | 0.64 | 0.81 | 0.75 | 0.69 | 0.81 | 0.84 | 0.78 | 0.91 | 0.71 | 0.63 | 0.78 | 0.75 | 0.71 | 0.80 | 0.84 | 0.79 | 0.89 | 0.73 | 0.66 | 0.78 |
| 976 | 0.74 | 0.67 | 0.81 | 0.81 | 0.69 | 0.93 | 0.72 | 0.63 | 0.80 | 0.75 | 0.69 | 0.80 | 0.82 | 0.74 | 0.91 | 0.72 | 0.65 | 0.79 | 0.76 | 0.72 | 0.80 | 0.83 | 0.77 | 0.90 | 0.72 | 0.68 | 0.78 |
| 977 | 0.75 | 0.67 | 0.82 | 0.77 | 0.62 | 0.91 | 0.74 | 0.65 | 0.83 | 0.75 | 0.67 | 0.79 | 0.82 | 0.75 | 0.89 | 0.71 | 0.64 | 0.78 | 0.75 | 0.70 | 0.79 | 0.81 | 0.74 | 0.87 | 0.73 | 0.67 | 0.78 |
| 978 | 0.75 | 0.67 | 0.83 | 0.83 | 0.68 | 0.99 | 0.73 | 0.64 | 0.82 | 0.73 | 0.67 | 0.79 | 0.79 | 0.68 | 0.90 | 0.71 | 0.63 | 0.78 | 0.76 | 0.71 | 0.80 | 0.82 | 0.73 | 0.90 | 0.72 | 0.68 | 0.79 |
| 979 | 0.75 | 0.66 | 0.84 | 0.89 | 0.75 | 1.00 | 0.71 | 0.61 | 0.82 | 0.73 | 0.69 | 0.79 | 0.82 | 0.72 | 0.92 | 0.69 | 0.62 | 0.77 | 0.76 | 0.71 | 0.81 | 0.86 | 0.78 | 0.93 | 0.73 | 0.67 | 0.78 |
| 980 | 0.74 | 0.66 | 0.82 | 0.81 | 0.68 | 0.94 | 0.72 | 0.62 | 0.81 | 0.75 | 0.68 | 0.81 | 0.82 | 0.74 | 0.91 | 0.71 | 0.64 | 0.78 | 0.76 | 0.71 | 0.80 | 0.83 | 0.77 | 0.90 | 0.73 | 0.68 | 0.78 |
| 981 | 0.76 | 0.67 | 0.84 | 0.85 | 0.68 | 1.00 | 0.73 | 0.63 | 0.83 | 0.74 | 0.67 | 0.80 | 0.86 | 0.78 | 0.93 | 0.69 | 0.62 | 0.77 | 0.77 | 0.73 | 0.82 | 0.88 | 0.82 | 0.94 | 0.73 | 0.68 | 0.79 |
| 982 | 0.75 | 0.66 | 0.83 | 0.86 | 0.71 | 1.00 | 0.71 | 0.61 | 0.81 | 0.73 | 0.68 | 0.79 | 0.79 | 0.68 | 0.90 | 0.70 | 0.63 | 0.78 | 0.74 | 0.69 | 0.79 | 0.81 | 0.73 | 0.90 | 0.71 | 0.66 | 0.77 |
| 983 | 0.76 | 0.69 | 0.83 | 0.83 | 0.75 | 0.92 | 0.74 | 0.65 | 0.82 | 0.74 | 0.68 | 0.81 | 0.81 | 0.74 | 0.89 | 0.71 | 0.64 | 0.79 | 0.75 | 0.71 | 0.80 | 0.82 | 0.77 | 0.88 | 0.73 | 0.67 | 0.78 |
| 984 | 0.75 | 0.68 | 0.83 | 0.82 | 0.72 | 0.93 | 0.73 | 0.65 | 0.82 | 0.74 | 0.68 | 0.81 | 0.85 | 0.78 | 0.92 | 0.70 | 0.62 | 0.77 | 0.76 | 0.71 | 0.80 | 0.85 | 0.79 | 0.90 | 0.72 | 0.66 | 0.77 |
| 985 | 0.75 | 0.68 | 0.82 | 0.81 | 0.68 | 0.95 | 0.73 | 0.65 | 0.81 | 0.74 | 0.68 | 0.80 | 0.81 | 0.73 | 0.89 | 0.72 | 0.64 | 0.79 | 0.75 | 0.70 | 0.80 | 0.82 | 0.75 | 0.88 | 0.72 | 0.68 | 0.78 |
| 986 | 0.76 | 0.68 | 0.83 | 0.83 | 0.74 | 0.93 | 0.73 | 0.64 | 0.82 | 0.74 | 0.68 | 0.80 | 0.83 | 0.76 | 0.90 | 0.71 | 0.63 | 0.78 | 0.76 | 0.71 | 0.80 | 0.84 | 0.78 | 0.89 | 0.72 | 0.67 | 0.79 |
| 987 | 0.75 | 0.67 | 0.83 | 0.84 | 0.70 | 0.97 | 0.72 | 0.62 | 0.82 | 0.74 | 0.66 | 0.80 | 0.84 | 0.75 | 0.93 | 0.70 | 0.63 | 0.79 | 0.74 | 0.69 | 0.79 | 0.84 | 0.76 | 0.91 | 0.71 | 0.65 | 0.76 |
| 988 | 0.73 | 0.63 | 0.82 | 0.90 | 0.81 | 0.98 | 0.68 | 0.57 | 0.79 | 0.73 | 0.66 | 0.79 | 0.76 | 0.64 | 0.87 | 0.72 | 0.64 | 0.79 | 0.75 | 0.70 | 0.80 | 0.82 | 0.74 | 0.90 | 0.72 | 0.66 | 0.78 |

| No. | G | H | I | J | K | L | M | N | O | P | Q | R | S | T | U | V | W | Z | AA | AB | AC | AD | AE | AF | AG | AH | AI |
|---|---|---|---|---|---|---|---|---|---|---|---|---|---|---|---|---|---|---|---|---|---|---|---|---|---|---|---|
| 989 | 0.77 | 0.69 | 0.85 | 0.87 | 0.76 | 0.97 | 0.74 | 0.64 | 0.83 | 0.74 | 0.68 | 0.80 | 0.85 | 0.76 | 0.94 | 0.70 | 0.63 | 0.77 | 0.76 | 0.71 | 0.81 | 0.86 | 0.79 | 0.93 | 0.72 | 0.67 | 0.78 |
| 990 | 0.74 | 0.66 | 0.82 | 0.78 | 0.63 | 0.92 | 0.73 | 0.64 | 0.82 | 0.74 | 0.68 | 0.80 | 0.80 | 0.73 | 0.88 | 0.71 | 0.64 | 0.79 | 0.75 | 0.70 | 0.79 | 0.80 | 0.74 | 0.86 | 0.72 | 0.67 | 0.78 |
| 991 | 0.75 | 0.68 | 0.82 | 0.83 | 0.75 | 0.92 | 0.72 | 0.64 | 0.81 | 0.74 | 0.68 | 0.80 | 0.82 | 0.75 | 0.89 | 0.70 | 0.63 | 0.78 | 0.75 | 0.70 | 0.80 | 0.83 | 0.78 | 0.88 | 0.72 | 0.66 | 0.77 |
| 992 | 0.76 | 0.68 | 0.85 | 0.82 | 0.69 | 0.95 | 0.74 | 0.64 | 0.84 | 0.73 | 0.66 | 0.79 | 0.81 | 0.70 | 0.91 | 0.69 | 0.62 | 0.77 | 0.76 | 0.71 | 0.80 | 0.82 | 0.74 | 0.90 | 0.73 | 0.68 | 0.79 |
| 993 | 0.75 | 0.68 | 0.82 | 0.82 | 0.72 | 0.93 | 0.72 | 0.64 | 0.81 | 0.74 | 0.68 | 0.80 | 0.82 | 0.75 | 0.90 | 0.70 | 0.63 | 0.78 | 0.75 | 0.71 | 0.80 | 0.83 | 0.78 | 0.89 | 0.72 | 0.66 | 0.78 |
| 994 | 0.74 | 0.66 | 0.82 | 0.85 | 0.71 | 0.98 | 0.71 | 0.62 | 0.80 | 0.74 | 0.68 | 0.80 | 0.82 | 0.72 | 0.93 | 0.70 | 0.63 | 0.77 | 0.75 | 0.70 | 0.79 | 0.84 | 0.76 | 0.92 | 0.71 | 0.66 | 0.77 |
| 995 | 0.74 | 0.67 | 0.82 | 0.81 | 0.69 | 0.93 | 0.72 | 0.63 | 0.81 | 0.74 | 0.68 | 0.80 | 0.79 | 0.71 | 0.88 | 0.72 | 0.64 | 0.79 | 0.75 | 0.71 | 0.80 | 0.81 | 0.75 | 0.88 | 0.73 | 0.67 | 0.78 |
| 996 | 0.75 | 0.67 | 0.83 | 0.85 | 0.71 | 0.99 | 0.72 | 0.62 | 0.81 | 0.74 | 0.68 | 0.80 | 0.83 | 0.73 | 0.93 | 0.70 | 0.63 | 0.77 | 0.75 | 0.71 | 0.80 | 0.84 | 0.77 | 0.92 | 0.72 | 0.67 | 0.78 |
| 997 | 0.74 | 0.67 | 0.82 | 0.82 | 0.70 | 0.93 | 0.72 | 0.63 | 0.80 | 0.74 | 0.67 | 0.80 | 0.78 | 0.70 | 0.87 | 0.72 | 0.64 | 0.79 | 0.75 | 0.70 | 0.79 | 0.81 | 0.74 | 0.87 | 0.72 | 0.67 | 0.77 |
| 998 | 0.77 | 0.68 | 0.85 | 0.85 | 0.68 | 1.00 | 0.74 | 0.65 | 0.84 | 0.75 | 0.69 | 0.81 | 0.85 | 0.76 | 0.93 | 0.71 | 0.64 | 0.78 | 0.78 | 0.74 | 0.83 | 0.87 | 0.81 | 0.94 | 0.75 | 0.69 | 0.80 |
| 999 | 0.76 | 0.68 | 0.84 | 0.84 | 0.67 | 1.00 | 0.73 | 0.64 | 0.82 | 0.72 | 0.65 | 0.78 | 0.77 | 0.66 | 0.88 | 0.69 | 0.62 | 0.77 | 0.74 | 0.70 | 0.79 | 0.81 | 0.72 | 0.89 | 0.72 | 0.66 | 0.77 |

Further, **Table 4C** provides an overview of the distribution of constituents (%) over the panels of Tables 4A and 4B. In **Table 4C,** column **AJ** represents the total number of panels; column **AK** represents the number of constituents in a panel (between 6 and 1 ) and columns **AL** and **AM** represent respectively the number and proportion (%) of panels that have the number of constituent stated in the respective row of column **AK;** column **AN** represents biomarkers and columns **AO** and **AP** represent respectively the number and proportion (%) of panels that contain the constituent stated in the respective row of column **AN;** and column **AQ** represents clinical parameters and columns **AR** and **AS** represent respectively the number and proportion (%) of panels that contain the clinical parameter stated in the respective row of column **AQ.**

**Table 4C**

| AJ | AK | AL | AM | AN | AO | AP | AQ | AR | AS |
|---|---|---|---|---|---|---|---|---|---|
| 1008 | 6 | 355 | 35.5 | IGFALS | 838 | 83.1 | BP20 | 462 | 45.8 |
| | 5 | 472 | 47.2 | MCAM | 507 | 50.3 | BP15 | 405 | 40.2 |
| | 4 | 162 | 16.2 | ENG | 480 | 47.6 | *alcoh* | 178 | 17.7 |
| | 3 | 19 | 1.9 | ADAM 12 | 437 | 43.4 | bmi | 105 | 10.4 |
| | 2 | 0 | 0.0 | PIGF | 366 | 36.3 | *fihd* | 85 | 8.4 |
| | 1 | 0 | 0.0 | MMRN2 | 319 | 31.6 | *fhpet* | 44 | 4.4 |
| | | | | SPINT1 | 314 | 31.2 | *vagbl* | 36 | 3.6 |
| | | | | QSOX1 | 143 | 14.2 | pbwgt | 8 | 0.8 |
| | | | | SEPP1 | 134 | 13.3 | *gest* | 7 | 0.7 |
| | | | | ECM1 | 89 | 8.8 | *age* | 1 | 0.1 |
| | | | | ROBO4 | 65 | 6.4 | mothpet | 2 | 0.2 |
| | | | | LNPEP | 54 | 5.4 | | | |
| | | | | ALDOA | 41 | 4.1 | | | |
| | | | | MAPRE1/3 | 33 | 3.3 | | | |
| | | | | ENPP2 | 34 | 3.4 | | | |
| | | | | LCAT | 4 | 0.4 | | | |
| | | | | PRDX2 | 2 | 0.2 | | | |
| | | | | PRCP | 2 | 0.2 | | | |

**Examples 5 to 13: Analysis of data Tables 4A, 4B**

While **Tables 4A and 4B** provide source data concerning relevant statistics pertinent to performance of illustrative, non-limiting panels useful for predicting PE, the following examples further process the data to extract additional information on certain subgroups of panels from those of **Tables 4A and 4B,** which may be particularly well-performing or otherwise useful or of interest. The tables also capture the frequency at which the constituent biomarkers and/or clinical parameters occur in such panels. It can be expected that the higher the frequency, the higher the relative importance of a biomarker and/or clinical parameter will be in the subgroup of panels in question, and in panels in general.

In this connection, it was explained above that **Tables 4A and 4B** are to some extent redundant with regard to the test panels. The following analysis was performed on the entire data set of **Tables 4A and 4B,** i.e., without removing said redundancy. Consequently, test panels containing biomarkers and/or clinical parameters that cause the redundancy, specifically ADAM12, ECM1, LCAT, and/or BP may be to some, comparatively minor extent overrepresented in **Tables 4A and 4B.** This, however, does not detract from the analysis of the frequencies of occurrence of the analysed biomarkers and clinical parameters in the panels, and the corresponding relative importance of the biomarkers and clinical parameters, as set forth in **Tables 5A-N, 6A-N, 7A-N, 8A-L, 9A-N** and **10A-F.**

In the following tables, and namely **Tables 5A-N, 6A-N, 7A-N, 8A-L, 9A-N** and **10A-F,** column **AJ** represents the total number of panels that meet the stated criterion or criteria; column **AK** represents the number of constituents in a panel (between 6 and 1) and columns **AL** and **AM** represent respectively the number and proportion (%) of panels that have the number of constituent stated in the respective row of column **AK;** column **AN** represents biomarkers and columns **AO** and **AP** represent respectively the number and proportion (%) of panels that contain the constituent stated in the respective row of column **AN;** and column **AQ** represents clinical parameters and columns **AR** and **AS** represent respectively the number and proportion (%) of panels that contain the clinical parameter stated in the respective row of column **AQ.**

**Example 5**

**Table 5A** captures panels of Tables 4A and 4B in which sensitivity at 20% PPV for predicting all PE (i.e., without distinction between preterm and term PE) at 20 weeks in training set (Australasian cohort) is equal to or greater than 0.495 and sensitivity at 20% PPV for predicting all PE at 20 weeks in testing set (European cohort) is equal to or greater than 0.495. Without limitation, such panels may be particularly useful as rule-in panels, more specifically for predicting PE, even more specifically for predicting PE without distinction between preterm and term PE.

**Table 5B** captures panels of Tables 4A and 4B in which specificity at 99% NPV for predicting all PE (i.e., without distinction between preterm and term PE) at 20 weeks in training set (Australasian cohort) is equal to or greater than 0.395 and specificity at 99% NPV for predicting all PE at 20 weeks in testing set (European cohort) is equal to or greater than 0.395. Without limitation, such panels may be particularly useful as rule-out panels, more specifically for predicting PE, even more specifically for predicting PE without distinction between preterm and term PE.

**Table 5C** captures panels of Tables 4A and 4B in which sensitivity at 20% PPV for predicting all PE (i.e., without distinction between preterm and term PE) at 20 weeks in training set (Australasian cohort) is equal to or greater than 0.495 and sensitivity at 20% PPV for predicting all PE at 20 weeks in testing set (European cohort) is equal to or greater than 0.495 and specificity at 99% NPV for predicting all PE at 20 weeks in training set (Australasian cohort) is equal to or greater than 0.395 and specificity at 99% NPV for predicting all PE at 20 weeks in testing set (European cohort) is equal to or greater than 0.395. Without limitation, such panels may be particularly useful as rule-in and rule-out panels, more specifically for predicting PE, even more specifically for predicting PE without distinction between preterm and term PE.

**Table 5D** captures panels of Tables 4A and 4B in which AUC for predicting all PE (i.e., without distinction between preterm and term PE) at 20 weeks in training set (Australasian cohort) is equal to or greater than 0.745 and AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 5E** captures panels of Tables 4A and 4B in which AUC for predicting all PE (i.e., without distinction between preterm and term PE) at 20 weeks in training set (Australasian cohort) is equal to or greater than 0.775 and AUC for predicting all PE at 20 weeks in testing set (European cohort) is equal to or greater than 0.775. Without limitation, such panels may be particularly useful for predicting PE, even more specifically for predicting PE without distinction between preterm and term PE.

**Table 5F** captures panels of Tables 4A and 4B in which AUC for predicting preterm PE at 20 weeks in the European cohort or in the Australasian cohort or in the combined European and Australasian cohort is equal to or greater than 0.745. Without limitation, such panels may be particularly useful for predicting preterm PE.

**Table 5G** captures panels of Tables 4A and 4B in which AUC for predicting preterm PE at 20 weeks in the European cohort is equal to or greater than 0.895. Without limitation, such panels may be particularly useful for predicting preterm PE, even more specifically in European ancestry patients.

**Table 5H** captures panels of Tables 4A and 4B in which AUC for predicting preterm PE at 20 weeks in the Australasian cohort is equal to or greater than 0.895. Without limitation, such panels may be particularly useful for predicting preterm PE, even more specifically in Australasian ancestry patients.

**Table 5I** captures panels of Tables 4A and 4B in which AUC for predicting preterm PE at 20 weeks in the combined European and Australasian cohort is equal to or greater than 0.895. Without limitation, such panels may be particularly useful for predicting preterm PE.

**Table 5J** captures panels of Tables 4A and 4B in which AUC for predicting term PE at 20 weeks in the European cohort is equal to or greater than 0.745. Without limitation, such panels may be particularly useful for predicting term PE, even more specifically in European ancestry patients.

**Table 5K** captures panels of Tables 4A and 4B in which AUC for predicting term PE at 20 weeks in the Australasian cohort is equal to or greater than 0.745. Without limitation, such panels may be particularly useful for predicting term PE, even more specifically in Australasian ancestry patients.

**Table 5L** captures panels of Tables 4A and 4B in which AUC for predicting term PE at 20 weeks in the combined European and Australasian cohort is equal to or greater than 0.745. Without limitation, such panels may be particularly useful for predicting term PE.

**Table 5M** captures panels of Tables 4A and 4B which are particularly preferred, which have good rule-in and/or rule-out performance, and which contain trivial clinical parameters, such as for example blood pressure. Table 5Ma lists these particular panels.

**Table 5N** captures panels of Tables 4A and 4B which are even more preferred, which have especially good rule-in and/or rule-out performance, and which contain trivial clinical parameters, such as for example blood pressure. Table 5Na lists these particular panels.

**Table 5A**

| AJ | AK | AL | AM | AN | AO | AP | AQ | AR | AS |
|---|---|---|---|---|---|---|---|---|---|
| 181 | 6 | 168 | 92.8 | ENG | 163 | 90.1 | BP15 | 93 | 51.4 |
| | 5 | 13 | 7.2 | IGFALS | 148 | 81.8 | BP20 | 60 | 33.1 |
| | 4 | 0 | 0.0 | MCAM | 140 | 77.3 | *fihd* | 33 | 18.2 |
| | 3 | 0 | 0.0 | SPINT1 | 121 | 66.9 | *alcoh* | 33 | 18.2 |
| | 2 | 0 | 0.0 | MMRN2 | 58 | 32.0 | bmi | 19 | 10.5 |
| | 1 | 0 | 0.0 | ADAM12 | 57 | 31.5 | *fhpet* | 6 | 3.3 |
| | | | | PIGF | 45 | 24.9 | *vagbl* | 6 | 3.3 |
| | | | | SEPP1 | 31 | 17.1 | *gest* | 3 | 1.7 |
| | | | | QSOX1 | 16 | 8.8 | *age* | 1 | 0.6 |
| | | | | ECM1 | 15 | 8.3 | | | |
| | | | | ROBO4 | 14 | 7.7 | | | |
| | | | | LNPEP | 6 | 3.3 | | | |
| | | | | LCAT | 2 | 1.1 | | | |
| | | | | PRCP | 2 | 1.1 | | | |
| | | | | ENPP2 | 1 | 0.6 | | | |

**Table 5B**

| AJ | AK | AL | AM | AN | AO | AP | AQ | AR | AS |
|---|---|---|---|---|---|---|---|---|---|
| 17 | 6 | 4 | 23.5 | IGFALS | 16 | 94.1 | *alcoh* | 8 | 47.1 |
| | 5 | 12 | 70.6 | ADAM12 | 13 | 76.5 | BP20 | 7 | 41.2 |
| | 4 | 1 | 5.9 | MMRN2 | 9 | 52.9 | BP15 | 4 | 23.5 |
| | 3 | 0 | 0.0 | PIGF | 8 | 47.1 | *fhpet* | 3 | 17.6 |
| | 2 | 0 | 0.0 | MCAM | 6 | 35.3 | bmi | 2 | 11.8 |
| | 1 | 0 | 0.0 | QSOX1 | 2 | 11.8 | pbwgt | 2 | 11.8 |
| | | | | SEPP1 | 2 | 11.8 | *vagbl* | 1 | 5.9 |
| | | | | ENPP2 | 2 | 11.8 | | | |
| | | | | MAPRE1/3 | 1 | 5.9 | | | |
| | | | | ALDOA | 1 | 5.9 | | | |
| | | | | LNPEP | 1 | 5.9 | | | |

**Table 5C**

| AJ | AK | AL | AM | AN | AO | AP | AQ | AR | AS |
|---|---|---|---|---|---|---|---|---|---|
| 1 | 6 | 1 | 100 | MMRN2 | 1 | 100 | BP15 | 1 | 100 |
| | 5 | 0 | 0 | ADAM12 | 1 | 100 | | | |
| | 4 | 0 | 0 | IGFALS | 1 | 100 | | | |

(continued)

| AJ | AK | AL | AM | AN | AO | AP | AQ | AR | AS |
|---|---|---|---|---|---|---|---|---|---|
| | 3 | 0 | 0 | MCAM | 1 | 100 | | | |
| | 2 | 0 | 0 | PIGF | 1 | 100 | | | |
| | 1 | 0 | 0 | | | | | | |

**Table 5D**

| AJ | AK | AL | AM | AN | AO | AP | AQ | AR | AS |
|---|---|---|---|---|---|---|---|---|---|
| 615 | 6 | 269 | 43.7 | IGFALS | 519 | 84.4 | BP20 | 345 | 56.0 |
| | 5 | 264 | 42.9 | MCAM | 349 | 56.7 | BP15 | 203 | 33.0 |
| | 4 | 80 | 13.0 | ENG | 313 | 50.9 | *alcoh* | 108 | 17.5 |
| | 3 | 2 | 0.3 | ADAM12 | 253 | 41.1 | bmi | 63 | 10.2 |
| | 2 | 0 | 0.0 | MMRN2 | 213 | 34.6 | *fihd* | 29 | 4.7 |
| | 1 | 0 | 0.0 | PIGF | 212 | 34.5 | *fhpet* | 28 | 4.5 |
| | | | | SPINT1 | 204 | 33.2 | *vagbl* | 24 | 3.9 |
| | | | | QSOX1 | 91 | 14.8 | pbwgt | 5 | 0.8 |
| | | | | SEPP1 | 89 | 14.5 | *gest* | 3 | 0.5 |
| | | | | ECM1 | 58 | 9.4 | *age* | 1 | 0.2 |
| | | | | ROBO4 | 44 | 7.2 | mothpet | 1 | 0.2 |
| | | | | LNPEP | 35 | 5.7 | | | |
| | | | | ENPP2 | 23 | 3.7 | | | |
| | | | | ALDOA | 22 | 3.6 | | | |
| | | | | MAPRE1/3 | 20 | 3.3 | | | |
| | | | | LCAT | 2 | 0.3 | | | |
| | | | | PRDX2 | 2 | 0.3 | | | |
| | | | | PRCP | 1 | 0.2 | | | |

**Table 5E**

| AJ | AK | AL | AM | AN | AO | AP | AQ | AR | AS |
|---|---|---|---|---|---|---|---|---|---|
| 22 | 6 | 11 | 50 | IGFALS | 22 | 100 | BP20 | 17 | 77.3 |
| | 5 | 11 | 50 | MCAM | 16 | 72.7 | BP15 | 4 | 18.2 |
| | 4 | 0 | 0 | MMRN2 | 15 | 68.2 | *alcoh* | 2 | 9.1 |
| | 3 | 0 | 0 | ADAM12 | 12 | 54.5 | *vagbl* | 2 | 9.1 |
| | 2 | 0 | 0 | ENG | 10 | 45.5 | bmi | 1 | 4.5 |
| | 1 | 0 | 0 | PIGF | 9 | 40.9 | | | |
| | | | | SPINT1 | 5 | 22.7 | | | |
| | | | | ECM1 | 2 | 9.1 | | | |
| | | | | LNPEP | 2 | 9.1 | | | |
| | | | | QSOX1 | 1 | 4.5 | | | |

(continued)

| AJ | AK | AL | AM | AN | AO | AP | AQ | AR | AS |
|----|----|----|----|----|----|----|----|----|----|
|    |    |    |    | SEPP1 | 1 | 4.5 |    |    |    |

**Table 5F**

| AJ | AK | AL | AM | AN | AO | AP | AQ | AR | AS |
|----|----|----|----|----|----|----|----|----|----|
| 999 | 6 | 348 | 34.8 | IGFALS | 838 | 83.9 | BP20 | 456 | 45.6 |
|    | 5 | 470 | 47.0 | MCAM | 498 | 49.8 | BP15 | 403 | 40.3 |
|    | 4 | 162 | 16.2 | ENG | 473 | 47.3 | *alcoh* | 175 | 17.5 |
|    | 3 | 19 | 1.9 | ADAM12 | 436 | 43.6 | bmi | 102 | 10.2 |
|    | 2 | 0 | 0.0 | PIGF | 362 | 36.2 | *fihd* | 85 | 8.5 |
|    | 1 | 0 | 0.0 | MMRN2 | 317 | 31.7 | *fhpet* | 41 | 4.1 |
|    |   |   |   | SPINT1 | 308 | 30.8 | *vagbl* | 36 | 3.6 |
|    |   |   |   | QSOX1 | 143 | 14.3 | pbwgt | 8 | 0.8 |
|    |   |   |   | SEPP1 | 134 | 13.4 | *gest* | 7 | 0.7 |
|    |   |   |   | ECM1 | 86 | 8.6 | *age* | 1 | 0.1 |
|    |   |   |   | ROBO4 | 65 | 6.5 | mothpet | 2 | 0.2 |
|    |   |   |   | LNPEP | 54 | 5.4 |    |    |    |
|    |   |   |   | ALDOA | 41 | 4.1 |    |    |    |
|    |   |   |   | MAPRE1/3 | 33 | 3.3 |    |    |    |
|    |   |   |   | ENPP2 | 32 | 3.2 |    |    |    |
|    |   |   |   | LCAT | 3 | 0.3 |    |    |    |
|    |   |   |   | PRDX2 | 2 | 0.2 |    |    |    |
|    |   |   |   | PRCP | 2 | 0.2 |    |    |    |

**Table 5G**

| AJ | AK | AL | AM | AN | AO | AP | AQ | AR | AS |
|----|----|----|----|----|----|----|----|----|----|
| 46 | 6 | 35 | 76.1 | SPINT1 | 36 | 78.3 | BP20 | 27 | 58.7 |
|    | 5 | 11 | 23.9 | ENG | 34 | 73.9 | *alcoh* | 9 | 19.6 |
|    | 4 | 0 | 0.0 | IGFALS | 33 | 71.7 | BP15 | 2 | 4.3 |
|    | 3 | 0 | 0.0 | MCAM | 32 | 69.6 | bmi | 2 | 4.3 |
|    | 2 | 0 | 0.0 | MMRN2 | 30 | 65.2 | *vagbl* | 1 | 2.2 |
|    | 1 | 0 | 0.0 | ADAM12 | 21 | 45.7 | *gest* | 1 | 2.2 |
|    |   |   |   | PIGF | 16 | 34.8 |    |    |    |
|    |   |   |   | ECM1 | 13 | 28.3 |    |    |    |
|    |   |   |   | QSOX1 | 2 | 4.3 |    |    |    |
|    |   |   |   | ROBO4 | 2 | 4.3 |    |    |    |
|    |   |   |   | PRCP | 2 | 4.3 |    |    |    |
|    |   |   |   | LNPEP | 1 | 2.2 |    |    |    |

(continued)

| AJ | AK | AL | AM | AN | AO | AP | AQ | AR | AS |
|----|----|----|----|------|----|-----|----|----|----|
|    |    |    |    | ENPP2 | 1 | 2.2 |    |    |    |

### Table 5H

| AJ | AK | AL | AM | AN | AO | AP | AQ | AR | AS |
|----|----|----|----|--------|-----|------|------|----|------|
| 161 | 6 | 133 | 82.6 | IGFALS | 156 | 96.9 | BP15 | 85 | 52.8 |
|    | 5 | 28 | 17.4 | ENG | 156 | 96.9 | BP20 | 49 | 30.4 |
|    | 4 | 0 | 0.0 | SPINT1 | 147 | 91.3 | *alcoh* | 21 | 13.0 |
|    | 3 | 0 | 0.0 | MCAM | 81 | 50.3 | *fihd* | 9 | 5.6 |
|    | 2 | 0 | 0.0 | MMRN2 | 75 | 46.6 | bmi | 6 | 3.7 |
|    | 1 | 0 | 0.0 | ADAM12 | 44 | 27.3 | *gesf* | 4 | 2.5 |
|    |   |   |   | PlGF | 35 | 21.7 | *fhpet* | 2 | 1.2 |
|    |   |   |   | QSOX1 | 35 | 21.7 | *age* | 1 | 0.6 |
|    |   |   |   | SEPP1 | 26 | 16.1 |    |    |    |
|    |   |   |   | ROBO4 | 4 | 2.5 |    |    |    |
|    |   |   |   | LNPEP | 2 | 1.2 |    |    |    |

### Table 5I

| AJ | AK | AL | AM | AN | AO | AP | AQ | AR | AS |
|----|----|----|----|--------|-----|------|------|----|------|
| 210 | 6 | 163 | 77.6 | SPINT1 | 209 | 99.5 | BP15 | 93 | 44.3 |
|    | 5 | 46 | 21.9 | ENG | 201 | 95.7 | BP20 | 60 | 28.6 |
|    | 4 | 1 | 0.5 | IGFALS | 193 | 91.9 | bmi | 28 | 13.3 |
|    | 3 | 0 | 0.0 | MCAM | 124 | 59.0 | *alcoh* | 26 | 12.4 |
|    | 2 | 0 | 0.0 | MMRN2 | 68 | 32.4 | *fihd* | 12 | 5.7 |
|    | 1 | 0 | 0.0 | ADAM12 | 61 | 29.0 | *fhpet* | 8 | 3.8 |
|    |   |   |   | PlGF | 41 | 19.5 | *gest* | 5 | 2.4 |
|    |   |   |   | QSOX1 | 37 | 17.6 | *vagbl* | 1 | 0.5 |
|    |   |   |   | SEPP1 | 28 | 13.3 | *age* | 1 | 0.5 |
|    |   |   |   | ROBO4 | 10 | 4.8 |    |    |    |
|    |   |   |   | LNPEP | 3 | 1.4 |    |    |    |
|    |   |   |   | ENPP2 | 3 | 1.4 |    |    |    |

### Table 5J

| AJ | AK | AL | AM | AN | AO | AP | AQ | AR | AS |
|----|----|----|----|--------|-----|------|------|----|------|
| 61 | 6 | 38 | 62.3 | IGFALS | 58 | 95.1 | BP20 | 43 | 70.5 |
|    | 5 | 21 | 34.4 | MCAM | 41 | 67.2 | BP15 | 16 | 26.2 |
|    | 4 | 2 | 3.3 | ADAM12 | 33 | 54.1 | *alcoh* | 12 | 19.7 |
|    | 3 | 0 | 0.0 | ENG | 27 | 44.3 | bmi | 9 | 14.8 |

(continued)

| AJ | AK | AL | AM | AN | AO | AP | AQ | AR | AS |
|---|---|---|---|---|---|---|---|---|---|
| | 2 | 0 | 0.0 | PIGF | 27 | 44.3 | *vagbl* | 6 | 9.8 |
| | 1 | 0 | 0.0 | MMRN2 | 27 | 44.3 | *fhpet* | 2 | 3.3 |
| | | | | SEPP1 | 12 | 19.7 | *fihd* | 1 | 1.6 |
| | | | | QSOX1 | 11 | 18.0 | | | |
| | | | | SPINT1 | 6 | 9.8 | | | |
| | | | | LNPEP | 4 | 6.6 | | | |
| | | | | ECM1 | 3 | 4.9 | | | |
| | | | | ALDOA | 2 | 3.3 | | | |
| | | | | MAPRE1/3 | 1 | 1.6 | | | |

**Table 5K**

| AJ | AK | AL | AM | AN | AO | AP | AQ | AR | AS |
|---|---|---|---|---|---|---|---|---|---|
| 426 | 6 | 208 | 48.8 | IGFALS | 358 | 84.0 | BP15 | 250 | 58.7 |
| | 5 | 199 | 46.7 | MCAM | 285 | 66.9 | BP20 | 112 | 26.3 |
| | 4 | 19 | 4.5 | ADAM12 | 238 | 55.9 | *alcoh* | 84 | 19.7 |
| | 3 | 0 | 0.0 | PIGF | 211 | 49.5 | bmi | 76 | 17.8 |
| | 2 | 0 | 0.0 | ENG | 200 | 46.9 | *fihd* | 67 | 15.7 |
| | 1 | 0 | 0.0 | SPINT1 | 113 | 26.5 | *fhpet* | 19 | 4.5 |
| | | | | MMRN2 | 104 | 24.4 | *vagbl* | 12 | 2.8 |
| | | | | SEPP1 | 45 | 10.6 | pbwgt | 5 | 1.2 |
| | | | | QSOX1 | 37 | 8.7 | *gest* | 1 | 0.2 |
| | | | | ECM1 | 33 | 7.7 | mothpet | 1 | 0.2 |
| | | | | ROBO4 | 18 | 4.2 | | | |
| | | | | ALDOA | 16 | 3.8 | | | |
| | | | | LNPEP | 12 | 2.8 | | | |
| | | | | ENPP2 | 11 | 2.6 | | | |
| | | | | MAPRE1/3 | 10 | 2.3 | | | |
| | | | | LCAT | 1 | 0.2 | | | |

**Table 5L**

| AJ | AK | AL | AM | AN | AO | AP | AQ | AR | AS |
|---|---|---|---|---|---|---|---|---|---|
| 589 | 6 | 273 | 46.3 | IGFALS | 521 | 88.5 | BP15 | 285 | 48.4 |
| | 5 | 264 | 44.8 | MCAM | 352 | 59.8 | BP20 | 233 | 39.6 |
| | 4 | 52 | 8.8 | ENG | 318 | 54.0 | *alcoh* | 139 | 23.6 |
| | 3 | 0 | 0.0 | PIGF | 250 | 42.4 | bmi | 90 | 15.3 |
| | 2 | 0 | 0.0 | ADAM12 | 247 | 41.9 | *fihd* | 55 | 9.3 |
| | 1 | 0 | 0.0 | SPINT1 | 175 | 29.7 | *fhpet* | 29 | 4.9 |

(continued)

| AJ | AK | AL | AM | AN | AO | AP | AQ | AR | AS |
|----|----|----|----|----|----|----|----|----|----|
|  |  |  |  | MMRN2 | 158 | 26.8 | *vagbl* | 19 | 3.2 |
|  |  |  |  | QSOX1 | 72 | 12.2 | pbwgt | 6 | 1.0 |
|  |  |  |  | SEPP11 | 63 | 10.7 | *gest* | 3 | 0.5 |
|  |  |  |  | ROBO4 | 35 | 5.9 | mothpet | 2 | 0.3 |
|  |  |  |  | ECM1 | 29 | 4.9 | *age* | 1 | 0.2 |
|  |  |  |  | ALDOA | 27 | 4.6 |  |  |  |
|  |  |  |  | LNPEP | 24 | 4.1 |  |  |  |
|  |  |  |  | MAPRE1/3 | 21 | 3.6 |  |  |  |
|  |  |  |  | ENPP2 | 12 | 2.0 |  |  |  |

**Table 5M**

| AJ | AK | AL | AM | AN | AO | AP | AQ | AR | AS |
|----|----|----|----|----|----|----|----|----|----|
| 50 | 6 | 39 | 78 | IGFALS | 50 | 100 | BP15 | 37 | 74 |
|  | 5 | 10 | 20 | SPINT1 | 43 | 86 | BP20 | 7 | 14 |
|  | 4 | 1 | 2 | ENG | 42 | 84 | *gest* | 3 | 6 |
|  | 3 | 0 | 0 | MCAM | 39 | 78 |  |  |  |
|  | 2 | 0 | 0 | PIGF | 23 | 46 |  |  |  |
|  | 1 | 0 | 0 | ADAM12 | 22 | 44 |  |  |  |
|  |  |  |  | MMRN2 | 22 | 44 |  |  |  |

**Table 5Ma**

| Panels |
|--------|
| BP15;ENG;SPINT1;IGFALS;MCAM;PIGF |
| BP15;MMRN2;ENG;IGFALS;MCAM;PIGF |
| BP20;ENG;SPINT1;IGFALS;MCAM;PIGF |
| MMRN2;ADAM12;ENG;SPINT1;IGFALS;MCAM |
| BP15;ENG;SPINT1;IGFALS;MCAM;PIGF |
| BP20;ENG;SPINT1;IGFALS;MCAM;PIGF |
| BP15;ADAM12;SPINT1;IGFALS;MCAM;PIGF |
| BP15;ADAM12;SPINT1;IGFALS;MCAM;PIGF |
| BP15;MMRN2;ENG;SPINT1;IGFALS;MCAM |
| ENG;SPINT1;IGFALS;MCAM;PIGF |
| BP20;ADAM12;IGFALS;MCAM;PIGF |
| BP15;ENG;SPINT1;IGFALS;MCAM |
| MMRN2;ENG;SPINT1;IGFALS;MCAM;PIGF |
| BP15;ADAM12;SPINT1;IGFALS;MCAM;PIGF |
| BP15;ENG;SPINT1;IGFALS;MCAM |

(continued)

| Panels |
| --- |
| BP15;ENG;SPINT1;IGFALS;MCAM;PIGF |
| BP15;ENG;SPINT1;IGFALS;MCAM;PIGF |
| BP15;ADAM12;SPINT1;IGFALS;MCAM;PIGF |
| BP15;ENG;SPINT1;IGFALS;MCAM |
| BP15;MMRN2;ADAM12;ENG;SPINT1;IGFALS |
| BP15;MMRN2;ADAM12;IGFALS;MCAM;PIGF |
| BP20;MMRN2;ENG;SPINT1;IGFALS;MCAM |
| BP15;ENG;SPINT1;IGFALS;MCAM |
| BP15;MMRN2;ADAM12;ENG;SPINT1;IGFALS |
| BP15;ENG;SPINT1;IGFALS;MCAM;PIGF |
| *gest*;ENG;SPINT1;IGFALS;MCAM;PIGF |
| BP15;ADAM12;ENG;SPINT1;IGFALS;PIGF |
| BP15;*gest*;ENG;SPINT1;IGFALS;PIGF |
| BP15;MMRN2;ADAM12;ENG;IGFALS;MCAM |
| BP20;*gest*;MMRN2;ENG;SPINT1;IGFALS |
| BP15;MMRN2;ADAM12;ENG;SPINT1;IGFALS |
| BP15;MMRN2;ENG;SPINT1;IGFALS;MCAM |
| MMRN2;ENG;SPINT1;IGFALS;MCAM |
| BP15;ADAM12;ENG;SPINT1;IGFALS;MCAM |
| BP15;ENG;SPINT1;IGFALS;MCAM |
| BP15;MMRN2;ADAM12;ENG;SPINT1;IGFALS |
| BP15;ADAM12;IGFALS;MCAM;PIGF |
| BP15;ADAM12;ENG;SPINT1;IGFALS;PIGF |
| BP15;MMRN2;ENG;SPINT;IGFALS;MCAM |
| BP15;MMRN2;ENG;SPINT1;IGFALS;MCAM |
| BP20;MMRN2;ENG;SPINT1;IGFALS;MCAM |
| BP15;MMRN2;ENG;SPINT1;IGFALS;MCAM |
| BP20;ADAM12;ENG;SPINT1;IGFALS;MCAM |
| BP15;MMRN2;ENG;SPINT1;IGFALS;PIGF |
| BP15;ADAM12;ENG;SPINT1;IGFALS;MCAM |
| BP15;ADAM12;ENG;SPINT1;IGFALS;MCAM |
| BP15;ADAM12;ENG;SPINT1;IGFALS;MCAM |
| BP15;MMRN2;ENG;IGFALS;MCAM |
| BP15;MMRN2;ADAM12;ENG;SPINT1;IGFALS |
| MMRN2;ADAM12;IGFALS;PIGF |

**Table 5N**

| AJ | AK | AL | AM | AN | AO | AP | AQ | AR | AS |
|----|----|----|----|----|----|----|----|----|----|
| 5 | 6 | 3 | 60 | IGFALS | 5 | 100 | BP20 | 3 | 60 |
|  | 5 | 1 | 20 | SPINT1 | 4 | 80 | BP15 | 1 | 20 |
|  | 4 | 1 | 20 | ENG | 4 | 80 |  |  |  |
|  | 3 | 0 | 0 | MCAM | 3 | 60 |  |  |  |
|  | 2 | 0 | 0 | PIGF | 3 | 60 |  |  |  |
|  | 1 | 0 | 0 | ADAM12 | 2 | 40 |  |  |  |
|  |  |  |  | MMRN2 | 2 | 40 |  |  |  |

**Table 5Na**

| |
|---|
| BP20;ENG;SPINT1;IGFALS;MCAM;PIGF |
| BP20;ADAM12;IGFALS;MCAM;PIGF |
| BP15;MMRN2;ADAM12;IGFALS;MCAM;PIGF |
| BP20;MMRN2;ENG;SPINT1;IGFALS;MCAM |
| MMRN2;ADAM12;IGFALS;PIGF |

## Example 6

This example relates to panels of Tables 4A and 4B all of which contain IGFALS and measurement of blood pressure (hence, IGFALS and measurement of blood pressure, which are always present in these panels, are not listed in the tables).

**Table 6A** captures panels of Tables 4A and 4B containing IGFALS and measurement of blood pressure in which sensitivity at 20% PPV for predicting all PE (i.e., without distinction between preterm and term PE) at 20 weeks in training set (Australasian cohort) is equal to or greater than 0.495 and sensitivity at 20% PPV for predicting all PE at 20 weeks in testing set (European cohort) is equal to or greater than 0.495. Without limitation, such panels may be particularly useful as rule-in panels, more specifically for predicting PE, even more specifically for predicting PE without distinction between preterm and term PE.

**Table 6B** captures panels of Tables 4A and 4B containing IGFALS and measurement of blood pressure in which specificity at 99% NPV for predicting all PE (i.e., without distinction between preterm and term PE) at 20 weeks in training set (Australasian cohort) is equal to or greater than 0.395 and specificity at 99% NPV for predicting all PE at 20 weeks in testing set (European cohort) is equal to or greater than 0.395. Without limitation, such panels may be particularly useful as rule-out panels, more specifically for predicting PE, even more specifically for predicting PE without distinction between preterm and term PE.

**Table 6C** captures panels of Tables 4A and 4B containing IGFALS and measurement of blood pressure in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495 and specificity at 99% NPV for predicting all PE at 20 weeks in training set (Australasian cohort) is equal to or greater than 0.395 **and** specificity at 99% NPV for predicting all PE at 20 weeks in testing set (European cohort) is equal to or greater than 0.395. Without limitation, such panels may be particularly useful as rule-in and rule-out panels, more specifically for predicting PE, even more specifically for predicting PE without distinction between preterm and term PE.

**Table 6D** captures panels of Tables 4A and 4B containing IGFALS and measurement of blood pressure in which AUC for predicting all PE (i.e., without distinction between preterm and term PE) at 20 weeks in training set (Australasian cohort) is equal to or greater than 0.745 **and** AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 6E** captures panels of Tables 4A and 4B containing IGFALS and measurement of blood pressure in which AUC for predicting all PE (i.e., without distinction between preterm and term PE) at 20 weeks in training set (Australasian cohort) is equal to or greater than 0.775 **and** AUC for predicting all PE at 20 weeks in testing set (European cohort) is equal to or greater than 0.775. Without limitation, such panels may be particularly useful for predicting PE, even more specifically for predicting PE without distinction between preterm and term PE.

**Table 6F** captures panels of Tables 4A and 4B containing IGFALS and measurement of blood pressure in which AUC for predicting preterm PE at 20 weeks in the European cohort or in the Australasian cohort **or** in the combined European and Australasian cohort is equal to or greater than 0.745. Without limitation, such panels may be particularly useful for predicting preterm PE.

**Table 6G** captures panels of Tables 4A and 4B containing IGFALS and measurement of blood pressure in which AUC for predicting preterm PE at 20 weeks in the European cohort is equal to or greater than 0.895. Without limitation, such panels may be particularly useful for predicting preterm PE, even more specifically in European ancestry patients.

**Table 6H** captures panels of Tables 4A and 4B containing IGFALS and measurement of blood pressure in which AUC for predicting preterm PE at 20 weeks in the Australasian cohort is equal to or greater than 0.895. Without limitation, such panels may be particularly useful for predicting preterm PE, even more specifically in Australasian ancestry patients.

**Table 6I ca**ptures panels of Tables 4A and 4B containing IGFALS and measurement of blood pressure in which AUC for predicting preterm PE at 20 weeks in the combined European and Australasian cohort is equal to or greater than 0.895. Without limitation, such panels may be particularly useful for predicting preterm PE.

**Table 6J** captures panels of Tables 4A and 4B containing IGFALS and measurement of blood pressure in which AUC for predicting term PE at 20 weeks in the European cohort is equal to or greater than 0.745. Without limitation, such panels may be particularly useful for predicting term PE, even more specifically in European ancestry patients.

**Table 6K** captures panels of Tables 4A and 4B containing IGFALS and measurement of blood pressure in which AUC for predicting term PE at 20 weeks in the Australasian cohort is equal to or greater than 0.745. Without limitation, such panels may be particularly useful for predicting term PE, even more specifically in Australasian ancestry patients.

**Table 6L** captures panels of Tables 4A and 4B containing IGFALS and measurement of blood pressure in which AUC for predicting term PE at 20 weeks in the combined European and Australasian cohort is equal to or greater than 0.745. Without limitation, such panels may be particularly useful for predicting term PE.

**Table 6M** captures panels of Table 5Ma containing IGFALS and measurement of blood pressure.

**Table 6N** captures panels of Table 5Na containing IGFALS and measurement of blood pressure.

**Table 6A**

| AJ | AK | AL | AM | AN | AO | AP | AQ | AR | AS |
|---|---|---|---|---|---|---|---|---|---|
| 125 | 6 | 117 | 93.6 | ENG | 114 | 91.2 | *alcoh* | 25 | 20.0 |
| | 5 | 8 | 6.4 | MCAM | 95 | 76.0 | *fihd* | 22 | 17.6 |
| | 4 | 0 | 0.0 | SPINT1 | 86 | 68.8 | *fhpet* | 3 | 2.4 |
| | 3 | 0 | 0.0 | MMRN2 | 39 | 31.2 | *vagbl* | 3 | 2.4 |
| | 2 | 0 | 0.0 | MADAM12 | 29 | 23.2 | *gest* | 2 | 1.6 |
| | 1 | 0 | 0.0 | SEPP1 | 26 | 20.8 | bmi | 1 | 0.8 |
| | | | | PIGF | 21 | 16.8 | *age* | 1 | 0.8 |
| | | | | ROBO4 | 13 | 10.4 | | | |
| | | | | QSOX1 | 9 | 7.2 | | | |
| | | | | LNPEP | 2 | 1.6 | | | |
| | | | | ECM1 | 1 | 0.8 | | | |

**Table 6B**

| AJ | AK | AL | AM | AN | AO | AP | AQ | AR | AS |
|---|---|---|---|---|---|---|---|---|---|
| 10 | 6 | 2 | 20.0 | ADAM12 | 6 | 60.0 | *alcoh* | 6 | 60.0 |
| | 5 | 8 | 80.0 | MMRN2 | 6 | 60.0 | *fhpet* | 1 | 10.0 |
| | 4 | 0 | 0.0 | PIGF | 5 | 50.0 | *vagbl* | 1 | 10.0 |

(continued)

| AJ | AK | AL | AM | AN | AO | AP | AQ | AR | AS |
|---|---|---|---|---|---|---|---|---|---|
|  | 3 | 0 | 0.0 | MCAM | 5 | 50.0 |  |  |  |
|  | 2 | 0 | 0.0 | SEPP1 | 1 | 10.0 |  |  |  |
|  | 1 | 0 | 0.0 | LNPEP | 1 | 10.0 |  |  |  |

**Table 6C**

| AJ | AK | AL | AM | AN | AO | AP | AQ | AR | AS |
|---|---|---|---|---|---|---|---|---|---|
| 1 | 6 | 1 | 100 | MMRN2 | 1 | 100 |  |  |  |
|  | 5 | 0 | 0 | ADAM12 | 1 | 100 |  |  |  |
|  | 4 | 0 | 0 | MCAM | 1 | 100 |  |  |  |
|  | 3 | 0 | 0 | PIGF | 1 | 100 |  |  |  |
|  | 2 | 0 | 0 |  |  |  |  |  |  |
|  | 1 | 0 | 0 |  |  |  |  |  |  |

**Table 6D**

| AJ | AK | AL | AM | AN | AO | AP | AQ | AR | AS |
|---|---|---|---|---|---|---|---|---|---|
| 460 | 6 | 196 | 42.6 | ENG | 229 | 49.8 | *alcoh* | 93 | 20.2 |
|  | 5 | 194 | 42.2 | MCAM | 227 | 49.3 | *fihd* | 25 | 5.4 |
|  | 4 | 68 | 14.8 | MMRN2 | 178 | 38.7 | *vagbl* | 21 | 4.6 |
|  | 3 | 2 | 0.4 | ADAM12 | 161 | 35.0 | bmi | 11 | 2.4 |
|  | 2 | 0 | 0.0 | SPINT1 | 137 | 29.8 | *fhpet* | 10 | 2.2 |
|  | 1 | 0 | 0.0 | PIGF | 134 | 29.1 | *gest* | 1 | 0.2 |
|  |  |  |  | SEPP1 | 81 | 17.6 | *age* | 1 | 0.2 |
|  |  |  |  | QSOX1 | 71 | 15.4 |  |  |  |
|  |  |  |  | ROBO4 | 42 | 9.1 |  |  |  |
|  |  |  |  | LNPEP | 23 | 5.0 |  |  |  |
|  |  |  |  | ALDOA | 16 | 3.5 |  |  |  |
|  |  |  |  | ECM1 | 15 | 3.3 |  |  |  |
|  |  |  |  | MAPRE1/3 | 15 | 3.3 |  |  |  |
|  |  |  |  | ENPP2 | 11 | 2.4 |  |  |  |
|  |  |  |  | PRDX2 | 2 | 0.4 |  |  |  |

**Table 6E**

| AJ | AK | AL | AM | AN | AO | AP | AQ | AR | AS |
|---|---|---|---|---|---|---|---|---|---|
| 31 | 6 | 17 | 54.8 | MCAM | 23 | 74.2 | *alcoh* | 4 | 12.9 |
|  | 5 | 14 | 45.2 | ADAM12 | 20 | 64.5 | *vagbl* | 2 | 6.5 |
|  | 4 |  | 0.0 | MMRN2 | 18 | 58.1 |  |  |  |
|  | 3 |  | 0.0 | PIGF | 17 | 54.8 |  |  |  |

(continued)

| AJ | AK | AL | AM | AN | AO | AP | AQ | AR | AS |
|---|---|---|---|---|---|---|---|---|---|
|  | 2 |  | 0.0 | ENG | 11 | 35.5 |  |  |  |
|  | 1 |  | 0.0 | SPINT1 | 6 | 19.4 |  |  |  |
|  |  |  | 100 | SEPP1 | 4 | 12.9 |  |  |  |
|  |  |  |  | ECM1 | 2 | 6.5 |  |  |  |
|  |  |  |  | LNPEP | 2 | 6.5 |  |  |  |
|  |  |  |  | QSOX1 | 1 | 3.2 |  |  |  |

**Table 6F**

| AJ | AK | AL | AM | AN | AO | AP | AQ | AR | AS |
|---|---|---|---|---|---|---|---|---|---|
| 712 | 6 | 240 | 33.7 | ENG | 326 | 45.8 | *alcoh* | 146 | 20.5 |
|  | 5 | 336 | 47.2 | MCAM | 298 | 41.9 | *fihd* | 67 | 9.4 |
|  | 4 | 122 | 17.1 | ADAM12 | 256 | 36.0 | *vagbl* | 30 | 4.2 |
|  | 3 | 14 | 2.0 | MMRN2 | 244 | 34.3 | bmi | 17 | 2.4 |
|  | 2 | 0 | 0.0 | PIGF | 213 | 29.9 | *fhpet* | 16 | 2.2 |
|  | 1 | 0 | 0.0 | SPINT1 | 196 | 27.5 | *gest* | 3 | 0.4 |
|  |  |  |  | SEPP1 | 118 | 16.6 | *age* | 1 | 0.1 |
|  |  |  |  | QSOX1 | 108 | 15.2 |  |  |  |
|  |  |  |  | ROBO4 | 61 | 8.6 |  |  |  |
|  |  |  |  | LNPEP | 36 | 5.1 |  |  |  |
|  |  |  |  | ALDOA | 26 | 3.7 |  |  |  |
|  |  |  |  | ECM1 | 23 | 3.2 |  |  |  |
|  |  |  |  | ENPP2 | 16 | 2.2 |  |  |  |
|  |  |  |  | PRDX2 | 2 | 0.3 |  |  |  |

**Table 6G**

| AJ | AK | AL | AM | AN | AO | AP | AQ | AR | AS |
|---|---|---|---|---|---|---|---|---|---|
| 16 | 6 | 14 | 87.5 | SPINT1 | 14 | 87.5 | *alcoh* | 5 | 31.3 |
|  | 5 | 2 | 12.5 | ENG | 14 | 87.5 |  |  |  |
|  | 4 | 0 | 0.0 | MMRN2 | 11 | 68.8 |  |  |  |
|  | 3 | 0 | 0.0 | MCAM | 9 | 56.3 |  |  |  |
|  | 2 | 0 | 0.0 | ADAM12 | 6 | 37.5 |  |  |  |
|  | 1 | 0 | 0.0 | ROBO4 | 2 | 12.5 |  |  |  |
|  |  |  |  | PIGF | 1 | 6.3 |  |  |  |

**Table 6H**

| AJ | AK | AL | AM | AN | AO | AP | AQ | AR | AS |
|---|---|---|---|---|---|---|---|---|---|
| 129 | 6 | 110 | 85.3 | ENG | 124 | 96.1 | *alcoh* | 18 | 14.0 |

(continued)

| AJ | AK | AL | AM | AN | AO | AP | AQ | AR | AS |
|---|---|---|---|---|---|---|---|---|---|
|  | 5 | 19 | 14.7 | SPINT1 | 115 | 89.1 | *fihd* | 7 | 5.4 |
|  | 4 | 0 | 0.0 | MCAM | 64 | 49.6 | bmi | 2 | 1.6 |
|  | 3 | 0 | 0.0 | MMRN2 | 57 | 44.2 | *gest* | 1 | 0.8 |
|  | 2 | 0 | 0.0 | ADAM12 | 27 | 20.9 | *age* | 1 | 0.8 |
|  | 1 | 0 | 0.0 | PIGF | 27 | 20.9 |  |  |  |
|  |  |  |  | QSOX1 | 26 | 20.2 |  |  |  |
|  |  |  |  | SEPP1 | 22 | 17.1 |  |  |  |
|  |  |  |  | ROBO4 | 4 | 3.1 |  |  |  |
|  |  |  |  | LNPEP | 2 | 1.6 |  |  |  |

**Table 6I**

| AJ | AK | AL | AM | AN | AO | AP | AQ | AR | AS |
|---|---|---|---|---|---|---|---|---|---|
| 140 | 6 | 115 | 82.1 | SPINT1 | 139 | 99.3 | *alcoh* | 20 | 14.3 |
|  | 5 | 25 | 17.9 | ENG | 132 | 94.3 | *fihd* | 7 | 5.0 |
|  | 4 | 0 | 0,0 | MCAM | 78 | 55.7 | bmi | 4 | 2.9 |
|  | 3 | 0 | 0.0 | MMRN2 | 44 | 31.4 | *gest* | 1 | 0.7 |
|  | 2 | 0 | 0.0 | ADAM12 | 30 | 21.4 | *age* | 1 | 0.7 |
|  | 1 | 0 | 0.0 | PIGF | 26 | 18.6 |  |  |  |
|  |  |  |  | QSOX1 | 23 | 16.4 |  |  |  |
|  |  |  |  | SEPP1 | 20 | 14.3 |  |  |  |
|  |  |  |  | ROBO4 | 8 | 5.7 |  |  |  |
|  |  |  |  | LNPEP | 2 | 1.4 |  |  |  |

**Table 6J**

| AJ | AK | AL | AM | AN | AO | AP | AQ | AR | AS |
|---|---|---|---|---|---|---|---|---|---|
| 56 | 6 | 33 | 58.9 | MCAM | 36 | 64.3 | *alcoh* | 12 | 21.4 |
|  | 5 | 21 | 37.5 | ADAM12 | 30 | 53.6 | bmi | 5 | 8.9 |
|  | 4 | 2 | 3.6 | PIGF | 26 | 46.4 | *vagbl* | 5 | 8.9 |
|  | 3 | 0 | 0.0 | MMRN2 | 26 | 46.4 | *fhpet* | 2 | 3.6 |
|  | 2 | 0 | 0.0 | ENG | 22 | 39.3 | *fihd* | 1 | 1.8 |
|  | 1 | 0 | 0.0 | SEPP1 | 12 | 21.4 |  |  |  |
|  |  |  |  | QSOX1 | 8 | 14.3 |  |  |  |
|  |  |  |  | SPINT1 | 5 | 8.9 |  |  |  |
|  |  |  |  | LNPEP | 4 | 7.1 |  |  |  |
|  |  |  |  | ECM1 | 2 | 3.6 |  |  |  |
|  |  |  |  | ALDOA | 2 | 3.6 |  |  |  |
|  |  |  |  | MAPRE1/3 | 1 | 1.8 |  |  |  |

**Table 6K**

| AJ | AK | AL | AM | AN | AO | AP | AQ | AR | AS |
|---|---|---|---|---|---|---|---|---|---|
| 308 | 6 | 148 | 48.1 | MCAM | 197 | 64.0 | *alcoh* | 75 | 24.4 |
| | 5 | 143 | 46.4 | MADAM12 | 141 | 45.8 | *fihd* | 57 | 18.5 |
| | 4 | 17 | 5.5 | PIGF | 138 | 44.8 | bmi | 13 | 4.2 |
| | 3 | 0 | 0.0 | ENG | 135 | 43.8 | *vagbl* | 9 | 2.9 |
| | 2 | 0 | 0.0 | MMRN2 | 81 | 26.3 | *fhpet* | 6 | 1.9 |
| | 1 | 0 | 0.0 | SPINT1 | 79 | 25.6 | | | |
| | | | | SEPP1 | 38 | 12.3 | | | |
| | | | | QSOX1 | 23 | 7.5 | | | |
| | | | | ROBO4 | 16 | 5.2 | | | |
| | | | | ECM1 | 13 | 4.2 | | | |
| | | | | ALDOA | 12 | 3.9 | | | |
| | | | | LNPEP | 9 | 2.9 | | | |
| | | | | MAPRE1/3 | 9 | 2.9 | | | |
| | | | | ENPP2 | 4 | 1.3 | | | |

**Table 6L**

| AJ | AK | AL | AM | AN | AO | AP | AQ | AR | AS |
|---|---|---|---|---|---|---|---|---|---|
| 463 | 6 | 207 | 44.7 | MCAM | 261 | 56.4 | *alcoh* | 127 | 27.4 |
| | 5 | 207 | 44.7 | ENG | 238 | 51.4 | *fihd* | 49 | 10.6 |
| | 4 | 49 | 10.6 | PIGF | 184 | 39.7 | bmi | 17 | 3.7 |
| | 3 | 0 | 0.0 | ADAM12 | 161 | 34.8 | *vagbl* | 15 | 3.2 |
| | 2 | 0 | 0.0 | MMRN2 | 132 | 28.5 | *fhpet* | 12 | 2.6 |
| | 1 | 0 | 0.0 | SPINT1 | 127 | 27.4 | *gest* | 2 | 0.4 |
| | | | | QSOX1 | 56 | 12.1 | *age* | 1 | 0.2 |
| | | | | SEPP1 | 55 | 11.9 | | | |
| | | | | ROBO4 | 33 | 7.1 | | | |
| | | | | ALDOA | 21 | 4.5 | | | |
| | | | | LNPEP | 20 | 4.3 | | | |
| | | | | MAPRE1/3 | 18 | 3.9 | | | |
| | | | | ECM1 | 13 | 2.8 | | | |
| | | | | ENPP2 | 5 | 1.1 | | | |

**Table 6M**

| AJ | AK | AL | AM | AN | AO | AP | AQ | AR | AS |
|---|---|---|---|---|---|---|---|---|---|
| 44 | 6 | 36 | 81.8 | SPINT1 | 38 | 86.4 | *gest* | 2 | 4.5 |
| | 5 | 8 | 18.2 | ENG | 37 | 84.1 | | | |
| | 4 | 0 | 0.0 | MCAM | 34 | 77.3 | | | |

(continued)

| AJ | AK | AL | AM | AN | AO | AP | AQ | AR | AS |
|---|---|---|---|---|---|---|---|---|---|
|  | 3 | 0 | 0.0 | ADAM12 | 20 | 45.5 |  |  |  |
|  | 2 | 0 | 0.0 | PlGF | 19 | 43.2 |  |  |  |
|  | 1 | 0 | 0.0 | MMRN2 | 18 | 40.9 |  |  |  |

**Table 6N**

| AJ | AK | AL | AM | AN | AO | AP | AQ | AR | AS |
|---|---|---|---|---|---|---|---|---|---|
| 4 | 6 | 3 | 75 | MCAM | 4 | 100 |  |  |  |
|  | 5 | 1 | 25 | PlGF | 3 | 75 |  |  |  |
|  | 4 | 0 | 0 | SPINT1 | 2 | 50 |  |  |  |
|  | 3 | 0 | 0 | ENG | 2 | 50 |  |  |  |
|  | 2 | 0 | 0 | ADAM12 | 2 | 50 |  |  |  |
|  | 1 | 0 | 0 | MMRN2 | 2 | 50 |  |  |  |

**Example 7**

This example relates to panels of Tables 4A and 4B all of which contain PlGF (hence, PlGF, which is always present in these panels, is not listed in the tables).

**Table 7A** captures panels of Tables 4A and 4B containing PlGF in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495. Without limitation, such panels may be particularly useful as rule-in panels, more specifically for predicting PE, even more specifically for predicting PE without distinction between preterm and term PE.

**Table 7B** captures panels of Tables 4A and 4B containing PlGF in which specificity at 99% NPV for predicting all PE (i.e., without distinction between preterm and term PE) at 20 weeks in training set (Australasian cohort) is equal to or greater than 0.395 **and** specificity at 99% NPV for predicting all PE at 20 weeks in testing set (European cohort) is equal to or greater than 0.395. Without limitation, such panels may be particularly useful as rule-out panels, more specifically for predicting PE, even more specifically for predicting PE without distinction between preterm and term PE.

**Table 7C** captures panels of Tables 4A and 4B containing PlGF in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495 **and** specificity at 99% NPV for predicting all PE at 20 weeks in training set (Australasian cohort) is equal to or greater than 0.395 and specificity at 99% NPV for predicting all PE at 20 weeks in testing set (European cohort) is equal to or greater than 0.395. Without limitation, such panels may be particularly useful as rule-in and rule-out panels, more specifically for predicting PE, even more specifically for predicting PE without distinction between preterm and term PE.

**Table 7D** captures panels of Tables 4A and 4B containing PlGF in which AUC for predicting all PE (i.e., without distinction between preterm and term PE) at 20 weeks in training set (Australasian cohort) is equal to or greater than 0.745 **and** AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 7E** captures panels of Tables 4A and 4B containing PlGF in which AUC for predicting all PE (i.e., without distinction between preterm and term PE) at 20 weeks in training set (Australasian cohort) is equal to or greater than 0.775 **and** AUC for predicting all PE at 20 weeks in testing set (European cohort) is equal to or greater than 0.775. Without limitation, such panels may be particularly useful for predicting PE, even more specifically for predicting PE without distinction between preterm and term PE.

**Table 7F** captures panels of Tables 4A and 4B containing PlGF in which AUC for predicting preterm PE at 20 weeks in the European cohort **or** in the Australasian cohort or in the combined European and Australasian cohort is equal to or greater than 0.745. Without limitation, such panels may be particularly useful for predicting preterm PE.

**Table 7G** captures panels of Tables 4A and 4B containing PIGF in which AUC for predicting preterm PE at 20 weeks in the European cohort is equal to or greater than 0.895. Without limitation, such panels may be particularly useful for predicting preterm PE, even more specifically in European ancestry patients.

**Table 7H** captures panels of Tables 4A and 4B containing PIGF in which AUC for predicting preterm PE at 20 weeks in the Australasian cohort is equal to or greater than 0.895. Without limitation, such panels may be particularly useful for predicting preterm PE, even more specifically in Australasian ancestry patients.

**Table 7I** captures panels of Tables 4A and 4B containing PIGF in which AUC for predicting preterm PE at 20 weeks in the combined European and Australasian cohort is equal to or greater than 0.895. Without limitation, such panels may be particularly useful for predicting preterm PE.

**Table 7J** captures panels of Tables 4A and 4B containing PIGF in which AUC for predicting term PE at 20 weeks in the European cohort is equal to or greater than 0.745. Without limitation, such panels may be particularly useful for predicting term PE, even more specifically in European ancestry patients.

**Table 7K** captures panels of Tables 4A and 4B containing PIGF in which AUC for predicting term PE at 20 weeks in the Australasian cohort is equal to or greater than 0.745. Without limitation, such panels may be particularly useful for predicting term PE, even more specifically in Australasian ancestry patients.

**Table 7L** captures panels of Tables 4A and 4B containing PIGF in which AUC for predicting term PE at 20 weeks in the combined European and Australasian cohort is equal to or greater than 0.745. Without limitation, such panels may be particularly useful for predicting term PE.

**Table 7M** captures panels of Table 5Ma containing PIGF.

**Table 7N** captures panels of Table 5Na containing PIGF.

**Table 7A**

| AJ | AK | AL | AM | AN | AO | AP | AQ | AR | AS |
|---|---|---|---|---|---|---|---|---|---|
| 45 | 6 | 41 | 91.1 | MCAM | 35 | 77.8 | BP15 | 19 | 42.2 |
| | 5 | 4 | 8.9 | ENG | 30 | 66.7 | BP20 | 18 | 40.0 |
| | 4 | | 0.0 | IGFALS | 26 | 57.8 | *alcoh* | 6 | 13.3 |
| | 3 | | 0.0 | SPINT1 | 26 | 57.8 | bmi | 6 | 13.3 |
| | 2 | | 0.0 | ADAM12 | 18 | 40.0 | *fihd* | 3 | 6.7 |
| | 1 | | 0.0 | ECM1 | 11 | 24.4 | *gest* | 2 | 4.4 |
| | | | | MMRN2 | 7 | 15.6 | *fhpet* | 1 | 2.2 |
| | | | | SEPP1 | 5 | 11.1 | | | |
| | | | | LNPEP | 4 | 8.9 | | | |
| | | | | PRCP | 2 | 4.4 | | | |
| | | | | QSOX1 | 1 | 2.2 | | | |
| | | | | LCAT | 1 | 2.2 | | | |

**Table 7B**

| AJ | AK | AL | AM | AN | AO | AP | AQ | AR | AS |
|---|---|---|---|---|---|---|---|---|---|
| 8 | 6 | 4 | 50.0 | IGFALS | 8 | 100.0 | BP15 | 3 | 37.5 |
| | 5 | 3 | 37.5 | ADAM12 | 7 | 87.5 | BP20 | 2 | 25.0 |
| | 4 | 1 | 12.5 | MCAM | 5 | 62.5 | bmi | 2 | 25.0 |
| | 3 | | 0.0 | MMRN2 | 2 | 25.0 | pbwgt | 2 | 25.0 |
| | 2 | | 0.0 | QSOX1 | 1 | 12.5 | *alcoh* | 1 | 12.5 |
| | 1 | | 0.0 | LNPEP | 1 | 12.5 | *fhpet* | 1 | 12.5 |

**Table 7C**

| AJ | AK | AL | AM | AN | AO | AP | AQ | AR | AS |
|----|----|----|-----|--------|----|-----|------|----|-----|
| 1 | 6 | 1 | 100 | MMRN2 | 1 | 100 | BP15 | 1 | 100 |
| | 5 | | 0 | ADAM12 | 1 | 100 | | | |
| | 4 | | 0 | IGFALS | 1 | 100 | | | |
| | 3 | | 0 | MCAM | 1 | 100 | | | |
| | 2 | | 0 | | | | | | |
| | 1 | | 0 | | | | | | |

**Table 7D**

| AJ | AK | AL | AM | AN | AO | AP | AQ | AR | AS |
|-----|----|----|------|----------|-----|------|-------|-----|------|
| 212 | 6 | 76 | 35.8 | IGFALS | 158 | 74.5 | BP20 | 104 | 49.1 |
| | 5 | 97 | 45.8 | ADAM12 | 116 | 54.7 | BP15 | 82 | 38.7 |
| | 4 | 37 | 17.5 | MCAM | 110 | 51.9 | *alcoh* | 23 | 10.8 |
| | 3 | 2 | 0.9 | ENG | 44 | 20.8 | bmi | 17 | 8.0 |
| | 2 | | 0.0 | MMRN2 | 43 | 20.3 | *fhpet* | 9 | 4.2 |
| | 1 | | 0.0 | SPINT1 | 40 | 18.9 | *fihd* | 6 | 2.8 |
| | | | | ECM1 | 33 | 15.6 | pbwgt | 3 | 1.4 |
| | | | | SEPP1 | 30 | 14.2 | *vagbl* | | |
| | | | | LNPEP | 20 | 9.4 | | | |
| | | | | ALDOA | 10 | 4.7 | | | |
| | | | | ROBO4 | 9 | 4.2 | | | |
| | | | | QSOX1 | 8 | 3.8 | | | |
| | | | | ENPP2 | 8 | 3.8 | | | |
| | | | | MAPRE1/3 | 8 | 3.8 | | | |
| | | | | LCAT | 1 | 0.5 | | | |
| | | | | PRCP | 1 | 0.5 | | | |

**Table 7E**

| AJ | AK | AL | AM | AN | AO | AP | AQ | AR | AS |
|----|----|----|------|--------|----|-----|-------|----|------|
| 18 | 6 | 5 | 27.8 | IGFALS | 18 | 100 | BP20 | 10 | 55.6 |
| | 5 | 13 | 72.2 | ADAM12 | 16 | 88.9 | BP15 | 7 | 38.9 |
| | 4 | | 0.0 | MCAM | 11 | 61.1 | *alcoh* | 2 | 11.1 |
| | 3 | | 0.0 | MMRN2 | 8 | 44.4 | | | |
| | 2 | | 0.0 | SEPP1 | 3 | 16.7 | | | |
| | 1 | | 0.0 | LNPEP | 2 | 11.1 | | | |

**Table 7F**

| AJ | AK | AL | AM | AN | AO | AP | AQ | AR | AS |
|---|---|---|---|---|---|---|---|---|---|
| 362 | 6 | 101 | 27.9 | IGFALS | 262 | 72.4 | BP15 | 172 | 47.5 |
| | 5 | 171 | 47.2 | ADAM12 | 192 | 53.0 | BP20 | 134 | 37.0 |
| | 4 | 80 | 22.1 | MCAM | 171 | 47.2 | *alcoh* | 41 | 11.3 |
| | 3 | 10 | 2.8 | ENG | 75 | 20.7 | *fihd* | 26 | 7.2 |
| | 2 | | 0.0 | SPINT1 | 69 | 19.1 | bmi | 25 | 6.9 |
| | 1 | | 0.0 | MMRN2 | 61 | 16.9 | *fhpet* | 14 | 3.9 |
| | | | | ECM1 | 53 | 14.6 | pbwgt | 5 | 1.4 |
| | | | | SEPP1 | 42 | 11.6 | *gest* | 3 | 0.8 |
| | | | | LNPEP | 29 | 8.0 | | | |
| | | | | ALDOA | 22 | 6.1 | | | |
| | | | | ROBO4 | 15 | 4.1 | | | |
| | | | | MAPRE1/3 | 14 | 3.9 | | | |
| | | | | SOX1 | 11 | 3.0 | | | |
| | | | | ENPP2 | 9 | 2.5 | | | |
| | | | | LCAT | 2 | 0.6 | | | |
| | | | | PRCP | 2 | 0.6 | | | |

**Table 7G**

| AJ | AK | AL | AM | AN | AO | AP | AQ | AR | AS |
|---|---|---|---|---|---|---|---|---|---|
| 16 | 6 | 9 | 56.3 | MCAM | 11 | 68.8 | BP20 | 10 | 62.5 |
| | 5 | 7 | 43.8 | ADAM12 | 10 | 62.5 | *alcoh* | 2 | 12.5 |
| | 4 | | 0.0 | ECM1 | 9 | 56.3 | | | |
| | 3 | | 0.0 | SPINT1 | 8 | 50.0 | | | |
| | 2 | | 0.0 | IGFALS | 7 | 43.8 | | | |
| | 1 | | 0.0 | MMRN2 | 7 | 43.8 | | | |
| | | | | ENG | 6 | 37.5 | | | |
| | | | | PRCP | 2 | 12.5 | | | |
| | | | | LNPEP | 1 | 6.3 | | | |

**Table 7H**

| AJ | AK | AL | AM | AN | AO | AP | AQ | AR | AS |
|---|---|---|---|---|---|---|---|---|---|
| 35 | 6 | 34 | 97.1 | IGFALS | 34 | 97.1 | BP15 | 21 | 60.0 |
| | 5 | 1 | 2.9 | ENG | 30 | 85.7 | BP20 | 7 | 20.0 |
| | 4 | | 0.0 | SPINT1 | 30 | 85.7 | *alcoh* | 1 | 2.9 |
| | 3 | | 0.0 | MCAM | 23 | 65.7 | *fhpet* | 1 | 2.9 |
| | 2 | | 0.0 | ADAM12 | 11 | 31.4 | | | |
| | 1 | | 0.0 | MMRN2 | 10 | 28.6 | | | |

(continued)

| AJ | AK | AL | AM | AN | AO | AP | AQ | AR | AS |
|----|----|----|----|----|----|----|----|----|----|
|    |    |    |    | SEPP1 | 6 | 17.1 |    |    |    |

**Table 7I**

| AJ | AK | AL | AM | AN | AO | AP | AQ | AR | AS |
|----|----|----|----|----|----|----|----|----|----|
| 41 | 6 | 37 | 90.2 | SPINT1 | 41 | 100.0 | BP15 | 19 | 46.3 |
|    | 5 | 4 | 9.8 | IGFALS | 37 | 90.2 | BP20 | 10 | 24.4 |
|    | 4 |   | 0.0 | ENG | 32 | 78.0 | bmi | 4 | 9.8 |
|    | 3 |   | 0.0 | MCAM | 25 | 61.0 | *alcoh* | 2 | 4.9 |
|    | 2 |   | 0.0 | ADAM12 | 18 | 43.9 | *fhpet* | 1 | 2.4 |
|    | 1 |   | 0.0 | SEPP1 | 6 | 14.6 | *gest* | 1 | 2.4 |
|    |   |   |   | MMRN2 | 5 | 12.2 |    |    |    |

**Table 7J**

| AJ | AK | AL | AM | AN | AO | AP | AQ | AR | AS |
|----|----|----|----|----|----|----|----|----|----|
| 27 | 6 | 10 | 37.0 | IGFALS | 27 | 100.0 | BP20 | 15 | 55.6 |
|    | 5 | 16 | 59.3 | ADAM12 | 22 | 81.5 | BP15 | 11 | 40.7 |
|    | 4 | 1 | 3.7 | MCAM | 12 | 44.4 | *alcoh* | 3 | 11.1 |
|    | 3 |   | 0.0 | MMRN2 | 9 | 33.3 | bmi | 1 | 3.7 |
|    | 2 |   | 0.0 | SEPP1 | 8 | 29.6 | *fhpet* | 1 | 3.7 |
|    | 1 |   | 0.0 | LNPEP | 3 | 11.1 | *fihd* | 1 | 3.7 |
|    |   |   |   | QSOX1 | 2 | 7.4 |    |    |    |
|    |   |   |   | ENG | 1 | 3.7 |    |    |    |
|    |   |   |   | ALDOA | 1 | 3.7 |    |    |    |

**Table 7K**

| AJ | AK | AL | AM | AN | AO | AP | AQ | AR | AS |
|----|----|----|----|----|----|----|----|----|----|
| 211 | 6 | 73 | 34.6 | IGFALS | 167 | 79.1 | BP15 | 128 | 60.7 |
|    | 5 | 122 | 57.8 | ADAM12 | 142 | 67.3 | BP20 | 47 | 22.3 |
|    | 4 | 16 | 7.6 | MCAM | 117 | 55.5 | *alcoh* | 27 | 12.8 |
|    | 3 |   | 0.0 | ENG | 43 | 20.4 | bmi | 23 | 10.9 |
|    | 2 |   | 0.0 | SPINT1 | 39 | 18.5 | *fihd* | 21 | 10.0 |
|    | 1 |   | 0.0 | MMRN2 | 35 | 16.6 | *fhpet* | 6 | 2.8 |
|    |   |   |   | ECM1 | 26 | 12.3 | pbwgt | 5 | 2.4 |
|    |   |   |   | SEPP1 | 21 | 10.0 | *gest* | 1 | 0.5 |
|    |   |   |   | ALDOA | 15 | 7.1 |    |    |    |
|    |   |   |   | LNPEP | 10 | 4.7 |    |    |    |
|    |   |   |   | MAPRE1/3 | 9 | 4.3 |    |    |    |

(continued)

| AJ | AK | AL | AM | AN | AO | AP | AQ | AR | AS |
|---|---|---|---|---|---|---|---|---|---|
| | | | | QSOX1 | 8 | 3.8 | | | |
| | | | | ENPP2 | 7 | 3.3 | | | |
| | | | | ROBO4 | 3 | 1.4 | | | |
| | | | | LCAT | 1 | 0.5 | | | |

**Table 7L**

| AJ | AK | AL | AM | AN | AO | AP | AQ | AR | AS |
|---|---|---|---|---|---|---|---|---|---|
| 250 | 6 | 83 | 33.2 | IGFALS | 212 | 84.8 | BP15 | 137 | 54.8 |
| | 5 | 128 | 51.2 | ADAM12 | 134 | 53.6 | BP20 | 79 | 31.6 |
| | 4 | 39 | 15.6 | MCAM | 114 | 45.6 | *alcoh* | 31 | 12.4 |
| | 3 | | 0.0 | ENG | 62 | 24.8 | bmi | 22 | 8.8 |
| | 2 | | 0.0 | MMRN2 | 51 | 20.4 | *fihd* | 19 | 7.6 |
| | 1 | | 0.0 | SPINT1 | 47 | 18.8 | *fhpet* | 9 | 3.6 |
| | | | | SEPP1 | 30 | 12.0 | pbwgt | 5 | 2.0 |
| | | | | ECM1 | 23 | 9.2 | *gest* | 3 | 1.2 |
| | | | | ALDOA | 17 | 6.8 | | | |
| | | | | LNPEP | 16 | 6.4 | | | |
| | | | | MAPRE1/3 | 11 | 4.4 | | | |
| | | | | QSOX1 | 8 | 3.2 | | | |
| | | | | ROBO4 | 8 | 3.2 | | | |
| | | | | ENPP2 | 6 | 2.4 | | | |

**Table 7M**

| AJ | AK | AL | AM | AN | AO | AP | AQ | AR | AS |
|---|---|---|---|---|---|---|---|---|---|
| 23 | 6 | 19 | 82.6 | IGFALS | 23 | | BP15 | 16 | |
| | 5 | 3 | 13.0 | SPINT1 | 18 | | BP20 | 3 | |
| | 4 | 1 | 4.3 | MCAM | 18 | | *gest* | 2 | |
| | 3 | | 0.0 | ENG | 15 | | | | |
| | 2 | | 0.0 | ADAM12 | 10 | | | | |
| | 1 | | 0.0 | MMRN2 | 5 | | | | |

**Table 7N**

| AJ | AK | AL | AM | AN | AO | AP | AQ | AR | AS |
|---|---|---|---|---|---|---|---|---|---|
| 4 | 6 | 2 | 50 | IGFALS | 4 | 100 | BP20 | 2 | 50 |
| | 5 | 1 | 25 | MCAM | 3 | 75 | BP15 | 1 | 25 |
| | 4 | 1 | 25 | ADAM12 | 3 | 75 | | | |
| | 3 | | 0 | MMRN2 | 2 | 50 | | | |

(continued)

| AJ | AK | AL | AM | AN | AO | AP | AQ | AR | AS |
|---|---|---|---|---|---|---|---|---|---|
| | 2 | | 0 | SPINT1 | 1 | 25 | | | |
| | 1 | | 0 | ENG | 1 | 25 | | | |

**Example 8**

This example relates to panels of Tables 4A and 4B all of which contain ENG (hence, ENG, which is always present in these panels, is not listed in the tables).

**Table 8A** captures panels of Tables 4A and 4B containing ENG in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495. Without limitation, such panels may be particularly useful as rule-in panels, more specifically for predicting PE, even more specifically for predicting PE without distinction between preterm and term PE.

**Table 8B** captures panels of Tables 4A and 4B containing ENG in which AUC for predicting all PE (i.e., without distinction between preterm and term PE) at 20 weeks in training set (Australasian cohort) is equal to or greater than 0.745 **and** AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 8C** captures panels of Tables 4A and 4B containing ENG in which AUC for predicting all PE (i.e., without distinction between preterm and term PE) at 20 weeks in training set (Australasian cohort) is equal to or greater than 0.775 **and** AUC for predicting all PE at 20 weeks in testing set (European cohort) is equal to or greater than 0.775. Without limitation, such panels may be particularly useful for predicting PE, even more specifically for predicting PE without distinction between preterm and term PE.

**Table 8D** captures panels of Tables 4A and 4B containing ENG in which AUC for predicting preterm PE at 20 weeks in the European cohort **or** in the Australasian cohort or in the combined European and Australasian cohort is equal to or greater than 0.745. Without limitation, such panels may be particularly useful for predicting preterm PE.

**Table 8E** captures panels of Tables 4A and 4B containing ENG in which AUC for predicting preterm PE at 20 weeks in the European cohort is equal to or greater than 0.895. Without limitation, such panels may be particularly useful for predicting preterm PE, even more specifically in European ancestry patients.

**Table 8F** captures panels of Tables 4A and 4B containing ENG in which AUC for predicting preterm PE at 20 weeks in the Australasian cohort is equal to or greater than 0.895. Without limitation, such panels may be particularly useful for predicting preterm PE, even more specifically in Australasian ancestry patients.

**Table 8G** captures panels of Tables 4A and 4B containing ENG in which AUC for predicting preterm PE at 20 weeks in the combined European and Australasian cohort is equal to or greater than 0.895. Without limitation, such panels may be particularly useful for predicting preterm PE.

**Table 8H** captures panels of Tables 4A and 4B containing ENG in which AUC for predicting term PE at 20 weeks in the European cohort is equal to or greater than 0.745. Without limitation, such panels may be particularly useful for predicting term PE, even more specifically in European ancestry patients.

**Table 8I** captures panels of Tables 4A and 4B containing ENG in which AUC for predicting term PE at 20 weeks in the Australasian cohort is equal to or greater than 0.745. Without limitation, such panels may be particularly useful for predicting term PE, even more specifically in Australasian ancestry patients.

**Table 8J** captures panels of Tables 4A and 4B containing ENG in which AUC for predicting term PE at 20 weeks in the combined European and Australasian cohort is equal to or greater than 0.745. Without limitation, such panels may be particularly useful for predicting term PE.

**Table 8K** captures panels of Table 5Ma containing ENG.

**Table 8L** captures panels of Table 5Na containing ENG.

**Table 8A**

| AJ | AK | AL | AM | AN | AO | AP | AQ | AR | AS |
|---|---|---|---|---|---|---|---|---|---|
| 163 | 6 | 152 | 93.3 | IGFALS | 137 | 84.0 | BP15 | 86 | 52.8 |
| | 5 | 11 | 6.7 | MCAM | 127 | 77.9 | BP20 | 49 | 30.1 |
| | 4 | | 0.0 | SPINT1 | 115 | 70.6 | *fihd* | 31 | 19.0 |

(continued)

| AJ | AK | AL | AM | AN | AO | AP | AQ | AR | AS |
|---|---|---|---|---|---|---|---|---|---|
| | 3 | | 0.0 | MMRN2 | 54 | 33.1 | *alcoh* | 29 | 17.8 |
| | 2 | | 0.0 | ADAM12 | 44 | 27.0 | bmi | 19 | 11.7 |
| | 1 | | 0.0 | PIGF | 30 | 18.4 | *fhpet* | 6 | 3.7 |
| | | | | SEPP1 | 28 | 17.2 | *vagbl* | 6 | 3.7 |
| | | | | QSOX1 | 16 | 9.8 | *gest* | 3 | 1.8 |
| | | | | ROBO4 | 12 | 7.4 | *age* | 1 | 0.6 |
| | | | | ECM1 | 7 | 4.3 | | | |
| | | | | LNPEP | 2 | 1.2 | | | |
| | | | | LCAT | 1 | 0.6 | | | |
| | | | | ENPP2 | 1 | 0.6 | | | |

**Table 8B**

| AJ | AK | AL | AM | AN | AO | AP | AQ | AR | AS |
|---|---|---|---|---|---|---|---|---|---|
| 313 | 6 | 219 | 70.0 | IGFALS | 262 | 83.7 | BP20 | 167 | 53.4 |
| | 5 | 90 | 28.8 | MCAM | 233 | 74.4 | BP15 | 105 | 33.5 |
| | 4 | 4 | 1.3 | SPINT1 | 190 | 60.7 | bmi | 51 | 16.3 |
| | 3 | | 0.0 | MMRN2 | 113 | 36.1 | *alcoh* | 44 | 14.1 |
| | 2 | | 0.0 | ADAM12 | 64 | 20.4 | *fihd* | 14 | 4.5 |
| | 1 | | 0.0 | QSOX1 | 64 | 20.4 | *vagbl* | 12 | 3.8 |
| | | | | PIGF | 44 | 14.1 | *fhpet* | 11 | 3.5 |
| | | | | SEPP1 | 41 | 13.1 | *gest* | 3 | 1.0 |
| | | | | ECM1 | 19 | 6.1 | *age* | 1 | 0.3 |
| | | | | ROBO4 | 19 | 6.1 | mothpet | 1 | 0.3 |
| | | | | ENPP2 | 5 | 1.6 | | | |
| | | | | LNPEP | 4 | 1.3 | | | |

**Table 8C**

| AJ | AK | AL | AM | AN | AO | AP | AQ | AR | AS |
|---|---|---|---|---|---|---|---|---|---|
| 12 | 6 | 12 | 100 | IGFALS | 12 | 100 | BP20 | 10 | 83.3 |
| | 5 | | 0 | MCAM | 12 | 100 | *vagbl* | 2 | 16.7 |
| | 4 | | 0 | MMRN2 | 9 | 75 | BP15 | 1 | 8.3 |
| | 3 | | 0 | SPINT1 | 7 | 58.3 | *alcoh* | 1 | 8.3 |
| | 2 | | 0 | ADAM12 | 2 | 16.7 | bmi | 1 | 8.3 |
| | 1 | | 0 | ECM1 | 1 | 8.3 | | | |
| | | | | QSOX1 | 1 | 8.3 | | | |
| | | | | SEPP1 | 1 | 8.3 | | | |

**Table 8D**

| AJ | AK | AL | AM | AN | AO | AP | AQ | AR | AS |
|---|---|---|---|---|---|---|---|---|---|
| 473 | 6 | 290 | 61.3 | IGFALS | 399 | 84.4 | BP20 | 208 | 44.0 |
|  | 5 | 175 | 37.0 | MCAM | 312 | 66.0 | BP15 | 180 | 38.1 |
|  | 4 | 8 | 1.7 | SPINT1 | 268 | 56.7 | bmi | 82 | 17.3 |
|  | 3 |  | 0.0 | MMRN2 | 166 | 35.1 | *alcoh* | 66 | 14.0 |
|  | 2 |  | 0.0 | ADAM12 | 116 | 24.5 | *fihd* | 40 | 8.5 |
|  | 1 |  | 0.0 | QSOX1 | 94 | 19.9 | *fhpet* | 17 | 3.6 |
|  |  |  |  | PlGF | 75 | 15.9 | *vagbl* | 16 | 3.4 |
|  |  |  |  | SEPP1 | 61 | 12.9 | *gest* | 7 | 1.5 |
|  |  |  |  | ROBO4 | 27 | 5.7 | mothpet | 2 | 0.4 |
|  |  |  |  | ECM1 | 24 | 5.1 | pbwgt | 1 | 0.2 |
|  |  |  |  | LNPEP | 6 | 1.3 | *age* | 1 | 0.2 |
|  |  |  |  | ENPP2 | 5 | 1.1 |  |  |  |
|  |  |  |  | LCAT | 1 | 0.2 |  |  |  |

**Table 8E**

| AJ | AK | AL | AM | AN | AO | AP | AQ | AR | AS |
|---|---|---|---|---|---|---|---|---|---|
| 34 | 6 | 32 | 94.1 | SPINT1 | 34 | 100.0 | BP20 | 18 | 52.9 |
|  | 5 | 2 | 5.9 | IGFALS | 28 | 82.4 | *alcoh* | 8 | 23.5 |
|  | 4 |  | 0.0 | MMRN2 | 24 | 70.6 | BP15 | 2 | 5.9 |
|  | 3 |  | 0.0 | MCAM | 23 | 67.6 | bmi | 2 | 5.9 |
|  | 2 |  | 0.0 | ADAM12 | 11 | 32.4 | *vagbl* | 1 | 2.9 |
|  | 1 |  | 0.0 | PlGF | 6 | 17.6 | *gest* | 1 | 2.9 |
|  |  |  |  | ECM1 | 5 | 14.7 |  |  |  |
|  |  |  |  | QSOX1 | 2 | 5.9 |  |  |  |
|  |  |  |  | ROBO4 | 2 | 5.9 |  |  |  |
|  |  |  |  | ENPP2 | 1 | 2.9 |  |  |  |

**Table 8F**

| AJ | AK | AL | AM | AN | AO | AP | AQ | AR | AS |
|---|---|---|---|---|---|---|---|---|---|
| 156 | 6 | 128 | 82.1 | IGFALS | 151 | 96.8 | BP15 | 80 | 51.3 |
|  | 5 | 28 | 17.9 | SPINT1 | 142 | 91.0 | BP20 | 49 | 31.4 |
|  | 4 |  | 0.0 | MCAM | 77 | 49.4 | *alcoh* | 21 | 13.5 |
|  | 3 |  | 0.0 | MMRN2 | 75 | 48.1 | *fihd* | 9 | 5.8 |
|  | 2 |  | 0.0 | ADAM12 | 39 | 25.0 | bmi | 6 | 3.8 |
|  | 1 |  | 0.0 | QSOX1 | 35 | 22.4 | *gest* | 4 | 2.6 |
|  |  |  |  | PlGF | 30 | 19.2 | *fhpet* | 2 | 1.3 |
|  |  |  |  | SEPP1 | 25 | 16.0 | *age* | 1 | 0.6 |

(continued)

| AJ | AK | AL | AM | AN | AO | AP | AQ | AR | AS |
|---|---|---|---|---|---|---|---|---|---|
|  |  |  |  | ROBO4 | 4 | 2.6 |  |  |  |
|  |  |  |  | LNPEP | 2 | 1.3 |  |  |  |

**Table 8G**

| AJ | AK | AL | AM | AN | AO | AP | AQ | AR | AS |
|---|---|---|---|---|---|---|---|---|---|
| 201 | 6 | 155 | 77.1 | SPINT1 | 200 | 99.5 | BP15 | 87 | 43.3 |
|  | 5 | 45 | 22.4 | IGFALS | 184 | 91.5 | BP20 | 58 | 28.9 |
|  | 4 | 1 | 0.5 | MCAM | 117 | 58.2 | bmi | 28 | 13.9 |
|  | 3 |  | 0.0 | MMRN2 | 68 | 33.8 | *alcoh* | 26 | 12.9 |
|  | 2 |  | 0.0 | ADAM12 | 52 | 25.9 | *fihd* | 12 | 6.0 |
|  | 1 |  | 0.0 | QSOX1 | 37 | 18.4 | *fhpet* | 8 | 4.0 |
|  |  |  |  | PIGF | 32 | 15.9 | *gest* | 5 | 2.5 |
|  |  |  |  | SEPP1 | 26 | 12.9 | *vagbl* | 1 | 0.5 |
|  |  |  |  | ROBO4 | 10 | 5.0 | *age* | 1 | 0.5 |
|  |  |  |  | LNPEP | 3 | 1.5 |  |  |  |
|  |  |  |  | ENPP2 | 3 | 1.5 |  |  |  |

**Table 8H**

| AJ | AK | AL | AM | AN | AO | AP | AQ | AR | AS |
|---|---|---|---|---|---|---|---|---|---|
| 27 | 6 | 27 | 100.0 | MCAM | 27 | 100.0 | BP20 | 22 | 81.5 |
|  | 5 |  | 0.0 | IGFALS | 24 | 88.9 | bmi | 9 | 33.3 |
|  | 4 |  | 0.0 | MMRN2 | 11 | 40.7 | *alcoh* | 8 | 29.6 |
|  | 3 |  | 0.0 | ADAM12 | 9 | 33.3 | BP15 | 3 | 11.1 |
|  | 2 |  | 0.0 | QSOX1 | 8 | 29.6 | *vagbl* | 3 | 11.1 |
|  | 1 |  | 0.0 | SPINT1 | 6 | 22.2 | *fhpet* | 1 | 3.7 |
|  |  |  |  | ECM1 | 2 | 7.4 |  |  |  |
|  |  |  |  | PIGF | 1 | 3.7 |  |  |  |
|  |  |  |  | SEPP1 | 1 | 3.7 |  |  |  |

**Table 8I**

| AJ | AK | AL | AM | AN | AO | AP | AQ | AR | AS |
|---|---|---|---|---|---|---|---|---|---|
| 200 | 6 | 164 | 82.0 | MCAM | 171 | 85.5 | BP15 | 108 | 54.0 |
|  | 5 | 36 | 18.0 | IGFALS | 164 | 82.0 | bmi | 66 | 33.0 |
|  | 4 |  | 0.0 | SPINT1 | 91 | 45.5 | BP20 | 51 | 25.5 |
|  | 3 |  | 0.0 | ADAM12 | 70 | 35.0 | *alcoh* | 31 | 15.5 |
|  | 2 |  | 0.0 | MMRN2 | 59 | 29.5 | *fihd* | 28 | 14.0 |
|  | 1 |  | 0.0 | PIGF | 43 | 21.5 | *fhpet* | 13 | 6.5 |

(continued)

| AJ | AK | AL | AM | AN | AO | AP | AQ | AR | AS |
|----|----|----|----|----|----|----|----|----|----|
| | | | | QSOX1 | 24 | 12.0 | *vagbl* | 7 | 3.5 |
| | | | | SEPP1 | 20 | 10.0 | pbwgt | 1 | 0.5 |
| | | | | ROBO4 | 11 | 5.5 | *gest* | 1 | 0.5 |
| | | | | ECM1 | 3 | 1.5 | mothpest | 1 | 0.5 |
| | | | | LNPEP | 1 | 0.5 | | | |

**Table 8J**

| AJ | AK | AL | AM | AN | AO | AP | AQ | AR | AS |
|----|----|----|----|----|----|----|----|----|----|
| 318 | 6 | 229 | 72.0 | IGFALS | 275 | 86.5 | BP15 | 142 | 44.7 |
| | 5 | 87 | 27.4 | MCAM | 250 | 78.6 | BP20 | 127 | 39.9 |
| | 4 | 2 | 0.6 | SPINT1 | 158 | 49.7 | bmi | 80 | 25.2 |
| | 3 | | 0.0 | MMRN2 | 94 | 29.6 | *alcoh* | 54 | 17.0 |
| | 2 | | 0.0 | ADAM12 | 77 | 24.2 | *fihd* | 27 | 8.5 |
| | 1 | | 0.0 | PIGF | 62 | 19.5 | *fhpet* | 15 | 4.7 |
| | | | | QSOX1 | 51 | 16.0 | *vagbl* | 11 | 3.5 |
| | | | | SEPP1 | 35 | 11.0 | *gest* | 3 | 0.9 |
| | | | | ROBO4 | 22 | 6.9 | mothpet | 2 | 0.6 |
| | | | | ECM1 | 7 | 2.2 | pbwgt | 1 | 0.3 |
| | | | | LNPEP | 4 | 1.3 | *age* | 1 | 0.3 |
| | | | | ENPP2 | 1 | 0.3 | | | |

**Table 8K**

| AJ | AK | AL | AM | AN | AO | AP | AQ | AR | AS |
|----|----|----|----|----|----|----|----|----|----|
| 42 | 6 | 34 | 81.0 | IGFALS | 42 | 100 | BP15 | 31 | 73.8 |
| | 5 | 8 | 19.0 | SPINT1 | 39 | 92.9 | BP20 | 6 | 14.3 |
| | 4 | | 0.0 | MCAM | 32 | 76.2 | *gest* | 3 | 7.1 |
| | 3 | | 0.0 | MMRN2 | 20 | 47.6 | | | |
| | 2 | | 0.0 | PIGF | 15 | 35.7 | | | |
| | 1 | | 0.0 | ADAM12 | 14 | 33.3 | | | |

**Table 8L**

| AJ | AK | AL | AM | AN | AO | AP | AQ | AR | AS |
|----|----|----|----|----|----|----|----|----|----|
| 2 | 6 | 2 | 100 | IGFALS | 2 | 100 | BP20 | 2 | 100 |
| | 5 | | 0 | SPINT1 | 2 | 100 | | | |
| | 4 | | 0 | MCAM | 2 | 100 | | | |
| | 3 | | 0 | PIGF | 1 | 50 | | | |
| | 2 | | 0 | MMRN2 | 1 | 50 | | | |

(continued)

| AJ | AK | AL | AM | AN | AO | AP | AQ | AR | AS |
|----|----|----|----|----|----|----|----|----|----|
|    | 1  |    | 0  |    |    |    |    |    |    |

## Example 9

This example relates to panels of Tables 4A and 4B all of which contain ADAM12 (hence, ADAM12, which is always present in these panels, is not listed in the tables).

**Table 9A** captures panels of Tables 4A and 4B containing ADAM12 in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495. Without limitation, such panels may be particularly useful as rule-in panels, more specifically for predicting PE, even more specifically for predicting PE without distinction between preterm and term PE.

**Table 9B** captures panels of Tables 4A and 4B containing ADAM12 in which specificity at 99% NPV for predicting all PE (i.e., without distinction between preterm and term PE) at 20 weeks in training set (Australasian cohort) is equal to or greater than 0.395 **and** specificity at 99% NPV for predicting all PE at 20 weeks in testing set (European cohort) is equal to or greater than 0.395. Without limitation, such panels may be particularly useful as rule-out panels, more specifically for predicting PE, even more specifically for predicting PE without distinction between preterm and term PE.

**Table 9C** captures panels of Tables 4A and 4B containing ADAM12 in which sensitivity at 20% PPV for predicting all PE (i.e., without distinction between preterm and term PE) at 20 weeks in training set (Australasian cohort) is equal to or greater than 0.495 **and** sensitivity at 20% PPV for predicting all PE at 20 weeks in testing set (European cohort) is equal to or greater than 0.495 and specificity at 99% NPV for predicting all PE at 20 weeks in training set (Australasian cohort) is equal to or greater than 0.395 **and** specificity at 99% NPV for predicting all PE at 20 weeks in testing set (European cohort) is equal to or greater than 0.395. Without limitation, such panels may be particularly useful as rule-in and rule-out panels, more specifically for predicting PE, even more specifically for predicting PE without distinction between preterm and term PE.

**Table 9D** captures panels of Tables 4A and 4B containing ADAM 12 in which AUC for predicting all PE (i.e., without distinction between preterm and term PE) at 20 weeks in training set (Australasian cohort) is equal to or greater than 0.745 **and** AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 9E** captures panels of Tables 4A and 4B containing ADAM12 in which AUC for predicting all PE (i.e., without distinction between preterm and term PE) at 20 weeks in training set (Australasian cohort) is equal to or greater than 0.775 **and** AUC for predicting all PE at 20 weeks in testing set (European cohort) is equal to or greater than 0.775. Without limitation, such panels may be particularly useful for predicting PE, even more specifically for predicting PE without distinction between preterm and term PE.

**Table 9F** captures panels of Tables 4A and 4B containing ADAM12 in which AUC for predicting preterm PE at 20 weeks in the European cohort or in the Australasian cohort **or** in the combined European and Australasian cohort is equal to or greater than 0.745. Without limitation, such panels may be particularly useful for predicting preterm PE.

**Table 9G** captures panels of Tables 4A and 4B containing ADAM12 in which AUC for predicting preterm PE at 20 weeks in the European cohort is equal to or greater than 0.895. Without limitation, such panels may be particularly useful for predicting preterm PE, even more specifically in European ancestry patients.

**Table 9H** captures panels of Tables 4A and 4B containing ADAM12 in which AUC for predicting preterm PE at 20 weeks in the Australasian cohort is equal to or greater than 0.895. Without limitation, such panels may be particularly useful for predicting preterm PE, even more specifically in Australasian ancestry patients.

**Table 9I** captures panels of Tables 4A and 4B containing ADAM12 in which AUC for predicting preterm PE at 20 weeks in the combined European and Australasian cohort is equal to or greater than 0.895. Without limitation, such panels may be particularly useful for predicting preterm PE.

**Table 9J** captures panels of Tables 4A and 4B containing ADAM12 in which AUC for predicting term PE at 20 weeks in the European cohort is equal to or greater than 0.745. Without limitation, such panels may be particularly useful for predicting term PE, even more specifically in European ancestry patients.

**Table 9K** captures panels of Tables 4A and 4B containing ADAM12 in which AUC for predicting term PE at 20 weeks in the Australasian cohort is equal to or greater than 0.745. Without limitation, such panels may be particularly useful for predicting term PE, even more specifically in Australasian ancestry patients.

**Table 9L** captures panels of Tables 4A and 4B containing ADAM12 in which AUC for predicting term PE at 20 weeks in the combined European and Australasian cohort is equal to or greater than 0.745. Without limitation, such panels may be particularly useful for predicting term PE.

**Table 9M** captures panels of Table 5Ma containing ADAM12.

**Table 9N** captures panels of Table 5Na containing ADAM12.

**Table 9A**

| AJ | AK | AL | AM | AN | AO | AP | AQ | AR | AS |
|---|---|---|---|---|---|---|---|---|---|
| 57 | 6 | 54 | 94.7 | ENG | 44 | 77.2 | BP15 | 33 | 57.9 |
| | 5 | 3 | 5.3 | SPINT1 | 40 | 70.2 | bmi | 18 | 31.6 |
| | 4 | | 0.0 | IGFALS | 38 | 66.7 | BP20 | 10 | 17.5 |
| | 3 | | 0.0 | MCAM | 33 | 57.9 | *fihd* | 9 | 15.8 |
| | 2 | | 0.0 | PIGF | 18 | 31.6 | *alcoh* | 4 | 7.0 |
| | 1 | | 0.0 | MMRN2 | 14 | 24.6 | *fhpet* | 3 | 5.3 |
| | | | | QSOX1 | 7 | 12.3 | | | |
| | | | | SEPP1 | 5 | 8.8 | | | |
| | | | | ECM1 | 4 | 7.0 | | | |
| | | | | ROBO4 | 2 | 3.5 | | | |

**Table 9B**

| AJ | AK | AL | AM | AN | AO | AP | AQ | AR | AS |
|---|---|---|---|---|---|---|---|---|---|
| 13 | 6 | 4 | 30.8 | IGFALS | 12 | 92.3 | *alcoh* | 5 | 38.5 |
| | 5 | 8 | 61.5 | PIGF | 7 | 53.8 | BP20 | 3 | 23.1 |
| | 4 | 1 | 7.7 | MMRN2 | 6 | 46.2 | BP15 | 4 | 30.8 |
| | 3 | | 0.0 | MCAM | 5 | 38.5 | *fhpet* | 2 | 15.4 |
| | 2 | | 0.0 | QSOX1 | 2 | 15.4 | bmi | 2 | 15.4 |
| | 1 | | 0.0 | ENPP2 | 2 | 15.4 | pbwgt | 2 | 15.4 |
| | | | | SEPP1 | 1 | 7.7 | | | |
| | | | | MAPRE1/3 | 1 | 7.7 | | | |
| | | | | ALDOA | 1 | 7.7 | | | |

**Table 9C**

| AJ | AK | AL | AM | AN | AO | AP | AQ | AR | AS |
|---|---|---|---|---|---|---|---|---|---|
| 1 | 6 | 1 | 100 | MMRN2 | 1 | 100 | BP15 | 1 | 100 |
| | 5 | | 0 | ADAM12 | 1 | 100 | | | |
| | 4 | | 0 | IGFALS | 1 | 100 | | | |
| | 3 | | 0 | MCAM | 1 | 100 | | | |
| | 2 | | 0 | PIGF | 1 | 100 | | | |
| | 1 | | 0 | | | | | | |

**Table 9D**

| AJ | AK | AL | AM | AN | AO | AP | AQ | AR | AS |
|---|---|---|---|---|---|---|---|---|---|
| 253 | 6 | 89 | 35.2 | IGFALS | 188 | 74.3 | BP20 | 117 | 46.2 |
| | 5 | 124 | 49.0 | MCAM | 143 | 56.5 | BP15 | 102 | 40.3 |
| | 4 | 40 | 15.8 | PIGF | 116 | 45.8 | *alcoh* | 42 | 16.6 |
| | 3 | | 0.0 | ENG | 64 | 25.3 | bmi | 38 | 15.0 |
| | 2 | | 0.0 | MMRN2 | 56 | 22.1 | *fihd* | 14 | 5.5 |
| | 1 | | 0.0 | SPINT1 | 44 | 17.4 | *fhpet* | 7 | 2.8 |
| | | | | QSOX1 | 35 | 13.8 | pbwgt | 5 | 2.0 |
| | | | | SEPP1 | 25 | 9.9 | | | |
| | | | | ECM1 | 21 | 8.3 | | | |
| | | | | ROBO4 | 18 | 7.1 | | | |
| | | | | ENPP2 | 17 | 6.7 | | | |
| | | | | ALDOA | 4 | 1.6 | | | |
| | | | | MAPRE1/3 | 2 | 0.8 | | | |
| | | | | LCAT | 1 | 0.4 | | | |
| | | | | PRDX2 | 1 | 0.4 | | | |

**Table 9E**

| AJ | AK | AL | AM | AN | AO | AP | AQ | AR | AS |
|---|---|---|---|---|---|---|---|---|---|
| 21 | 6 | 8 | 38.1 | IGFALS | 21 | 100 | BP20 | 11 | 52.4 |
| | 5 | 13 | 61.9 | PIGF | 16 | 76.2 | BP15 | 9 | 42.9 |
| | 4 | | 0.0 | MCAM | 14 | 66.7 | *alcoh* | 3 | 14.3 |
| | 3 | | 0.0 | MMRN2 | 12 | 57.1 | | | |
| | 2 | | 0.0 | SEPP1 | 3 | 14.3 | | | |
| | 1 | | 0.0 | ENG | 2 | 9.5 | | | |
| | | | | ECM1 | 1 | 4.8 | | | |

**Table 9F**

| AJ | AK | AL | AM | AN | AO | AP | AQ | AR | AS |
|---|---|---|---|---|---|---|---|---|---|
| 436 | 6 | 131 | 30.0 | IGFALS | 313 | 71.8 | BP15 | 206 | 47.2 |
| | 5 | 225 | 51.6 | MCAM | 210 | 48.2 | BP20 | 159 | 36.5 |
| | 4 | 75 | 17.2 | PIGF | 192 | 44.0 | *alcoh* | 70 | 16.1 |
| | 3 | 5 | 1.1 | ENG | 116 | 26.6 | bmi | 66 | 15.1 |
| | 2 | | 0.0 | SPINT1 | 98 | 22.5 | *fihd* | 40 | 9.2 |
| | 1 | | 0.0 | MMRN2 | 93 | 21.3 | *fhpet* | 11 | 2.5 |
| | | | | QSOX1 | 61 | 14.0 | pbwgt | 8 | 1.8 |
| | | | | SEPP1 | 41 | 9.4 | mothpet | 1 | 0.2 |
| | | | | ECM1 | 35 | 8.0 | | | |

(continued)

| AJ | AK | AL | AM | AN | AO | AP | AQ | AR | AS |
|----|----|----|----|------|----|-----|----|----|----|
|    |    |    |    | ENPP2 | 25 | 5.7 |    |    |    |
|    |    |    |    | ROBO4 | 23 | 5.3 |    |    |    |
|    |    |    |    | ALDOA | 13 | 3.0 |    |    |    |
|    |    |    |    | MARE1/3 | 7 | 1.6 |    |    |    |
|    |    |    |    | LCAT | 1 | 0.2 |    |    |    |
|    |    |    |    | PRDX2 | 1 | 0.2 |    |    |    |

**Table 9G**

| AJ | AK | AL | AM | AN | AO | AP | AQ | AR | AS |
|----|----|----|------|--------|----|------|------|----|------|
| 21 | 6 | 12 | 57.1 | IGFALS | 14 | 66.7 | BP20 | 11 | 52.4 |
|    | 5 | 9 | 42.9 | SPINT1 | 13 | 61.9 | BP15 | 2 | 9.5 |
|    | 4 |   | 0.0 | MCAM | 13 | 61.9 | bmi | 1 | 4.8 |
|    | 3 |   | 0.0 | MMRN2 | 12 | 57.1 |    |    |    |
|    | 2 |   | 0.0 | ENG | 11 | 52.4 |    |    |    |
|    | 1 |   | 0.0 | PIGF | 10 | 47.6 |    |    |    |
|    |   |   |     | ECM1 | 7 | 33.3 |    |    |    |
|    |   |   |     | QSOX1 | 2 | 9.5 |    |    |    |

**Table 9H**

| AJ | AK | AL | AM | AN | AO | AP | AQ | AR | AS |
|----|----|----|------|--------|----|-------|------|----|------|
| 44 | 6 | 41 | 93.2 | SPINT1 | 44 | 100.0 | BP15 | 29 | 65.9 |
|    | 5 | 3 | 6.8 | IGFALS | 39 | 88.6 | *fihd* | 4 | 9.1 |
|    | 4 |   | 0.0 | ENG | 39 | 88.6 | bmi | 4 | 9.1 |
|    | 3 |   | 0.0 | MCAM | 20 | 45.5 | BP20 | 3 | 6.8 |
|    | 2 |   | 0.0 | MMRN2 | 11 | 25.0 | *alcoh* | 2 | 4.5 |
|    | 1 |   | 0.0 | PIGF | 11 | 25.0 |    |    |    |
|    |   |   |     | QSOX1 | 9 | 20.5 |    |    |    |
|    |   |   |     | SEPP1 | 2 | 4.5 |    |    |    |

**Table 9I**

| AJ | AK | AL | AM | AN | AO | AP | AQ | AR | AS |
|----|----|----|------|--------|----|------|------|----|------|
| 61 | 6 | 55 | 90.2 | SPINT1 | 60 | 98.4 | BP15 | 35 | 57.4 |
|    | 5 | 6 | 9.8 | ENG | 52 | 85.2 | bmi | 15 | 24.6 |
|    | 4 |   | 0.0 | IGFALS | 44 | 72.1 | BP20 | 8 | 13.1 |
|    | 3 |   | 0.0 | MCAM | 33 | 54.1 | *fihd* | 5 | 8.2 |
|    | 2 |   | 0.0 | PIGF | 18 | 29.5 | *alcoh* | 2 | 3.3 |
|    | 1 |   | 0.0 | MMRN2 | 13 | 21.3 | *fhpet* | 1 | 1.6 |

(continued)

| AJ | AK | AL | AM | AN | AO | AP | AQ | AR | AS |
|----|----|----|----|----|----|----|----|----|----|
|  |  |  |  | QSOX1 | 9 | 14.8 |  |  |  |
|  |  |  |  | SEPP1 | 3 | 4.9 |  |  |  |
|  |  |  |  | ENPP2 | 1 | 1.6 |  |  |  |

**Table 9J**

| AJ | AK | AL | AM | AN | AO | AP | AQ | AR | AS |
|----|----|----|----|----|----|----|----|----|----|
| 33 | 6 | 18 | 54.5 | IGFALS | 31 | 93.9 | BP20 | 18 | 54.5 |
|  | 5 | 15 | 45.5 | MCAM | 22 | 66.7 | BP15 | 14 | 42.4 |
|  | 4 |  | 0.0 | PIGF | 22 | 66.7 | *alcoh* | 5 | 15.2 |
|  | 3 |  | 0.0 | MMRN2 | 11 | 33.3 | bmi | 5 | 15.2 |
|  | 2 |  | 0.0 | ENG | 9 | 27.3 | *fihd* | 1 | 3.0 |
|  | 1 |  | 0.0 | SEPP1 | 6 | 18.2 |  |  |  |
|  |  |  |  | QSOX1 | 5 | 15.2 |  |  |  |
|  |  |  |  | ALDOA | 1 | 3.0 |  |  |  |

**Table 9K**

| AJ | AK | AL | AM | AN | AO | AP | AQ | AR | AS |
|----|----|----|----|----|----|----|----|----|----|
| 238 | 6 | 94 | 39.5 | IGFALS | 172 | 72.3 | BP15 | 142 | 59.7 |
|  | 5 | 133 | 55.9 | PIGF | 142 | 59.7 | bmi | 55 | 23.1 |
|  | 4 | 11 | 4.6 | MCAM | 133 | 55.9 | BP20 | 51 | 21.4 |
|  | 3 |  | 0.0 | ENG | 70 | 29.4 | *alcoh* | 43 | 18.1 |
|  | 2 |  | 0.0 | SPINT1 | 52 | 21.8 | *fihd* | 35 | 14.7 |
|  | 1 |  | 0.0 | MMRN2 | 38 | 16.0 | *fhpet* | 9 | 3.8 |
|  |  |  |  | SEPP1 | 24 | 10.1 | pbwgt | 5 | 2.1 |
|  |  |  |  | QSOX1 | 21 | 8.8 | mothpet | 1 | 0.4 |
|  |  |  |  | ECM1 | 19 | 8.0 |  |  |  |
|  |  |  |  | ENPP2 | 11 | 4.6 |  |  |  |
|  |  |  |  | ROBO4 | 8 | 3.4 |  |  |  |
|  |  |  |  | ALDOA | 4 | 1.7 |  |  |  |

**Table 9L**

| AJ | AK | AL | AM | AN | AO | AP | AQ | AR | AS |
|----|----|----|----|----|----|----|----|----|----|
| 247 | 6 | 95 | 38.5 | IGFALS | 188 | 76.1 | BP15 | 133 | 53.8 |
|  | 5 | 133 | 53.8 | PIGF | 134 | 54.3 | BP20 | 74 | 30.0 |
|  | 4 | 19 | 7.7 | MCAM | 132 | 53.4 | *alcoh* | 58 | 23.5 |
|  | 3 |  | 0.0 | ENG | 77 | 31.2 | bmi | 55 | 22.3 |
|  | 2 |  | 0.0 | SPINT1 | 46 | 18.6 | *fihd* | 21 | 8.5 |

(continued)

| AJ | AK | AL | AM | AN | AO | AP | AQ | AR | AS |
|---|---|---|---|---|---|---|---|---|---|
|  | 1 |  | 0.0 | MMRN2 | 40 | 16.2 | *fhpet* | 10 | 4.0 |
|  |  |  |  | QSOX1 | 27 | 10.9 | pbwgt | 6 | 2.4 |
|  |  |  |  | SEPP1 | 20 | 8.1 | mothpet | 1 | 0.4 |
|  |  |  |  | ECM1 | 14 | 5.7 |  |  |  |
|  |  |  |  | ROBO4 | 10 | 4.0 |  |  |  |
|  |  |  |  | ENPP2 | 10 | 4.0 |  |  |  |
|  |  |  |  | ALDOA | 6 | 2.4 |  |  |  |
|  |  |  |  | MAPRE1/3 | 2 | 0.8 |  |  |  |

**Table 9M**

| AJ | AK | AL | AM | AN | AO | AP | AQ | AR | AS |
|---|---|---|---|---|---|---|---|---|---|
| 22 | 6 | 19 | 86.4 | IGFALS | 22 | 100 | BP15 | 18 | 81.8 |
|  | 5 | 2 | 9.1 | SPINT1 | 17 | 77.3 | BP20 | 2 | 9.1 |
|  | 4 | 1 | 4.5 | ENG | 14 | 63.6 |  |  |  |
|  | 3 |  | 0.0 | MCAM | 14 | 63.6 |  |  |  |
|  | 2 |  | 0.0 | PIGF | 10 | 45.5 |  |  |  |
|  | 1 |  | 0.0 | MMRN2 | 9 | 40.9 |  |  |  |

**Table 9N**

| AJ | AK | AL | AM | AN | AO | AP | AQ | AR | AS |
|---|---|---|---|---|---|---|---|---|---|
| 3 | 6 | 1 | 33.3 | IGFALS | 3 | 100 | BP20 | 1 | 33.3 |
|  | 5 | 1 | 33.3 | PIGF | 3 | 100 | BP15 | 1 | 33.3 |
|  | 4 | 1 | 33.3 | MCAM | 2 | 66.7 |  |  |  |
|  | 3 |  | 0.0 | MMRN2 | 2 | 66.7 |  |  |  |
|  | 2 |  | 0.0 |  |  |  |  |  |  |
|  | 1 |  | 0.0 |  |  |  |  |  |  |

**Example 10**

This example relates to panels of Tables 4A and 4B all of which do not contain PIGF, ENG and ADAM 12 (hence, PIGF, ENG and ADAM12, which are always absent from these panels, are not listed in the tables).

**Table 10A** captures panels of Tables 4A and 4B not containing PIGF, ENG and ADAM12 in which specificity at 99% NPV for predicting all PE (i.e., without distinction between preterm and term PE) at 20 weeks in training set (Australasian cohort) is equal to or greater than 0.395 **and** specificity at 99% NPV for predicting all PE at 20 weeks in testing set (European cohort) is equal to or greater than 0.395. Without limitation, such panels may be particularly useful as rule-out panels, more specifically for predicting PE, even more specifically for predicting PE without distinction between preterm and term PE.

**Table 10B** captures panels of Tables 4A and 4B not containing PIGF, ENG and ADAM12 in which AUC for predicting all PE (i.e., without distinction between preterm and term PE) at 20 weeks in training set (Australasian cohort) is equal to or greater than 0.745 **and** AUC for predicting all PE at 20 weeks in testing set (European cohort) is equal to or greater than 0.745. Without limitation, such panels may be particularly useful for predicting PE, even more specifically for predicting PE without distinction between preterm and term PE.

**Table 10C** captures panels of Tables 4A and 4B not containing PIGF, ENG and ADAM12 in which AUC for predicting preterm PE at 20 weeks in the European cohort **or** in the Australasian cohort or in the combined European and Australasian cohort is equal to or greater than 0.745. Without limitation, such panels may be particularly useful for predicting preterm PE.

**Table 10D** captures panels of Tables 4A and 4B not containing PIGF, ENG and ADAM 12 in which AUC for predicting term PE at 20 weeks in the European cohort is equal to or greater than 0.745. Without limitation, such panels may be particularly useful for predicting term PE, even more specifically in European ancestry patients.

**Table 10E** captures panels of Tables 4A and 4B not containing PIGF, ENG and ADAM 12 in which AUC for predicting term PE at 20 weeks in the Australasian cohort is equal to or greater than 0.745. Without limitation, such panels may be particularly useful for predicting term PE, even more specifically in Australasian ancestry patients.

**Table 10F** captures panels of Tables 4A and 4B not containing PIGF, ENG and ADAM 12 in which AUC for predicting term PE at 20 weeks in the combined European and Australasian cohort is equal to or greater than 0.745. Without limitation, such panels may be particularly useful for predicting term PE.

**Table 10A**

| AJ | AK | AL | AM | AN | AO | AP | AQ | AR | AS |
|---|---|---|---|---|---|---|---|---|---|
| 3 | 6 | | 0.0 | IGFALS | 3 | 100.0 | *alcoh* | 3 | 100.0 |
| | 5 | 3 | 100.0 | MMRN2 | 3 | 100.0 | BP20 | 3 | 100.0 |
| | 4 | | 0.0 | SEPP1 | 1 | 33.3 | *fhpet* | 1 | 33.3 |
| | 3 | | 0.0 | | | | *vagbl* | 1 | 33.3 |
| | 2 | | 0.0 | | | | | | |
| | 1 | | 0.0 | | | | | | |

**Table 10B**

| AJ | AK | AL | AM | AN | AO | AP | AQ | AR | AS |
|---|---|---|---|---|---|---|---|---|---|
| 52 | 6 | 5 | 9.6 | IGFALS | 52 | 100.0 | BP20 | 46 | 88.5 |
| | 5 | 32 | | MMRN2 | 38 | 73.1 | *alcoh* | 17 | 32.7 |
| | 4 | 15 | | SEPP1 | 16 | 30.8 | *vagbl* | 12 | 23.1 |
| | 3 | | | LNPEP | 11 | 21.2 | *fhpet* | 6 | 11.5 |
| | 2 | | | ALDOA | 10 | 19.2 | BP15 | 4 | 7.7 |
| | 1 | | | MAPRE1/3 | 10 | 19.2 | *fihd* | 2 | 3.8 |
| | | | | MCAM | 8 | 15.4 | | | |
| | | | | ECM1 | 6 | 11.5 | | | |
| | | | | ROBO4 | 5 | 9.6 | | | |
| | | | | QSOX1 | 3 | 5.8 | | | |
| | | | | ENPP2 | 3 | 5.8 | | | |
| | | | | PRDX2 | 1 | 1.9 | | | |

**Table 10C**

| AJ | AK | AL | AM | AN | AO | AP | AQ | AR | AS |
|---|---|---|---|---|---|---|---|---|---|
| 93 | 6 | 5 | 5.4 | IGFALS | 93 | 100.0 | BP20 | 73 | 78.5 |
| | 5 | 51 | 54.8 | MMRN2 | 60 | 64.5 | *alcoh* | 31 | 33.3 |
| | 4 | 30 | 32.3 | SEPP1 | 22 | 23.7 | *vagbl* | 20 | 21.5 |
| | 3 | 7 | 7.5 | LNPEP | 19 | 20.4 | BP15 | 17 | 18.3 |

(continued)

| AJ | AK | AL | AM | AN | AO | AP | AQ | AR | AS |
|----|----|----|-----|----------|----|------|-------|----|------|
|    | 2  |    | 0.0 | MCAM     | 16 | 17.2 | *fhpet* | 10 | 10.8 |
|    | 1  |    | 0.0 | ALDOA    | 14 | 15.1 | *fihd*  | 6  | 6.5  |
|    |    |    |     | MAPRE1/3 | 14 | 15.1 |       |    |      |
|    |    |    |     | ECM1     | 10 | 10.8 |       |    |      |
|    |    |    |     | ROBO4    | 9  | 9.7  |       |    |      |
|    |    |    |     | QSOX1    | 8  | 8.6  |       |    |      |
|    |    |    |     | ENPP2    | 3  | 3.2  |       |    |      |
|    |    |    |     | PRDX2    | 1  | 1.1  |       |    |      |

**Table 10D**

| AJ | AK | AL | AM   | AN       | AO | AP    | AQ      | AR | AS    |
|----|----|----|------|----------|----|-------|---------|----|-------|
| 5  | 6  | 2  | 40.0 | IGFALS   | 5  | 100.0 | BP20    | 5  | 100.0 |
|    | 5  | 2  | 40.0 | MMRN2    | 5  | 100.0 | *vagbl* | 3  | 100.0 |
|    | 4  | 1  | 20.0 | SEPP1    | 3  | 60.0  |         |    |       |
|    | 3  |    | 0.0  | QSOX1    | 1  | 20.0  |         |    |       |
|    | 2  |    | 0.0  | LNPEP    | 1  | 20.0  |         |    |       |
|    | 1  |    | 0.0  | ECM1     | 1  | 20.0  |         |    |       |
|    |    |    |      | ALDOA    | 1  | 20.0  |         |    |       |
|    |    |    |      | MAPRE1/3 | 1  | 20.0  |         |    |       |
|    |    |    |      | MCAM     |    |       |         |    |       |

**Table 10E**

| AJ | AK | AL | AM   | AN       | AO | AP    | AQ      | AR | AS   |
|----|----|----|------|----------|----|-------|---------|----|------|
| 14 | 6  | 1  | 7.1  | IGFALS   | 14 | 100.0 | BP20    | 8  | 57.1 |
|    | 5  | 13 | 92.9 | MMRN2    | 11 | 78.6  | *alcoh* | 8  | 57.1 |
|    | 4  |    | 0.0  | MCAM     | 6  | 42.9  | BP15    | 6  | 42.9 |
|    | 3  |    | 0.0  | ECM1     | 4  | 28.6  | *vagbl* | 5  | 35.7 |
|    | 2  |    | 0.0  | SEPP1    | 1  | 7.1   | *fihd*  | 3  | 21.4 |
|    | 1  |    | 0.0  | ROBO4    | 1  | 7.1   | *fhpet* | 1  | 7.1  |
|    |    |    |      | ALDOA    | 1  | 7.1   |         |    |      |
|    |    |    |      | LNPEP    | 1  | 7.1   |         |    |      |
|    |    |    |      | MAPRE1/3 | 1  | 7.1   |         |    |      |

**Table 10F**

| AJ | AK | AL | AM   | AN     | AO | AP    | AQ      | AR | AS   |
|----|----|----|------|--------|----|-------|---------|----|------|
| 30 | 6  | 3  | 10.0 | IGFALS | 30 | 100.0 | BP20    | 25 | 83.3 |
|    | 5  | 23 | 76.7 | MMRN2  | 19 | 63.3  | *alcoh* | 21 | 70.0 |

(continued)

| AJ | AK | AL | AM | AN | AO | AP | AQ | AR | AS |
|---|---|---|---|---|---|---|---|---|---|
| | 4 | 4 | 13.3 | ALDOA | 8 | 26.7 | *vagbl* | 8 | 26.7 |
| | 3 | | 0.0 | MAPRE1/3 | 8 | 26.7 | *fhpet* | 6 | 20.0 |
| | 2 | | 0.0 | MCAM | 4 | 13.3 | BP15 | 4 | 13.3 |
| | 1 | | 0.0 | LNPEP | 4 | 13.3 | *fihd* | 3 | 10.0 |
| | | | | QSOX1 | 3 | 10.0 | | | |
| | | | | SEPP1 | 3 | 10.0 | | | |
| | | | | ROBO4 | 2 | 6.7 | | | |
| | | | | ENPP2 | 1 | 3.3 | | | |

## EXAMPLE 11 : Further test panels for the prediction of PE

The data and analyses in this example have been obtained using the Australasian case-control set. Trough logistic regression on the above-mentioned training set, panels or combinations of markers were obtained to develop a model that estimates the probability of contracting preeclampsia.

Powerful results were realised for the test panel comprising measurement of the level of PIGF and measurement of the level of IGFALS as shown in **Table 11. Table 11** illustrates that the test panel consisting of measurement of the level of PIGF and measurement of the level of IGFALS allowed an improved prediction of PE compared with PIGF or IGFALS alone.

Even further improvements were obtained with panels consisting of PIGF level, IGFALS level and the level of another biomarker as specified in **Table 11.**

**Table 11 :** Overview of the performance of different test panels for the prediction of PE

*AUC = area under the curve; lCI = lower confidence interval; uCI = upper confidence interval; imp.1, imp.2 and imp. 3 refer to the improvement of the AUC of the combination compared with each respective biomarker, n.a. = not applicable.*

| Test panel | AUC | AUC.lCI | AUC.uCI | imp.1 | imp.2 | imp.3 |
|---|---|---|---|---|---|---|
| PIGF + IGFALS | 0.781 | 0.701 | 0.861 | 0.017 | 0.091 | n.a. |
| PIGF + IGFALS + SEPP1 | 0.807 | 0.732 | 0.882 | 0.004 | 0.022 | <0.001 |
| PIGF + XPNPEP2 + IGFALS | 0.811 | 0.728 | 0.895 | 0.005 | <0.001 | 0.034 |
| PIGF + TNXB + IGFALS | 0.807 | 0.725 | 0.888 | 0.005 | 0.001 | 0.040 |
| PIGF + PCYOX1 + IGFALS | 0.802 | 0.722 | 0.882 | 0.007 | <0.001 | 0.045 |
| PIGF + MMRN2 + IGFALS | 0.800 | 0.721 | 0.879 | 0.007 | <0.001 | 0.071 |
| PIGF + ENG + IGFALS | 0.798 | 0.720 | 0.875 | 0.007 | 0.002 | 0.055 |
| PIGF + FLT4 + IGFALS | 0.798 | 0.720 | 0.876 | 0.007 | 0.010 | 0.042 |
| PIGF + PRDX1 + IGFALS | 0.796 | 0.719 | 0.873 | 0.007 | <0.001 | 0.034 |
| PIGF + ADAM12+ IGFALS | 0.796 | 0.719 | 0.873 | 0.008 | 0.002 | 0.061 |

(continued)

| AUC = area under the curve; lCI = lower confidence interval; uCI = upper confidence interval; imp.1, imp.2 and imp. 3 refer to the improvement of the AUC of the combination compared with each respective biomarker, n.a. = not applicable. | | | | | | |
|---|---|---|---|---|---|---|
| **Test panel** | **AUC** | **AUC.lCI** | **AUC.uCI** | **imp.1** | **imp.2** | **imp.3** |
| PIGF + MCAM + IGFALS | 0.797 | 0.717 | 0.877 | 0.008 | 0.004 | 0.059 |
| PIGF + LNPEP + IGFALS | 0.793 | 0.716 | 0.871 | 0.009 | 0.003 | 0.079 |
| PIGF + ENPP2 + IGFALS | 0.793 | 0.713 | 0.873 | 0.010 | 0.002 | 0.076 |
| PIGF + HSPG +IGFALS | 0.813 | 0.737 | 0.888 | 0.003 | <0.001 | 0.25 |
| PIGF + QSOX1+IGFALS | 0.803 | 0.724 | 0.881 | 0.006 | <0.001 | 0.34 |

**Example 12: Further illustrative test panels for the prediction of PE**

The data and analyses in this example have been obtained using the European case-control set as captured in Table 2, and applying the statistical analysis methods as elucidated in Example 3. Panels or combinations of markers and/or clinical parameters were obtained to develop models that estimate the probability of contracting preeclampsia. The panels were selected to contain TFF3.

Whereas the outcome of the test panels exemplified herein is the prediction of preeclampsia at 20 weeks of gestation, the test panels are useful throughout the second trimester, such as between 13 and 28 weeks of gestation, e.g., at 20 +/- 2, 20 +/- 1 , 15 +/-2 or 15 +/- 1 weeks of gestation, and can even be applied with success in the first trimester.

**Table 12** captures relevant statistics pertinent to performance of panels useful for predicting PE, which illustrate various embodiments of the present invention. The following abbreviations are used in the table: **AUC:** area under the ROC curve; **lCI:** lower confidence interval; **uCI:** upper confidence interval. The following column denotations are used in the table: Panel composition: constituents forming up a panel (i.e. the panel consists of the recited constituents) (note that in **Table 12** IGFALS stands for IGFALS measurement by MASSTERCLASS® assay, IGFALS-e stands for IGFALS measurement by ELISA); **BA:** AUC for predicting all PE (i.e., without distinction between preterm and term PE) at 20 weeks - European cohort; BAa: lCI AUC for predicting all PE at 20 - European cohort; Bab: uCI AUC for predicting all PE at 20 - European cohort; **BB:** Sensitivity at 20% PPV for predicting all PE at 20 weeks - European cohort; **BC:** Specificity at 99% NPV for predicting all PE at 20 weeks - European cohort; **BD:** AUC for predicting preterm PE at 20 weeks - European cohort; **BE:** lCI AUC for predicting preterm PE at 20 weeks - European cohort; **BF:** uCI AUC for predicting preterm PE at 20 weeks - European cohort; **BG:** AUC for predicting term PE at 20 weeks - European cohort; **BH:** lCI AUC for predicting term PE at 20 weeks - European cohort; **BI:** uCI AUC for predicting term PE at 20 weeks - European cohort.

In Table 12, some redundancy is apparent among the panels, i.e., Table 12 may list a test panel of a given composition more than once. One cause of the redundancy is when a given biomarker was measured in two or more distinct ways. For example, as set forth in Table 3, each ADAM 12, ECM1, and LCAT could be measured by MASSTERCLASS® assays using two distinct peptides. Also for example, IGFALS was measured using MASSTERCLASS® (denoted IGFALS in **Table 12)** and by ELISA (denoted IGFALS-e in **Table 12).** Another cause of the redundancy is that the clinical parameter blood pressure (BP) or even the more specific parameters BP at 15 weeks (BP15) and BP at 20 weeks (BP20), cover a multiplicity of blood pressure measurements, such as the measurement of systolic, diastolic or mean arterial blood pressure, as well as 1st or 2nd measurements (see Table 2).

**Table 12**

| Panel composition | BA | BAa | Bab | BB | BC | BD | BE | BF | BG | BH | BI |
|---|---|---|---|---|---|---|---|---|---|---|---|
| BP20;MMRN2;ECM1; LNPEP;TFF3;PIGF | 0.81 | 0.74 | 0.88 | 0.53 | 0.45 | 0.93 | 0.86 | 0.99 | 0.77 | 0.69 | 0.85 |

(continued)

| Panel composition | BA | BAa | Bab | BB | BC | BD | BE | BF | BG | BH | BI |
|---|---|---|---|---|---|---|---|---|---|---|---|
| BP20;MMRN2;ECM1; TFF3;IGFALS-e;PIGF | 0.82 | 0.75 | 0.88 | 0.51 | 0.65 | 0.94 | 0.89 | 0.99 | 0.77 | 0.69 | 0.86 |
| BP20;MMRN2;ECM1; LNPEP;TFF3;PIGF | 0.80 | 0.73 | 0.88 | 0.60 | 0.39 | 0.94 | 0.89 | 0.98 | 0.76 | 0.67 | 0.85 |
| BP20;MMRN2;ECM1; TFF3;IGFALS-e;PIGF | 0.81 | 0.75 | 0.88 | 0.53 | 0.66 | 0.94 | 0.89 | 1.00 | 0.77 | 0.69 | 0.86 |
| BP20;MMRN2; ADAM12;ECM1;TFF3; PIGF | 0.82 | 0.75 | 0.88 | 0.60 | 0.61 | 0.95 | 0.89 | 1.00 | 0.77 | 0.69 | 0.86 |
| BP20;MMRN2;ECM1; ENG;TFF3;PIGF | 0.77 | 0.69 | 0.85 | 0.61 | 0.57 | 0.90 | 0.80 | 1.00 | 0.73 | 0.63 | 0.83 |
| BP20;MMRN2;ECM1; ENG;TFF3;PIGF | 0.77 | 0.69 | 0.85 | 0.59 | 0.56 | 0.90 | 0.79 | 1.00 | 0.73 | 0.64 | 0.83 |
| BP20;MMRN2;ECM1; TFF3;IGFALS-e;PIGF | 0.81 | 0.74 | 0.88 | 0.53 | 0.42 | 0.95 | 0.89 | 1.00 | 0.77 | 0.68 | 0.85 |
| BP20;MMRN2;ECM1; LNPEP;TFF3;PIGF | 0.80 | 0.72 | 0.87 | 0.63 | 0.21 | 0.94 | 0.90 | 0.98 | 0.75 | 0.66 | 0.84 |
| BP20;MMRN2; ADAM12;TFF3;IGFALS-e;PIGF | 0.83 | 0.76 | 0.89 | 0.55 | 0.29 | 0.95 | 0.89 | 1.00 | 0.79 | 0.71 | 0.87 |
| BP20;MMRN2;ECM1; LNPEP;TFF3;MCAM | 0.79 | 0.72 | 0.87 | 0.56 | 0.50 | 0.88 | 0.73 | 1.00 | 0.76 | 0.68 | 0.85 |
| BP20;MMRN2;ECM1; TFF3;IGFALS-e;PIGF | 0.81 | 0.74 | 0.88 | 0.49 | 0.30 | 0.95 | 0.91 | 0.99 | 0.76 | 0.68 | 0.85 |
| BP20;MMRN2;ECM1; ENG;TFF3;MCAM | 0.79 | 0.72 | 0.87 | 0.58 | 0.23 | 0.88 | 0.77 | 0.99 | 0.76 | 0.67 | 0.86 |
| BP20;MMRN2;ECM1; TFF3;IGFALS-e;PIGF | 0.81 | 0.74 | 0.88 | 0.51 | 0.37 | 0.95 | 0.90 | 1.00 | 0.76 | 0.68 | 0.85 |
| BP20;MMRN2; ADAM12;ECM1;TFF3; PIGF | 0.80 | 0.73 | 0.87 | 0.56 | 0.28 | 0.95 | 0.92 | 0.99 | 0.75 | 0.67 | 0.84 |
| BP20;MMRN2;ECM1; ENG;TFF3;MCAM | 0.78 | 0.70 | 0.86 | 0.58 | 0.53 | 0.90 | 0.81 | 0.98 | 0.74 | 0.64 | 0.85 |
| BP20;ECM1;LNPEP; TFF3;QSOX1;MCAM | 0.78 | 0.70 | 0.86 | 0.59 | 0.10 | 0.83 | 0.66 | 1.00 | 0.76 | 0.68 | 0.85 |
| BP20;MMRN2;ECM1; LNPEP;TFF3;PIGF | 0.79 | 0.72 | 0.87 | 0.55 | 0.40 | 0.93 | 0.86 | 0.99 | 0.75 | 0.66 | 0.84 |
| BP20;MMRN2;ECM1; LNPEP;TFF3;PIGF | 0.79 | 0.71 | 0.87 | 0.52 | 0.28 | 0.94 | 0.90 | 0.98 | 0.74 | 0.65 | 0.83 |
| BP20;MMRN2;ECM1; TFF3;PIGF | 0.78 | 0.70 | 0.86 | 0.58 | 0.16 | 0.92 | 0.87 | 0.98 | 0.73 | 0.64 | 0.83 |
| BP20;ADAM12;TFF3; QSOX1;IGFALS;PIGF | 0.81 | 0.75 | 0.88 | 0.43 | 0.53 | 0.91 | 0.80 | 1.00 | 0.78 | 0.70 | 0.85 |
| BP20;MMRN2;ECM1; TFF3;IGFALS-e;MCAM | 0.80 | 0.73 | 0.88 | 0.60 | 0.45 | 0.91 | 0.80 | 1.00 | 0.77 | 0.68 | 0.86 |

(continued)

| Panel composition | BA | BAa | Bab | BB | BC | BD | BE | BF | BG | BH | BI |
|---|---|---|---|---|---|---|---|---|---|---|---|
| MMRN2;ECM1;TFF3; MAPRE1/3;ALDOA; PIGF | 0.77 | 0.68 | 0.86 | 0.70 | 0.19 | 0.95 | 0.91 | 0.99 | 0.72 | 0.61 | 0.83 |
| BP20;ADAM12;TFF3; QSOX1;IGFALS;PIGF | 0.81 | 0.74 | 0.88 | 0.48 | 0.39 | 0.89 | 0.78 | 1.00 | 0.78 | 0.71 | 0.86 |
| BP20;MMRN2;FCM1; TFF3;COL6A3;MCAM | 0.78 | 0.70 | 0.86 | 0.59 | 0.32 | 0.89 | 0.75 | 1.00 | 0.74 | 0.65 | 0.83 |
| BP20;MMRN2;ECM1; LNPEP;PRDX2;TFF3 | 0.80 | 0.73 | 0.87 | 0.52 | 0.45 | 0.91 | 0.82 | 0.99 | 0.77 | 0.68 | 0.85 |
| BP20;MMRN2;ECM1; TFF3;IGFALS-e:PIGF | 0.80 | 0.73 | 0.87 | 0.58 | 0.38 | 0.95 | 0.90 | 1.00 | 0.75 | 0.67 | 0.84 |
| BP20;MMRN2;ECM1; TFF3;PIGF | 0.78 | 0.70 | 0.86 | 0.58 | 0.26 | 0.92 | 0.86 | 0.98 | 0.73 | 0.64 | 0.83 |
| BP20;MMRN2;ECM1; TFF3;MCAM;PIGF | 0.79 | 0.70 | 0.87 | 0.60 | 0.06 | 0.94 | 0.88 | 0.99 | 0.74 | 0.63 | 0.84 |
| BP20;MMRN2;ECM1; ENG;TFF3;PIGF | 0.76 | 0.67 | 0.84 | 0.56 | 0.17 | 0.89 | 0.78 | 0.99 | 0.71 | 0.61 | 0.81 |
| BP15;MMRN2; ADAM12;ECM1;TFF3; PIGF | 0.80 | 0.73 | 0.86 | 0.50 | 0.47 | 0.92 | 0.87 | 0.98 | 0.75 | 0.67 | 0.84 |
| BP20;MMRN2;ECM1; TFF3;MCAM | 0.78 | 0.70 | 0.86 | 0.63 | 0.45 | 0.86 | 0.72 | 1.00 | 0.75 | 0.66 | 0.84 |
| BP15;MMRN2; ADAM12;ECM1;TFF3; PIGF | 0.80 | 0.73 | 0.87 | 0.48 | 0.40 | 0.93 | 0.88 | 0.98 | 0.75 | 0.67 | 0.84 |
| BP20;ADAM12;TFF3; QSOX1;IGFALS-e;PIGF | 0.81 | 0.75 | 0.88 | 0.45 | 0.16 | 0.88 | 0.75 | 1.00 | 0.79 | 0.72 | 0.87 |
| BP15;MMRN2; ADAM12;ECM1;TFF3; PIGF | 0.80 | 0.73 | 0.87 | 0.45 | 0.47 | 0.92 | 0.86 | 0.98 | 0.76 | 0.67 | 0.84 |
| BP15;MMRN2; ADAM12;ECM1;TFF3; PIGF | 0.79 | 0.72 | 0.86 | 0.55 | 0.48 | 0.92 | 0.87 | 0.98 | 0.75 | 0.67 | 0.84 |
| BP20;MMRN2; ADAM12;TFF3;PIGF | 0.81 | 0.74 | 0.88 | 0.52 | 0.22 | 0.94 | 0.89 | 0.99 | 0.76 | 0.68 | 0.85 |
| BP20;ADAM12;TFF3; QSOX1;IGFALS-e;PIGF | 0.81 | 0.75 | 0.88 | 0.36 | 0.16 | 0.90 | 0.76 | 1.00 | 0.79 | 0.71 | 0.87 |
| BP20;MMRN2; ADAM12;ECM1;TFF3; PIGF | 0.79 | 0.72 | 0.87 | 0.60 | 0.26 | 0.95 | 0.92 | 0.99 | 0.74 | 0.66 | 0.83 |
| BP20;MMRN2;ECM1; TFF3;COL6A3;MCAM | 0.78 | 0.69 | 0.86 | 0.58 | 0.28 | 0.91 | 0.79 | 1.00 | 0.73 | 0.63 | 0.83 |
| BP20;ADAM12;TFF3; IGFALS-e;MCAM;PIGF | 0.82 | 0.75 | 0.89 | 0.59 | 0.16 | 0.89 | 0.76 | 1.00 | 0.80 | 0.72 | 0.88 |
| BP20;MMRN2; ADAM12;TFF3;PIGF | 0.80 | 0.73 | 0.88 | 0.52 | 0.22 | 0.95 | 0.91 | 0.99 | 0.76 | 0.67 | 0.84 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;EGM1;TFF3; PIGF | 0.80 | 0.73 | 0.86 | 0.44 | 0.42 | 0.93 | 0.89 | 0.98 | 0.75 | 0.67 | 0.83 |
| MMRN2;ADAM12; ECM1;TFF3;IGFALS-e; PIGF | 0.80 | 0.73 | 0.87 | 0.52 | 0.40 | 0.96 | 0.93 | 1.00 | 0.75 | 0.67 | 0.83 |
| BP20;MMRN2; ADAM12;TFF3;IGFALS-e;PIGF | 0.81 | 0.75 | 0.88 | 0.61 | 0.39 | 0.94 | 0.87 | 1.00 | 0.77 | 0.69 | 0.86 |
| BP20;MMRN2;ECM1; TFF3;PIGF | 0.78 | 0.69 | 0.86 | 0.47 | 0.15 | 0.93 | 0.88 | 0.97 | 0.72 | 0.63 | 0.82 |
| BP20;MMRN2;ECM1; ENG;TFF3;MCAM | 0.77 | 0.69 | 0.86 | 0.55 | 0.46 | 0.90 | 0.82 | 0.98 | 0.73 | 0.63 | 0.83 |
| BP20;MMRN2;ECM1; LNPEP;TFF3;OSOX1 | 0.79 | 0.71 | 0.86 | 0.55 | 0.07 | 0.87 | 0.74 | 1.00 | 0.76 | 0.67 | 0.85 |
| BP20;MMRN2;ECM1; TFF3;MCAM | 0.77 | 0.69 | 0.86 | 0.58 | 0.42 | 0.89 | 0.76 | 1.00 | 0.74 | 0.64 | 0.84 |
| BP20;MMRN2;ECM1; TFF3;COL6A3;PIGF | 0.77 | 0.69 | 0.85 | 0.47 | 0.32 | 0.95 | 0.91 | 0.99 | 0.72 | 0.62 | 0.81 |
| BP20;MMRN2;ECM1; ENG;TFF3;MCAM | 0.78 | 0.70 | 0.86 | 0.59 | 0.22 | 0.89 | 0.79 | 0.99 | 0.74 | 0.64 | 0.84 |
| BP20;MMRN2;ECM1; TFF3;IGFALS-e;MCAM | 0.80 | 0.72 | 0.87 | 0.58 | 0.37 | 0.92 | 0.82 | 1.00 | 0.76 | 0.66 | 0.85 |
| BP20;ECM1;TFF3; MCAM;PIGF | 0.78 | 0.69 | 0.86 | 0.45 | 0.03 | 0.88 | 0.77 | 0.99 | 0.74 | 0.64 | 0.84 |
| BP20;MMRN2;ECM1; LNPEP;TFF3;PIGF | 0.78 | 0.70 | 0.86 | 0.41 | 0.14 | 0.94 | 0.90 | 0.98 | 0.73 | 0.63 | 0.82 |
| MMRN2;ADAM12; TFF3;IGFALS;PIGF | 0.80 | 0.74 | 0.87 | 0.57 | 0.23 | 0.95 | 0.92 | 0.99 | 0.76 | 0.68 | 0.84 |
| MMRN2;ECM1;ENG; PRDX2;TFF3;SPINT1 | 0.78 | 0.70 | 0.86 | 0.36 | 0.31 | 0.96 | 0.92 | 1.00 | 0.73 | 0.63 | 0.82 |
| BP20;MMRN2;ECM1; TFF3;COL6A3;MCAM | 0.77 | 0.69 | 0.85 | 0.55 | 0.32 | 0.89 | 0.76 | 1.00 | 0.73 | 0.64 | 0.82 |
| BP20;MMRN2;ECM1; TFF3;COL6A3;PIGF | 0.77 | 0.70 | 0.85 | 0.38 | 0.27 | 0.94 | 0.90 | 0.99 | 0.72 | 0.63 | 0.81 |
| BP15;ADAM12;TFF3; IGFALS-e;MCAM;PIGF | 0.82 | 0.75 | 0.89 | 0.37 | 0.56 | 0.89 | 0.77 | 1.00 | 0.80 | 0.72 | 0.87 |
| BP20;ADAM12;TFF3; MCAM;PIGF | 0.81 | 0.73 | 0.88 | 0.54 | 0.12 | 0.87 | 0.75 | 0.99 | 0.78 | 0.70 | 0.87 |
| MMRN2;ADAM12; ECM1;TFF3;IGFALS-e; PIGF | 0.80 | 0.73 | 0.87 | 0.47 | 0.40 | 0.97 | 0.94 | 1.00 | 0.75 | 0.67 | 0.83 |
| BP20;ECM1;TFF3; IGFALS-e;MCAM | 0.79 | 0.71 | 0.86 | 0.51 | 0.16 | 0.83 | 0.67 | 1.00 | 0.77 | 0.68 | 0.86 |

(continued)

| Panel composition | BA | BAa | Bab | BB | BC | BD | BE | BF | BG | BH | BI |
|---|---|---|---|---|---|---|---|---|---|---|---|
| BP20;ECM1;TFF3; IGFALS-e;MCAM;PIGF | 0.79 | 0.72 | 0.87 | 0.41 | 0.36 | 0.87 | 0.75 | 0.99 | 0.77 | 0.68 | 0.85 |
| BP20;MMRN2;ECM1; TFF3;COL6A3;MCAM | 0.77 | 0.69 | 0.86 | 0.60 | 0.29 | 0.93 | 0.83 | 1.00 | 0.72 | 0.62 | 0.82 |
| BP20;ADAM12;LCAT; TFF3;IGFALS;MCAM | 0.79 | 0.72 | 0.87 | 0.55 | 0.28 | 0.85 | 0.70 | 0.99 | 0.77 | 0.69 | 0.86 |
| BP24;ECM1;LNPEP; TFF3;QSOX1;MCAM | 0.77 | 0.69 | 0.85 | 0.55 | 0.11 | 0.84 | 0.67 | 1.00 | 0.74 | 0.65 | 0.83 |
| BP20;ECM1;TFF3; MCAM;PIGF | 0.78 | 0.70 | 0.86 | 0.53 | 0.15 | 0.86 | 0.72 | 0.99 | 0.75 | 0.65 | 0.84 |
| BP20;LNPEP;TFF3; COL6A3;QSOX1; MCAM | 0.77 | 0.69 | 0.85 | 0.49 | 0.23 | 0.81 | 0.65 | 0.98 | 0.76 | 0.67 | 0.84 |
| BP20;MMRN2;LNPEP; TFF3;PIGF | 0.79 | 0.72 | 0.86 | 0.51 | 0.44 | 0.92 | 0.87 | 0.97 | 0.75 | 0.66 | 0.84 |
| BP20;ADAM12;TFF3; QSOX1;IGFALS;PIGF | 0.80 | 0.74 | 0.87 | 0.41 | 0.53 | 0.91 | 0.80 | 1.00 | 0.77 | 0.70 | 0.84 |
| MMRN2;ECM1;TFF3; IGFALS-e;PIGF | 0.79 | 0.72 | 0.86 | 0.47 | 0.39 | 0.96 | 0.91 | 1.00 | 0.73 | 0.65 | 0.82 |
| BP20;MMRN2;LNPEP; TFF3;COL6A3;PIGF | 0.79 | 0.71 | 0.86 | 0.58 | 0.31 | 0.95 | 0.90 | 0.99 | 0.74 | 0.65 | 0.83 |
| BP20;MMRN2;LNPEP; TFF3;PIGF | 0.79 | 0.72 | 0.86 | 0.58 | 0.33 | 0.90 | 0.83 | 0.98 | 0.75 | 0.67 | 0.84 |
| BP20;ADAM12;TFF3; QSOX1;IGFALS-e;PIGF | 0.81 | 0.74 | 0.88 | 0.32 | 0.12 | 0.90 | 0.76 | 1.00 | 0.78 | 0.71 | 0.86 |
| BP20;ADAM12;TFF3; MCAM;PIGF | 0.80 | 0.73 | 0.87 | 0.41 | 0.08 | 0.89 | 0.79 | 0.99 | 0.77 | 0.69 | 0.86 |
| BP20;LCAT;TFF3; IGFALS;MCAM;PIGF | 0.78 | 0.71 | 0.85 | 0.55 | 0.33 | 0.86 | 0.76 | 0.96 | 0.75 | 0.67 | 0.84 |
| BP15;MMRN2; ADAM12;TFF3;PIGF | 0.80 | 0.73 | 0.87 | 0.39 | 0.27 | 0.93 | 0.88 | 0.98 | 0.76 | 0.68 | 0.84 |
| BP20;ADAM12;TFF3; IGFALS-e;PIGF | 0.81 | 0.74 | 0.88 | 0.48 | 0.55 | 0.87 | 0.73 | 1.00 | 0.79 | 0.71 | 0.87 |
| BP20;MMRN2;ECM1; PRDX2;TFF3 | 0.78 | 0.70 | 0.86 | 0.52 | 0.34 | 0.88 | 0.79 | 0.97 | 0.74 | 0.65 | 0.84 |
| BP15;MMRN2; ADAM12;TFF3;PIGF | 0.80 | 0.73 | 0.87 | 0.43 | 0.40 | 0.93 | 0.88 | 0.98 | 0.76 | 0.68 | 0.84 |
| BP20;TFF3;CRP; IGFALS;MCAM;PIGF | 0.79 | 0.72 | 0.86 | 0.55 | 0.27 | 0.89 | 0.79 | 0.99 | 0.76 | 0.68 | 0.85 |
| BP15;MMRN2;ECM1; TFF3;COL6A3;MCAM | 0.78 | 0.70 | 0.86 | 0.66 | 0.14 | 0.90 | 0.77 | 1.00 | 0.74 | 0.64 | 0.84 |
| ADAM12;TFF3;QSOX1; IGFALS;MCAM;PIGF | 0.81 | 0.75 | 0.87 | 0.39 | 0.38 | 0.92 | 0.83 | 1.00 | 0.78 | 0.70 | 0.85 |
| BP20;ADAM12;TFF3; QSOX1;PIGF | 0.79 | 0.72 | 0.86 | 0.45 | 0.38 | 0.88 | 0.76 | 1.00 | 0.77 | 0.69 | 0.85 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;TFF3;PIGF | 0.80 | 0.73 | 0.87 | 0.50 | 0.41 | 0.93 | 0.88 | 0.98 | 0.76 | 0.68 | 0.84 |
| BP20;MMRN2;ECM1; TFF3;COL6A3;MCAM | 0.77 | 0.68 | 0.85 | 0.59 | 0.27 | 0.91 | 0.81 | 1.00 | 0.72 | 0.62 | 0.81 |
| MMRN2;ADAM12; ECM1;TFF3;SPINT1; PIGF | 0.77 | 0.68 | 0.85 | 0.55 | 0.24 | 0.98 | 0.96 | 1.00 | 0.70 | 0.61 | 0.80 |
| BP20;MMRN2;ECM1; TFF3;PIGF | 0.76 | 0.68 | 0.84 | 0.51 | 0.18 | 0.93 | 0.88 | 0.97 | 0.71 | 0.61 | 0.81 |
| BP20;MMRN2;ECM1; PRDX2;TFF3 | 0.78 | 0.70 | 0.86 | 0.67 | 0.04 | 0.91 | 0.84 | 0.97 | 0.73 | 0.63 | 0.83 |
| bmi;ADAM12;LCAT; TFF3;IGFALS;PIGF | 0.81 | 0.75 | 0.86 | 0.36 | 0.57 | 0.89 | 0.81 | 0.97 | 0.78 | 0.71 | 0.84 |
| BP20;MMRN2;ECM1: TFF3;COL6A3;PIGF | 0.77 | 0.69 | 0.85 | 0.44 | 0.30 | 0.94 | 0.91 | 0.98 | 0.71 | 0.61 | 0.81 |
| MMRN2;ECM1;ENG; TFF3;SPINT1;QSOX1 | 0.77 | 0.69 | 0.85 | 0.44 | 0.38 | 0.94 | 0.89 | 0.99 | 0.72 | 0.62 | 0.81 |
| bmi;ADAM12;TFF3; QSOX1;IGFALS-e;PIGF | 0.81 | 0.74 | 0.87 | 0.33 | 0.56 | 0.91 | 0.77 | 1.00 | 0.77 | 0.70 | 0.84 |
| MMRN2;ECM1;ENG; PRDX2;TFF3;MCAM | 0.76 | 0.68 | 0.85 | 0.47 | 0.06 | 0.88 | 0.74 | 1.00 | 0.72 | 0.62 | 0.82 |
| BP20;MMRN2; ADAM12;TFF3;PIGF | 0.80 | 0.72 | 0.87 | 0.45 | 0.26 | 0.95 | 0.91 | 0.98 | 0.75 | 0.66 | 0.83 |
| BR15;MMRN2;ECM1; TFF3;COL6A3;MCAM | 0.78 | 0.70 | 0.86 | 0.54 | 0.15 | 0.90 | 0.77 | 1.00 | 0.74 | 0.64 | 0.83 |
| BP20;ECM1;TFF3; MCAM | 0.76 | 0.68 | 0.85 | 0.61 | 0.03 | 0.83 | 0.66 | 0.99 | 0.74 | 0.65 | 0.84 |
| BP20;MMRN2;ECM1; TFF3;PIGF | 0.77 | 0.69 | 0.84 | 0.42 | 0.24 | 0.93 | 0.87 | 0.98 | 0.71 | 0.62 | 0.81 |
| BP20;MMRN2;ECM1; TFF3;MCAM | 0.77 | 0.69 | 0.85 | 0.60 | 0.28 | 0.90 | 0.79 | 1.00 | 0.73 | 0.62 | 0.83 |
| bmi;ADAM12;TFF3; CRP;MCAM;PIGF | 0.81 | 0.74 | 0.88 | 0.33 | 0.32 | 0.92 | 0.84 | 1.00 | 0.78 | 0.69 | 0.86 |
| BP20;ADAM12;TFF3; QSOX1;PIGF | 0.79 | 0.72 | 0.86 | 0.39 | 0.44 | 0.90 | 0.80 | 1.00 | 0.76 | 0.68 | 0.84 |
| MMRN2;ECM1;TFF3; IGFALS-e;PIGF | 0.78 | 0.71 | 0.85 | 0.49 | 0.42 | 0.96 | 0.92 | 1.00 | 0.73 | 0.65 | 0.81 |
| BP20;ECM1;TFF3; MCAM;PIGF | 0.77 | 0.69 | 0.85 | 0.38 | 0.02 | 0.89 | 0.80 | 0.98 | 0.73 | 0.63 | 0.83 |
| BP15;MMRN2; ADAM12;TFF3;PIGF | 0.80 | 0.73 | 0.86 | 0.48 | 0.38 | 0.93 | 0.88 | 0.98 | 0.75 | 0.67 | 0.83 |
| BP15;MMRN2;ECM1; TFF3;COL6A3;PIGF | 0.77 | 0.69 | 0.84 | 0.44 | 0.33 | 0.92 | 0.86 | 0.98 | 0.72 | 0.63 | 0.81 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;ECM1;TFF3; MCAM | 0.79 | 0.71 | 0.87 | 0.53 | 0.33 | 0.90 | 0.76 | 1.00 | 0.75 | 0.66 | 0.84 |
| BP20;MMRN2; ADAM12;ECM1;TFF3; QSOX1 | 0.79 | 0.71 | 0.86 | 0.50 | 0.31 | 0.90 | 0.79 | 1.00 | 0.75 | 0.66 | 0.83 |
| MMRN2;ADAM12; ECM1;TFF3;PIGF | 0.78 | 0.70 | 0.85 | 0.43 | 0.27 | 0.95 | 0.91 | 0.98 | 0.72 | 0.63 | 0.81 |
| BP15;MMRN2;ECM1; ENG;PRDX2;TFF3 | 0.75 | 0.66 | 0.84 | 0.56 | 0.08 | 0.85 | 0.70 | 1.00 | 0.72 | 0.62 | 0.82 |
| BP20;ENG;TFF3; QSOX1;ICFALS-e;PIGF | 0.78 | 0.70 | 0.86 | 0.60 | 0.11 | 0.87 | 0.73 | 1.00 | 0.75 | 0.66 | 0.85 |
| BP20;MMRN2;ECM1; LNPEP;TFF3;QSOX1 | 0.77 | 0.69 | 0.85 | 0.44 | 0.08 | 0.88 | 0.75 | 1.00 | 0.74 | 0.65 | 0.83 |
| BP15;MMRN2; ADAM12;TFF3;PIGF | 0.79 | 0.72 | 0.86 | 0.45 | 0.31 | 0.93 | 0.88 | 0.98 | 0.75 | 0.67 | 0.83 |
| BP20;MMRN2;ECM1; TFF3;MCAM | 0.76 | 0.68 | 0.84 | 0.55 | 0.05 | 0.86 | 0.72 | 1.00 | 0.73 | 0.63 | 0.82 |
| BP20;ECM1;TFF3; MCAM;PIGF | 0.76 | 0.68 | 0.85 | 0.44 | 0.02 | 0.89 | 0.78 | 0.99 | 0.72 | 0.62 | 0.83 |
| BP15;MMRN2; ADAM12;EGM1;TFF3; MCAM | 0.79 | 0.72 | 0.87 | 0.53 | 0.34 | 0.90 | 0.76 | 1.00 | 0.76 | 0.67 | 0.84 |
| BP20;ADAM12;TFF3; IGFALS-e;PIGF | 0.80 | 0.73 | 0.87 | 0.25 | 0.05 | 0.88 | 0.75 | 1.00 | 0.78 | 0.70 | 0.86 |
| bmi;MMRN2;ECM1; ENG;TFF3;PIGF | 0.74 | 0.65 | 0.83 | 0.51 | 0.21 | 0.87 | 0.72 | 1.00 | 0.70 | 0.60 | 0.80 |
| BP20;LNPEP;TFF3; CRP;MCAM;PIGF | 0.78 | 0.70 | 0.85 | 0.50 | 0.14 | 0.86 | 0.75 | 0.98 | 0.75 | 0.66 | 0.84 |
| BP15;MMRN2;ECM1; TFF3;COL6A3;MCAM | 0.77 | 0.69 | 0.86 | 0.60 | 0.19 | 0.90 | 0.79 | 1.00 | 0.73 | 0.63 | 0.83 |
| BP20;MMRN2;ECM1; TFF3;COL6A3;MCAM | 0.76 | 0.68 | 0.85 | 0.54 | 0.30 | 0.93 | 0.84 | 1.00 | 0.71 | 0.61 | 0.81 |
| BP15;ADAM12;TFF3; QSOX1;IGFALS;MCAM | 0.80 | 0.73 | 0.87 | 0.50 | 0.45 | 0.86 | 0.74 | 0.98 | 0.78 | 0.71 | 0.86 |
| BP15;MMRN2;ECM1; TFF3;COL6A3;MCAM | 0.77 | 0.69 | 0.85 | 0.49 | 0.42 | 0.90 | 0.77 | 1.00 | 0.73 | 0.64 | 0.82 |
| MMRN2;ADAM12; TFF3;IGFALS-e;PIGF | 0.80 | 0.73 | 0.86 | 0.50 | 0.41 | 0.94 | 0.87 | 1.00 | 0.75 | 0.67 | 0.83 |
| BP15;MMRN2; ADAM12;ECM1;TFF3; MCAM | 0.79 | 0.72 | 0.87 | 0.50 | 0.37 | 0.90 | 0.77 | 1.00 | 0.75 | 0.67 | 0.84 |
| BP15;MMRN2;ECM1; TFF3;COL6A3;MCAM | 0.77 | 0.69 | 0.85 | 0.61 | 0.16 | 0.90 | 0.79 | 1.00 | 0.73 | 0.63 | 0.83 |

(continued)

| Panel composition | BA | BAa | Bab | BB | BC | BD | BE | BF | BG | BH | BI |
|---|---|---|---|---|---|---|---|---|---|---|---|
| BP20;ECM1;TFF3; IGFALS-e;MCAM | 0.78 | 0.70 | 0.85 | 0.50 | 0.18 | 0.84 | 0.68 | 1.00 | 0.75 | 0.67 | 0.84 |
| BP20;ECM1;TFF3; COL6A3;MCAM | 0.76 | 0.68 | 0.84 | 0.52 | 0.17 | 0.84 | 0.68 | 1.00 | 0.73 | 0.64 | 0.82 |
| BP20;ECM1;LNPEP; TFF3:QSOX1;PIGF | 0.76 | 0.68 | 0.84 | 0.48 | 0.26 | 0.89 | 0.79 | 0.98 | 0.72 | 0.63 | 0.81 |
| MMRN2;ECM1;ENG; PRDX2;TFF3;QSOX1 | 0.74 | 0.66 | 0.83 | 0.48 | 0.03 | 0.88 | 0.73 | 1.00 | 0.70 | 0.60 | 0.80 |
| BP15;MMRN2;ECM1; TFF3;COL6A3;MCAM | 0.77 | 0.69 | 0.85 | 0.57 | 0.20 | 0.90 | 0.78 | 1.00 | 0.73 | 0.63 | 0.82 |
| BP15;MMRN2;ECM1; ENG;PRDX2;TFF3 | 0.75 | 0.66 | 0.83 | 0.46 | 0.10 | 0.85 | 0.70 | 1.00 | 0.71 | 0.61 | 0.81 |
| BP15;ADAM12;TFF3; MCAM;PIGF | 0.80 | 0.74 | 0.86 | 0.15 | 0.47 | 0.89 | 0.79 | 0.98 | 0.77 | 0.70 | 0.85 |
| BP20;MMRN2;ECM1; TFF3;PIGF | 0.76 | 0.68 | 0.84 | 0.40 | 0.18 | 0.93 | 0.89 | 0.97 | 0.70 | 0.60 | 0.80 |
| MMRN2;ADAM12; ECM1;TFF3;SPINT1; PIGF | 0.76 | 0.68 | 0.84 | 0.54 | 0.26 | 0.98 | 0.96 | 1.00 | 0.70 | 0.60 | 0.79 |
| BP15;MMRN2;ECM1; TFF3;COL6A3;PIGF | 0.77 | 0.69 | 0.84 | 0.42 | 0.31 | 0.92 | 0.85 | 0.98 | 0.72 | 0.63 | 0.81 |
| BP15;MMRN2; ADAM12;ECM1;TFF3; MCAM | 0.79 | 0.72 | 0.87 | 0.51 | 0.08 | 0.91 | 0.79 | 1.00 | 0.75 | 0.66 | 0.84 |
| BP20;ADAM12;TFF3; IGFALS-e;MCAM | 0.79 | 0.71 | 0.87 | 0.60 | 0.20 | 0.83 | 0.67 | 1.00 | 0.77 | 0.69 | 0.86 |
| BP20;ADAM12;TFF3; MCAM;PIGF | 0.80 | 0.73 | 0.87 | 0.39 | 0.15 | 0.91 | 0.82 | 0.99 | 0.76 | 0.68 | 0.85 |
| MMRN2;ECM1;ENG; TFF3;QSOX1;IGFALS-e | 0.77 | 0.69 | 0.85 | 0.48 | 0.33 | 0.93 | 0.88 | 0.98 | 0.72 | 0.62 | 0.81 |
| MMRN2;ADAM12; ECM1;TFF3;PIGF | 0.77 | 0.70 | 0.85 | 0.51 | 0.24 | 0.95 | 0.91 | 0.99 | 0.72 | 0.63 | 0.80 |
| BP15;ADAM12;TFF3; MCAM;PIGF | 0.80 | 0.74 | 0.87 | 0.37 | 0.50 | 0.88 | 0.78 | 0.98 | 0.77 | 0.70 | 0.85 |
| bmi;ADAM12;TFF3; COL6A3;QSOX1; MCAM | 0.80 | 0.73 | 0.86 | 0.40 | 0.34 | 0.90 | 0.80 | 1.00 | 0.76 | 0.68 | 0.84 |
| BP20;MMRN2;LNPEP; TFF3;PIGF | 0.78 | 0.70 | 0.85 | 0.40 | 0.27 | 0.93 | 0.88 | 0.97 | 0.73 | 0.64 | 0.82 |
| BP15;MMRN2;ECM1; TFF3;COL6A3;MCAM | 0.77 | 0.69 | 0.85 | 0.50 | 0.22 | 0.90 | 0.78 | 1.00 | 0.73 | 0.64 | 0.82 |
| BP15;MMRN2;ECM1; ENG;TFF3;SPINT1 | 0.76 | 0.68 | 0.85 | 0.36 | 0.05 | 0.89 | 0.82 | 0.96 | 0.72 | 0.62 | 0.83 |
| BP20;ECM1;TFF3; MCAM;PIGF | 0.76 | 0.68 | 0.84 | 0.41 | 0.05 | 0.86 | 0.74 | 0.99 | 0.73 | 0.63 | 0.83 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;ECM1;TFF3; MCAM | 0.79 | 0.72 | 0.87 | 0.50 | 0.15 | 0.91 | 0.79 | 1.00 | 0.75 | 0.67 | 0.84 |
| bmi;ADAM12;LCAT; TFF3;IGFALS;MCAM | 0.80 | 0.73 | 0.86 | 0.28 | 0.32 | 0.88 | 0.77 | 0.98 | 0.77 | 0.69 | 0.85 |
| MMRN2;ECM1;ENG; TFF3;SPINT1;QSOX1 | 0.76 | 0.68 | 0.84 | 0.48 | 0.43 | 0.95 | 0.90 | 1.00 | 0.71 | 0.62 | 0.80 |
| BP20;LNPEP;TFF3; MCAM;PIGF | 0.77 | 0.69 | 0.85 | 0.48 | 0.27 | 0.82 | 0.66 | 0.97 | 0.76 | 0.67 | 0.85 |
| BP20;ADAM12;TFF3; PIGF | 0.79 | 0.72 | 0.86 | 0.34 | 0.39 | 0.88 | 0.78 | 0.98 | 0.76 | 0.68 | 0.84 |
| BP20;MMRN2;ECM1; TFF3;MCAM | 0.76 | 0.67 | 0.84 | 0.56 | 0.04 | 0.89 | 0.77 | 1.00 | 0.71 | 0.61 | 0.82 |
| bmi;ADAM12;TFF3; IGFALS-e;PIGF | 0.80 | 0.73 | 0.87 | 0.17 | 0.56 | 0.88 | 0.75 | 1.00 | 0.77 | 0.70 | 0.85 |
| BP15;MMRN2;ECM1; TFF3;COL6A3;PIGF | 0.76 | 0.69 | 0.84 | 0.36 | 0.27 | 0.92 | 0.86 | 0.98 | 0.71 | 0.62 | 0.80 |
| BP20;MMRN2;ECM1; LNPEP;TFF3;QSOX1 | 0.76 | 0.68 | 0.84 | 0.48 | 0.14 | 0.91 | 0.80 | 1.00 | 0.72 | 0.62 | 0.81 |
| BP20;ADAM12;TFF3; MCAM | 0.78 | 0.70 | 0.86 | 0.52 | 0.11 | 0.83 | 0.67 | 1.00 | 0.76 | 0.67 | 0.85 |
| BP15;MMRN2;ECM1; ENG;TFF3;MCAM | 0.76 | 0.67 | 0.85 | 0.50 | 0.05 | 0.84 | 0.71 | 0.96 | 0.73 | 0.63 | 0.84 |
| BP15;ADAM12;TFF3; MCAM;PIGF | 0.80 | 0.74 | 0.87 | 0.39 | 0.42 | 0.90 | 0.81 | 0.98 | 0.77 | 0.69 | 0.85 |
| ECM1;ENG;PRDX2; TFF3;SPINT1;QSOX1 | 0.77 | 0.69 | 0.85 | 0.36 | 0.51 | 0.92 | 0.84 | 1.00 | 0.73 | 0.63 | 0.82 |
| bmi;ADAM12;TFF3; MCAM;PIGF | 0.80 | 0.73 | 0.87 | 0.41 | 0.25 | 0.91 | 0.83 | 0.99 | 0.77 | 0.69 | 0.85 |
| BP15;ADAM12;TFF3; IGFALS-e;PIGF | 0.80 | 0.73 | 0.87 | 0.16 | 0.68 | 0.86 | 0.73 | 1.00 | 0.78 | 0.70 | 0.85 |
| BP15;MMRN2;ECM1; ENG;TFF3;MCAM | 0.76 | 0.68 | 0.84 | 0.47 | 0.31 | 0.83 | 0.71 | 0.96 | 0.74 | 0.63 | 0.84 |
| BP20;ADAM12;TFF3; QSOX1;IGFALS | 0.78 | 0.71 | 0.86 | 0.51 | 0.19 | 0.84 | 0.70 | 0.98 | 0.76 | 0.68 | 0.85 |
| BP20;MMRN2;LNPEP; TFF3;COL6A3;PIGF | 0.77 | 0.70 | 0.85 | 0.47 | 0.29 | 0.95 | 0.92 | 0.98 | 0.72 | 0.63 | 0.81 |
| BP20;MMRN2;ECM1; PRDX2;TFF3 | 0.77 | 0.69 | 0.85 | 0.44 | 0.03 | 0.91 | 0.86 | 0.96 | 0.72 | 0.62 | 0.82 |
| BP15;ADAM12;TFF3; IGFALS-e;PIGF | 0.80 | 0.73 | 0.86 | 0.14 | 0.58 | 0.87 | 0.73 | 1.00 | 0.77 | 0.70 | 0.85 |
| BP20;ADAM12;TFF3; PIGF | 0.79 | 0.72 | 0.86 | 0.36 | 0.41 | 0.86 | 0.74 | 0.99 | 0.76 | 0.68 | 0.85 |
| BP15;MMRN2;ECM1; TFF3;MCAM | 0.77 | 0.68 | 0.85 | 0.56 | 0.05 | 0.86 | 0.72 | 1.00 | 0.74 | 0.64 | 0.84 |

(continued)

| Panel composition | BA | BAa | Bab | BB | BC | BD | BE | BF | BG | BH | BI |
|---|---|---|---|---|---|---|---|---|---|---|---|
| BP15;MMRN2;ECM1; ENG;TFF3;MCAM | 0.76 | 0.68 | 0.84 | 0.47 | 0.39 | 0.85 | 0.73 | 0.97 | 0.73 | 0.63 | 0.83 |
| BP20;ECM1;LNPEP; TFF3;QSOX1;PIGF | 0.76 | 0.68 | 0.84 | 0.48 | 0.29 | 0.87 | 0.74 | 0.99 | 0.73 | 0.63 | 0.82 |
| BP20;MMRN2;ECM1; TFF3 | 0.76 | 0.67 | 0.84 | 0.49 | 0.04 | 0.87 | 0.74 | 0.99 | 0.72 | 0.62 | 0.82 |
| BP15;MMRN2;ECM1; ENG;TFF3;SPINT1 | 0.76 | 0.68 | 0.85 | 0.36 | 0.15 | 0.89 | 0.81 | 0.97 | 0.73 | 0.63 | 0.83 |
| bmi;ECMI;TFF3;CRP; MCAM;PIGF | 0.78 | 0.71 | 0.85 | 0.26 | 0.60 | 0.92 | 0.86 | 0.98 | 0.73 | 0.64 | 0.82 |
| BP20;ECM1;TFF3; MCAM | 0.76 | 0.67 | 0.84 | 0.60 | 0.03 | 0.86 | 0.73 | 1.00 | 0.72 | 0.62 | 0.82 |
| ECM1;ENG;TFF3; QSQX1;IGFALS-e; MCAM | 0.77 | 0.70 | 0.85 | 0.23 | 0.35 | 0.88 | 0.77 | 0.99 | 0.74 | 0.65 | 0.83 |
| BP15;ADAM12;TFF3; MCAM;PIGF | 0.80 | 0.73 | 0.86 | 0.15 | 0.41 | 0.90 | 0.82 | 0.98 | 0.76 | 0.69 | 0.84 |
| MMRN2;ECM1;TFF3; IGFALS;MCAM | 0.78 | 0.71 | 0.85 | 0.39 | 0.44 | 0.91 | 0.82 | 1.00 | 0.74 | 0.65 | 0.82 |
| BP15;MMRN2; ADAM12;ECM1;TFF3; QSOX1 | 0.78 | 0.71 | 0.85 | 0.41 | 0.41 | 0.89 | 0.79 | 1.00 | 0.74 | 0.66 | 0.82 |
| BP15;MMRN2; ADAM12;ECM1;TFF3; MCAM | 0.78 | 0.71 | 0.86 | 0.46 | 0.31 | 0.90 | 0.77 | 1.00 | 0.74 | 0.66 | 0.83 |
| bmi;MMRN2;ADAM12; TFF3;COL6A3;MCAM | 0.79 | 0.72 | 0.87 | 0.50 | 0.19 | 0.94 | 0.87 | 1.00 | 0.75 | 0.66 | 0.83 |
| bmi;ADAM12;LCAT; TFF3;IGFALS;PIGF | 0.80 | 0.74 | 0.86 | 0.26 | 0.46 | 0.88 | 0.77 | 0.99 | 0.77 | 0.70 | 0.84 |
| BP15;MMRN2;ECM1; ENG;TFF3;MCAM | 0.76 | 0.68 | 0.85 | 0.51 | 0.34 | 0.87 | 0.76 | 0.97 | 0.73 | 0.62 | 0.83 |
| ECM1;ENG;TFF3; SPINT1;QSOX1; IGFALS-e | 0.78 | 0.70 | 0.85 | 0.45 | 0.36 | 0.90 | 0.75 | 1.00 | 0.74 | 0.66 | 0.83 |
| MMRN2;ADAM12; ECM1;TFF3;QSOX1; IGFALS-e | 0.78 | 0.71 | 0.86 | 0.43 | 0.17 | 0.94 | 0.89 | 1.00 | 0.73 | 0.65 | 0.82 |
| BP15:ADAM12;PRDX2; TFF3;SPINT1;QSOX1 | 0.78 | 0.71 | 0.85 | 0.44 | 0.38 | 0.92 | 0.83 | 1.00 | 0.74 | 0.66 | 0.83 |
| bmi;ECM1;TFF3;CRP; MCAM;PIGF | 0.78 | 0.70 | 0.85 | 0.26 | 0.18 | 0.91 | 0.84 | 0.98 | 0.73 | 0.64 | 0.83 |
| BP15;MMRN2; ADAM12;ECM1;TFF3; QSOX1 | 0.77 | 0.70 | 0.84 | 0.30 | 0.39 | 0.89 | 0.78 | 0.99 | 0.73 | 0.65 | 0.82 |
| BP15;MMRN2;ECM1; TFF3;MCAM | 0.77 | 0.69 | 0.85 | 0.41 | 0.04 | 0.85 | 0.71 | 1.00 | 0.74 | 0.64 | 0.83 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;ECM1;TFF3; MCAM | 0.79 | 0.71 | 0.86 | 0.48 | 0.18 | 0.91 | 0.80 | 1.00 | 0.75 | 0.66 | 0.83 |
| BP20;ECM1;TFF3; MCAM | 0.75 | 0.67 | 0.83 | 0.55 | 0.04 | 0.83 | 0.66 | 1.00 | 0.72 | 0.63 | 0.82 |
| BP15;MMRN2; ADAM12;ECM1;TFF3; MCAM | 0.79 | 0.71 | 0.86 | 0.41 | 0.31 | 0.90 | 0.78 | 1.00 | 0.75 | 0.66 | 0.83 |
| BP15;MMRN2;ECM1; TFF3;COL6A3;MCAM | 0.76 | 0.69 | 0.84 | 0.45 | 0.29 | 0.90 | 0.79 | 1.00 | 0.72 | 0.63 | 0.81 |
| BP15;MMRN2;ECM1; TFF3;COL6A3;MCAM | 0.77 | 0.69 | 0.85 | 0.59 | 0.17 | 0.90 | 0.79 | 1.00 | 0.72 | 0.62 | 0.81 |
| BP15;MMRN2; ADAM12;ECM1;TFF3; MCAM | 0.79 | 0.71 | 0.86 | 0.44 | 0.30 | 0.90 | 0.76 | 1.00 | 0.75 | 0.66 | 0.83 |
| ECM1;ENG;PRDX2; TFF3;SPINT1;QSOX1 | 0.77 | 0.69 | 0.84 | 0.33 | 0.43 | 0.94 | 0.87 | 1.00 | 0.72 | 0.63 | 0.81 |
| BP15;MMRN2;ECM1; TFF3;COL6A3;MCAM | 0.76 | 0.69 | 0.84 | 0.48 | 0.28 | 0.91 | 0.80 | 1.00 | 0.72 | 0.62 | 0.81 |
| BP15;MMRN2;ECM1; ENG;TFF3;SPINT1 | 0.76 | 0.68 | 0.84 | 0.34 | 0.21 | 0.90 | 0.83 | 0.97 | 0.72 | 0.62 | 0.82 |
| bmi;ADAM12;ENG; TFF3;SPINT1;QSOX1 | 0.79 | 0.71 | 0.86 | 0.34 | 0.19 | 0.89 | 0.74 | 1.00 | 0.76 | 0.68 | 0.84 |
| BP15;ADAM12;PRDX2; TFF3;SPINT1;QSOX1 | 0.78 | 0.71 | 0.85 | 0.41 | 0.43 | 0.91 | 0.83 | 0.99 | 0.74 | 0.66 | 0.82 |
| BP20;MMRN2;TFF3; COL6A3;PlGF | 0.76 | 0.68 | 0.84 | 0.59 | 0.14 | 0.94 | 0.91 | 0.98 | 0.71 | 0.61 | 0.80 |
| MMRN2;ECM1;ENG; PRDX2;TFF3 | 0.73 | 0.64 | 0.82 | 0.44 | 0.05 | 0.86 | 0.71 | 1.00 | 0.68 | 0.58 | 0.79 |
| BP15;ADAM12;TFF3; SPINT1;QSOX1;PlGF | 0.78 | 0.71 | 0.85 | 0.40 | 0.33 | 0.90 | 0.78 | 1.00 | 0.74 | 0.66 | 0.82 |
| bmi;MMRN2;ECM1; TFF3;COL6A3;MCAM | 0.76 | 0.69 | 0.84 | 0.45 | 0.27 | 0.91 | 0.81 | 1.00 | 0.72 | 0.62 | 0.81 |
| BP20;MMRN2,TFF3; COL6A3;PlGF | 0.76 | 0.69 | 0.84 | 0.57 | 0.15 | 0.93 | 0.88 | 0.98 | 0.71 | 0.62 | 0.81 |
| BP15;ADAM12;TFF3; QSOX1;PlGF | 0.79 | 0.72 | 0.85 | 0.23 | 0.36 | 0.87 | 0.76 | 0.98 | 0.76 | 0.68 | 0.83 |
| ADAM12;TFF3;IGFALS; MCAM;PlGF | 0.80 | 0.73 | 0.86 | 0.20 | 0.28 | 0.90 | 0.82 | 0.99 | 0.76 | 0.68 | 0.84 |
| BP15;ECM1;TFF3; COL6A3;MCAM;PlGF | 0.77 | 0.69 | 0.84 | 0.20 | 0.46 | 0.91 | 0.83 | 0.98 | 0.72 | 0.63 | 0.81 |
| BP20;ECM1;TFF3; MCAM | 0.75 | 0.66 | 0.84 | 0.56 | 0.04 | 0.85 | 0.70 | 1.00 | 0.72 | 0.61 | 0.82 |
| BP15;ADAM12;TFF3; QSOX1;PlGF | 0.78 | 0.72 | 0.85 | 0.23 | 0.37 | 0.87 | 0.77 | 0.98 | 0.75 | 0.68 | 0.82 |

(continued)

| Panel composition | BA | BAa | Bab | BB | BC | BD | BE | BF | BG | BH | BI |
|---|---|---|---|---|---|---|---|---|---|---|---|
| ADAM12;TFF3;QSOX1; IGFALS-e;PIGF | 0.79 | 0.72 | 0.85 | 0.25 | 0.42 | 0.90 | 0.77 | 1.00 | 0.75 | 0.68 | 0.82 |
| bmi;ADAM12;TFF3; QSOX1;PIGF | 0.79 | 0.72 | 0.86 | 0.43 | 0.35 | 0.92 | 0.83 | 1.00 | 0.75 | 0.67 | 0.83 |
| MMRN2;ADAM12; ECM1;TFF3;SPINT1; QSOX1 | 0.75 | 0.67 | 0.83 | 0.40 | 0.29 | 0.95 | 0.91 | 0.99 | 0.69 | 0.60 | 0.78 |
| BP20;MMRN2;ECM1; TFF3;COL6A3 | 0.75 | 0.67 | 0.83 | 0.60 | 0.14 | 0.90 | 0.80 | 1.00 | 0.70 | 0.60 | 0.80 |
| BP20;ECM1;ENG; TFF3;SPINT1;QSOX1 | 0.76 | 0.68 | 0.84 | 0.40 | 0.34 | 0.90 | 0.81 | 0.99 | 0.72 | 0.62 | 0.81 |
| BP15;MMRN2;ECM1; TFF3;COL6A3;MCAM | 0.76 | 0.68 | 0.84 | 0.51 | 0.24 | 0.91 | 0.80 | 1.00 | 0.71 | 0.62 | 0.81 |
| BP15;MMRN2;ECM1; TFF3;MCAM | 0.77 | 0.68 | 0.85 | 0.43 | 0.17 | 0.87 | 0.74 | 1.00 | 0.73 | 0.63 | 0.83 |
| BP15;MMRN2;ECM1; TFF3;MCAM | 0.76 | 0.68 | 0.84 | 0.44 | 0.42 | 0.86 | 0.71 | 1.00 | 0.73 | 0.64 | 0.83 |
| BP20;ECM1;ENG; TFF3;QSOX1;PIGF | 0.74 | 0.66 | 0.82 | 0.40 | 0.18 | 0.86 | 0.77 | 0.96 | 0.70 | 0.60 | 0.80 |
| BP15;MMRN2;ECM1; TFF3;COL6A3;IGFALS-e | 0.76 | 0.69 | 0.84 | 0.54 | 0.43 | 0.91 | 0.82 | 1.00 | 0.72 | 0.63 | 0.80 |
| BP15;ECM1;TFF3; COL6A3;MCAM;PIGF | 0.76 | 0.69 | 0.84 | 0.24 | 0.32 | 0.88 | 0.78 | 0.99 | 0.72 | 0.63 | 0.81 |
| MMRN2;ECM1;ENG; PRDX2;TFF3;MCAM | 0.75 | 0.66 | 0.83 | 0.54 | 0.05 | 0.88 | 0.75 | 1.00 | 0.71 | 0.60 | 0.81 |
| BP15;ADAM12;TFF3; MCAM;PIGF | 0.79 | 0.72 | 0.86 | 0.22 | 0.52 | 0.88 | 0.77 | 0.98 | 0.76 | 0.68 | 0.84 |
| BP15;MMRN2;ECM1; TFF3;COL6A3;IGFALS-e | 0.77 | 0.69 | 0.84 | 0.52 | 0.41 | 0.91 | 0.82 | 1.00 | 0.72 | 0.63 | 0.81 |
| MMRN2;ADAM12; TFF3;PIGF | 0.77 | 0.70 | 0.85 | 0.52 | 0.22 | 0.95 | 0.92 | 0.98 | 0.72 | 0.63 | 0.80 |
| BP20;MMRN2;ECM1; TFF3;MCAM | 0.75 | 0.67 | 0.84 | 0.56 | 0.04 | 0.90 | 0.79 | 1.00 | 0.70 | 0.60 | 0.81 |
| bmi;MMRN2;ADAM12; TFF3;COL6A3;QSOX1 | 0.78 | 0.70 | 0.85 | 0.30 | 0.27 | 0.93 | 0.87 | 0.99 | 0.73 | 0.64 | 0.81 |
| ADAM12;TFF3;IGFALS-e;MCAM;PIGF | 0.80 | 0.73 | 0.87 | 0.24 | 0.18 | 0.89 | 0.76 | 1.00 | 0.76 | 0.68 | 0.84 |
| bmi;ADAM12;TFF3; QSOX1;IGFALS | 0.78 | 0.72 | 0.85 | 0.47 | 0.43 | 0.87 | 0.76 | 0.97 | 0.75 | 0.68 | 0.83 |
| BP20;ENG;PRDX2; TFF3;QSOX1 | 0.75 | 0.67 | 0.83 | 0.44 | 0.03 | 0.82 | 0.71 | 0.94 | 0.72 | 0.62 | 0.82 |
| BP20;ADAM12;TFF3; MCAM | 0.78 | 0.70 | 0.85 | 0.37 | 0.10 | 0.86 | 0.72 | 1.00 | 0.75 | 0.66 | 0.84 |

(continued)

| Panel composition | BA | BAa | Bab | BB | BC | BD | BE | BF | BG | BH | BI |
|---|---|---|---|---|---|---|---|---|---|---|---|
| BP15;MMRN2;ECM1; TFF3;MCAM | 0.76 | 0.68 | 0.84 | 0.39 | 0.15 | 0.86 | 0.72 | 0.99 | 0.73 | 0.63 | 0.83 |
| MMRN2;ECM1;ENG; TFF3;SPINT1 | 0.75 | 0.66 | 0.83 | 0.51 | 0.21 | 0.94 | 0.87 | 1.00 | 0.69 | 0.59 | 0.80 |
| BP20;ADAM12;TFF3; QSOX9;IGFALS-e | 0.78 | 0.70 | 0.85 | 0.49 | 0.18 | 0.83 | 0.67 | 0.99 | 0.76 | 0.68 | 0.85 |
| MMRN2;ECM1;TFF3; PIGF | 0.75 | 0.67 | 0.83 | 0.47 | 0.26 | 0.91 | 0.85 | 0.98 | 0.69 | 0.60 | 0.79 |
| BP15;ADAM12;PRDX2; TFF3;SPINT1;QSOX1 | 0.77 | 0.70 | 0.84 | 0.41 | 0.18 | 0.91 | 0.84 | 0.99 | 0.73 | 0.64 | 0.81 |
| BP20;ADAM12;TFF3; PIGF | 0.78 | 0.71 | 0.85 | 0.18 | 0.40 | 0.88 | 0.78 | 0.98 | 0.75 | 0.67 | 0.83 |
| BP20;MMRN2;ECM1; TFF3 | 0.75 | 0.66 | 0.83 | 0.35 | 0.28 | 0.87 | 0.76 | 0.97 | 0.71 | 0.61 | 0.81 |
| BP20;ECM1;ENG; TFF3;MCAM | 0.76 | 0.67 | 0.84 | 0.49 | 0.19 | 0.83 | 0.69 | 0.97 | 0.73 | 0.63 | 0.83 |
| BP15;MMRN2;ECM1; TFF3;COL6A3;MCAM | 0.76 | 0.68 | 0.84 | 0.43 | 0.27 | 0.91 | 0.81 | 1.00 | 0.71 | 0.62 | 0.80 |
| BP20;MMRN2;TFF3; PIGF | 0.76 | 0.68 | 0.84 | 0.39 | 0.01 | 0.91 | 0.86 | 0.95 | 0.71 | 0.62 | 0.81 |
| BP15;ECM1;TFF3; COL6A3;MCAM | 0.76 | 0.67 | 0.84 | 0.29 | 0.04 | 0.85 | 0.70 | 0.99 | 0.73 | 0.63 | 0.83 |
| BP15;MMRN2;ECM1; TFF3;COL6A3;IGFALS-e | 0.77 | 0.69 | 0.84 | 0.52 | 0.38 | 0.92 | 0.83 | 1.00 | 0.72 | 0.63 | 0.80 |
| MMRN2;TFF3; MAPRE1/3;COL6A3; ALDOA;PIGF | 0.75 | 0.66 | 0.84 | 0.43 | 0.27 | 0.95 | 0.91 | 0.99 | 0.69 | 0.59 | 0.80 |
| BP15;MMRN2;ECM1; TFF3;COL6A3 | 0.75 | 0.66 | 0.83 | 0.45 | 0.07 | 0.85 | 0.71 | 0.98 | 0.72 | 0.61 | 0.82 |
| BP15;MMRN2; ADAM12;ECM1;TFF3; MCAM | 0.78 | 0.71 | 0.86 | 0.48 | 0.15 | 0.92 | 0.80 | 1.00 | 0.74 | 0.65 | 0.83 |
| BP15;ECM1;ENG; PRDX2;TFF3;QSOX1 | 0.74 | 0.65 | 0.82 | 0.12 | 0.47 | 0.82 | 0.69 | 0.94 | 0.71 | 0.61 | 0.81 |
| BP15;MMRN2; ADAM12;TFF3;COL6A3 | 0.77 | 0.69 | 0.84 | 0.47 | 0.06 | 0.90 | 0.82 | 0.99 | 0.73 | 0.63 | 0.82 |
| BP15;MMRN2; ADAM12;TFF3;COL6A3 | 0.77 | 0.69 | 0.84 | 0.40 | 0.42 | 0.90 | 0.81 | 0.98 | 0.73 | 0.64 | 0.82 |
| BP15;ADAM12;TFF3; QSOX1;PIGF | 0.78 | 0.72 | 0.84 | 0.30 | 0.39 | 0.87 | 0.76 | 0.97 | 0.75 | 0.68 | 0.83 |
| ADAM12;TFF3;SPINT1; IGFALS-e;PIGF | 0.78 | 0.70 | 0.85 | 0.23 | 0.11 | 0.90 | 0.73 | 1.00 | 0.74 | 0.66 | 0.82 |
| BP15;ECM1;TFF3; COL6A3;MCAM | 0.76 | 0.67 | 0.84 | 0.29 | 0.05 | 0.85 | 0.70 | 1.00 | 0.73 | 0.63 | 0.82 |

(continued)

| Panel composition | BA | BAa | Bab | BB | BC | BD | BE | BF | BG | BH | BI |
|---|---|---|---|---|---|---|---|---|---|---|---|
| ADAM12;TFF3;QSOX1; IGFALS;PIGF | 0.78 | 0.72 | 0.85 | 0.11 | 0.44 | 0.90 | 0.79 | 1.00 | 0.75 | 0.68 | 0.82 |
| ECM1;ENG;TFF3; SPINT1;QSOX1; IGFALS-e | 0.77 | 0.69 | 0.85 | 0.45 | 0.31 | 0.90 | 0.74 | 1.00 | 0.73 | 0.65 | 0.82 |
| BP15;MMRN2;ECM1; PRDX2;TFF3 | 0.75 | 0.67 | 0.84 | 0.48 | 0.15 | 0.84 | 0.73 | 0.94 | 0.73 | 0.63 | 0.83 |
| BP15;MMRN2;ECM1; ENG;PRDX2;TFF3 | 0.73 | 0.65 | 0.82 | 0.51 | 0.11 | 0.84 | 0.70 | 0.99 | 0.70 | 0.60 | 0.80 |
| BP15;MMRN2; ADAM12;TFF3;COL6A3 | 0.77 | 0.70 | 0.85 | 0.38 | 0.38 | 0.90 | 0.80 | 0.99 | 0.73 | 0.64 | 0.82 |
| ADAM12;TFF3;IGFALS-e;PIGF | 0.78 | 0.72 | 0.85 | 0.14 | 0.13 | 0.88 | 0.75 | 1.00 | 0.75 | 0.68 | 0.83 |
| MMRN2;ECM1;TFF3; COL6A3;MCAM | 0.75 | 0.67 | 0.83 | 0.44 | 0.06 | 0.91 | 0.81 | 1.00 | 0.70 | 0.60 | 0.80 |
| BP15;MMRN2;ENG; PRDX2;TFF3;PIGF | 0.74 | 0.66 | 0.83 | 0.41 | 0.03 | 0.85 | 0.70 | 1.00 | 0.71 | 0.61 | 0.81 |
| BP15;ADAM12;ENG; TFF3;SPINT1;QSOX1 | 0.77 | 0.70 | 0.85 | 0.45 | 0.44 | 0.86 | 0.71 | 1.00 | 0.75 | 0.67 | 0.83 |
| BP15;ADAM12;TFF3; QSOX1;IGFALS-e | 0.77 | 0.70 | 0.84 | 0.24 | 0.37 | 0.84 | 0.69 | 0.99 | 0.75 | 0.68 | 0.83 |
| BP15;MMRN2; ADAM12;ECM1;TFF3; MCAM | 0.78 | 0.71 | 0.86 | 0.49 | 0.18 | 0.92 | 0.81 | 1.00 | 0.74 | 0.65 | 0.82 |
| ADAM12;TFF3;QSOX1; IGFALS;MCAM | 0.78 | 0.71 | 0.85 | 0.31 | 0.29 | 0.88 | 0.79 | 0.97 | 0.75 | 0.67 | 0.83 |
| BP20;LNPEP;TFF3; QSOX1;PIGF | 0.76 | 0.68 | 0.84 | 0.40 | 0.09 | 0.82 | 0.68 | 0.96 | 0.74 | 0.65 | 0.83 |
| BP15;ADAM12;TFF3; IGFALS-e;MCAM | 0.78 | 0.71 | 0.86 | 0.33 | 0.20 | 0.84 | 0.68 | 1.00 | 0.76 | 0.68 | 0.84 |
| BP15;ADAM12;TFF3; IGFALS-e;MCAM | 0.79 | 0.71 | 0.86 | 0.43 | 0.27 | 0.84 | 0.68 | 1.00 | 0.77 | 0.69 | 0.85 |
| BP20;LNPEP;TFF3; MCAM;PIGF | 0.76 | 0.69 | 0.84 | 0.26 | 0.28 | 0.86 | 0.75 | 0.96 | 0.73 | 0.64 | 0.82 |
| BP15;ADAM12;TFF3; PIGF | 0.78 | 0.71 | 0.84 | 0.11 | 0.40 | 0.84 | 0.72 | 0.96 | 0.76 | 0.68 | 0.83 |
| BP15;MMRN2;LNPEP; PRDX2;TFF3;PIGF | 0.77 | 0.70 | 0.85 | 0.42 | 0.34 | 0.92 | 0.86 | 0.98 | 0.73 | 0.64 | 0.81 |
| BP15;ADAM12;ENG; TFF3;SPINT1;QSOX1 | 0.77 | 0.70 | 0.85 | 0.33 | 0.42 | 0.86 | 0.71 | 1.00 | 0.75 | 0.67 | 0.83 |
| BP15;MMRN2; ADAM12;TFF3;IGFALS-e | 0.77 | 0.70 | 0.85 | 0.47 | 0.08 | 0.91 | 0.83 | 0.99 | 0.73 | 0.64 | 0.82 |
| BP15;MMRN2; ADAM12;TFF3;COL6A3 | 0.77 | 0.69 | 0.85 | 0.48 | 0.05 | 0.92 | 0.84 | 0.99 | 0.72 | 0.63 | 0.81 |

(continued)

| Panel composition | BA | BAa | Bab | BB | BC | BD | BE | BF | BG | BH | BI |
|---|---|---|---|---|---|---|---|---|---|---|---|
| BP15;MMRN2; ADAM12;PRDX2;TFF3 | 0.77 | 0.69 | 0.85 | 0.49 | 0.13 | 0.92 | 0.86 | 0.97 | 0.72 | 0.63 | 0.82 |
| ECM1;TFF3;IGFALS-e; MCAM;PlGF | 0.77 | 0.70 | 0.84 | 0.25 | 0.35 | 0.89 | 0.78 | 0.99 | 0.73 | 0.64 | 0.81 |
| BP15;ADAM12;TFF3; QSOX1;IGFALS | 0.78 | 0.71 | 0.84 | 0.02 | 0.38 | 0.85 | 0.74 | 0.95 | 0.75 | 0.68 | 0.83 |
| BP15;ADAM12;PRDX2; TFF3;SPINT1;QSOX1 | 0.77 | 0.69 | 0.85 | 0.43 | 0.16 | 0.92 | 0.84 | 1.00 | 0.73 | 0.64 | 0.82 |
| BP20;ADAM12;TFF3; QSOX1 | 0.77 | 0.69 | 0.84 | 0.47 | 0.10 | 0.82 | 0.66 | 0.98 | 0.75 | 0.66 | 0.84 |
| BP15;ADAM12;TFF3; IGFALS-e;MCAM | 0.79 | 0.71 | 0.86 | 0.38 | 0.14 | 0.85 | 0.70 | 1.00 | 0.76 | 0.68 | 0.85 |
| BP15;MMRN2; ADAM12;ECM1;TFF3 | 0.76 | 0.68 | 0.84 | 0.44 | 0.17 | 0.86 | 0.72 | 0.99 | 0.73 | 0.64 | 0.82 |
| BP15;ADAM12;PRDX2; TFF3;QSOX1;ENPP2 | 0.77 | 0.70 | 0.85 | 0.36 | 0.18 | 0.91 | 0.85 | 0.97 | 0.73 | 0.64 | 0.82 |
| BP15;MMRN2;ECM1; ENG;PRDX2;TFF3 | 0.73 | 0.64 | 0.82 | 0.53 | 0.11 | 0.85 | 0.70 | 1.00 | 0.69 | 0.59 | 0.80 |
| bmi;ADAM12;TFF3; QSOX1;IGFALS-e | 0.78 | 0.71 | 0.84 | 0.23 | 0.40 | 0.86 | 0.72 | 1.00 | 0.75 | 0.67 | 0.82 |
| MMRN2;TFF3;IGFALS-e;PlGF | 0.77 | 0.70 | 0.85 | 0.48 | 0.26 | 0.93 | 0.83 | 1.00 | 0.72 | 0.64 | 0.81 |
| ECM1;ENG;PRDX2; TFF3;5PINT1;QSOX1 | 0.76 | 0.68 | 0.84 | 0.45 | 0.34 | 0.93 | 0.86 | 1.00 | 0.71 | 0.61 | 0.80 |
| BP15;ECM1;TFF3; COL6A3;MCAM | 0.75 | 0.67 | 0.83 | 0.17 | 0.08 | 0.85 | 0.70 | 0.99 | 0.72 | 0.63 | 0.82 |
| BP20;MMRN2;ECM1; TFF3;COL6A3 | 0.74 | 0.66 | 0.83 | 0.51 | 0.21 | 0.91 | 0.82 | 1.00 | 0.69 | 0.59 | 0.79 |
| BP20;TFF3;COL6A3; MCAM | 0.74 | 0.66 | 0.82 | 0.51 | 0.16 | 0.82 | 0.66 | 0.98 | 0.72 | 0.63 | 0.81 |
| BP15;MMRN2; ADAM12;ECM1;TFF3 | 0.76 | 0.68 | 0.84 | 0.44 | 0.32 | 0.86 | 0.72 | 0.99 | 0.73 | 0.64 | 0.82 |
| BP15;MMRN2; ADAM12;TFF3;COL6A3 | 0.76 | 0.69 | 0.84 | 0.47 | 0.25 | 0.90 | 0.80 | 0.99 | 0.72 | 0.63 | 0.81 |
| BP20;MMRN2;PRDX2; TFF3 | 0.76 | 0.68 | 0.84 | 0.48 | 0.01 | 0.90 | 0.85 | 0.96 | 0.72 | 0.62 | 0.81 |
| BP15;ECM1;ENG; TFF3;SPINT1;QSOX1 | 0.76 | 0.67 | 0.84 | 0.36 | 0.24 | 0.82 | 0.69 | 0.96 | 0.74 | 0.64 | 0.84 |
| BP15;ADAM12;ECM1; TFF3;SPINT1;QSOX1 | 0.75 | 0.67 | 0.82 | 0.45 | 0.38 | 0.88 | 0.79 | 0.97 | 0.71 | 0.62 | 0.80 |
| BP20;MMRN2;TFF3; COL6A3;PlGF | 0.75 | 0.67 | 0.84 | 0.57 | 0.17 | 0.95 | 0.91 | 0.98 | 0.69 | 0.59 | 0.79 |
| BP15;ECM1;TFF3; COL6A3;MCAM | 0.75 | 0.67 | 0.83 | 0.19 | 0.08 | 0.85 | 0.71 | 0.99 | 0.72 | 0.63 | 0.81 |

(continued)

| Panel composition | BA | BAa | Bab | BB | BC | BD | BE | BF | BG | BH | BI |
|---|---|---|---|---|---|---|---|---|---|---|---|
| BP20;ECM1;TFF3; MCAM | 0.74 | 0.66 | 0.83 | 0.56 | 0.03 | 0.86 | 0.73 | 1.00 | 0.70 | 0.60 | 0.81 |
| BP15;MMRN2; ADAM12;ECM1;TFF3 | 0.75 | 0.67 | 0.83 | **0.47** | **0.29** | **0.86** | **0.72** | 1.00 | 0.72 | 0.63 | 0.81 |
| bmi;ENG;PRDX2;TFF3; QSOX1 | 0.74 | 0.66 | 0.82 | 0.15 | 0.30 | 0.80 | 0.63 | 0.97 | 0.72 | 0.63 | 0.81 |
| BP15;MMRN2;ECM1; TFF3;COL6A3 | 0.75 | 0.66 | 0.83 | 0.45 | 0.15 | 0.84 | 0.70 | 0.97 | 0.72 | 0.62 | 0.81 |
| BP20;TFF3;COL6A3; MCAM | 0.75 | 0.67 | 0.83 | 0.43 | 0.17 | 0.86 | 0.72 | 0.99 | 0.71 | 0.62 | 0.80 |
| BP15;ADAM12;TFF3; QSQX1;IGFALS-e | 0.77 | 0.70 | 0.84 | 0.18 | 0.27 | 0.83 | 0.69 | 0.98 | 0.75 | 0.68 | 0.83 |
| BP20;ENG;TFF3; MCAM | 0.76 | 0.67 | 0.84 | 0.43 | 0.10 | 0.78 | 0.64 | 0.92 | 0.75 | 0.65 | 0.85 |
| ADAM12;TFF3;IGFALS; PIGF | 0.78 | 0.71 | 0.84 | 0.16 | 0.33 | 0.87 | 0.77 | 0.97 | 0.75 | 0.67 | 0.82 |
| BP15;MMRN2;ECM1; TFF3;COL6A3 | 0.74 | 0.66 | 0.82 | 0.37 | 0.19 | 0.85 | 0.72 | 0.97 | 0.71 | 0.62 | 0.81 |
| MMRN2;ADAM12; ECM1;PRDX2;TFF3; SPINT1 | 0.74 | 0.66 | 0.83 | 0.43 | 0.29 | 0.96 | 0.92 | 1.00 | 0.68 | 0.58 | 0.78 |
| BP15;ADAM12;TFF3; PIGF | 0.78 | 0.71 | 0.84 | 0.11 | 0.44 | 0.84 | 0.72 | 0.96 | 0.76 | 0.68 | 0.83 |
| BP15;ECM1;ENG; TFF3;SPINT1;QSOX1 | 0.75 | 0.67 | 0.84 | 0.38 | 0.30 | 0.85 | 0.75 | 0.95 | 0.73 | 0.63 | 0.82 |
| BP15;ECM1;TFF3; COL6A3;MCAM | 0.75 | 0.67 | 0.84 | 0.23 | 0.14 | 0.85 | 0.71 | 0.99 | 0.72 | 0.62 | 0.82 |
| BP15:MMRN2;ECM1; TFF3;COL6A3 | 0.74 | 0.66 | 0.83 | 0.40 | 0.15 | 0.85 | 0.73 | 0.97 | 0.71 | 0.61 | 0.81 |
| MMRN2;ECM1;ENG; TFF3;MCAM | 0.74 | 0.66 | 0.82 | 0.45 | 0.42 | 0.85 | 0.73 | 0.97 | 0.70 | 0.60 | 0.80 |
| BP15;ECM1;TFF3; COL6A3;MCAM | 0.75 | 0.67 | 0.83 | 0.19 | 0.27 | 0.85 | 0.71 | 1.00 | 0.72 | 0.62 | 0.81 |
| BP15;MMRN2;ECM1; TFF3;MCAM | 0.75 | 0.67 | 0.83 | 0.43 | 0.14 | 0.85 | 0.72 | 0.99 | 0.72 | 0.62 | 0.82 |
| BP15;ADAM12;TFF3; PIGF | 0.78 | 0.71 | 0.84 | 0.11 | 0.49 | 0.85 | 0.74 | 0.96 | 0.75 | 0.67 | 0.83 |
| BP20;TFF3;MCAM | 0.75 | 0.67 | 0.83 | 0.21 | 0.02 | 0.82 | 0.69 | 0.95 | 0.72 | 0.63 | 0.82 |
| BP15;ADAM12;TFF3; QSOX1;IGFALS-e | 0.77 | 0.70 | 0.84 | 0.24 | 0.18 | 0.84 | 0.70 | 0.98 | 0.75 | 0.67 | 0.83 |
| BP20;LNPEP;TFF3; PIGF | 0.76 | 0.68 | 0.84 | 0.44 | 0.05 | 0.81 | 0.67 | 0.96 | 0.74 | 0.65 | 0.83 |
| MMRN2;ECM1;TFF3; COL6A3;MCAM | 0.75 | 0.67 | 0.83 | 0.36 | 0.18 | 0.91 | 0.82 | 1.00 | 0.69 | 0.60 | 0.79 |

(continued)

| Panel composition | BA | BAa | Bab | BB | BC | BD | BE | BF | BG | BH | BI |
|---|---|---|---|---|---|---|---|---|---|---|---|
| BP20;MMRN2;PRDX2; TFF3 | 0.75 | 0.68 | 0.83 | 0.38 | 0.02 | 0.87 | 0.79 | 0.94 | 0.72 | 0.62 | 0.81 |
| MMRN2;ECM1;TFF3; MCAM | 0.75 | 0.67 | 0.83 | 0.39 | 0.21 | 0.87 | 0.75 | 0.99 | 0.71 | 0.62 | 0.81 |
| MMRN2;ADAM12; ECM1;PRDX2;TFF3 | 0.75 | 0.67 | 0.83 | 0.23 | 0.05 | 0.92 | 0.85 | 0.98 | 0.70 | 0.60 | 0.80 |
| BP15;MMRN2;ECM1; PRDX2;TFF3 | 0.75 | 0.67 | 0.83 | 0.43 | 0.22 | 0.83 | 0.72 | 0.94 | 0.72 | 0.62 | 0.82 |
| bmi;ADAM12;TFF3; PlGF | 0.78 | 0.71 | 0.85 | 0.19 | 0.19 | 0.89 | 0.80 | 0.98 | 0.74 | 0.66 | 0.82 |
| BP15;MMRN2;ECM1; PRDX2;TFF3 | 0.75 | 0.67 | 0.83 | 0.47 | 0.18 | 0.85 | 0.75 | 0.94 | 0.72 | 0.62 | 0.82 |
| BP15;MMRN2; ADAM12;ENG;TFF3; SPINT1 | 0.76 | 0.68 | 0.85 | 0.48 | 0.12 | 0.92 | 0.84 | 0.99 | 0.72 | 0.62 | 0.82 |
| BP20;MMRN2;ECM1; TFF3 | 0.74 | 0.65 | 0.82 | 0.50 | 0.05 | 0.83 | 0.69 | 0.97 | 0.71 | 0.61 | 0.80 |
| BP15;MMRN2; ADAM12;ENG;TFF3; SPINT1 | 0.76 | 0.68 | 0.84 | 0.48 | 0.16 | 0.92 | 0.84 | 0.99 | 0.72 | 0.62 | 0.82 |
| ENG;PRDX2;TFF3; QSOX1;IGFALS-e | 0.75 | 0.67 | 0.84 | 0.44 | 0.15 | 0.87 | 0.73 | 1.00 | 0.72 | 0.62 | 0.81 |
| BP20;MMRN2;ENG; TFF3;PlGF | 0.73 | 0.65 | 0.81 | 0.39 | 0.03 | 0.87 | 0.78 | 0.96 | 0.69 | 0.59 | 0.79 |
| MMRN2;ADAM12; ECM1;TFF3;QSOX1 | 0.75 | 0.68 | 0.83 | 0.25 | 0.43 | 0.92 | 0.85 | 0.99 | 0.70 | 0.61 | 0.78 |
| ADAM12;TFF3;SPINT1; QSOX1;IGFALS-e | 0.76 | 0.69 | 0.84 | 0.44 | 0.22 | **0.88** | 0.71 | 1.00 | 0.73 | 0.65 | 0.81 |
| BP15;ADAM12;TFF3; PlGF | 0.77 | 0.71 | 0.84 | 0.11 | 0.46 | 0.85 | 0.74 | 0.96 | 0.75 | 0.67 | 0.82 |
| BP20;MMRN2;ECM1; TFF3 | 0.74 | 0.65 | 0.82 | 0.42 | 0.05 | 0.87 | 0.75 | 0.98 | 0.70 | 0.59 | 0.80 |
| BP15;MMRN2;ECM1; TFF3;COL6A3 | 0.74 | 0.66 | 0.82 | 0.41 | 0.15 | 0.84 | 0.72 | 0.96 | 0.70 | 0.61 | 0.80 |
| BP20;ADAM12;TFF3; QSOX1 | 0.76 | 0.69 | 0.84 | 0.39 | 0.13 | 0.86 | 0.73 | 0.99 | 0.73 | 0.64 | 0.82 |
| BP15;ADAM12;TFF3; SPINT1;QSOX1 | 0.76 | 0.68 | 0.83 | 0.32 | 0.35 | 0.85 | 0.73 | 0.98 | 0.73 | 0.65 | 0.82 |
| BP15;ADAM12;TFF3; QSOX1;IGFALS-e | 0.77 | 0.70 | 0.84 | 0.20 | 0.18 | 0.83 | 0.69 | 0.98 | 0.75 | 0.67 | 0.83 |
| BP15;MMRN2; ADAM12;ECM1;TFF3 | 0.75 | 0.68 | 0.83 | 0.41 | 0.17 | 0.86 | 0.73 | 0.99 | 0.72 | 0.63 | 0.81 |
| BP20;MMRN2;TFF3; PlGF | 0.75 | 0.67 | 0.83 | 0.35 | 0.02 | 0.91 | 0.86 | 0.96 | 0.70 | 0.60 | 0.80 |

(continued)

| Panel composition | BA | BAa | Bab | BB | BC | BD | BE | BF | BG | BH | BI |
|---|---|---|---|---|---|---|---|---|---|---|---|
| BP15;MMRN2;ECM1; PRDX2;TFF3 | 0.75 | 0.67 | 0.83 | 0.41 | 0.22 | 0.84 | 0.74 | 0.93 | 0.72 | 0.62 | 0.81 |
| BP15;ADAM12;TFF3; IGFALS-e;MCAM | 0.78 | 0.70 | 0.85 | 0.43 | 0.27 | 0.85 | 0.71 | 1.00 | 0.75 | 0.67 | 0.84 |
| ADAM12;TFF3;MCAM; PIGF | 0.78 | 0.71 | 0.85 | 0.24 | 0.30 | 0.91 | 0.84 | 0.98 | 0.73 | 0.65 | 0.82 |
| BP15;MMRN2;ECM1; PRDX2;TFF3 | 0.74 | 0.66 | 0.82 | 0.41 | 0.28 | 0.83 | 0.72 | 0.94 | 0.72 | 0.62 | 0.81 |
| BP15;MMRN2;ECM1; TFF3;MCAM | 0.75 | 0.67 | 0.83 | 0.36 | 0.15 | 0.85 | 0.71 | 0.99 | 0.72 | 0.62 | 0.81 |
| BP15;ADAM12;TFF3; MCAM | 0.77 | 0.70 | 0.85 | 0.21 | 0.08 | 0.84 | 0.69 | 0.98 | 0.75 | 0.67 | 0.83 |
| bml;TFF3;CRP; COL6A3;MCAM;PIGF | 0.77 | 0.70 | 0.85 | 0.40 | 0.24 | 0.93 | 0.87 | 0.99 | 0.72 | 0.63 | 0.81 |
| BP15;ADAM12;TFF3; QSOX1;IGFALS | 0.77 | 0.70 | 0.84 | 0.02 | 0.37 | 0.83 | 0.70 | 0.95 | 0.75 | 0.68 | 0.83 |
| BP20;ECM1;TFF3;PIGF | 0.74 | 0.66 | 0.82 | 0.36 | 0.29 | 0.85 | 0.73 | 0.97 | 0.70 | 0.61 | 0.80 |
| BP15;ADAM12;TFF3; SPINT1;QSOX1 | 0.76 | 0.68 | 0.83 | 0.37 | 0.34 | 0.85 | 0.73 | 0.98 | 0.73 | 0.65 | 0.81 |
| BP15;ECM1;TFF3; COL6A3;MCAM | 0.75 | 0.67 | 0.83 | 0.19 | 0.16 | 0.86 | 0.72 | 0.99 | 0.71 | 0.62 | 0.80 |
| BP15;MMRN2;LNPEP; TFF3;PIGF | 0.76 | 0.69 | 0.84 | 0.38 | 0.25 | 0.90 | 0.82 | 0.97 | 0.72 | 0.63 | 0.81 |
| BP15;TFF3;COL6A3; IGFALS-e;MCAM | 0.76 | 0.69 | 0.84 | 0.44 | 0.27 | 0.84 | 0.70 | 0.98 | 0.74 | 0.65 | 0.83 |
| BP15;MMRN2;TFF3; COL6A3;PIGF | 0.75 | 0.68 | 0.83 | 0.43 | 0.17 | 0.92 | 0.86 | 0.98 | 0.70 | 0.61 | 0.79 |
| BP15;MMRN2; ADAM12;ECM1;TFF3; SPINT1 | 0.74 | 0.65 | 0.82 | 0.43 | 0.30 | 0.91 | 0.82 | 0.99 | 0.69 | 0.59 | 0.79 |
| BP15;TFF3;COL6A3; IGFALS-e;MCAM | 0.76 | 0.69 | 0.84 | 0.40 | 0.29 | 0.84 | 0.71 | 0.98 | 0.74 | 0.65 | 0.82 |
| BP15;ECM1;TFF3; MCAM | 0.75 | 0.66 | 0.83 | 0.24 | 0.02 | 0.82 | 0.66 | 0.97 | 0.72 | 0.62 | 0.82 |
| BP15;ADAM12;TFF3; SPINT1;QSOX1 | 0.75 | 0.68 | 0.83 | 0.20 | 0.33 | 0.87 | 0.76 | 0.97 | 0.72 | 0.64 | 0.80 |
| BP15;MMRN2;ECM1; TFF3;COL6A3 | 0.74 | 0.65 | 0.82 | 0.45 | 0.22 | 0.83 | 0.69 | 0.96 | 0.70 | 0.61 | 0.80 |
| BP15;MMRN2;ECM1; TFF3;COL6A3 | 0.74 | 0.66 | 0.81 | 0.36 | 0.21 | 0.86 | 0.75 | 0.97 | 0.70 | 0.60 | 0.79 |
| BP15;MMRN2;ECM1; TFF3;COL6A3 | 0.74 | 0.66 | 0.82 | 0.39 | 0.26 | 0.84 | 0.72 | 0.97 | 0.71 | 0.62 | 0.80 |
| BP15;ECM1;TFF3; MCAM | 0.74 | 0.66 | 0.83 | 0.37 | 0.03 | 0.82 | 0.67 | 0.97 | 0.72 | 0.62 | 0.82 |

(continued)

| Panel composition | BA | BAa | Bab | BB | BC | BD | BE | BF | BG | BH | BI |
|---|---|---|---|---|---|---|---|---|---|---|---|
| MMRN2;ECM1;TFF3; IGFALS-e | 0.75 | 0.67 | 0.82 | 0.36 | 0.29 | 0.90 | 0.81 | 0.99 | 0.70 | 0.60 | 0.79 |
| BP15;ADAM12;TFF3; MCAM | 0.77 | 0.70 | 0.84 | 0.21 | 0.10 | 0.84 | 0.70 | 0.98 | 0.75 | 0.66 | 0.83 |
| BP15;ECM1;TFF3; COL6A3;MCAM | 0.75 | 0.67 | 0.83 | 0.20 | 0.16 | 0.85 | 0.72 | 0.98 | 0.71 | 0.62 | 0.80 |
| MMRN2;ECM1;ENG; TFF3;SPINT1 | 0.73 | 0.65 | 0.82 | 0.48 | 0.16 | 0.94 | 0.87 | 1.00 | 0.68 | 0.58 | 0.77 |
| BP15;ADAM12;TFF3; QSOX1;IGFALS | 0.77 | 0.70 | 0.83 | 0.02 | 0.36 | 0.83 | 0.70 | 0.96 | 0.75 | 0.67 | 0.82 |
| BP15;MMRN2;TFF3; COL6A3;PIGF | 0.76 | 0.68 | 0.84 | 0.48 | 0.12 | 0.92 | 0.86 | 0.97 | 0.71 | 0.61 | 0.80 |
| BP15;MMRN2;TFF3; COL6A3;PIGF | 0.75 | 0.67 | 0.83 | 0.46 | 0.13 | 0.91 | 0.86 | 0.97 | 0.70 | 0.61 | 0.80 |
| BP15;MMRN2;ECM1; ENG;TFF3 | 0.71 | 0.62 | 0.80 | 0.41 | 0.06 | 0.78 | 0.63 | 0.94 | 0.69 | 0.58 | 0.80 |
| BP15;ADAM12;TFF3; QSOX1;IGFALS | 0.77 | 0.70 | 0.84 | 0.13 | 0.41 | 0.84 | 0.73 | 0.95 | 0.75 | 0.67 | 0.82 |
| BP15;MMRN2;ECM1; ENG;TFF3 | 0.72 | 0.63 | 0.81 | 0.41 | 0.07 | 0.78 | 0.61 | 0.94 | 0.70 | 0.59 | 0.80 |
| BP15;ENG;PRDX2; TFF3;QSOX1 | 0.73 | 0.65 | 0.81 | 0.21 | 0.27 | 0.78 | 0.64 | 0.93 | 0.72 | 0.62 | 0.81 |
| TFF3;QSOX1;IGFALS; PIGF | 0.76 | 0.69 | 0.83 | 0.22 | 0.34 | 0.86 | 0.76 | 0.97 | 0.72 | 0.64 | 0.80 |
| BP15;MMRN2;ECM1; ENG;TFF3 | 0.71 | 0.63 | 0.80 | 0.41 | 0.08 | 0.78 | 0.62 | 0.94 | 0.69 | 0.59 | 0.79 |
| BP20;ADAM12;TFF3; MCAM | 0.77 | 0.69 | 0.84 | 0.39 | 0.17 | 0.87 | 0.75 | 1.00 | 0.73 | 0.65 | 0.82 |
| BP15;MMRN2; ADAM12;TFF3 | 0.76 | 0.68 | 0.83 | 0.42 | 0.05 | 0.88 | 0.78 | 0.97 | 0.72 | 0.62 | 0.81 |
| SP15;MMRN2; ADAM12;TFF3 | 0.75 | 0.68 | 0.83 | 0.44 | 0.13 | 0.87 | 0.76 | 0.97 | 0.72 | 0.63 | 0.81 |
| BP1S;ADAM12;TFF3; MCAM | 0.77 | 0.70 | 0.84 | 0.14 | 0.08 | 0.84 | 0.70 | 0.99 | 0.74 | 0.66 | 0.82 |
| BP15;ADAM12;TFF3; PIGF | 0.77 | 0.70 | 0.84 | 0.11 | 0.41 | 0.84 | 0.72 | 0.95 | 0.74 | 0.67 | 0.82 |
| SP15;MMRN2;ECM1; TFF3 | 0.73 | 0.65 | 0.82 | 0.43 | 0.05 | 0.79 | 0.64 | 0.95 | 0.71 | 0.61 | 0.81 |
| bmi;ECM1;TFF3;MCAM | 0.75 | 0.66 | 0.83 | 0.28 | 0.06 | 0.84 | 0.69 | 0.98 | 0.72 | 0.62 | 0.81 |
| BP15;MMRN2;ECM1; ENG;TFF3 | 0.72 | 0.63 | 0.81 | 0.43 | 0.06 | 0.81 | 0.67 | 0.94 | 0.69 | 0.58 | 0.80 |
| BP15;ECM1;TFF3; IGFALS-e;MCAM | 0.75 | 0.67 | 0.83 | 0.49 | 0.22 | 0.84 | 0.69 | 0.98 | 0.72 | 0.63 | 0.81 |

(continued)

| Panel composition | BA | BAa | Bab | BB | BC | BD | BE | BF | BG | BH | BI |
|---|---|---|---|---|---|---|---|---|---|---|---|
| MMRN2;ECM1;ENG; TFF3;MCAM | 0.73 | 0.65 | 0.82 | 0.41 | 0.35 | 0.86 | 0.74 | 0.97 | 0.69 | 0.59 | 0.79 |
| BP15;ADAM12;TFF3; QSOX1;IGFALS-e | 0.76 | 0.69 | 0.84 | 0.29 | 0.26 | 0.84 | 0.71 | 0.98 | 0.74 | 0.66 | 0.82 |
| bmi;ADAM12;TFF3; MCAM | 0.77 | 0.70 | 0.84 | 0.28 | 0.25 | 0.87 | 0.74 | 0.99 | 0.74 | 0.66 | 0.82 |
| ECM1;ENG;TFF3; SPINT1;QSOX1 | 0.74 | 0.66 | 0.82 | 0.30 | 0.31 | 0.90 | 0.80 | 0.99 | 0.70 | 0.60 | 0.79 |
| BP20;ECM1;ENG; TFF3;QSQX1 | 0.72 | 0.63 | 0.80 | 0.29 | 0.01 | 0.84 | 0.75 | 0.92 | 0.68 | 0.57 | 0.78 |
| BP20;MMRN2;ECM1; TFF3 | 0.73 | 0.64 | 0.81 | 0.36 | 0.03 | 0.87 | 0.76 | 0.97 | 0.69 | 0.58 | 0.79 |
| ENG;PRDX2;TFF3; QSOX1;IGFALS-e | 0.75 | 0.66 | 0.83 | 0.42 | 0.00 | 0.87 | 0.73 | 1.00 | 0.71 | 0.61 | 0.80 |
| BP15;MMRN2;ECM1; ENG;TFF3 | 0.71 | 0.63 | 0.80 | 0.44 | 0.06 | 0.80 | 0.65 | 0.95 | 0.69 | 0.58 | 0.79 |
| BP15;MMRN2;ECM1; TFF3 | 0.74 | 0.65 | 0.82 | 0.39 | 0.04 | 0.79 | 0.64 | 0.95 | 0.72 | 0.62 | 0.81 |
| ADAM12;TFF3;QSOX1; PIGF | 0.76 | 0.69 | 0.83 | 0.14 | 0.41 | 0.91 | 0.83 | 1.00 | 0.71 | 0.63 | 0.79 |
| BP20;TFF3;COL6A3; MCAM | 0.74 | 0.66 | 0.82 | 0.38 | 0.18 | 0.88 | 0.77 | 0.98 | 0.70 | 0.60 | 0.79 |
| ECM1;ENG;TFF3; SPINT1:QSOX1 | 0.74 | 0.66 | 0.82 | 0.31 | 0.32 | 0.87 | 0.76 | 0.99 | 0.70 | 0.60 | 0.80 |
| BP15;MMRN2; ADAM12;TFF3 | 0.76 | 0.68 | 0.83 | 0.36 | 0.14 | 0.86 | 0.75 | 0.97 | 0.72 | 0.63 | 0.81 |
| BP15;ENG;PRDX2; TFF3;QSOX1 | 0.73 | 0.65 | 0.81 | 0.19 | 0.36 | 0.78 | 0.62 | 0.94 | 0.71 | 0.62 | 0.80 |
| BP15;ECM1;TFF3; COL6A3;MCAM | 0.74 | 0.66 | 0.82 | 0.29 | 0.19 | 0.86 | 0.73 | 0.99 | 0.70 | 0.61 | 0.80 |
| BP15;ECM1;TFF3; MCAM | 0.74 | 0.66 | 0.83 | 0.22 | 0.04 | 0.82 | 0.68 | 0.97 | 0.71 | 0.61 | 0.81 |
| BP20;ADAM12;TFF3; QSOX1 | 0.76 | 0.69 | 0.83 | 0.27 | 0.27 | 0.88 | 0.77 | 0.98 | 0.72 | 0.63 | 0.80 |
| BP20:MMRN2;PRDX2: TFF3 | 0.75 | 0.67 | 0.83 | 0.37 | 0.01 | 0.91 | 0.86 | 0.96 | 0.70 | 0.60 | 0.80 |
| ADAM12;PRDX2;TFF3; SPINT1;QSOX1 | 0.74 | 0.67 | 0.82 | 0.17 | 0.27 | 0.92 | 0.85 | 1.00 | 0.69 | 0.61 | 0.78 |
| BP15;MMRN2; ADAM12;TFF3 | 0.75 | 0.67 | 0.83 | 0.24 | 0.09 | 0.87 | 0.76 | 0.97 | 0.71 | 0.62 | 0.81 |
| MMRN2;ECM1;PRDX2; TFF3 | 0.73 | 0.65 | 0.81 | 0.23 | 0.19 | 0.85 | 0.76 | 0.94 | 0.69 | 0.59 | 0.79 |
| BP15;ENG;PRDX2; TFF3;MCAM | 0.74 | 0.66 | 0.83 | 0.50 | 0.14 | 0.80 | 0.67 | 0.94 | 0.72 | 0.62 | 0.82 |

(continued)

| Panel composition | BA | BAa | Bab | BB | BC | BD | BE | BF | BG | BH | BI |
|---|---|---|---|---|---|---|---|---|---|---|---|
| BP20;ADAM12;TFF3 | 0.75 | 0.67 | 0.84 | 0.38 | 0.03 | 0.81 | 0.64 | 0.99 | 0.74 | 0.64 | 0.83 |
| BP15;MMRN2; ADAM12;TFF3 | 0.75 | 0.68 | 0.83 | 0.20 | 0.04 | 0.88 | 0.79 | 0.97 | 0.71 | 0.62 | 0.81 |
| BP15;MMRN2;ENG; PRDX2;TFF3 | 0.73 | 0.64 | 0.82 | 0.45 | 0.05 | 0.82 | 0.67 | 0.98 | 0.70 | 0.59 | 0.80 |
| BP15;MMRN2;ECM1; ENG;TFF3;COL6A3 | 0.71 | 0.63 | 0.79 | 0.42 | 0.29 | 0.83 | 0.69 | 0.97 | 0.67 | 0.58 | 0.77 |
| BP15;MMRN2;ECM1; TFF3 | 0.73 | 0.65 | 0.81 | 0.34 | 0.15 | 0.80 | 0.65 | 0.94 | 0.71 | 0.61 | 0.81 |
| BP15;MMRN2;ECM1; TFF3 | 0.73 | 0.65 | 0.81 | 0.41 | 0.06 | 0.79 | 0.64 | 0.94 | 0.71 | 0.61 | 0.80 |
| BP15;ADAM12;TFF3; MCAM | 0.77 | 0.69 | 0.84 | 0.20 | 0.14 | 0.86 | 0.73 | 0.99 | 0.73 | 0.65 | 0.82 |
| ADAM12;TFF3;QSOX1; IGFALS | 0.76 | 0.69 | 0.83 | 0.07 | 0.31 | 0.85 | 0.75 | 0.95 | 0.73 | 0.65 | 0.81 |
| bmi;ADAM12;TFF3; QSOX1 | 0.76 | 0.69 | 0.83 | 0.21 | 0.29 | 0.86 | 0.75 | 0.97 | 0.72 | 0.64 | 0.80 |
| BP15;ECM1;ENG; TFF3;MCAM | 0.74 | 0.65 | 0.83 | 0.34 | 0.08 | 0.79 | 0.63 | 0.94 | 0.72 | 0.62 | 0.82 |
| BP15;ADAM12;PRDX2; TFF3;ENPP2 | 0.76 | 0.68 | 0.83 | 0.38 | 0.14 | 0.88 | 0.80 | 0.96 | 0.72 | 0.62 | 0.81 |
| BP20;TFF3;MCAM | 0.74 | 0.66 | 0.82 | 0.52 | 0.02 | 0.84 | 0.73 | 0.95 | 0.71 | 0.61 | 0.81 |
| BP15;ENG;PRDX2; TFF3;MCAM | 0.74 | 0.65 | 0.82 | 0.33 | 0.15 | 0.79 | 0.64 | 0.94 | 0.72 | 0.62 | 0.82 |
| BP15;ENG;PRDX2; TFF3;QSOX1 | 0.73 | 0.64 | 0.81 | 0.21 | 0.26 | 0.77 | 0.62 | 0.92 | 0.71 | 0.62 | 0.81 |
| BP15;MMRN2;ECM1; TFF3;COL6A3 | 0.73 | 0.65 | 0.80 | 0.39 | 0.38 | 0.84 | 0.71 | 0.96 | 0.69 | 0.60 | 0.78 |
| BP15;ADAM12;TFF3; IGFALS-e | 0.76 | 0.68 | 0.83 | 0.27 | 0.04 | 0.79 | 0.61 | 0.97 | 0.74 | 0.66 | 0.83 |
| BP15;MMRN2;ENG; PRDX2;TFF3 | 0.73 | 0.64 | 0.82 | 0.50 | 0.05 | 0.82 | 0.66 | 0.97 | 0.70 | 0.59 | 0.80 |
| BP15;ADAM12:TFF3; QSOX1 | 0.75 | 0.68 | 0.82 | 0.13 | 0.29 | 0.82 | 0.68 | 0.95 | 0.73 | 0.65 | 0.80 |
| BP15;MMRN2; ADAM12;TFF3 | 0.75 | 0.67 | 0.82 | 0.33 | 0.13 | 0.87 | 0.78 | 0.97 | 0.70 | 0.61 | 0.80 |
| BP20;ECM1;TFF3; QSOX1 | 0.72 | 0.63 | 0.81 | 0.38 | 0.05 | 0.79 | 0.63 | 0.95 | 0.70 | 0.60 | 0.80 |
| BP20;ENG;TFF3; QSOX1 | 0.73 | 0.64 | 0.81 | 0.33 | 0.05 | 0.76 | 0.63 | 0.89 | 0.71 | 0.61 | 0.81 |
| BP15;ADAM12;TFF3; MCAM | 0.76 | 0.69 | 0.84 | 0.26 | 0.19 | 0.86 | 0.73 | 0.98 | 0.73 | 0.64 | 0.82 |
| ENG;TFF3;QSOX1; IGFALS-e | 0.74 | 0.66 | 0.82 | 0.30 | 0.00 | 0.85 | 0.70 | 1.00 | 0.71 | 0.62 | 0.80 |

(continued)

| Panel composition | BA | BAa | Bab | BB | BC | BD | BE | BF | BG | BH | BI |
|---|---|---|---|---|---|---|---|---|---|---|---|
| BP15;ECM1;ENG; TFF3;MCAM | 0.73 | 0.65 | 0.82 | 0.39 | 0.05 | 0.79 | 0.64 | 0.95 | 0.71 | 0.61 | 0.82 |
| BP15;ENG;PRDX2; TFF3;QSOX1 | 0.73 | 0.64 | 0.81 | 0.23 | 0.29 | 0.77 | 0.63 | 0.91 | 0.71 | 0.61 | 0.81 |
| BP15;MMRN2;ECM1; TFF3 | 0.73 | 0.64 | 0.81 | 0.40 | 0.05 | 0.80 | 0.65 | 0.94 | 0.71 | 0.61 | 0.81 |
| BP20;TFF3;PlGF | 0.74 | 0.66 | 0.82 | 0.28 | 0.01 | 0.84 | 0.74 | 0.94 | 0.70 | 0.61 | 0.80 |
| BP15;MMRN2;ENG; PRDX2;TFF3 | 0.72 | 0.63 | 0.81 | 0.45 | 0.08 | 0.83 | 0.67 | 0.99 | 0.69 | 0.58 | 0.79 |
| bmi;ECM1;TFF3;MCAM | 0.74 | 0.66 | 0.82 | 0.15 | 0.18 | 0.83 | 0.68 | 0.97 | 0.71 | 0.61 | 0.80 |
| BP20;TFF3;PlGF | 0.74 | 0.66 | 0.82 | 0.30 | 0.03 | 0.82 | 0.70 | 0.94 | 0.71 | 0.61 | 0.80 |
| MMRN2;ADAM12; ECM1;TFF3 | 0.73 | 0.65 | 0.81 | 0.28 | 0.05 | 0.89 | 0.78 | 0.99 | 0.68 | 0.59 | 0.78 |
| ECM1;TFF3;MCAM; PlGF | 0.74 | 0.66 | 0.82 | 0.28 | 0.19 | 0.88 | 0.79 | 0.97 | 0.69 | 0.59 | 0.79 |
| ADAM12;TFF3;IGFALS; MCAM | 0.76 | 0.69 | 0.83 | 0.17 | 0.30 | 0.85 | 0.74 | 0.97 | 0.73 | 0.65 | 0.81 |
| bmi;MMRN2;ADAM12; TFF3 | 0.75 | 0.67 | 0.83 | 0.37 | 0.15 | 0.90 | 0.80 | 0.99 | 0.70 | 0.60 | 0.79 |
| BP15;ECM1;ENG; TFF3;MCAM | 0.73 | 0.65 | 0.82 | 0.33 | 0.11 | 0.82 | 0.68 | 0.95 | 0.71 | 0.61 | 0.80 |
| BP15;ADAM12;TFF3; QSOX1 | 0.75 | 0.68 | 0.82 | 0.07 | 0.37 | 0.81 | 0.67 | 0.94 | 0.73 | 0.65 | 0.81 |
| BP15;MMRN2;ECM1; TFF3;COL6A3 | 0.72 | 0.64 | 0.80 | 0.28 | 0.19 | 0.85 | 0.74 | 0.96 | 0.68 | 0.59 | 0.77 |
| BP15;TFF3;COL6A3; MCAM;PlGF | 0.75 | 0.67 | 0.83 | 0.31 | 0.22 | 0.89 | 0.82 | 0.96 | 0.71 | 0.61 | 0.80 |
| BP15;MMRN2;ECM1; PRDX2;TFF3 | 0.73 | 0.65 | 0.81 | 0.37 | 0.19 | 0.84 | 0.75 | 0.93 | 0.70 | 0.60 | 0.79 |
| MMRN2;ADAM12; ECM1;TFF3;SPINT1 | 0.72 | 0.63 | 0.80 | 0.40 | 0.07 | 0.94 | 0.87 | 1.00 | 0.65 | 0.56 | 0.75 |
| ADAM12;ENG;TFF3; SPINT1;QSOX1 | 0.74 | 0.67 | 0.82 | 0.35 | 0.29 | 0.88 | 0.73 | 1.00 | 0.70 | 0.62 | 0.79 |
| BP15;ADAM12;TFF3; QSOX1 | 0.75 | 0.67 | 0.82 | 0.13 | 0.17 | 0.82 | 0.71 | 0.94 | 0.72 | 0.64 | 0.80 |
| BP15;ECM1;TFF3; MCAM | 0.73 | 0.65 | 0.81 | 0.12 | 0.04 | 0.81 | 0.66 | 0.96 | 0.70 | 0.61 | 0.80 |
| ADAM12;TFF3;SPINT1; PlGF | 0.74 | 0.67 | 0.82 | 0.23 | 0.34 | 0.91 | 0.80 | 1.00 | 0.69 | 0.61 | 0.77 |
| BP15;MMRN2;ECM1; ENG;TFF3;COL6A3 | 0.70 | 0.62 | 0.78 | 0.33 | 0.30 | 0.83 | 0.68 | 0.98 | 0.66 | 0.57 | 0.75 |
| ECM1;TFF3;MCAM; PlGF | 0.74 | 0.66 | 0.82 | 0.24 | 0.15 | 0.89 | 0.81 | 0.97 | 0.68 | 0.59 | 0.78 |

(continued)

| Panel composition | BA | BAa | Bab | BB | BC | BD | BE | BF | BG | BH | BI |
|---|---|---|---|---|---|---|---|---|---|---|---|
| BP20;MMRN2;TFF3; COL6A3 | 0.73 | 0.65 | 0.81 | 0.48 | 0.18 | 0.92 | 0.86 | 0.98 | 0.67 | 0.57 | 0.77 |
| BP15;ECM1;TFF3; MCAM | 0.73 | 0.64 | 0.81 | 0.26 | 0.04 | 0.80 | 0.65 | 0.95 | 0.70 | 0.61 | 0.80 |
| BP15;ADAM12;PRDX2; TFF3;ENPP2 | 0.75 | 0.67 | 0.83 | 0.39 | 0.00 | 0.90 | 0.82 | 0.97 | 0.70 | 0.61 | 0.80 |
| BP15;ECM1;TFF3; MCAM | 0.73 | 0.65 | 0.82 | 0.17 | 0.03 | 0.81 | 0.66 | 0.96 | 0.71 | 0.61 | 0.80 |
| TFF3;IGFALS-e;PIGF | 0.75 | 0.67 | 0.82 | 0.22 | 0.19 | 0.86 | 0.72 | 1.00 | 0.71 | 0.63 | 0.79 |
| BP15;ECM1;ENG; TFF3;QSOX1 | 0.71 | 0.63 | 0.80 | 0.17 | 0.00 | 0.76 | 0.64 | 0.88 | 0.70 | 0.59 | 0.80 |
| ADAM12;TFF3;PIGF | 0.75 | 0.68 | 0.82 | 0.09 | 0.41 | 0.88 | 0.79 | 0.96 | 0.71 | 0.63 | 0.79 |
| BP15;ADAM12;TFF3; IGFALS-e | 0.75 | 0.68 | 0.83 | 0.24 | 0.04 | 0.79 | 0.61 | 0.97 | 0.74 | 0.66 | 0.82 |
| BP15;MMRN2;ECM1; TFF3 | 0.72 | 0.64 | 0.80 | 0.39 | 0.15 | 0.79 | 0.64 | 0.94 | 0.70 | 0.60 | 0.79 |
| BP15;ECM1;ENG; TFF3;QSOX1 | 0.71 | 0.63 | 0.80 | 0.17 | 0.00 | 0.76 | 0.62 | 0.89 | 0.70 | 0.59 | 0.80 |
| BP15;MMRN2;ECM1; ENG;TFF3 | 0.70 | 0.61 | 0.79 | 0.40 | 0.06 | 0.79 | 0.64 | 0.93 | 0.68 | 0.57 | 0.78 |
| BP15;TFF3;COL6A3; MCAM;PIGF | 0.75 | 0.67 | 0.83 | 0.36 | 0.16 | 0.89 | 0.82 | 0.96 | 0.70 | 0.61 | 0.80 |
| BP15;ECM1;ENG; TFF3;QSOX1 | 0.71 | 0.62 | 0.80 | 0.14 | 0.00 | 0.76 | 0.62 | 0.89 | 0.69 | 0.59 | 0.80 |
| MMRN2;TFF3;COL6A3; PIGF | 0.73 | 0.65 | 0.81 | 0.37 | 0.05 | 0.94 | 0.89 | 0.99 | 0.67 | 0.57 | 0.76 |
| ECM1;TFF3;IGFALS-e; MCAM | 0.74 | 0.66 | 0.81 | 0.24 | 0.29 | 0.82 | 0.68 | 0.97 | 0.71 | 0.62 | 0.80 |
| ADAM12;TFF3;QSOX1; IGFALS | 0.75 | 0.68 | 0.82 | 0.16 | 0.40 | 0.84 | 0.74 | 0.94 | 0.72 | 0.64 | 0.80 |
| BP15;ADAM12;PRDX2; TFF3;SPINT1 | 0.74 | 0.66 | 0.82 | 0.24 | 0.24 | 0.90 | 0.82 | 0.98 | 0.69 | 0.60 | 0.78 |
| MMRN2;ECM1;ENG; TFF3 | 0.69 | 0.60 | 0.78 | 0.39 | 0.05 | 0.81 | 0.66 | 0.96 | 0.65 | 0.55 | 0.76 |
| BP15;ECM1;TFF3; COL6A3;QSOX1 | 0.72 | 0.63 | 0.80 | 0.33 | 0.08 | 0.79 | 0.66 | 0.93 | 0.69 | 0.59 | 0.79 |
| TFF3;IGFALS;PIGF | 0.74 | 0.68 | 0.81 | 0.17 | 0.41 | 0.83 | 0.71 | 0.94 | 0.72 | 0.64 | 0.79 |
| MMRN2;ADAM12; ECM1;TFF3 | 0.73 | 0.65 | 0.81 | 0.27 | 0.06 | 0.89 | 0.80 | 0.99 | 0.67 | 0.58 | 0.77 |
| ECM1;ENG;TFF3; SPINT1;QSOX1 | 0.73 | 0.64 | 0.81 | 0.30 | 0.29 | 0.89 | 0.79 | 0.99 | 0.68 | 0.58 | 0.78 |
| BP15;ECM1;ENG; TFF3;QSOX1 | 0.71 | 0.62 | 0.80 | 0.20 | 0.00 | 0.73 | 0.59 | 0.88 | 0.70 | 0.60 | 0.81 |

(continued)

| Panel composition | BA | BAa | Bab | BB | BC | BD | BE | BF | BG | BH | BI |
|---|---|---|---|---|---|---|---|---|---|---|---|
| BP15;ADAM12;PRDX2; TFF3;SPINT1 | 0.74 | 0.66 | 0.82 | 0.29 | 0.21 | 0.90 | 0.82 | 0.98 | 0.69 | 0.60 | 0.79 |
| BP15;ADAM12;TFF3; QSOX1 | 0.75 | 0.68 | 0.82 | 0.09 | 0.11 | 0.83 | 0.71 | 0.94 | 0.72 | 0.64 | 0.81 |
| BP15;ADAM12;TFF3; IGFALS-e | 0.75 | 0.67 | 0.83 | 0.36 | 0.05 | 0.80 | 0.63 | 0.96 | 0.73 | 0.65 | 0.82 |
| BP20;MMRN2;TFF3 | 0.73 | 0.65 | 0.81 | 0.22 | 0.01 | 0.86 | 0.78 | 0.94 | 0.68 | 0.59 | 0.78 |
| MMRN2;ECM1;TFF3; COL6A3 | 0.71 | 0.63 | 0.79 | 0.20 | 0.16 | 0.86 | 0.78 | 0.95 | 0.66 | 0.57 | 0.75 |
| BP15;ADAM12;PRDX2; TFF3;SPINT1 | 0.74 | 0.65 | 0.82 | 0.39 | 0.14 | 0.90 | 0.83 | 0.98 | 0.69 | 0.59 | 0.79 |
| ADAM12;TFF3;QSOX1; IGFALS-e | 0.74 | 0.67 | 0.82 | 0.31 | 0.17 | 0.85 | 0.71 | 0.99 | 0.71 | 0.63 | 0.80 |
| BP15;ECM1;ENG; TFF3;QSOX1 | 0.70 | 0.62 | 0.79 | 0.12 | 0.00 | 0.73 | 0.58 | 0.88 | 0.69 | 0.59 | 0.80 |
| BP15;ECM1;TFF3; MCAM | 0.73 | 0.64 | 0.81 | 0.24 | 0.06 | 0.81 | 0.67 | 0.96 | 0.70 | 0.60 | 0.79 |
| ADAM12;TFF3;IGFALS-e;MCAM | 0.75 | 0.68 | 0.83 | 0.26 | 0.28 | 0.85 | 0.71 | 0.98 | 0.72 | 0.63 | 0.81 |
| BP15;MMRN2;TFF3; COL6A3 | 0.73 | 0.65 | 0.81 | 0.47 | 0.18 | 0.85 | 0.75 | 0.95 | 0.69 | 0.59 | 0.79 |
| MMRN2;ECM1;TFF3 | 0.71 | 0.63 | 0.79 | 0.34 | 0.15 | 0.82 | 0.69 | 0.94 | 0.67 | 0.58 | 0.77 |
| BP20;TFF3;PIGF | 0.73 | 0.65 | 0.81 | 0.28 | 0.02 | 0.84 | 0.74 | 0.94 | 0.69 | 0.59 | 0.79 |
| MMRN2;PRDX2;TFF3; PIGF | 0.73 | 0.65 | 0.81 | 0.28 | 0.03 | 0.91 | 0.84 | 0.98 | 0.68 | 0.58 | 0.77 |
| ECM1;TFF3;IGFALS-e; MCAM | 0.73 | 0.65 | 0.81 | 0.24 | 0.30 | 0.82 | 0.68 | 0.96 | 0.70 | 0.61 | 0.79 |
| BP20;PRDX2;TFF3 | 0.73 | 0.65 | 0.81 | 0.00 | 0.02 | 0.86 | 0.80 | 0.92 | 0.69 | 0.59 | 0.79 |
| BP15;MMRN2;TFF3; COL6A3 | 0.73 | 0.65 | 0.80 | 0.43 | 0.27 | 0.85 | 0.77 | 0.94 | 0.69 | 0.59 | 0.78 |
| BP15;TFF3;COL6A3; MCAM | 0.73 | 0.65 | 0.82 | 0.30 | 0.15 | 0.83 | 0.70 | 0.96 | 0.70 | 0.60 | 0.80 |
| BP15;MMRN2;ECM1; ENG;TFF3 | 0.70 | 0.61 | 0.78 | 0.42 | 0.27 | 0.80 | 0.66 | 0.95 | 0.66 | 0.56 | 0.76 |
| BP15;MMRN2;ECM1; TFF3 | 0.72 | 0.63 | 0.80 | 0.37 | 0.13 | 0.79 | 0.63 | 0.94 | 0.69 | 0.60 | 0.79 |
| BP15;MMRN2;TFF3; COL6A3 | 0.73 | 0.65 | 0.81 | 0.28 | 0.21 | 0.87 | 0.78 | 0.95 | 0.69 | 0.59 | 0.78 |
| BP15;MMRN2;TFF3; COL6A3 | 0.73 | 0.65 | 0.81 | 0.43 | 0.27 | 0.85 | 0.74 | 0.95 | 0.69 | 0.60 | 0.79 |
| BP20;ADAM12;TFF3 | 0.74 | 0.66 | 0.82 | 0.32 | 0.07 | 0.84 | 0.71 | 0.97 | 0.71 | 0.62 | 0.81 |
| BP15;ADAM12;TFF3; IGFALS-e | 0.74 | 0.67 | 0.82 | 0.36 | 0.22 | 0.81 | 0.65 | 0.96 | 0.72 | 0.64 | 0.81 |

(continued)

| Panel composition | BA | BAa | Bab | BB | BC | BD | BE | BF | BG | BH | BI |
|---|---|---|---|---|---|---|---|---|---|---|---|
| BR15;MMRN2;ECM1; TFF3 | 0.71 | 0.63 | 0.79 | 0.33 | 0.17 | 0.79 | 0.65 | 0.93 | 0.69 | 0.59 | 0.78 |
| MMRN2;ECM1;PRDX2; TFF3 | 0.71 | 0.63 | 0.80 | 0.22 | 0.19 | 0.85 | 0.76 | 0.94 | 0.67 | 0.57 | 0.77 |
| BP15;TFF3;COL6A3; MCAM | 0.73 | 0.65 | 0.81 | 0.33 | 0.19 | 0.84 | 0.73 | 0.96 | 0.70 | 0.60 | 0.79 |
| BP15;TFF3;COL6A3; MCAM | 0.73 | 0.65 | 0.81 | 0.30 | 0.21 | 0.84 | 0.72 | 0.96 | 0.70 | 0.60 | 0.79 |
| BP15;TFF3;COL6A3; MCAM | 0.73 | 0.65 | 0.81 | 0.40 | 0.13 | 0.83 | 0.70 | 0.96 | 0.70 | 0.60 | 0.79 |
| BP15;ECM1;TFF3;PIGF | 0.72 | 0.63 | 0.80 | 0.21 | 0.18 | 0.79 | 0.65 | 0.94 | 0.69 | 0.59 | 0.79 |
| BP15;MMRN2;ECM1; TFF3 | 0.71 | 0.63 | 0.79 | 0.37 | 0.16 | 0.79 | 0.64 | 0.94 | 0.68 | 0.59 | 0.78 |
| ADAM12;PRDX2;TFF3; QSOX1 | 0.68 | 0.58 | 0.77 | 0.19 | 0.03 | 0.81 | 0.60 | 1.00 | 0.64 | 0.54 | 0.75 |
| BP15;TFF3;COL6A3; MCAM | 0.73 | 0.65 | 0.81 | 0.33 | 0.17 | 0.83 | 0.70 | 0.96 | 0.70 | 0.60 | 0.79 |
| BP15;MMRN2;ECM1; TFF3 | 0.71 | 0.63 | 0.80 | 0.38 | 0.15 | 0.79 | 0.65 | 0.94 | 0.69 | 0.59 | 0.79 |
| MMRN2;ADAM12;TFF3 | 0.72 | 0.64 | 0.80 | 0.24 | 0.13 | 0.91 | 0.84 | 0.97 | 0.66 | 0.56 | 0.76 |
| ADAM12;TFF3;SPINT1; QSOX1 | 0.72 | 0.64 | 0.80 | 0.07 | 0.22 | 0.89 | 0.78 | 0.99 | 0.67 | 0.59 | 0.76 |
| ADAM12;TFF3;QSOX1; PIGF | 0.68 | 0.58 | 0.77 | 0.31 | 0.23 | 0.75 | 0.54 | 0.95 | 0.66 | 0.56 | 0.77 |
| BP15;ADAM12;TFF3 | 0.73 | 0.65 | 0.81 | 0.09 | 0.05 | 0.77 | 0.59 | 0.94 | 0.72 | 0.64 | 0.80 |
| BR15;MMRN2;TFF3; COL6A3 | 0.72 | 0.64 | 0.80 | 0.36 | 0.19 | 0.85 | 0.76 | 0.94 | 0.68 | 0.58 | 0.77 |
| BP15;MMRN2;TFF3; COL6A3 | 0.72 | 0.64 | 0.80 | 0.40 | 0.24 | 0.84 | 0.74 | 0.95 | 0.68 | 0.59 | 0.78 |
| ECM1;TFF3;MCAM | 0.72 | 0.63 | 0.80 | 0.37 | 0.01 | 0.81 | 0.66 | 0.97 | 0.68 | 0.58 | 0.78 |
| BP15;ADAM12;TFF3 | 0.73 | 0.66 | 0.81 | 0.11 | 0.05 | 0.76 | 0.59 | 0.94 | 0.72 | 0.64 | 0.81 |
| BP15;MMRN2;PRDX2; TFF3 | 0.73 | 0.64 | 0.81 | 0.46 | 0.01 | 0.84 | 0.73 | 0.95 | 0.69 | 0.59 | 0.80 |
| BP15;ADAM12;TFF3; IGFALS | 0.74 | 0.66 | 0.81 | 0.04 | 0.21 | 0.79 | 0.65 | 0.93 | 0.72 | 0.64 | 0.81 |
| BP15;TFF3;COL6A3; MCAM | 0.73 | 0.64 | 0.81 | 0.43 | 0.27 | 0.84 | 0.73 | 0.96 | 0.69 | 0.59 | 0.79 |
| bmi;ADAM12;TFF3 | 0.73 | 0.66 | 0.81 | 0.12 | 0.22 | 0.82 | 0.68 | 0.97 | 0.70 | 0.62 | 0.79 |
| BP20;TFF3 | 0.71 | 0.63 | 0.80 | 0.23 | 0.00 | 0.72 | 0.56 | 0.89 | 0.71 | 0.61 | 0.81 |
| MMRN2;TFF3;PIGF | 0.72 | 0.64 | 0.80 | 0.30 | 0.05 | 0.89 | 0.83 | 0.96 | 0.66 | 0.56 | 0.76 |
| BR15;MMRN2;PRDX2; TFF3 | 0.72 | 0.64 | 0.81 | 0.36 | 0.03 | 0.82 | 0.72 | 0.93 | 0.69 | 0.59 | 0.79 |

(continued)

| Panel composition | BA | BAa | Bab | BB | BC | BD | BE | BF | BG | BH | BI |
|---|---|---|---|---|---|---|---|---|---|---|---|
| BP15;MMRN2;PRDX2; TFF3 | 0.72 | 0.64 | 0.80 | 0.38 | 0.03 | 0.82 | 0.72 | 0.93 | 0.69 | 0.59 | 0.79 |
| BP15;ENG;TFF3; MCAM | 0.72 | 0.64 | 0.81 | 0.30 | 0.15 | 0.75 | 0.60 | 0.89 | 0.71 | 0.61 | 0.81 |
| ENG;TFF3;SPINT1; QSOX1 | 0.72 | 0.64 | 0.81 | 0.15 | 0.01 | 0.80 | 0.62 | 0.98 | 0.70 | 0.61 | 0.80 |
| BP15;PRDX2;TFF3; PIGF | 0.72 | 0.65 | 0.80 | 0.26 | 0.19 | 0.85 | 0.76 | 0.94 | 0.69 | 0.59 | 0.78 |
| ENG;PRDX2;TFF3; MCAM | 0.72 | 0.63 | 0.80 | 0.33 | 0.05 | 0.81 | 0.66 | 0.97 | 0.68 | 0.58 | 0.79 |
| ECM1;ENG;TFF3; QSOX1 | 0.69 | 0.61 | 0.78 | 0.14 | 0.00 | 0.81 | 0.70 | 0.92 | 0.66 | 0.56 | 0.76 |
| bmi;TFF3;MCAM | 0.73 | 0.65 | 0.81 | 0.00 | 0.16 | 0.80 | 0.68 | 0.93 | 0.70 | 0.61 | 0.79 |
| MMRN2;ECM1;TFF3 | 0.70 | 0.62 | 0.78 | 0.20 | 0.12 | 0.81 | 0.69 | 0.93 | 0.66 | 0.57 | 0.76 |
| BP15;ENG;TFF3; MCAM | 0.72 | 0.63 | 0.80 | 0.05 | 0.30 | 0.74 | 0.58 | 0.89 | 0.71 | 0.61 | 0.81 |
| ECM1;ENG;TFF3; MCAM | 0.71 | 0.63 | 0.80 | 0.28 | 0.05 | 0.83 | 0.68 | 0.97 | 0.68 | 0.58 | 0.78 |
| ADAM12;TFF3;MCAM | 0.73 | 0.66 | 0.81 | 0.14 | 0.31 | 0.86 | 0.75 | 0.97 | 0.69 | 0.60 | 0.78 |

**Analysis of data Table 12**

While **Table 12** provides source data concerning relevant statistics pertinent to performance of illustrative, non-limiting panels containing TFF3 useful for predicting PE, the following examples further process the data to extract additional information on certain subgroups of panels from those of **Table 12,** which may be particularly well-performing or otherwise useful or of interest. The tables also capture the frequency at which the constituent biomarkers and/or clinical parameters occur in such panels. It can be expected that the higher the frequency, the higher the relative importance of a biomarker and/or clinical parameter will be in the subgroup of panels in question, and in panels in general.

In this connection, it was explained above that **Table 12** is to some extent redundant with regard to the test panels. The following analysis was performed on the entire data set of **Table 12,** i.e., without removing said redundancy. Consequently, test panels containing biomarkers and/or clinical parameters that cause the redundancy, specifically ADAM12, ECM1 , LCAT, IGFALS, and/or BP may be to some, comparatively minor extent overrepresented in **Table 12.** This, however, does not detract from the analysis of the frequencies of occurrence of the analysed biomarkers and clinical parameters in the panels, and the corresponding relative importance of the biomarkers and clinical parameters, as set forth in **Tables 12A-F.**

In the following tables, and namely **Tables 12A-F,** column **AJ** represents the total number of panels that meet the stated criterion or criteria; column **AK** represents the number of constituents in a panel (between 6 and 1) and columns **AL** and **AM** represent respectively the number and proportion (%) of panels that have the number of constituent stated in the respective row of column **AK;** column **AN** represents biomarkers and columns **AO** and **AP** represent respectively the number and proportion (%) of panels that contain the constituent stated in the respective row of column **AN;** and column **AQ** represents clinical parameters and columns **AR** and **AS** represent respectively the number and proportion (%) of panels that contain the clinical parameter stated in the respective row of column **AQ.** Note that because all panels in Table 12 contain TFF3, this marker is not repeated in **Tables 12A-F.** Note that in **Tables 12A-F,** IGFALS collectively denotes both IGFALS measurement by MASSTERCLASS® assay and by ELISA.

**Table 12A** captures all panels of **Table 12.**

**Table 12B** captures panels of **Table 12** in which sensitivity at 20% PPV for predicting all PE (i.e., without distinction between preterm and term PE) at 20 weeks in European cohort is equal to or greater than 0.495. Without limitation, such panels may be particularly useful as rule-in panels, more specifically for predicting PE, , even more specifically for predicting PE without distinction between preterm and term PE, even more specifically in European ancestry patients.

**Table 12C** captures panels of **Table 12** in which specificity at 99% NPV for predicting all PE (i.e., without distinction between preterm and term PE) at 20 weeks in European cohort is equal to or greater than 0.395. Without limitation, such panels may be particularly useful as rule-out panels, more specifically for predicting PE, even more specifically for predicting PE without distinction between preterm and term PE, even more specifically in European ancestry patients.

**Table 12D** captures panels of **Table 12** in which AUC for predicting all PE (i.e., without distinction between preterm and term PE) at 20 weeks in European cohort is equal to or greater than 0.795. Without limitation, such panels may be particularly useful for predicting PE, even more specifically for predicting PE without distinction between preterm and term PE, even more specifically in European ancestry patients.

**Table 12E** captures panels of **Table 12** in which AUC for predicting preterm PE at 20 weeks in the European cohort is equal to or greater than 0.895. Without limitation, such panels may be particularly useful for predicting preterm PE, even more specifically in European ancestry patients.

**Table 12F** captures panels of **Table 12** in which AUC for predicting term PE at 20 weeks in the European cohort is equal to or greater than 0.795. Without limitation, such panels may be particularly useful for predicting term PE, even more specifically in European ancestry patients.

**Table 12A**

| AJ | AK | AL | AM | AN | AO | AP | AQ | AR | AS |
|---|---|---|---|---|---|---|---|---|---|
| 532 | 6 | 171 | 32.1 | ECM1 | 277 | 52.1 | BP15 | 246 | 46.2 |
| | 5 | 202 | 38.0 | MMRN2 | 270 | 50.8 | BP20 | 158 | 29.7 |
| | 4 | 136 | 25.6 | ADAM12 | 206 | 38.7 | bmi | 28 | 5.3 |
| | 3 | 22 | 4.1 | MCAM | 182 | 34.2 | | | |
| | 2 | 1 | 0.2 | PIGF | 173 | 32.5 | | | |
| | 1 | | 0.0 | QSOX1 | 119 | 22.4 | | | |
| | | | | IGFALS | 103 | 19.4 | | | |
| | | | | COL6A3 | 98 | 18.4 | | | |
| | | | | ENG | 96 | 18.0 | | | |
| | | | | PRDX2 | 65 | 12.2 | | | |
| | | | | SPINT1 | 49 | 9.2 | | | |
| | | | | LNPEP | 28 | 5.3 | | | |
| | | | | CRP | 6 | 1.1 | | | |
| | | | | LCAT | 5 | 0.9 | | | |
| | | | | ENPP2 | 3 | 0.6 | | | |
| | | | | MAPRE1/3 | 2 | 0.4 | | | |
| | | | | ALDOA | 2 | 0.4 | | | |

**Table 12B**

| AJ | AK | AL | AM | AN | AO | AP | AQ | AR | AS |
|---|---|---|---|---|---|---|---|---|---|
| 120 | 6 | 74 | 61.7 | MMRN2 | 95 | 79.2 | BP20 | 80 | 66.7 |
| | 5 | 36 | 30.0 | ECM1 | 90 | 75.0 | BP15 | 28 | 23.3 |
| | 4 | 9 | 7.5 | MCAM | 61 | 50.8 | bmi | 2 | 1.7 |
| | 3 | 1 | 0.8 | PIGF | 49 | 40.8 | | | |
| | 2 | | 0.0 | ADAM12 | 31 | 25.8 | | | |
| | 1 | | 0.0 | COL6A3 | 26 | 21.7 | | | |

(continued)

| AJ | AK | AL | AM | AN | AO | AP | AQ | AR | AS |
|---|---|---|---|---|---|---|---|---|---|
|  |  |  |  | IGFALS | 25 | 20.8 |  |  |  |
|  |  |  |  | ENG | 18 | 15.0 |  |  |  |
|  |  |  |  | LNPEP | 14 | 11.7 |  |  |  |
|  |  |  |  | PRDX2 | 9 | 7.5 |  |  |  |
|  |  |  |  | QSOX1 | 7 | 5.8 |  |  |  |
|  |  |  |  | SPINT1 | 3 | 2.5 |  |  |  |
|  |  |  |  | CRP | 2 | 1.7 |  |  |  |
|  |  |  |  | LCAT | 2 | 1.7 |  |  |  |
|  |  |  |  | ALDOA | 1 | 0.8 |  |  |  |
|  |  |  |  | MAPRE1/3 | 1 | 0.8 |  |  |  |

**Table 12C**

| AJ | AK | AL | AM | AN | AO | AP | AQ | AR | AS |
|---|---|---|---|---|---|---|---|---|---|
| 78 | 6 | 40 | 51.3 | PIGF | 50 | 64.1 | BP15 | 33 | 42.3 |
|  | 5 | 27 | 34.6 | ADAM12 | 49 | 62.8 | BP20 | 22 | 28.2 |
|  | 4 | 9 | 11.5 | ECM1 | 37 | 47.4 | bmi | 7 | 9.0 |
|  | 3 | 2 | 2.6 | MMRN2 | 37 | 47.4 |  |  |  |
|  | 2 |  | 0.0 | IGFALS | 29 | 37.2 |  |  |  |
|  | 1 |  | 0.0 | QSOX1 | 21 | 26.9 |  |  |  |
|  |  |  |  | MCAM | 19 | 24.4 |  |  |  |
|  |  |  |  | ENG | 11 | 14.1 |  |  |  |
|  |  |  |  | SPINT1 | 6 | 7.7 |  |  |  |
|  |  |  |  | COL6A3 | 5 | 6.4 |  |  |  |
|  |  |  |  | PRDX2 | 5 | 6.4 |  |  |  |
|  |  |  |  | LNPEP | 5 | 6.4 |  |  |  |
|  |  |  |  | LCAT | 2 | 2.6 |  |  |  |
|  |  |  |  | CRP | 1 | 1.3 |  |  |  |
|  |  |  |  | ENPP2 | 0 | 0.0 |  |  |  |
|  |  |  |  | MAPRE1/3 | 0 | 0.0 |  |  |  |

**Table 12D**

| AJ | AK | AL | AM | AN | AO | AP | AQ | AR | AS |
|---|---|---|---|---|---|---|---|---|---|
| 62 | 6 | 38 | 61.3 | PIGF | 56 | 90.3 | BP20 | 31 | 50 |
|  | 5 | 24 | 38.7 | ADAM12 | 50 | 80.6 | BP15 | 16 | 25.8 |
|  | 4 |  | 0.0 | IGFALS | 35 | 56.5 | bmi | 8 | 12.9 |
|  | 3 |  | 0.0 | MMRN2 | 31 | 50 |  |  |  |
|  | 2 |  | 0.0 | ECM1 | 20 | 32.3 |  |  |  |

EP 2 791 683 B1

(continued)

| AJ | AK | AL | AM | AN | AO | AP | AQ | AR | AS |
|----|----|----|----|----|----|----|----|----|----|
|  | 1 |  | 0.0 | MCAM | 19 | 30.6 |  |  |  |
|  |  |  |  | QSOX1 | 10 | 16.1 |  |  |  |
|  |  |  |  | LNPEP | 4 | 6.5 |  |  |  |
|  |  |  |  | LCAT | 3 | 4.8 |  |  |  |
|  |  |  |  | COL6A3 | 1 | 1.6 |  |  |  |
|  |  |  |  | PRDX2 | 1 | 1.6 |  |  |  |
|  |  |  |  | CRP | 1 | 1.6 |  |  |  |

**Table 12E**

| AJ | AK | AL | AM | AN | AO | AP | AQ | AR | AS |
|----|----|----|----|----|----|----|----|----|----|
| 198 | 6 | 115 | 58.1 | MMRN2 | 155 | 78.3 | BP20 | 69 | 34.8 |
|  | 5 | 67 | 33.8 | ECM1 | 117 | 59.1 | BP15 | 68 | 34.3 |
|  | 4 | 15 | 7.6 | PIGF | 102 | 51.5 | bmi | 12 | 6.1 |
|  | 3 | 1 | 0.5 | ADAM12 | 91 | 46.0 |  |  |  |
|  | 2 |  | 0.0 | MCAM | 52 | 26.3 |  |  |  |
|  | 1 |  | 0.0 | COL6A3 | 51 | 25.8 |  |  |  |
|  |  |  |  | IGFALS | 37 | 18.7 |  |  |  |
|  |  |  |  | QSOX1 | 35 | 17.7 |  |  |  |
|  |  |  |  | SPINT1 | 32 | 16.2 |  |  |  |
|  |  |  |  | PRDX2 | 24 | 12.1 |  |  |  |
|  |  |  |  | ENG | 20 | 10.1 |  |  |  |
|  |  |  |  | LNPEP | 15 | 7.6 |  |  |  |
|  |  |  |  | CRP | 4 | 2.0 |  |  |  |
|  |  |  |  | ENPP2 | 2 | 1.0 |  |  |  |
|  |  |  |  | MAPRE1/3 | 2 | 1.0 |  |  |  |
|  |  |  |  | ALDOA | 2 | 1.0 |  |  |  |

**Table 12F**

| AJ | AK | AL | AM | AN | AO | AP | AQ | AR | AS |
|----|----|----|----|----|----|----|----|----|----|
| 2 | 6 | 2 | 100 | ADAM12 | 2 | 100 | BP15 | 1 | 50.0 |
|  | 5 |  | 0 | MCAM | 2 | 100 | BP20 | 1 | 50.0 |
|  | 4 |  | 0 | PIGF | 2 | 100 |  |  |  |
|  | 3 |  | 0 | IGFALS | 2 | 100 |  |  |  |
|  | 2 |  | 0 |  |  |  |  |  |  |
|  | 1 |  | 0 |  |  |  |  |  |  |

Further, **Table 12G** summarises illustrative, particularly well-performing test panels, which consist of TFF3 in combination with any one or more of BP, ADAM12, PIGF, and IGFALS. Single-marker performance of TFF3 is included to allow for comparison with the test panels embodying the principles of the invention. The data indicates that TFF3 may be a particularly well suited alternative for IGFALS in test panels embodying the principles of the invention.

**Table 12G**

| **Panel composition** (i.e. the panel consists of the recited constituents) (note that IGFALS-e herein stands for IGFALS measurement by ELISA) | **AUC** for predicting all PE at 20 weeks - (European cohort) |
|---|---|
| TFF3 | 0.63 |
| TFF3; BP | 0.71 |
| TFF3; BP; ADAM12 | 0.76 |
| TFF3; BP; ADAM12; PIGF | 0.79 |
| TFF3; BP; ADAM12; PIGF; IGFALS-e | 0.808 |

**Example 13: Further illustrative test panels for the prediction of PE**

The data and analyses in this example have been obtained using the combined European and Australasian case-control set as captured in Table 2, and applying the statistical analysis methods as elucidated below. Panels or combinations of markers and/or clinical parameters were obtained to develop models that estimate the probability of contracting preeclampsia.

Whereas the outcome of the test panels exemplified herein is the prediction of preeclampsia at 20 weeks of gestation, the test panels are useful throughout the second trimester, such as between 13 and 28 weeks of gestation, e.g., at 20 +/- 2, 20 +/- 1, 15 +/- 2 or 15 +/- 1 weeks of gestation, and can even be applied with success in the first trimester.

Multivariate models that predict pre-eclampsia were computed using logistic regression (Royston et al. 2009, Prognosis and prognostic research: Developing a prognostic model, BMJ 2009: 338:b604).

The covariates or predictors (biomarkers and parameters) used were robust clinical parameters measured during pregnancy and the concentration of protein biomarkers in blood measured either with ELISA or Multiple Reaction Monitoring (MRM) (see description of MASSTERCLASS® assays in Example 2).

The covariates were normalised. The binary variables were coded 0/1 , the analyte concentrations and relative concentrations (MASSTERCLASS® measurements) were log- transformed.

Algorithms were made with any combination of two, three, four, five and six of the above mentioned covariates. The predictive performance of the algorithms was computed in the same samples.

The Wald test was used to check the statistical significance of the coefficients in the algorithms. Two predictive performances were computed: the C-statistic (also referred to as area under the curve and AUC) and the sensitivity at 20% positive predictive value (PPV or "rule-in" performance).

PPV is computed as follows (see also Example 3):

$$PPV = TPR <*> p \ / \ (TPR <*> p + FPR \ (1 - p))$$

where:

p is the prevalence of the disease (proportion of cases in the population),

TPR is the True Positive Rate or sensitivity or case detection rate

TNR is the True Negative Rate or specificity or control detection rate

FNR is the False Negative Rate (FNR = 1 - TPR).

PPV is used as they can be more clinically relevant. The sensitivity at 20% PPV gives the proportion of cases correctly predicted (or prognosed, or diagnosed) if one fifth of the patient predicted positive develop pre-eclampsia.

The performances were computed for 1) the prediction of pre-eclampsia (i.e., all pre-eclampsia), 2) the prediction of preterm pre-eclampsia (i.e., clinical manifestation before 37 weeks of gestation), and 3) the prediction of term pre-eclampsia (i.e., clinical manifestation at or after 37 weeks of gestation). PPV was not computed for the second and third outcomes due to a limited number of cases.

Some predictors were having missing values. Algorithms that could not compute a risk index for at least 85% of patients were not considered.

Distinct analyses were performed. A first group of analyses was focused on test panels consisting of between 2 and 6 constituents and containing the most clinically relevant parameters (parameters that are routinely collected in a reliable fashion) and protein biomarker concentrations (ELISA readouts) or protein relative concentrations (MASSTERCLASS® readouts). The best performing algorithms within this group of analyses comprised or consisted of combinations of the following covariates: blood pressure (at 15 or 20 weeks), IGFALS, MCAM, SPINT1 , MMRN2, ADAM12, ENG, PIGF.

The performance of the algorithms that used a combination of these predictors is given in tables **13A to 13E.** Single-marker performances may be included in the tables to allow for comparison with the test panels embodying the principles of the invention. The following abbreviations are used in **Tables 13A to 13H: AUC:** area under the ROC curve; **ICI:** lower confidence interval; **uCI:** upper confidence interval. The following column denotations are used in the tables. **Panel composition:** constituents forming up a panel (i.e. the panel consists of the recited constituents); **CA:** AUC for predicting all PE (i.e., without distinction between preterm and term PE) at 20 weeks - combined set (combined Australasian and European cohorts); **CB:** ICI AUC for predicting all PE at 20 weeks - combined set (combined Australasian and European cohorts); **CC:** uCI AUC for predicting all PE at 20 weeks - combined set (combined Australasian and European cohorts); **CD:** AUC for predicting preterm PE at 20 weeks - combined set (combined Australasian and European cohorts); **CE:** ICI AUC for predicting preterm PE at 20 weeks - combined set (combined Australasian and European cohorts); **CF:** uCI AUC for predicting preterm PE at 20 weeks - combined set (combined Australasian and European cohorts); **CG:** AUC for predicting term PE at 20 weeks - combined set (combined Australasian and European cohorts); **CH:** ICI AUC for predicting term PE at 20 weeks - combined set (combined Australasian and European cohorts); **CI:** uCI AUC for predicting term PE at 20 weeks - combined set (combined Australasian and European cohorts).

**Table 13A** summarises illustrative, particularly well-performing test panels, which consist of IGFALS in combination with any one or more of BP, MCAM, MMRN2, PIGF, ADAM 12, SPINT1 and ENG.

**Table 13A**

| Panel composition | CA | CB | CC | CD | CE | CF | CG | CH | CI |
|---|---|---|---|---|---|---|---|---|---|
| IGFALS | 0.72 | 0.67 | 0.76 | 0.76 | 0.69 | 0.83 | 0.70 | 0.64 | 0.76 |
| IGFALS+BP | 0.74 | 0.70 | 0.79 | 0.80 | 0.73 | 0.87 | 0.72 | 0.67 | 0.78 |
| IGFALS+MCAM | 0.73 | 0.68 | 0.78 | 0.77 | 0.70 | 0.85 | 0.71 | 0.65 | 0.77 |
| IGFALS+MCAM+BP | 0.75 | 0.71 | 0.80 | 0.81 | 0.75 | 0.88 | 0.73 | 0.67 | 0.78 |
| IGFALS+MCAM+BP+ENG+SPINT 1 | 0.79 | 0.75 | 0.84 | 0.89 | 0.84 | 0.95 | 0.76 | 0.70 | 0.81 |
| IGFALS+MCAM+BP+ENG+SPINT 1+MMRN2 | 0.80 | 0.76 | 0.85 | 0.92 | 0.88 | 0.97 | 0.76 | 0.70 | 0.81 |
| IGFALS+MCAM+BP+PIGF | 0.77 | 0.73 | 0.82 | 0.86 | 0.80 | 0.92 | 0.77 | 0.72 | 0.81 |
| IGFALS+MCAM+BP+ADAM12 | 0.77 | 0.73 | 0.82 | 0.85 | 0.78 | 0.91 | 0.74 | 0.69 | 0.80 |
| IGFALS+MCAM+BP+PIGF+ ADAM12 | 0.80 | 0.76 | 0.84 | 0.87 | 0.80 | 0.94 | 0.77 | 0.72 | 0.82 |
| IGFALS+MCAM+BP+PIGF+ ADAM12+MMRN2 | 0.81 | 0.77 | 0.85 | 0.89 | 0.83 | 0.95 | 0.79 | 0.74 | 0.83 |

**Table 13B** summarises illustrative, particularly well-performing test panels, which consist of ADAM 12 in combination with any one or more of BP, IGFALS, MCAM, MMRN2 and PIGF.

**Table 13B**

| Panel composition | CA | CB | CC | CD | CE | CF | CG | CH | CI |
|---|---|---|---|---|---|---|---|---|---|
| ADAM12 | 0.63 | 0.58 | 0.68 | 0.72 | 0.65 | 0.80 | 0.59 | 0.53 | 0.66 |
| ADAM12;PIGF | 0.70 | 0.65 | 0.75 | 0.81 | 0.73 | 0.89 | 0.66 | 0.60 | 0.72 |
| BP;ADAM12 | 0.71 | 0.66 | 0.76 | 0.80 | 0.72 | 0.89 | 0.67 | 0.61 | 0.72 |
| ADAM12;IGFALS | 0.73 | 0.68 | 0.77 | 0.80 | 0.74 | 0.86 | 0.70 | 0.64 | 0.76 |
| ADAM12;MCAM | 0.66 | 0.61 | 0.72 | 0.75 | 0.67 | 0.83 | 0.63 | 0.57 | 0.69 |

(continued)

| Panel composition | CA | CB | CC | CD | CE | CF | CG | CH | CI |
|---|---|---|---|---|---|---|---|---|---|
| MMRN2;ADAM12 | 0.64 | 0.58 | 0.69 | 0.75 | 0.67 | 0.82 | 0.59 | 0.53 | 0.66 |
| ADAM12;IGFALS;PIGF | 0.76 | 0.72 | 0.81 | 0.85 | 0.79 | 0.92 | 0.73 | 0.68 | 0.78 |
| MMRN2;ADAM12;IGFALS | 0.75 | 0.70 | 0.79 | 0.85 | 0.80 | 0.90 | 0.71 | 0.65 | 0.76 |
| BP;ADAM12;PIGF | 0.76 | 0.71 | 0.80 | 0.84 | 0.77 | 0.92 | 0.72 | 0.67 | 0.77 |
| ADAM12;MCAM;PIGF | 0.72 | 0.67 | 0.77 | 0.84 | 0.76 | 0.92 | 0.68 | 0.63 | 0.74 |
| BP;ADAM12;IGFALS | 0.76 | 0.71 | 0.80 | 0.83 | 0.77 | 0.90 | 0.73 | 0.68 | 0.78 |
| BP;MMRN2;ADAM12 | 0.72 | 0.67 | 0.77 | 0.83 | 0.76 | 0.90 | 0.68 | 0.62 | 0.74 |
| MMRN2;ADAM12;PIGF | 0.71 | 0.65 | 0.76 | 0.82 | 0.74 | 0.91 | 0.66 | 0.60 | 0.72 |
| BP;ADAM12;MCAM | 0.72 | 0.68 | 0.77 | 0.82 | 0.74 | 0.90 | 0.69 | 0.63 | 0.74 |
| ADAM12;IGFALS;MCAM | 0.75 | 0.70 | 0.79 | 0.81 | 0.74 | 0.88 | 0.72 | 0.66 | 0.77 |
| MMRN2;ADAM12;MCAM | 0.67 | 0.62 | 0.72 | 0.76 | 0.68 | 0.85 | 0.64 | 0.58 | 0.70 |
| MMRN2;ADAM12;IGFALS;PIG F | 0.78 | 0.74 | 0.83 | 0.88 | 0.82 | 0.94 | 0.75 | 0.70 | 0.80 |
| BP;MMRN2;ADAM12;IGFALS | 0.77 | 0.73 | 0.82 | 0.87 | 0.82 | 0.92 | 0.73 | 0.68 | 0.79 |
| ADAM12;IGFAL.S;MCAM;PIGF | 0.78 | 0.74 | 0.83 | 0.87 | 0.81 | 0.93 | 0.75 | 0.70 | 0.80 |
| BP;MMRN2;ADAM12;PIGF | 0.76 | 0.71 | 0.81 | 0.87 | 0.79 | 0.94 | 0.72 | 0.67 | 0.78 |
| BP;ADAM12;IGFALS;PIGF | 0.79 | 0.75 | 0.83 | 0.86 | 0.80 | 0.93 | 0.76 | 0.71 | 0.81 |
| BP;ADAM12;MCAM;PIGF | 0.76 | 0.72 | 0.81 | 0.86 | 0.78 | 0.94 | 0.73 | 0.68 | 0.78 |
| MMRN2;ADAM12;MCAM;PIGF | 0.73 | 0.68 | 0.78 | 0.85 | 0.77 | 0.94 | 0.69 | 0.63 | 0.74 |
| MMRN2;ADAM12;IGFALS;MC AM | 0.76 | 0.71 | 0.81 | 0.85 | 0.79 | 0.91 | 0.73 | 0.67 | 0.78 |
| BP;ADAM12;IGFALS;MCAM | 0.77 | 0.73 | 0.82 | 0.85 | 0.78 | 0.91 | 0.75 | 0.69 | 0.80 |
| BP;MMRN2;ADAM12;MCAM | 0.73 | 0.68 | 0.78 | 0.84 | 0.77 | 0.92 | 0.69 | 0.63 | 0.75 |
| BP;MMRN2;ADAM12;IGFALS; PIGF | 0.80 | 0.76 | 0.84 | 0.90 | 0.84 | 0.95 | 0.77 | 0.72 | 0.82 |
| MMRN2:ADAM12;IGFALS;MC AM;PIGF | 0.80 | 0.76 | 0.84 | 0.90 | 0.84 | 0.95 | 0.77 | 0.72 | 0.82 |
| BP;MMRN2;ADAM12;MCAM;P IGF | 0.77 | 0.73 | 0.82 | 0.89 | 0.81 | 0.96 | 0.73 | 0.68 | 0.79 |
| BP;ADAM12;IGFALS;MCAM;P IGF | 0.80 | 0.76 | 0.84 | 0.88 | 0.82 | 0.94 | 0.77 | 0.73 | 0.82 |
| BP;MMRN2;ADAM12;IGFALS; MCAM | 0.78 | 0.74 | 0.83 | 0.88 | 0.83 | 0.93 | 0.75 | 0.69 | 0.80 |
| BP;MMRN2;ADAM12;IGFALS; MCAM; PIGF | 0.82 | 0.77 | 0.86 | 0.91 | 0.86 | 0.96 | 0.78 | 0.73 | 0.83 |

**Table 13C** summarises illustrative, particularly well-performing test panels, which consist of PIGF in combination with any one or more of BP, IGFALS, MCAM and MMRN2.

**Table 13C**

| Panel composition | CA | CB | CC | CD | CE | CF | CG | CH | CI |
|---|---|---|---|---|---|---|---|---|---|
| PIGF | 0.64 | 0.59 | 0.69 | 0.73 | 0.65 | 0.82 | 0.60 | 0.54 | 0.66 |
| IGFALS;PIGF | 0.75 | 0.70 | 0.79 | 0.82 | 0.75 | 0.89 | 0.72 | 0.67 | 0.77 |
| BP;PIGF | 0.70 | 0.65 | 0.75 | 0.80 | 0.72 | 0.88 | 0.67 | 0.61 | 0.72 |
| MCAM;PIGF | 0.67 | 0.62 | 0.72 | 0.77 | 0.69 | 0.85 | 0.63 | 0.57 | 0.69 |
| MMRN2;PIGF | 0.64 | 0.59 | 0.69 | 0.74 | 0.66 | 0.82 | 0.61 | 0.55 | 0.67 |

(continued)

| Panel composition | CA | CB | CC | CD | CE | CF | CG | CH | CI |
|---|---|---|---|---|---|---|---|---|---|
| MMRN2;IGFALS;PIGF | 0.76 | 0.72 | 0.81 | 0.85 | 0.79 | 0.91 | 0.73 | 0.68 | 0.79 |
| IGFALS;MCAM;PIGF | 0.76 | 0.72 | 0.81 | 0.84 | 0.78 | 0.91 | 0.73 | 0.68 | 0.79 |
| BP;IGFALS;PIGF | 0.77 | 0.72 | 0.81 | 0.84 | 0.78 | 0.90 | 0.74 | 0.69 | 0.79 |
| BP;MCAM;PIGF | 0.71 | 0.66 | 0.76 | 0.82 | 0.74 | 0.89 | 0.68 | 0.62 | 0.74 |
| BP;MMRN2;PIGF | 0.71 | 0.66 | 0.76 | 0.81 | 0.74 | 0.89 | 0.67 | 0.61 | 0.73 |
| MMRN2;MCAM;PIGF | 0.67 | 0.62 | 0.72 | 0.77 | 0.69 | 0.85 | 0.64 | 0.58 | 0.70 |
| BP;MMRN2;IGFALS;PIGF | 0.78 | 0.74 | 0.82 | 0.87 | 0.82 | 0.93 | 0.75 | 0.70 | 0.80 |
| MMRN2;IGFALS;MCAM; PIGF | 0.78 | 0.74 | 0.82 | 0.87 | 0.81 | 0.93 | 0.75 | 0.70 | 0.80 |
| BP;IGFALS;MCAM;PIGF | 0.78 | 0.73 | 0.82 | 0.86 | 0.80 | 0.92 | 0.75 | 0.70 | 0.80 |
| BP;MMRN2;MCAM;PIGF | 0.72 | 0.67 | 0.77 | 0.83 | 0.76 | 0.91 | 0.68 | 0.62 | 0.74 |
| BP;MMRN2;IGFALS; MCAM;PIGF | 0.79 | 0.75 | 0.84 | 0.89 | 0.84 | 0.94 | 0.76 | 0.70 | 0.81 |

**Table 13D** summarises illustrative, particularly well-performing test panels, which consist of ENG in combination with any one or more of BP, IGFALS, MCAM, MMRN2 and SPI NT1 . Synergism between ENG and SPI NT1 can be particularly well noted here. Indeed, for the best performing algorithms in the present analyses, SPINT1 was only having a significant effect when combined with ENG, when significance was estimated using the Wald test, and a cutoff for the p value of 0.05. PIGF did not appear to increase significantly the discriminative power of ENG.

**Table 13D**

| Panel composition | CA | CB | CC | CD | CE | CF | CG | CH | CI |
|---|---|---|---|---|---|---|---|---|---|
| ENG | 0.61 | 0.55 | 0.66 | 0.71 | 0.61 | 0.81 | 0.57 | 0.51 | 0.63 |
| ENG;SPINT1 | 0.68 | 0.63 | 0.73 | 0.83 | 0.76 | 0.90 | 0.62 | 0.56 | 0.68 |
| ENG;IGFALS | 0.73 | 0.68 | 0.77 | 0.80 | 0.72 | 0.87 | 0.70 | 0.64 | 0.75 |
| BP;ENG | 0.70 | 0.65 | 0.75 | 0.78 | 0.70 | 0.86 | 0.66 | 0.60 | 0.72 |
| ENG;MCAM | 0.67 | 0.62 | 0.73 | 0.74 | 0.64 | 0.83 | 0.65 | 0.59 | 0.71 |
| MMRN2;ENG | 0.62 | 0.57 | 0.68 | 0.73 | 0.63 | 0.83 | 0.58 | 0.52 | 0.65 |
| ENG;SPINT1;IGFALS | 0.76 | 0.71 | 0.80 | 0.88 | 0.82 | 0.94 | 0.71 | 0.66 | 0.77 |
| BP;ENG;SPINT1 | 0.73 | 0.68 | 0.78 | 0.86 | 0.79 | 0.92 | 0.68 | 0.62 | 0.74 |
| MMRN2;ENG;IGFALS | 0.74 | 0.70 | 0.79 | 0.84 | 0.78 | 0.90 | 0.71 | 0.65 | 0.76 |
| ENG;SPINT3;MCAM | 0.72 | 0.67 | 0.77 | 0.84 | 0.77 | 0.91 | 0.67 | 0.62 | 0.73 |
| MMRN2;ENG;SPINT1 | 0.68 | 0.63 | 0.73 | 0.83 | 0.76 | 0.90 | 0.62 | 0.57 | 0.68 |
| BP;ENG;IGFALS | 0.76 | 0.71 | 0.80 | 0.83 | 0.76 | 0.89 | 0.73 | 0.67 | 0.78 |
| ENG;IGFALS;MCAM | 0.75 | 0.70 | 0.80 | 0.83 | 0.75 | 0.90 | 0.72 | 0.67 | 0.78 |
| BP;MMRN2;ENG | 0.70 | 0.65 | 0.76 | 0.81 | 0.74 | 0.88 | 0.66 | 0.60 | 0.72 |
| BP;ENG;MCAM | 0.74 | 0.69 | 0.78 | 0.81 | 0.73 | 0.89 | 0.71 | 0.65 | 0.76 |
| MMRN2;ENG;MCAM | 0.67 | 0.62 | 0.72 | 0.74 | 0.64 | 0.84 | 0.64 | 0.58 | 0.70 |
| MMRN2;ENG;SPINT1;IGFALS | 0.77 | 0.73 | 0.82 | 0.90 | 0.86 | 0.95 | 0.72 | 0.66 | 0.78 |
| ENG;SPINT1;IGFALS;MCAM | 0.78 | 0.74 | 0.83 | 0.89 | 0.84 | 0.95 | 0.74 | 0.68 | 0.79 |
| BP;ENG;SPINT1;IGFALS | 0.77 | 0.73 | 0.82 | 0.89 | 0.84 | 0.94 | 0.73 | 0.67 | 0.78 |
| BP;MMRN2;ENG;IGFALS | 0.77 | 0.72 | 0.82 | 0.87 | 0.82 | 0.92 | 0.73 | 0.67 | 0.79 |

(continued)

| Panel composition | CA | CB | CC | CD | CE | CF | CG | CH | CI |
|---|---|---|---|---|---|---|---|---|---|
| BP;ENG;SPINT1;MCAM | 0.76 | 0.71 | 0.81 | 0.87 | 0.80 | 0.94 | 0.72 | 0.67 | 0.78 |
| BP;MMRN2;ENG;SPINT1 | 0.73 | 0.69 | 0.78 | 0.87 | 0.80 | 0.93 | 0.68 | 0.62 | 0.74 |
| MMRN2;ENG;IGFALS;MCAM | 0.76 | 0.72 | 0.81 | 0.85 | 0.78 | 0.92 | 0.73 | 0.67 | 0.79 |
| BP;ENG;IGFALS;MCAM | 0.78 | 0.73 | 0.82 | 0.85 | 0.78 | 0.92 | 0.75 | 0.69 | 0.81 |
| MMRN2;ENG;SPINT1;MCAM | 0.72 | 0.67 | 0.77 | 0.84 | 0.76 | 0.91 | 0.67 | 0.62 | 0.73 |
| BP;MMRN2;ENG;MCAM | 0.74 | 0.68 | 0.79 | 0.83 | 0.75 | 0.90 | 0.70 | 0.64 | 0.76 |
| BP;MMRN2;ENG;SPINT1;IGFALS | 0.79 | 0.75 | 0.83 | 0.92 | 0.87 | 0.96 | 0.74 | 0.69 | 0.80 |
| MMRN2;ENG;SPINT1;ICFALS; MCAM | 0.79 | 0.74 | 0.84 | 0.91 | 0.86 | 0.96 | 0.75 | 0.69 | 0.80 |
| BP;ENG;SPINT1;IGFALS;MCAM | 0.79 | 0.75 | 0.84 | 0.91 | 0.85 | 0.96 | 0.75 | 0.70 | 0.81 |
| BP;MMRN2;ENG;IGFALS;MCAM | 0.79 | 0.74 | 0.83 | 0.88 | 0.82 | 0.94 | 0.75 | 0.69 | 0.81 |
| BP;MMRN2;ENG;SPINT1;MCAM | 0.76 | 0.71 | 0.81 | 0.88 | 0.81 | 0.94 | 0.72 | 0.66 | 0.78 |
| BP;MMRN2;ENG;SPINT1;IGFALS; MCAM | 0.81 | 0.76 | 0.85 | 0.93 | 0.88 | 0.97 | 0.76 | 0.71 | 0.82 |

**Table 13E** summarises illustrative, particularly well-performing test panels, which do not contain ADAM12, PIGF and ENG. In particular, these panels consist of a combination of two or more of BP, IGFALS, MCAM and MMRN2. As can be seen, these panels do not contain SPINT1. However, adding SPINT1 to any single marker, any 2-marker panel or any 3-marker panel leads to a significant improvement. SPINT1 is particularly relevant in combination with ENG (see Table 13D).

**Table 13E**

| Panel composition | CA | CB | CC | CD | CE | CF | CG | CH | CI |
|---|---|---|---|---|---|---|---|---|---|
| IGFALS | 0.72 | 0.67 | 0.76 | 0.76 | 0.69 | 0.83 | 0.70 | 0.64 | 0.76 |
| BP | 0.66 | 0.61 | 0.71 | 0.72 | 0.64 | 0.81 | 0.63 | 0.57 | 0.69 |
| MCAM | 0.58 | 0.53 | 0.64 | 0.61 | 0.51 | 0.70 | 0.57 | 0.51 | 0.63 |
| SPINT1 | 0.55 | 0.50 | 0.61 | 0.63 | 0.54 | 0.73 | 0.52 | 0.46 | 0.59 |
| MMRN2 | 0.52 | 0.47 | 0.58 | 0.56 | 0.46 | 0.65 | 0.51 | 0.44 | 0.57 |
| MMRN2;IGFALS | 0.73 | 0.69 | 0.78 | 0.81 | 0.75 | 0.87 | 0.70 | 0.65 | 0.76 |
| BP;IGFALS | 0.74 | 0.70 | 0.79 | 0.80 | 0.73 | 0.87 | 0.72 | 0.67 | 0.78 |
| IGFALS;MCAM | 0.73 | 0.68 | 0.78 | 0.77 | 0.70 | 0.85 | 0.71 | 0.65 | 0.77 |
| BP;MMRN2 | 0.66 | 0.61 | 0.72 | 0.75 | 0.66 | 0.83 | 0.63 | 0.57 | 0.69 |
| BP;MCAM | 0.67 | 0.62 | 0.73 | 0.75 | 0.66 | 0.83 | 0.65 | 0.59 | 0.71 |
| MMRN2;MCAM | 0.59 | 0.54 | 0.64 | 0.61 | 0.52 | 0.71 | 0.58 | 0.52 | 0.64 |
| BP;MMRN2;IGFALS | 0.76 | 0.71 | 0.80 | 0.84 | 0.78 | 0.89 | 0.72 | 0.67 | 0.78 |
| BP;IGFALS;MCAM | 0.75 | 0.71 | 0.80 | 0.81 | 0.74 | 0.88 | 0.73 | 0.68 | 0.79 |
| MMRN2;IGFALS;MCAM | 0.74 | 0.70 | 0.79 | 0.81 | 0.74 | 0.88 | 0.72 | 0.66 | 0.78 |
| BP;MMRN2;MCAM | 0.68 | 0.63 | 0.74 | 0.76 | 0.68 | 0.85 | 0.65 | 0.59 | 0.71 |
| BP;MMRN2;IGFALS;MCAM | 0.77 | 0.72 | 0.81 | 0.84 | 0.79 | 0.90 | 0.74 | 0.68 | 0.79 |

In a second group of analyses, the performance of all test panels with two predictors was computed. All available clinical parameters, protein biomarker concentrations (ELISA readouts) and relative protein concentrations (MASSTERCLASS® readouts) were used as predictors. Algorithms for all combinations of two of these predictors were computed using logistic regression. The performance was computed for all these algorithms as described above. The Wald test was used to check the statistical significance of the covariates in the algorithms. Algorithms with a p value for the Wald test for any of the covariates above 0.05 were ignored. Only the best performing algorithms were retained and only algorithms giving an AUC higher than or equal to 0.75 were retained (AUC for either of the three outcomes, pre-eclampsia, preterm pre-eclampsia or term pre-eclampsia). The performance of the algorithms consisting of a combination of 2 covariates, with an AUC higher than or equal to 0.75 and p values for the Wald test for all the covariates below or equal to 0.05, is captured in **Table 13F.**

**Table 13F**

| Panel composition | CA | CB | CC | CD | CE | CF | CG | CH | CI |
|---|---|---|---|---|---|---|---|---|---|
| IGFALS;PIGF | 0.75 | 0.70 | 0.79 | 0.82 | 0.75 | 0.89 | 0.72 | 0.67 | 0.77 |
| BP;IGFALS | 0.74 | 0.70 | 0.79 | 0.80 | 0.73 | 0.87 | 0.72 | 0.67 | 0.78 |
| MMRN2:IGFALS | 0.73 | 0.69 | 0.78 | 0.81 | 0.75 | 0.87 | 0.70 | 0.65 | 0.76 |
| alcoh;IGFALS | 0.73 | 0.69 | 0.78 | 0.78 | 0.71 | 0.85 | 0.72 | 0.66 | 0.77 |
| fhpet;IGFALS | 0.73 | 0.68 | 0.77 | 0.79 | 0.72 | 0.85 | 0.70 | 0.65 | 0.76 |
| SPINT1;IGFALS | 0.73 | 0.68 | 0.77 | 0.79 | 0.72 | 0.86 | 0.70 | 0.64 | 0.76 |
| IGFALS;pbwgt | 0.73 | 0.68 | 0.78 | 0.78 | 0.71 | 0.85 | 0.70 | 0.65 | 0.76 |
| MAPRE1/3; IGFALS | 0.73 | 0.68 | 0.77 | 0.78 | 0.70 | 0.85 | 0.71 | 0.65 | 0.76 |
| fihd; IGFALS | 0.72 | 0.68 | 0.77 | 0.76 | 0.69 | 0.84 | 0.71 | 0.65 | 0.77 |
| CST3;IGFALS | 0.72 | 0.68 | 0.77 | 0.77 | 0.70 | 0.85 | 0.70 | 0.65 | 0.76 |
| IGFALS;vagbl | 0.72 | 0.68 | 0.77 | 0.76 | 0.68 | 0.83 | 0.71 | 0.65 | 0.77 |
| BP;CST3 | 0.71 | 0.66 | 0.76 | 0.75 | 0.67 | 0.83 | 0.69 | 0.64 | 0.75 |
| BP;PIGF | 0.70 | 0.65 | 0.75 | 0.80 | 0.72 | 0.88 | 0.67 | 0.61 | 0.72 |
| ADAM12; *waist* | 0.70 | 0.65 | 0.75 | 0.75 | 0.66 | 0.84 | 0.68 | 0.62 | 0.73 |
| alcoh;BP | 0.70 | 0.65 | 0.75 | 0.77 | 0.69 | 0.85 | 0.67 | 0.61 | 0.73 |
| ENG;*waist* | 0.70 | 0.65 | 0.75 | 0.75 | 0.67 | 0.84 | 0.68 | 0.62 | 0.73 |
| PIGF;*waist* | 0.69 | 0.63 | 0.74 | 0.76 | 0.68 | 0.83 | 0.66 | 0.60 | 0.72 |
| BP;PRDX2 | 0.68 | 0.63 | 0.74 | 0.77 | 0.69 | 0.85 | 0.65 | 0.59 | 0.72 |
| PRCP;PIGF | 0.68 | 0.63 | 0.73 | 0.75 | 0.66 | 0.83 | 0.65 | 0.59 | 0.71 |
| BP;MCAM | 0.67 | 0.62 | 0.73 | 0.75 | 0.66 | 0.83 | 0.65 | 0.59 | 0.71 |
| BP;pbwgt | 0.67 | 0.62 | 0.72 | 0.75 | 0.66 | 0.83 | 0.64 | 0.58 | 0.70 |
| ADAM12:SPINT1 | 0.67 | 0.62 | 0.72 | 0.82 | 0.76 | 0.89 | 0.61 | 0.55 | 0.67 |

In a third group of analyses, the performance of all test panels with three predictors was computed. All available clinical parameters, protein biomarker concentrations (ELISA readouts) and relative protein concentrations (MASSTERCLASS® readouts) were used as predictors. Algorithms for all combinations of three of these predictors were computed using logistic regression. The performance was computed for all these algorithms as described above. The Wald test was used to check the statistical significance of the covariates in the algorithms. Algorithms with a p value for the Wald test for any of the covariates above 0.05 were ignored. Only the best performing algorithms were retained and only algorithms giving an AUC higher than or equal to 0.80 were retained (AUC for either of the three outcomes, pre-eclampsia, preterm pre-eclampsia or term pre- eclampsia). The performance of the algorithms consisting of a combination of 3 covariates, with an AUC higher than or equal to 0.80 or 0.85 and p values for the Wald test for all the covariates below or equal to 0.05, is captured in **Tables 13G and 13H,** respectively.

**Table 13G**

| Panel composition | CA | CB | CC | CD | CE | CF | CG | CH | CI |
|---|---|---|---|---|---|---|---|---|---|
| BP;IGFALS;PIGF | 0.77 | 0.72 | 0.81 | 0.84 | 0.78 | 0.90 | 0.74 | 0.69 | 0.79 |
| alcoh;BP;IGFALS | 0.77 | 0.72 | 0.81 | 0.82 | 0.75 | 0.88 | 0.74 | 0.69 | 0.80 |
| MMRN2;IGFALS;PIGF | 0.76 | 0.72 | 0.81 | 0.85 | 0.79 | 0.91 | 0.73 | 0.68 | 0.79 |
| IGFALS;MCAM;PIGF | 0.76 | 0.72 | 0.81 | 0.84 | 0.78 | 0.91 | 0.73 | 0.68 | 0.79 |
| ADAM12;IGFALS;PIGF | 0.76 | 0.72 | 0.81 | 0.85 | 0.79 | 0.92 | 0.73 | 0.68 | 0.78 |
| bmi;ENG;IGFALS | 0.76 | 0.72 | 0.81 | 0.81 | 0.73 | 0.88 | 0.74 | 0.69 | 0.79 |
| BP;ADAM12;IGFALS | 0.76 | 0.71 | 0.80 | 0.83 | 0.77 | 0.90 | 0.73 | 0.68 | 0.78 |
| alcoh;IGFALS;PIGF | 0.76 | 0.72 | 0.80 | 0.84 | 0.77 | 0.90 | 0.73 | 0.68 | 0.78 |
| ENG;SPINT1;IGFALS | 0.76 | 0.71 | 0.80 | 0.88 | 0.82 | 0.94 | 0.71 | 0.66 | 0.77 |
| BP;MMRN2;IGFALS | 0.76 | 0.71 | 0.80 | 0.84 | 0.78 | 0.89 | 0.72 | 0.67 | 0.78 |
| CST3;IGFALS;PIGF | 0.76 | 0.71 | 0.80 | 0.83 | 0.76 | 0.90 | 0.73 | 0.68 | 0.78 |
| BP;ENG;IGFALS | 0.76 | 0.71 | 0.80 | 0.83 | 0.76 | 0.89 | 0.73 | 0.67 | 0.78 |
| BP;ADAM12;PIGF | 0.76 | 0.71 | 0.80 | 0.84 | 0.77 | 0.92 | 0.72 | 0.67 | 0.77 |
| fhpet;IGFALS;PIGF | 0.76 | 0.71 | 0.80 | 0.83 | 0.77 | 0.90 | 0.73 | 0.67 | 0.78 |
| IGFALS;pbwgt;PIGF | 0.75 | 0.71 | 0.80 | 0.83 | 0.75 | 0.90 | 0.73 | 0.67 | 0.78 |
| fihd;IGFALS;PIGF | 0.75 | 0.71 | 0.80 | 0.82 | 0.75 | 0.89 | 0.73 | 0.68 | 0.78 |
| IGFALS;ROBO4;PIGF | 0.75 | 0.71 | 0.80 | 0.83 | 0.77 | 0.89 | 0.73 | 0.67 | 0.78 |
| BP;fihd;IGFALS | 0.75 | 0.71 | 0.80 | 0.80 | 0.74 | 0.87 | 0.74 | 0.68 | 0.79 |
| bmi;ADAM12;IGFALS | 0.75 | 0.71 | 0.80 | 0.80 | 0.74 | 0.87 | 0.73 | 0.68 | 0.78 |
| ENC;IGFALS;*waist* | 0.75 | 0.71 | 0.80 | 0.81 | 0.73 | 0.88 | 0.73 | 0.68 | 0.78 |
| BP; IGFALS; pbwgt | 0.75 | 0.71 | 0.80 | 0.81 | 0.75 | 0.88 | 0.73 | 0.67 | 0.79 |
| BP;IGFALS;MCAM | 0.75 | 0.71 | 0.80 | 0.81 | 0.74 | 0.88 | 0.73 | 0.68 | 0.79 |
| ENG;IGFALS;MCAM | 0.75 | 0.70 | 0.80 | 0.83 | 0.75 | 0.90 | 0.72 | 0.67 | 0.78 |
| alcoh;MMRN2;IGFALS | 0.75 | 0.71 | 0.80 | 0.83 | 0.78 | 0.88 | 0.72 | 0.67 | 0.77 |
| BP;CST3;IGFALS | 0.75 | 0.71 | 0.80 | 0.80 | 0.74 | 0.87 | 0.73 | 0.68 | 0.78 |
| BP;fhpet;IGFALS | 0.75 | 0.70 | 0.79 | 0.81 | 0.74 | 0.87 | 0.73 | 0.67 | 0.78 |
| ADAM12;IGFALS;*waist* | 0.75 | 0.71 | 0.79 | 0.80 | 0.74 | 0.87 | 0.73 | 0.68 | 0.78 |
| BP;LNPEP;IGFALS | 0.75 | 0.70 | 0.79 | 0.81 | 0.74 | 0.88 | 0.72 | 0.67 | 0.78 |
| alcoh;fhpet;IGFALS | 0.75 | 0.70 | 0.79 | 0.80 | 0.73 | 0.87 | 0.73 | 0.68 | 0.78 |
| BP;IGFALS;ROBO4 | 0.75 | 0.70 | 0.79 | 0.82 | 0.75 | 0.88 | 0.72 | 0.66 | 0.77 |
| MMRN2;ADAM12;IGFALS | 0.75 | 0.70 | 0.79 | 0.85 | 0.80 | 0.90 | 0.71 | 0.65 | 0.76 |
| ADAM12;SPINT1;IGFALS | 0.75 | 0.70 | 0.79 | 0.86 | 0.80 | 0.91 | 0.70 | 0.65 | 0.76 |
| MMRN2;CST3;IGFALS | 0.75 | 0.70 | 0.79 | 0.81 | 0.75 | 0.87 | 0.72 | 0.66 | 0.78 |
| ADAM12;IGFALS;MCAM | 0.75 | 0.70 | 0.79 | 0.81 | 0.74 | 0.88 | 0.72 | 0.66 | 0.77 |
| fhpet;MMRN12;IGFALS | 0.75 | 0.70 | 0.79 | 0.83 | 0.78 | 0.89 | 0.71 | 0.66 | 0.77 |
| alcoh;SPINT1;IGFALS | 0.74 | 0.70 | 0.79 | 0.81 | 0.74 | 0.87 | 0.72 | 0.67 | 0.77 |
| MMRN2;IGFALS;MCAM | 0.74 | 0.70 | 0.79 | 0.81 | 0.74 | 0.88 | 0.72 | 0.66 | 0.78 |
| bmi;ADAM12;PIGF | 0.74 | 0.70 | 0.79 | 0.81 | 0.73 | 0.89 | 0.72 | 0.66 | 0.77 |

(continued)

| Panel composition | CA | CB | CC | CD | CE | CF | CG | CH | CI |
|---|---|---|---|---|---|---|---|---|---|
| MMRN2;ENG;IGFALS | 0.74 | 0.70 | 0.79 | 0.84 | 0.78 | 0.90 | 0.71 | 0.65 | 0.76 |
| alcoh;ADAM12;IGFALS | 0.74 | 0.70 | 0.79 | 0.81 | 0.74 | 0.87 | 0.72 | 0.66 | 0.77 |
| fhpet;IGFALS;MCAM | 0.74 | 0.69 | 0.79 | 0.80 | 0.73 | 0.87 | 0.72 | 0.66 | 0.77 |
| bmi;ENG;SPINT1 | 0.74 | 0.69 | 0.79 | 0.84 | 0.77 | 0.91 | 0.70 | 0.65 | 0.76 |
| MMRN2;IGFALS;vagbl | 0.74 | 0.69 | 0.79 | 0.80 | 0.74 | 0.87 | 0.72 | 0.66 | 0.77 |
| fihd;MMRN2;IGFALS | 0.74 | 0.69 | 0.79 | 0.81 | 0.74 | 0.87 | 0.71 | 0.66 | 0.77 |
| alcoh;IGFALS;sispet | 0.74 | 0.69 | 0.78 | 0.80 | 0.73 | 0.86 | 0.72 | 0.66 | 0.77 |
| BP;CST3;PIGF | 0.74 | 0.69 | 0.78 | 0.80 | 0.72 | 0.87 | 0.72 | 0.66 | 0.77 |
| CST3;SPINT1;IGFALS | 0.74 | 0.69 | 0.79 | 0.81 | 0.74 | 0.88 | 0.71 | 0.65 | 0.77 |
| fhpet;ADAM12;IGFALS | 0.74 | 0.69 | 0.78 | 0.82 | 0.75 | 0.88 | 0.71 | 0.65 | 0.76 |
| MMRN2;IGFALS;IL6ST | 0.74 | 0.69 | 0.78 | 0.82 | 0.76 | 0.88 | 0.70 | 0.65 | 0.76 |
| ENG;QSOX1;IGFALS | 0.74 | 0.69 | 0.79 | 0.82 | 0.75 | 0.89 | 0.70 | 0.65 | 0.76 |
| fhpet;SPINT1;IGFALS | 0.74 | 0.69 | 0.78 | 0.82 | 0.75 | 0.88 | 0.71 | 0.65 | 0.76 |
| fhpet;CST3;IGFALS | 0.74 | 0.69 | 0.78 | 0.80 | 0.72 | 0.87 | 0.71 | 0.66 | 0.77 |
| ADAM12;PIGF;waist | 0.74 | 0.69 | 0.78 | 0.82 | 0.73 | 0.90 | 0.71 | 0.65 | 0.76 |
| alcoh;BP;ADAM12 | 0.74 | 0.69 | 0.78 | 0.82 | 0.74 | 0.90 | 0.70 | 0.65 | 0.75 |
| BP;ENG;MCAM | 0.74 | 0.69 | 0.78 | 0.81 | 0.73 | 0.89 | 0.71 | 0.65 | 0.76 |
| BP;ADAM12;CST3 | 0.74 | 0.69 | 0.78 | 0.81 | 0.73 | 0.88 | 0.71 | 0.66 | 0.76 |
| fihd;ADAM12;IGFALS | 0.74 | 0.69 | 0.78 | 0.80 | 0.73 | 0.87 | 0.71 | 0.65 | 0.76 |
| ADAM12;IGFALS;ROBO4 | 0.73 | 0.69 | 0.78 | 0.81 | 0.75 | 0.87 | 0.70 | 0.65 | 0.76 |
| ADAM12;QSOX1;IGFALS | 0.73 | 0.69 | 0.78 | 0.82 | 0.75 | 0.88 | 0.70 | 0.65 | 0.76 |
| fhpet;ENG;IGFALS | 0.73 | 0.69 | 0.78 | 0.81 | 0.74 | 0.88 | 0.71 | 0.65 | 0.76 |
| ADAM12;CST3;IGFALS | 0.73 | 0.69 | 0.78 | 0.80 | 0.73 | 0.87 | 0.71 | 0.65 | 0.76 |
| BP;PRDX2;IGFALS | 0.73 | 0.68 | 0.78 | 0.80 | 0.73 | 0.88 | 0.71 | 0.65 | 0.77 |
| ENG;IGFALS;ROBO4 | 0.73 | 0.69 | 0.78 | 0.81 | 0.74 | 0.87 | 0.71 | 0.65 | 0.76 |
| ADAM12;CST3;PIGF | 0.73 | 0.69 | 0.78 | 0.81 | 0.72 | 0.89 | 0.71 | 0.65 | 0.76 |
| fhpet;IGFALS;ROBO4 | 0.73 | 0.69 | 0.78 | 0.80 | 0.74 | 0.87 | 0.71 | 0.65 | 0.76 |
| SPINT1;IGFALS;ROBO4 | 0.73 | 0.68 | 0.78 | 0.80 | 0.73 | 0.87 | 0.70 | 0.65 | 0.76 |
| SPINT1;IGFALS;mothpet | 0.73 | 0.68 | 0.78 | 0.80 | 0.73 | 0.87 | 0.70 | 0.65 | 0.76 |
| LNPEP;SPINT1;IGFALS | 0.73 | 0.68 | 0.78 | 0.82 | 0.75 | 0.88 | 0.70 | 0.64 | 0.75 |
| bmi;BP;ADAM12 | 0.73 | 0.68 | 0.78 | 0.80 | 0.72 | 0.88 | 0.70 | 0.65 | 0.76 |
| ENG;SPINT1;waist | 0.73 | 0.68 | 0.78 | 0.84 | 0.77 | 0.91 | 0.69 | 0.63 | 0.74 |
| BP;ENG;SPINT1 | 0.73 | 0.68 | 0.78 | 0.86 | 0.79 | 0.92 | 0.68 | 0.62 | 0.74 |
| BP;ADAM12;waist | 0.73 | 0.68 | 0.78 | 0.81 | 0.72 | 0.89 | 0.70 | 0.65 | 0.75 |
| BP;LNPEP;PIGF | 0.73 | 0.68 | 0.77 | 0.81 | 0.74 | 0.89 | 0.70 | 0.64 | 0.75 |
| IGFALS;ROBO4;sispet | 0.73 | 0.68 | 0.77 | 0.80 | 0.74 | 0.87 | 0.70 | 0.64 | 0.75 |
| MMRN2;PRDX2;IGFALS | 0.73 | 0.68 | 0.78 | 0.82 | 0.75 | 0.89 | 0.70 | 0.64 | 0.76 |
| BP;ADAM12;MCAM | 0.72 | 0.68 | 0.77 | 0.82 | 0.74 | 0.90 | 0.69 | 0.63 | 0.74 |

(continued)

| Panel composition | CA | CB | CC | CD | CE | CF | CG | CH | CI |
|---|---|---|---|---|---|---|---|---|---|
| alcoh;BP;PlGF | 0.72 | 0.68 | 0.77 | 0.82 | 0.74 | 0.89 | 0.69 | 0.63 | 0.75 |
| ADAM12;MCAM;PlGF | 0.72 | 0.67 | 0.77 | 0.84 | 0.76 | 0.92 | 0.68 | 0.63 | 0.74 |
| ENG;CST3;SPINT1 | 0.72 | 0.68 | 0.77 | 0.84 | 0.77 | 0.92 | 0.68 | 0.62 | 0.73 |
| alcoh;BP;ENG | 0.72 | 0.67 | 0.77 | 0.80 | 0.72 | 0.89 | 0.69 | 0.63 | 0.75 |
| BP;ADAM12;PRDX2 | 0.72 | 0.67 | 0.77 | 0.83 | 0.76 | 0.91 | 0.69 | 0.63 | 0.75 |
| PRDX2;IGFALS;MCAM | 0.72 | 0.67 | 0.78 | 0.81 | 0.72 | 0.89 | 0.70 | 0.63 | 0.76 |
| BP;ENG;PlGF | 0.72 | 0.67 | 0.77 | 0.81 | 0.73 | 0.89 | 0.69 | 0.63 | 0.75 |
| BP;ADAM12;ENG | 0.72 | 0.67 | 0.77 | 0.81 | 0.74 | 0.89 | 0.68 | 0.63 | 0.74 |
| BP;MMRN2;ADAM12 | 0.72 | 0.67 | 0.77 | 0.83 | 0.76 | 0.90 | 0.68 | 0.62 | 0.74 |
| ADAM12;ENPP2;PlGF | 0.72 | 0.67 | 0.77 | 0.80 | 0.72 | 0.89 | 0.69 | 0.63 | 0.74 |
| BP;ADAM12;PROC | 0.72 | 0.66 | 0.77 | 0.83 | 0.73 | 0.92 | 0.69 | 0.62 | 0.75 |
| BP;ADAM12;ENPP2 | 0.72 | 0.67 | 0.77 | 0.80 | 0.72 | 0.88 | 0.69 | 0.63 | 0.74 |
| BP;fihd;ADAM12 | 0.72 | 0.67 | 0.77 | 0.80 | 0.72 | 0.88 | 0.68 | 0.63 | 0.74 |
| ENG;SPINT1;MCAM | 0.72 | 0.67 | 0.77 | 0.84 | 0.77 | 0.91 | 0.67 | 0.62 | 0.73 |
| ADAM12;PRCP;PlGF | 0.72 | 0.67 | 0.77 | 0.82 | 0.74 | 0.90 | 0.68 | 0.62 | 0.74 |
| BP;ENG;PRCP | 0.72 | 0.67 | 0.76 | 0.81 | 0.74 | 0.88 | 0.68 | 0.63 | 0.74 |
| PRDX2;SPINT1;IGFALS | 0.72 | 0.66 | 0.77 | 0.81 | 0.73 | 0.89 | 0.69 | 0.62 | 0.75 |
| ADAM12;LCAT;PlGF | 0.72 | 0.67 | 0.77 | 0.80 | 0.72 | 0.89 | 0.68 | 0.63 | 0.74 |
| CST3;PRCP;PlGF | 0.71 | 0.67 | 0.76 | 0.80 | 0.73 | 0.87 | 0.69 | 0.63 | 0.74 |
| BP;fhpet;ADAM12 | 0.71 | 0.67 | 0.76 | 0.80 | 0.72 | 0.89 | 0.68 | 0.62 | 0.74 |
| BP;ALDOA;PlGF | 0.71 | 0.67 | 0.76 | 0.80 | 0.73 | 0.87 | 0.68 | 0.63 | 0.74 |
| ADAM12;ENG;SPINT1 | 0.71 | 0.67 | 0.76 | 0.89 | 0.84 | 0.95 | 0.65 | 0.59 | 0.70 |
| BP;MCAM;PlGF | 0.71 | 0.66 | 0.76 | 0.82 | 0.74 | 0.89 | 0.68 | 0.62 | 0.74 |
| BP;pbwgt;PlGF | 0.71 | 0.66 | 0.77 | 0.81 | 0.72 | 0.89 | 0.68 | 0.62 | 0.74 |
| ADAM12;PRDX2;*waist* | 0.71 | 0.66 | 0.77 | 0.80 | 0.71 | 0.88 | 0.69 | 0.63 | 0.75 |
| BP;ADAM12;pbwgt | 0.71 | 0.66 | 0.76 | 0.81 | 0.73 | 0.89 | 0.68 | 0.62 | 0.73 |
| BP;fihd;PlGF | 0.71 | 0.66 | 0.76 | 0.80 | 0.72 | 0.88 | 0.68 | 0.62 | 0.74 |
| BP;ADAM12;SPINT1 | 0.71 | 0.66 | 0.76 | 0.83 | 0.76 | 0.91 | 0.67 | 0.61 | 0.72 |
| bmi;ADAM12;SPINT1 | 0.71 | 0.66 | 0.76 | 0.80 | 0.73 | 0.87 | 0.68 | 0.62 | 0.74 |
| ADAM12;SPINT1;PlGF | 0.71 | 0.66 | 0.76 | 0.87 | 0.79 | 0.94 | 0.66 | 0.60 | 0.72 |
| ADAM12;pbwgt;PlGF | 0.71 | 0.66 | 0.76 | 0.81 | 0.73 | 0.89 | 0.68 | 0.62 | 0.74 |
| BP;ENPP2;PlGF | 0.71 | 0.66 | 0.76 | 0.80 | 0.72 | 0.87 | 0.68 | 0.62 | 0.74 |
| BP;PRDX2;PlGF | 0.71 | 0.66 | 0.76 | 0.81 | 0.73 | 0.90 | 0.68 | 0.62 | 0.74 |
| BP;IL6ST;PlGF | 0.71 | 0.66 | 0.76 | 0.80 | 0.73 | 0.88 | 0.67 | 0.62 | 0.73 |
| ADAM12;PROC;MCAM | 0.71 | 0.65 | 0.76 | 0.80 | 0.71 | 0.89 | 0.68 | 0.62 | 0.74 |
| ADAM12;CST3;SPINT1 | 0.71 | 0.66 | 0.75 | 0.83 | 0.76 | 0.89 | 0.66 | 0.60 | 0.71 |
| alcoh;ADAM12;PlGF | 0.71 | 0.66 | 0.76 | 0.82 | 0.74 | 0.89 | 0.67 | 0.61 | 0.72 |
| MMRN2;ADAM12;PlGF | 0.71 | 0.65 | 0.76 | 0.82 | 0.74 | 0.91 | 0.66 | 0.60 | 0.72 |

(continued)

| Panel composition | CA | CB | CC | CD | CE | CF | CG | CH | CI |
|---|---|---|---|---|---|---|---|---|---|
| fhpet;ADAM12;PlGF | 0.70 | 0.65 | 0.76 | 0.81 | 0.73 | 0.89 | 0.67 | 0.61 | 0.73 |
| BP;MMRN2;ENG | 0.70 | 0.65 | 0.76 | 0.81 | 0.74 | 0.88 | 0.66 | 0.60 | 0.72 |
| MAPRE1/3;ALDOA;PlGF | 0.70 | 0.65 | 0.75 | 0.81 | 0.74 | 0.89 | 0.66 | 0.61 | 0.72 |
| ADAM12;ECM1;PlGF | 0.70 | 0.65 | 0.75 | 0.80 | 0.71 | 0.88 | 0.67 | 0.61 | 0.73 |
| ADAM12;PlGF;sispet | 0.70 | 0.65 | 0.75 | 0.82 | 0.74 | 0.89 | 0.66 | 0.60 | 0.72 |
| ADAM12;PRDX2;PlGF | 0.70 | 0.65 | 0.76 | 0.82 | 0.71 | 0.92 | 0.67 | 0.60 | 0.73 |
| ENG;LCAT;SPINT1 | 0.70 | 0.65 | 0.75 | 0.83 | 0.75 | 0.90 | 0.65 | 0.59 | 0.71 |
| alcoh;ENG;SPINT1 | 0.70 | 0.65 | 0.75 | 0.84 | 0.78 | 0.91 | 0.64 | 0.59 | 0.70 |
| fihd;ADAM12;PlGF | 0.70 | 0.65 | 0.75 | 0.81 | 0.73 | 0.89 | 0.66 | 0.60 | 0.72 |
| fhpet;ENG;SPINT1 | 0.70 | 0.65 | 0.75 | 0.83 | 0.75 | 0.90 | 0.65 | 0.59 | 0.71 |
| ENG;SPINT1;ENPP2 | 0.70 | 0.65 | 0.75 | 0.83 | 0.75 | 0.90 | 0.65 | 0.59 | 0.71 |
| alcoh;ADAM12;SPINT1 | 0.70 | 0.65 | 0.75 | 0.83 | 0.76 | 0.91 | 0.64 | 0.59 | 0.70 |
| ENG;PRCP;SPINT1 | 0.70 | 0.65 | 0.75 | 0.83 | 0.77 | 0.90 | 0.64 | 0.58 | 0.70 |
| BP;PRDX2;MCAM | 0.70 | 0.64 | 0.75 | 0.81 | 0.74 | 0.88 | 0.66 | 0.59 | 0.72 |
| ENG;SPINT1;mothpet | 0.69 | 0.65 | 0.74 | 0.82 | 0.74 | 0.89 | 0.65 | 0.59 | 0.70 |
| BP;ENG;PRDX2 | 0.69 | 0.64 | 0.75 | 0.80 | 0.72 | 0.88 | 0.66 | 0.60 | 0.72 |
| ECM1;ENG;SPINT1 | 0.69 | 0.64 | 0.74 | 0.82 | 0.75 | 0.89 | 0.64 | 0.58 | 0.70 |
| ENG;SPINT1;PlGF | 0.69 | 0.64 | 0.74 | 0.85 | 0.77 | 0.92 | 0.64 | 0.58 | 0.70 |
| ADAM12;PRDX2;SPINT1 | 0.69 | 0.64 | 0.75 | 0.87 | 0.81 | 0.93 | 0.63 | 0.57 | 0.70 |
| ADAM12;PRDX2;MCAM | 0.69 | 0.64 | 0.75 | 0.81 | 0.73 | 0.89 | 0.65 | 0.59 | 0.72 |
| ENG;PRCP;PlGF | 0.69 | 0.64 | 0.74 | 0.80 | 0.71 | 0.88 | 0.65 | 0.59 | 0.71 |
| alcoh;MCAM;PlGF | 0.69 | 0.64 | 0.74 | 0.80 | 0.72 | 0.87 | 0.65 | 0.59 | 0.71 |
| PRDX2;PRCP;PlGF | 0.69 | 0.63 | 0.74 | 0.81 | 0.72 | 0.91 | 0.65 | 0.59 | 0.71 |
| ENG;PCDH12;SPINT1 | 0.69 | 0.64 | 0.74 | 0.84 | 0.77 | 0.91 | 0.63 | 0.57 | 0.69 |
| ADAM12;SPINT1;MCAM | 0.69 | 0.64 | 0.74 | 0.82 | 0.75 | 0.89 | 0.64 | 0.58 | 0.70 |
| fihd;ENG;SPINT1 | 0.69 | 0.63 | 0.74 | 0.83 | 0.75 | 0.90 | 0.63 | 0.57 | 0.69 |
| ENG;PRDX2;SPINT1 | 0.68 | 0.63 | 0.74 | 0.84 | 0.76 | 0.92 | 0.63 | 0.57 | 0.69 |
| ADAM12;LCAT;SPINT1 | 0.68 | 0.63 | 0.73 | 0.80 | 0.72 | 0.87 | 0.64 | 0.58 | 0.70 |
| ADAM12;PRCP;SPINT1 | 0.68 | 0.63 | 0.73 | 0.81 | 0.74 | 0.87 | 0.64 | 0.58 | 0.69 |
| fihd;ADAM12;SPINT1 | 0.68 | 0.63 | 0.73 | 0.84 | 0.78 | 0.90 | 0.62 | 0.56 | 0.68 |
| MMRN2;ENG;SPINT1 | 0.68 | 0.63 | 0.73 | 0.83 | 0.76 | 0.90 | 0.62 | 0.57 | 0.68 |
| ADAM12;SPINT1;ENPP2 | 0.68 | 0.63 | 0.73 | 0.81 | 0.74 | 0.88 | 0.63 | 0.57 | 0.69 |
| ADAM12;PROC;SPINT1 | 0.68 | 0.62 | 0.74 | 0.85 | 0.77 | 0.93 | 0.63 | 0.57 | 0.69 |
| fhpet;ADAM12;SPINT1 | 0.68 | 0.63 | 0.73 | 0.82 | 0.75 | 0.89 | 0.62 | 0.56 | 0.68 |
| ADAM12;SPINT1;mothpet | 0.68 | 0.63 | 0.73 | 0.82 | 0.75 | 0.88 | 0.62 | 0.56 | 0.68 |
| MMRN2;ADAM12;PRDX2 | 0.67 | 0.62 | 0.73 | 0.80 | 0.71 | 0.89 | 0.63 | 0.57 | 0.70 |
| MCAM;PlGF;sispet | 0.67 | 0.62 | 0.73 | 0.80 | 0.72 | 0.87 | 0.63 | 0.57 | 0.69 |
| ADAM12;ECM1;SPINT1 | 0.67 | 0.62 | 0.73 | 0.80 | 0.73 | 0.88 | 0.62 | 0.56 | 0.68 |

(continued)

| Panel composition | CA | CB | CC | CD | CE | CF | CG | CH | CI |
|---|---|---|---|---|---|---|---|---|---|
| ADAM12;PCDH12;SP1NT1 | 0.67 | 0.62 | 0.72 | 0.82 | 0.75 | 0.89 | 0.62 | 0.56 | 0.68 |
| ADAM12;PRDX2;*sispet* | 0.67 | 0.61 | 0.73 | 0.80 | 0.72 | 0.88 | 0.63 | 0.56 | 0.69 |
| LNPEP;SPINT1;PIGF | 0.67 | 0.62 | 0.72 | 0.82 | 0.76 | 0.89 | 0.62 | 0.56 | 0.67 |
| ENG;PROC;SPINT1 | 0.67 | 0.61 | 0.72 | 0.80 | 0.69 | 0.90 | 0.63 | 0.56 | 0.69 |
| LNPEP;PRDX2;SPINT1 | 0.65 | 0.60 | 0.71 | 0.81 | 0.74 | 0.87 | 0.60 | 0.54 | 0.67 |

**Table 13H**

| Panel composition | CA | CB | CC | CD | CE | CF | CG | CH | CI |
|---|---|---|---|---|---|---|---|---|---|
| MMRN2;IGFALS;PIGF | 0.76 | 0.72 | 0.81 | 0.85 | 0.79 | 0.91 | 0.73 | 0.68 | 0.79 |
| ADAM12;IGFALS;PIGF | 0.76 | 0.72 | 0.81 | 0.85 | 0.79 | 0.92 | 0.73 | 0.68 | 0.78 |
| ENG;SPINT1;IGFALS | 0.76 | 0.71 | 0.80 | 0.88 | 0.82 | 0.94 | 0.71 | 0.66 | 0.77 |
| MMRN2;ADAM12;IGFALS | 0.75 | 0.70 | 0.79 | 0.85 | 0.80 | 0.90 | 0.71 | 0.65 | 0.76 |
| ADAM12;SPINT1;IGFALS | 0.75 | 0.70 | 0.79 | 0.86 | 0.80 | 0.91 | 0.70 | 0.65 | 0.76 |
| BP;ENG;SPINT1 | 0.73 | 0.68 | 0.78 | 0.86 | 0.79 | 0.92 | 0.68 | 0.62 | 0.74 |
| ADAM12;ENG;SPINT1 | 0.71 | 0.67 | 0.76 | 0.89 | 0.84 | 0.95 | 0.65 | 0.59 | 0.70 |
| ADAM12;SPINT1;PIGF | 0.71 | 0.66 | 0.76 | 0.87 | 0.79 | 0.94 | 0.66 | 0.60 | 0.72 |
| ENG;SPINT1;PIGF | 0.69 | 0.64 | 0.74 | 0.85 | 0.77 | 0.92 | 0.64 | 0.58 | 0.70 |
| ADAM12;PRDX2;SPINT1 | 0.69 | 0.64 | 0.75 | 0.87 | 0.81 | 0.93 | 0.63 | 0.57 | 0.70 |
| ADAM12;PROC;SPINT1 | 0.68 | 0.62 | 0.74 | 0.85 | 0.77 | 0.93 | 0.63 | 0.57 | 0.69 |

## Example 14: Further illustrative test panels for the prediction of PE

Additional analyses of the data using statistical methods comparable to the above identified particularly well-performing and promising test panels for the prediction of preeclampsia at 20 weeks of gestation, which may also be useful throughout the second trimester, such as between 13 and 28 weeks of gestation, e.g., at 20 +/- 2, 20 +/- 1, 15 +/- 2 or 15 +/- 1 weeks of gestation, and can even be applied with success in the first trimester. The panels are summarised in Table 14 (BP preferably denotes MAP herein).

**Table 14**

| Panel composition |
|---|
| BP20;sEng;SPINT1;IGFALS;MCAM;PIGF |
| BP20;ADAM12;ECM1;MCAM;PIGF |
| BP20;MMRN2;sEng;MAPRE1/3;IGFALS;ALDOA |
| BP20;sEng;SEPP1;IGFALS;MCAM;PIGF |
| BP20;MMRN2;sEng;SPINT1;SEPP1;IGFALS |
| BP20;sEng;SPINT1;SEPP1;IGFALS;PIGF |
| sEng;SPINT1;SEPP1;IGFALS;MCAM;PIGF |
| sEng;SPINT1;IGFALS;MCAM;PIGF |

**Claims**

1. An *in vitro* method for the prediction of pre-eclampsia (PE) in a subject comprising testing or evaluating a sample obtained from the subject, the method comprising:

   - measurement of the level of placental growth factor (PIGF) in the subject; and
   - measurement of the level of insulin-like growth factor-binding protein complex acid labile subunit (IGFALS) in the subject.

2. An *in vitro* method according to Claim 1, wherein the method further comprises:

   - i) measurement of the level of multimerin-2 (MMRN2) in the subject.

3. An *in vitro* method according to Claim 1, wherein the method further comprises two or more measurements selected from the group consisting of: measurement of the level of cell surface glycoprotein (CD146, MUC18, MCAM) in the subject, measurement of the level of endoglin (soluble endoglin, s-ENG or ENG) in the subject, measurement of the level of disintegrin and metalloproteinase domain-containing protein 12 (ADAM12) in the subject, measurement of the level of multimerin-2 (MMRN2) in the subject, measurement of the level of Kunitz-type protease inhibitor 1 (SPINT1) in the subject, measurement of the level of sulfhydryl oxidase 1 (QSOX1) in the subject, measurement of the level of selenoprotein P (SEPP1) in the subject, measurement of the level of extracellular matrix protein 1 (ECM1) in the subject, measurement of the level of roundabout homolog 4 (ROBO4) in the subject, measurement of the level of leucyl-cystinyl aminopeptidase (LNPEP, OTASE) in the subject, measurement of the level of fructose-bisphosphate aldolase A (ALDOA) in the subject, measurement of the level of microtubule-associated protein RP (MAPRE1 and/or 3) in the subject, measurement of the level of ectonucleotide pyrophosphatase/phosphodiesterase family member 2 (ENPP2) in the subject, measurement of the level of phosphatidylcholine-sterol acyltransferase (LCAT) in the subject, measurement of the level of peroxiredoxin-2 (PRDX2) in the subject, measurement of the level of lysosomal Pro-X carboxypeptidase (PRCP) in the subject, measurement of the level of trefoil factor 3 (TFF3) in the subject, measurement of the level of cystatin-C (CST3) in the subject, measurement of the level of C-reactive protein (CRP) in the subject, measurement of the level of collagen alpha-3(VI) chain (COL6A3) in the subject, measurement of the level of Interleukin-6 receptor subunit beta (IL6ST) in the subject, measurement of the level of Vitamin K-dependent protein C (PROC) in the subject, measurement of the level of Protocadherin-12 (PCDH12) in the subject, measurement of blood pressure of the subject (BP), a score for the parameter 'alcohol consumed in the 1st trimester' (yes/no) (esp. 1st trimester) *("alcoh"),* measurement of body mass index of the subject (bmi), a score for the maternal history parameter 'father of subject has/had ischemic heart disease' in the subject *("father any ihd"* or *"fihd'),* a score for the maternal history parameter 'mother or sister of subject has/had preeclampsia' in the subject *("fh_pet" or "fhpet'),* a score for the parameter 'occurrence of vaginal bleeding (esp. for (more than) 5 days before 15 weeks visit)' (yes/no) *("vagbl"),* a value for the parameter 'birth weight of the subject' *("pbwgr"),* a value for the parameter 'the gestational *age* at blood sampling calculated from the date of the last menstrual period and/or from an ultrasound dating scan' *("gest'),* a value for the parameter 'age of the subject' *("age"),* a score for the maternal history parameter 'mother of subject has/had preeclampsia' *("mothpet'),* a score for the maternal history parameter 'sister of subject has/had preeclampsia' *("sispet"),* and measurement of the *waist* circumference in the subject *("waist').*

4. A method according to any one of Claims 1 to 3, wherein the sample is whole blood, plasma, serum, or a cell pellet.

**Patentansprüche**

1. In-Vitro-Methode zur Vorhersage von Präeklampsie (PE) bei einer Patientin, umfassend Versuche oder Bewertung einer von der Patientin stammenden Probe, wobei die Methode folgendes umfasst:

   - Messung des Spiegels von Plazenta-Wachstumsfaktor (PIGF) bei der Patientin; und

- Messung des Spiegels der säurelabilen Subeinheit des insulin-ähnlichen Wachstumsfaktor bindenden Proteinkomplexes (IGFALS) bei der Patientin.

2. In-Vitro-Methode gemäß Anspruch 1, worin die Methode außerdem folgendes umfasst:

- Messung des Multimerin-2-Spiegels (MMRN2) bei der Patientin.

3. In-Vitro-Methode gemäß Anspruch 1, worin die Methode außerdem zwei oder mehr Messungen aus der nachfolgenden Gruppe umfasst: Messung des Spiegels des Zelloberflächen-Glykoproteins (CD 146, MUC18, MCAM) bei der Patientin, Messung des Endoglin-Spiegels (lösliches Endoglin, s-ENG oder ENG) bei der Patientin, Messung des Spiegels von Disintegrin und Metall-Proteinase enthaltendem Protein 12 (ADAM 12) bei der Patientin, Messung des Multimerin-2-Spiegels (MMRN2) bei der Patientin, Messung des Spiegels von Kunitz-artigem Protease-Inhibitor 1 (SPINT1) bei der Patientin, Messung des Spiegels von Sulfhydryl-Oxidase 1 (QSOX1) bei der Patientin, Messung des Selenoprotein-P-Spiegels (SEPP1) bei der Patientin, Messung des Spiegels von extrazellulärem Matrixprotein 1 (ECM1) bei der Patientin, Messung des Spiegels von Robo-Homologprotein 4 (ROBO4) bei der Patientin, Messung des Spiegels von Zeukyl/Zystinyl-Aminopeptidase (LNPEP, OTASE) bei der Patientin, Messung des Spiegels von Fruktose-Bisphosphat-Aldolase A (ALDOA) bei der Patientin, Messung des Spiegels von Mikrotubuli-assoziiertem Protein RP (MAPRE 1 und/oder 3) bei der Patientin, Messung des Spiegels von Ectonukleotid-Pyrophosphatase/Phosphodiesterase, Familienmitglied 2 (ENPP2) bei der Patientin, Messung des Spiegels von Phosphatidylcholin-Sterol-Acyltransferase (LCAT) bei der Patientin, Messung des Spiegels von Peroxiredoxin-2 (PRDX2) bei der Patientin, Messung des Spiegels von lysosomaler Pro-X Karboxypeptidase (PRCP) bei der Patientin, Messung des Spiegels von Trefoil Factor 3-Peptid (TFF3) bei der Patientin, Messung des Spiegels von Zystatin-C (CST3) bei der Patientin, Messung des Spiegels von C-reaktivem Protein (CRP) bei der Patientin, Messung des Spiegels der Kollagen-Alpha-3(VI)-Kette (COL6A3) bei der Patientin, Messung des Spiegels der Interleukin-6-Rezeptoren-Subeinheit Beta (IL6ST) bei der Patientin, Messung des Spiegels von Vitamin-K-dependentem Protein C (PROC) bei der Patientin, Messung des Spiegels von Protocadherin-12 (PCDH12) bei der Patientin, Messung des Blutdrucks der Patientin (BP), ein Ergebnis für den Parameter "Alkoholverzehr im 1. Vierteljahr" (ja/nein) (spez. 1. Vierteljahr) *("alcoh"),* Berechnung des Body Mass Index (bmi) der Patientin, ein Ergebnis für die familiäre Vorgeschichte der Patientin mit Parametern "Vater der Patientin hat/hatte eine ischämische Herzkrankheit" (*"father any ihd"* oder *"fihd"*), ein Ergebnis für die familiäre Vorgeschichte der Patientin mit Parametern "Mutter oder Schwester der Patientin haben/hatten Präeklampsie" *("fh_pet" oder "fhpet"),* ein Ergebnis für den Parameter "Auftreten von vaginalen Blutungen (speziell (mehr als) 5 Tage vor der Untersuchung der 15. Woche)" (ja/nein) *("vagbl"),* ein Wert für den Parameter "Geburtsgewicht der Patientin" *("pbwgr"),* ein Wert für den Parameter "Schwangerschaftsalter auf Grundlage der Blutprobe, berechnet ab Datum der letzten Menstruation und/oder der Datierung der Schwangerschaft durch Ultraschall" *("gest'),* ein Wert für den Parameter "Alter der Patientin" *("age"),* ein Ergebnis für die mütterliche Vorgeschichte mit Parameter "Mutter der Patientin hat/hatte Präeklampsie" *("mothpet"),* ein Ergebnis für die mütterliche Vorgeschichte mit Parameter "Schwester der Patientin hat/hatte Präeklampsie" *("sispet"),* und Messung des *Taillen*umfangs der Patientin *("waist").*

4. Eine Methode gemäß einem der Ansprüche 1 bis 3, worin die Probe aus Vollblut, aus Plasma, aus Serum oder ein Zellpellet ist.

## Revendications

1. Méthode *in vitro* pour la prédiction de la pré-éclampsie (PE) chez un sujet comprenant le test ou l'évaluation d'un échantillon obtenu chez le sujet, la méthode comprenant :

- la mesure du niveau du facteur de croissance placentaire (PIGF) chez le sujet ; et
- la mesure du niveau de la sous-unité acide labile du complexe protéique de liaison au facteur de croissance apparenté à l'insuline (IGFALS) chez le sujet.

2. Méthode *in vitro* selon la revendication 1, dans laquelle la méthode comprend en outre :

- i) la mesure du niveau de multimérine-2 (MMRN2) chez le sujet.

3. Méthode *in vitro* selon la revendication 1, dans laquelle la méthode comprend en outre deux ou plusieurs mesures sélectionnées dans le groupe constitué de : mesure du niveau de la glycoprotéine de surface cellulaire (CD146, MUC18, MCAM) chez le sujet, mesure du niveau d'endogline (endogline soluble, s-ENG ou ENG) chez le sujet, mesure du niveau de disintégrine et de la protéine 12 contenant le domaine de la métalloprotéinase (ADAM 12) chez le sujet, mesure du niveau de multimérine-2 (MMRN2) chez le sujet, mesure du niveau d'inhibiteur 1 de la protéase de type Kunitz (SPINT1) chez le sujet, mesure du niveau de sulfhydryle oxydase 1 (QSOX1) chez le sujet, mesure du niveau de sélénoprotéine P (SEPP1) chez le sujet, mesure du niveau de protéine 1 de la matrice extracellulaire (ECM1) chez le sujet, mesure du niveau de l'homologue 4 « roundabout » (ROB04) chez le sujet, mesure du niveau de la leucyl-cystinyl aminopeptidase (LNPEP, OTASE) chez le sujet, mesure du niveau de l'aldolase A fructose-bisphosphate (ALDOA) chez le sujet, mesure du niveau de la protéine RP associée aux microtubules (MAPRE1 et/ou 3) chez le sujet, mesure du niveau du membre 2 de la famille de l'ectonucléotide pyrophosphatase / phosphodiestérase (ENPP2) chez le sujet, mesure du niveau de l'acyltransférase phosphatidylcholine-stérol (LCAT) chez le sujet, mesure du niveau de péroxirédoxine 2 (PRDX2) chez le sujet, mesure du niveau de carboxypeptidase lysosomale Pro-X (PRCP) chez le sujet, mesure du niveau du facteur 3 du trèfle (TFF3) chez le sujet, mesure du niveau de la cystatine C (CST3) chez le sujet, mesure du niveau de la protéine C-réactive (CRP) chez le sujet, mesure du niveau de la chaîne alpha 3 du collagène type VI (COL6A3) chez le sujet, mesure du niveau de la sous-unité bêta du récepteur de l'interleukine 6 (IL6ST) chez le sujet, mesure du niveau de protéine C dépendante de la vitamine K (PROC) chez le sujet, mesure du niveau de Protocadhérine 12 (PCDH12) chez le sujet, mesure de la pression sanguine du sujet (BP), score pour le paramètre « alcool consommé pendant le 1er trimestre » (oui/non) (en particulier 1er trimestre) (« *alcoh* »), mesure de l'index de masse corporelle du sujet (bmi), score pour le paramètre antécédent paternel (« père du sujet a/a eu une cardiopathie ischémique » chez le sujet (« *father any ihd* » ou « *fihd* »), score pour le paramètre antécédent maternel « mère ou soeur du sujet a/a eu une pré-éclampsie » chez le suj et *(« fh_pet » ou « fhpet »),* score pour le paramètre « occurrence de saignements vaginaux, en particulier pour (plus de) 5 jours avant la visite de la 15ème semaine (oui/non) (« *vagbl* »), valeur pour le paramètre « poids de naissance du sujet » (« *pbwgr* »), valeur pour le paramètre « *âge* gestationnel de l'échantillon de sang calculé à partir de la date de la dernière période menstruelle et/ou à partir d'une échographie de datation » (« *gest* »), valeur pour le paramètre « âge du sujet » (« *age* »), core pour le paramètre antécédent maternel « mère du sujet a/a eu une pré-éclampsie » (« *mothpet* »), score pour le paramètre antécédent maternel « soeur du sujet a/a eu une pré-éclampsie » (« *sispet* »), et mesure de la circonférence du tour de taille chez le sujet (« *waist* »).

4. Méthode selon l'une des revendications 1 à 3, dans laquelle l'échantillon est du sang total, plasma, sérum ou un culot cellulaire.

FIG 1

```
SEQ ID 30 1    MPVMRLFPCFLQLLAGLALPAVPPQQWALSAGNGSSEVEVVPFQEVWGRSYCRALERLVD   60   P49763-2 NP_001193941
SEQ ID 31 1    MPVMRLFPCFLQLLAGLALPAVPPQQWALSAGNGSSEVEVVPFQEVWGRSYCRALERLVD   60   P49763-1
SEQ ID 32 1    MPVMRLFPCFLQLLAGLALPAVPPQQWALSAGNGSSEVEVVPFQEVWGRSYCRALERLVD   60   P49763-3 NP_002623

SEQ ID 30 61   VVSEYPSEVEHMFSPSCVSLLRCTGCCGDENLHCVPVETANVTMQLLKIRSGDRPSYVEL  120   P49763-2 NP_001193941
SEQ ID 31 61   VVSEYPSEVEHMFSPSCVSLLRCTGCCGDENLHCVPVETANVTMQLLKIRSGDRPSYVEL  120   P49763-1
SEQ ID 32 61   VVSEYPSEVEHMFSPSCVSLLRCTGCCGDENLHCVPVETANVTMQLLKIRSGDRPSYVEL  120   P49763-3 NP_002623

SEQ ID 30 121  TFSQHVRCECR-------------------------------------------------  131   P49763-2 NP_001193941
SEQ ID 31 121  TFSQHVRCECRHSPGRQSPDMPGDFRADAPSFLPPRRSLPMLFRMEWGCALTGSQSAVWP  180   P49763-1
SEQ ID 32 121  TFSQHVRCECR----------------------PLR----------------------   134   P49763-3 NP_002623

SEQ ID 30 132  -----------------------PLREKMKP---ERCGDAVPRR                149   P49763-2 NP_001193941
SEQ ID 31 181  SSPVPEEIPRMHPGRNGKKQQRKPLREKMKP---ERCGDAVPRR                221   P49763-1
SEQ ID 32 135  --------EKMKPERRRPKGRGKRRREKQRPTDCHLCGDAVPRR                170   P49763-3 NP_002623
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009094665 A
- WO 2011128357 A
- WO 2004008946 A
- WO 2010059952 A
- WO 2009097584 A1
- WO 2009108073 A1
- US 2006105415 A
- US 4816567 A
- US 5270163 A
- US 5578577 A
- US 5141850 A
- US 4775636 A
- US 4853335 A
- US 4859612 A
- US 5079172 A
- US 5202267 A
- US 5514602 A
- US 5616467 A
- US 5681775 A
- US 6107045 A
- US 6974706 B
- US 5108889 A
- US 6027944 A
- US 6482156 B
- US 6511814 B
- US 5824268 A
- US 5726010 A
- US 6001658 A
- US 20080090305 A
- US 20030109067 A

### Non-patent literature cited in the description

- **SOLOMON ; SEELY.** *Endocrinol Metab Clin North Am,* 2006, vol. 35 (1), 157-71
- **SIBAI ; BARTON.** *Am J Obstet Gynecol,* 2007, vol. 196 (6), 514e1-9
- **ROBERTS et al.** *Hypertension,* 2003, vol. 41 (3), 37-45
- **CONDE-AGUDELO et al.** *Obstet Gynecol,* 2004, vol. 104, 1367-91
- **LEWITT et al.** *Journal of Endocrinology,* 1998, vol. 159, 265
- **MISTRY et al.** *Hypertension,* 2008, vol. 52, 881
- **RAYMAN et al.** *Am J Obstet Gynecol,* 2003, vol. 189, 1343
- **PARK et al.** *Experimental and Molecular Medicine,* 2011, vol. 43 (7), 427-435
- **ROHRA ; QIDWAI.** *J. Pakistan Medical Association,* 2005, vol. 55 (2), 79-82
- **BUJOLD et al.** *Obstet Gynecol,* 2010, vol. 116, 402-14
- **MAYNARD et al.** *J Clin Invest,* 2003, vol. 111 (5), 649-58
- **POLLIOTTI et al.** *Obstet Gynecol,* 2003, vol. 101, 1266-74
- **ALBERTI et al.** *Diabetic Medicine,* 2006, vol. 23, 469-480
- **CRUZ et al.** Applications of Machine Learning in Cancer Prediction and Prognosis. *Cancer Informatics,* 2007, vol. 2, 59-77
- **LOWRY et al.** *J Biol Chem,* 1951, vol. 193, 265
- **HARTREE.** *Anal Biochem,* 1972, vol. 48, 422-427
- **KOHLER et al.** *Nature,* 1975, vol. 256, 495
- **CLACKSON et al.** *Nature,* 1991, vol. 352, 624-628
- **MARKS et al.** *J Mol Biol,* vol. 222, 581-597
- **HARLOW ; LANE.** Antibodies: A Laboratory Manual. Cold Spring Harbour Laboratory, 1988
- **HARLOW ; LANE.** Using Antibodies: A Laboratory Manual. Cold Spring Harbour Laboratory, 1999
- Monoclonal Antibodies: A Manual of Techniques. CRC Press, 1987
- Monoclonal Antibodies: A Practical Approach. Oxford University Press, 2000
- Antibody Engineering: Methods and Protocols. Methods in Molecular Biology. Humana Press, 2004, vol. 248
- **ELLINGTON ; SZOSTAK.** *Nature,* 1990, vol. 346, 818-822
- **TUERK ; GOLD.** *Science,* 1990, vol. 249, 505-510
- The Aptamer Handbook: Functional Oligonucleotides and Their Applications. Wiley-VCH, 2006
- **HORWELL.** *Trends Biotechnol,* 1995, vol. 13, 132-134
- **G. FRENS.** *Nature Physical Science,* 1973, vol. 241, 20

- **JOHN R. CROWTHER.** The ELISA Guidebook. Humana Press, 2000
- **NIELSEN ; GEIERSTANGER.** *J Immunol Methods,* 2004, vol. 290, 107-20
- **LING et al.** *Expert Rev Mol Diagn,* 2007, vol. 7, 87-98
- An Introduction to Radioimmunoassay and Related Techniques. Science. Elsevier, 1995
- Mass Spectrometry of Proteins and Peptides. Methods in Molecular Biology. Humana Press, 2000, vol. 146
- **BIEMANN.** *Methods Enzymol,* 1990, vol. 193, 455-79
- Biological Mass Spectrometry. Methods in Enzymology. Academic Press, 2005, vol. 402
- **KUHN et al.** *Proteomics 4,* 2004, vol. 1, 175-86
- **BIDLINGMEYER, B. A.** Practical HPLC Methodology and Applications. John Wiley & Sons Inc, 1993
- **ZHONG et al.** *Prenatal Diagnosis,* 2010, vol. 30, 293-308
- **ROYSTON et al.** Prognosis and prognostic research: Developing a prognostic model. *BMJ,* 2009, vol. 338, b604
- **TIBSHIRANI.** Regression shrinkage and selection via the lasso. *J. Royal. Statist. Soc B.,* 1996, vol. 58 (1), 267-288
- *BMJ,* 2011, vol. 342, d1875